# EUROPEAN PATENT APPLICATION

(11) **EP 4 471 046 A1**
(43) Date of publication of application: **04.12.2024**
(21) Application number: 23746278.3
(22) Date of filing: 19.01.2023
(51) Int. Cl.: C07J 9/00, C07J 71/00, A61P 3/06, A61P 9/10, A61P 9/12

(54) **STEROID COMPOUND, AND PREPARATION METHOD THEREFOR AND APPLICATION THEREOF**

(30) Priority: 28.01.2022 CN 202210107190; 22.07.2022 CN 202210869282
(71) Applicant: Cholesgen (Shanghai) Co., Ltd., Shanghai 201203 (CN)
(72) Inventor: LIU, Jinbiao, Shanghai 201203 (CN); TANG, Jingjie, Shanghai 201203 (CN)
(74) Representative: Calysta NV
(86) International application number: PCT/CN2023/073236
(87) International publication number: WO 2023/143399

(57) **Abstract**

Disclosed in the present invention are a steroid compound, and a preparation method therefor and an application thereof. The steroid compound may have a structure as shown in formula XXI, *etc.* The compound of the present invention has SREBP pathway inhibitory activity, and can be used for preventing and/or treating diseases such as obesity, hyperlipidemia, fatty liver, diabetes, atherosclerosis, cardiovascular and cerebrovascular diseases, liver cancer, and skin damage.

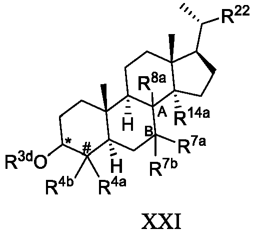

## Description

The present application claims the right of the priorities of Chinese patent application 2022101071901 filed on January 28, 2022 and Chinese patent application 2022108692823 filed on July 22, 2022. The contents of the above Chinese patent applications are incorporated herein by reference in their entireties.

### TECHNICAL FIELD

The present disclosure relates to a steroid compound, a preparation method therefor, and a use thereof.

### BACKGROUND

With the change in lifestyle, including intake of high-calorie food and high-sugar drinks, and lack of exercise and physical activity, metabolic diseases represented by hyperlipidemia, obesity, type 2 diabetes, and fatty liver have become an increasingly serious health issue globally. Among them, fatty liver has become a significant cause of chronic liver disease in Europe and America as well as affluent regions of China. The prevalence of simple hepatic lipid accumulation in ordinary adults ranges from 10% to 30%, of which 10% to 20% is steatohepatitis, with an incidence of 25% progressing to cirrhosis and liver cancer within ten years. However, as of now, the pathophysiological mechanism of fatty liver has not been fully elucidated, and there is still a lack of effective and specific therapeutic drugs in clinical practice. The accumulation of lipids such as cholesterol and triglycerides in the blood and liver is known to be a major cause of hyperlipidemia, which in turn is an important pathogenic factor in atherosclerosis, stroke, and fatty liver disease. Therefore, the development of new drugs targeting lipid metabolism regulatory pathways, oriented towards lowering lipids is increasingly becoming an important direction in the research and development of new drugs for metabolic diseases.

The lipid synthesis pathway in mammalian cells is known to be an important factor in the regulation of lipid metabolism homeostasis. The key factor regulating cholesterol and fatty acid synthesis is a transcription factor protein called SREBP (sterol regulatory element binding protein). This type of protein is first synthesized on the endoplasmic reticulum (ER) as a precursor, which is transported to the Golgi apparatus through SREBP cleavage-activating protein (SCAP), and then cleaved by two proteases (site-1 protease (S1P) and site-2 protease (S2P)), leading to the release of an active domain at the N-terminal. The active domain enters the nucleus to function as a transcription factor, and binds to the sterol regulatory element (SRE) in the promoter region of target genes to initiate the expression of downstream genes. The cleavage and maturation of SREBP proteins are strictly regulated by the levels of intracellular sterols (*e.g*., cholesterol and 25-hydroxycholesterol). When cells accumulate sufficient cholesterol in the endoplasmic reticulum, cholesterol binds to SCAP and alters the conformation of SCAP, causing the SCAP-SREBP complex to bind to Insig (insulin-induced gene) protein, thereby blocking the transport of SREBP to the Golgi apparatus and subsequent activation of SREBP. Conversely, an increase in the active form of nuclear SREBP promotes cellular lipid synthesis. In addition to cholesterol, 25-hydroxycholesterol (25-HC) is another potent endogenous inhibitor of the SREBP pathway. Unlike the binding of cholesterol to SCAP, 25-HC directly binds to Insig and induces the binding of SCAP to Insig.

Previous studies have found that inhibiting the SREBP pathway is an effective strategy and method for preventing and/or treating metabolic diseases such as obesity, hyperlipidemia, fatty liver, atherosclerosis, and diabetes, as well as other diseases such as cardiovascular and cerebrovascular disease, skin damage, and liver cancer.

For hyperlipidemia, the pathogenesis is mainly the excessive accumulation of lipids such as cholesterol and fatty acids in the blood due to increased lipid synthesis or abnormal lipid transport caused by factors such as diet or genetic mutations. Currently, statins and fibrates are the main lipid-lowering drugs in clinical practice, in which the mechanism of action of statins involves inhibiting the cellular cholesterol synthesis pathway while promoting the reverse transport of cholesterol in the blood. It indicates that targeting key factors in the cellular lipid synthesis pathway is an important means of effectively reducing lipid levels.

To date, there are no approved therapeutic drugs specifically for fatty liver disease, making it crucial to identify therapeutic targets and develop new effective therapies. The pathogenesis of fatty liver disease is known to involve a variety of risk factors, such as steatosis that may be triggered by the accumulation of triglycerides in the form of lipid droplets, and abnormally high levels of cholesterol and fatty acids in cells, which can cause endoplasmic reticulum stress and mitochondrial dysfunction, leading to cell death, inflammation, and fibrosis. Accumulation of free cholesterol has been reported as a key driving factor in the transition from simple steatosis to aggressive steatohepatitis. Moreover, by establishing a fatty liver model in mice, even prolonged feeding of a simple cholesterol-free high-fat diet can only induce steatosis, and the addition of 1 to 2% cholesterol to the diet is necessary to induce inflammation and fibrosis. Therefore, lowering cholesterol may become a new treatment strategy for fatty liver disease. Studies have shown abnormal activation of SREBP in patients with fatty liver and fatty liver models in mice. Liver-specific Scap deletion or knockout in mice can eliminate the activation of all SREBPs, thereby preventing the development of fatty liver and hyperlipidemia. Furthermore, recent studies have shown that endoplasmic reticulum stress-induced abnormal activation of SREBP promotes lipogenesis and fatty liver. Thus, these evidences suggest that lowering the levels of triglycerides and cholesterol in the liver by inhibiting the SREBP pathway is an effective strategy for preventing and/or treating metabolic disorders, including fatty liver.

### CONTENT OF THE PRESENT INVENTION

The technical problem to be solved by the present disclosure is to provide a new compound with SREBP pathway inhibitory activity.

The present disclosure also provides a compound of formula XXI or a pharmaceutically acceptable salt thereof: 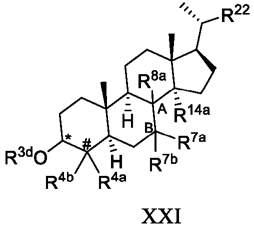 wherein R^{3d} is H or R^{3c};
R^{3c} is -S(O)₂OH, -C(O)-CH₂CH₂COOH, -C(O)-CH₂COOH, -C(O)-CH₂OH, -C(O)-COOH, -CH₂CH₂OH, or -CH₂COOH;
R^{4a} is H, F, CH₃, CF₃, or OH;
R^{4b} is H, F, NH₂, OH or CF₃;
R^{4a} and R^{4b} together with the carbon atom to which they are attached form 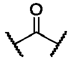 or 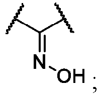
R^{7a} is H, F, NH₂, CH₃, or CF₃;
R^{7b} is For OH;
or, R^{7a} and R^{7b} together with the carbon atom to which they are attached form 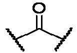 or 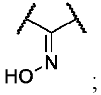
R^{8a} is H or F;
R^{14a} is H;
R²² is 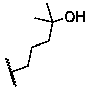 or R²¹;
R²¹ is -L¹-C(O)R^{A}, -L¹-S(O)₂R^{A}, -L¹-R^{B}, 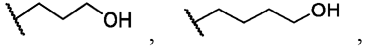 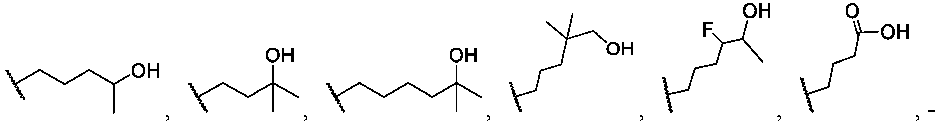 L²-R^{C}, or -L³-R^{D};
L¹ and L² are each independently a single bond, -CH₂-, -(CH₂)₂-, -(CH₂)₃-, -(CH₂)₄-, - (CH₂)₅-, or -(CH₂)₆-, wherein one -CH₂- moiety of the -CH₂-, -(CH₂)₂-, -(CH₂)₃-, -(CH₂)₄-, - (CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 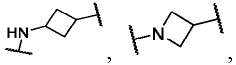 -CH=CH-, 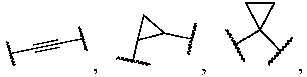 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)₂R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 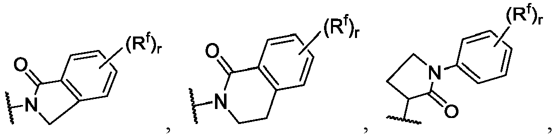 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 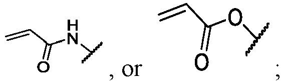
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, C₁₋₄ alkoxy, or fluoro-C₁₋₄ alkoxy;
the number of heteroatoms in the heterocycloalkyl and heteroaryl is independently 1, 2, 3, or 4, and each heteroatom is independently N, O, or S; in formula XXI, the carbon atom marked with * is in R configuration, S configuration, or a mixture of both; the carbon atom marked with # is in R configuration, S configuration, or a mixture of both when it is a chiral carbon atom; the carbon atom marked with A is in R configuration, S configuration, or a mixture of both when it is a chiral carbon atom; the carbon atom marked with B is in R configuration, S configuration, or a mixture of both when it is a chiral carbon atom.

In certain preferred embodiments of the present disclosure, certain groups in the compound of formula XXI, XVI, XIX, XXIII, XXIV, or XXVI, or the pharmaceutically acceptable salt thereof are defined as follows, and unmentioned groups are as described in any one of the embodiments of the present disclosure (referred to as "in some embodiments" or "in some preferred embodiments").

In some embodiments, in the compound of formula XXI as described above, R^{3d} is H or R^{3c}; R^{3c} is -S(O)₂OH, -C(O)-CH₂COOH, -C(O)-CH₂OH, -C(O)-COOH, -CH₂CH₂OH, or - CH₂COOH;
R^{4a} is H, F, CH₃, CF₃, or OH;
R^{4b} is H, F, OH, CH₂OH, CN, COOH, CF₃, or cyclopropyl;
or, R^{4a} and R^{4b} together with the carbon atom to which they are attached form 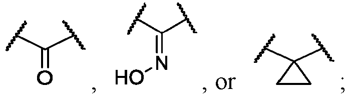
R^{7a} is H, F, CH₃, or CF₃; R^{7b} is F or OH; or, R^{7a} and R^{7b} together with the carbon atom to which they are attached form 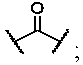
R^{8a} is H or F;
R^{14a} is H;
R²² is 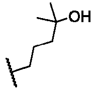 or R²¹;
R²¹ is -L¹-C(O)R^{A}, -L¹-S(O)₂R^{A}, -L¹-R^{B}, 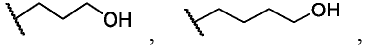 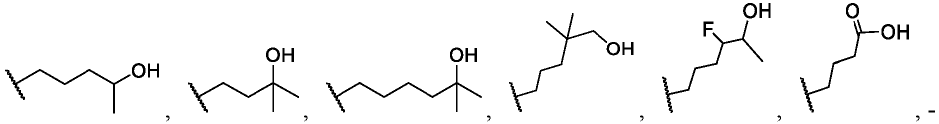 L²-R^{C}, or -L³-R^{D};
L¹ and L² are each independently a single bond, -CH₂-, -(CH₂)₂-, -(CH₂)₃-, -(CH₂)₄-, - (CH₂)₅-, or -(CH₂)₆-, wherein one -CH₂- moiety of the -CH₂-, -(CH₂)₂-, -(CH₂)₃-, -(CH₂)₄-, - (CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 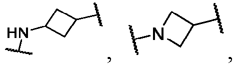 -CH=CH-, 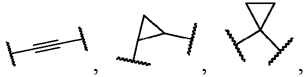 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)₂R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 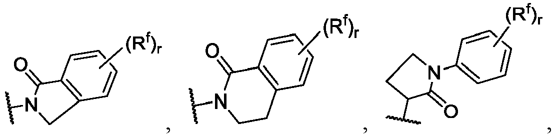 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 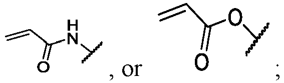
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, or C₁₋₄ alkoxy;
the number of heteroatoms in the heterocycloalkyl and heteroaryl is independently 1, 2, 3, or 4, and each heteroatom is independently N, O, or S;
in formula XXI, the carbon atom marked with * is in *R* configuration, *S* configuration, or a mixture of both; the carbon atom marked with # is in *R* configuration, *S* configuration, or a mixture of both when it is a chiral carbon atom; the carbon atom marked with A is in *R* configuration, *S* configuration, or a mixture of both when it is a chiral carbon atom; the carbon atom marked with B is in *R* configuration, *S* configuration, or a mixture of both when it is a chiral carbon atom.

In some embodiments, in the compound of formula XXI as described above, R^{3d} is H.

In some embodiments, in the compound of formula XXI as described above, R^{4a} is H; R^{4b} is H or OH; or, R^{4a} and R^{4b} together with the carbon atom to which they are attached form

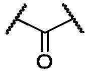

In some embodiments, in the compound of formula XXI as described above, R^{4a} is H; R^{4b} is H.

In some embodiments, in the compound of formula XXI as described above, R^{4a} is H; R^{4b} is OH.

In some embodiments, in the compound of formula XXI as described above, R^{4a} and R^{4b} together with the carbon atom to which they are attached form

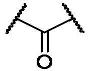

In some embodiments, in the compound of formula XXI as described above, R^{7a} is H; R^{7b} is F.

In some embodiments, in the compound of formula XXI as described above, R^{7a} is F; R^{7b} is F.

In some embodiments, in the compound of formula XXI as described above, R^{7a} is H; R^{7b} is OH.

In some embodiments, in the compound of formula XXI as described above, R^{7a} and R^{7b} together with the carbon atom to which they are attached form

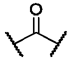

In some embodiments, in the compound of formula XXI as described above, R^{7a} and R^{7b} together with the carbon atom to which they are attached form

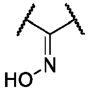

In some embodiments, in the compound of formula XXI as described above, R^{7a} is H; R^{7b} is NH₂.

In some embodiments, in the compound of formula XXI as described above, R^{8a} is H.

In some embodiments, in the compound of formula XXI as described in any one of the embodiments, R²² is

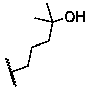

In some embodiments, in the compound of formula XXI as described in any one of the embodiments, R²² is R²¹.

In some embodiments, in the compound of formula XXI as described in any one of the embodiments, each X is independently -CHR^{5a}- or -CR^{5a}R^{5b}-, such as -CHR^{5a}-.

In some embodiments, in the compound of formula XXI as described in any one of the embodiments, L¹ is a single bond.

In some embodiments, in the compound of formula XXI as described in any one of the embodiments, L¹ is -(CH₂)₂- or -CH₂-CHR^{5a}-.

In some embodiments, in the compound of formula XXI as described in any one of the embodiments, R^{5a} and R^{5b} are each independently F, methyl, ethyl, or trifluoromethyl.

In some embodiments, in the compound of formula XXI as described in any one of the embodiments, L¹ is -(CH₂)₃-.

In some embodiments, in the compound of formula XXI as described in any one of the embodiments, L¹ is -(CH₂)₄-.

In some embodiments, in the compound of formula XXI as described in any one of the embodiments, L¹ is -(CH₂)₅-.

In some embodiments, in the compound of formula XXI as described in any one of the embodiments, L¹ is -(CH₂)₆-.

In some embodiments, in the compound of formula XXI as described in any one of the embodiments, R²¹ is -L¹-C(O)R^{A}.

In some embodiments, in the compound of formula XXI as described in any one of the embodiments, R²¹ is -L¹-S(O)₂R^{A}.

In some embodiments, in the compound of formula XXI as described in any one of the embodiments, R²¹ is -L¹-R^{B}.

In some embodiments, in the compound of formula XXI as described in any one of the embodiments, each R^{21a} is independently H, C₁₋₆ alkyl, or C₃₋₆ cycloalkyl, wherein the C₁₋₆ alkyl and C₃₋₆ cycloalkyl are independently unsubstituted or substituted by m R^{a}.

In some embodiments, in the compound of formula XXI as described in any one of the embodiments, m is 1, 2, or 3.

In some embodiments, in the compound of formula XXI as described in any one of the embodiments, each R^{a} is independently F, OH, CN, or methoxy.

In some embodiments, in the compound of formula XXI as described in any one of the embodiments, each R^{21a} is independently H, C₁₋₄ alkyl, -C₁₋₄ alkylene-OH, -C₁₋₄ alkylene-OC₁₋₄ alkyl, fluoro-C₁₋₄ alkyl, or cyclopropyl.

In some embodiments, in the compound of formula XXI as described in any one of the embodiments, each R^{21a} is independently H, methyl, ethyl, n-propyl, isopropyl, n-butyl, isobutyl, sec-butyl, 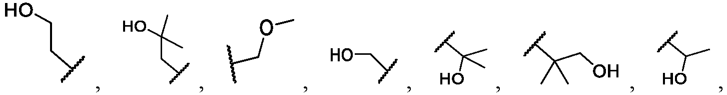 cyclopropyl, or trifluoromethyl.

In some embodiments, in the compound of formula XXI as described in any one of the embodiments, each R^{21b} is independently H, C₁₋₆ alkyl, C₆₋₁₀ aryl, or 5- to 10-membered heteroaryl, wherein the C₁₋₆ alkyl, C₆₋₁₀ aryl, and 5- to 10-membered heteroaryl are independently unsubstituted or substituted by q R^{b}.

In some embodiments, in the compound of formula XXI as described in any one of the embodiments, each R^{21b} is independently H, -C₁₋₄ alkylene-OH, -C₁₋₄ alkylene-OC₁₋₄ alkyl, fluoro-C₁₋₄ alkyl, tetrahydromorpholinyl, phenyl, pyridyl, thienyl, furyl, pyrrolyl, pyrazolyl, imidazolyl, oxazolyl, isoxazolyl, thiazolyl, isothiazolyl, thiadiazolyl, pyrimidinyl, or benzopyrazolyl, wherein the phenyl, pyridyl, thienyl, furyl, pyrrolyl, pyrazolyl, imidazolyl, oxazolyl, isoxazolyl, thiazolyl, isothiazolyl, thiadiazolyl, pyrimidinyl, and benzopyrazolyl are independently unsubstituted or substituted by q R^{b}.

In some embodiments, in the compound of formula XXI as described in any one of the embodiments, each R^{21b} is independently H, -C₁₋₄ alkylene-OH, -C₁₋₄ alkylene-OC₁₋₄ alkyl, fluoro-C₁₋₄ alkyl, tetrahydromorpholinyl, phenyl, 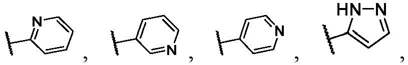 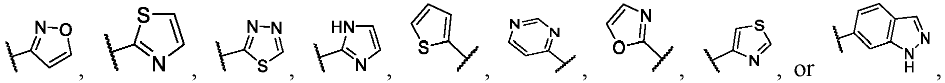 wherein the phenyl, 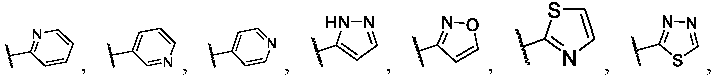 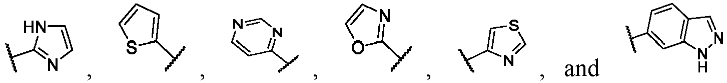 are independently unsubstituted or substituted by q R^{b}.

In some embodiments, in the compound of formula XXI as described in any one of the embodiments, q is 1, 2, or 3.

In some embodiments, in the compound of formula XXI as described in any one of the embodiments, each R^{b} is independently F, Cl, OH, COOH, CN, NO₂, methyl, trifluoromethyl, methoxy,

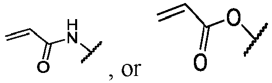

In some embodiments, in the compound of formula XXI as described in any one of the embodiments, each R^{21b} is independently H, methyl, ethyl, *n*-propyl, isopropyl, *n*-butyl, isobutyl, *sec*-butyl, phenyl, 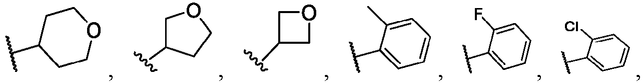 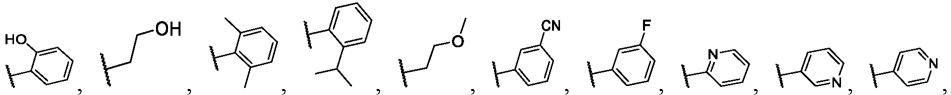 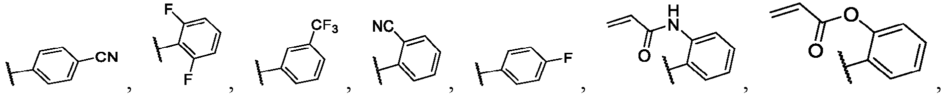 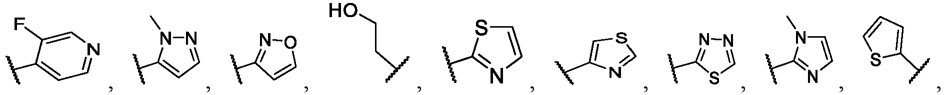 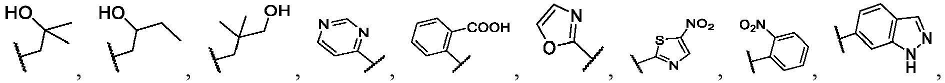 or

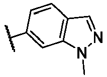

In some embodiments, in the compound of formula XXI as described in any one of the embodiments, each R^{21b} is independently

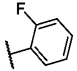

In some embodiments, in the compound of formula XXI as described in any one of the embodiments, in -NR^{21a}R^{21b}, R^{21a} and R^{21b} together with the nitrogen atom to which they are attached form 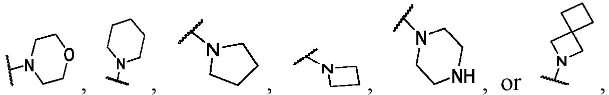 wherein the 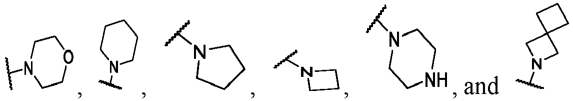 are unsubstituted or substituted by p R^{c}.

In some embodiments, in the compound of formula XXI as described in any one of the embodiments, p is 1, 2, or 3.

In some embodiments, in the compound of formula XXI as described in any one of the embodiments, each R^{c} is independently F, OH, CN, C₁₋₄ alkyl, or fluoro-C₁₋₄ alkyl.

In some embodiments, in the compound of formula XXI as described in any one of the embodiments, each R^{c} is independently F, OH, CN, methyl, or trifluoromethyl.

In some embodiments, in the compound of formula XXI as described in any one of the embodiments, in -NR^{21a}R^{21b}, R^{21a} and R^{21b} together with the nitrogen atom to which they are attached form

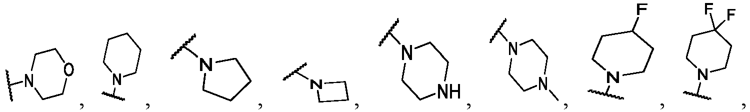

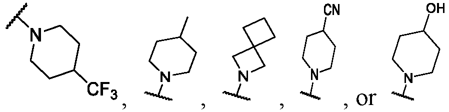

In some embodiments, in the compound of formula XXI as described in any one of the embodiments, each R^{A} is independently -NH₂,

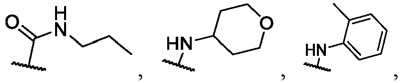

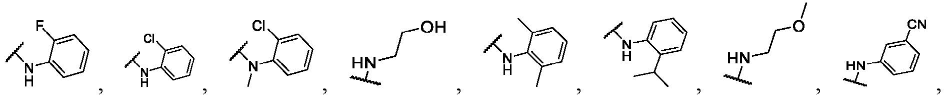

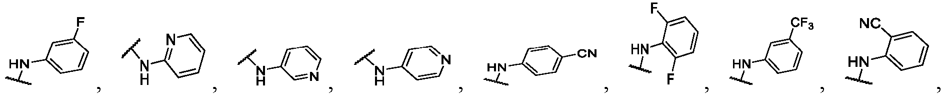

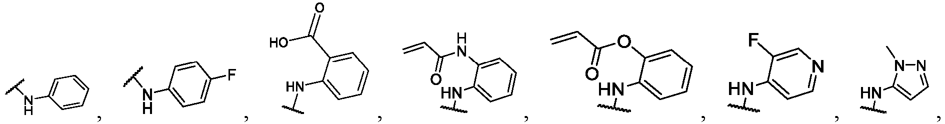

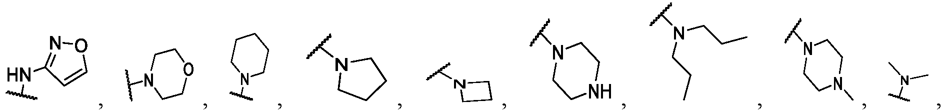

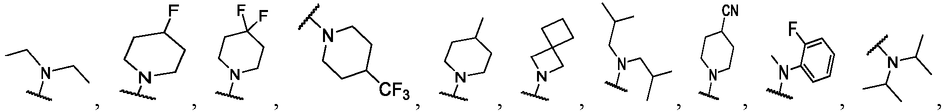

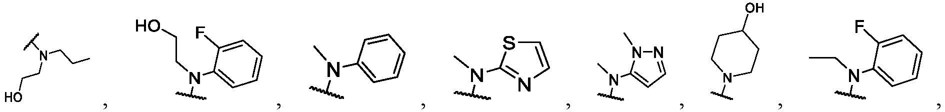

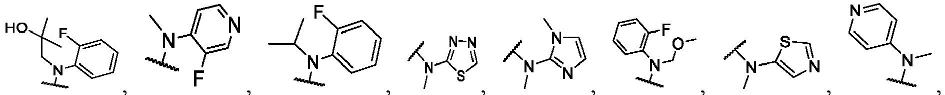

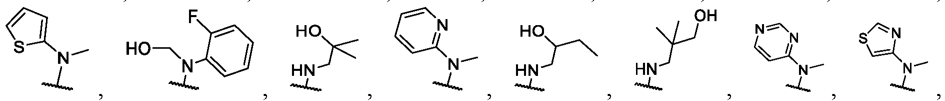

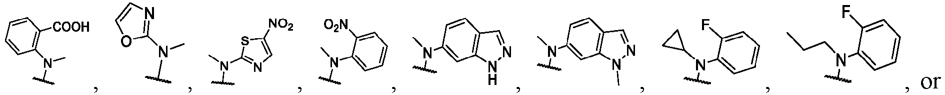

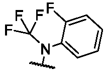

In some preferred embodiments, in the compound of formula XXI as described in any one of the embodiments, each R^{A} is independently

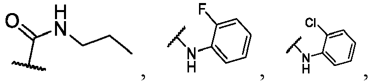

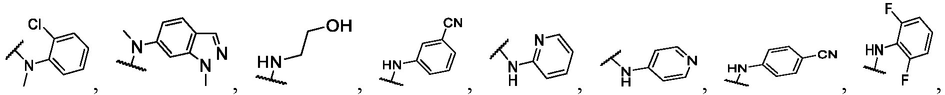

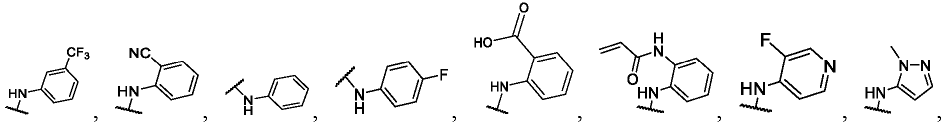

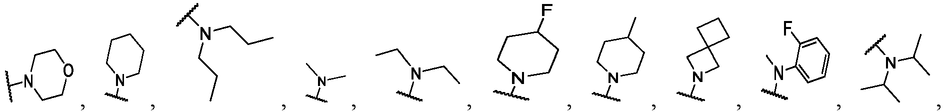

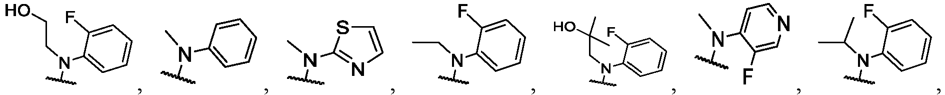

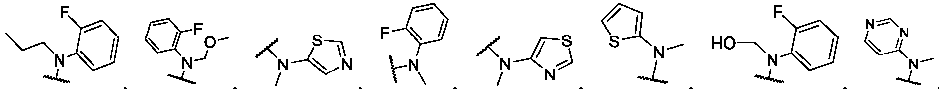

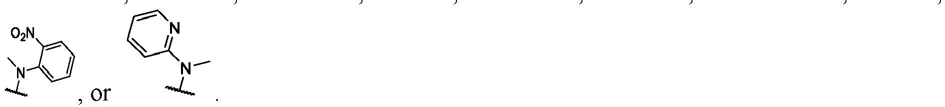

In some preferred embodiments, in the compound of formula XXI as described in any one of the embodiments, each R^{A} is independently

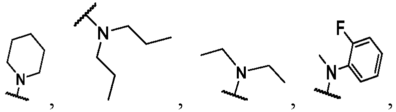

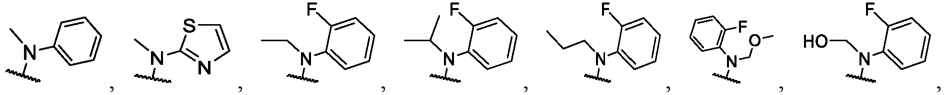

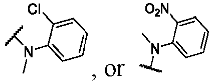

In some preferred embodiments, in the compound of formula XXI as described in any one of the embodiments, -L¹-C(O)R^{A} is

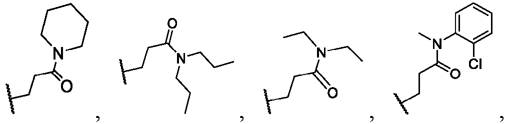

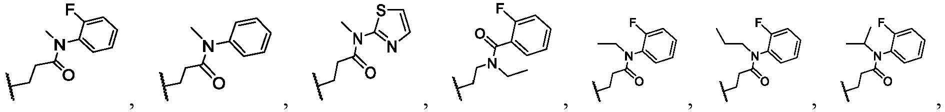

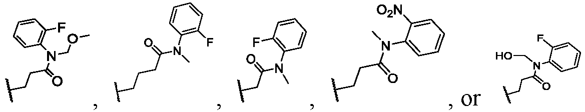

In some embodiments, in the compound of formula XXI as described in any one of the embodiments, R^{B} is 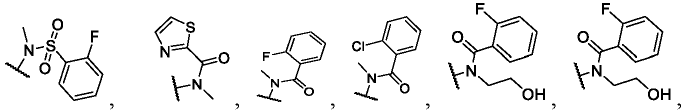 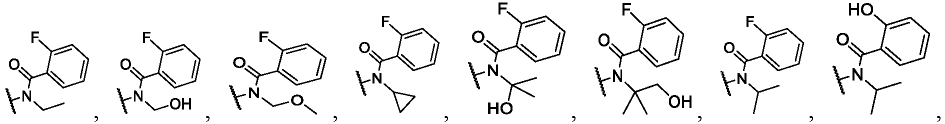 or

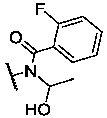

In some preferred embodiments, in the compound of formula XXI as described in any one of the embodiments, R^{B} is

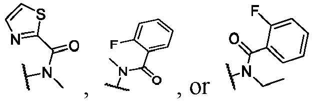

In some embodiments, in the compound of formula XXI as described in any one of the embodiments, R²¹ is -L²-R^{C}.

In some embodiments, in the compound of formula XXI as described in any one of the embodiments, L² is a single bond.

In some embodiments, in the compound of formula XXI as described in any one of the embodiments, L² is -CH₂-.

In some embodiments, in the compound of formula XXI as described in any one of the embodiments, L² is -(CH₂)₂-.

In some embodiments, in the compound of formula XXI as described in any one of the embodiments, L² is -(CH₂)₃-.

In some embodiments, in the compound of formula XXI as described in any one of the embodiments, L² is -(CH₂)₄-.

In some embodiments, in the compound of formula XXI as described in any one of the embodiments, L² is -(CH₂)₅-.

In some embodiments, in the compound of formula XXI as described in any one of the embodiments, L² is -(CH₂)₆-.

In some embodiments, in the compound of formula XXI as described in any one of the embodiments, each R^{C} is independently -C(R^{21c})(R^{21d})-OH.

In some embodiments, in the compound of formula XXI as described in any one of the embodiments, when R^{C} is -C(R^{21c})(R^{21d})-OH, R^{21c} is H, R^{21d} is phenyl, or R^{21c} and R^{21d} together with the carbon atom to which they are attached form a C₃₋₆ cycloalkyl group (*e.g.,* a cyclopropyl group); wherein the phenyl is optionally substituted by 1 or 2 R^{d}, and each R^{d} is independently F or C₁₋₄ alkoxy.

In some embodiments, in the compound of formula XXI as described in any one of the embodiments, in R^{C}, -C(R^{21c})(R^{21d})-OH is

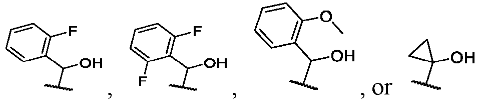

In some embodiments, in the compound of formula XXI as described in any one of the embodiments, when R^{C} is -C(R^{21c})(R^{21d})-OH, R^{21c} is H, and R^{21d} is C₂₋₄ alkyl, fluoro-C₁₋₄ alkyl, C₃₋₆ cycloalkyl, phenyl, fluorophenyl, 5- to 6-membered heteroaryl, or -L⁴-R^{21e}.

In some embodiments, in the compound of formula XXI as described in any one of the embodiments, when R^{C} is -C(R^{21c})(R^{21d})-OH, R^{21c} is H, and R^{21d} is C₂₋₄ alkyl, fluoro-C₁₋₄ alkyl, C₃₋₆ cycloalkyl, phenyl, 5- to 6-membered heteroaryl, or -L⁴-R^{21e}, wherein the phenyl is optionally substituted by 1 or 2 R^{d}, and each R^{d} is independently F, OH, CN, or C₁₋₄ alkoxy.

In some embodiments, in the compound of formula XXI as described in any one of the embodiments, L⁴ is methylene or ethylene.

In some embodiments, in the compound of formula XXI as described in any one of the embodiments, R^{21e} is OH, CN, C₁₋₄ alkoxy, phenyl, or

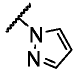

In some embodiments, in the compound of formula XXI as described in any one of the embodiments, in R^{C}, -C(R^{21c})(R^{21d})-OH is

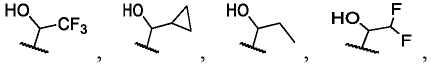

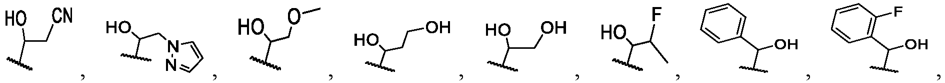

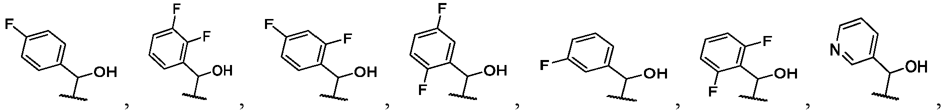

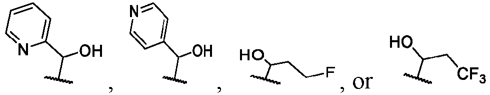

In some embodiments, in the compound of formula XXI as described in any one of the embodiments, when R^{C} is -C(R^{21c})(R^{21d})-OH, R^{21c} is F, and R^{21d} is C₂₋₄ alkyl (*e.g.,* ethyl).

In some embodiments, in the compound of formula XXI as described in any one of the embodiments, in R^{C}, -C(R^{21c})(R^{21d})-OH is

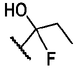

In some embodiments, in the compound of formula XXI as described in any one of the embodiments, when R^{C} is -C(R^{21c})(R^{21d})-OH, R^{21c} is CH₃ or trifluoromethyl, and R^{21d} is C₂₋₄ alkyl (*e.g.,* ethyl) or trifluoromethyl.

In some embodiments, in the compound of formula XVI as described in any one of the embodiments, in R^{C}, -C(R^{21c})(R^{21d})-OH is

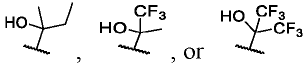

In some embodiments, in the compound of formula XXI as described in any one of the embodiments, when R^{C} is -C(R^{21c})(R^{21d})-OH, R^{21c} is C₂₋₄ alkyl, C₂₋₄ alkenyl (*e.g.,* 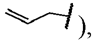 or C₃₋₆ cycloalkyl (*e.g.,* cyclopropyl), and R^{21d} is C₂₋₄ alkyl, C₂₋₄ alkenyl (*e.g.,* 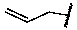 ), or C₃₋₆ cycloalkyl (*e.g*., cyclopropyl).

In some embodiments, in the compound of formula XXI as described in any one of the embodiments, when R^{C} is -C(R^{21c})(R^{21d})-OH, R^{21c} is ethyl, *n*-propyl, isopropyl, 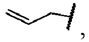 or cyclopropyl, and R^{21d} is ethyl, *n*-propyl, isopropyl, 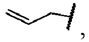 or cyclopropyl.

In some embodiments, in the compound of formula XXI as described in any one of the embodiments, in R^{C}, -C(R^{21c})(R^{21d})-OH is

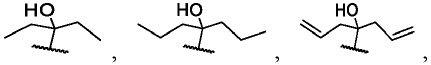

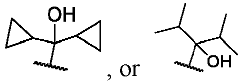

In some embodiments, in the compound of formula XXI as described in any one of the embodiments, when R^{C} is -C(R^{21c})(R^{21d})-OH, R^{21c} and R^{21d} together with the carbon atom to which they are attached form a C₃₋₆ cycloalkyl group or a 3- to 6-membered heterocycloalkyl group (*e.g.,* 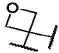 ).

In some embodiments, in the compound of formula XXI as described in any one of the embodiments, when R^{C} is -C(R^{21c})(R^{21d})-OH, R^{21c} and R^{21d} together with the carbon atom to which they are attached form a cyclopropyl group, a cyclobutyl group, or

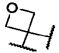

In some embodiments, in the compound of formula XXI as described in any one of the embodiments, in R^{C}, -C(R^{21c})(R^{21d})-OH is

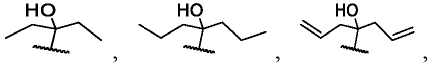

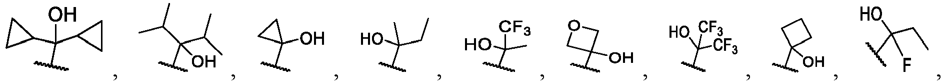

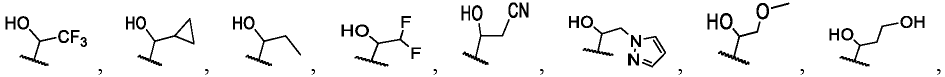

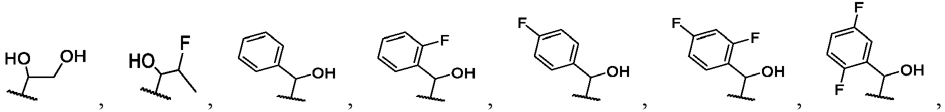

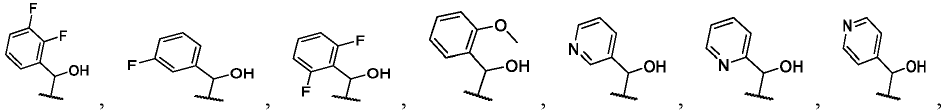

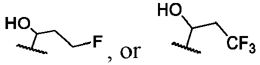

In some preferred embodiments, in the compound of formula XXI as described in any one of the embodiments, in R^{C}, -C(R^{21c})(R^{21d})-OH is

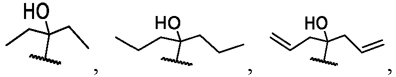

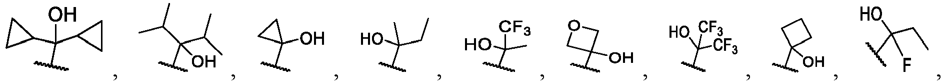

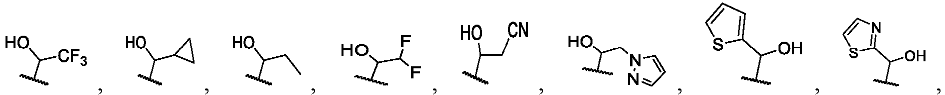

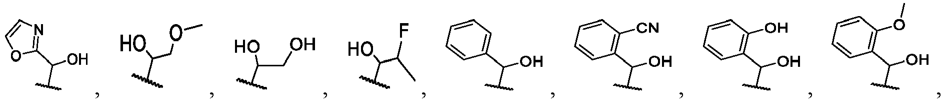

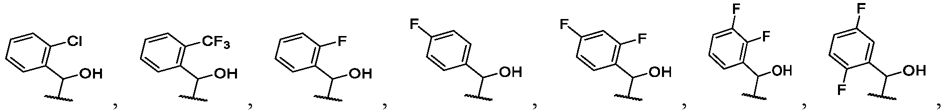

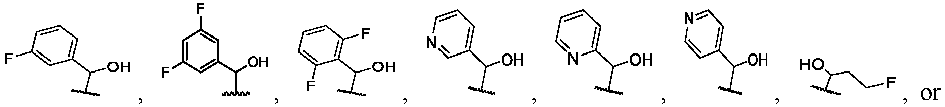

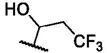

In some embodiments, in the compound of formula XXI as described in any one of the embodiments, in R^{C}, -C(R^{21c})(R^{21d})-OH is

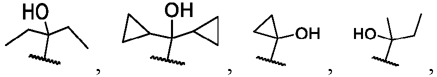

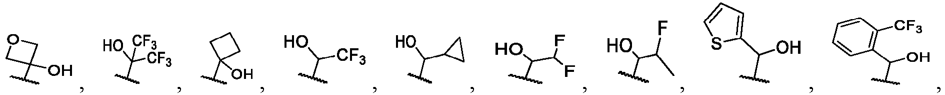

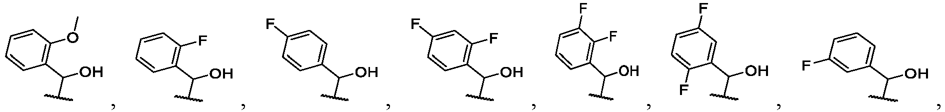

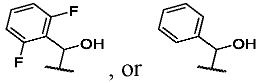

In some embodiments, in the compound of formula XXI as described in any one of the embodiments, R^{C} is CN.

In some embodiments, in the compound of formula XXI as described in any one of the embodiments, R^{C} is -C(O)R^{E}.

In some embodiments, in the compound of formula XXI as described in any one of the embodiments, R^{E} is C₁₋₄ alkyl or C₃₋₆ cycloalkyl.

In some embodiments, in the compound of formula XXI as described in any one of the embodiments, R^{E} is methyl, ethyl, or cyclohexyl.

In some embodiments, in the compound of formula XXI as described in any one of the embodiments, R^{C} is NH₂.

In some embodiments, in the compound of formula XXI as described in any one of the embodiments, R^{C} is C₁₋₄ alkoxy, such as methoxy.

In some embodiments, in the compound of formula XXI as described in any one of the embodiments, R^{C} is 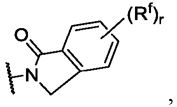 such as

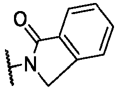

In some embodiments, in the compound of formula XXI as described in any one of the embodiments, R^{C} is 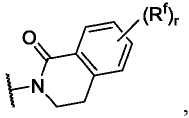 such as

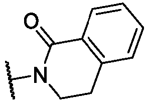

In some embodiments, in the compound of formula XXI as described in any one of the embodiments, R^{C} is 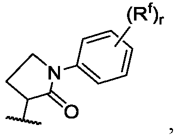 such as

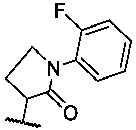

In some embodiments, in the compound of formula XXI as described in any one of the embodiments, R^{C} is 5- to 10-membered heteroaryl, wherein the 5- to 10-membered heteroaryl is unsubstituted or substituted by p R^{c}.

In some embodiments, in the compound of formula XXI as described in any one of the embodiments, in the definition of R^{C}, 5- to 10-membered heteroaryl is

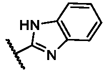

In some embodiments, in the compound of formula XXI as described in any one of the embodiments, R^{C} is

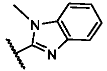

In some embodiments, in the compound of formula XXI as described in any one of the embodiments, R^{C} is 3- to 6-membered heterocycloalkyl.

In some embodiments, in the compound of formula XXI as described in any one of the embodiments, in the definition of R^{C}, 3- to 6-membered heterocycloalkyl is

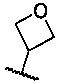

In some preferred embodiments, in the compound of formula XXI as described in any one of the embodiments, -L²-R^{C} is

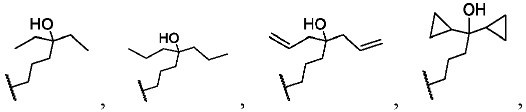

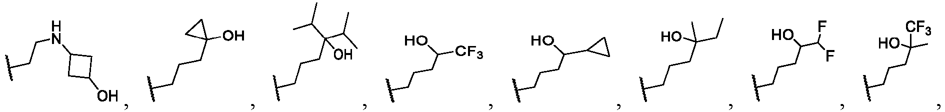

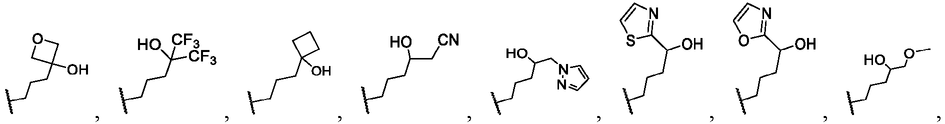

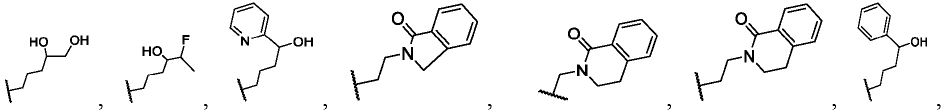

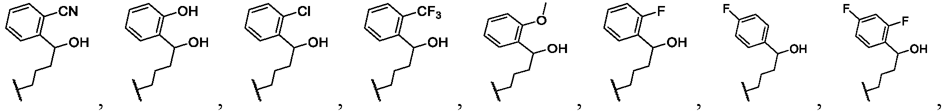

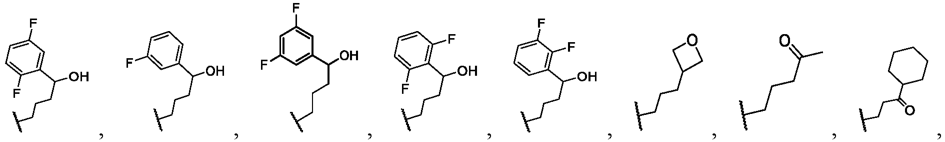

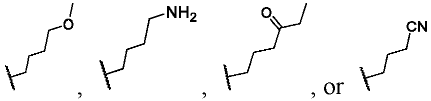

In some preferred embodiments, in the compound of formula XXI as described in any one of the embodiments, -L²-R^{C} is

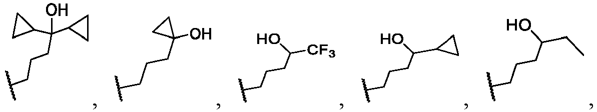

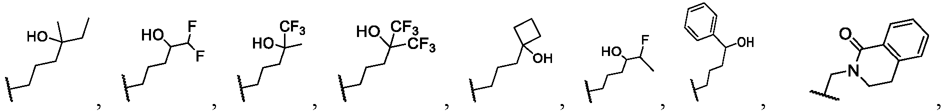

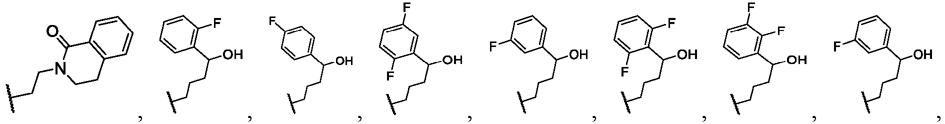

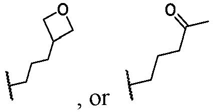

In some embodiments, in the compound of formula XXI as described in any one of the embodiments, R²¹ is -L³-R^{D}.

In some embodiments, in the compound of formula XXI as described in any one of the embodiments, L³ is 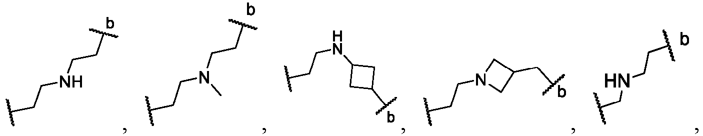 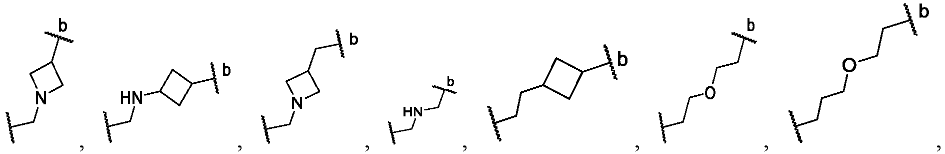 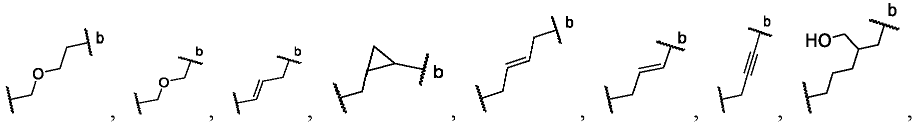 or 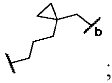 the b end of the structure is attached to R^{D}.

In some embodiments, in the compound of formula XXI as described in any one of the embodiments, R^{D} is -OH, -CH₂OH, 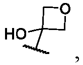 -CH(CH₃)-OH, or -C(CH₃)₂-OH.

In some embodiments, in the compound of formula XXI as described in any one of the embodiments, in R²¹, -L³-R^{D} is

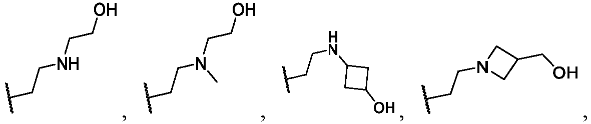

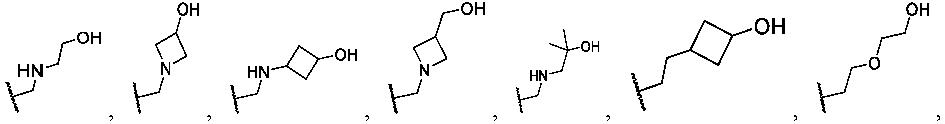

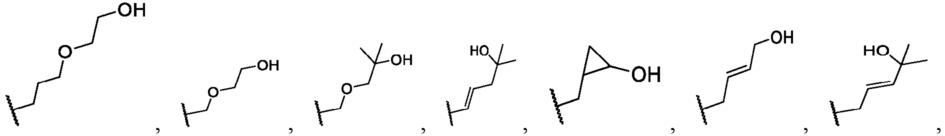

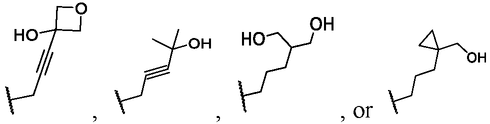

In some preferred embodiments, in the compound of formula XXI as described in any one of the embodiments, in R²¹, -L³-R^{D} is

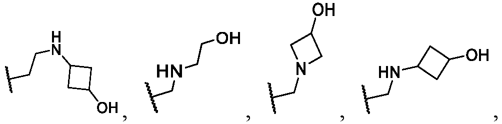

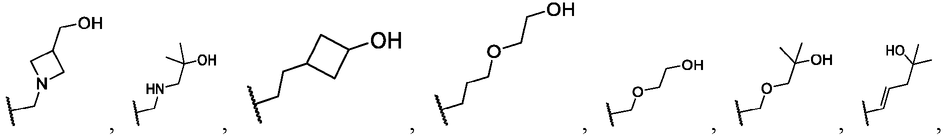

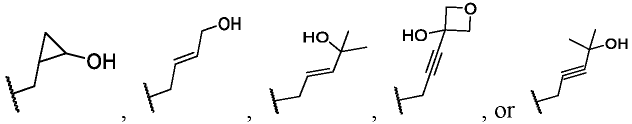

In some preferred embodiments, in the compound of formula XXI as described in any one of the embodiments, in R²¹, -L³-R^{D} is

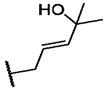

In some embodiments, in the compound of formula XXI as described in any one of the embodiments, R²¹ is

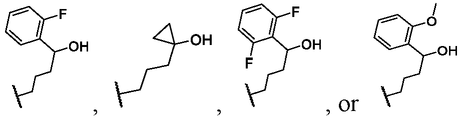

In some embodiments, in the compound of formula XXI as described in any one of the embodiments, R²² is

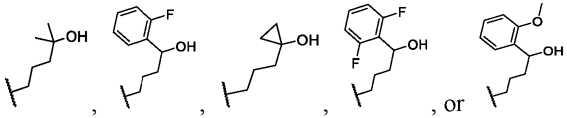

In some embodiments, in the compound of formula XXI as described in any one of the embodiments, the carbon atom marked with * is in *R* configuration.

In some embodiments, in the compound of formula XXI as described in any one of the embodiments, the carbon atom marked with * is in *S* configuration.

In some embodiments, in the compound of formula XXI as described in any one of the embodiments, the carbon atom marked with * is in a mixture of *S* configuration and *R* configuration, such as a mixture of *S* configuration and *R* configuration with a ratio of 1:1.

In some embodiments, in the compound of formula XXI as described in any one of the embodiments, the carbon atom marked with # is in *R* configuration.

In some embodiments, in the compound of formula XXI as described in any one of the embodiments, the carbon atom marked with # is in *S* configuration.

In some embodiments, in the compound of formula XXI as described in any one of the embodiments, the carbon atom marked with # is in a mixture of *S* configuration and *R* configuration, such as a mixture of *S* configuration and *R* configuration with a ratio of 1:1.

In some embodiments, in the compound of formula XXI as described in any one of the embodiments, the carbon atom marked with A is in *R* configuration.

In some embodiments, in the compound of formula XXI as described in any one of the embodiments, the carbon atom marked with A is in *S* configuration.

In some embodiments, in the compound of formula XXI as described in any one of the embodiments, the carbon atom marked with A is in a mixture of *S* configuration and *R* configuration, such as a mixture of *S* configuration and *R* configuration with a ratio of 1:1.

In some embodiments, in the compound of formula XXI as described in any one of the embodiments, the carbon atom marked with B is in *R* configuration.

In some embodiments, in the compound of formula XXI as described in any one of the embodiments, the carbon atom marked with B is in *S* configuration.

In some embodiments, in the compound of formula XXI as described in any one of the embodiments, the carbon atom marked with B is in a mixture of *S* configuration and *R* configuration, such as a mixture of *S* configuration and *R* configuration with a ratio of 1:1.

In some embodiments, the compound of formula XXI is a compound of formula XXI-1: 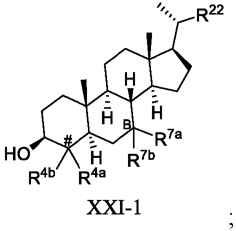 wherein #, B, R^{4a}, R^{4b}, R^{7a}, R^{7b}, and R²² are as defined in any one of the embodiments of the present disclosure.

In some embodiments, the compound of formula XXI is a compound of formula XXI-2, XXI-3, or XXI-4: 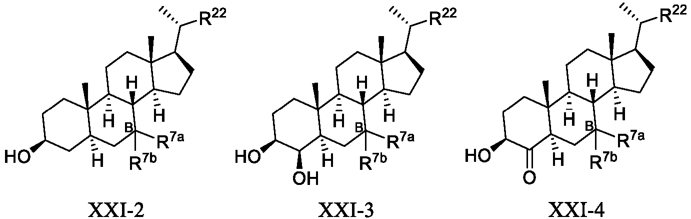 wherein B, R^{7a}, R^{7b}, and R²² are as defined in any one of the embodiments of the present disclosure.

In some embodiments, the compound of formula XXI is any one of the following compounds:

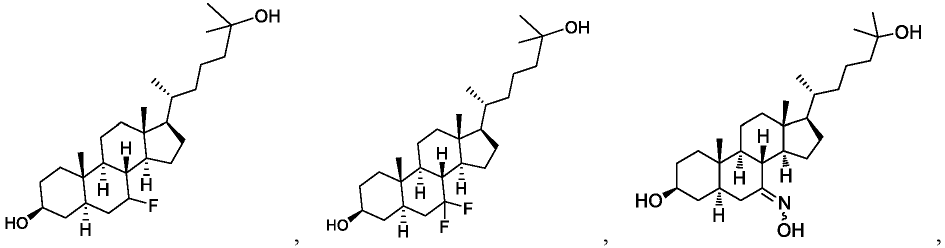

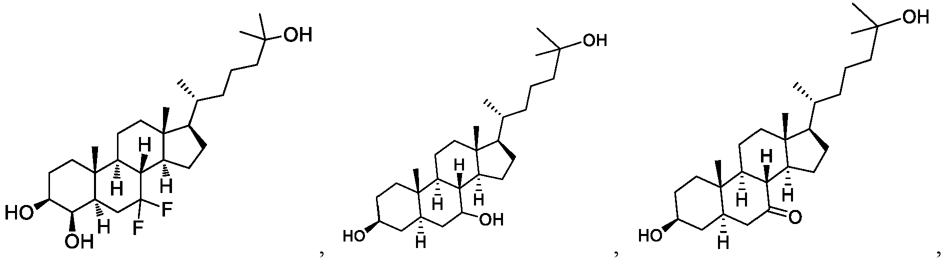

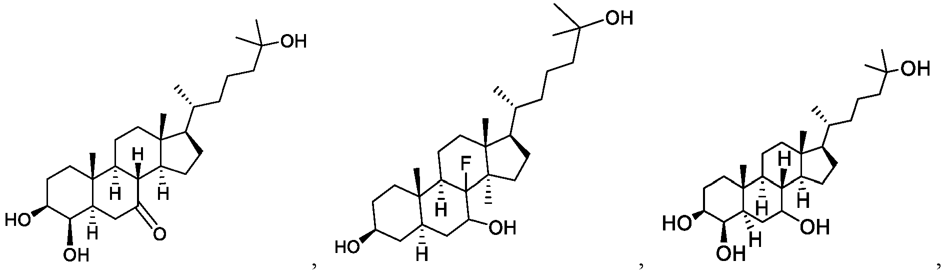

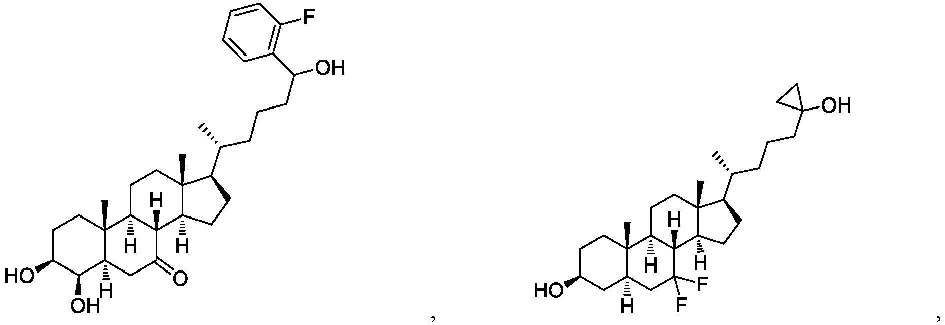

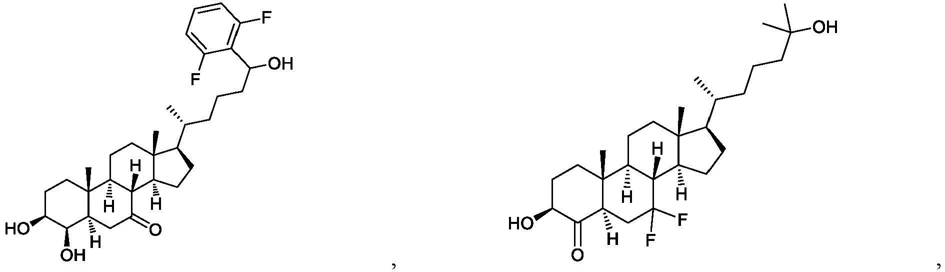

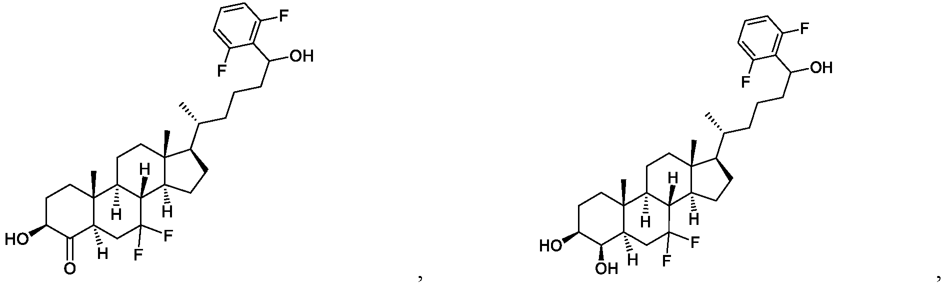

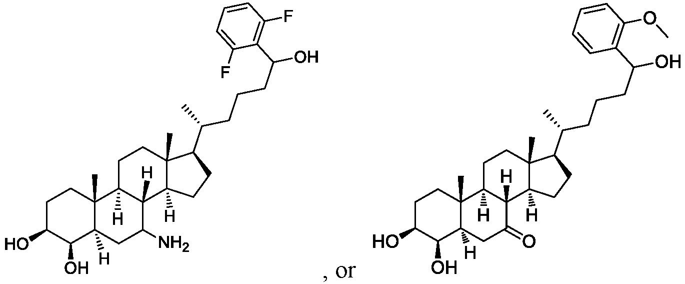

The present disclosure provides a compound of formula XVI or a pharmaceutically acceptable salt thereof: 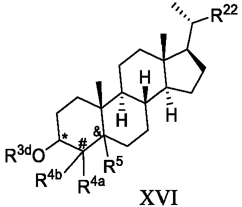 wherein R^{3d} is H or R^{3c};
R^{3c} is -S(O)₂OH, -C(O)-CH₂CH₂COOH, -C(O)-CH₂COOH, -C(O)-CH₂OH, -C(O)-COOH, -CH₂CH₂OH, or -CH₂COOH;
R^{4a} is H, F, CH₃, CF₃, or OH;
R^{4b} is H, F, NH₂, OH, or CF₃;
or, R^{4a} and R^{4b} together with the carbon atom to which they are attached form 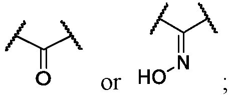
R⁵ is H or R^{5c}; R^{5c} is OH or F;
R²² is 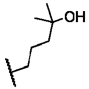 or R²¹; R²¹ is as defined in formula XXI;
in formula XVI, the carbon atom marked with * is in *R* configuration, *S* configuration, or a mixture of both; the carbon atom marked with # is in *R* configuration, *S* configuration, or a mixture of both when it is a chiral carbon atom; the carbon atom marked with & is in *R* configuration, *S* configuration, or a mixture of both when it is a chiral carbon atom.

In some embodiments, in the compound of formula XVI as described above, R^{3d} is H or R^{3c};
R^{3c} is -S(O)₂OH, -C(O)-CH₂COOH, -C(O)-CH₂OH, -C(O)-COOH, -CH₂CH₂OH, or -CH₂COOH;
R^{4a} is H, F, CH₃, CF₃, or OH;
R^{4b} is H, F, OH, or CF₃;
or, R^{4a} and R^{4b} together with the carbon atom to which they are attached form 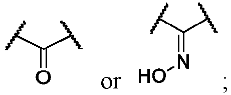
R⁵ is H or R^{5c}; R^{5c} is OH or F;
R²² is 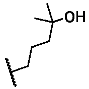 or R²¹;
R²¹ is -L¹-C(O)R^{A}, -L¹-S(O)₂R^{A}, -L¹-R^{B}, 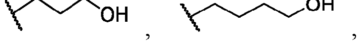 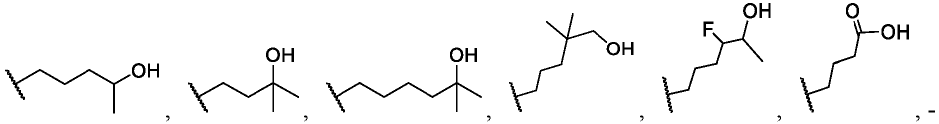 L²-R^{C}, or -L³-R^{D};
L¹ and L² are each independently a single bond, -CH₂-, -(CH₂)₂-, -(CH₂)₃-, -(CH₂)₄-, - (CH₂)₅-, or -(CH₂)₆-, wherein one -CH₂- moiety of the -CH₂-, -(CH₂)₂-, -(CH₂)₃-, -(CH₂)₄-, - (CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 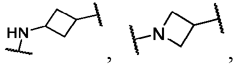 -CH=CH-, 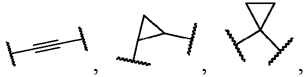 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)₂R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 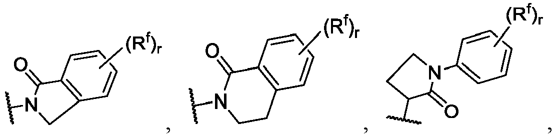 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 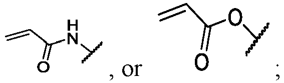
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, or C₁₋₄ alkoxy;
the number of heteroatoms in the heterocycloalkyl and heteroaryl is independently 1, 2, 3, or 4, and each heteroatom is independently N, O, or S;
in formula XVI, the carbon atom marked with * is in R configuration, S configuration, or a mixture of both; the carbon atom marked with # is in *R* configuration, *S* configuration, or a mixture of both when it is a chiral carbon atom; the carbon atom marked with & is in *R* configuration, *S* configuration, or a mixture of both when it is a chiral carbon atom.

In some embodiments, in the compound of formula XVI as described above, R^{3d} is H, -C(O)-CH₂OH, -C(O)-CH₂COOH, or -C(O)-CH₂CH₂COOH.

In some embodiments, in the compound of formula XVI as described above, R^{3d} is H.

In some embodiments, in the compound of formula XVI as described above, R^{3d} is R^{3c}.

In some embodiments, in the compound of formula XVI as described above, R^{3c} is - S(O)₂OH.

In some embodiments, in the compound of formula XVI as described above, R^{3c} is - C(O)-CH₂COOH.

In some embodiments, in the compound of formula XVI as described above, R^{3c} is - C(O)-CH₂OH.

In some embodiments, in the compound of formula XVI as described above, R^{3c} is - C(O)-COOH.

In some embodiments, in the compound of formula XVI as described above, R^{3c} is - CH₂CH₂OH.

In some embodiments, in the compound of formula XVI as described above, R^{3c} is - CH₂COOH.

In some embodiments, in the compound of formula XVI as described above, R^{4a} is H, and R^{4b} is OH.

In some embodiments, in the compound of formula XVI as described above, R^{4a} is CH₃, and R^{4b} is F.

In some embodiments, in the compound of formula XVI as described above, R^{4a} is F, and R^{4b} is F.

In some embodiments, in the compound of formula XVI as described above, R^{4a} is H, and R^{4b} is CF₃.

In some embodiments, in the compound of formula XVI as described above, R^{4a} is CH₃, and R^{4b} is OH.

In some embodiments, in the compound of formula XVI as described above, R^{4a} is OH, and R^{4b} is CF₃.

In some embodiments, in the compound of formula XVI as described above, R^{4a} and R^{4b} together with the carbon atom to which they are attached form

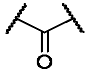

In some embodiments, in the compound of formula XVI as described above, R^{4a} is H, and R^{4b} is F.

In some embodiments, in the compound of formula XVI as described above, R^{4a} is CF₃, and R^{4b} is CF₃.

In some embodiments, in the compound of formula XVI as described above, R^{4a} and R^{4b} together with the carbon atom to which they are attached form

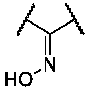

In some embodiments, in the compound of formula XVI as described above, R^{4a} is H, and R^{4b} is H.

In some embodiments, in the compound of formula XVI as described above, R^{4a} is H, and R^{4b} is NH₂.

In some embodiments, in the compound of formula XVI as described above, R⁵ is H.

In some embodiments, in the compound of formula XVI as described above, R⁵ is OH.

In some embodiments, in the compound of formula XVI as described above, R⁵ is F.

In some embodiments, in the compound of formula XVI as described in any one of the embodiments, R²² is

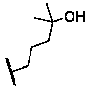

In some embodiments, in the compound of formula XVI as described in any one of the embodiments, R²² is R²¹; R²¹ is as defined in any one of the embodiments of formula XXI.

In some embodiments, in the compound of formula XVI as described in any one of the embodiments, R²¹ is -L¹-C(O)R^{A}, -L²-R^{C}, or

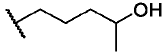

In some embodiments, in the compound of formula XVI as described in any one of the embodiments, L¹ is -(CH₂)₂- or -CH₂-CHR^{5a}-; preferably, L¹ is -(CH₂)₂-.

In some embodiments, in the compound of formula XVI as described in any one of the embodiments, each R^{21a} is independently H, C₁₋₄ alkyl, -C₁₋₄ alkylene-OH, -C₁₋₄ alkylene-OCₗ₋₄ alkyl, fluoro-C₁₋₄ alkyl, or cyclopropyl; preferably, each R^{21a} is independently C₁₋₄ alkyl.

In some embodiments, in the compound of formula XVI as described in any one of the embodiments, each R^{21a} is independently H, methyl, ethyl, *n*-propyl, isopropyl, *n*-butyl, isobutyl, *sec*-butyl, 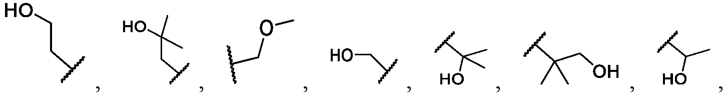 cyclopropyl, or trifluoromethyl; such as methyl.

In some embodiments, in the compound of formula XVI as described in any one of the embodiments, each R^{21b} is independently H, C₁₋₆ alkyl, C₆₋₁₀ aryl, or 5- to 10-membered heteroaryl, wherein the C₁₋₆ alkyl, C₆₋₁₀ aryl, and 5- to 10-membered heteroaryl are independently unsubstituted or substituted by q R^{b}; preferably, each R^{21b} is independently C₆₋₁₀ aryl, wherein the C₆₋₁₀ aryl is unsubstituted or substituted by q R^{b}.

In some embodiments, in the compound of formula XVI as described in any one of the embodiments, each R^{21b} is independently H, -C₁₋₄ alkylene-OH, -C₁₋₄ alkylene-OC₁₋₄ alkyl, fluoro-C₁₋₄ alkyl, tetrahydromorpholinyl, phenyl, pyridyl, thienyl, furyl, pyrrolyl, pyrazolyl, imidazolyl, oxazolyl, isoxazolyl, thiazolyl, isothiazolyl, thiadiazolyl, pyrimidinyl, or benzopyrazolyl, wherein the phenyl, pyridyl, thienyl, furyl, pyrrolyl, pyrazolyl, imidazolyl, oxazolyl, isoxazolyl, thiazolyl, isothiazolyl, thiadiazolyl, pyrimidinyl, and benzopyrazolyl are independently unsubstituted or substituted by q R^{b}; preferably, each R^{21b} is independently phenyl, wherein the phenyl is unsubstituted or substituted by q R^{b}.

In some embodiments, in the compound of formula XVI as described in any one of the embodiments, q is 1, 2, or 3; such as 1.

In some embodiments, in the compound of formula XVI as described in any one of the embodiments, each R^{b} is independently F, Cl, OH, COOH, CN, NO₂, methyl, trifluoromethyl, methoxy, 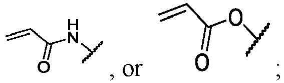 such as F.

In some embodiments, in the compound of formula XVI as described in any one of the embodiments, each R^{21b} is independently

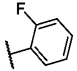

In some preferred embodiments, in the compound of formula XVI as described in any one of the embodiments, each R^{A} is independently 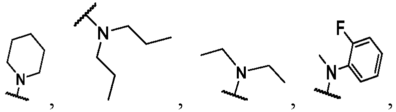 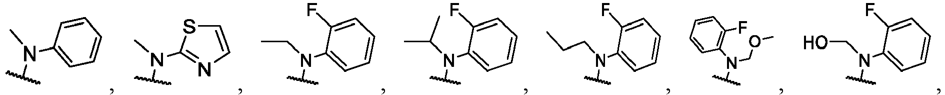 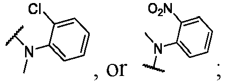 such as

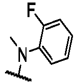

In some preferred embodiments, in the compound of formula XXI as described in any one of the embodiments, -L¹-C(O)R^{A} is 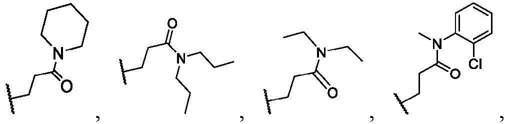 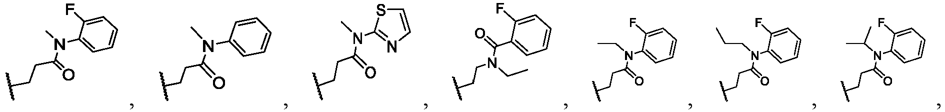 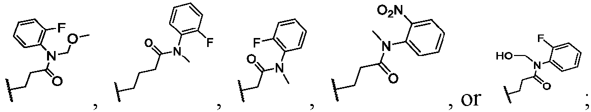 such as

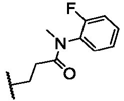

In some embodiments, in the compound of formula XVI as described in any one of the embodiments, L² is -(CH₂)₂- or -(CH₂)₃.

In some embodiments, in the compound of formula XVI as described in any one of the embodiments, each R^{C} is independently -C(R^{21c})(R^{21d})-OH or

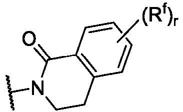

In some embodiments, in the compound of formula XVI as described in any one of the embodiments, when R^{C} is -C(R^{21c})(R^{21d})-OH, R^{21c} is H, and R^{21d} is C₂₋₄ alkyl, fluoro-C₁₋₄ alkyl, C₃₋₆ cycloalkyl, phenyl, 5- to 6-membered heteroaryl, or -L⁴-R^{21e}, wherein the phenyl is optionally substituted by 1 or 2 R^{d}, and each R^{d} is independently F, OH, CN, or C₁₋₄ alkoxy.

In some embodiments, in the compound of formula XVI as described in any one of the embodiments, when R^{C} is -C(R^{21c})(R^{21d})-OH, R^{21c} is H, and R^{21d} is C₂₋₄ alkyl, fluoro-C₁₋₄ alkyl, C₃₋₆ cycloalkyl, or phenyl, wherein the phenyl is optionally substituted by 1 or 2 R^{d}, and each R^{d} is independently F, Cl, OH, fluoro-C₁₋₄ alkyl, C₁₋₄ alkoxy, or fluoro-C₁₋₄ alkoxy.

In some embodiments, in the compound of formula XVI as described in any one of the embodiments, when R^{C} is -C(R^{21c})(R^{21d})-OH, R^{21c} and R^{21d} together with the carbon atom to which they are attached form a C₃₋₆ cycloalkyl group or a 3- to 6-membered heterocycloalkyl group (*e.g.,* 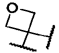 ).

In some embodiments, in the compound of formula XVI as described in any one of the embodiments, when R^{C} is -C(R^{21c})(R^{21d})-OH, R^{21c} and R^{21d} together with the carbon atom to which they are attached form a cyclopropyl group, a cyclobutyl group, or 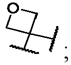 preferably a cyclopropyl group.

In some embodiments, in the compound of formula XVI as described in any one of the embodiments, when R^{C} is -C(R^{21c})(R^{21d})-OH, R^{21c} is CH₃ or trifluoromethyl, and R^{21d} is C₂₋₄ alkyl (*e.g*., ethyl) or trifluoromethyl.

In some embodiments, in the compound of formula XVI as described in any one of the embodiments, in R^{C}, -C(R^{21c})(R^{21d})-OH is

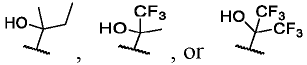

In some embodiments, in the compound of formula XVI as described in any one of the embodiments, when R^{C} is -C(R^{21c})(R^{21d})-OH, R^{21c} is CH₃ or trifluoromethyl, and R^{21d} is phenyl or trifluoromethyl, wherein the phenyl is unsubstituted or substituted by j R^{d}.

In some embodiments, in the compound of formula XVI as described in any one of the embodiments, in R^{C}, -C(R^{21c})(R^{21d})-OH is

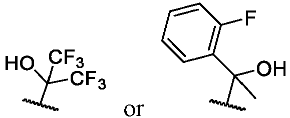

In some embodiments, in the compound of formula XVI as described in any one of the embodiments, r is 0.

In some embodiments, in the compound of formula XVI as described in any one of the embodiments, R²¹ is

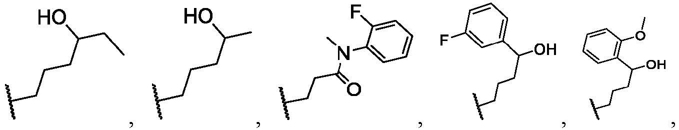

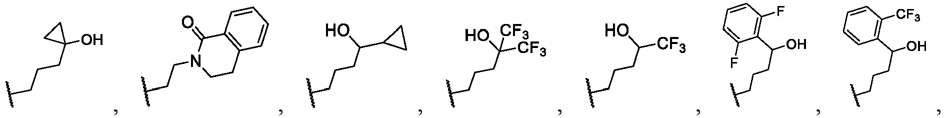

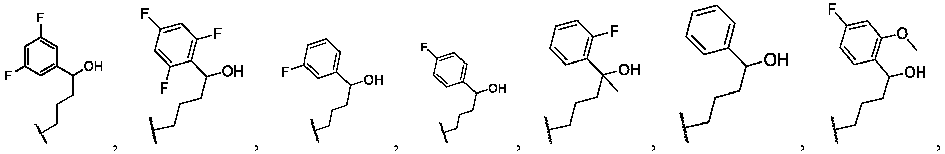

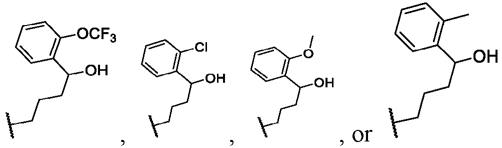

In some embodiments, in the compound of formula XVI as described in any one of the embodiments, the carbon atom marked with * is in R configuration.

In some embodiments, in the compound of formula XVI as described in any one of the embodiments, the carbon atom marked with * is in S configuration.

In some embodiments, in the compound of formula XVI as described in any one of the embodiments, the carbon atom marked with * is in a mixture of *S* configuration and *R* configuration, such as a mixture of *S* configuration and *R* configuration with a ratio of 1:1.

In some embodiments, in the compound of formula XVI as described in any one of the embodiments, in formula XVI, the carbon atom marked with # is in *R* configuration when it is a chiral carbon atom.

In some embodiments, in the compound of formula XVI as described in any one of the embodiments, in formula XVI, the carbon atom marked with # is in *S* configuration when it is a chiral carbon atom.

In some embodiments, in the compound of formula XVI as described in any one of the embodiments, in formula XVI, the carbon atom marked with # is in a mixture of *S* configuration and *R* configuration, such as a mixture of *S* configuration and *R* configuration with a ratio of 1:1.

In some embodiments, in the compound of formula XVI as described in any one of the embodiments, in formula XVI, the carbon atom marked with & is in *R* configuration when it is a chiral carbon atom.

In some embodiments, in the compound of formula XVI as described in any one of the embodiments, in formula XVI, the carbon atom marked with & is in *S* configuration when it is a chiral carbon atom.

In some embodiments, in the compound of formula XVI as described in any one of the embodiments, in formula XVI, the carbon atom marked with & is in a mixture of *S* configuration and *R* configuration, such as a mixture of *S* configuration and *R* configuration with a ratio of 1:1.

In some embodiments, the compound of formula XVI is a compound of formula XVI-1: 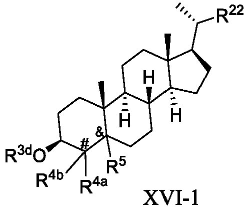 wherein #, &, R^{3d}, R^{4a}, R^{4b}, R⁵, and R²² are as defined in any one of the embodiments of the present disclosure.

In some embodiments, the compound of formula XVI is any one of the following compounds:

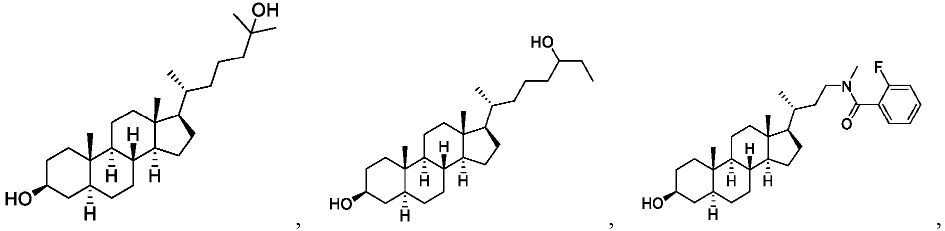

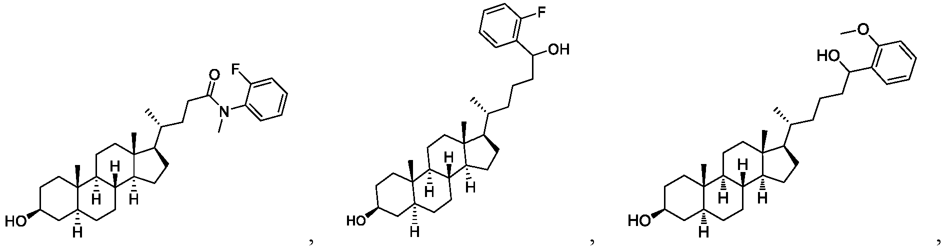

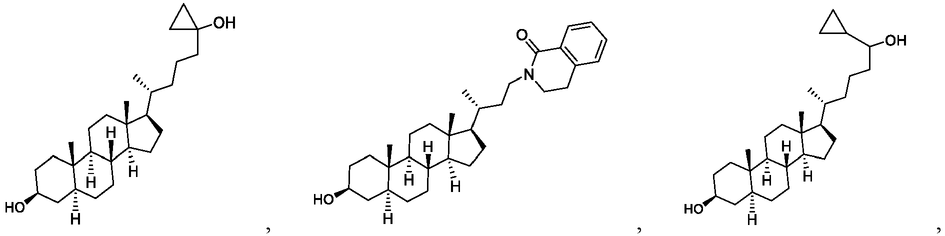

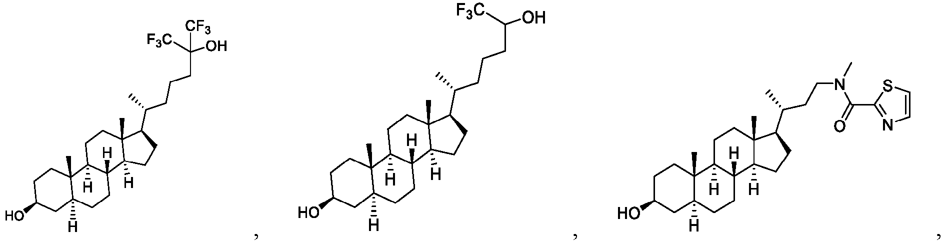

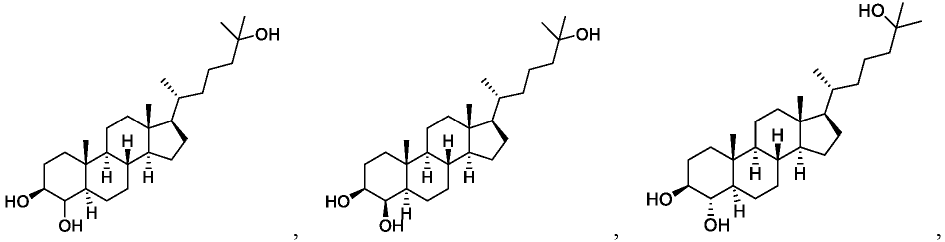

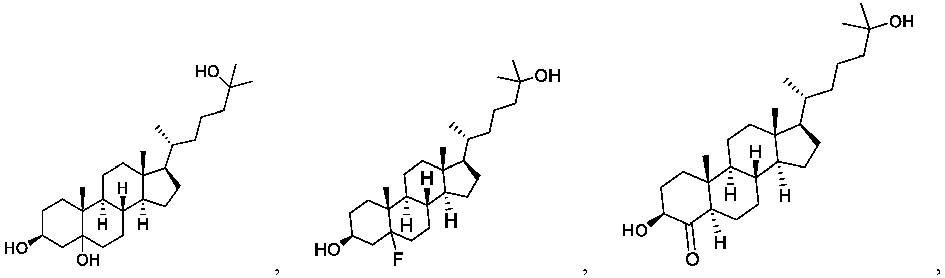

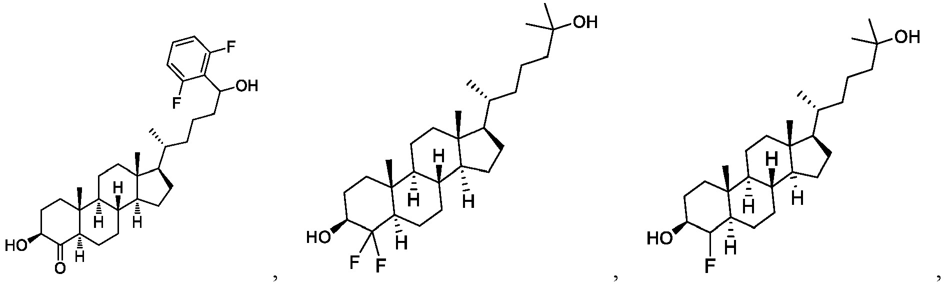

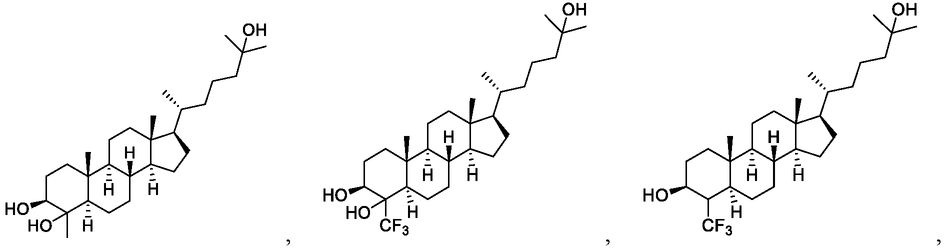

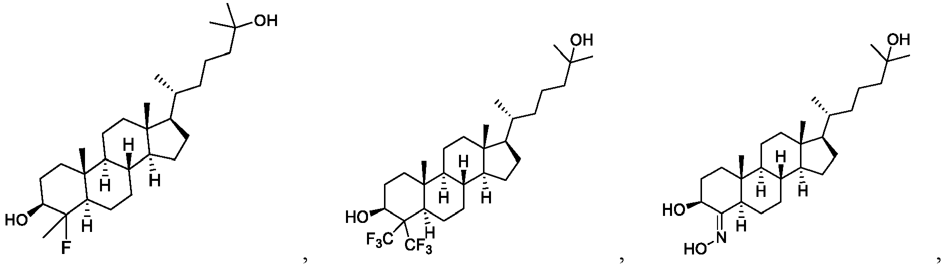

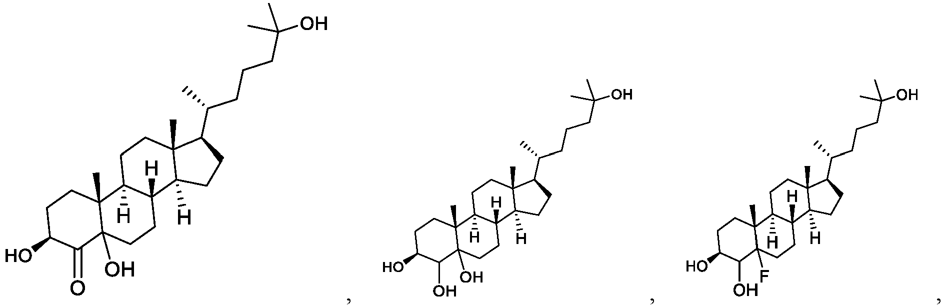

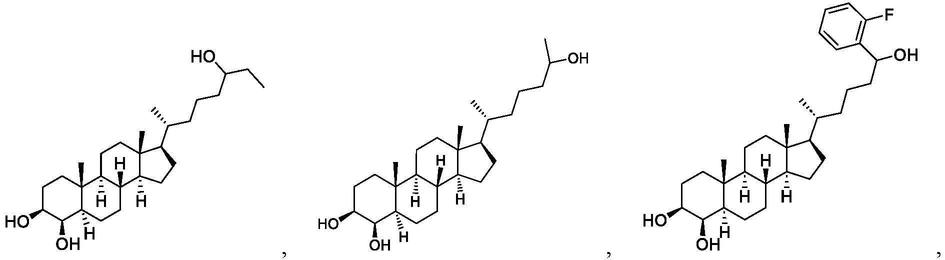

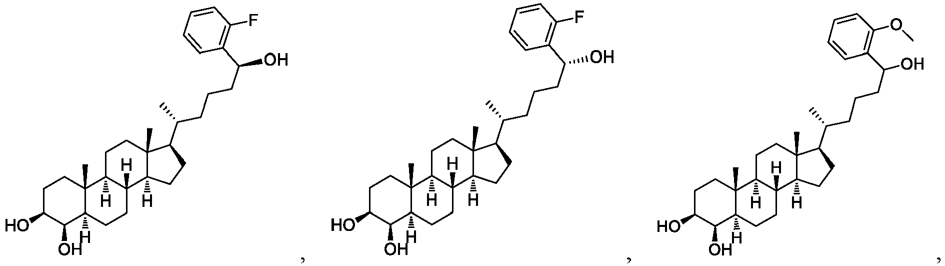

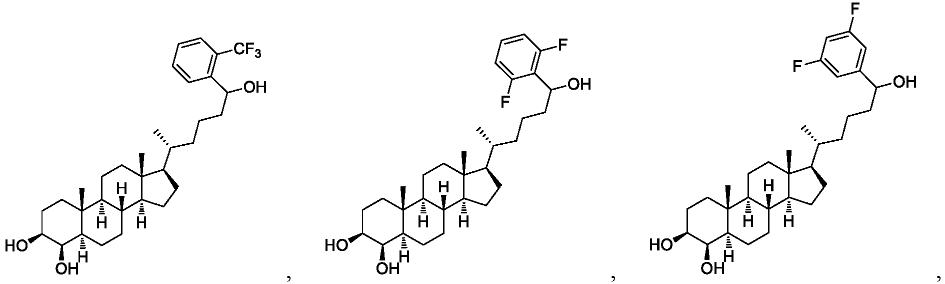

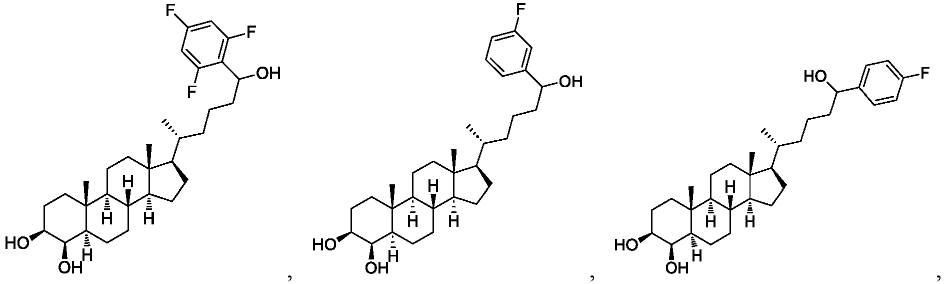

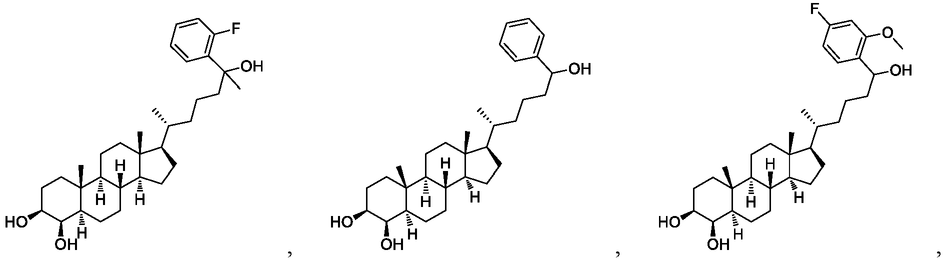

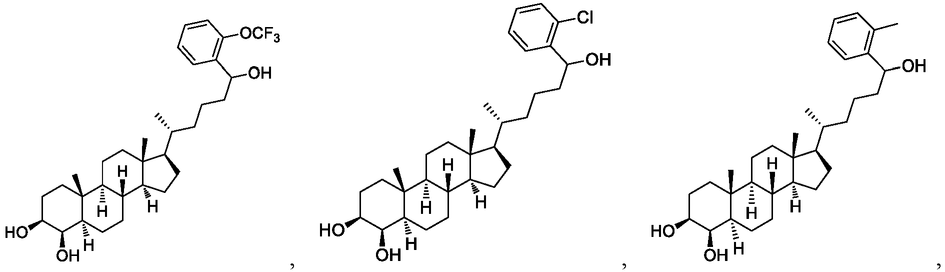

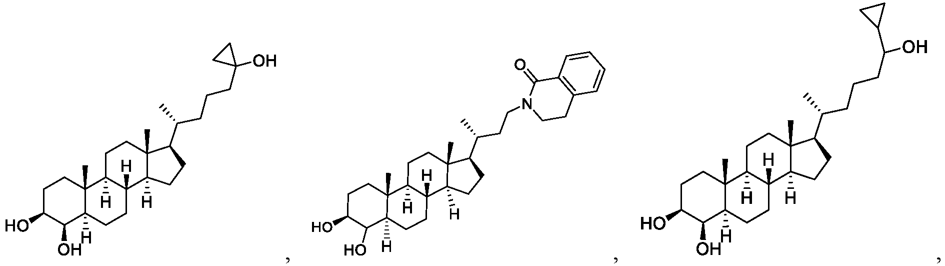

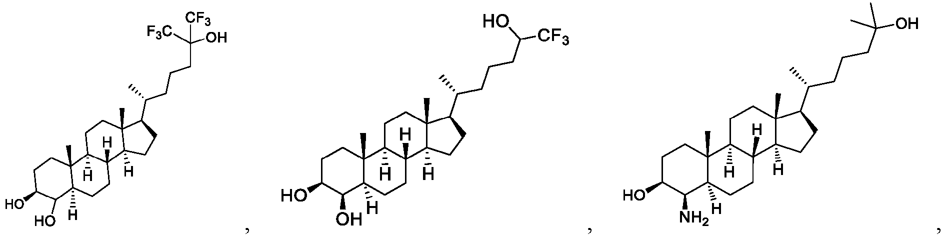

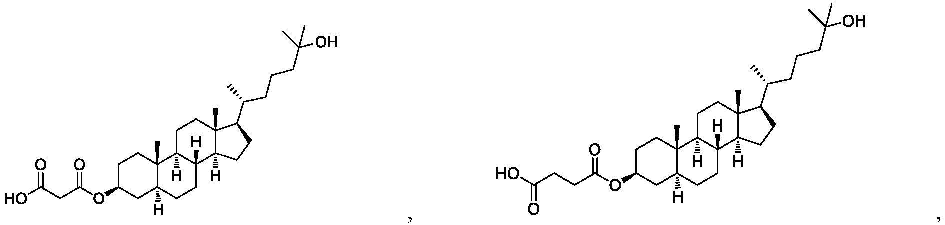

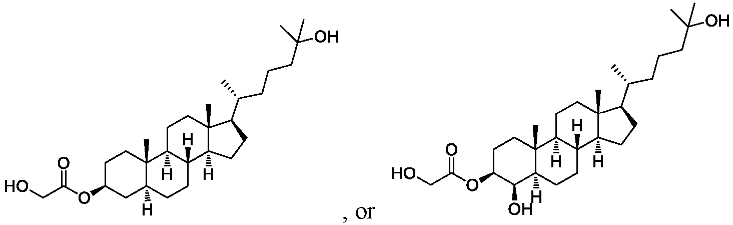

In some embodiments, the compound of formula XVI is a compound of formula VII: 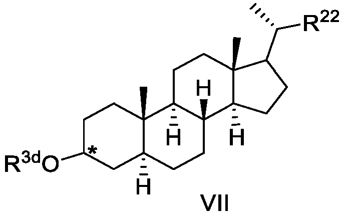 wherein R^{3d} is H or R^{3c}; R^{3c} is as defined in any one of the embodiments of formula XXI;
R²² is 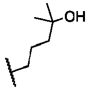 or R²¹; R²¹ is as defined in any one of the embodiments of formula XXI;
in formula VII, the carbon atom marked with * is in *R* configuration, *S* configuration, or a mixture of both.

In some embodiments, in the compound of formula VII as described above, R^{3d} is H.

In some embodiments, in the compound of formula VII as described above, R^{3d} is R^{3c}; R^{3c} is as defined in any one of the embodiments of formula XXI.

In some embodiments, in the compound of formula VII as described in any one of the embodiments, R²² is

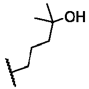

In some embodiments, in the compound of formula VII as described in any one of the embodiments, R²² is R²¹; R²¹ is as defined in any one of the embodiments of formula XXI.

In some embodiments, in the compound of formula VII as described in any one of the embodiments, the carbon atom marked with * is in *R* configuration.

In some embodiments, in the compound of formula VII as described in any one of the embodiments, the carbon atom marked with * is in *S* configuration.

In some embodiments, in the compound of formula VII as described in any one of the embodiments, the carbon atom marked with * is in a mixture of *S* configuration and R configuration, such as a mixture of *S* configuration and *R* configuration with a ratio of 1:1.

In some embodiments, the compound of formula VII is the following compound:

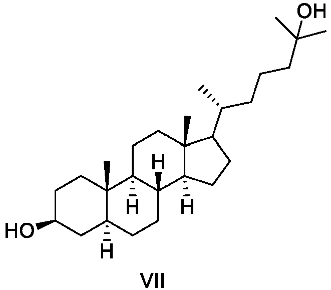

The present disclosure also provides a compound of formula XIX or a pharmaceutically acceptable salt thereof: 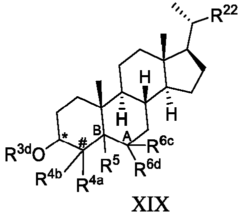
wherein R^{3d} is H or R^{3c}; R^{3c} is as defined in any one of the embodiments of formula XXI;
R^{4a} is as defined in any one of the embodiments of formula XXI;
R^{4b} is as defined in any one of the embodiments of formula XXI;
R⁵ is H or R^{5c}; R^{5c} is OH or F;
R^{6c} is H, F, CH₃, or CF₃;
R^{6d} is CN, NH₂, F, COOH, C₁₋₄ alkoxy (*e.g*., methoxy), or OH;
or, R^{6c} and R^{6d} together with the carbon atom to which they are attached form 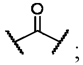
R²² is 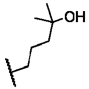 or R²¹; R²¹ is as defined in formula XXI;
in formula XIX, the carbon atom marked with * is in *R* configuration, *S* configuration, or a mixture of both; the carbon atom marked with # is in *R* configuration, *S* configuration, or a mixture of both when it is a chiral carbon atom; the carbon atom marked with A is in *R* configuration, *S* configuration, or a mixture of both when it is a chiral carbon atom; the carbon atom marked with B is in *R* configuration, *S* configuration, or a mixture of both when it is a chiral carbon atom.

In some embodiments, in the compound of formula XIX as described above, R^{3d} is H or R^{3c}; R^{3c} is -S(O)₂OH, -C(O)-CH₂COOH, -C(O)-CH₂OH, -C(O)-COOH, -CH₂CH₂OH, or - CH₂COOH;
R^{4a} is H, F, CH₃, CF₃, or OH;
R^{4b} is H, F, OH, or CF₃;
or, R^{4a} and R^{4b} together with the carbon atom to which they are attached form 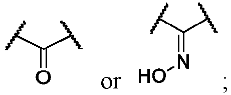
R⁵ is H or R^{5c}; R^{5c} is OH or F;
R^{6c} is H, F, CH₃, or CF₃;
R^{6d} is CN, F, COOH, C₁₋₄ alkoxy (*e.g*., methoxy), or OH;
or, R^{6c} and R^{6d} together with the carbon atom to which they are attached form 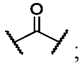
R²² is 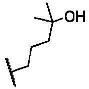 or R²¹;
R²¹ is -L¹-C(O)R^{A}, -L¹-S(O)₂R^{A}, -L¹-R^{B}, 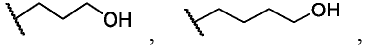 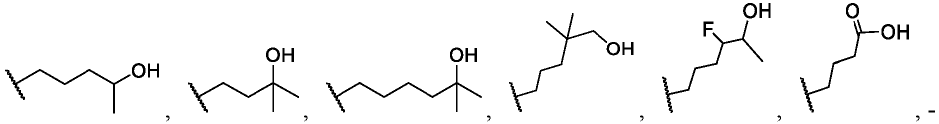 L²-R^{C}, or -L³-R^{D};
L¹ and L² are each independently a single bond, -CH₂-, -(CH₂)₂-, -(CH₂)₃-, -(CH₂)₄-, - (CH₂)₅-, or -(CH₂)₆-, wherein one -CH₂- moiety of the -CH₂-, -(CH₂)₂-, -(CH₂)₃-, -(CH₂)₄-, - (CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 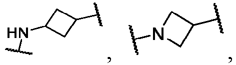 -CH=CH-, 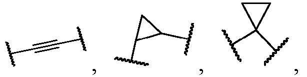 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)₂R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 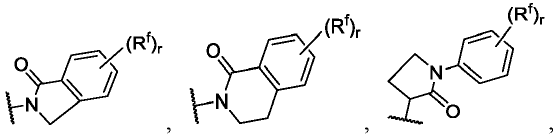 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 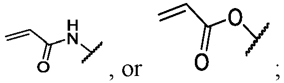
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, or C₁₋₄ alkoxy;
the number of heteroatoms in the heterocycloalkyl and heteroaryl is independently 1, 2, 3, or 4, and each heteroatom is independently N, O, or S;
in formula XIX, the carbon atom marked with * is in R configuration, S configuration, or a mixture of both; the carbon atom marked with # is in R configuration, S configuration, or a mixture of both when it is a chiral carbon atom; the carbon atom marked with A is in R configuration, S configuration, or a mixture of both when it is a chiral carbon atom; the carbon atom marked with B is in R configuration, S configuration, or a mixture of both when it is a chiral carbon atom.

In some embodiments, in the compound of formula XIX as described above, R^{3d} is H.

In some embodiments, in the compound of formula XIX as described above, R^{4a} is H, and R^{4b} is OH.

In some embodiments, in the compound of formula XIX as described above, R^{4a} is H, and R^{4b} is H.

In some embodiments, in the compound of formula XIX as described in any one of the embodiments, R⁵ is OH.

In some embodiments, in the compound of formula XIX as described in any one of the embodiments, R⁵ is F.

In some embodiments, in the compound of formula XIX as described in any one of the embodiments, R⁵ is H.

In some embodiments, in the compound of formula XIX as described in any one of the embodiments, R^{6c} is H, and R^{6d} is CN.

In some embodiments, in the compound of formula XIX as described in any one of the embodiments, R^{6c} is H, and R^{6d} is COOH.

In some embodiments, in the compound of formula XIX as described in any one of the embodiments, R^{6c} is H, and R^{6d} is OH.

In some embodiments, in the compound of formula XIX as described in any one of the embodiments, R^{6c} is H, and R^{6d} is OCH₃.

In some embodiments, in the compound of formula XIX as described in any one of the embodiments, R^{6c} and R^{6d} together with the carbon atom to which they are attached form

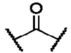

In some embodiments, in the compound of formula XIX as described in any one of the embodiments, R²² is

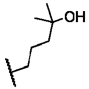

In some embodiments, in the compound of formula XIX as described in any one of the embodiments, the carbon atom marked with * is in R configuration.

In some embodiments, in the compound of formula XIX as described in any one of the embodiments, the carbon atom marked with * is in S configuration.

In some embodiments, in the compound of formula XIX as described in any one of the embodiments, the carbon atom marked with * is in a mixture of S configuration and R configuration, such as a mixture of S configuration and R configuration with a ratio of 1:1.

In some embodiments, in the compound of formula XIX as described in any one of the embodiments, the carbon atom marked with # is in R configuration.

In some embodiments, in the compound of formula XIX as described in any one of the embodiments, the carbon atom marked with # is in S configuration.

In some embodiments, in the compound of formula XIX as described in any one of the embodiments, the carbon atom marked with # is in a mixture of S configuration and R configuration, such as a mixture of S configuration and R configuration with a ratio of 1:1.

In some embodiments, in the compound of formula XIX as described in any one of the embodiments, the carbon atom marked with A is in R configuration.

In some embodiments, in the compound of formula XIX as described in any one of the embodiments, the carbon atom marked with A is in S configuration.

In some embodiments, in the compound of formula XIX as described in any one of the embodiments, the carbon atom marked with A is in a mixture of S configuration and R configuration, such as a mixture of S configuration and R configuration with a ratio of 1:1.

In some embodiments, in the compound of formula XIX as described in any one of the embodiments, the carbon atom marked with B is in R configuration.

In some embodiments, in the compound of formula XIX as described in any one of the embodiments, the carbon atom marked with B is in S configuration.

In some embodiments, in the compound of formula XIX as described in any one of the embodiments, the carbon atom marked with B is in a mixture of S configuration and R configuration, such as a mixture of S configuration and R configuration with a ratio of 1:1.

In some embodiments, the compound of formula XIX is a compound of formula XIX-1: 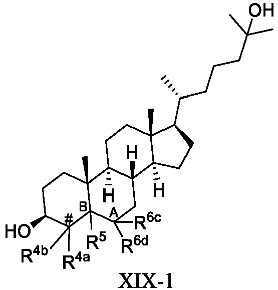 wherein #, A, B, R^{4a}, R^{4b}, R⁵, R^{6c}, and R^{6d} are as defined in any one of the embodiments of the present disclosure.

In some embodiments, the compound of formula XIX is any one of the following compounds:

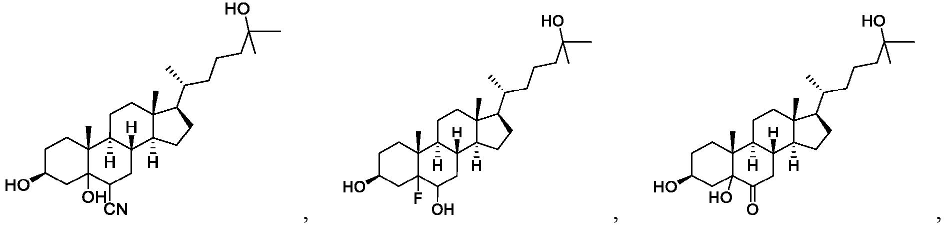

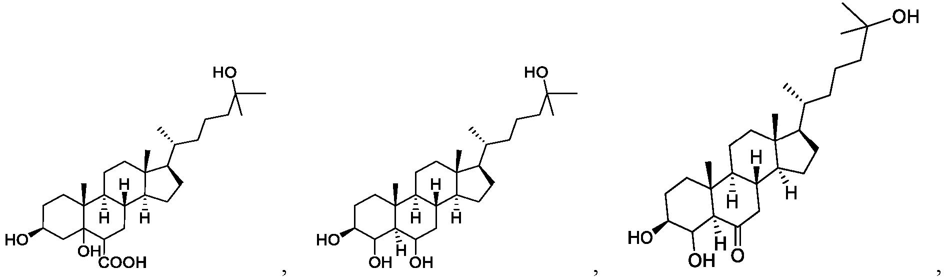

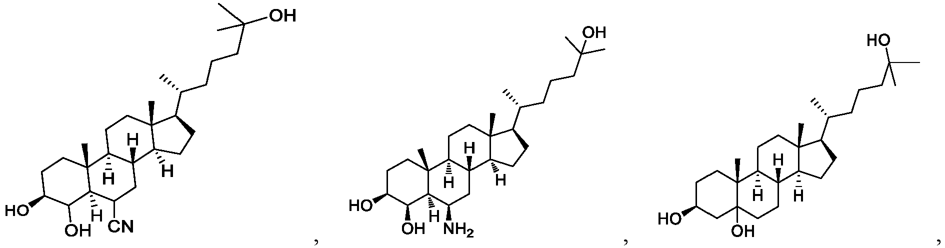

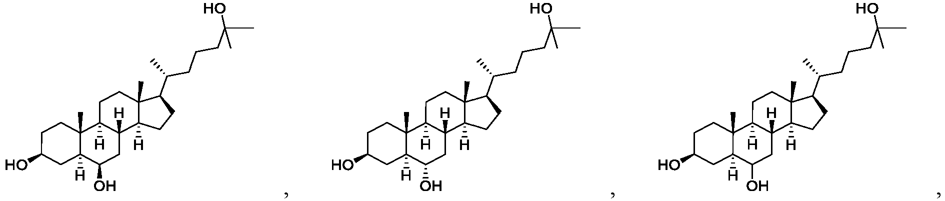

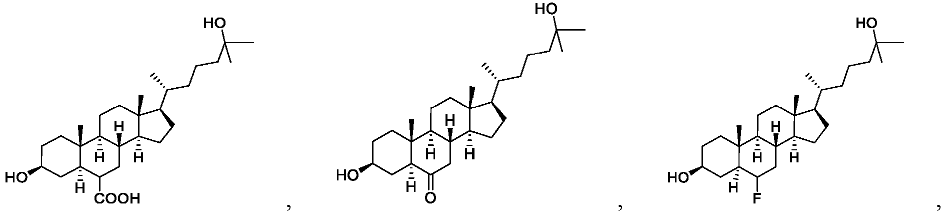

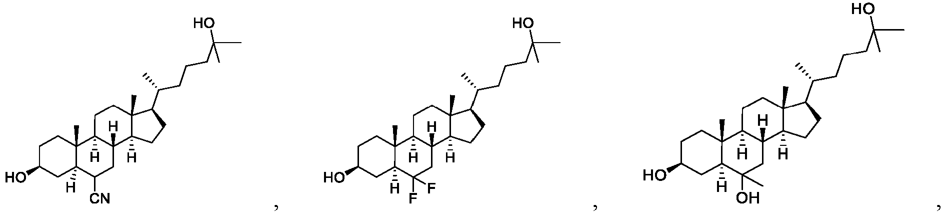

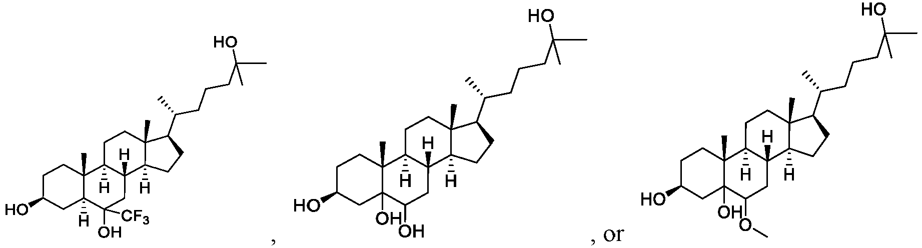

The present disclosure also provides a compound of formula XXIII or a pharmaceutically acceptable salt thereof: 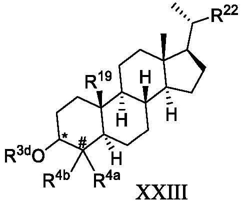 wherein R^{3d} is H or R^{3c}; R^{3c} is as defined in any one of the embodiments of formula XXI;
R^{4a} is as defined in any one of the embodiments of formula XXI;
R^{4b} is as defined in any one of the embodiments of formula XXI;
R¹⁹ is CH₂OH, CH₂CN, CH₂COOH, CH₂F, or CHF₂;
R²² is 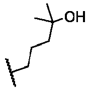 or R²¹; R²¹ is as defined in any one of the embodiments of formula XXI;
in formula XXIII, the carbon atom marked with * is in R configuration, S configuration, or a mixture of both; the carbon atom marked with # is in R configuration, S configuration, or a mixture of both when it is a chiral carbon atom.

In some embodiments, in the compound of formula XXIII as described above, R^{3d} is H or R^{3c}; R^{3c} is -S(O)₂OH, -C(O)-CH₂COOH, -C(O)-CH₂OH, -C(O)-COOH, -CH₂CH₂OH, or -CH₂COOH;
R^{4a} is H, F, CH₃, CF₃, or OH;
R^{4b} is H, F, OH, or CF₃;
or, R^{4a} and R^{4b} together with the carbon atom to which they are attached form 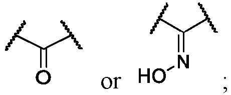
R¹⁹ is CH₂OH, CH₂CN, CH₂COOH, CH₂F, or CHF₂;
R²² is 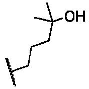 or R²¹;
R²¹ is -L¹-C(O)R^{A}, -L¹-S(O)₂R^{A}, -L¹-R^{B}, 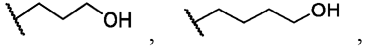 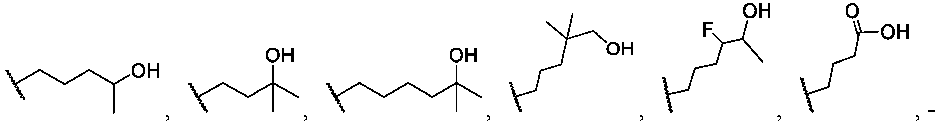 L²-R^{C}, or -L³-R^{D};
L¹ and L² are each independently a single bond, -CH₂-, -(CH₂)₂-, -(CH₂)₃-, -(CH₂)₄-, - (CH₂)₅-, or -(CH₂)₆-, wherein one -CH₂- moiety of the -CH₂-, -(CH₂)₂-, -(CH₂)₃-, -(CH₂)₄-, - (CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 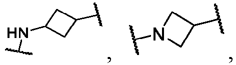 -CH=CH-, 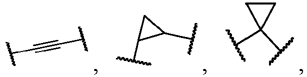 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)₂R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 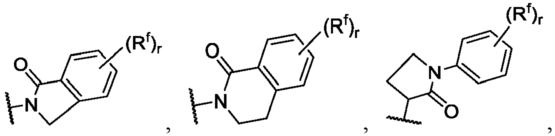 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 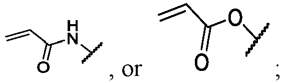
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, or C₁₋₄ alkoxy;
the number of heteroatoms in the heterocycloalkyl and heteroaryl is independently 1, 2, 3, or 4, and each heteroatom is independently N, O, or S;
in formula XXIII, the carbon atom marked with * is in R configuration, S configuration, or a mixture of both; the carbon atom marked with # is in R configuration, S configuration, or a mixture of both when it is a chiral carbon atom.

In some embodiments, in the compound of formula XXIII as described above, R^{3d} is H.

In some embodiments, in the compound of formula XXIII as described above, R^{4a} is H; R^{4b} is H.

In some embodiments, in the compound of formula XXIII as described in any one of the embodiments, R²² is

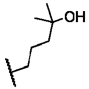

In some embodiments, in the compound of formula XXIII as described in any one of the embodiments, the carbon atom marked with * is in R configuration.

In some embodiments, in the compound of formula XXIII as described in any one of the embodiments, the carbon atom marked with * is in S configuration.

In some embodiments, in the compound of formula XXIII as described in any one of the embodiments, the carbon atom marked with * is in a mixture of S configuration and R configuration, such as a mixture of S configuration and R configuration with a ratio of 1:1.

In some embodiments, in the compound of formula XXIII as described in any one of the embodiments, the carbon atom marked with # is in R configuration.

In some embodiments, in the compound of formula XXIII as described in any one of the embodiments, the carbon atom marked with # is in S configuration.

In some embodiments, in the compound of formula XXIII as described in any one of the embodiments, the carbon atom marked with # is in a mixture of S configuration and R configuration, such as a mixture of S configuration and R configuration with a ratio of 1:1.

In some embodiments, the compound of formula XXIII is any one of the following compounds:

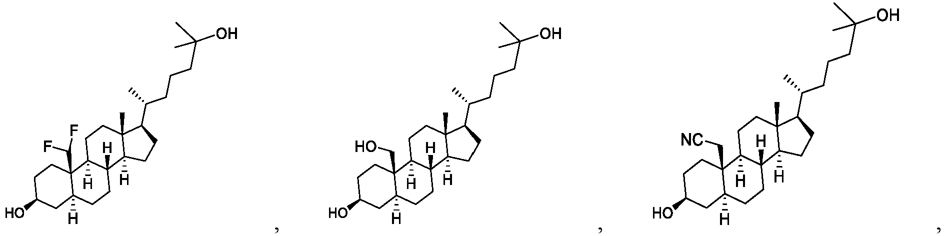

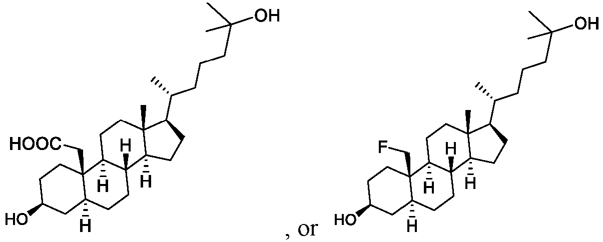

The present disclosure also provides a compound of formula XXIV or a pharmaceutically acceptable salt thereof: 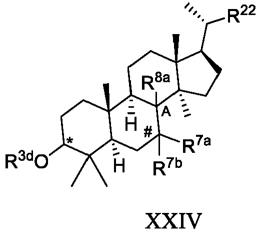 wherein R^{3d} is H or R^{3c}; R^{3c} is as defined in any one of the embodiments of formula XXI;
R^{7a} is as defined in any one of the embodiments of formula XXI;
R^{7b} is as defined in any one of the embodiments of formula XXI;
R^{8a} is H or F;
R²² is 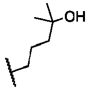 or R²¹; R²¹ is as defined in any one of the embodiments of formula XXI;
in formula XXIV, the carbon atom marked with * is in R configuration, S configuration, or a mixture of both; the carbon atom marked with # is in R configuration, S configuration, or a mixture of both when it is a chiral carbon atom; the carbon atom marked with A is in R configuration, S configuration, or a mixture of both when it is a chiral carbon atom.

In some embodiments, in the compound of formula XXIV as described above, R^{3d} is H or R^{3c}; R^{3c} is -S(O)₂OH, -C(O)-CH₂COOH, -C(O)-CH₂OH, -C(O)-COOH, -CH₂CH₂OH, or -CH₂COOH;
R^{7a} is H, F, CH₃, or CF₃; R^{7b} is F or OH; or, R^{7a} and R^{7b} together with the carbon atom to which they are attached form 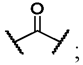
R^{8a} is H or F;
R²² is 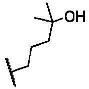 or R²¹;
R²¹ is -L¹-C(O)R^{A}, -L¹-S(O)₂R^{A}, -L¹-R^{B}, 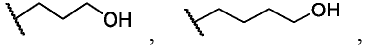 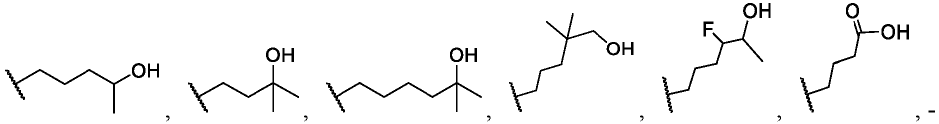 L²-R^{C}, or -L³-R^{D};
L¹ and L² are each independently a single bond, -CH₂-, -(CH₂)₂-, -(CH₂)₃-, -(CH₂)₄-, - (CH₂)₅-, or -(CH₂)₆-, wherein one -CH₂- moiety of the -CH₂-, -(CH₂)₂-, -(CH₂)₃-, -(CH₂)₄-, - (CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 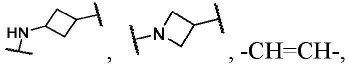 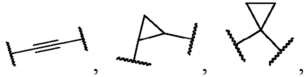 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)₂R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 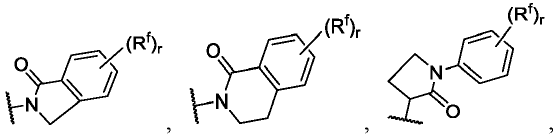 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 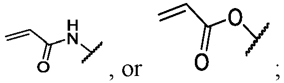
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, or C₁₋₄ alkoxy;
the number of heteroatoms in the heterocycloalkyl and heteroaryl is independently 1, 2, 3, or 4, and each heteroatom is independently N, O, or S;
in formula XXIV, the carbon atom marked with * is in R configuration, S configuration, or a mixture of both; the carbon atom marked with # is in R configuration, S configuration, or a mixture of both when it is a chiral carbon atom; the carbon atom marked with A is in R configuration, S configuration, or a mixture of both when it is a chiral carbon atom.

In some embodiments, in the compound of formula XXIV as described above, R^{3d} is H.

In some embodiments, in the compound of formula XXIV as described above, R^{7a} is H; R^{7b} is OH.

In some embodiments, in the compound of formula XXIV as described above, R^{7a} and R^{7b} together with the carbon atom to which they are attached form

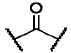

In some embodiments, in the compound of formula XXIV as described above, R^{8a} is H.

In some embodiments, in the compound of formula XXIV as described in any one of the embodiments, R²² is

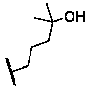

In some embodiments, in the compound of formula XXIV as described in any one of the embodiments, the carbon atom marked with * is in R configuration.

In some embodiments, in the compound of formula XXIV as described in any one of the embodiments, the carbon atom marked with * is in S configuration.

In some embodiments, in the compound of formula XXIV as described in any one of the embodiments, the carbon atom marked with * is in a mixture of S configuration and R configuration, such as a mixture of S configuration and R configuration with a ratio of 1:1.

In some embodiments, in the compound of formula XXIV as described in any one of the embodiments, the carbon atom marked with # is in R configuration.

In some embodiments, in the compound of formula XXIV as described in any one of the embodiments, the carbon atom marked with # is in S configuration.

In some embodiments, in the compound of formula XXIV as described in any one of the embodiments, the carbon atom marked with # is in a mixture of S configuration and R configuration, such as a mixture of S configuration and R configuration with a ratio of 1:1.

In some embodiments, in the compound of formula XXIV as described in any one of the embodiments, the carbon atom marked with A is in R configuration.

In some embodiments, in the compound of formula XXIV as described in any one of the embodiments, the carbon atom marked with A is in S configuration.

In some embodiments, in the compound of formula XXIV as described in any one of the embodiments, the carbon atom marked with A is in a mixture of S configuration and R configuration, such as a mixture of S configuration and R configuration with a ratio of 1:1.

In some embodiments, the compound of formula XXIV is any one of the following compounds:

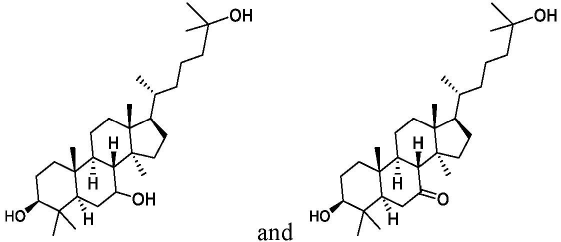

The present disclosure also provides a compound of formula XXVI or a pharmaceutically acceptable salt thereof: 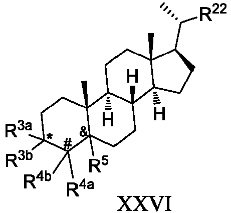
R^{3a} and R^{3b} are as defined in any one of the following schemes:
   (i) R^{3a} is H, and R^{3b} is -NH₂, -NHS(O)₂CH₃, -NHS(O)₂CH₂CH₃, - NHS(O)₂CH₂CH₂CH₃, -NHS(O)₂CF₃, -NH-C(O)-CH₂OH, -CH₂OH, -COOH, -C(O)-OCH₃, - CH(CH₃)OH, -CH₂-C(O)-CH₂OH, or -CH(OH)-CH₂CH₂OH;
   (ii) R^{3a} is CH₃, and R^{3b} is -OH; and
   (iii) R^{3a} and R^{3b} together with the carbon atom to which they are attached form 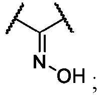
R^{4a} is as defined in any one of the embodiments of formula XXI;
R^{4b} is as defined in any one of the embodiments of formula XXI;
R⁵ is as defined in any one of the embodiments of formula XVI;
R²² is 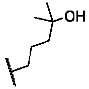 or R²¹; R²¹ is as defined in any one of the embodiments of formula XXI;
in formula XXVI, the carbon atom marked with * is in R configuration, S configuration, or a mixture of both; the carbon atom marked with # is in R configuration, S configuration, or a mixture of both when it is a chiral carbon atom; the carbon atom marked with & is in R configuration, S configuration, or a mixture of both when it is a chiral carbon atom.

In some embodiments, in the compound of formula XXVI as described above, R^{3a} and R^{3b} are as defined in any one of the following schemes:
(i) R^{3a} is H, and R^{3b} is -NH₂, -NHS(O)₂CH₃, -NHS(O)₂CH₂CH₃, - NHS(O)₂CH₂CH₂CH₃, -NHS(O)₂CF₃, -NH-C(O)-CH₂OH, -CH₂OH, -COOH, -C(O)-OCH₃, - CH(CH₃)OH, -CH₂-C(O)-CH₂OH, or -CH(OH)-CH₂CH₂OH;
(ii) R^{3a} is CH₃, and R^{3b} is -OH; and
(iii) R^{3a} and R^{3b} together with the carbon atom to which they are attached form 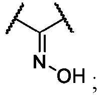
   R^{4a} is H, F, CH₃, CF₃, or OH;
   R^{4b} is H, F, OH, or CF₃;
   or, R^{4a} and R^{4b} together with the carbon atom to which they are attached form 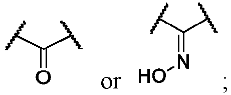
   R⁵ is H or R^{5c}; R^{5c} is OH or F;
   R²² is 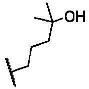 or R²¹;
   R²¹ is -L¹-C(O)R^{A}, -L¹-S(O)₂R^{A}, -L¹-R^{B}, 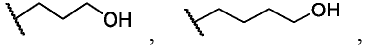 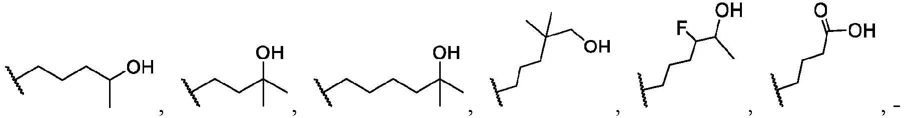 L²-R^{C}, or -L³-R^{D};
   L¹ and L² are each independently a single bond, -CH₂-, -(CH₂)₂-, -(CH₂)₃-, -(CH₂)₄-, - (CH₂)₅-, or -(CH₂)₆-, wherein one -CH₂- moiety of the -CH₂-, -(CH₂)₂-, -(CH₂)₃-, -(CH₂)₄-, - (CH₂)₅-, and -(CH₂)₆- is optionally substituted by -X-;
   L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
   each X is independently -CHR^{5a}-, -CR^{5a}R^{5b}-, or -Y-;
   R^{5a} and R^{5b} are each independently F, C₁₋₄ alkyl, or fluoro-C₁₋₄ alkyl;
   each Y is independently -O-, -NH-, -N(C₁₋₄ alkyl)-, 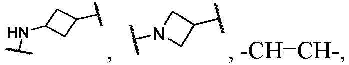 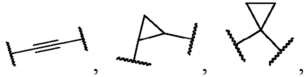 or -CHR^{g}-;
   R^{g} is -C₁₋₄ alkylene-OH;
   each R^{A} is independently -NR^{21a}R^{21b};
   R^{B} is -N(R^{21a})-C(O)R^{21b} or -N(R^{21a})-S(O)₂R^{21b};
   R^{D} is -OH, -CH₂OH, R^{C}, -CH(CH₃)-OH, or -C(CH₃)₂-OH;
   each R^{C} is independently -C(R^{21c})(R^{21d})-OH, CN, -C(O)R^{E}, NH₂, C₁₋₄ alkoxy, 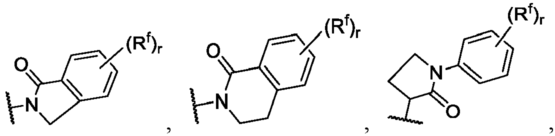 3- to 6-membered heterocycloalkyl, or 5- to 10-membered heteroaryl; the 5- to 10-membered heteroaryl is unsubstituted or substituted by p R^{c};
   R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
   each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
   each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
   or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
   m, p, and q are each independently 1, 2, 3, 4, or 5;
   R^{a}, R^{b}, and R^{c} are each independently F, Cl, OH, COOH, CN, NO₂, C₁₋₄ alkyl, fluoro-C₁₋₄ alkyl, C₁₋₄ alkoxy, 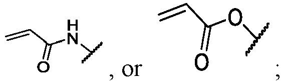
   R^{21c} is H, F, C₁₋₄ alkyl, fluoro-C₁₋₄ alkyl, C₂₋₄ alkenyl, or C₃₋₆ cycloalkyl;
   R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
   or, R^{21c} and R^{21d} together with the carbon atom to which they are attached form a C₃₋₆ cycloalkyl group or a 3- to 6-membered heterocycloalkyl group;
   L⁴ is C₁₋₄ alkylene;
   R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
   each j is independently 1, 2, 3, or 4;
   each r is independently 0, 1, 2, 3, or 4;
   R^{d} and R^{f} are each independently F, Cl, OH, CN, NO₂, C₁₋₄ alkyl, fluoro-C₁₋₄ alkyl, or C₁₋₄ alkoxy;
   the number of heteroatoms in the heterocycloalkyl and heteroaryl is independently 1, 2, 3, or 4, and each heteroatom is independently N, O, or S;
   in formula XXVI, the carbon atom marked with * is in R configuration, S configuration, or a mixture of both; the carbon atom marked with # is in R configuration, S configuration, or a mixture of both when it is a chiral carbon atom; the carbon atom marked with & is in R configuration, S configuration, or a mixture of both when it is a chiral carbon atom.

In some embodiments, in the compound of formula XXVI as described above, R^{3a} and R^{3b} are as defined in any one of the following schemes:
a) R^{3a} is H, and R^{3b} is -NHS(O)₂CH₃, -CH₂OH, -COOH, -C(O)-OCH₃, or - CH(CH₃)OH; and
b) R^{3a} is CH₃, and R^{3b} is -OH.

In some embodiments, in the compound of formula XIX as described above, R^{4a} is H, and R^{4b} is H.

In some embodiments, in the compound of formula XXVI as described above, R⁵ is H.

In some embodiments, in the compound of formula XXVI as described above, R²² is

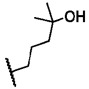

In some embodiments, in the compound of formula XXVI as described in any one of the embodiments, the carbon atom marked with * is in R configuration.

In some embodiments, in the compound of formula XXVI as described in any one of the embodiments, the carbon atom marked with * is in S configuration.

In some embodiments, in the compound of formula XXVI as described in any one of the embodiments, the carbon atom marked with * is in a mixture of S configuration and R configuration, such as a mixture of S configuration and R configuration with a ratio of 1:1.

In some embodiments, in the compound of formula XXVI as described in any one of the embodiments, in formula XXVI, the carbon atom marked with # is in R configuration when it is a chiral carbon atom.

In some embodiments, in the compound of formula XXVI as described in any one of the embodiments, in formula XXVI, the carbon atom marked with # is in S configuration when it is a chiral carbon atom.

In some embodiments, in the compound of formula XXVI as described in any one of the embodiments, in formula XXVI, the carbon atom marked with # is in a mixture of S configuration and R configuration, such as a mixture of S configuration and R configuration with a ratio of 1:1.

In some embodiments, in the compound of formula XXVI as described in any one of the embodiments, in formula XXVI, the carbon atom marked with & is in R configuration when it is a chiral carbon atom.

In some embodiments, in the compound of formula XXVI as described in any one of the embodiments, in formula XXVI, the carbon atom marked with & is in S configuration when it is a chiral carbon atom.

In some embodiments, in the compound of formula XXVI as described in any one of the embodiments, in formula XXVI, the carbon atom marked with & is in a mixture of S configuration and R configuration, such as a mixture of S configuration and R configuration with a ratio of 1:1.

In some embodiments, the compound of formula XXVI is a compound of formula XXVI-1: 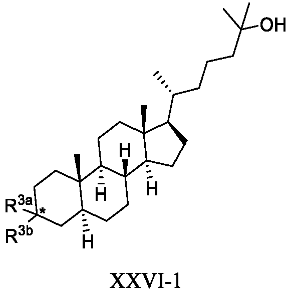 wherein *, R^{3a}, and R^{3b} are as defined in any one of the embodiments of the present disclosure.

In some embodiments, the compound of formula XXVI is any one of the following compounds:

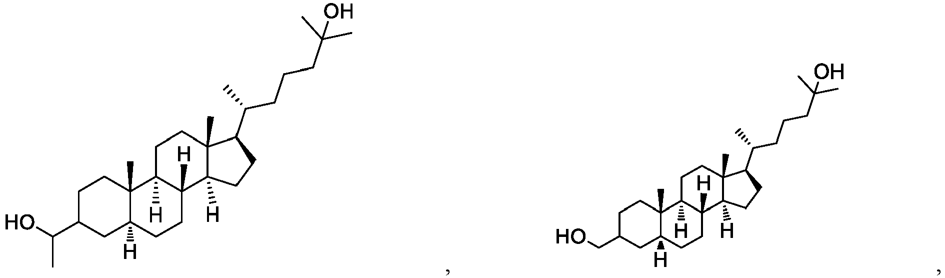

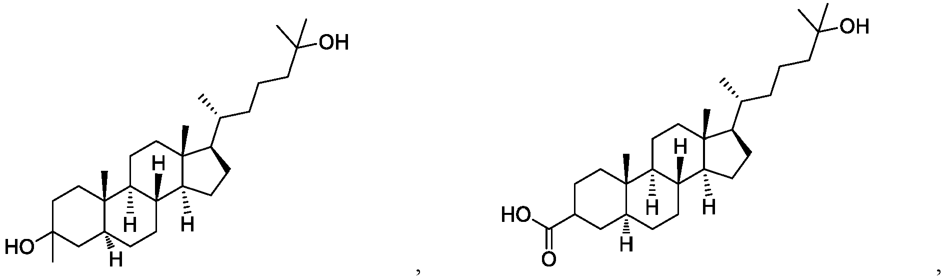

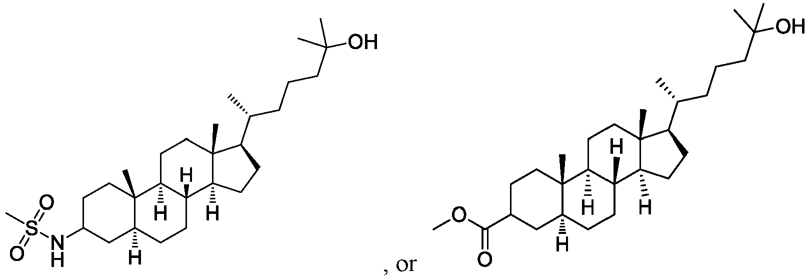

The compounds of the present disclosure can be prepared from known raw materials (e.g., lanosterol) by various conventional reaction methods in the art (e.g., hydroxyl protection, double bond ozonation, Wittig reaction, hydrolysis reaction, amide condensation reaction, Grignard reagent addition reaction, reduction reaction, nucleophilic substitution reaction, and epoxidation reaction). Exemplary preparation methods are as described in the preparation examples of the present disclosure.

For example, in the preparation method of compound 37 and analogs thereof, compound 37 and analogs thereof can be obtained through hydroxyl protection, double bond ozonation, Wittig reaction, hydrolysis, and condensation by using lanosterol as a starting material. The reaction route is as follows:

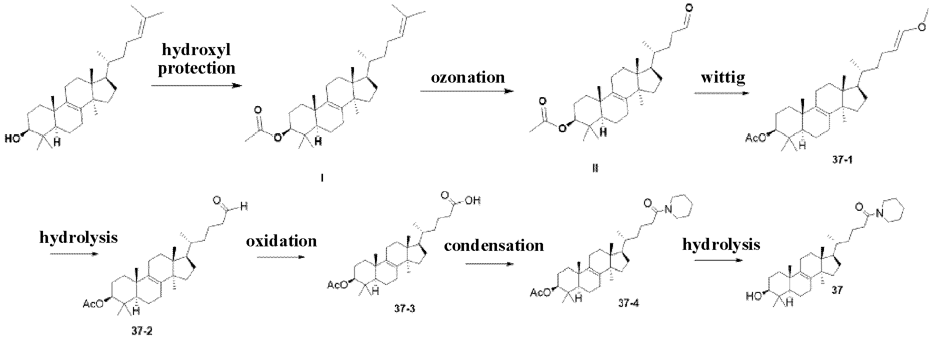

For example, in the preparation method of compound 101 and analogs thereof, compound 101 and analogs thereof can be obtained through hydroxyl protection, double bond ozonation, Wittig reaction, hydrolysis, and Grignard reagent addition reaction by using lanosterol as a starting material. The reaction route is as follows:

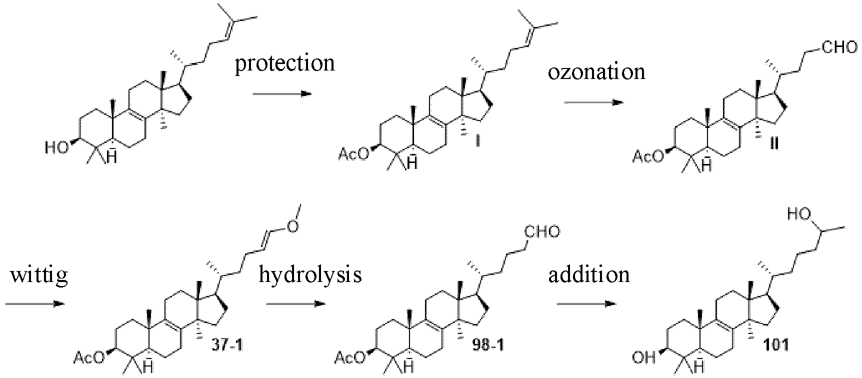

For example, in the preparation method of compound 80 and analogs thereof, compound 80 and analogs thereof can be obtained through hydroxyl protection, double bond ozonation, reduction, two-step substitution, and addition reaction by using lanosterol as a starting material. The reaction route is as follows:

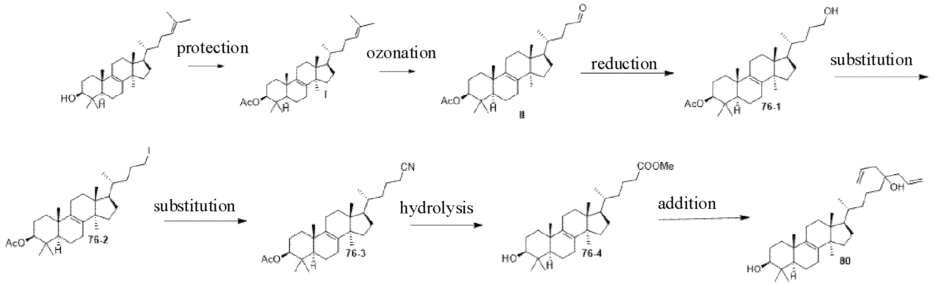

For example, in the preparation method of compound 125 and analogs thereof, compound 125 and analogs thereof can be finally obtained through hydroxyl protection, double bond ozonation, Wittig reaction, hydrolysis, epoxidation, and hydrolysis by using lanosterol as a starting material. The reaction route is as follows:

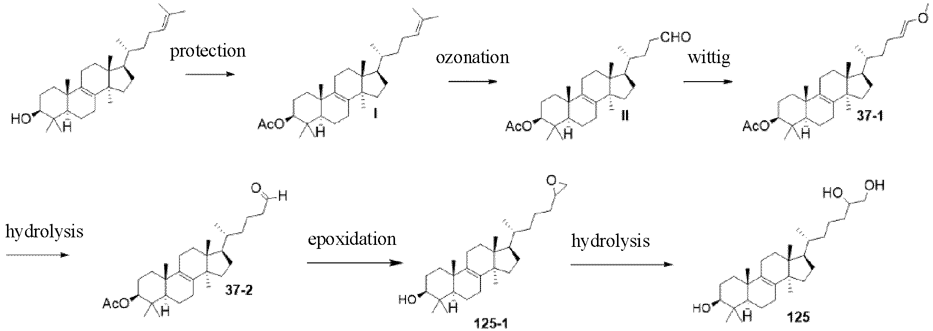

For example, in the preparation method of compound 193 and analogs thereof, compound 193 can be obtained through hydroxyl protection, double bond ozonation, double bond displacement, double bond ozonation, reductive amination, condensation, and hydrolysis by using lanosterol as a starting material. The reaction route of the preparation method is as follows:

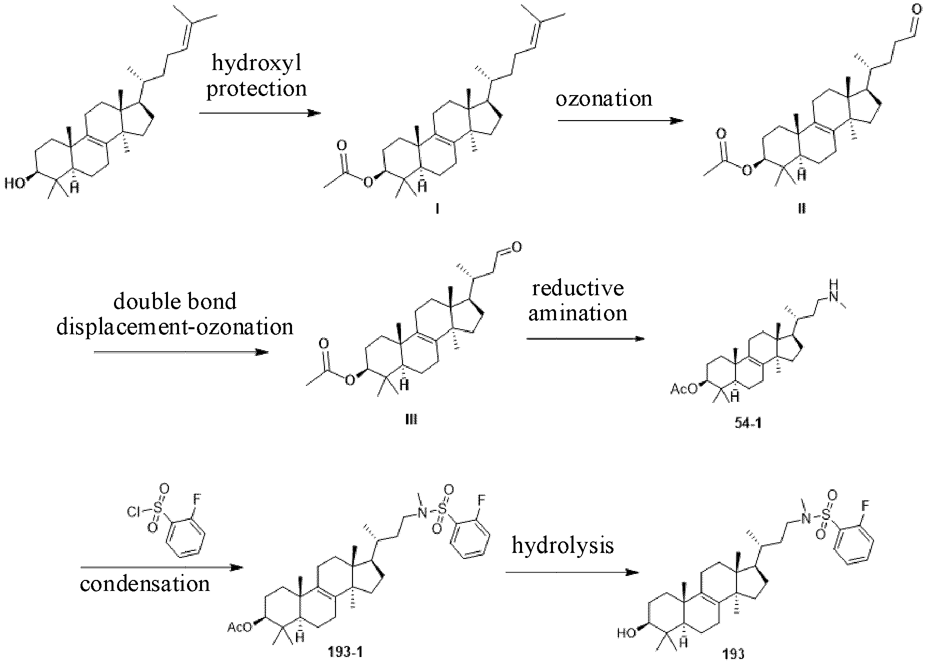

The present disclosure also provides a pharmaceutical composition comprising the compound or the pharmaceutically acceptable salt thereof as described above, and at least one pharmaceutical excipient.

The present disclosure also provides a use of the compound or the pharmaceutically acceptable salt thereof as described above, or the pharmaceutical composition as described above in the manufacture of a medicament for preventing and/or treating a disease, wherein the disease is obesity, hyperlipidemia, fatty liver, diabetes, atherosclerosis, cardiovascular and cerebrovascular disease, liver cancer, or skin damage.

The present disclosure also provides a use of the compound or the pharmaceutically acceptable salt thereof as described above, or the pharmaceutical composition as described above in the manufacture of a medicament for inhibiting the SREBP pathway.

The present disclosure also provides a method for inhibiting the SREBP pathway, comprising administering to a subject an effective amount of the compound or the pharmaceutically acceptable salt thereof as described above.

The present disclosure also provides a method for preventing and/or treating a disease, comprising administering to a subject an effective amount of the compound or the pharmaceutically acceptable salt thereof as described above, wherein the disease is obesity, hyperlipidemia, fatty liver, diabetes, atherosclerosis, cardiovascular and cerebrovascular disease, liver cancer, or skin damage.

### Definition and description

Unless otherwise specified, the following terms and phrases used herein have the following meanings. A specific term or phrase should not be considered indefinite or unclear in the absence of a particular definition, but should be understood in the ordinary sense. When a trade name appears herein, it is intended to refer to its corresponding commodity or active ingredient thereof.

As used herein, the term "substituted" or "substituent" means that a hydrogen atom in a group is replaced by a designated group. When the position of a substitution is not specified, the substitution may be at any position, but is allowed only if it results in a stable or chemically viable chemical entity. An example is given as follows: The structure of 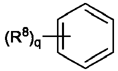 indicates that the hydrogen atom on the benzene ring is substituted by q R⁸. When more than one R⁸ is present, each R⁸ is the same or different.

When any variable (such as R) occurs in the constitution or structure of the compound more than once, the definition of the variable at each occurrence is independent. Thus, for example, if a group is substituted by 0 to 2 R, the group can be optionally substituted by up to two R, wherein the definition of R at each occurrence is independent. Moreover, a combination of the substituent and/or the variant thereof is allowed only if the combination results in a stable compound.

When the linking group listed herein does not indicate the direction for linking, the direction for linking may be arbitrary, including both left-to-right linking and right-to-left linking. By way of example, the linking group L in -A-L-B is -C-D-, and when L does not indicate the direction for linking, -A-L-B includes -A-C-D-B and -A-D-C-B.

When one of the variables is selected from a single bond, it means that the two groups linked by the single bond are connected directly. For example, when L in A-L-Z represents a single bond, the structure of A-L-Z is actually A-Z.

As used herein, the term "alkyl" refers to a saturated linear or branched monovalent hydrocarbon group. C₁-C₆ alkyl means an alkyl group having 1 to 6 carbon atoms. In some embodiments, C₁-C₆ alkyl may be C₁-C₄ alkyl. C₁-C₄ alkyl includes methyl, ethyl, n-propyl, isopropyl, n-butyl, isobutyl, sec-butyl, and tert-butyl.

As used herein, the term "alkenyl" refers to a linear or branched monovalent hydrocarbon group containing at least one carbon-carbon double bond. When the alkenyl group contains both saturated and unsaturated carbon atoms, it can be attached to other structures either through saturated carbon atoms or through unsaturated carbon atoms. C₂-C₄ alkenyl means an alkenyl group having 2, 3, or 4 carbon atoms. Specific examples of alkenyl include, but are not limited to, vinyl and allyl.

As used herein, the term "alkylene" refers to a saturated linear or branched divalent hydrocarbon group. C₁-C₄ alkylene refers to an alkylene group having 1 to 4 carbon atoms, specifically methylene, ethylene (e.g., -CH₂CH₂-, -CH(CH₃)-), propylene (e.g., -CH₂CH₂CH₂-, -C(CH₃)₂-, -CH₂CH(CH₃)-), or butylene (e.g., -CH₂CH₂CH₂CH₂-, -CH(CH₃)CH(CH₃)-, - CH₂CH(CH₃)CH₂-).

As used herein, the term "fluoroalkyl" refers to a group in which one or more hydrogen atoms in an alkyl group is substituted by fluorine, wherein the alkyl is as defined above. Examples of fluoroalkyl include, but are not limited to, fluoromethyl, difluoromethyl, trifluoromethyl, and pentafluoroethyl.

As used herein, the term "alkoxy" refers to -O-alkyl, wherein the alkyl is as defined above. C₁-C₄ alkoxy refers to -O-(C₁-C₄ alkyl), wherein the C₁-C₄ alkyl is as defined above, that is, C₁-C₄ alkoxy may specifically be methoxy, ethoxy, n-propoxy, isopropoxy, n-butoxy, isobutoxy, sec-butoxy, or tert-butoxy.

As used herein, the term "cycloalkyl" refers to a saturated monocyclic or polycyclic (e.g., fused, spiro, or bridged) cyclic hydrocarbon group. Examples of cycloalkyl include, but are not limited to, cyclopropyl, cyclobutyl, cyclopentyl, cyclohexyl, 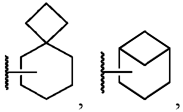 and 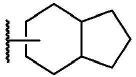 C₃₋₁₀ cycloalkyl may specifically be C₃ cycloalkyl, C₄ cycloalkyl, C₅ cycloalkyl, C₆ cycloalkyl, C₇ cycloalkyl, C₈ cycloalkyl, C₉ cycloalkyl, or C₁₀ cycloalkyl. C₃₋₆ cycloalkyl may specifically be C₃ cycloalkyl, C₄ cycloalkyl, C₅ cycloalkyl, or C₆ cycloalkyl. In some embodiments, the cycloalkyl is monocyclic. In some embodiments, the cycloalkyl is polycyclic (e.g., fused, spiro, or bridged).

As used herein, the term "heterocycloalkyl" refers to a saturated monocyclic or polycyclic (e.g., fused, spiro, or bridged) cyclic group consisting of a carbon atom and at least one heteroatom, wherein the heteroatom is independently selected from N, O, and S. The heterocycloalkyl group can be attached to other structures through a carbon atom and a heteroatom on the ring. Examples of heterocycloalkyl include, but are not limited to, 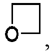 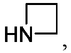 tetrahydrofuranyl, tetrahydrothienyl, tetrahydropyrrolyl, piperidinyl, piperazinyl, and morpholinyl. 3- to 10-membered heterocycloalkyl may specifically be 3-membered, 4-membered, 5-membered, 6-membered, 7-membered, 8-membered, 9-membered, or 10-membered heterocycloalkyl. 3- to 6-membered heterocycloalkyl may specifically be 3-membered, 4-membered, 5-membered, or 6-membered heterocycloalkyl. In some embodiments, the heterocycloalkyl is monocyclic. In some embodiments, the heterocycloalkyl is polycyclic (e.g., fused, spiro, or bridged).

As used herein, the term "C₆₋₁₀ aryl" refers to phenyl or naphthyl.

As used herein, the term "heteroaryl" refers to an aromatic monocyclic or fused cyclic group consisting of a carbon atom and at least one heteroatom, wherein the heteroatom is independently selected from N, O, and S. 5- to 10-membered heteroaryl may specifically be 5-membered, 6-membered, 7-membered, 8-membered, 9-membered, or 10-membered heteroaryl, such as 5- to 6-membered heteroaryl or 8- to 10-membered heteroaryl. The 5- to 6-membered heteroaryl is monocyclic. Specific examples include, but are not limited to, pyrrolyl, furyl, thienyl, oxazolyl, isoxazolyl, thiazolyl, isothiazolyl, pyrazolyl, imidazolyl, pyridyl, pyrimidinyl, and pyrazinyl. Examples of 8- to 10-membered fused heteroaryl include, but are not limited to, benzopyrrolyl, benzofuranyl, benzothienyl, benzoxazolyl, benzisoxazolyl, benzothiazolyl, benzisothiazolyl, benzopyrazolyl, benzimidazolyl, benzopyridinyl, benzopyrimidinyl, benzopyrazinyl, thiazolothiazolyl, pyridopyridinyl, pyridopyrazinyl, and pyridopyrimidinyl.

As used herein, 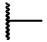 in the chemical structural formula indicates the position for linking. When 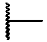 is contained in a cyclic group and the ring atom to which 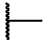 is attached is not specified, 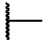 can be attached to any ring atom, but is allowed only if it results in a stable or chemically viable chemical entity. For example, 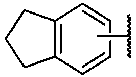 includes 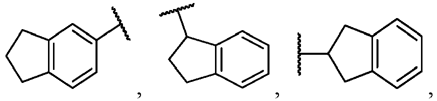 and other structures.

As used herein, the term "pharmaceutically acceptable salt" refers to a salt formed from a suitable non-toxic organic acid, inorganic acid, organic base, or inorganic base with a compound which retains the biological activity of the compound. The organic acid may be a variety of organic acids conventional in the art that can form salts, preferably one or more of methanesulfonic acid, *p*-toluenesulfonic acid, maleic acid, fumaric acid, citric acid, tartaric acid, malic acid, lactic acid, formic acid, acetic acid, propionic acid, trifluoroacetic acid, oxalic acid, succinic acid, benzoic acid, isethionic acid, naphthalenesulfonic acid, and salicylic acid. The inorganic acid may be a variety of inorganic acids conventional in the art that can form salts, preferably one or more of hydrochloric acid, sulfuric acid, and phosphoric acid. The organic base may be a variety of organic bases conventional in the art that can form salts, preferably one or more of pyridines, imidazoles, pyrazines, indoles, purines, tertiary amines, and anilines. The tertiary amine organic base is preferably triethylamine and/or *N,N*-diisopropylethylamine. The aniline organic base is preferably *N,N-*dimethylaniline. The pyridine organic base is preferably one or more of pyridine, methylpyridine, 4-dimethylaminopyridine, and 2-methyl-5-ethylpyridine. The inorganic base may be a variety of inorganic bases conventional in the art that can form salts, preferably one or more of alkali metal hydride, alkali metal hydroxide, alkali metal alkoxide, potassium carbonate, sodium carbonate, lithium carbonate, cesium carbonate, potassium bicarbonate, and sodium bicarbonate. The alkali metal hydride is preferably sodium hydride and/or potassium hydride. The alkali metal hydroxide is preferably one or more of sodium hydroxide, potassium hydroxide, and lithium hydroxide. The alkali metal alkoxide is preferably one or more of sodium methoxide, sodium ethoxide, potassium *tert*-butoxide, and sodium *tert*-butoxide.

In chemical structures, the absolute configuration of a stereogenic center is represented by a wedged solid bond 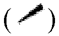 and a wedged dashed bond 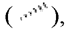 and the relative configuration of a stereogenic center is represented by a straight solid bond 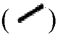 and a straight dashed bond 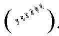 The bond" 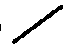 " does not specify a configuration, that is, if configurational isomerism exists in the chemical structure, the bond " 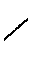 " may be " 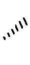 "" or "" 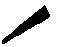 ", or includes both " 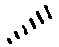 " and " 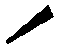 " configurations (*e.g*., " 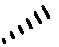 " and " 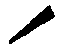 " with a ratio of 1:1). When the carbon-carbon double bond does not indicate the specific configuration, it can be in *E* or *Z* configuration. Stereoisomers can be synthesized using chiral starting materials, prepared by chiral resolution, or can be resolved using conventional techniques such as, but not limited to, high performance liquid chromatography (HPLC) using chiral columns.

As used herein, the term "subject" includes any animal, preferably a mammal, more preferably a human.

As used herein, the term "effective amount" refers to a sufficient amount of a drug or agent that is non-toxic but can achieve the desired effect. The determination of the effective amount varies with each individual, depending on the age and general condition of the subject, as well as the specific active substance. The appropriate effective amount in each case can be determined by those skilled in the art according to routine experiments.

On the basis of not violating common knowledge in the art, the above preferred conditions can be arbitrarily combined to obtain preferred examples of the present disclosure.

The reagents and raw materials used in the present disclosure are all commercially available.

The positive and progressive effect of the present disclosure is that the present disclosure provides a new class of compounds with SREBP pathway inhibitory activity, which can be used for preventing and/or treating diseases such as obesity, hyperlipidemia, fatty liver, diabetes, atherosclerosis, cardiovascular and cerebrovascular disease, liver cancer, and skin damage.

### BRIEF DESCRIPTION OF THE DRAWINGS

Fig. 1 shows the results of inhibition of AMI,N diet-induced weight gain in mice by 25-hydroxylanosterol.
Fig. 2 shows the effect of 25-hydroxylanosterol on food intake in mice.
Fig. 3 shows the reduction of total blood cholesterol levels in mice by 25-hydroxy lanosterol.
Fig. 4 shows the reduction of total blood triglyceride levels in mice by 25-hydroxy lanosterol.
Fig. 5 shows the reduction of total liver cholesterol levels in mice by 25-hydroxy lanosterol.
Fig. 6 shows the reduction of total liver triglyceride levels in mice by 25-hydroxy lanosterol.
Fig. 7 shows the reduction of blood aspartate aminotransferase (AST) levels in mice by 25-hydroxylanosterol.
Fig. 8 shows the reduction of blood alanine aminotransferase (ALT) levels in mice by 25-hydroxylanosterol.
Fig. 9 shows the results of HE staining of liver sections of mice: C57BL/6J CD group.
Fig. 10 shows the results of HE staining of liver sections of mice: *Ldlr*^{*-*/*-*} CD group.
Fig. 11 shows the results of HE staining of liver sections of mice: *Ldlr*^{*-*/*-*} AMLN group.
Fig. 12 shows the results of HE staining of liver sections of mice: *Ldlr*^{*-*/*-*} AMI,N + 25-HL group.
Fig. 13 shows the results of HE staining of liver sections and quantitative statistical results of NAFLD activity scores of mice in four groups.
Fig. 14 shows the results of Oil Red O staining of liver sections of mice: C57BL/6J CD group.
Fig. 15 shows the results of Oil Red O staining of liver sections of mice: *Ldlr*^{*-*/*-*} CD group.
Fig. 16 shows the results of Oil Red O staining of liver sections of mice: *Ldlr*^{*-*/*-*} AMI,N group.
Fig. 17 shows the results of Oil Red O staining of liver sections of mice: *Ldlr*^{*-*/*-*} AMI,N + 25-HL group.
Fig. 18 shows the quantitative results of Oil Red O staining of liver sections of mice in four groups.
Fig. 19 shows the results of Sirius Red staining of liver sections of mice: C57BL/6J CD group.
Fig. 20 shows the results of Sirius Red staining of liver sections of mice: *Ldlr*^{*-*/*-*} CD group.
Fig. 21 shows the results of Sirius Red staining of liver sections of mice: *Ldlr*^{*-*/-} AMI,N group.
Fig. 22 shows the results of Sirius Red staining of liver sections of mice: *Ldlr*^{*-*/-} AMI,N + 25-HL group.
Fig. 23 shows the quantitative results of Sirius Red staining of liver sections of mice in four groups.
Fig. 24 shows the results of F4/80 immunostaining of liver sections of mice as well as the imaging results of polarized light imaging indicating cholesterol crystals: C57BL/6J CD group.
Fig. 25 shows the results of F4/80 immunostaining of liver sections of mice as well as the imaging results of polarized light imaging indicating cholesterol crystals: *Ldlr*^{*-*/*-*} CD group.
Fig. 26 shows the results of F4/80 immunostaining of liver sections of mice as well as the imaging results of polarized light imaging indicating cholesterol crystals: *Ldlr*^{*-*/*-*} AMI,N group.
Fig. 27 shows the results of F4/80 immunostaining of liver sections of mice as well as the imaging results of polarized light imaging indicating cholesterol crystals: *Ldlr^{-l-}* AMI,N + 25-HL group.
Fig. 28 shows the quantitative results of F4/80 immunostaining of liver sections of mice in four groups.
Fig. 29 shows the quantitative imaging results of polarized light imaging indicating cholesterol crystals of liver sections of mice in four groups.
Fig. 30 shows the results of Sudan IV staining of aortic trees of mice: C57BL/6J CD group.
Fig. 31 shows the results of Sudan IV staining of aortic trees of mice: *Ldlr*^{*-*/*-*} CD group.
Fig. 32 shows the results of Sudan IV staining of aortic trees of mice: *Ldlr*^{*-*/*-*} AMI,N group.
Fig. 33 shows the results of Sudan IV staining of aortic trees of mice: *Ldlr*^{*-*/*-*} AMI,N + 25-HL group.
Fig. 34 shows the quantitative results of Sudan IV staining of aortic trees of mice in four groups.
Fig. 35 shows the reduction of expression of lipogenesis-related genes (*Hmgcs, Hmgcr, SCD1,* and *FASN*) in liver organoids by 25-hydroxylanosterol.
Fig. 36 shows the reduction of expression of fibrosis-related genes (*Col1α1* and *αSMA*) in liver organoids by 25-hydroxylanosterol.
Fig. 37 shows the results of bright field imaging of liver organoids of mice: vehicle control group.
Fig. 38 shows the results of bright field imaging of liver organoids of mice: obeticholic acid 1 µM group.
Fig. 39 shows the results of bright field imaging of liver organoids of mice: obeticholic acid 3 µM group.
Fig. 40 shows the results of bright field imaging of liver organoids of mice: 25-HL 1 µM group.
Fig. 41 shows the results of bright field imaging of liver organoids of mice: 25-HL 3 µM group.
Fig. 42 shows the results of Nile Red staining of liver organoids of mice: vehicle control group.
Fig. 43 shows the results of Nile Red staining of liver organoids of mice: obeticholic acid 1 µM group.
Fig. 44 shows the results of Nile Red staining of liver organoids of mice: obeticholic acid 3 µM group.
Fig. 45 shows the results of Nile Red staining of liver organoids of mice: 25-HL 1 µM group.
Fig. 46 shows the results of Nile Red staining of liver organoids of mice: 25-HL 3 µM group.
Fig. 47 shows the quantitative results of Nile Red staining of liver organoids of mice in five groups.
Fig. 48 shows the results of fibrosis marker protein αSMA immunofluorescence staining of liver organoids of mice: vehicle control group.
Fig. 49 shows the results of fibrosis marker protein αSMA immunofluorescence staining of liver organoids of mice: obeticholic acid 1 µM group.
Fig. 50 shows the results of fibrosis marker protein αSMA immunofluorescence staining of liver organoids of mice: obeticholic acid 3 µM group.
Fig. 51 shows the results of fibrosis marker protein αSMA immunofluorescence staining of liver organoids of mice: 25-HL 1 µM group.
Fig. 52 shows the results of fibrosis marker protein αSMA immunofluorescence staining of liver organoids of mice: 25-HL 3 µM group.
Fig. 53 shows the results of fibrosis marker protein αSMA immunofluorescence staining of liver organoids of mice in five groups.
Fig. 54 shows the full 2D NMR spectrum of compound 151-7.
Fig. 55 shows the partially enlarged 2D NMR spectrum of compound 151-7.
Fig. 56 shows the full 2D NMR spectrum of compound 151.
Fig. 57 shows the partially enlarged 2D NMR spectrum of compound 151.
Fig. 58 shows the full 2D NMR spectrum of compound 203.
Fig. 59 shows the partially enlarged 2D NMR spectrum of compound 203.
Fig. 60 shows the full 2D NMR spectrum of compound 206.
Fig. 61 shows the partially enlarged 2D NMR spectrum of compound 206.

### DETAILED DESCRIPTION OF THE PREFERRED EMBODIMENT

The present disclosure is further described below by way of examples, but the present disclosure is not thereby limited to the scope of the described examples. The experimental methods for which the specific conditions are not specified in the following examples are selected according to the conventional methods and conditions, or according to the commodity instructions.

In the following examples, 25-hydroxylanosterol (25-HL) refers to the compound of example 68.

### Bioassay examples

**Animals:** Adult male C57BL/6J mice were purchased from Shanghai SLAC, and low-density lipoprotein receptor knockout mice (*Ldlr*^{*-*/*-*} mice, T001464) were purchased from Jiangsu GemPharmatech Co., Ltd. Mice were housed in an environment free of pathogens, maintained in a 12-hour light/dark cycle, and had free access to water and feed. The vehicle for oral gavage was 0.5% Tween-80, 0.5% methylcellulose, and 0.9% sodium chloride. AMLN feed (AMLN, Dyets) contains 40% (kcal%) fat (80% of which is trans fat), 22% (mass%) fructose, and 2% (mass%) cholesterol.

**Reagents:** Mevalonate (41288), paraformaldehyde (P6148), Tween-80 (P8074), methylcellulose (V900506), Oil Red O (O0625), and nuclear staining reagent DAPI were purchased from Sigma-Aldrich. Lovastatin (purity ≥ 98.5%, HPLC) was purchased from Shanghai Pharm Valley. Dulbecco's modified eagle medium (DMEM) for cell culture was purchased from Thermo Scientific, and fetal bovine serum (S1580) was purchased from Biowest. Lipoprotein-deficient serum (LPDS) was prepared in our laboratory by ultracentrifugation. Obeticholic acid (OCA, CAS No.: 459789-99-2, purity: 98% (HPLC)). Total cholesterol and total triglyceride kits were purchased from Shanghai Kehua Bio-Engineering Co., Ltd. Alanine aminotransferase (ALT) and aspartate aminotransferase (AST) kits were purchased from Lai Er Bio-Tech. Hematoxylin-eosin staining kits (6765001, 6766010) were purchased from Thermo Scientific. Sirius Red staining kit (ab150681) was purchased from Abcam. Sudan IV (A610914) was purchased from Sangon Biotech (Shanghai) Co., Ltd. Nile Red (HY-D0718) was purchased from MCE.

**Antibodies:** The antibodies used for immunofluorescence staining analysis are as follows: anti-alpha smooth muscle Actin (αSMA, ab7817, 1:500) antibody was purchased from Abcam; fluorescein isothiocyanate (FITC)-conjugated goat anti-mouse IgG (H+L) secondary antibody (115-095-003) was purchased from Jackson ImmunoResearch. Anti-F4/80 primary antibody (14-4801-85, Invitrogen, 1:100) and Alexa Fluor Plus 488-conjugated goat anti-rat IgG fluorescent secondary antibody (A-11006, 1:500) were purchased from Invitrogen.

### Bioassay example 1: Cell culture

Huh-7/SRE-Luc, a human hepatocellular carcinoma cell line, was grown in a culture medium containing DMEM, 10% fetal bovine serum, 100 units/mL penicillin, 100 µg/mL streptomycin, and 200 µg/mL G418.

### Bioassay example 2: SREBP luciferase reporter gene system

The Huh-7/SRE-Luc cell line is a human hepatocellular carcinoma cell line Huh-7 stably expressing LDLR promoter-luciferase and green fluorescent protein (GFP). The LDLR promoter region contains a sterol regulatory element (SRE) that can effectively and sensitively respond to the regulation of the transcription factor SREBP. The GFP signal serves as an internal reference to indicate changes in cell number. Therefore, this cell line can be used to screen for active small molecules that regulate the SREBP signaling pathway. In our work, this cell line was used to screen for inhibitors that were able to effectively inhibit the SREBP signaling pathway. Cells were incubated in a sterol-deficient medium (5% lipoprotein-deficient serum, 2 µM Lovastatin, 10 µM mevalonate) and treated with corresponding concentrations of compounds for 16 hours. After compound treatment, the cells were lysed with lysis buffer (E397A, Promega), followed by the addition of luciferase substrate (E1500, Promega). The SRE-driven luciferase activity was measured using a BioTek Synergy HTX microplate reader (including but not limited to this type of instrument). The fluorescence intensity of green fluorescent protein (EGFP) was also measured using the BioTek microplate reader (including but not limited to this type of instrument) and used as an internal reference. The ratio of SRE-driven luciferase activity to GFP fluorescence intensity was used as an indicator of SREBP pathway activity. The test data of each test compound was analyzed using Prism software to obtain the IC₅₀ parameter of the compound.

### Bioassay example 3: Real-time fluorescent quantitative PCR

Liver samples or liver organoid samples were homogenized in TRI Reagent (T9424, Sigma), and total RNA was extracted according to the manufacturer's instructions. Equal amounts of RNA template were used to synthesize cDNA with oligo dT primers and MLV reverse transcriptase (Promega). Quantitative data for genes were collected using the Bio-Rad CFX96 real-time PCR system, and the relative amount of gene mRNA was quantified using the comparative CT method. The primer sequences used are shown in the table below.

| **Species** | **Gene name** | **Forward/reverse primer sequences** |
|---|---|---|
| *Mus musculus* | *Gapdh* | AACTTTGGCATTGTGGAAGG |
| | | GGATGCAGGGATGATGTTCT |
| | *FASN* | GCTGCGGAAACTTCAGGAAAT |
| | | AGAGACGTGTCACTCCTGGACTT |
| | *Scd1* | CCGGAGACCCCTTAGATCGA |
| | | TAGCCTGTAAAAGATTTCTGCAAACC |
| | *Hmgcr* | CTTGTGGAATGCCTTGTGATTG |
| | | AGCCGAAGCAGCACATGAT |
| | *Hmgcs* | GCCGTGAACTGGGTCGAA |
| | | GCATATATAGCAATGTCTCCTGCAA |
| | *Col1α1* | GTCCCTGAAGTCAGCTGCATA |
| | | TGGGACAGTCCAGTTCTTCAT |
| | *αSMA* | TGCTGACAGAGGCACCACTGAA |
| | | CAGTTGTACGTCCAGAGGCATAG |

### Bioassay example 4: Measurement of serum and liver metabolic parameters

After drug treatment, mice were fasted for 4 hours before being euthanized, and blood and liver samples were collected. Blood was coagulated and centrifuged at 1,500 g for 10 minutes at 4°C to obtain the supernatant (*i.e*., serum). Lipids from liver were extracted using the chloroform/methanol method. The samples were first homogenized and crushed using a Precelly 24 homogenizer, and centrifuged at 16,000 g for 10 minutes at 4°C.The organic phase was transferred to a new tube, dried with nitrogen, and dissolved in ethanol. Total cholesterol and triglyceride levels in blood and liver were measured by cholesterol and triglyceride kits (Shanghai Kehua Bio-Engineering Co., Ltd.), respectively. ALT (LE-M0477, Lai Er Bio-Tech) and AST (LE-M0568, Lai Er Bio-Tech) levels in serum were measured according to the corresponding manufacturer's manuals, using an analytical system from Sysmex Shanghai Ltd..

### Bioassay example 5: Liver tissue section analysis

Hematoxylin-eosin staining: The extracted liver was fixed in 4% paraformaldehyde at 4°C, embedded in paraffin, sectioned using a paraffin microtome (Leica RM2235) with a section thickness of 7 µm, dewaxed, rehydrated, and stained with Hematoxylin-eosin staining kits (6765001, 6766010, Thermo Scientific). The images were captured with an Olympus VS 120 glass microscope, and quantified using ImageJ software. Oil Red O staining: The liver was embedded in OCT embedding medium (Leica), sectioned to a thickness of 7 µm using a cryostat microtome (Leica CM1950), stained with Oil Red O (O0625, Sigma). The images were captured with an Olympus VS 120 glass microscope, and quantified using ImageJ software. Sirius Red collagen staining: Liver paraffin sections were dewaxed, rehydrated, and stained with Sirius Red staining kit (ab150681, Abcam) according to the manufacturer's instructions. Immunofluorescence staining: The liver was embedded in OCT embedding medium (Leica) and sectioned to a thickness of 7 µm using a cryostat microtome (Leica CM1950). Cryostat sections were stained with anti-F4/80 rat monoclonal antibody (14-4801-85, Invitrogen, 1:100) and Alexa Fluor 488-conjugated goat anti-rat IgG secondary antibody (A-11006, Invitrogen, 1:500), and the nuclei were specifically stained with DAPI (Sigma) 30. After staining and sealing, the images were captured using a spinning disk confocal microscope (Nikon CSU-W1 SoRa), and quantified using ImageJ software. Polarized light imaging of cholesterol crystals: Cryostat sections were stained with F4/F80 and DAPI. The images were captured using a spinning disk confocal microscope (Nikon CSU-W1 SoRa) equipped with a polarized light filter, and quantified using ImageJ software.

### Bioassay example 6: Isolation of arterial tree and Sudan IV staining of atherosclerotic plaques in mice

After the administration experiment in mice, the aorta was isolated and fixed in 4% PFA. Perivascular adipose tissues were removed with ophthalmic forceps under a stereo microscope, stained with Sudan IV, and atherosclerotic plaques were washed in 70% ethanol. After staining, the aortic tree was imaged using a ZEISS Axio Zoom.V16 stereo microscope. Atherosclerotic lesions were quantified using ImageJ software.

### Bioassay example 7: Preparation and culture of liver organoids in mice

C57BL/6N mice were fed with fatty liver-inducing diet (TrophicDiet, TP2630052A, containing 10.2% kcal protein, 37.3% kcal carbohydrate, and 52.6% kcal fat) and fructose-containing drinking water (23.1 g of fructose and 18.9 g of dextrose were dissolved in 1 L of water, then filtered and sterilized) for a total of 16 weeks starting at 12 weeks of age to generate a fatty liver model. To generate fatty liver organoids, liver tissues from mice with fatty liver were minced and digested in digestion buffer at 37°C for 30 to 60 minutes. The digestion buffer included DMEM/F-12 (Cytiva, SH30023.01), 2.5 mg/mL collagenase D (Roche, COLLD-RO), and 0.1 mg/mL DNaseI (Sigma-Aldrich, DN25). Isolated single hepatocytes were filtered through a 70 µm filter membrane and washed once. Cells were collected by centrifugation, and resuspended in a mixture of culture medium and basement membrane extract (BME) (R&D Systems, 3533-010-02) at a ratio of 1:3. The culture medium included AdDMEM/F12, 10 mM Hepes, 1 × Glutamax, 1% pen/strep, 1 × B27, 1 × N2, NAC (1 mM), NIC (10 mM), Gastrin (10 nM), EGF (50 ng/mL), FGF10 (100 ng/mL), A83-01 (5 µM), Rki (10 µM), 10% RSPO1 conditioned medium, 30% Wnt3a conditioned medium, and 5% Noggin conditioned medium under culture conditions. Prior to administration, the organoids were digested with trypsin (Gibco, Cat# 25200072) and resuspended. After 24 hours, the organoids were given a medium change with culture medium containing different concentrations of OCA or 25-HL, using DMSO as a blank control. The fatty liver organoids were divided into 5 groups (1‰ DMSO, 1 µM OCA, 3 µM OCA, 1 µM 25-HL, and 3 µM 25-HL) and treated with drugs for 72 hours.

### Bioassay example 8: Histochemical staining of liver organoids in mice

Immunocytochemistry: The organoids were fixed in immunostaining fixative (Beyotime Biotechnology, P0098) at 4°C overnight. The organoids were then washed in PBS and treated with PBS containing 0.5% Triton X-100 at room temperature for 20 minutes. The organoids were then treated with PBS blocking solution containing 10% goat serum at room temperature for 1 hour and incubated with primary antibody (anti-alpha smooth muscle Actin, ab7817, Abcam, diluted 1:500) at 4°C overnight. The following day, the organoids were washed and co-incubated with fluorescein isothiocyanate (FITC)-conjugated goat anti-mouse IgG (H+L) secondary antibody (Jackson ImmunoResearch, 115-095-003). The nuclei were counterstained with Fluoroshield^{™} with DAPI (Sigma-Aldrich, F6057).

To observe lipid droplets, the organoids were fixed in 4% paraformaldehyde (PFA) for 1 hour and stained with 250 nmol/L Nile Red (MCE, HY-D0718) at room temperature for 3 minutes. The organoids were rinsed twice with PBS before imaging. Stained organoids were observed under the Dragonfly high-speed confocal microscope system (Andor, Dragonfly 200).

### Bioassay example 9: Inhibitory effect of compounds of the present disclosure on SREBP pathway

The inhibitory effect of the compounds of the present disclosure on the SREBP pathway was tested by the method of bioassay example 2. The concentration gradient of each compound was designed as 0.01, 0.03, 0.1, 0.3, 1.0, 3.0, and 10 µM, with DMSO as a vehicle control. The IC₅₀ values of some of the compounds are shown in Table 1.

**Table 1: Activity data of compounds in some examples**

| **Compound No.** | **Structural formula** | **SRE-luciferase assay (IC₅₀ µM)** |
|---|---|---|
| **68** | 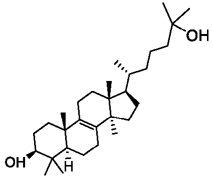 | 0.293 |
| **185** | 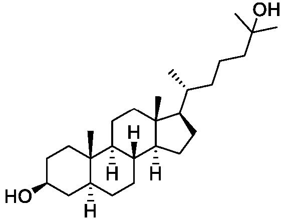 | 0.233 |
| **198** | 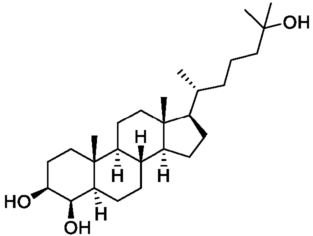 | 0.106 |
| **204** | 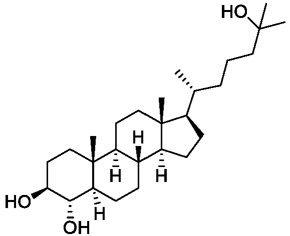 | 0.395 |
| **205** | 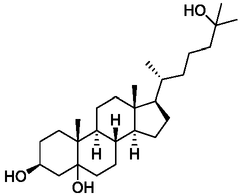 | 0.817 |
| **206** | 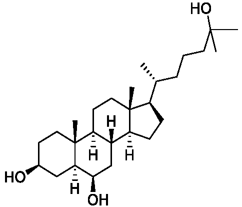 | 0.464 |
| **207** | 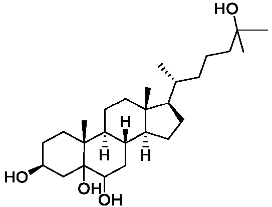 | 3.088 |
| **208** | 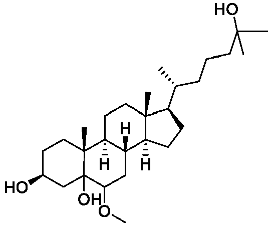 | 1.925 |
| **210** | 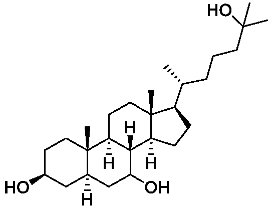 | 0.837 |
| **211** | 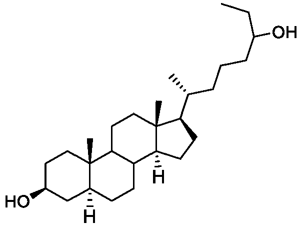 | 0.395 |
| **216** | 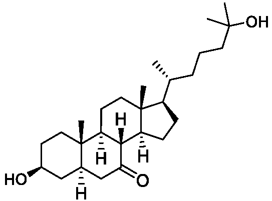 | 0.379 |
| **217** | 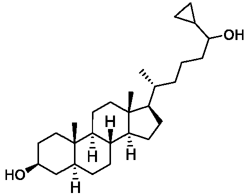 | 0.329 |
| **218** | 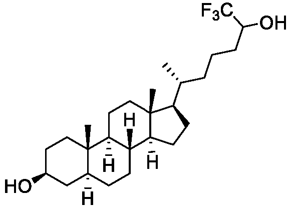 | 0.296 |
| **224** | 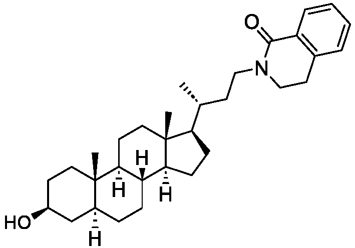 | >10 |
| **225** | 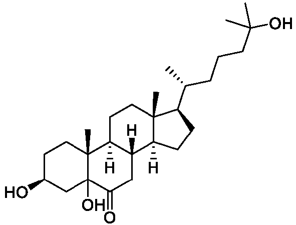 | 0.667 |
| **226** | 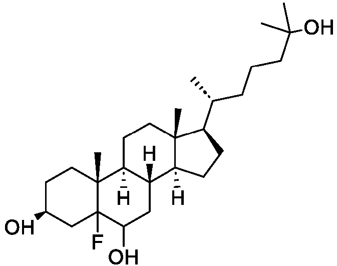 | 46.64 |
| **227** | 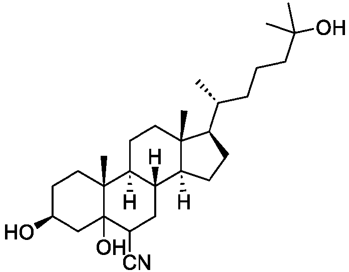 | 1.33 |
| **231** | 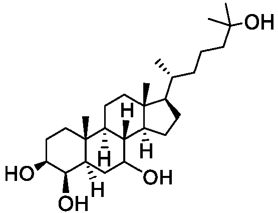 | 1.026 |
| **232** | 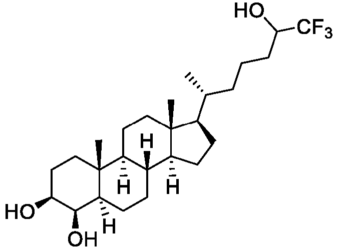 | 0.084 |
| **233** | 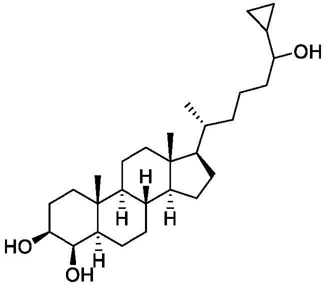 | 0.2317 |
| **234** | 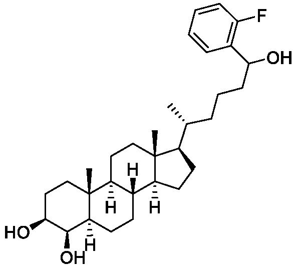 | 0.3598 |
| **235** | 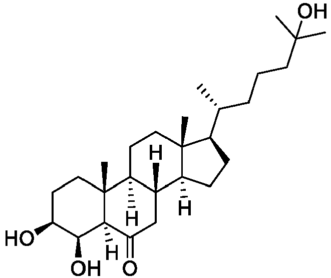 | 10.76 |
| **237** | 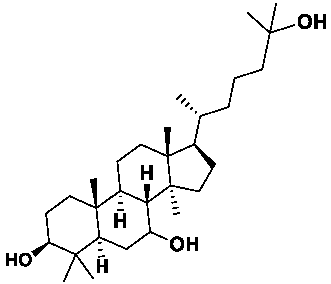 | 7.045 |
| **238** | 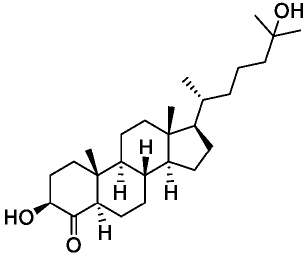 | 0.049 |
| **239** | 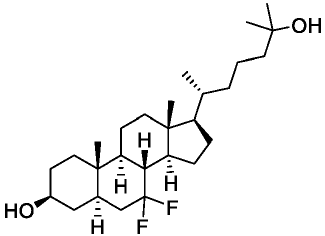 | 0.106 |
| **240** | 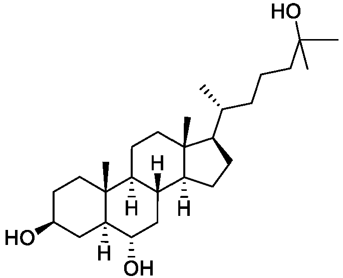 | 0.67 |
| **241** | 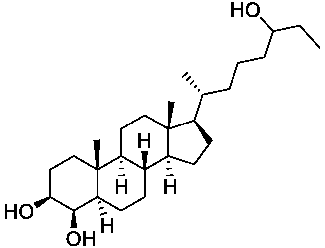 | 0.328 |
| **242** | 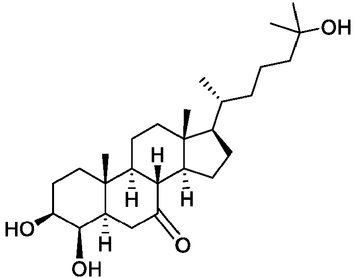 | 0.556 |
| **244** | 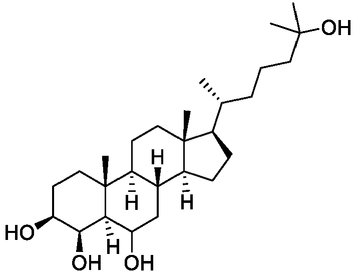 | 3.761 |
| **245** | 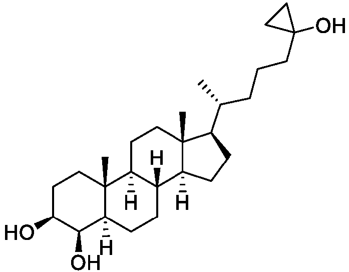 | 0.192 |
| **246** | 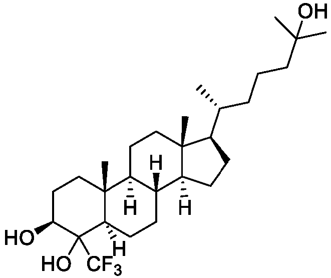 | 0.9878 |
| **248** | 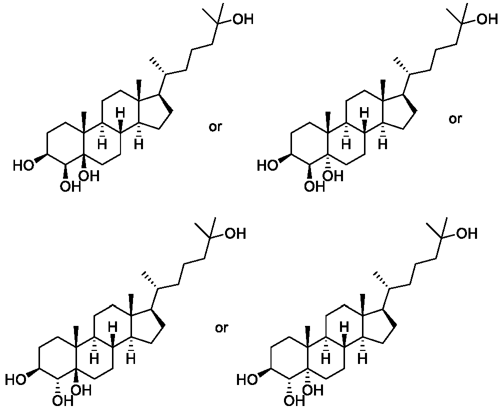 | 0.7655 |
| **249** | 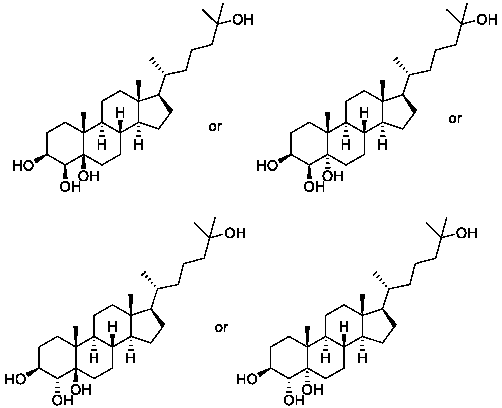 | 0.7039 |
| **250** | 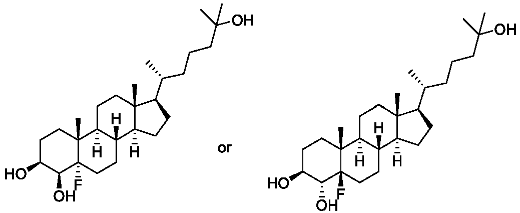 | 0.245 |
| **251** | 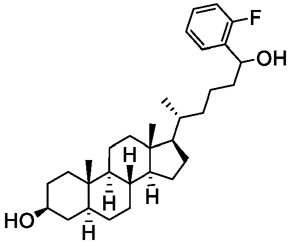 | 0.3205 |
| **253** | 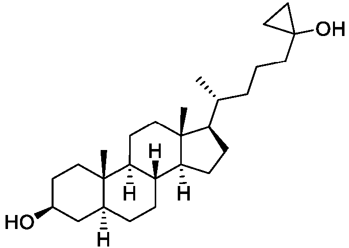 | 0.1873 |
| **254** | 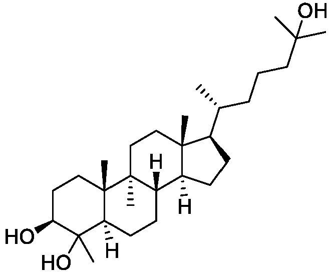 | 6.19 |
| **255** | 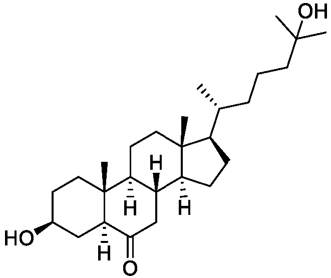 | 0.452 |
| **266** | 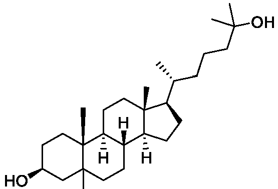 | 0.1755 |
| **268** | 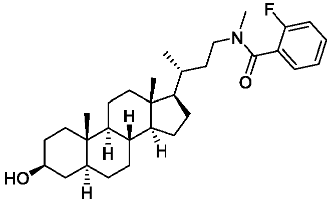 | 0.825 |
| **271** | 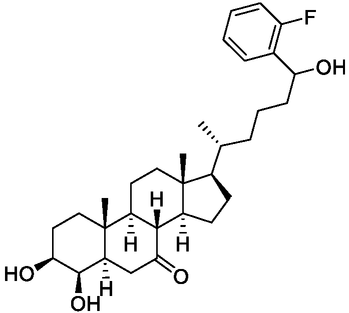 | 0.139 |
| **272** | 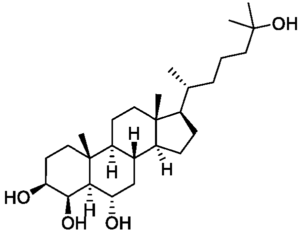 | 20.14 |
| **273** | 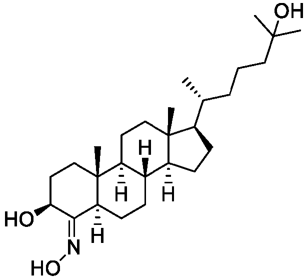 | 0.279 |
| **274** | 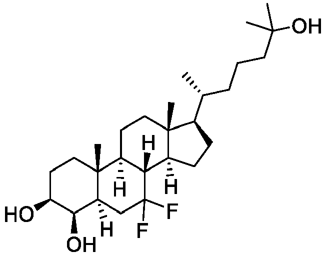 | 0.313 |
| **275** | 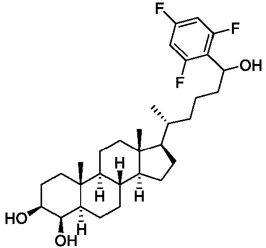 | 0.141 |
| **276** | 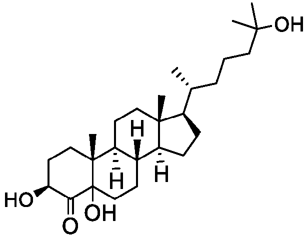 | 0.5219 |
| **278** | 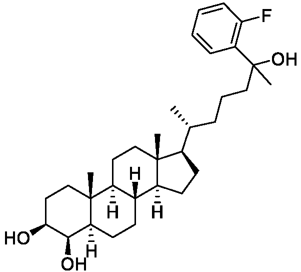 | 0.1063 |
| **279** | 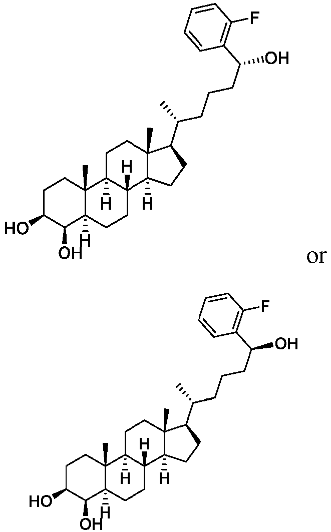 | 0.1956 |
| **280** | 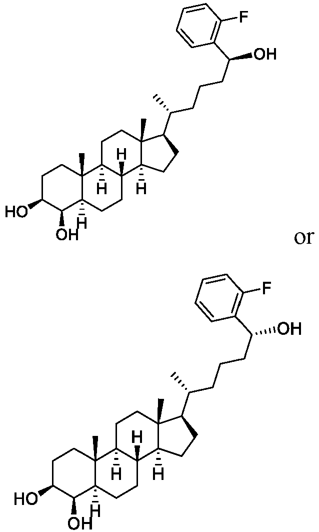 | 0.1176 |
| **281** | 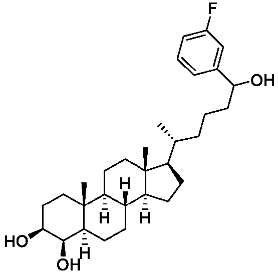 | 0.111 |
| **282** | 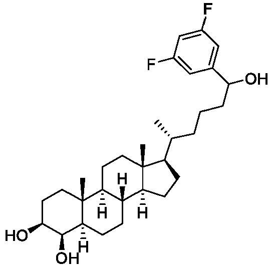 | 0.1165 |
| **284** | 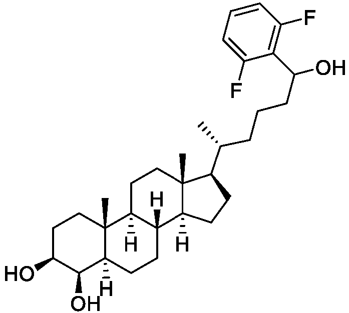 | 0.0874 |
| **285** | 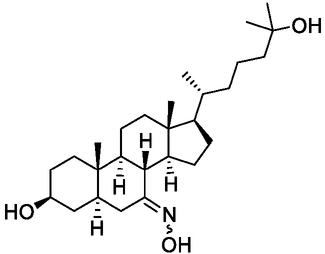 | 0.793 |
| **286** | 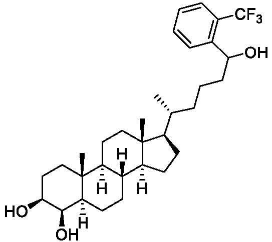 | 0.5752 |
| **287** | 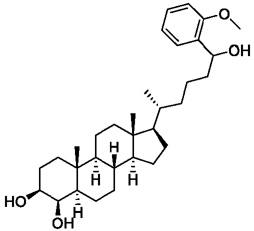 | 0.159 |
| **288** | 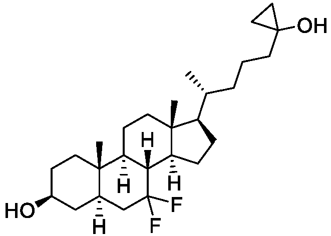 | 0.308 |
| **289** | 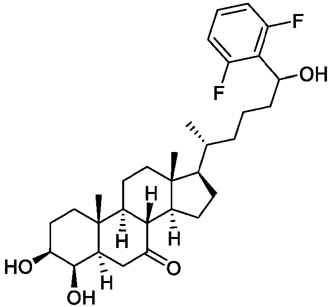 | 0.117 |
| **290** | 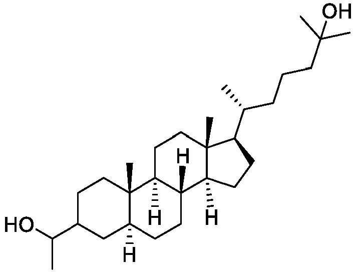 | 0.699 |
| **291** | 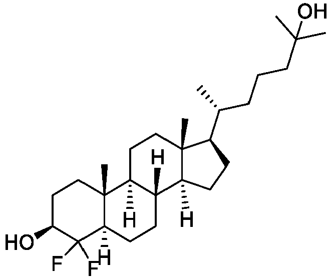 | 0.065 |
| **292** | 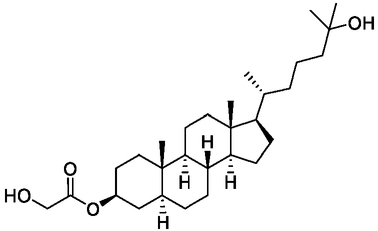 | 0.171 |
| **293** | 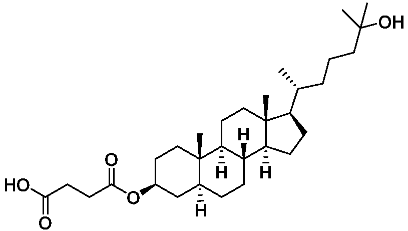 | 0.136 |
| **294** | 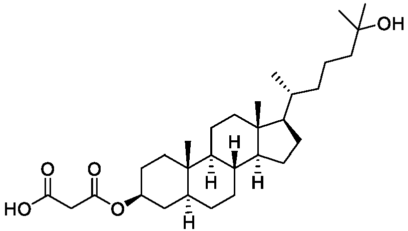 | 0.139 |
| **295** | 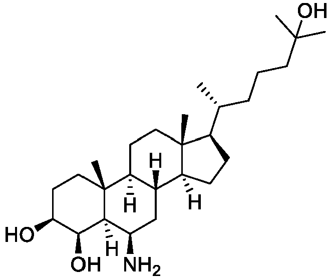 | >10 |
| **296** | 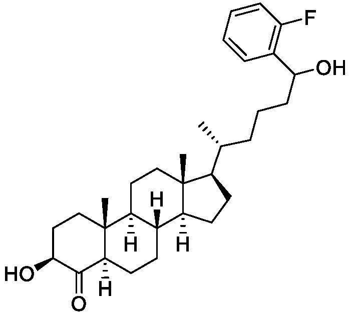 | 0.035 |
| **298** | 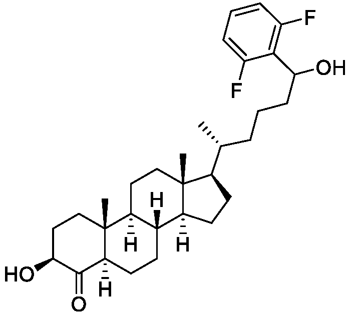 | 0.028 |
| **299** | 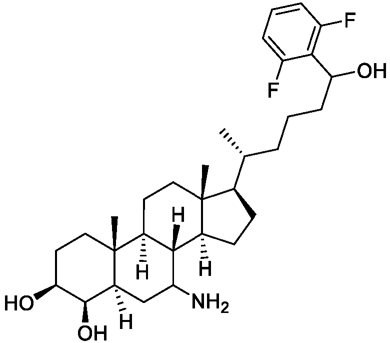 | 4.83 |
| **300** | 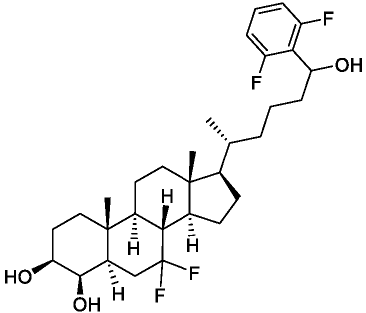 | 0.036 |
| **301** | 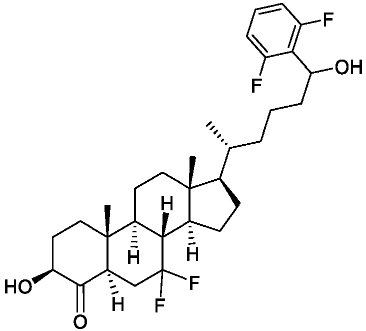 | 0.040 |
| **302** | 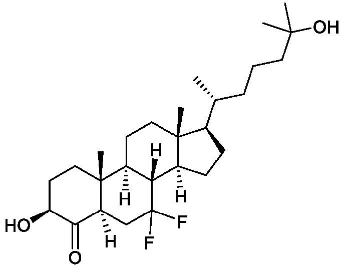 | 0.035 |
| **303** | 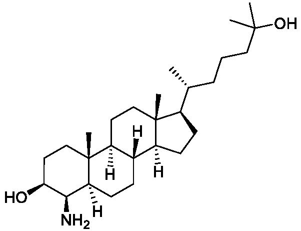 | 7.083 |
| **304** | 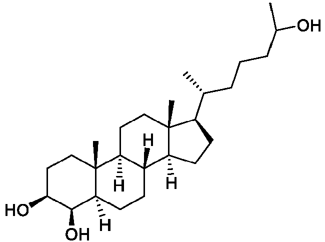 | 0.173 |
| **305** | 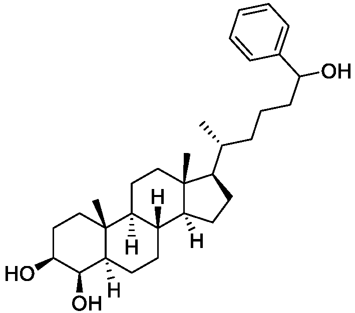 | 0.119 |
| **306** | 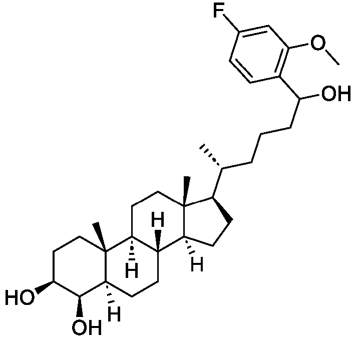 | 0.08 |
| **307** | 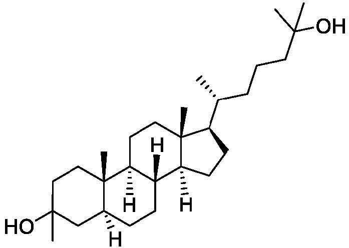 | 0.588 |
| **308** | 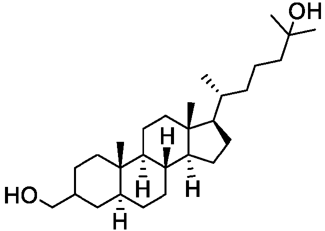 | 0.979 |
| **309** | 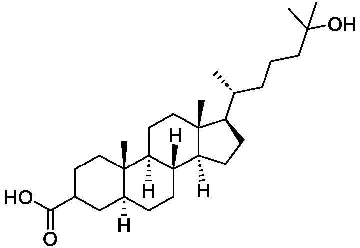 | 8.96 |
| **310** | 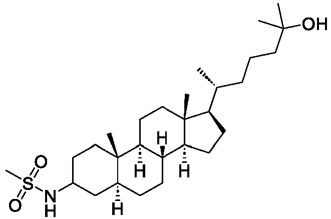 | 1.41 |
| **312** | 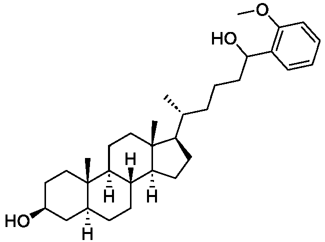 | 0.18 |
| **313** | 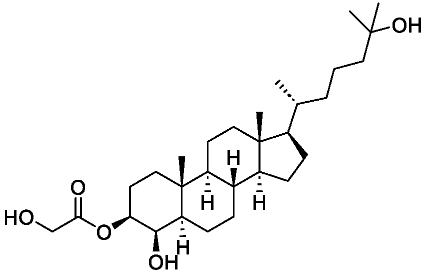 | 0.07 |
| **316** | 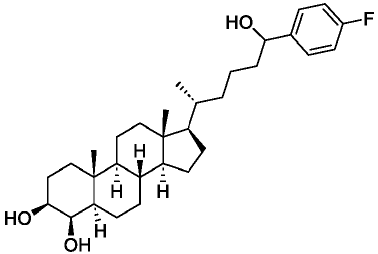 | 0.10 |
| **317** | 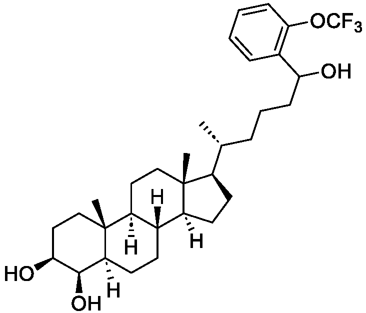 | 0.14 |
| **320** | 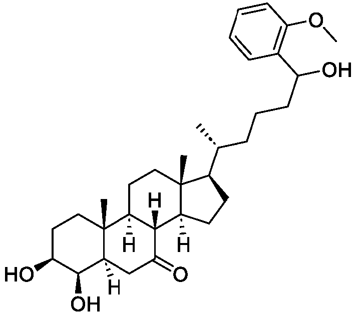 | 0.16 |
| **321** | 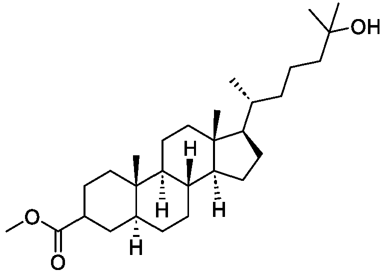 | 7.20 |
| **322** | 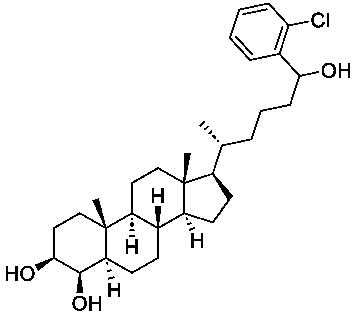 | 0.61 |
| **323** | 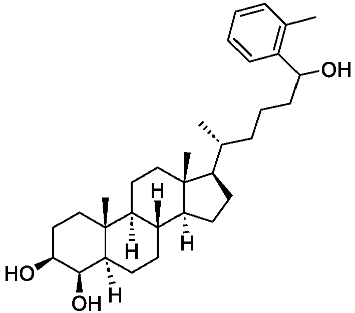 | 0.25 |
| **324** | 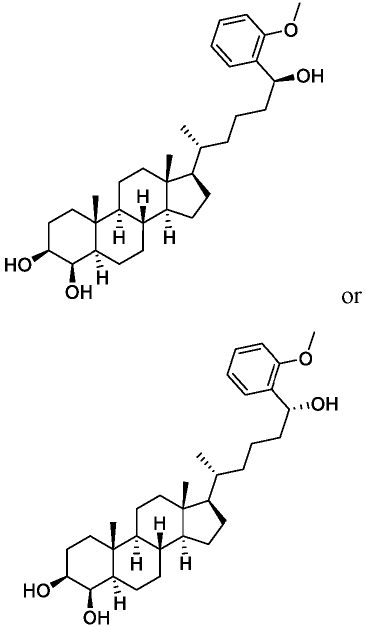 | 0.36 |
| **325** | 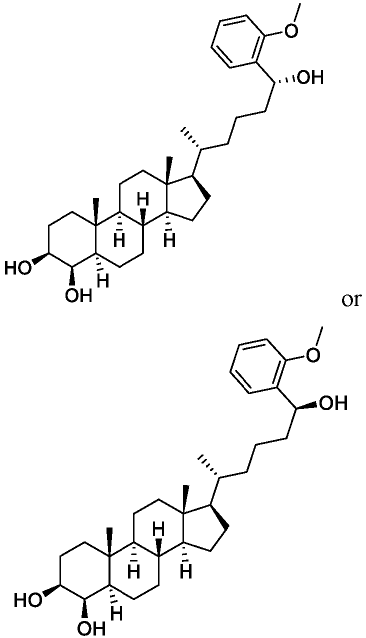 | 0.09 |

### Bioassay example 10: Reduction of blood lipid levels as well as alleviation of fatty liver and liver damage by 25-hydroxylanosterol

As elevated blood lipid levels and hepatic lipid accumulation are known to be high-risk factors for fatty liver, the present disclosure further analyzed whether 25-hydroxylanosterol could alleviate the typical symptoms of diet-induced fatty liver in mice: lipid accumulation, liver damage, inflammation, and fibrosis.

Male C57BL/6J mice and male *Ldlr*^{-/-} mice (T001464, Jiangsu GemPharmatech Co., Ltd.) at 8 weeks of age were purchased, grouped, fed with different diets, and treated with different drugs. The mice were randomly divided into 4 groups with 8 to 9 mice in each group: One group consisted of C57BL/6J wild-type mice, which was the chow diet (CD) vehicle control group. The other three groups consisted of *Ldlr*^{-/-} knockout mice, including chow diet (CD) vehicle control group, AMLN diet (containing 20% fat, 22% fructose, and 2% cholesterol) vehicle control group, and AMLN diet combined with 25-hydroxylanosterol administration group (25-hydroxylanosterol was administered at a concentration of 30 mg/kg/day). The mice in four groups were intragastrically administered once a day, during which the changes in food intake and body weight of the mice in different treatment groups were counted. After 8 weeks, blood and liver samples were collected from the mice to analyze blood lipids, liver lipids, and liver damage phenotypes.

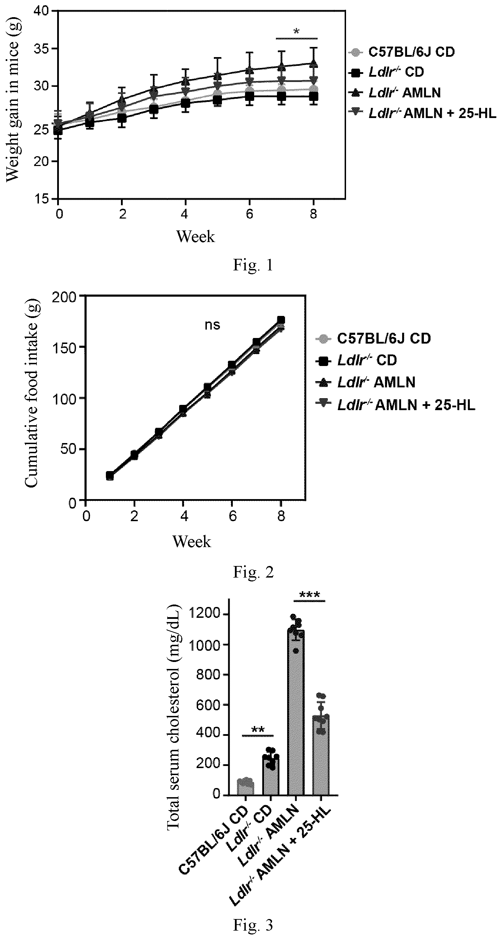

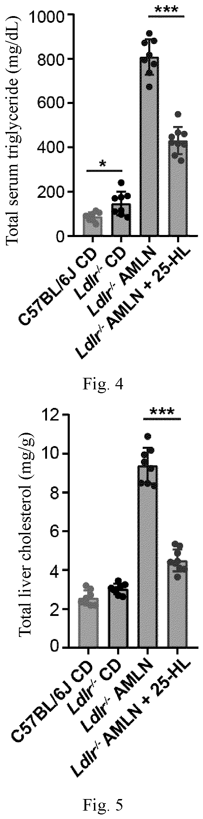

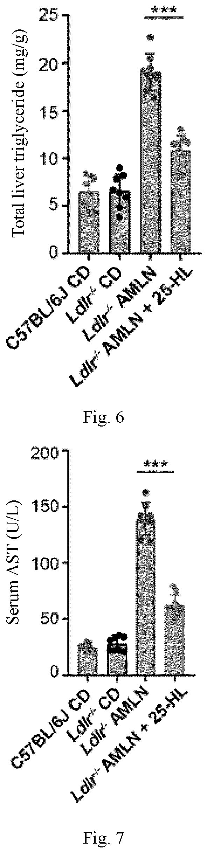

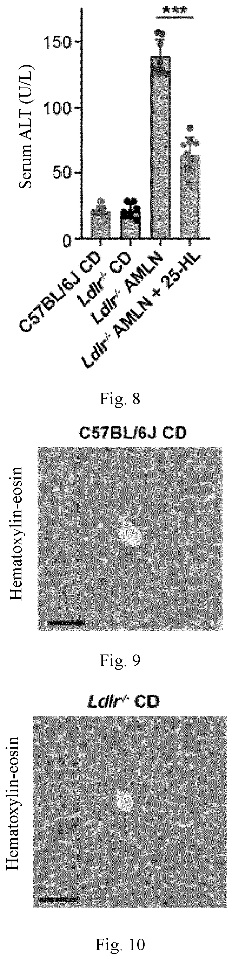

The results are shown in Figs. 1 to 8. Fig. 1 shows the statistical results of weekly weighing of mice in each group. Fig. 2 shows the cumulative statistics of the food intake of mice in each group. Fig. 3 shows the reduction of total blood cholesterol levels in mice by 25-hydroxylanosterol. Fig. 4 shows the reduction of total blood triglyceride levels in mice by 25-hydroxylanosterol. Fig. 5 shows the reduction of total liver cholesterol levels in mice by 25-hydroxylanosterol. Fig. 6 shows the reduction of total liver triglyceride levels in mice by 25-hydroxylanosterol. Fig. 7 shows the reduction of blood aspartate aminotransferase (AST) levels in mice by 25-hydroxylanosterol. Fig. 8 shows the reduction of blood alanine aminotransferase (ALT) levels in mice by 25-hydroxylanosterol.

In Figs. 1 and 2, P values were calculated using the statistical analysis of two-way ANOVA (Dunnett's multiple comparisons test); * indicates P < 0.05; ns indicates no statistical difference. In Figs. 3 to 8, data are represented as mean ± standard deviation. P values were calculated using one-way ANOVA; * indicates P < 0.05; ** indicates P < 0.01; *** indicates P < 0.001.

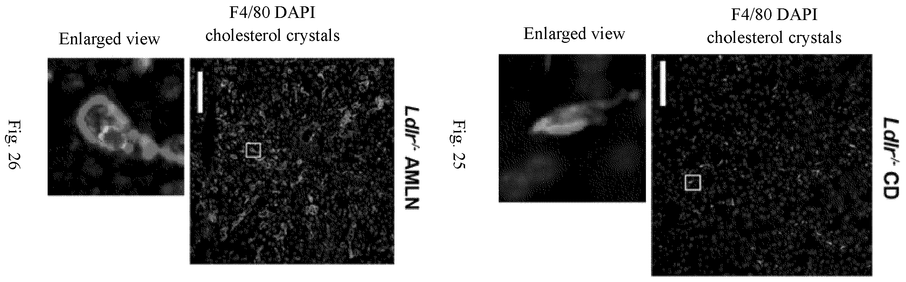

The results showed that for *Ldlr*^{-/-} mice, the AMLN diet combined with 25-hydroxylanosterol administration group had significantly lower weight gain compared to the AMLN diet vehicle control group after 8 weeks of continuous administration. It indicates that 25-hydroxylanosterol has a good inhibitory effect on AMLN diet-induced weight gain. The pathological characteristics of fatty liver disease mainly include liver steatosis, liver damage, inflammatory infiltration, and fibrosis. After 8 weeks of administration to the mice in four groups, we first tested the mice for the changes in lipid levels such as total blood cholesterol levels and total blood triglyceride levels as well as the changes in total liver cholesterol levels and total liver triglyceride levels. As shown in Figs. 3 to 4, 25-hydroxylanosterol significantly reduced total cholesterol levels and total triglyceride levels in serum compared to the control group. Meanwhile, as shown in Figs. 5 to 6, 25-hydroxylanosterol significantly reduced total liver cholesterol levels and total liver triglyceride levels in mice. It indicates that 25-hydroxylanosterol has a good effect on reducing blood lipids and liver lipids. As can be seen from Figs. 7 to 8, aspartate aminotransferase (AST) and alanine aminotransferase (ALT), two liver damage markers in serum, were significantly reduced by 25-hydroxylanosterol. It indicates that 25-hydroxylanosterol has a good effect on ameliorating liver damage.

### Bioassay example 11: Alleviation of fatty liver and atherosclerosis by 25-hydroxylanosterol

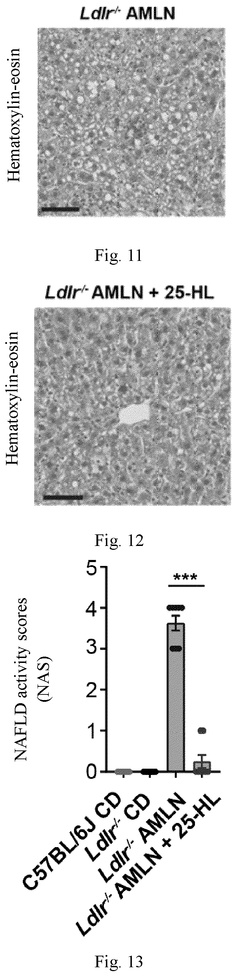

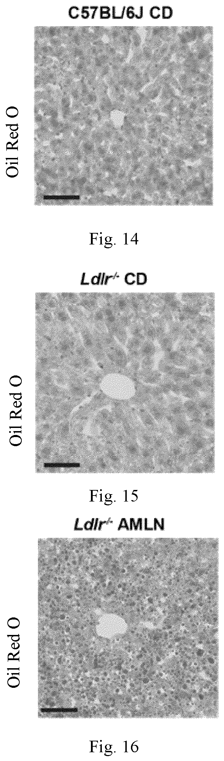

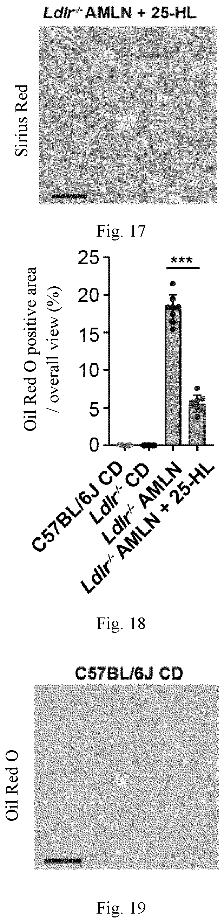

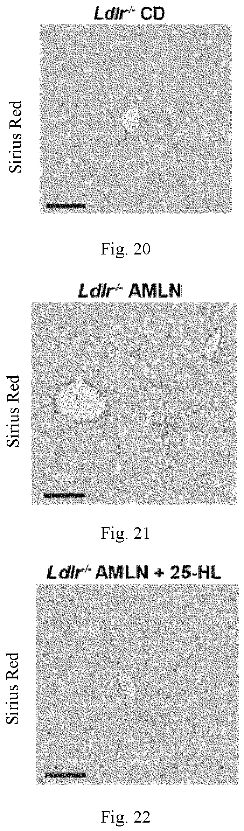

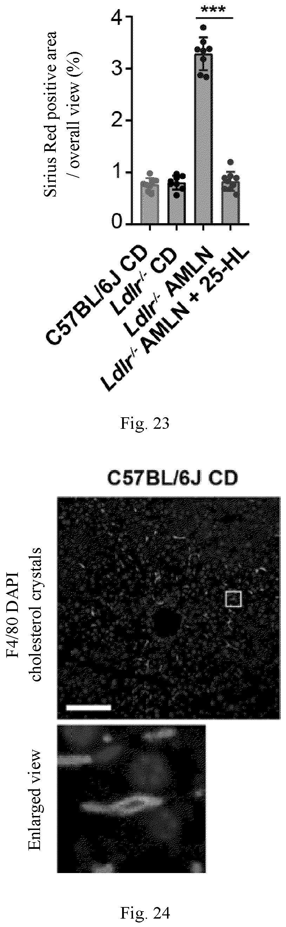

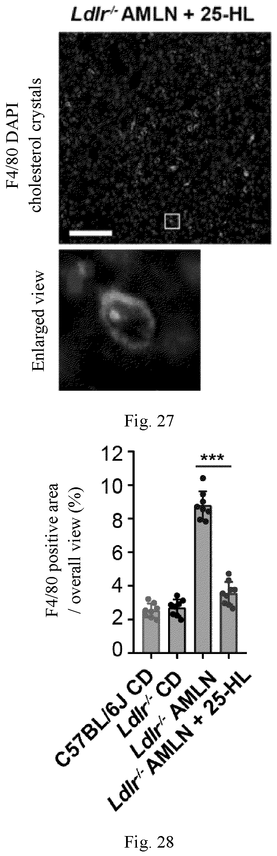

Furthermore, we performed various staining or immunohistochemical procedures on liver tissue sections from the mice in each group to analyze the phenotypic changes in hepatic lipid accumulation and fatty liver. The results are shown in Figs. 9 to 29. Figs. 9 to 13 show the results of HE staining of liver sections of mice and quantitative results of NAFLD activity scores: 25-hydroxylanosterol reduced liver steatosis and significantly reduced NAFLD activity scores. Figs. 14 to 18 show the results of Oil Red O staining and quantification of liver sections of mice: 25-hydroxylanosterol reduced lipid droplets in the liver. Figs. 19 to 23 show the results of Sirius Red staining and quantification of liver sections of mice: 25-hydroxylanosterol reduced fibrosis in the liver. Figs. 24 to 29 show the results of F4/80 immunostaining and quantification of liver sections of mice as well as the results of imaging and quantification of polarized light imaging indicating cholesterol crystals: 25-hydroxylanosterol reduced the aggregation of Kupffer cells and formation of cholesterol crystals in the liver.

In Figs. 9 to 29, quantitative analysis was performed using ImageJ software, and data are represented as mean ± standard deviation. P values were calculated using one-way ANOVA; * indicates P < 0.05; ** indicates P < 0.01; *** indicates P < 0.001.

As shown in the results of HE staining in Figs. 9 to 13, male *Ldlr*^{-/-} mice fed with AMLN diet for 8 weeks exhibited significant macrovesicular lipid droplets and vesicular degenerated hepatocytes in the liver. 25-hydroxylanosterol significantly reduced these phenotypes compared to the control group. Concurrently, as shown in the results of Oil Red O staining of liver tissue sections in Figs. 14 to 18, 25-hydroxylanosterol significantly reduced the accumulation of lipid droplets of neutral lipids including cholesterol and fatty acids in the liver compared to the control group. These results are consistent with the results of 25-hydroxylanosterol reducing hepatic lipid content in Figs. 1 to 8, indicating that 25-hydroxylanosterol is effective in improving the AMLN diet-induced hepatic lipid accumulation phenotype. In addition, the present disclosure further analyzed the changes in inflammatory and fibrotic phenotypes associated with fatty liver in the liver. As shown in the results of immunofluorescence staining in Figs. 24 to 29, for *Ldlr*^{-/-}mice in the AMLN diet vehicle control group, F4/80-specific staining showed the aggregation of Kupffer cells and formation of a crown-like structure around cholesterol crystals (enlarged view in Figs. 24 to 27). 25-hydroxylanosterol significantly reduced the crown-like structure formed by Kupffer cells, indicating that 25-hydroxylanosterol can significantly reduce the inflammatory infiltration in the liver. Polarized light imaging of cholesterol crystals in liver sections showed numerous cholesterol crystals in the crown-like structure formed by Kupffer cells in vehicle control mice. These cholesterol crystals, along with the crown-like structure formed by Kupffer cells, are characteristic features of fatty liver compared to simple steatosis. Macrophages are attracted to cholesterol crystals and attempt to clear these residual lipid droplets, similar to the phenomenon described in atherosclerosis. More importantly, 25-hydroxylanosterol significantly reduced the number of crown-like structures and cholesterol crystals (Figs. 24 to 29). Concurrently, as shown in the results of Sirius Red in Figs. 19 to 23, 25-hydroxylanosterol significantly reduced the fibrotic phenotype of collagen fiber formation in liver tissue sections of mice. The above results suggest that 25-hydroxylanosterol alleviates hepatic lipid accumulation and symptoms of fatty liver, and can be used to prevent and/or treat diseases such as hyperlipidemia and fatty liver.

AMLN-fed *Ldlr*^{-/-} mice also served as a common model for atherosclerosis, allowing simultaneous study of both fatty liver and atherosclerosis. Male *Ldlr*^{-/-} mice at 8 weeks of age were fed with AMLN diet and intragastrically administered once a day for a total of 8 weeks. After the mice were euthanized, the aorta was isolated and fixed in 4% paraformaldehyde. Perivascular adipose tissues were removed under a stereo microscope, stained with Sudan IV, and rinsed with 70% ethanol. The aortic tree was imaged using a stereo microscope (Axio Zoom.V16, ZEISS, Germany). Atherosclerotic plaques were quantified using Imaged software.

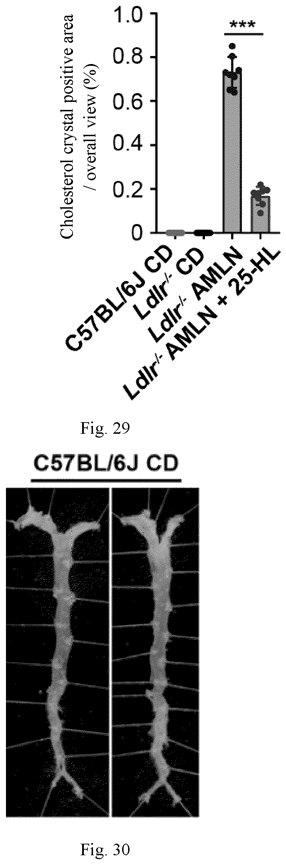

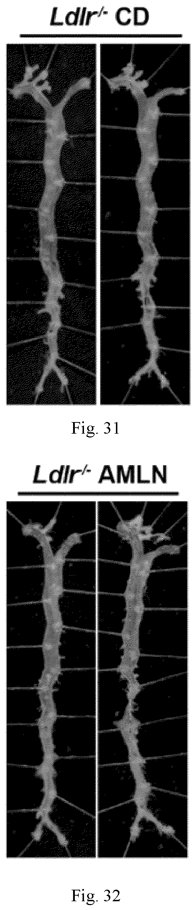

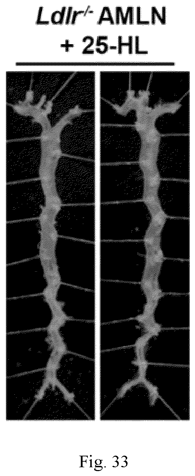

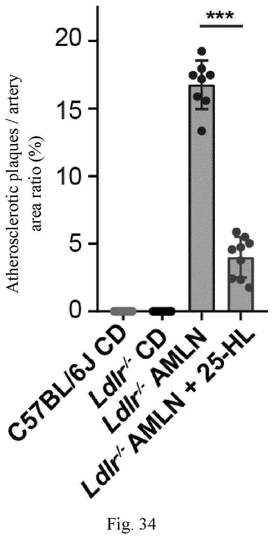

The results are shown in Figs. 30 to 34, which show the results of Sudan IV staining and quantification of aortic trees of mice: 25-hydroxylanosterol reduced the formation of atherosclerotic plaques.

The results showed that after 8 weeks of continuous administration, the aortic trees of mice in each group were isolated, and the aorta was subjected to Sudan IV lipid-specific staining. As shown in Figs. 30 to 34, 25-hydroxylanosterol significantly reduced the formation and quantity of atherosclerotic plaques in mice compared to the control group. 25-hydroxylanosterol demonstrates an alleviating effect on the formation of atherosclerosis.

Data from Figs. 1 to 8 and 9 to 34 showed that 25-hydroxylanosterol lowered elevated blood lipid levels, alleviated hepatic lipid accumulation, cholesterol crystals, hepatocyte damage, inflammatory infiltration, and fibrosis, and reduced the formation and quantity of atherosclerotic plaques. It indicates that 25-hydroxylanosterol has a good therapeutic effect on hyperlipidemia, fatty liver, and atherosclerosis.

### Bioassay example 12: Demonstration of the inhibitory effect of 25-hydroxylanosterol on lipogenesis and fibrosis in fatty liver using in vitro liver organoids

To distinguish whether the inhibitory effect of 25-hydroxylanosterol is directly based on its effect on the liver or a systemic effect, we utilized 3D liver organoids as an *in vitro* model for the study of fatty liver to analyze the effect of 25-hydroxylanosterol on lipogenesis and fibrosis marker expression in liver organoids. C57BL/6N mice were fed with fatty liver-inducing diet (TrophicDiet, TP2630052A, containing 10.2% kcal protein, 37.3% kcal carbohydrate, and 52.6% kcal fat) and fructose-containing drinking water (23.1 g of fructose and 18.9 g of dextrose were dissolved in 1 L of water, then filtered and sterilized) for a total of 16 weeks starting at 12 weeks of age to generate a fatty liver model. Liver organoids were isolated from the liver organ of the murine model and prepared for *in vitro* culture. Meanwhile, the organoids were also subjected to *in vitro* drug treatment with obeticholic acid (OCA), an agonist of farnesoid X receptor (FXR), which has demonstrated a favorable anti-fatty liver effect in phase III clinical trials, and which was used as a control drug in this example. The organoids were grouped and treated with the same concentration gradient of obeticholic acid and 25-hydroxylanosterol for 3 days. RNA from the organoids was collected and analyzed for the differential expression of lipogenesis-related genes (*Hmgcs*, *Hmgcr*, *SCD1*, and *FASN*) and fibrosis markers (*αSMA* and *Col1α1*) using fluorescent real-time quantitative PCR.

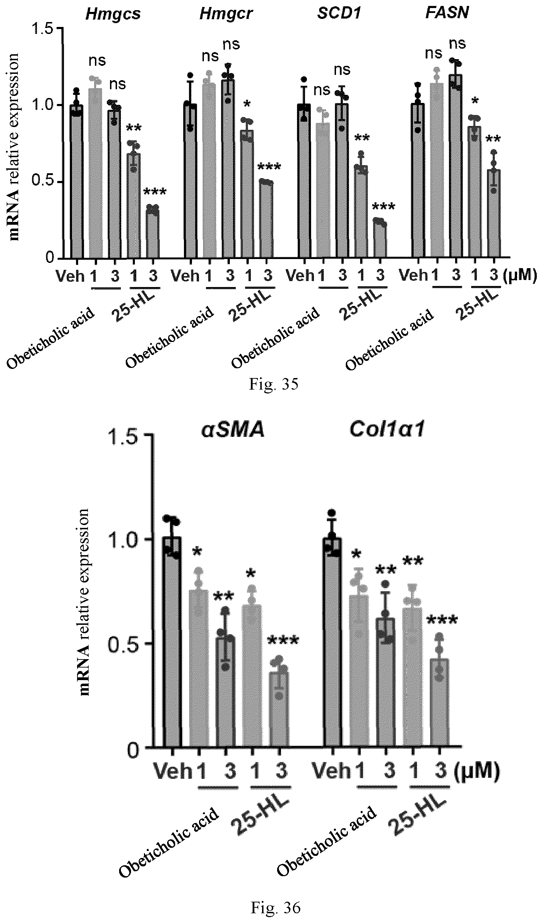

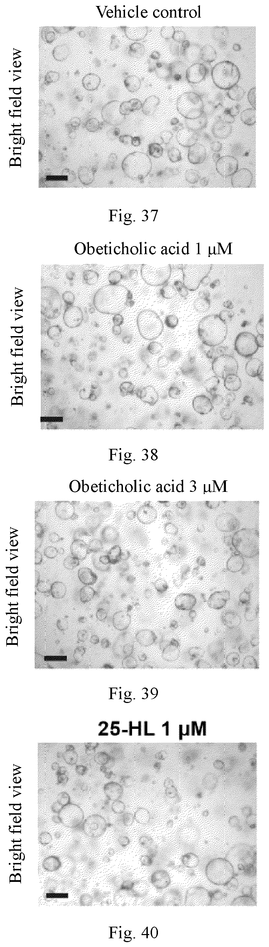

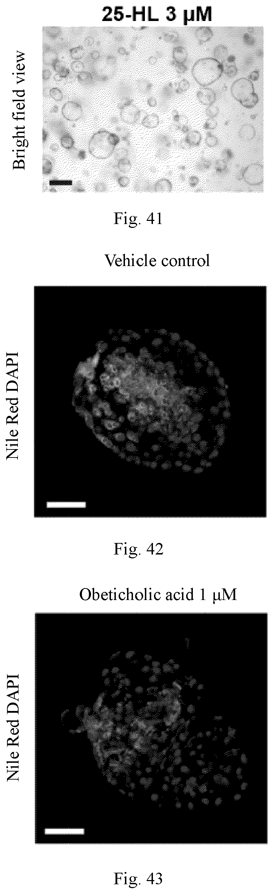

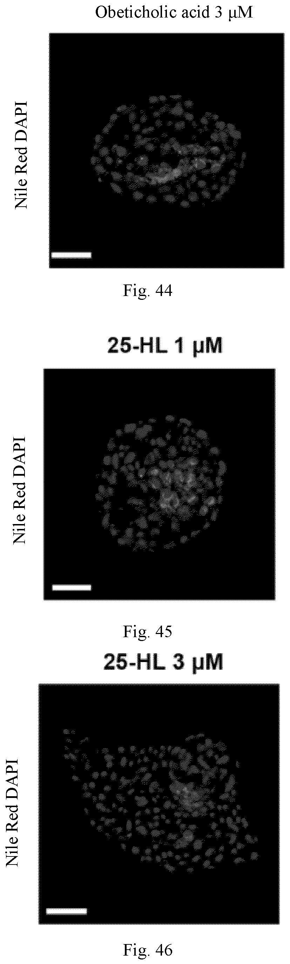

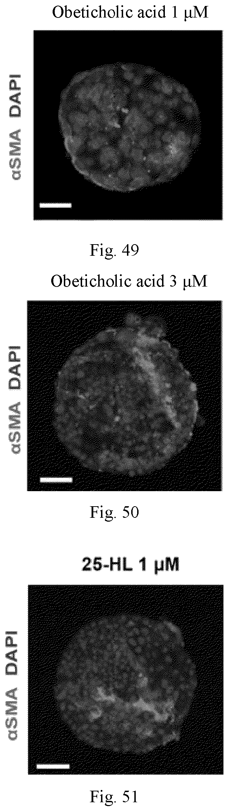

The results are shown in Figs. 35 to 53. Fig. 35 shows the reduction of expression of lipogenesis-related genes (*Hmgcs*, *Hmgcr*, *SCD1*, and *FASN*) in liver organoids by 25-hydroxylanosterol, with a better effect compared to the control drug obeticholic acid at the same concentration. Fig. 36 shows the reduction of expression of fibrosis-related genes (*Col1α1* and *αSMA*) in liver organoids by 25-hydroxylanosterol, with a better effect compared to the control drug obeticholic acid at the same concentration. Figs. 37 to 53 show the results of bright field imaging, the results of Nile Red staining and quantification, and the results of fibrosis marker protein αSMA immunofluorescence staining and quantification of liver organoids: 25-hydroxylanosterol reduced lipid accumulation in liver organoids while decreasing the expression of fibrosis marker protein αSMA in liver organoids.

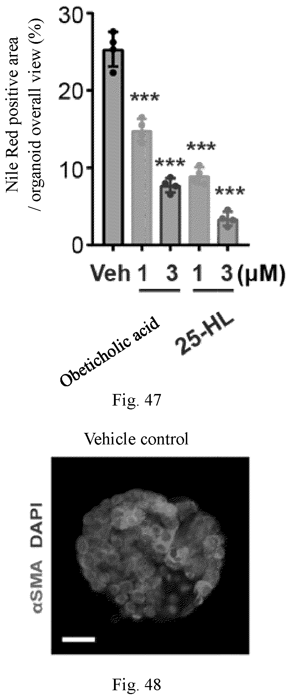

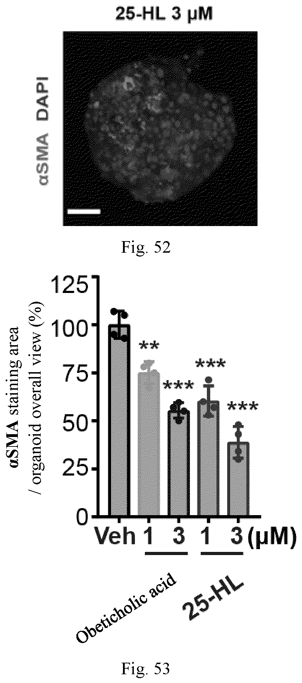

In Figs. 35 and 36, data are represented as mean ± standard deviation. P values were calculated using one-way ANOVA; ns indicates no statistical difference; * indicates P < 0.05; ** indicates P < 0.01; *** indicates P < 0.001. In Figs. 47 and 53, quantitative analysis was performed using Imaged software. Data are represented as mean ± standard deviation. P values were calculated using one-way ANOVA; * indicates P < 0.05; ** indicates P < 0.01; *** indicates P < 0.001.

The results showed that 25-hydroxylanosterol significantly inhibited the expression levels of both lipogenesis-related genes (Fig. 35) and fibrosis-related genes (Fig. 36) compared to obeticholic acid, which failed to inhibit the expression of lipogenesis-related genes. Meanwhile, Nile Red staining experiments were performed to detect neutral lipid accumulation in organoids. As shown in Figs. 37 to 53, both 25-hydroxylanosterol and OCA reduced lipid accumulation in organoids. The staining data of fibrosis marker protein αSMA showed a significant decrease in its expression. The results in Figs. 35 to 53 illustrate that 25-hydroxylanosterol directly reduces hepatic lipid accumulation and fibrosis by inhibiting the expression of lipogenesis- and fibrosis-related genes, while OCA may indirectly inhibit hepatic lipid accumulation by promoting lipid oxidation. These data demonstrate that 25-hydroxylanosterol directly targets the regulation of hepatic lipid synthesis pathways and fibrosis-related genes.

### Effect example 13: Liver microsomal metabolic stability assay

PBS solution (100 mM), MgCl₂ solution (100 mM), and NADPH solution (20 mM) were prepared respectively. Compound and testosterone (positive control) stock solutions were then prepared with dimethyl sulfoxide (DMSO), diluted to 100 µM with methanol for sample incubation, and stored at -10 to -30°C. 12.5 µL of rat liver microsomes (purchased from XenoTech, Cat. No.: R1000, Lot No.: 1310030, specification: 20 mg/mL), 432.5 µL of PBS solution (100 mM), 25 µL of NADPH solution (20 mM), and 25 µL of MgCl₂ solution (100 mM) were added to a 96-well plate, mixed well, and pre-incubated at 37°C for 5 minutes. 5 µL of substrate (test compound) solution was added to start the reaction. At specified time points of 0, 5, 15, 30, 45, and 60 minutes (0 and 60 minutes for the negative control group), 50 µL of the incubated samples were added to a stop plate containing 100 µL of ice-cold stop solution, vortexed for 1 minute to inactivate the reaction, and stored at -60 to -90°C for subsequent analysis. Sample analysis of the test compound and the control compound testosterone was performed using LC-MS/MS method. Chromatographic peaks were integrated, calculated, and processed using Analyst software. Semi-quantitative analysis was performed for both the test compound and the control compound testosterone by calculating peak area ratios. The results are shown in Table 2 below.

**Table 2: Rat liver microsomal stability results of compounds in some examples**

| **Example ID** | **Rat microsomal metabolic half-life (t_{1/2}) (minutes)** |
|---|---|
| 68 | 8.36 |
| 216 | 23.1 |
| 239 | >120 |
| 302 | 58.72 |
| 274 | >186 |
| 300 | 24.15 |
| 279 | 55.3 |
| 281 | 70.1 |
| 287 | 76.28 |
| 275 | 91.15 |
| 185 | 190 |
| 204 | 238 |
| 198 | 71.94 |
| 238 | 75.75 |
| 298 | 18.97 |
| 296 | 30.81 |
| 284 | >120 |
| 306 | 104.33 |
| 317 | 136.63 |

The compounds of the present disclosure exhibit significantly improved rat liver microsomal metabolic stability compared to the control compound 68, which is significantly better than that of compound 68.

### Preparation of key intermediates

### Preparation examples

### Example 68

### Preparation of (1R,3aR,5aR,7S,9aS,11aR)-1-[(2R)-6-hydroxy-6-methylheptan-2-yl]-3a,6,6,9a,11a-pentamethyl-2,3,3a,4,5,5a,6,7,8,9,9a,10,11,11a-tetradecahydro-1H-cyclopenta[1,2-a]phenanthren-7-ol (compound 68)

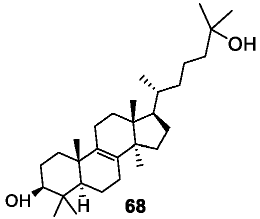

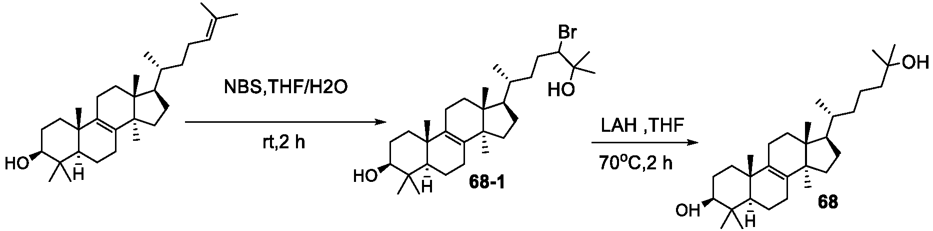

**Step 1:** The starting material lanosterol (3.00 g, 7.03 mmol, 1.0 *eq*) was dissolved in THF (tetrahydrofuran) (273 mL), then water (68 mL) was added thereto, and NBS (0.73 g, 4.08 mmol, 0.58 *eq*) was added thereto at room temperature. The resulting mixture was stirred at 25°C for 2 hours. TLC (*n*-hexane: EtOAc = 5:1, phosphomolybdic acid) showed that the reaction was completed. The reaction mixture was extracted three times with dichloromethane, dried over sodium sulfate, evaporated to dryness by rotary evaporation, subjected to column chromatography (PE: EtOAc = 50:1 to 5:1), and concentrated to obtain (1*R*,3a*R*,5a*R*,7*S*,9a*S*,11a*R*)-1-[(2*R*)-5-bromo-6-hydroxy-6-methylheptan-2-yl]-3a,6,6,9a,11a-pentamethyl-2,3,3a,4,5,5a,6,7,8,9,9a,10,11,11a-tetradecahydro-1*H*-cyclopenta[1,2-*a*]phenanthren-7-ol (**68-1**) (1.3 g, purity: 90%, yield: 35.3%). ¹H NMR (400 MHz, CDCl₃) δ 3.98 (dd, *J =* 21.0, 12.3 Hz, 1H), 3.27 - 3.19 (m, 1H), 2.14 (d, *J =* 6.0 Hz, 1H), 2.00 (d, *J =* 11.7 Hz, 5H), 1.84 - 1.61 (m, 6H), 1.58 (s, 9H), 1.33 (t, *J =* 10.8 Hz, 9H), 1.27 - 1.12 (m, 3H), 1.04 (d, *J=* 12.2 Hz, 1H), 0.99 (d, *J=* 7.9 Hz, 7H), 0.94 - 0.84 (m, 7H), 0.80 (s, 3H), 0.69 (d, *J =* 2.2 Hz, 3H).

**Step 2:** (1*R*,3a*R*,5a*R*,7*S*,9a*S*,11a*R*)-1-[(2*r*)-5-Bromo-6-hydroxy-6-methylheptan-2-yl]-3a,6,6,9a,11a-pentamethyl-2,3,3a,4,5,5a,6,7,8,9,9a,10,11,11a-tetradecahydro-1*H-*cyclopenta[1,2-*a*]phenanthren-7-ol (**68-1**) (900 mg, 1.72 mmol, 1.0 *eq*) was dissolved in THF (tetrahydrofuran) (90 mL), and LAH (433 mg, 12.40 mmol, 7.2 *eq*) was added thereto. After the addition was completed, the reaction mixture was heated to 70°C and refluxed, and stirred for 2 hours. TLC (*n*-hexane: EtOAc = 5:1, phosphomolybdic acid) detected that the reaction was completed. The reaction system was then poured into ice water (10 mL) to quench, extracted with DCM (dichloromethane) (30 mL × 3), dried over anhydrous sodium sulfate, and evaporated to dryness by rotary evaporation to obtain (1*R*,3a*R*,5a*R*,7*S*,9a*S*,11a*R*)-1-[(2*R*)-6-hydroxy-6-methylheptan-2-yl]-3a,6,6,9a,11a-pentamethyl-2,3,3a,4,5,5a,6,7,8,9,9a,10,11,11a-tetradecahydro-1*H*-cyclopenta[1,2-*a*]phenanthren-7-ol (**68**) (0.4 g, purity: 90%, yield: 48.7%). ¹H NMR (400 MHz, CDCl₃) δ 3.23 (d, *J=* 8.8 Hz, 1H), 2.02 (s, 4H), 1.92 (d, *J=* 8.4 Hz, 1H), 1.69 (d, *J =* 24.3 Hz, 7H), 1.58 (d, *J =* 11.1 Hz, 2H), 1.52 - 1.33 (m, 8H), 1.30 (s, 2H), 1.18 (d, *J =* 25.9 Hz, 8H), 1.01 (dd, *J =* 22.8, 9.8 Hz, 8H), 0.93 - 0.84 (m, 6H), 0.80 (s, 3H), 0.68 (s, 3H). ¹³C NMR (101 MHz, CDCl₃) δ 134.38, 78.97, 77.33, 77.01, 76.69, 71.13, 50.49, 50.38, 49.79, 44.46, 44.39, 38.87, 37.00, 36.72, 36.45, 35.57, 30.97, 30.82, 29.31, 29.18, 28.23, 27.95, 27.82, 26.48, 24.26, 21.10, 20.99, 19.13, 18.67, 18.24, 15.74, 15.41.

### Example 146

### Preparation of (1R,3aR,5aR,7S,9aS,11aR)-1-[(2R)-7-hydroxyheptan-2-yl]-3a,6,6,9a,11a-pentamethyl-2,3,3a,5,5a,6,7,8,9,9a,11,11a-dodecahydro-1H-cyclopenta[1,2-a]phenanthren-7-ol (compound 146)

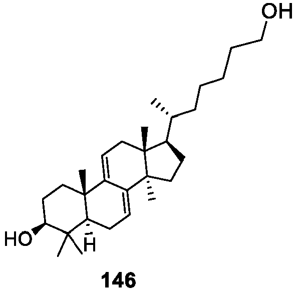

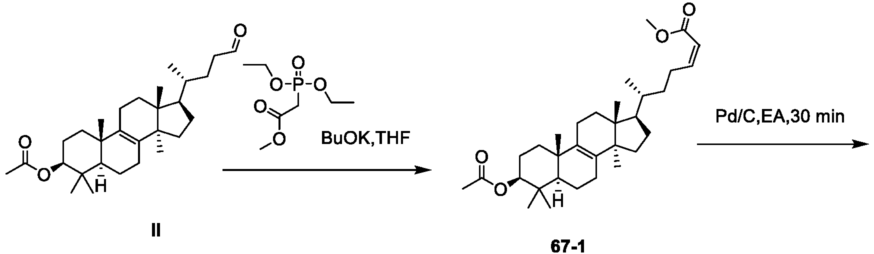

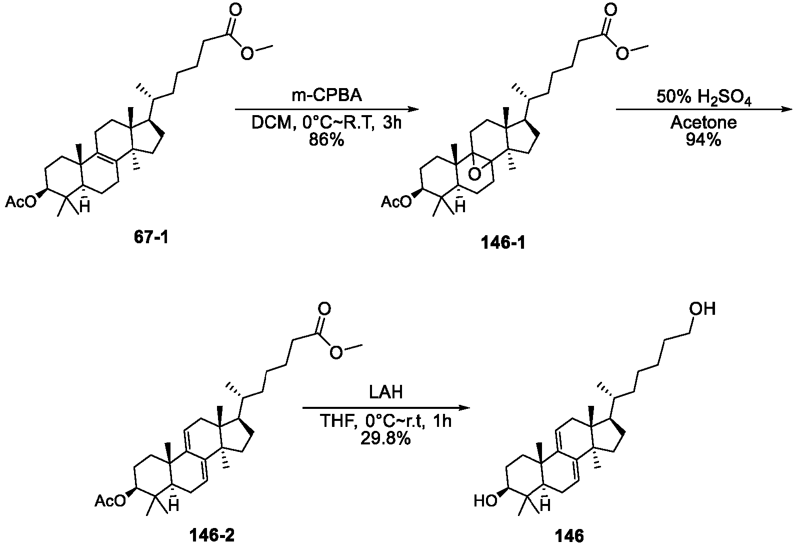

**Step 1:** (1*R*,3a*R*,5a*R*,7*S*,9a*S*,11a*R*)-1-[(2*R*)-4-Formylbutan-2-yl]-3a,6,6,9a,11a-pentamethyl-2,3,3a,4,5,5a,6,7,8,9,9a,10,11,11a-tetradecahydro-1*H*-cyclopenta[2,1-*i*]phenanthren-7-yl acetate **(II)** (500 mg, 1.1 mmol) was dissolved in tetrahydrofuran (30 mL). The reaction mixture was replaced with N₂ three times, cooled to 0°C, added with solid potassium *tert*-butoxide (253 mg, 2.3 mmol), and stirred at 0°C for 30 minutes. The compound diethyl (2-methoxy-2-oxoethyl)phosphonate (500 mg, 1.1 mmol) was dissolved in THF (5 mL) and added dropwise to the above reaction system. The reaction mixture was naturally warmed to room temperature and stirred for 2 hours. TLC (PE: EtOAc = 10:1) showed that there was no starting material remaining. The reaction mixture was cooled to 0°C, added with 1 M HCl (2 mL) for phase separation, extracted twice with ethyl acetate, dried over anhydrous sodium sulfate, evaporated to dryness by rotary evaporation, and subjected to column chromatography to obtain methyl (2*E*,6*R*)-6-[(1*R*,3a*R*,5a*R*,7*S*,9a*S*,11a*R*)-7-acetyloxy-3a,6,6,9a,11a-pentamethyl-2,3,3a,4,5,5a,6,7,8,9,9a,10,11,11a-tetradecahydro-1*H-*cyclopenta[1,2-*a*]phenanthren-1-yl]hept-2-enoate (**67-1**) (430 mg, 0.86 mmol, 76.65%) as a white solid. ¹H NMR (399 MHz, CDCl₃) δ 7.03 - 6.85 (m, 1H), 5.80 (d, *J =* 15.6 Hz, 1H), 4.48 (dd, *J =* 11.7, 4.5 Hz, 1H), 3.77 - 3.62 (m, 3H), 2.25 (s, 1H), 2.12 - 1.92 (m, 8H), 1.89 (d, *J* = 21.3 Hz, 1H), 1.57 - 1.39 (m, 4H), 1.57 - 1.39 (m, 5H), 1.36 - 1.21 (m, 3H), 1.15 (dd, *J=* 19.7, 10.5 Hz, 3H), 0.98 (s, 3H), 0.87 (dd, *J =* 14.6, 4.9 Hz, 12H), 0.66 (s, 3H).

**Step 2:** Methyl (2*E*,6*R*)-6-[(1*R*,3a*R*,5a*R*,7*S*,9a*S*,11a*R*)-7-acetyloxy-3a,6,6,9a,11a-pentamethyl-2,3,3a,4,5,5a,6,7,8,9,9a,10,11,11a-tetradecahydro-1*H*-cyclopenta[1,2-*a*]phenanthren-1-yl]hept-2-enoate (**67-1**) (380 mg, 0.762 mmol) was dissolved in ethyl acetate (380 mL), and Pd/C (palladium on carbon) (38 mg, 0.762 mmol) was added thereto. The reaction mixture was replaced with hydrogen three times and stirred at room temperature for 30 minutes. TLC (PE: EtOAc = 10:1) detected that there was no starting material remaining and the reaction was completed. The reaction mixture was filtered through diatomite, rinsed with ethyl acetate, evaporated to dryness by rotary evaporation, subjected to column chromatography (PE: EtOAc = 100% to 20%), and concentrated to obtain methyl (6*R*)-6-[(1*R*,3a*R*,5a*R*,7*S*,9a*S*,11a*R*)-7-acetyloxy-3a,6,6,9a,11a-pentamethyl-2,3,3a,4,5,5a,6,7,8,9,9a,10,11,11a-tetradecahydro-1*H*-cyclopenta[1,2-*a*]phenanthren-1-yl]heptanoate (**67-2**) (350 mg, 0.629 mmol, 82.56%) as a white solid. ¹H NMR (400 MHz, CDCl₃) δ 4.49 (dd, *J =* 11.5, 4.6 Hz, 1H), 3.66 (d, *J =* 1.1 Hz, 3H), 2.30 (t, *J =* 8.0 Hz, 2H), 2.11 - 1.94 (m, 7H), 1.94 - 1.82 (m, 1H), 1.82 - 1.46 (m, 8H), 1.46 - 1.09 (m, 8H), 0.99 (s, 3H), 0.87 (q, *J =* 2.7, 2.2 Hz, 12H), 0.67 (s, 3H). ¹³C NMR (101 MHz, CDCl₃) δ 134.42, 134.20, 51.46, 50.38, 49.77, 44.43, 37.78, 36.86, 36.25, 35.77, 35.24, 34.18, 30.92, 30.78, 28.18, 27.90, 26.35, 25.90, 25.39, 24.23, 24.15, 21.35, 20.97, 19.17, 18.62, 18.10, 16.52, 15.73, 1.02.

**Step 3:** Methyl (6*R*)-6-[(1*R*,3a*R*,5a*R*,7*S*,9a*S*,11a*R*)-7-acetyloxy-3a,6,6,9a,11a-pentamethyl-2,3,3a,4,5,5a,6,7,8,9,9a,10,11,11a-tetradecahydro-1*H*-cyclopenta[1,2-*a*]phenanthren-1-yl]heptanoate (**67-1**) (100 mg, 0.2 mmol, 1.0 *eq.*) was weighed and dissolved in dichloromethane (10 mL), and then *m*-chloroperoxybenzoic acid (77.61 mg, 0.45 mmol, 2.3 *eq.*) was added thereto in an ice bath. After the addition was completed, the reaction mixture was returned to room temperature and stirred. The reaction was monitored by TLC (PE: EA = 10:1, developing with phosphomolybdic acid, Rf₁ = 0.5, Rf₂ = 0.3). After the reaction was completed, the reaction mixture was added with saturated sodium bicarbonate aqueous solution (5.0 mL) and extracted with dichloromethane (5.0 mL * 3). The organic phase was dried and concentrated to obtain methyl (6*R*)-6-((3*S*,5*S*,10*S*,13*R*,14*R*,17*R*)-3-acetoxy-4,4,10,13,14-pentamethyltetradecahydro-11*H*-8,9-epoxycyclopenta[*a*]phenanthren-17-yl)heptanoate (**146-1**) (89 mg, 0.17 mmol, 86%) as a white solid crude product, which was used in the next reaction step without purification.

**Step 4:** Methyl (6*R*)-6-((3*S*,5*S*,10*S*,13*R*,14*R*,17*R*)-3-acetoxy-4,4,10,13,14-pentamethyltetradecahydro-11*H*-8,9-epoxycyclopenta[*a*]phenanthren-17-yl)heptanoate (**146-1**) (89 mg, 0.17 mmol) was weighed and dissolved in acetone (10 mL), then concentrated sulfuric acid (1 d) was added thereto at room temperature, and the reaction mixture was stirred at room temperature for 5 hours. The reaction was monitored by TLC (PE: EtOAc = 10:1, developing with phosphomolybdic acid, Rf₁ = 0.3, Rf₂ = 0.6). After the reaction was completed, the reaction mixture was concentrated to remove acetone, added with saturated sodium bicarbonate aqueous solution (10.0 mL), and extracted with ethyl acetate (8.0 mL * 3). The organic phase was separated, dried, concentrated, and purified by column chromatography (petroleum ether: ethyl acetate = 30:1 to 10:1) to obtain methyl (6R)-6-[(1*R*,3a*R*,5a*R*,7*S*,9a*S*,11a*R*)-7-acetoxy-3a,6,6,9a,11a-pentamethyl-2,3,3a,5,5a,6,7,8,9,9a,11,11a-dodecahydro-1*H*-cyclopenta[1,2-*a*]phenanthren-1-yl]heptanoate (**146-2**) (81 mg, 0.16 mmol, 94%). ¹H NMR (399 MHz, CDCl₃) δ 5.43 (s, 1H), 5.29 (d, *J =* 5.8 Hz, 1H), 4.49 (dd, *J =* 11.1, 4.8 Hz, 1H), 3.65 (s, 3H), 2.29 (t, *J* = 7.4 Hz, 2H), 2.18 (d, *J =* 17.8 Hz, 1H), 2.10 - 2.04 (m, 2H), 2.03 (s, 4H), 2.00 - 1.93 (m, 2H), 1.76 - 1.68 (m, 2H), 1.65 - 1.57 (m, 3H), 1.50 (d, *J=* 4.6 Hz, 1H), 1.42 - 1.34 (m, 4H), 1.27 (d, *J=* 10.2 Hz, 2H), 1.20 - 1.11 (m, 2H), 1.07 - 1.00 (m, 1H), 0.98 (s, 3H), 0.93 (s, 3H), 0.86 (d, *J =* 6.9 Hz, 9H), 0.53 (s, 3H). ¹³C NMR (101 MHz, CDCl₃) d 174.36, 170.99, 145.53, 142.71, 119.83, 116.53, 80.81, 77.31, 76.99, 76.68, 51.46, 50.91, 50.28, 49.20, 43.68, 37.77, 37.57, 37.19, 36.03, 35.70, 35.35, 34.16, 31.44, 28.06, 27.85, 25.90, 25.50, 25.37, 24.22, 22.76, 21.33, 18.40, 16.91, 15.62, 1.00.

**Step 5:** Methyl (6*R*)-6-[(1*R*,3a*R*,5a*R*,7*S*,9a*S*,11a*R*)-7-acetoxy-3a,6,6,9a,11a-pentamethyl-2,3,3a,5,5a,6,7,8,9,9a,11,11a-dodecahydro-1*H*-cyclopenta[1,2-*a*]phenanthren-1-yl]heptanoate (**146-2**) (50 mg, 0.1 mmol) was dissolved in tetrahydrofuran (5 mL). The reaction mixture was replaced with N₂, cooled to 0°C, added with lithium aluminum hydride (41 mg, 1.0 mmol), and reacted at 0°C for 1 hour. TLC (PE: EA = 10:1) showed that there was no starting material remaining. The reaction mixture was added with methanol, filtered through diatomite, concentrated, and purified by preparative HPLC to obtain (1*R*,3a*R*,5a*R*,7*S*,9a*S*,11a*R*)-1-[(2*R*)-7-hydroxyheptan-2-yl]-3a,6,6,9a,11a-pentamethyl-2,3,3a,5,5a,6,7,8,9,9a,11,11a-dodecahydro-1*H*-cyclopenta[1,2-*a*]phenanthren-7-ol (**146**) (12.8 mg, 0.03 mmol, 29.8%) as a white solid. ¹H NMR (399 MHz, CDCl₃) 5.45 (s, 1H), 5.30 (s, 1H), 3.62 (t, *J =* 6.6 Hz, 2H), 3.23 (dd, *J =* 11.2, 4.5 Hz, 1H), 2.18 (d, *J =* 17.4 Hz, 1H), 2.06 (d, *J* = 11.3 Hz, 3H), 1.97 (d, *J =* 13.0 Hz, 2H), 1.64 (m, 4H), 1.43 (m, 2H), 1.34 (dd, *J=* 20.2, 7.5 Hz, 6H), 1.25 (d, *J=* 11.6 Hz, 3H), 1.06 (m, 2H), 0.98 (s, 3H), 0.96 (s, 3H), 0.86 (s, 9H), 0.54 (s, 3H). ¹³C NMR (101 MHz, CDCl₃) 120.13, 116.29, 78.95, 77.31, 77.20, 76.99, 76.67, 63.10, 50.95, 50.29, 49.08, 43.70, 38.68, 37.79, 37.34, 36.13, 36.09, 35.68, 32.86, 31.48, 28.12, 27.90, 27.78, 26.19, 26.14, 25.56, 22.98, 22.73, 18.48, 15.78, 15.62. LC-MS: [M-17]⁺ = 411.

### Example 151

### Preparation of cholest-5(6)-en-3β,4β,25-triol (compound 151)

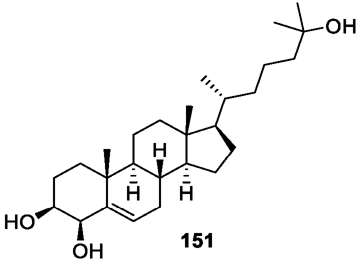

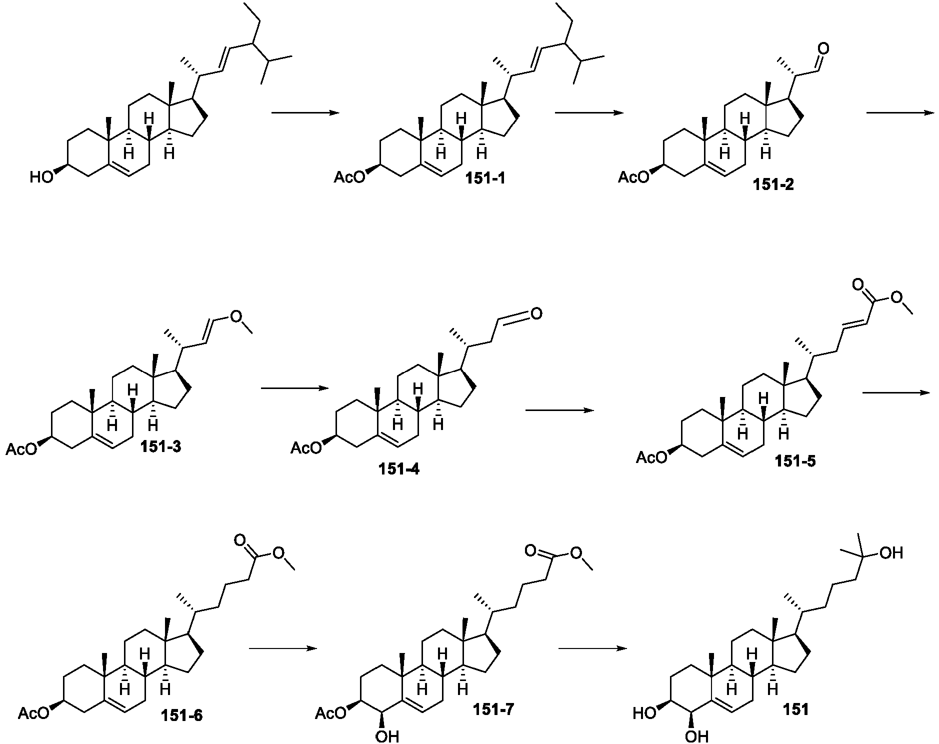

**Step 1:** (22*E*,24*S*)-Stigmasta-6(5),22(23)-dien-3β-ol (50.0 g, 0.12 mol, 1.0 *eq.*) and 4-dimethylaminopyridine (2.96 g, 24.23 mmol) were weighed and dissolved in dichloromethane (500 mL). Triethylamine (61.90 g, 605.77 mmol) was added thereto, then acetic anhydride (37.11 g, 0.36 mol) was added thereto with stirring at room temperature, and the reaction mixture was stirred at room temperature for 2 hours. The reaction was monitored by TLC (PE: EtOAc = 10:1). After the reaction was completed, the reaction mixture was concentrated. The resulting crude product was added with methanol (300 mL), stirred, and filtered. The cake was washed with methanol (20 mL * 2) and then dried to obtain (1*R*,3a*S*,3b*S*,7*S*,9a*R*,9b*S*,11a*R*)-1-[(2*R*,3*E*)-5-ethyl-6-methylhept-3-en-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate (**151-1**) (53.8 g, 112.4 mmol, 92.77%) as a white solid. ¹H NMR (399 MHz, Chloroform-d) δ 5.35 (d, *J =* 4.9 Hz, 1H), 5.14 (dd, *J =* 15.1, 8.5 Hz, 1H), 4.99 (dd, *J =* 15.2, 8.5 Hz, 1H), 4.67 - 4.48 (m, 1H), 2.30 (d, *J=* 7.8 Hz, 2H), 2.01 (s, 3H), 2.00 - 1.91 (m, 2H), 1.84 (d, *J=* 11.3 Hz, 2H), 1.68 (dd, *J=* 9.4, 4.8 Hz, 1H), 1.48 (dt, *J=* 24.5, 9.2 Hz, 7H), 1.28 - 1.07 (m, 6H), 1.00 (d, *J=* 3.2 Hz, 6H), 0.80 (dd, *J=* 20.1, 6.4 Hz, 9H), 0.68 (s, 3H).

**Step 2:** (1*R*,3a*S*,3b*S*,7*S*,9a*R*,9b*S*,11a*R*)-1-[(2*R*,3*E*)-5-Ethyl-6-methylhept-3-en-2-yl 9a,11a-dimethyl-2,3,3a,3b,4,6,7,8,9,9a,9b,10,11,11a-tetradecahydro-1*H*-cyclopenta[1,2-*i*]phenanthren-7-yl acetate (**151-1**) (5.0 g, 11 mmol) was weighed and dissolved in tetrahydrofuran (200 mL) and water (20.0 mL). Pyridine (2.12 mL, 27.5 mmol), *N-*methylmorpholinium oxide (5.15 g, 43.98 mmol), and potassium osmate (7.3 mL, 1.1 mmol) were added thereto at room temperature, and the reaction mixture was stirred at room temperature overnight. TLC (PE: EtOAc = 3:1, developing with phosphomolybdic acid) detected that there was some starting material remaining, and an intermediate (vicinal diol) was generated. The reaction mixture was then added with sodium periodate (9.41 g, 43.98 mmol) at 0°C and stirred at room temperature for 1 hour. TLC (PE: EtOAc = 3:1, developing with phosphomolybdic acid) detected that there was some starting material remaining, and the intermediate was converted into a product. The reaction mixture was then added with water (50 mL), extracted with ethyl acetate (50 mL * 3), dried, and concentrated to obtain a crude product, which was then purified by column chromatography (PE: EtOAc = 60:1) to obtain (1*R*,3a*S*,3b*S*,7*S*,9a*R*,9b*S*,11a*R*)-1-[(1*S*)-1-formylethyl]-9a,11a-dimethyl-2,3,3a,3b,4,6,7,8,9,9a,9b,10,11,11a-tetradecahydro-1*H*-cyclopenta[1,2-*i*]phenanthren-7-yl acetate (**151-2**) (0.64 g, 1.6 mmol, 14.73%) as a white solid. ¹H NMR (399 MHz, CDCl₃) δ 9.55 (s, 1H), 5.36 (s, 1H), 4.60 (s, 1H), 2.31 (s, 3H), 2.02 (s, 3H), 1.93 (s, 2H), 1.84 (d, *J =* 11.2 Hz, 3H), 1.63 (s, 3H), 1.49 (d, *J* = 10.9 Hz, 4H), 1.42 - 1.19 (m, 3H), 1.15 (s, 1H), 1.11 (d,*J* = 6.8 Hz, 3H), 1.06 (s, 1H), 1.01 (s, 3H), 0.98 (d, *J=* 5.8 Hz, 1H), 0.71 (s, 3H).

**Step 3:** (Methoxymethyl)triphenylphosphonium chloride (8.7 g, 25.0 mmol) was weighed and dissolved in tetrahydrofuran (38 mL). The reaction system was replaced with nitrogen three times, cooled to 0°C, and added dropwise with sodium bis(trimethylsilyl)amide (12.8 mL, 25.0 mmol). After the dropwise addition was completed, the reaction mixture was stirred at 0°C for 0.5 hours, then added with a solution of (1*R*,3a*S*,3b*S*,7*S*,9a*R*,9b*S*,11a*R*)-1-[(1*S*)-1-fonnylethyl]-9a,11a-dimethyl-2,3,3a,3b,4,6,7,8,9,9a,9b,10,11,11a-tetradecahydro-1*H-*cyclopenta[1,2-*i*]phenanthren-7-yl acetate (**151-2**) (1.9 g, 5.1 mmol) in tetrahydrofuran (18 mL), warmed to room temperature, and stirred for 1 hour. TLC (PE: EtOAc = 10:1, developing with phosphomolybdic acid) detected that the starting material was completely reacted. The reaction mixture was then added with water (30 mL), extracted with ethyl acetate (15 mL * 3), dried, and concentrated to obtain a crude product, which was then purified by column chromatography (PE: EtOAc = 100:1 to 30:1) to obtain (1*R*,3a*S*,3b*S*,7*S*,9a*R*,9b*S*,11a*R*)-1-[(2*S*,3*E*)-4-methoxybut-3-en-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate (**151-3**) (0.75 g, 1.8 mmol, 34.87%) as a white solid. ¹H NMR (399 MHz, CDCl₃) δ 6.22 (d, *J =* 12.6 Hz, 1H), 5.71 (d, *J* = 6.4 Hz, 0H), 5.35 (d, *J* = 4.1 Hz, 1H), 4.57 (m, 2H), 4.15 (m, 0H), 3.52 (d, *J =* 10.6 Hz, 1H), 3.46 (d, *J =* 8.7 Hz, 3H), 2.59 (d, *J =* 6.6 Hz, 0H), 2.30 (d, *J =* 7.6 Hz, 3H), 2.01 (s, 3H), 1.95 (d, *J =* 5.8 Hz, 3H), 1.84 (d, *J =* 10.9 Hz, 3H), 1.69 (d, *J =* 4.6 Hz, 1H), 1.52 (s, 2H), 1.48 (s, 2H), 1.14 (m, 3H), 1.03 (s, 3H), 1.00 (s, 3H), 0.67 (s, 3H).

**Step 4:** (1*R*,3a*S*,3b*S*,7*S*,9a*R*,9b*S*,11a*R*)-1-[(2*S*,3*E*)-4-Methoxybut-3-en-2-yl]-9a,11a-dimethyl-2,3,3a,3b,4,6,7,8,9,9a,9b,10,11,11a-tetradecahydro-1*H*-cyclopenta[1,2-i]phenanthren-7-yl acetate (**151-3**) (0.75 g, 1.87 mmol) was weighed and dissolved in tetrahydrofuran (7.5 mL), and 5 N hydrochloric acid (2.25 mL, 11.2 mmol) was added thereto. The reaction mixture was then heated to 50°C and stirred for 1 hour. TLC (PE: EtOAc = 10:1, developing with phosphomolybdic acid) detected that the starting material was completely reacted. The reaction mixture was then added with water (15 mL), extracted with ethyl acetate (10 mL * 3), dried, and concentrated to obtain a crude product, which was then purified by column chromatography (PE: EtOAc = 50:1 to 20:1) to obtain (1*R*,3a*S*,3b*S*,7*S*,9a*R*,9b*S*,11a*R*)-1-[(2*R*)-1-formylpropan-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate (**151-4**) (0.52 g, 1.3 mmol, 68.26%) as a white solid. ¹H NMR (399 MHz, CDCl₃) δ 9.74 (s, 1H), 5.36 (d, *J =* 4.4 Hz, 1H), 4.60 (s, 1H), 2.44 (d, *J =* 16.5 Hz, 1H), 2.30 (d, *J =* 6.7 Hz, 2H), 2.20 - 2.10 (m, 1H), 2.01 (s, 3H), 1.95 (d, *J =* 14.4 Hz, 2H), 1.84 (d, *J =* 11.5 Hz, 3H), 1.57 (dd, *J =* 19.7, 8.6 Hz, 4H), 1.46 (dd, *J =* 14.2, 10.3 Hz, 3H), 1.29 - 1.16 (m, 3H), 1.15 - 1.03 (m, 3H), 1.01 (d, *J =* 4.5 Hz, 6H), 0.96 - 0.89 (m, 1H), 0.70 (d, *J =* 12.6 Hz, 3H).

**Step 5:** (1*R*,3a*S*,3b*S*,7*S*,9a*R*,9b*S*,11a*R*)-1-[(2*R*)-1-Formylpropan-2-yl]-9a,11a-dimethyl-2,3,3a,3b,4,6,7,8,9,9a,9b,10,11,11a-tetradecahydro-1*H*-cyclopenta[1,2-i]phenanthren-7-yl acetate (**151-4**) (0.52 g, 1.34 mmol) was weighed and dissolved in tetrahydrofuran (30 mL), and (triphenyl-λ5-phosphanylidene)methyl acetate (2.25 g, 6.73 mmol) was added thereto. The reaction system was heated to 90°C and stirred for 3 hours. HPLC detected that there was no starting material remaining. The reaction mixture was then cooled to room temperature, added with water (15 mL), extracted with ethyl acetate (10 mL * 3), dried, and concentrated to obtain a crude product, which was then purified by column chromatography (PE: EtOAc = 50:1 to 20:1) to obtain methyl (2E,5R)-5-[(1*R*,3a*S*,3b*S*,7*S*,9a*R*,9b*S*,11a*R*)-7-acetoxy-9a,11a-dimethyl-2,3,3a,3b,4,6,7,8,9,9a,9b,10,11,11a-tetradecahydro-1*H*-cyclopenta[1,2-*a*]phenanthren-1-yl]hex-2-enoate (**151-5**) (0.44 g, 0.94 mmol, 70.20%) as a white solid. ¹H NMR (399 MHz, CDCl₃) δ 6.99 - 6.86 (m, 1H), 5.80 (d, *J =* 15.6 Hz, 1H), 5.36 (s, 1H), 4.59 (s, 1H), 3.71 (s, 3H), 2.30 (d, *J =* 6.7 Hz, 3H), 2.01 (s, 3H), 2.00 - 1.90 (m, 3H), 1.84 (d, *J =* 10.9 Hz, 3H), 1.54 (d, *J =* 16.6 Hz, 5H), 1.50 - 1.37 (m, 3H), 1.33 - 1.17 (m, 2H), 1.16 - 1.02 (m, 4H), 1.00 (s, 3H), 0.93 (d, *J=* 6.6 Hz, 3H), 0.67 (s, 3H).

**Step 6:** Methyl (2*E*,5*R*)-5-[(1*R*,3a*S*,3b*S*,7*S*,9a*R*,9b*S*,11a*R*)-7-acetoxy-9a,11a-dimethyl-2,3,3a,3b,4,6,7,8,9,9a,9b,10,11,11a-tetradecahydro-1*H*-cyclopenta[1,2-*a*]phenanthren-1-yl]hex-2-enoate (**151-5**) (0.44 g, 1.0 mmol) was weighed and dissolved in a mixed solvent of tetrahydrofuran (9.0 mL) and methanol (4.5 mL). The reaction mixture was added with nickel chloride (130 mg, 1.0 mmol), then added with sodium borohydride (56.92 mg, 1.5 mmol) with stirring at room temperature to generate a large number of bubbles, and stirred at room temperature for 1 hour. A sample was taken and concentrated. NMR monitored that the starting material was completely reacted. The reaction mixture was added with water (15 mL), extracted with ethyl acetate (10 mL * 3), dried, and concentrated to obtain a crude product, which was then purified by column chromatography (PE: EtOAc = 60:1 to 20:1) to obtain methyl (5*R*)-5-[(1*R*,3a*S*,3b*S*,7*S*,9a*R*,9b*S*,11a*R*)-7-acetoxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate (**151-6**) (0.36 g, 0.78 mmol, 77.74%) as a white solid. ¹H NMR (399 MHz, CDCl₃) δ 5.36 (s, 1H), 4.59 (s, 1H), 3.65 (s, 3H), 2.35 - 2.18 (m, 4H), 2.01 (s, 3H), 1.94 (d, *J* = 15.3 Hz, 2H), 1.84 (d, *J=* 10.9 Hz, 3H), 1.68 (s, 1H), 1.55 (s, 2H), 1.53 - 1.44 (m, 4H), 1.39 (d, *J =* 10.2 Hz, 3H), 1.22 (dd, *J =* 22.8, 12.0 Hz, 2H), 1.15 - 1.09 (m, 2H), 1.08 - 1.03 (m, 2H), 1.00 (s, 3H), 0.96 (s, 1H), 0.91 (d, *J=* 6.3 Hz, 3H), 0.89 - 0.79 (m, 1H), 0.64 (d, *J=* 12.6 Hz, 3H).

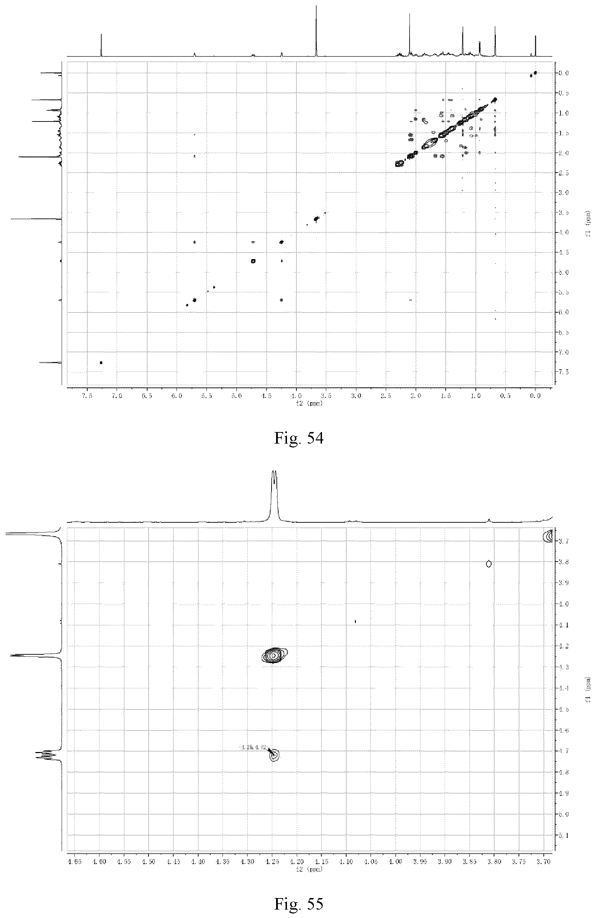

**Step 7:** Methyl (5*R*)-5-[(1*R*,3a*S*,3b*S*,7*S*,9a*R*,9b*S*,11a*R*)-7-acetoxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate (**151-6**) (0.15 g, 0.34 mmol) was weighed and dissolved in a mixed solvent of chloroform (6.0 mL). Selenium dioxide (52.4 mg, 0.47 mmol) was added thereto, and then N-methylmorpholine (102.36 mg, 1.01 mmol) was added thereto with stirring. The reaction system was heated to 70°C, and refluxed and stirred at this temperature for 64 hours. TLC (PE: EtOAc = 3:1) monitored that a large polar material was generated and there was starting material remaining. The reaction mixture was then quenched with water (10 mL), extracted with ethyl acetate (10 mL * 3), dried, and concentrated to obtain a crude product, which was then purified by column chromatography (PE: EtOAc = 100:1 to 20:1) to obtain the starting material (1*R*,3a*S*,3b*S*,7*S*,9a*R*,9b*S*,11a*R*)-1-[(2*R*)-6-methoxy-6-oxohexan-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate (20 mg) and the product methyl (5*R*)-5-[(1*R*,3a*S*,3b*S*,6*R*,7*S*,9a*R*,9b*S*,11a*R*)-7-acetoxy-6-hydroxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate (**151-7**) (66 mg, 0.14 mmol, 40.35%) as a white solid. The chiral configuration was confirmed by two-dimensional spectrum, and the results are shown in Figs. 54 to 55. ¹H NMR (399 MHz, CDCl₃) δ 5.69 (s, 1H), 4.70 (d, *J =* 11.7 Hz, 1H), 4.23 (s, 1H), 3.65 (s, 3H), 2.25 (dd, *J =* 16.6, 8.5 Hz, 2H), 2.09 (s, 3H), 2.05 - 1.94 (m, 2H), 1.83 (s, 3H), 1.67 (s, 3H), 1.56 (s, 3H), 1.45 (s, 2H), 1.39 (s, 3H), 1.24 (s, 2H), 1.20 (s, 3H), 1.17 - 1.08 (m, 3H), 0.99 (d, *J =* 9.5 Hz, 3H), 0.91 (d, *J =* 6.4 Hz, 3H), 0.65 (s, 3H).

**Step 8:** Methyl (5*R*)-5-[(1*R*,3a*S*,3b*S*,6*R*,7*S*,9a*R*,9b*S*,11a*R*)-7-acetoxy-6-hydroxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate (**151-7**) (30 mg, 0.06 mmol) was weighed and dissolved in a mixed solvent of tetrahydrofuran (2.0 mL). The reaction mixture was replaced with nitrogen, cooled to -78°C, added dropwise with methyllithium (0.2 mL, 0.33 mmol), then warmed to room temperature, and stirred for 3 hours. TLC (DCM: MeOH = 20:1) monitored that the starting material was completely reacted. The reaction mixture was then quenched with water (5 mL), extracted with ethyl acetate (3 mL * 3), dried, and concentrated to obtain a crude product, which was then purified by column chromatography (DCM: MeOH = 500:1 to 100:1) to obtain cholest-5(6)-en-3β,4β,25-triol (**151**) (13 mg, 0.03 mmol, 45.3%) as a white solid. ¹H NMR (399 MHz, Chloroform-d) δ 5.71 - 5.61 (m, 1H), 4.12 (s, 1H), 3.54 (s, 1H), 2.16 - 1.96 (m, 4H), 1.85 (dd, *J* = 28.6, 13.7 Hz, 3H), 1.64 (s, 2H), 1.55 (d, *J=* 20.5 Hz, 3H), 1.42 (s, 4H), 1.36 (d, *J=* 11.0 Hz, 4H), 1.23 (s, 2H), 1.19 (s, 6H), 1.16 (s, 3H), 1.10 (d, *J =* 10.0 Hz, 2H), 1.05 (d, *J =* 10.5 Hz, 2H), 0.99 (d, *J =* 13.0 Hz, 2H), 0.91 (d, *J =* 6.5 Hz, 3H), 0.66 (s, 3H). ¹³C NMR (100 MHz, CDCl₃) δ 142.69, 134.69, 128.79, 77.31, 77.24, 77.00, 76.68, 72.46, 71.11, 56.86, 55.96, 50.12, 44.38, 42.29, 39.64, 36.86, 36.40, 35.96, 35.71, 32.05, 31.78, 29.35, 29.18, 28.22, 25.38, 24.22, 21.03, 20.72, 20.50, 18.65, 11.85.

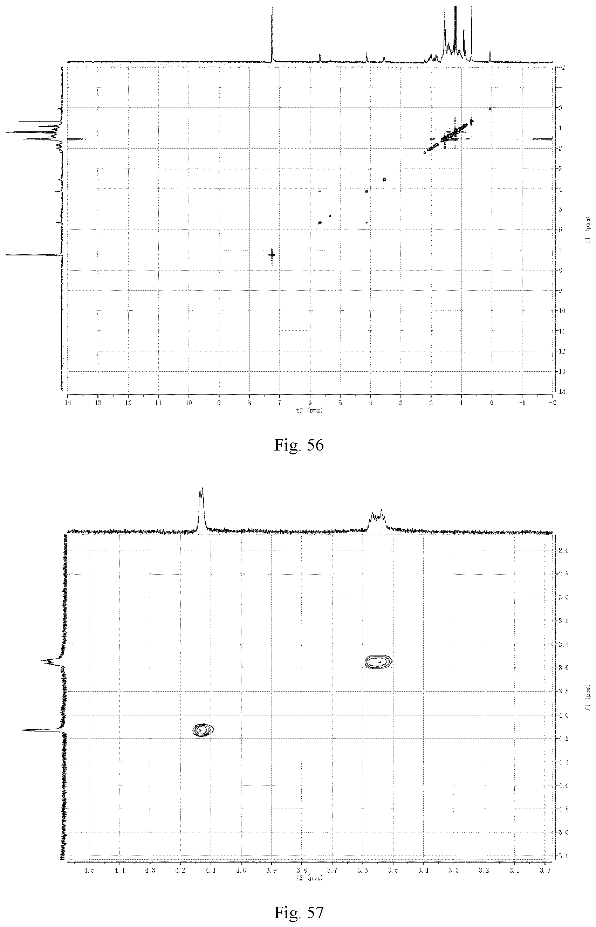

The two-dimensional spectrum of compound **151** is shown in Figs. 56 to 57.

### Example 177

### Preparation of cholest-5(6)-en-3β,25-diol (compound 177)

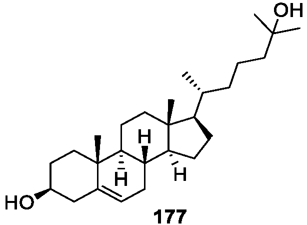

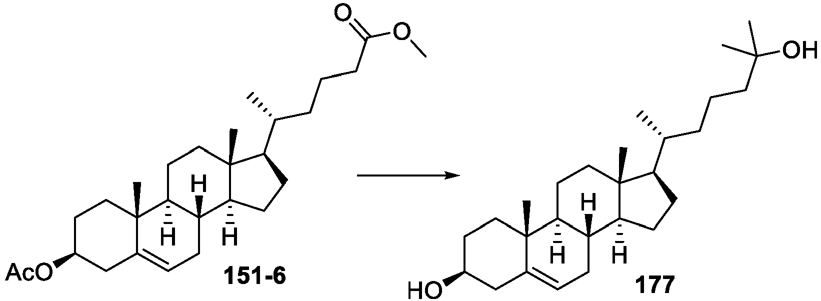

(1*R*,3a*S*,3b*S*,7*S*,9a*R*,9b*S*,11a*R*)-1-[(2*R*)-6-Methoxy-6-oxohexan-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate (**151-6**) (77 mg, 0.17 mol, 1.0 *eq.*) was weighed and dissolved in tetrahydrofuran (2 mL). The reaction mixture was replaced with nitrogen, cooled to -78°C in a dry ice-acetone bath, added with methyllithium (0.54 mL, 0.87 mmol), then warmed to room temperature, and stirred. The reaction was monitored by TLC (petroleum ether: ethyl acetate = 10:1). After the reaction was completed, the reaction mixture was quenched with water (6 mL) and extracted with ethyl acetate (5 mL * 3). The organic phase was dried and concentrated to obtain a crude product, which was subjected to column chromatography (DCM: MeOH = 300:1 to 100:1) to obtain cholest-5(6)-en-3β,25-diol (**177**) (35 mg, 0.083 mmol, 47.66%) as a white solid. ¹H NMR (399 MHz, CDCl₃) δ 5.32 (s, 1H), 3.50 (s, 1H), 2.24 (m, 2H), 1.98 (dd, *J=* 18.6, 10.9 Hz, 2H), 1.82 (d, *J=* 10.3 Hz, 3H), 1.53 (d, *J=* 10.0 Hz, 3H), 1.46 (d, *J* = 7.2 Hz, 3H), 1.42 (d, *J=* 10.1 Hz, 3H), 1.36 (d, *J=* 12.5 Hz, 4H), 1.21 (s, 3H), 1.19 (s, 6H), 1.12 (dd, *J=* 14.4, 7.2 Hz, 2H), 1.03 (m, 4H), 0.99 (s, 3H), 0.91 (d, *J=* 6.6 Hz, 3H), 0.66 (s, 3H). ¹³C NMR (100 MHz, CDCl₃) δ 140.72, 121.68, 77.31, 77.00, 76.68, 71.76, 71.11, 56.70, 56.00, 50.05, 44.38, 42.57, 42.29, 42.25, 39.72, 37.20, 36.46, 36.39, 35.72, 31.87, 31.85, 31.61, 29.34, 29.17, 28.70, 28.23, 24.26, 21.04, 20.72, 19.38, 18.66, 11.84. LC-MS: [M-OH]⁺ = 385.30.

### Example 185

### Preparation of cholest-3β,25-diol (compound 185)

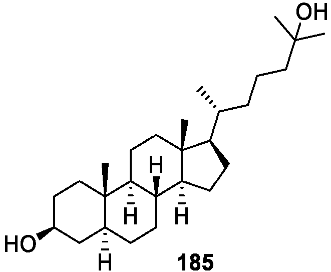

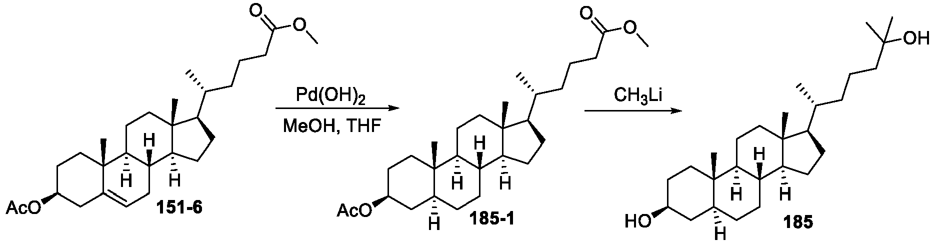

**Step 1:** (1*R*,3a*S*,3b*S*,7*S*,9a*R*,9b*S*,11a*R*)-1-[(2*R*)-6-Methoxy-6-oxohexan-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate (**151-6**) (80 mg, 0.180 mmol) was dissolved in tetrahydrofuran (2 mL) and methanol (1 mL), and Pd(OH)₂ (20 mg, 0.142 mmol) was added thereto. The reaction system was replaced with hydrogen three times and stirred at 40°C for 48 hours. The reaction was monitored by HNMR. The reaction mixture was filtered, and the filtrate was concentrated to obtain (1*R*,3a*S*,3b*R*,7*S*,9a*S*,9b*S*,11aR)-1-[(2*R*)-6-methoxy-6-oxohexan-2-yl]-9a,11a-dimethylhexadecahydro-1*H*-cyclopenta[1,2-*i*]phenanthren-7-yl acetate (**185-1**) (80 mg, 0.170 mmol, 94.57%) as an off-white solid. ¹H NMR (399 MHz, CDCl₃) δ 4.74 - 4.58 (m, 1H), 3.64 (s, 3H), 2.32 - 2.17 (m, 2H), 2.00 (s, 3H), 1.92 (d, *J=* 12.7 Hz, 1H), 1.78 (d, *J=* 9.5 Hz, 2H), 1.73 - 1.61 (m, 3H), 1.53 (d, *J* = 10.1 Hz, 3H), 1.48 - 1.40 (m, 3H), 1.33 (dd, *J=* 23.5, 11.4 Hz, 4H), 1.24 (d, *J =* 9.9 Hz, 4H), 1.20 - 1.12 (m, 3H), 1.10 - 1.02 (m, 3H), 0.96 (d, *J =* 15.7 Hz, 2H), 0.89 (d, *J=* 6.3 Hz, 3H), 0.79 (s, 3H), 0.62 (s, 3H).

**Step 2:** Methyl (1*R*,3a*S*,3b*R*,7*S*,9a*S*,9b*S*,11a*R*)-1-[(2*R*)-6-methoxy-6-oxohexan-2-yl]-9a,11a-dimethylhexadecahydro-1*H*-cyclopenta[1,2-*i*]phenanthren-7-yl acetate (**185-1**) (80 mg, 0.179 mmol) was dissolved in tetrahydrofuran (3 mL). The reaction mixture was replaced with nitrogen three times, cooled to -78°C, added with methyllithium (0.560 mL, 0.896 mmol), then warmed to room temperature, and stirred for 3 hours. The reaction was monitored by TLC (petroleum ether: ethyl acetate = 10:1). The reaction mixture was quenched with water (15 mL), extracted with ethyl acetate (10 mL * 3), washed once with brine, dried over anhydrous sodium sulfate, and concentrated to obtain a crude product (80 mg). The crude product was purified by column chromatography (DCM: MeOH = 300:1 to 100:1) to obtain cholest-3β,25-diol (**185**) (20 mg, 0.040 mmol, 22.08%). ¹H NMR (399 MHz, CDCl₃) δ 5.32 (s, 0H), 3.56 (s, 1H), 1.93 (d, *J=* 12.9 Hz, 1H), 1.75 (s, 2H), 1.70 - 1.60 (m, 2H), 1.55 (s, 3H), 1.43 (s, 3H), 1.35 (s, 3H), 1.28 (s, 3H), 1.24 (s, 3H), 1.23 (s, 3H), 1.19 (s, 6H), 1.11 - 1.01 (m, 4H), 0.95 (d, *J=* 10.9 Hz, 3H), 0.89 (d, *J=* 6.5 Hz, 3H), 0.77 (s, 3H), 0.62 (s, 3H). ¹³C NMR (101 MHz, CDCl₃) δ 77.31, 77.20, 77.00, 76.68, 71.35, 71.10, 56.45, 56.16, 54.30, 44.81, 44.39, 42.58, 40.00, 38.19, 36.97, 36.39, 35.73, 35.47, 35.43, 32.06, 31.50, 30.95, 29.33, 29.18, 28.71, 28.25, 27.19, 24.19, 21.23, 20.74, 18.61, 12.31, 12.06. LC-MS: [M+Na]⁺ = 427.15.

### Example 187

### Preparation of 2-fluoro-N-[(3R)-3-[(1R,3aS,3bS,7S,9aR,9bS,11aR)-7-hydroxy-9a,11a-dimethyl-2,3,3a,3b,4,6,7,8,9,9a,9b,10,11,11a-tetradecahydro-1H-cyclopenta[1,2-a]phenanthren-1-yl]butyl]-N-methylbenzamide (compound 187)

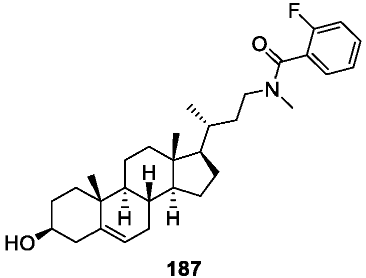

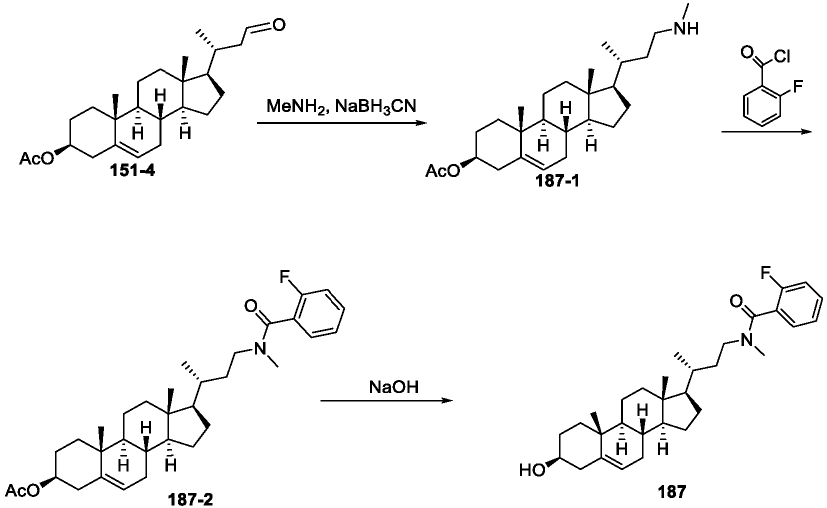

**Step 1:** (1*R*,3a*S*,3b*S*,7*S*,9a*R*,9b*S*,11a*R*)-1-[(2*R*)-1-Formylpropan-2-yl]-9a,11a-dimethyl-2,3,3a,3b,4,6,7,8,9,9a,9b,10,11,11a-tetradecahydro-1*H*-cyclopenta[1,2-i]phenanthren-7-yl acetate (**151-4**) (100 mg, 0.259 mmol) was dissolved in THF, and methylamine (32.18 mg, 1.036 mmol) and zinc chloride (17.65 mg, 0.130 mmol) were added thereto. The reaction system was stirred for 1 hour, then added with sodium cyanoborohydride (81.38 mg, 1.295 mmol), and stirred at room temperature overnight. TLC (petroleum ether: ethyl acetate = 10: 1) monitored that there was no starting material remaining. The reaction mixture was added with water (10 mL) and extracted with ethyl acetate (8 mL * 3). The organic phase was washed once with saturated brine, dried over anhydrous sodium sulfate, and concentrated to obtain (1*R*,3a*S*,3b*S*,7S,9a*R*,9b*S*,11a*R*)-9a,11a-dimethyl-1-[(2*R*)-4-(methylamino)butan-2-yl]-2,3,3a,3b,4,6,7,8,9,9a,9b,10,11,11a-tetradecahydro-1*H-*cyclopenta[1,2-*i*]phenanthren-7-yl acetate (**187-1**) (120 mg, 0.209 mmol, 80.85%), which was directly used in the next step without purification.

**Step 2:** (1*R*,3a*S*,3b*S*,7*S*,9a*R*,9b*S*,11a*R*)-9a,11a-Dimethyl-1-[(2*R*)-4-(methylamino)butan-2-yl]-2,3,3a,3b,4,6,7,8,9,9a,9b,10,11,11a-tetradecahydro-1*H-*cyclopenta[1,2-*i*]phenanthren-7-yl acetate (**187-1**) (120 mg, 0.209 mmol) and triethylamine (0.087 mL, 0.627 mmol) were dissolved in dichloromethane (3 mL), then o-fluorobenzoyl chloride (66.32 mg, 0.418 mmol) was added thereto, and the reaction mixture was stirred at room temperature for 2 hours. The reaction was monitored by TLC (petroleum ether: ethyl acetate = 3:1) and LC-MS. The reaction mixture was added with water (15 mL) and extracted with ethyl acetate (10 mL * 3). The organic phase was washed once with saturated brine, dried over anhydrous sodium sulfate, and concentrated to obtain a crude product (**187-2**) (60 mg), which was directly used in the next step.

**Step 3:** (1*R*,3a*S*,3b*S*,7*S*,9a*R*,9b*S*,11a*R*)-1-[(2*R*)-4-{[(2-fluorophenyl)carbonyl](methyl)amino}butan-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate (**187-2**) (60 mg, 0.115 mmol) was weighed and dissolved in tetrahydrofuran (2 mL) and methanol (1 mL), then sodium hydroxide aqueous solution (1 mL, 1.000 mmol) was added thereto, and the reaction mixture was stirred at room temperature for 8 hours. The reaction was monitored by TLC (petroleum ether: ethyl acetate = 3:1). The reaction mixture was added with water (10 mL) and extracted with ethyl acetate (8 mL * 3). The organic phase was washed once with saturated brine, dried over anhydrous sodium sulfate, and concentrated to obtain a crude product (60 mg), which was purified by preparative liquid chromatography to obtain 2-fluoro-*N*-[(3*R*)-3-[(1*R*,3a*S*,3b*S*,7*S*,9a*R*,9b*S*,11a*R*)-7-hydroxy-9a,11a-dimethyl-2,3,3a,3b,4,6,7,8,9,9a,9b,10,11,11a-tetradecahydro-1*H*-cyclopenta[1,2-*a*]phenanthren-1-yl]butyl]-N-methylbenzamide (**187**) (30 mg, 0.059 mmol, 51.64%) as a white solid. ¹H NMR (399 MHz, CDCl₃) δ 7.39 - 7.28 (m, 2H), 7.17 (t, *J =* 7.4 Hz, 1H), 7.07 (q, *J =* 8.4 Hz, 1H), 5.33 (s, 1H), 3.68 (s, 0.5H), 3.55 - 3.47 (m, 1H), 3.46 - 3.37 (m, 0.5H), 3.17 (d, *J=* 10.5 Hz, 1H), 3.07 (s, 1.5H), 2.86 (s, 1.5H), 2.23 (d, *J =* 20.6 Hz, 2H), 1.94 (d, *J =* 40.2 Hz, 3H), 1.82 (t, *J=* 9.7 Hz, 3H), 1.63 (s, 2H), 1.53 - 1.45 (m, 4H), 1.40 (d, *J=* 11.5 Hz, 2H), 1.32 (s, 2H), 1.15 (d, *J =* 9.3 Hz, 2H), 1.08 (d, *J =* 9.7 Hz, 2H), 1.03 (d, *J =* 6.5 Hz, 1.5H), 0.99 (s, 1.5H), 0.97 (s, 1.5H), 0.91 (dd, *J =* 17.3, 7.5 Hz, 2H), 0.69 (s, 1.5H), 0.63 (d, *J =* 5.8 Hz, 1.5H), 0.59 (s, 1.5H). ¹³C NMR (101 MHz, CDCl₃) δ 140.71, 121.66, 121.57, 77.32, 77.20, 77.00, 76.68, 71.75, 56.72, 56.61, 55.88, 55.52, 50.04, 49.97, 44.89, 42.23, 39.72, 39.58, 37.19, 36.44, 33.77, 33.42, 32.74, 31.87, 31.80, 31.63, 30.95, 28.29, 28.06, 24.26, 24.16, 21.05, 20.97, 19.39, 18.79, 18.49, 11.78, 11.70. ¹⁹F NMR (376 MHz, CDCl₃) δ -115.21, -115.37. LC-MS: [M+H]⁺ = 482.50.

### Example 198

### Preparation of 5α-cholest-3β,4β,25-triol (compound 198)

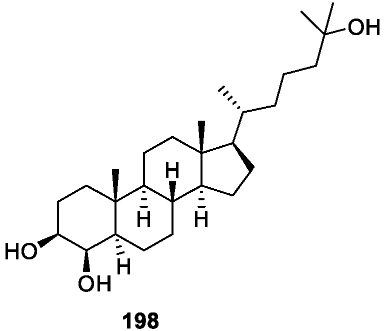

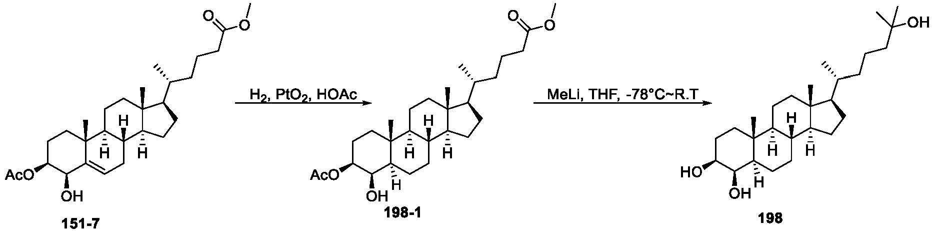

**Step 1:** Methyl (5*R*)-5-[(1*R*,3a*S*,3b*S*,6*R*,7*S*,9a*R*,9b*S*,11a*R*)-7-acetoxy-6-hydroxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate (**151-7**) (200 mg, 0.43 mol, 1.0 *eq.)* was weighed and dissolved in acetic acid (10 mL), and platinum dioxide (40 mg, 0.18 mmol) was added thereto. The reaction mixture was then heated to 40°C and stirred for 16 hours. The reaction was monitored by TLC (petroleum ether: ethyl acetate = 3:1). After the reaction was completed, the reaction mixture was filtered and concentrated to obtain a crude product (250 mg), which was then purified by column chromatography (petroleum ether: ethyl acetate = 20:1 to 15:1) to obtain methyl (5*R*)-5-[(1*R*,3a*S*,3b*S*,5a*R*,6*R*,7*S*,9a*R*,9b*S*,11a*R*)-7-acetoxy-6-hydroxy-9a,11a-dimethylhexadecahydro-1*H*-cyclopenta[1,2-*a*]phenanthren-1-yl]hexanoate (**198-1**) (130 mg, 0.28 mmol, 65.12%) as a white solid.

¹H NMR (399 MHz, Chloroform-d) δ 4.70 (ddd, *J =* 12.2, 4.7, 3.2 Hz, 1H), 3.81 (t, *J* = 3.0 Hz, 1H), 3.64 (s, 3H), 2.29 - 2.21 (m, 2H), 2.06 (s, 3H), 1.91 (dt, *J =* 8.5, 4.6 Hz, 2H), 1.74 (dp, *J=* 13.0, 5.1, 4.6 Hz, 5H), 1.54 - 1.48 (m, 3H), 1.38 - 1.33 (m, 6H), 1.09 (t, *J=* 4.7 Hz, 5H), 1.03 (s, 4H), 0.90 (d, *J=* 6.5 Hz, 4H), 0.83 (d, *J=* 3.5 Hz, 5H), 0.62 (s, 3H).

**Step 2:** Methyl (5*R*)-5-[(1*R*,3a*S*,3b*S*,5a*R*,6*R*,7*S*,9a*R*,9b*S*,11a*R*)-7-acetoxy-6-hydroxy-9a,11a-dimethylhexadecahydro-1*H*-cyclopenta[1,2-*a*]phenanthren-1-yl]hexanoate (**198-1**) (100 mg, 0.22 mmol) was weighed and dissolved in tetrahydrofuran (10 mL). The reaction mixture was cooled in a petroleum ether-dry ice bath, then added dropwise with a solution of methyllithium in tetrahydrofuran (C: 1.6 M, 2.2 mmol, 10.0 *eq.*) at low temperature, warmed to room temperature, and stirred for 16 hours. The reaction was detected by TLC (DCM: MeOH = 20:1). After the reaction was completed, the reaction mixture was added with water (10 mL), extracted with ethyl acetate (10 mL * 3), dried, and concentrated to obtain a crude product, which was then purified by column chromatography (DCM: MeOH = 300:1 to 100:1) to obtain 5α-cholest-3β,4β,25-triol (**198**) (25 mg, 0.06 mmol, 27.47%) as a white solid.

¹H NMR (399 MHz, Chloroform-d) δ 3.72 (s, 1H), 3.52 (s, 1H), 1.94 (d, *J* = 12.4 Hz, 1H), 1.73 (s, 4H), 1.68 (s, 3H), 1.53 (s, 3H), 1.42 (d, *J=* 11.3 Hz, 3H), 1.33 (d, *J=* 11.6 Hz, 4H), 1.26 (s, 3H), 1.19 (s, 6H), 1.09 - 1.02 (m, 4H), 0.99 (s, 3H), 0.97 - 0.92 (m, 2H), 0.89 (d, *J=* 6.5 Hz, 3H), 0.63 (s, 3H), 0.61 - 0.52 (m, 1H).

¹³C NMR (101 MHz, Chloroform-d) δ 77.31, 76.99, 76.68, 74.80, 72.26, 71.13, 56.53, 56.13, 55.21, 50.87, 48.79, 44.38, 42.58, 39.87, 36.84, 36.39, 35.72, 35.46, 35.36, 32.37, 29.32, 29.16, 28.23, 25.95, 25.83, 24.18, 20.74, 20.56, 18.61, 14.65, 12.05. LCMS: [M-OH-H₂O]⁺ = 385.3.

### Example 203

### Preparation of cholest-5(6)-en-3β,4α,25-triol (compound 203)

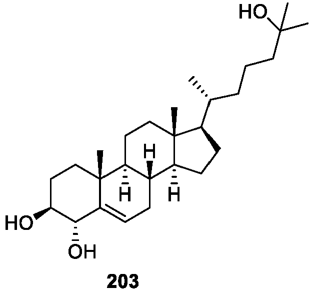

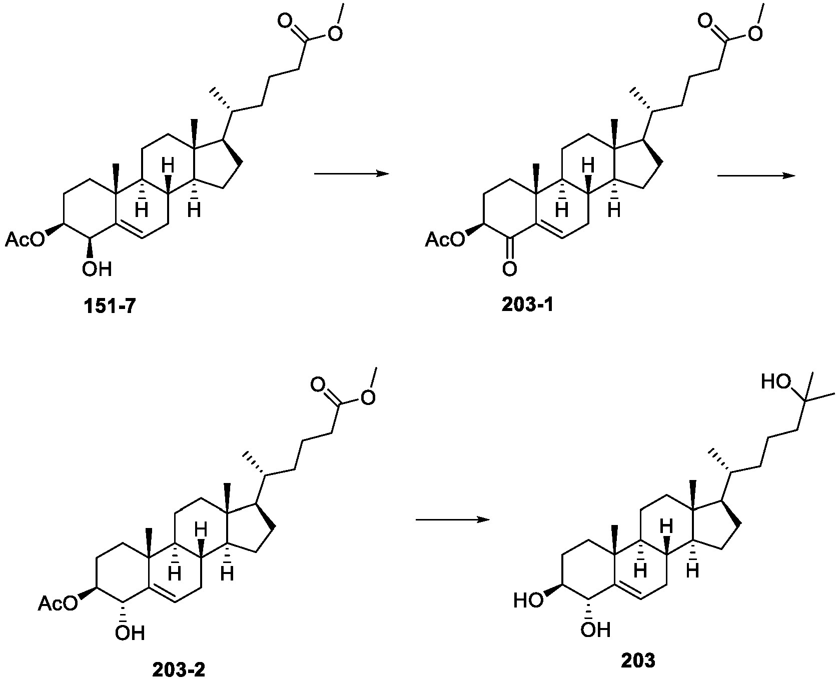

**Step 1:** Methyl (5*R*)-5-[(1*R*,3a*S*,3b*S*,6*R*,7*S*,9a*R*,9b*S*,11a*R*)-7-acetoxy-6-hydroxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate (**151-7**) (780 mg, 1.69 mmol, 1.0 *eq.*) was weighed and dissolved in dichloromethane (24 mL). Tetrapropylammonium perruthenate (30 mg, 0.09 mmol), N-methylmorpholine oxide (290 mg, 2.46 mmol), and 3A molecular sieve (3.9 g) were added thereto, and the reaction mixture was stirred at room temperature. The reaction was monitored by TLC (petroleum ether: ethyl acetate = 5:1). After the reaction was completed, the reaction mixture was filtered and concentrated to obtain a crude product, which was then purified by column chromatography (petroleum ether: ethyl acetate = 20:1 to 10:1) to obtain methyl (5*R*)-5-[(1*R*,3a*S*,3b*S*,7*S*,9a*R*,9b*S*,11a*R*)-7-acetoxy-9a,11a-dimethyl-6-oxo-2,3,3a,3b,4,6,7,8,9,9a,9b,10,11,11a-tetradecahydro-1*H*-cyclopenta[1,2-*a*]phenanthren-1-yl]hexanoate (**203-1**) (540 mg, 1.12 mmol, 66.06%) as a white solid.

¹H NMR (399 MHz, CDCl₃) δ 6.40 - 6.31 (m, 1H), 5.19 (dd, *J =* 12.6, 7.1 Hz, 1H), 3.65 (d, *J =* 0.8 Hz, 3H), 2.33 - 2.18 (m, 3H), 2.15 (d, *J=* 0.7 Hz, 4H), 2.13 - 2.06 (m, 1H), 2.05 - 1.97 (m, 3H), 1.83 (s, 1H), 1.70 (dd, *J=* 19.2, 10.0 Hz, 2H), 1.53 - 1.29 (m, 5H), 1.27 - 1.18 (m, 2H), 1.17 - 1.00 (m, 5H), 0.97 (s, 3H), 0.92 (d, *J=* 6.5 Hz, 3H), 0.66 (s, 3H).

**Step 2:** Methyl (5*R*)-5-[(1*R*,3a*S*,3b*S*,7*S*,9a*R*,9b*S*,11a*R*)-7-acetoxy-9a,11a-dimethyl-6-oxo-2,3,3a,3b,4,6,7,8,9,9a,9b,10,11,11a-tetradecahydro-1*H*-cyclopenta[1,2-*a*]phenanthren-1-yl]hexanoate (**203-1**) (80 mg, 0.17 mmol) was weighed and dissolved in tetrahydrofuran (10 mL), then sodium borohydride (13.2 mg, 0.35 mmol, 2.0 *eq.*) was added thereto at room temperature, and the reaction mixture was stirred at room temperature for 16 hours. The reaction was monitored by TLC (petroleum ether: ethyl acetate = 5:1). After the reaction was completed, the reaction mixture was added with water (10 mL), extracted with ethyl acetate (10 mL * 3), dried, and concentrated to obtain a crude product, which was then purified by column chromatography (petroleum ether: ethyl acetate = 20:1 to 10:1 to 5:1) to obtain methyl (5*R*)-5-[(1*R*,3a*S*,3b*S*,6*S*,7*S*,9a*R*,9b*S*,11a*R*)-7-acetoxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate (**203-2**) (41 mg, 0.085 mmol, 48.48%) as a white solid.

¹H NMR (399 MHz, CDCl₃) δ 5.87 - 5.80 (m, 1H), 4.48 (dt, *J =* 10.7, 5.3 Hz, 1H), 4.21 (d, *J=* 10.0 Hz, 1H), 3.65 (s, 3H), 2.30 - 2.18 (m, 2H), 2.09 (d, *J=* 1.4 Hz, 3H), 1.98 (d, *J=* 12.4 Hz, 1H), 1.92 - 1.78 (m, 3H), 1.72 - 1.62 (m, 2H), 1.52 (s, 1H), 1.43 (dt, *J=* 20.6, 5.6 Hz, 6H), 1.25 (d, *J =* 11.9 Hz, 2H), 1.17 - 1.02 (m, 5H), 1.00 (s, 3H), 0.91 (d, *J =* 6.5 Hz, 3H), 0.81 (s, 1H), 0.66 (s, 3H).

**Step 3:** Methyl (5*R*)-5-[(1*R*,3a*S*,3b*S*,6*S*,7*S*,9a*R*,9b*S*,11a*R*)-7-acetoxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate (**203-2**) (12 mg, 0.029 mmol) was weighed and dissolved in tetrahydrofuran (5 mL). The reaction mixture was cooled in a petroleum ether-dry ice bath, then added dropwise with a solution of methyllithium in tetrahydrofuran (C: 1.6 M, 0.29 mmol, 10.0 *eq.*) at low temperature, slowly warmed to room temperature, and stirred for 16 hours. The reaction was detected by TLC (dichloromethane: methanol = 20:1). After the reaction was completed, the reaction mixture was added with water (10 mL), extracted with ethyl acetate (10 mL * 3), dried, and concentrated to obtain a crude product, which was then purified by column chromatography (dichloromethane: methanol = 300:1 to 100:1) to obtain cholest-5(6)-en-3β,4α,25-triol (203) (5.3 mg, 0.011 mmol, 36.58%) as a white solid. ¹H NMR (399 MHz, CDCl₃) δ 5.71 (dt, *J =* 5.0, 2.2 Hz, 1H), 4.00 - 3.92 (m, 1H), 3.18 (td, *J =* 10.4, 9.6, 4.4 Hz, 1H), 2.07 - 1.92 (m, 2H), 1.80 (ddd, *J=* 18.9, 11.0, 4.2 Hz, 3H), 1.62 - 1.46 (m, 3H), 1.45 - 1.25 (m, 9H), 1.22 (d, *J=* 12.1 Hz, 3H), 1.15 (s, 6H), 1.09 - 0.99 (m, 4H), 0.97 (s, 3H), 0.92 (d, *J=* 6.2 Hz, 1H), 0.88 (d, *J=* 6.5 Hz, 3H), 0.63 (s, 3H). ¹³C NMR (100 MHz, Chloroform-d) δ 141.91, 117.78, 71.04, 56.66, 56.01, 49.87, 49.66, 49.44, 49.23, 49.02, 48.81, 44.25, 42.20, 39.66, 37.89, 36.39, 35.70, 31.50, 31.35, 28.20, 27.89, 24.21, 20.84, 20.73. LC-MS: [M+Na]⁺ = 441.5.

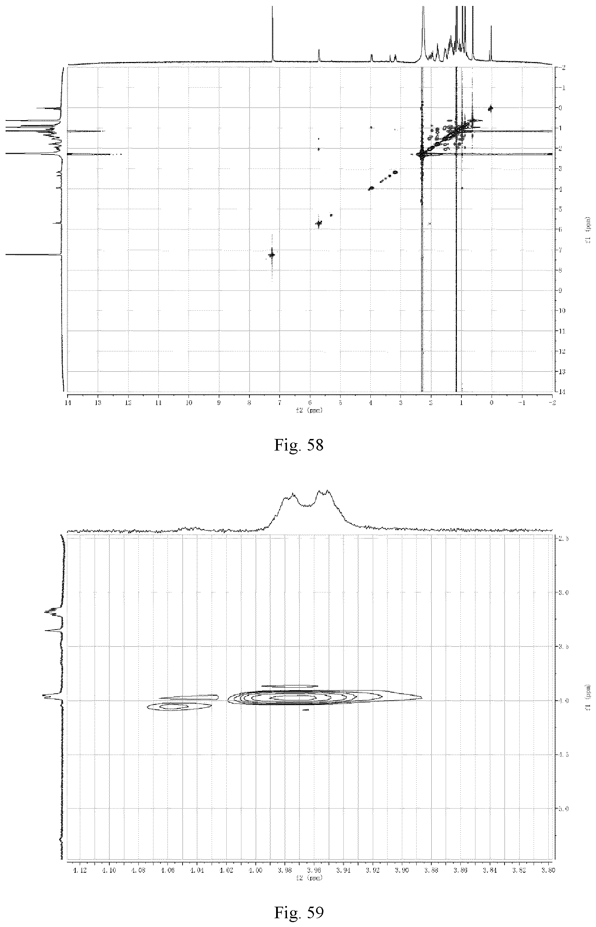

The two-dimensional spectrum of compound **203** is shown in Figs. 58 to 59.

### Example 204

### Preparation of 5α-cholest-3β,4α,25-triol (compound 204)

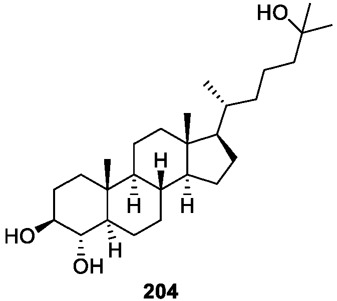

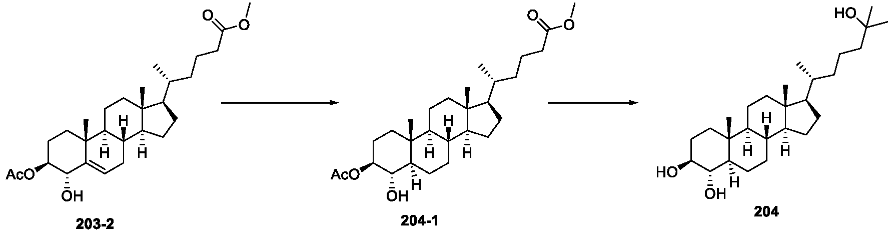

**Step 1:** (1*R*,3a*S*,3b*S*,6*S*,7*S*,9a*R*,9b*S*,11a*R*)-6-Hydroxy-1-[(2*R*)-6-hydroxyhexan-2-yl]-9a,11a-dimethyl-2,3,3a,3b,4,6,7,8,9,9a,9b,10,11,11a-tetradecahydro-1*H*-cyclopenta[1,2-i]phenanthren-7-yl acetate (**203-2**) (64 mg, 0.14 mmol, 1.0 *eq.*) was weighed and dissolved in glacial acetic acid (10 mL), and platinum dioxide (6.3 mg, 0.03 mmol) was added thereto. The reaction mixture was replaced with hydrogen, then heated to 40°C, and stirred. The reaction was monitored by TLC (petroleum ether: ethyl acetate = 5:1). After the reaction was completed, the reaction mixture was filtered and concentrated to obtain methyl (5*R*)-5-[(1*R*,3a*S*,3b*S*,5a*R*,6*S*,7*S*,9a*R*,9b*S*,11a*R*)-7-acetoxy-6-hydroxy-9a,11a-dimethylhexadecahydro-1*H*-cyclopenta[1,2-*a*]phenanthren-1-yl]hexanoate (**204-1**) (80 mg, 0.14 mmol, 99.56%) as a white solid crude product, which was directly used in the next reaction step without purification.

**Step 2:** Methyl (5*R*)-5-[(1*R*,3a*S*,3b*S*,5a*R*,6*S*,7*S*,9a*R*,9b*S*,11a*R*)-7-acetoxy-6-hydroxy-9a,11a-dimethylhexadecahydro-1*H*-cyclopenta[1,2-*a*]phenanthren-1-yl]hexanoate (**204-1**) (60 mg, 0.14 mmol) was weighed and dissolved in tetrahydrofuran (5 mL). The reaction mixture was cooled in a petroleum ether-dry ice bath, then added dropwise with a solution of methyllithium in tetrahydrofuran (C: 1.6 M, 1.3 mmol, 10.0 *eq.*) at low temperature, warmed to room temperature, and stirred for 16 hours. The reaction was detected by TLC (dichloromethane: methanol = 20:1). After the reaction was completed, the reaction mixture was added with saturated sodium bicarbonate aqueous solution (10 mL), extracted with ethyl acetate (10 mL * 3), dried, and concentrated to obtain a crude product, which was then purified by column chromatography (dichloromethane: methanol = 300:1 to 100:1) to obtain 5α-cholest-3β,4α,25-triol (**204**) (12 mg, 0.026 mmol, 19.98%) as a white solid. ¹H NMR (399 MHz, CDCl₃) δ 3.38 - 3.23 (m, 2H), 1.95 (d, *J=* 12.8 Hz, 1H), 1.84 (d, *J=* 13.1 Hz, 2H), 1.78 - 1.73 (m, 1H), 1.73 - 1.64 (m, 2H), 1.49 - 1.39 (m, 5H), 1.36 (t, *J=* 6.7 Hz, 4H), 1.30 - 1.25 (m, 3H), 1.24 (d, *J =* 3.2 Hz, 4H), 1.19 (s, 6H), 1.15 - 1.09 (m, 2H), 1.07 - 1.03 (m, 3H), 0.98 (d, *J=* 10.6 Hz, 2H), 0.89 (d, *J=* 6.5 Hz, 3H), 0.84 (s, 1H), 0.82 (s, 3H), 0.69 - 0.64 (m, 1H), 0.63 (s, 3H). ¹³C NMR (100 MHz, CDCl₃ + 10% CD₃OD) δ 76.05, 75.07, 70.93, 56.30, 56.12, 54.34, 50.60, 49.67, 49.46, 49.24, 49.03, 48.82, 48.60, 48.39, 44.16, 42.42, 39.85, 37.07, 36.12, 35.68, 34.92, 28.15, 28.03, 24.04, 22.54, 20.85, 18.46, 11.91. LCMS: [M+Na]⁺ = 443.55.

### Example 205

### Preparation of compound cholest-3β,5,25-triol

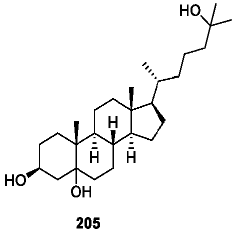

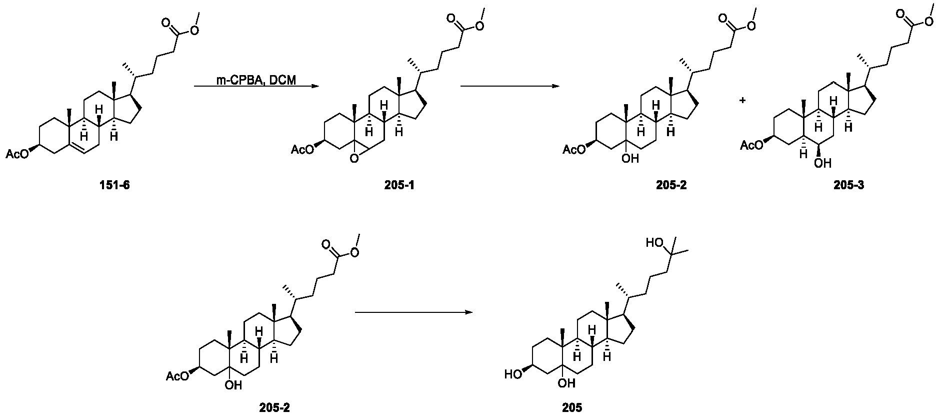

**Step 1:** (6*R*,7*S*,9a*R*,11a*R*)-6-Hydroxy-1-[(2*R*)-6-methoxy-6-oxohexan-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate (**151-6**) (800 mg, 1.8 mmol, 1.0 *eq.*) was weighed and dissolved in dichloromethane (50 mL), then *m*-chloroperoxybenzoic acid (541.41 mg, 2.7 mmol, 1.5 *eq.*) was added thereto in an ice bath, and the reaction mixture was stirred at room temperature for 4 hours. The reaction was monitored by TLC (petroleum ether: ethyl acetate = 5:1). After the reaction was completed, the reaction mixture was added with saturated sodium bicarbonate aqueous solution (30 mL) and extracted with dichloromethane (40 mL * 3). The organic phase was dried and concentrated to obtain a crude product, which was then purified by column chromatography (petroleum ether: ethyl acetate = 100:1 to 5:1) to obtain methyl (5*R*)-5-[(3*S*,6a*S*,6b*S*,9a*R*,9*R*,11a*S*,11b*R*)-3-acetyloxy-9a,11b-dimethyl-1,2,3,4,5a,6,6a,6b,7,8,9,9a,10,11,11a,11b-hexadecahydrocyclopenta[1',2':1,2]phenanthro[8a,9-*b*]oxazepin-9-yl]hexanoate (**205-1**) (600 mg, 1.34 mmol, 74.54%) as a white solid. ¹H NMR (399 MHz, CDCl₃) δ 4.97 - 4.87 (m, 1H), 2.87 (d, *J=* 4.4 Hz, 1H), 2.31 - 2.17 (m, 2H), 2.00 (d, *J=* 6.4 Hz, 3H), 1.96 - 1.86 (m, 2H), 1.77 (ddd, *J=* 14.8, 9.3, 5.6 Hz, 1H), 1.70 - 1.58 (m, 2H), 1.52 - 1.41 (m, 3H), 1.33 (ddd, *J=* 12.8, 9.8, 6.1 Hz, 4H), 1.25 - 1.14 (m, 3H), 1.04 (s, 3H), 0.98 - 0.92 (m, 2H), 0.88 (dd, *J* = 6.5, 1.6 Hz, 3H), 0.59 (d, *J=* 11.7 Hz, 3H).

**Step 2:** Methyl (5*R*)-5-[(3*S*,6a*S*,6b*S*,9a*R*,9*R*,11a*S*,11b*R*)-3-acetyloxy-9a,11b-dimethyl-1,2,3,4,5a,6,6a,6b,7,8,9,9a,10,11,11a,11b-hexadecahydrocyclopenta[1',2':1,2]phenanthro[8a,9-*b*]oxazepin-9-yl]hexanoate (100 mg, 0.22 mmol, 1.0 *eq.*) was weighed and dissolved in tetrahydrofuran (10 mL), and bromocresol green (2.4 mg, 0.22 mmol) and sodium cyanoborohydride (66 mg, 1.06 mmol, 5.0 *eq.*) were added thereto at room temperature. The reaction mixture was heated to 50°C under a nitrogen atmosphere, and boron trifluoride diethyl etherate (0.11 mmol) was added thereto with stirring. The color of the mixture changed from green to yellow. Boron trifluoride diethyl etherate was then added dropwise thereto so that sodium cyanoborohydride was slowly consumed. The reaction mixture was heated to reflux overnight. The reaction was monitored by TLC (petroleum ether: ethyl acetate = 5:1). After the reaction was completed, the reaction mixture was cooled to room temperature, added with water (10 mL), extracted with ethyl acetate (10 mL * 3), dried, and concentrated to obtain a crude product, which was then purified by column chromatography (petroleum ether: ethyl acetate = 100:1 to 30:1 to 5:1) to obtain methyl (5*R*)-5-[(1*R*,3a*S*,3b*S*,7*S*,9a*R*,9b*S*,11a*R*)-7-acetoxy-5a-hydroxy-9a,11a-dimethylhexadecahydro-1*H-*cyclopenta[1,2-*a*]phenanthren-1-yl]hexanoate (**205-2, first component**) (30 mg, 0.058 mmol, 26.88%) as a white solid and methyl (5*R*)-5-[(1*R*,3a*S*,3b*S*,7*S*,9a*R*,9b*S*,11a*R*)-7-acetoxy-5-hydroxy-9a,11a-dimethylhexadecahydro-1*H*-cyclopenta[1,2-*a*]phenanthren-1-yl]hexanoate **(205-3, second component)** (30 mg, 0.058 mmol, 26.88%).

**205-2** (**first component**): ¹H NMR (400 MHz, CDCl₃) δ 4.77 - 4.66 (m, 1H), 3.78 (d, *J =* 3.1 Hz, 1H), 3.66 (s, 3H), 2.37 - 2.18 (m, 2H), 2.02 (s, 3H), 1.97 (d, *J =* 12.6 Hz, 1H), 1.84 - 1.75 (m, 4H), 1.72 - 1.64 (m, 4H), 1.52 - 1.45 (m, 3H), 1.43 - 1.31 (m, 4H), 1.29 - 1.20 (m, 6H), 1.15 - 1.07 (m, 4H), 1.03 (s, 3H), 1.01 - 0.95 (m, 2H), 0.91 (d, *J =* 6.5 Hz, 3H), 0.89 - 0.82 (m, 1H), 0.67 (s, 3H).

**205-3** (**second component**): ¹H NMR (400 MHz, CDCl₃) δ 5.15 (s, 1H), 3.65 (s, 3H), 2.28 (ddt, *J=* 25.9, 16.3, 7.8 Hz, 2H), 2.01 (s, 3H), 1.95 (d, *J =* 12.2 Hz, 1H), 1.90 - 1.75 (m, 2H), 1.68 (d, *J=* 8.0 Hz, 4H), 1.64 - 1.52 (m, 7H), 1.48 - 1.33 (m, 8H), 1.24 (s, 10H), 1.06 (q, *J=* 9.8 Hz, 4H), 0.98 (s, 3H), 0.91 (d, *J =* 6.4 Hz, 3H), 0.88 - 0.79 (m, 1H), 0.63 (s, 3H).

**Step 3:** Methyl (5*R*)-5-[(1*R*,3a*S*,3b*S*,7*S*,9a*R*,9b*S*,11a*R*)-7-acetoxy-5a-hydroxy-9a,11a-dimethylhexadecahydro-1*H*-cyclopenta[1,2-*a*]phenanthren-1-yl]hexanoate (**205-2**) (30 mg, 0.06 mmol) was weighed and dissolved in tetrahydrofuran (5 mL). The reaction mixture was cooled in a petroleum ether-dry ice bath, then added dropwise with a solution of methyllithium in tetrahydrofuran (C: 1.6 M, 0.6 mmol, 10.0 *eq.*) at low temperature, warmed to room temperature, and stirred for 16 hours. The reaction was monitored by TLC (dichloromethane: methanol = 20:1). After the reaction was completed, the reaction mixture was added with water (10 mL), extracted with ethyl acetate (10 mL * 3), dried, and concentrated to obtain a crude product, which was then purified by column chromatography (dichloromethane: methanol = 300:1 to 100:1) to obtain cholest-3β,5,25-triol (**205**) (6.7 mg, 0.015 mmol, 23.06%) as a white solid.

¹H NMR (399 MHz, CDCl₃) δ 4.08 (dd, *J=* 10.9, 5.9 Hz, 1H), 1.96 (d, *J=* 12.4 Hz, 1H), 1.83 (d, *J=* 11.4 Hz, 2H), 1.69 - 1.52 (m, 5H), 1.42 (dd, *J=* 25.4, 14.2 Hz, 12H), 1.30 - 1.21 (m, 7H), 1.19 (s, 6H), 1.06 (d, *J=* 21.4 Hz, 5H), 0.97 (s, 3H), 0.89 (d, *J=* 6.4 Hz, 3H), 0.63 (s, 3H). ¹³C NMR (100 MHz, CDCl₃) δ 77.31, 76.99, 76.68, 75.32, 71.10, 67.34, 56.14, 56.13, 45.87, 44.38, 43.85, 42.70, 39.98, 38.76, 36.39, 35.74, 34.68, 34.36, 30.85, 30.77, 29.31, 29.19, 28.24, 25.91, 24.06, 21.32, 20.79, 18.59, 16.23, 12.11, 0.99. LCMS: [M-H₂O-OH]⁺ = 385.

### Example 206

### Preparation of compound cholest-3β,6,25-triol

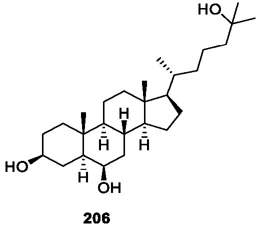

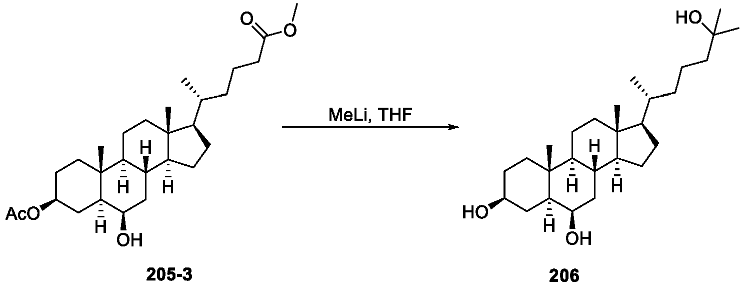

Methyl (5*R*)-5-[(1*R*,3a*S*,3b*S*,7*S*,9a*R*,9b*S*,11a*R*)-7-acetoxy-5-hydroxy-9a,11a-dimethylhexadecahydro-1*H*-cyclopenta[1,2-*a*]phenanthren-1-yl]hexanoate (**205-3**) (50 mg, 0.11 mmol) was weighed and dissolved in tetrahydrofuran (5 mL). The reaction mixture was cooled in a petroleum ether-dry ice bath, then added dropwise with a solution of methyllithium in tetrahydrofuran (C: 1.6 M, 1.1 mmol, 10.0 *eq.*) at low temperature, warmed to room temperature, and stirred for 16 hours. The reaction was monitored by TLC (dichloromethane: methanol = 20:1). After the reaction was completed, the reaction mixture was added with water (10 mL), extracted with ethyl acetate (10 mL * 3), dried, and concentrated to obtain a crude product, which was then purified by column chromatography (dichloromethane: methanol = 300:1 to 100:1) to obtain cholest-3β,6,25-triol (**206**) (8.2 mg, 0.019 mmol, 17.59%) as a white solid. ¹H NMR (399 MHz, CDCl₃) δ 3.78 (s, 1H), 3.63 (s, 1H), 1.97 (d, *J =* 12.6 Hz, 1H), 1.78 (d, *J* = 14.2 Hz, 3H), 1.66 (dd, *J =* 23.5, 12.0 Hz, 5H), 1.50 (d, *J =* 28.4 Hz, 6H), 1.42 - 1.29 (m, 8H), 1.23 (s, 5H), 1.19 (s, 6H), 1.10 (s, 4H), 1.01 (s, 3H), 0.90 (d, *J=* 6.2 Hz, 3H), 0.67 (s, 3H). ¹³C NMR (101 MHz, CDCl₃) δ 77.32, 77.00, 76.68, 72.07, 71.71, 71.12, 56.17, 56.15, 54.20, 47.33, 44.39, 42.67, 39.90, 39.59, 38.49, 36.40, 35.72, 35.36, 31.49, 30.35, 29.70, 29.36, 29.17, 28.21, 24.21, 21.04, 20.74, 18.64, 15.79, 12.10, 1.01. LCMS: [M-H₂O-OH]⁺ = 385.

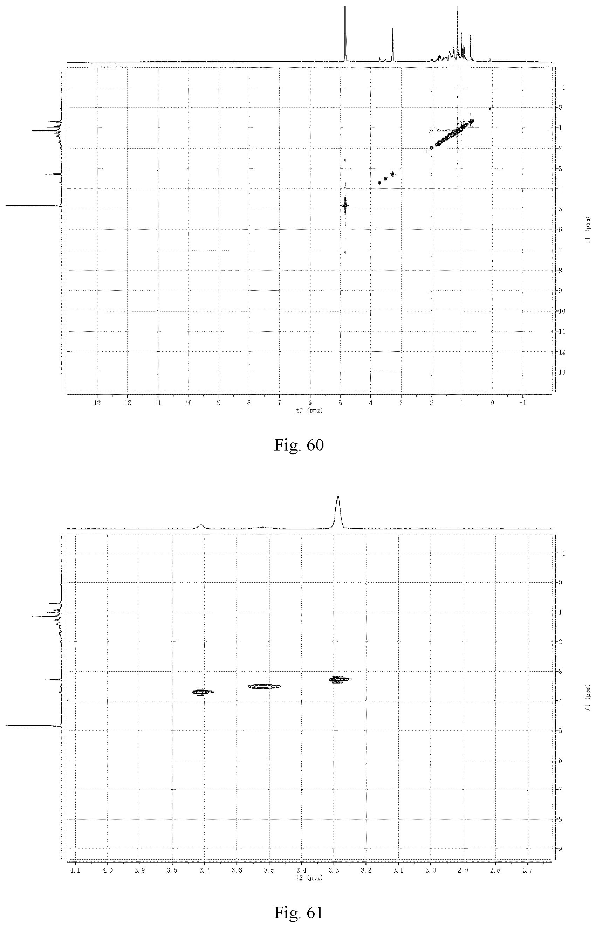

The two-dimensional spectrum of compound **206** is shown in Figs. 60 to 61.

### Example 207

### Preparation of compound cholest-3β,5,6,25-tetraol

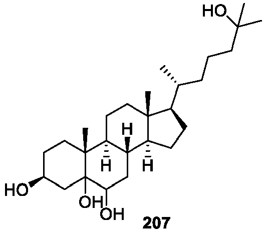

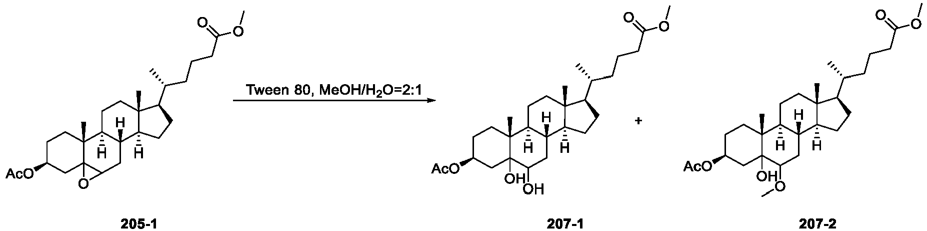

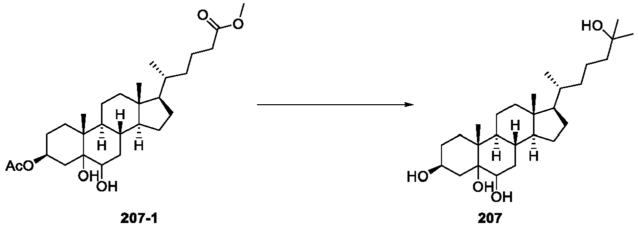

**Step 1:** Methyl (5*R*)-5-[(3*S*,6a*S*,6b*S*,9a*R*,9*R*,11a*S*,11b*R*)-3-acetyloxy-9a,11b-dimethyl-1,2,3,4,5a,6,6a,6b,7,8,9,9a,10,11,11a,11b-hexadecahydrocyclopenta[1',2':1,2]phenanthro[8a,9-*b*]oxazepin-9-yl]hexanoate (**205-1**) (100 mg, 0.22 mmol, 1.0 *eq.*) was weighed and dissolved in a mixed solvent of methanol (8 mL) and water (4.0 mL), and Tween 80 (50 mg, 0.22 mmol) was added thereto. The system was then heated to 100°C and stirred overnight. The reaction was monitored by TLC (dichloromethane: methanol = 10:1). After the reaction was completed, the reaction mixture was cooled to room temperature, filtered, concentrated, diluted with water (10 mL), and extracted with ethyl acetate (10 mL * 3). The organic phase was dried and concentrated to obtain a crude product, which was then purified by column chromatography (dichloromethane: methanol = 100:1 to 10:1) to obtain methyl (5*R*)-5-[(1*R*,3a*S*,3b*S*,7*S*,9a*R*,9b*S*,11a*R*)-7-acetoxy-5,5a-dihydroxy-9a,11a-dimethylhexadecahydro-1*H*-cyclopenta[1,2-*a*]phenanthren-1-yl]hexanoate (**207-1, second component**) (20 mg, 0.04 mmol, 17.33%) as a white solid and methyl (5*R*)-5-[(1*R*,3a*S*,3b*S*,7*S*,9a*R*,9b*S*,11a*R*)-7-acetoxy-5a-hydroxy-5-methoxy-9a,11a-dimethylhexadecahydro-1*H*-cyclopenta[1,2-*a*]phenanthren-1-yl]hexanoate (**207-2, first component**) (30 mg, 0.06 mmol, 25.25%).

**207-1 (second component**): ¹H NMR (399 MHz, CDCl₃) δ 5.14 (q, *J=* 5.6 Hz, 1H), 3.64 (s, 3H), 3.50 (s, 1H), 2.25 (ddd,*J =* 9.9, 8.6, 6.7 Hz, 2H), 2.15 (dd, *J =* 12.9, 11.3 Hz, 1H), 2.00 (s, 3H), 1.96 (d, *J* = 12.1 Hz, 1H), 1.81 (dd, *J* = 13.6, 7.8 Hz, 2H), 1.71 - 1.58 (m, 7H), 1.57 - 1.44 (m, 4H), 1.42 - 1.31 (m, 5H), 1.25 (dt, *J =* 18.8, 4.2 Hz, 4H), 1.16 (s, 3H), 1.06 (q, *J=* 10.4, 10.0 Hz, 4H), 0.90 (d, *J =* 6.5 Hz, 3H), 0.86 - 0.77 (m, 1H), 0.65 (s, 3H).

**207-2** (**first component**): ¹H NMR (399 MHz, CDCl₃) δ 5.10 (dt, *J =* 11.0, 5.6 Hz, 1H), 3.64 (s, 3H), 3.24 (s, 3H), 2.91 (d, *J* = 2.8 Hz, 1H), 2.33 - 2.12 (m, 3H), 2.00 (s, 3H), 1.94 (d, *J =* 12.5 Hz, 1H), 1.86 - 1.66 (m, 5H), 1.61 - 1.49 (m, 5H), 1.46 - 1.30 (m, 7H), 1.30 - 1.16 (m, 5H), 1.05 (d, *J =* 11.3 Hz, 6H), 0.89 (d, *J =* 6.4 Hz, 3H), 0.85 - 0.74 (m, 1H), 0.63 (s, 3H).

**Step 2:** Methyl (5*R*)-5-[(1*R*,3a*S*,3b*S*,7*S*,9a*R*,9b*S*,11a*R*)-7-acetoxy-5,5a-dihydroxy-9a,11a-dimethylhexadecahydro-1*H*-cyclopenta[1,2-*a*]phenanthren-1-yl]hexanoate (**207-1**) (50 mg, 0.11 mmol) was weighed and dissolved in tetrahydrofuran (5 mL). The reaction mixture was cooled in a petroleum ether-dry ice bath, then added dropwise with a solution of methyllithium in tetrahydrofuran (C: 1.6 M, 1.1 mmol, 10.0 *eq.*) at low temperature, warmed to room temperature, and stirred for 16 hours. The reaction was detected by TLC (dichloromethane: methanol = 20:1). After the reaction was completed, the reaction mixture was added with water (10 mL), extracted with ethyl acetate (10 mL * 3), dried, and concentrated to obtain a crude product, which was then purified by column chromatography (dichloromethane: methanol = 300:1 to 100:1) to obtain cholest-3β,5,6,25-tetraol (**207**) (8.2 mg, 0.017 mmol, 16.05%) as a white solid. ¹H NMR (399 MHz, CD₃OD) δ 3.98 (dd, *J* = 11.3, 5.6 Hz, 1H), 3.43 (s, 1H), 2.11 - 1.96 (m, 2H), 1.83 (s, 1H), 1.71 (dd, *J=* 19.9, 11.4 Hz, 3H), 1.63 - 1.47 (m, 5H), 1.37 (dd, *J =* 22.6, 10.3 Hz, 9H), 1.27 (q, *J =* 9.5, 7.9 Hz, 5H), 1.14 (d, *J* = 3.7 Hz, 10H), 1.06 (d, *J =* 21.4 Hz, 4H), 0.93 (d, *J =* 6.4 Hz, 3H), 0.88 (s, 1H), 0.70 (s, 3H). ¹³C NMR (101 MHz, CD₃OD) δ 141.04, 75.38, 75.07, 70.05, 56.04, 48.20, 48.06, 47.99, 47.84, 47.78, 47.63, 47.56, 47.42, 47.35, 47.26, 47.13, 47.05, 46.92, 45.13, 43.87, 42.50, 40.07, 37.86, 35.72, 33.83, 30.18, 27.82, 27.67, 23.78, 20.86, 17.77, 15.89, 11.16. LC-MS: [M-OH-OH]⁺ = 402.

### Example 208

### Preparation of compound 6-methoxycholest-3β,5,25-triol

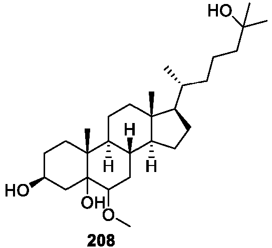

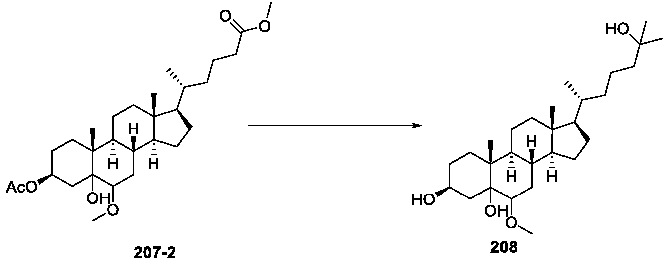

Methyl (5*R*)-5-[(1*R*,3a*S*,3b*S*,7*S*,9a*R*,9b*S*,11a*R*)-7-acetoxy-5a-hydroxy-5-methoxy-9a,11a-dimethylhexadecahydro-1*H*-cyclopenta[1,2-*a*]phenanthren-1-yl]hexanoate (**207-2**) (50 mg, 0.06 mmol) was weighed and dissolved in tetrahydrofuran (5 mL). The reaction mixture was cooled in a petroleum ether-dry ice bath, then added dropwise with a solution of methyllithium in tetrahydrofuran (C: 1.6 M, 0.6 mmol, 10.0 *eq.*) at low temperature, warmed to room temperature, and stirred for 16 hours. The reaction was monitored by TLC (DCM: MeOH = 20: 1). After the reaction was completed, the reaction mixture was added with water (10 mL), extracted with ethyl acetate (10 mL * 3), dried, and concentrated to obtain a crude product, which was then purified by column chromatography (dichloromethane: methanol = 300:1 to 100:1) to obtain 6-methoxycholest-3β,5,25-triol (**208**) (8.2 mg, 0.018 mmol, 16.36%) as a white solid.

¹H NMR (399 MHz, CDCl₃) δ 4.05 (dd, *J =* 11.4, 5.9 Hz, 1H), 3.65 (d, *J =* 6.0 Hz, 1H), 3.26 (s, 3H), 2.93 (s, 1H), 2.10 (t, *J=* 12.3 Hz, 1H), 1.96 (d, *J* = 12.9 Hz, 1H), 1.82 - 1.64 (m, 4H), 1.62 - 1.45 (m, 8H), 1.41 - 1.30 (m, 7H), 1.26 (d, *J =* 2.5 Hz, 3H), 1.19 (s, 9H), 1.07 (s, 3H), 0.90 (d, *J=* 6.5 Hz, 3H), 0.82 (s, 2H), 0.65 (s, 3H). ¹³C NMR (100 MHz, CDCl₃) δ 85.56, 77.30, 77.16, 76.99, 76.86, 76.67, 76.30, 71.11, 70.67, 67.68, 57.98, 57.94, 56.13, 55.94, 45.94, 44.38, 42.72, 40.98, 39.92, 38.47, 36.39, 35.74, 32.08, 30.87, 30.50, 29.68, 29.30, 28.22, 27.48, 24.13, 21.10, 20.78, 18.60, 16.34, 12.14, 1.01. LC-MS: [M-H₂O-OH]⁺ = 415.

### Example 210

### Preparation of 5α-cholest-3β,7,25-triol (compound 210)

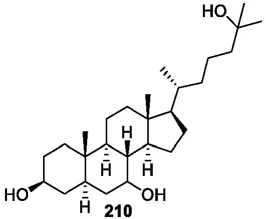

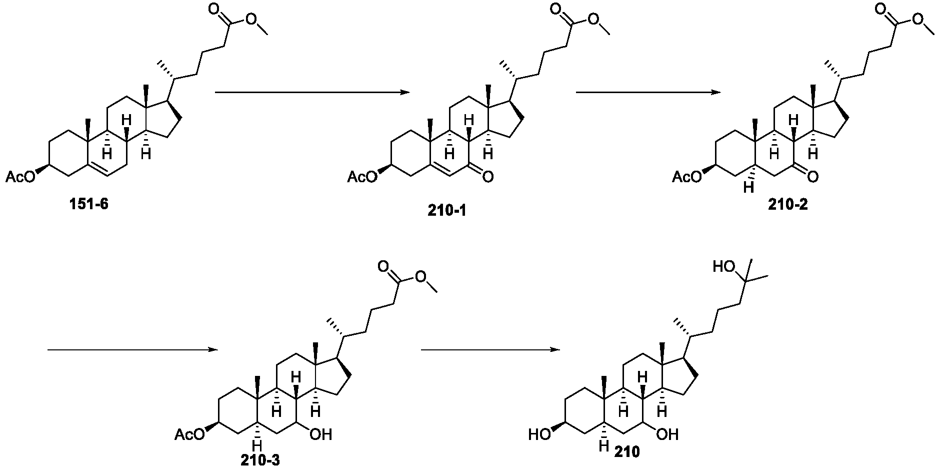

**Step 1:** Methyl (5*R*)-5-[(1*R*,3a*S*,3b*S*,7*S*,9a*R*,9b*S*,11a*R*)-7-acetoxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate (**151-6**) (500 mg, 1.12 mmol, 1.0 *eq.*) was weighed and dissolved in acetone (50 mL). Cobalt acetate (9.95 mg, 0.056 mmol), N-hydroxyphthalimide (18.34 mg, 0.11 mmol), and tert-butyl hydroperoxide (405.34 mg, 4.5 mmol) were added thereto, and the reaction mixture was stirred at room temperature. The reaction was monitored by TLC (petroleum ether: ethyl acetate = 5:1). After the reaction was completed, the reaction mixture was filtered and concentrated to obtain a crude product, which was then purified by column chromatography (petroleum ether: ethyl acetate = 50:1 to 30:1 to 10:1) to obtain methyl (5*R*)-5-[(1*R*,3a*S*,3b*S*,7*S*,9a*R*,9b*S*,11a*R*)-7-acetoxy-9a,11a-dimethyl-4-oxo-2,3,3a,3b,4,6,7,8,9,9a,9b,10,11,11a-tetradecahydro-1*H*-cyclopenta[1,2-*a*]phenanthren-1-yl]hexanoate (**210-1**) (240 mg, 0.47 mmol, 41.88%) as a white solid. ¹H NMR (399 MHz, Chloroform-d) δ 5.68 (d, *J =* 1.8 Hz, 1H), 4.76 - 4.63 (m, 1H), 3.65 (s, 3H), 2.58 - 2.32 (m, 3H), 2.23 (dq, *J=* 18.1, 10.4, 9.2 Hz, 3H), 2.03 (s, 3H), 1.97 (d, *J* = 10.9 Hz, 2H), 1.66 (d, *J* = 12.2 Hz, 2H), 1.51 (s, 3H), 1.35 (d, *J =* 16.7 Hz, 3H), 1.23 (s, 5H), 1.19 (s, 3H), 1.07 (q, *J =* 8.9 Hz, 4H), 0.92 (d, *J=* 6.5 Hz, 3H), 0.66 (s, 3H).

**Step 2:** Methyl (5*R*)-5-[(1*R*,3a*S*,3b*S*,7*S*,9a*R*,9b*S*,11a*R*)-7-acetoxy-9a,11a-dimethyl-4-oxo-2,3,3a,3b,4,6,7,8,9,9a,9b,10,11,11a-tetradecahydro-1*H*-cyclopenta[1,2-*a*]phenanthren-1-yl]hexanoate (**210-1**) (64 mg, 0.14 mmol) was weighed and dissolved in methanol (15 mL), and Pd/C (1.49 mg, 0.014 mmol, 0.1 *eq.*) was added thereto at room temperature. The reaction mixture was replaced with hydrogen and then stirred at room temperature for 18 hours. The reaction was monitored by TLC (petroleum ether: ethyl acetate = 5:1). After the reaction was completed, the reaction mixture was filtered to remove Pd/C. The organic phase was dried and purified by column chromatography (petroleum ether: ethyl acetate = 30:1 to 20:1 to 10:1) to obtain methyl (5*R*)-5-[(1*R*,3a*S*,3b*R*,5a*R*,7*S*,9a*S*,9b*S*,11a*R*)-7-acetoxy-9a,11a-dimethyl-4-oxohexadecahydro-1*H*-cyclopenta[1,2-*a*]phenanthren-1-yl]hexanoate (**210-2**) (57 mg, 0.1 mmol, 70.94%) as a white solid. ¹H NMR (400 MHz, CDCl₃) δ 4.66 (dt, *J* = 11.1, 5.9 Hz, 1H), 3.66 (s, 3H), 2.39 - 2.22 (m, 4H), 2.22 - 2.15 (m, 1H), 2.03 (d, *J=* 3.0 Hz, 1H), 2.01 (s, 3H), 1.97 (d, *J=* 12.9 Hz, 1H), 1.86 (s, 2H), 1.78 (d, *J=* 13.4 Hz, 1H), 1.71 - 1.58 (m, 3H), 1.55 - 1.44 (m, 5H), 1.39 (dd, *J* = 11.6, 6.8 Hz, 3H), 1.22 (d, *J* = 17.8 Hz, 2H), 1.12 (d, *J* = 6.9 Hz, 1H), 1.08 (s, 5H), 1.05 (d, *J* = 4.0 Hz, 2H), 0.92 (d, *J* = 6.5 Hz, 3H), 0.64 (s, 3H).

**Step 3:** Methyl (5*R*)-5-[(1*R*,3a*S*,3b*R*,5a*R*,7*S*,9a*S*,9b*S*,11a*R*)-7-acetoxy-9a,11a-dimethyl-4-oxohexadecahydro-1*H*-cyclopenta[1,2-*a*]phenanthren-1-yl]hexanoate (**210-2**) (50 mg, 0.11 mmol) was weighed and dissolved in a mixed solvent of tetrahydrofuran (8 mL) and methanol (8 mL). The reaction mixture was cooled in an ice bath, then added with sodium borohydride (8.21 mg, 0.22 mmol) at 0°C, warmed to room temperature, and stirred. The reaction was monitored by TLC (petroleum ether: ethyl acetate = 5:1). After the reaction was completed, the reaction mixture was quenched with saturated sodium bicarbonate aqueous solution and extracted with ethyl acetate. The organic phase was separated, dried, and concentrated to obtain methyl (5*R*)-5-[(1*R*,3a*S*,3b*S*,5a*R*,6*S*,7*S*,9a*R*,9b*S*,11a*R*)-7-acetoxy-6-hydroxy-9a,11a-dimethylhexadecahydro-1*H*-cyclopenta[1,2-*a*]phenanthren-1-yl]hexanoate **(210-3)** (40 mg, 0.07 mmol, 63.72%) as a white solid, which was directly used in the next reaction step without purification.

**Step 4:** Methyl (5*R*)-5-[(1*R*,3a*S*,3b*S*,5a*R*,6*S*,7*S*,9a*R*,9b*S*,11a*R*)-7-acetoxy-6-hydroxy-9a,11a-dimethylhexadecahydro-1*H*-cyclopenta[1,2-*a*]phenanthren-1-yl]hexanoate (**210-3**) (40 mg, 0.09 mmol) was weighed and dissolved in tetrahydrofuran (10 mL). The reaction mixture was cooled in a dry ice-acetone bath, and methyllithium (1.6 M, 18.9 mg, 0.86 mmol) was added dropwise thereto at -78°C. After the dropwise addition was completed, the reaction mixture was warmed to room temperature. The reaction was monitored by TLC (dichloromethane: methanol = 20:1). After the reaction was completed, the reaction mixture was quenched with saturated sodium bicarbonate aqueous solution and extracted with ethyl acetate. The organic phase was separated, dried, and concentrated to obtain a crude product, which was then purified by column chromatography (dichloromethane: methanol = 100:1 to 30:1 to 10:1) to obtain 5α-cholest-3β,7,25-triol (**210**) (10.54 mg, 0.023 mmol, 26.44%) as a white solid. ¹H NMR (399 MHz, CDCl₃) δ 3.70 - 3.52 (m, 1H), 2.02 - 1.90 (m, 1H), 1.79 (d, *J=* 11.9 Hz, 2H), 1.69 (d, *J* = 13.8 Hz, 3H), 1.49 (s, 2H), 1.46 - 1.35 (m, 7H), 1.33 - 1.22 (m, 9H), 1.19 (s, 6H), 1.16 - 0.98 (m, 5H), 0.95 - 0.87 (m, 3H), 0.80 (d, *J=* 10.2 Hz, 3H), 0.65 (d, *J =* 8.5 Hz, 3H). ¹³C NMR (100 MHz, CDCl₃) δ 71.10, 50.55, 43.60, 42.65, 39.54, 37.69, 35.53, 34.91, 31.38. LC-MS: [M-2H₂O-OH]⁺ = 367.5.

### Example 211

### Preparation of (1R,3aS,5aS,7S,9aS,11aR)-1-[(2R)-6-hydroxyoctan-2-yl]-3a,9a,11a-trimethylhexadecahydro-1H-cyclopenta[1,2-a]phenanthren-7-ol (compound 211)

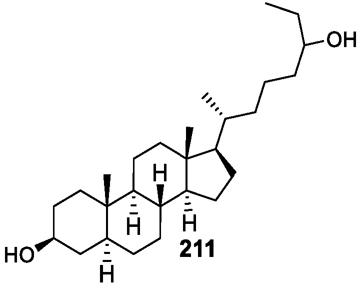

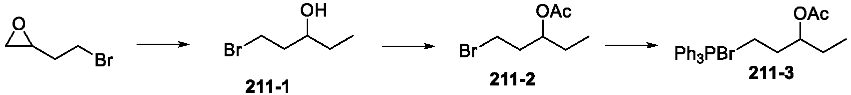

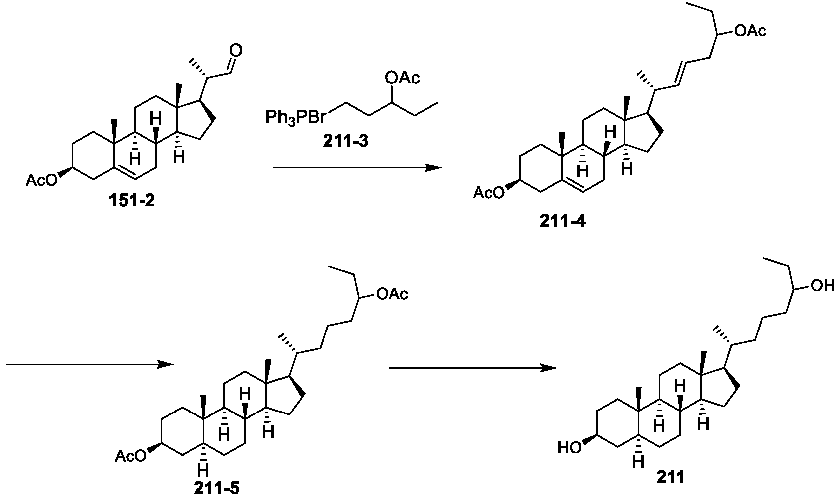

**Step 1:** Cuprous iodide (355 mg, 2.98 mmol, 1.0 *eq*) was dissolved in THF (tetrahydrofuran) (7 mL). The reaction system was cooled to about -30°C, slowly added with methylmagnesium bromide (355.35 mg, 2.980 mmol, 1.5 *eq*, 3 M in ether), and stirred at the same temperature for 20 minutes. 2-(2-Bromoethyl)oxirane (300 mg, 1.987 mmol, 0.2 *eq*) was then slowly added thereto, and the reaction system was stirred at 0°C for 2 hours. The reaction was monitored by TLC (petroleum ether: ethyl acetate = 5:1). After the reaction was completed, the reaction mixture was extracted with ethyl acetate (25 mL * 3). The organic phase was collected, dried over anhydrous sodium sulfate, and evaporated to dryness by rotary evaporation under vacuum to obtain a crude product, which was then purified by flash chromatography (petroleum ether: ethyl acetate = 92:8 to 90:10) to obtain 1-bromopentan-3-ol **(211-1)** (200 mg, purity: 90.0%, yield: 54.23%) as a yellow oil. ¹H NMR (400 MHz, CDCl₃) δ 3.80 - 3.71 (m, 1H), 3.62 - 3.51 (m, 2H), 2.08 - 1.89 (m, 2H), 1.59 - 1.45 (m, 2H), 0.97 (t, *J =* 7.4 Hz, 3H).

**Step 2:** 1-Bromopentan-3-ol (**211-1**) (200 mg, 1.197 mmol, 1.0 *eq*) was dissolved in DCM (dichloromethane) (20 mL), and the reaction system was cooled to about 5°C in an ice-water bath. Acetic anhydride (0.04 mL, 3.60 mmol, 3.0 *eq*), DMAP (29.25 mg, 0.24 mmol, *0.2 eq*), and TEA (triethylamine) (0.5 mL, 3.60 mmol, 5.0 *eq*) were sequentially added thereto, and the reaction system was stirred at room temperature for 2 hours. The reaction was monitored by TLC (petroleum ether: ethyl acetate = 10:1). After the reaction was completed, the reaction mixture was quenched with methanol (20 mL), washed once with saturated sodium bicarbonate (20 mL) and once with water (20 mL), dried over anhydrous sodium sulfate, and concentrated to obtain a crude product. The crude product was purified by flash chromatography (petroleum ether: ethyl acetate = 97:3 to 95:5) to obtain 1-bromopentan-3-yl acetate (**211-2**) (15 mg, purity: 90%, yield: 20.75%) as a colorless oil. ¹H NMR (400 MHz, CDCl₃) δ 4.95 (dq, *J =* 12.4, 6.2 Hz, 1H), 3.45 - 3.28 (m, 2H), 2.12 (dt, *J =* 7.8, 5.7 Hz, 2H), 2.07 (s, 3H), 1.61 (dt, *J =* 13.6, 6.9 Hz, 2H), 0.91 (t, *J =* 7.5 Hz, 3H).

**Step 3:** 1-Bromopentan-3-yl acetate (**211-2**) (180 mg, 0.86 mmol) (200 mg, 1.20 mmol, 1.0 *eq*) was dissolved in acetonitrile (10 mL), then triphenylphosphine (677 mg, 2.583 mmol, 3 *eq*) was added thereto, and the reaction system was stirred at 90°C for 18 hours under a nitrogen atmosphere. The reaction was monitored by TLC (petroleum ether: ethyl acetate = 10:1). After the reaction was completed, the reaction system was concentrated to obtain a crude product. The crude product was purified by flash chromatography (dichloromethane: methanol = 95:5 to 90:10) to obtain 1-(bromotriphenyl-λ5-phosphanyl)pentan-3-yl acetate (**211-3**) (132 mg, purity: 90%, yield: 29.27%) as a white solid. ¹H NMR (400 MHz, CDCl₃) δ 7.94 - 7.63 (m, 15H), 4.83 - 4.74 (m, 1H), 3.95 - 3.88 (m, 2H), 2.06 (s, 3H), 2.03 - 1.92 (m, 2H), 1.80 - 1.75 (m, 2H), 0.84 (t, *J=* 7.4 Hz, 3H).

**Step 4:** The starting materials 1-(bromotriphenyl-λ5-phosphanyl)pentan-3-yl acetate (**211-3**) (126 mg, 0.27 mmol, 2.0 *eq*), 18-crown-6 (35 mg, 0.13 mmol, 1.0 *eq*), and potassium carbonate (55 mg, 0.40 mmol, 3.0 *eq*) were dissolved in DCM (dichloromethane) (5 mL), and the reaction mixture was reacted at 50°C for 1 hour under a nitrogen atmosphere. A solution of (1*R*,3a*S*,3b*S*,7*S*,9a*R*,9b*S*,11a*S*)-1-[(1*S*)-1-formylethyl]-9a,11a-dimethyl-2,3,3a,3b,4,6,7,8,9,9a,9b,10,11,11a-tetradecahydro-1*H*-cyclopenta[1,2-*i*]phenanthren-7-yl acetate (**151-2**) (50 mg, 0.13 mmol, 1.0 *eq*) in dichloromethane (5 mL) was added thereto, and the reaction system was refluxed and stirred for 48 hours. The reaction was monitored by TLC (petroleum ether: ethyl acetate = 10:1). The reaction system was cooled to room temperature and then concentrated to obtain a crude product. The crude product was purified by flash chromatography (petroleum ether: ethyl acetate = 93:7 to 94:4) to obtain (5E,7R)-7-[(1*R*,3a*S*,7*S*,9a*R*,11a*R*)-7-acetoxy-9a,11a-dimethyl-2,3,3a,3b,4,6,7,8,9,9a,9b,10,11,11a-tetradecahydro-1*H*-cyclopenta[1,2-*a*]phenanthren-1-yl]oct-5-en-3-yl acetate (**211-4**) (15 mg, purity: 90%, yield: 20.75%) as a white solid. ¹H NMR (400 MHz, CDCl₃) δ 5.37 (d, *J* = 4.9 Hz, 1H), 5.29 - 5.11 (m, 2H), 4.85 - 4.78 (m, 1H), 4.61 (ddd, *J =* 15.9, 8.9, 4.2 Hz, 1H), 2.40 (ddd, *J=* 16.1, 9.3, 1.8 Hz, 1H), 2.32 (d, *J =* 6.5 Hz, 2H), 2.28 - 2.16 (m, 1H), 2.03 (t, *J=* 3.3 Hz, 6H), 1.97 (ddd, *J=* 15.1, 9.2, 4.1 Hz, 2H), 1.86 (d, *J=* 10.2 Hz, 2H), 1.70 - 1.43 (m, 10H), 1.26 - 1.07 (m, 5H), 1.03 (s, 4H), 0.97 (dd, *J=* 6.6, 2.0 Hz, 3H), 0.90 (t, *J=* 7.4 Hz, 3H), 0.71 (t, *J =* 8.4 Hz, 3H).

**Step 5:** The starting material (5*E*,7*R*)-7-[(1*R*,3a*S*,7*S*,9a*R*,11a*R*)-7-acetoxy-9a,11a-dimethyl-2,3,3a,3b,4,6,7,8,9,9a,9b,10,11,11a-tetradecahydro-1*H*-cyclopenta[1,2-*a*]phenanthren-1-yl]oct-5-en-3-yl acetate (**211-4**) (20 mg, 0.04 mmol, 1.0 *eq)* was dissolved in ethanol (3 mL), then Pd/C (5 mg, 0.04 mmol, 45%) was added thereto, and the reaction system was replaced with a hydrogen atmosphere and stirred at room temperature for 18 hours. The reaction was monitored by TLC (petroleum ether: ethyl acetate = 5:1, heating with phosphomolybdic acid). After the reaction was completed, the reaction mixture was filtered through diatomite, and the filtrate was evaporated to dryness by rotary evaporation under vacuum to obtain (7*R*)-7-[(1*R*,3a*S*,7*S*,9a*S*,11a*R*)-7-acetoxy-9a,11a-dimethylhexadecahydro-1*H*-cyclopenta[1,2-*a*]phenanthren-1-yl]octan-3-yl acetate (**211-5**) (15 mg, purity: 85%, yield: 63.22%) as a white solid. ¹H NMR (400 MHz, CDCl₃) δ 4.84 - 4.76 (m, 1H), 4.73 - 4.63 (m, 1H), 2.05 (s, 3H), 2.02 (s, 3H), 1.95 (dd, *J =* 9.3, 3.2 Hz, 1H), 1.85 - 1.77 (m, 2H), 1.76 - 1.70 (m, 1H), 1.68 - 0.95 (m, 29H), 0.89 (s, 2H), 0.88 (s, 3H), 0.81 (s, 3H), 0.64 (s, 3H).

**Step 6:** (7*R*)-7-[(1*R*,3a*S*,7*S*,9a*S*,11a*R*)-7-Acetoxy-9a,11a-dimethylhexadecahydro-1*H*-cyclopenta[1,2-*a*]phenanthren-1-yl]octan-3-yl acetate (**211-5**) (50 mg, 0.10 mmol, 1 *eq*) was dissolved in THF (tetrahydrofuran) (1 mL) and MeOH (methanol) (1 mL), then 1 N lithium hydroxide (0.6 mL) was added dropwise thereto, and the reaction mixture was stirred at 50°C for 2 hours. The reaction was monitored by TLC (dichloromethane: methanol = 20:1). After the reaction was completed, the reaction mixture was cooled to room temperature, acidified with 1 N HCl to adjust the pH to 3 to 4, concentrated to remove ethanol, and extracted with EtOAc (ethyl acetate) (20 mL × 3). The combined organic phases were washed with saturated brine (50 mL), then dried over anhydrous sodium sulfate, filtered, and concentrated to obtain a crude product. The crude product was purified by flash chromatography (dichloromethane: methanol = 95:5 to 94:6) to obtain (1*R*,3a*S*,5a*S*,7*S*,9a*S*,11a*R*)-1-[(2*R*)-6-hydroxyoctan-2-yl]-3a,9a,11a-trimethylhexadecahydro-1*H*-cyclopenta[1,2-*a*]phenanthren-7-ol (**211**) (21 mg, yield: 50.43%) as a white solid. ¹H NMR (400 MHz, CDCl₃) δ 3.59 (s, 1H), 3.54 - 3.46 (m, 1H), 1.99 - 1.92 (m, 1H), 1.79 (d, *J=* 9.5 Hz, 2H), 1.69 (s, 2H), 1.61 - 1.53 (m, 2H), 1.51 - 1.20 (m, 20H), 1.13 - 0.98 (m, 5H), 0.97 - 0.89 (m, 7H), 0.80 (s, 3H), 0.64 (s, 3H).

### Example 216

### Preparation of 3β,25-dihydroxy-5α-cholest-7-one (compound 216)

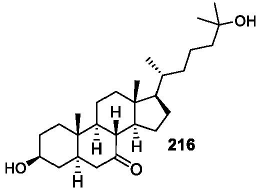

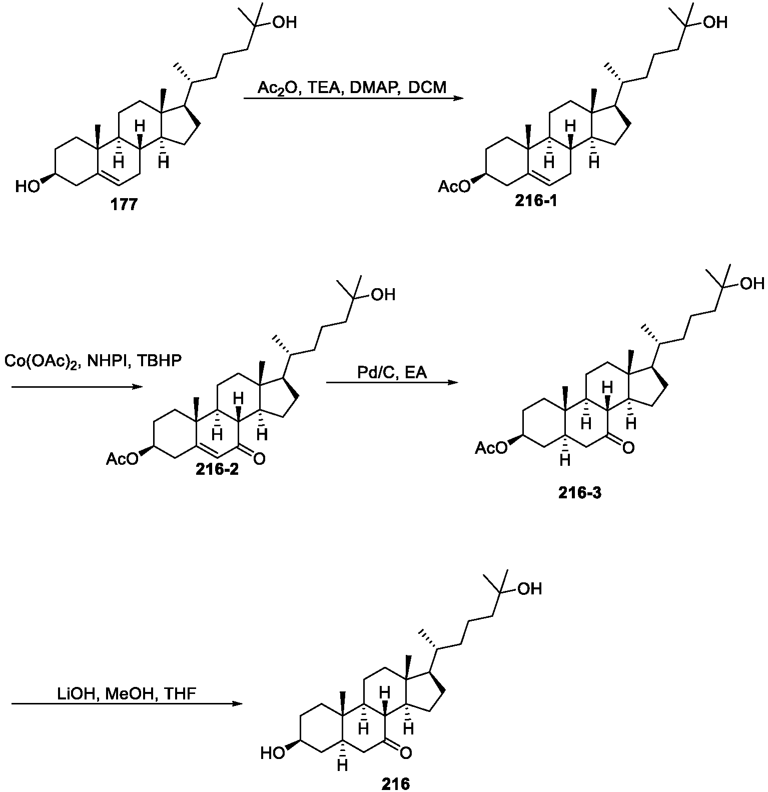

**Step 1:** Cholest-6(5)-en-3β,25-diol (**177**) (300 mg, 0.745 mmol) was dissolved in dichloromethane (30 mL). Triethylamine (0.52 mL, 3.73 mmol), 4-dimethylaminopyridine (18.2 mg, 0.15 mmol), and acetic anhydride (0.084 mL, 0.89 mmol) were added thereto under a nitrogen atmosphere, and the resulting mixture was reacted at room temperature for 2 hours. The reaction was monitored by TLC (petroleum ether: ethyl acetate = 1:1). After the reaction was completed, the reaction mixture was quenched with saturated ammonium chloride and extracted with dichloromethane. The organic phase was separated, dried over anhydrous sodium sulfate, and the reaction mixture was concentrated to obtain a crude product, which was then purified by column chromatography (petroleum ether: ethyl acetate = 60:1 to 5:1) to obtain (1*R*,3a*S*,3b*S*,7*S*,9a*R*,9b*S*,11a*R*)-1-[(2*R*)-6-hydroxy-6-methylheptan-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate (**216-1**) (200 mg, 0.41 mmol, 54.33%) as an off-white solid. ¹H NMR (399 MHz, Chloroform-d) δ 5.35 (d, *J=* 5.0 Hz, 1H), 4.57 (q, *J=* 6.4, 5.3 Hz, 1H), 2.29 (d, *J =* 7.9 Hz, 2H), 2.01 (s, 3H), 1.95 (d, *J =* 18.2 Hz, 2H), 1.84 (d, *J =* 11.6 Hz, 2H), 1.61 - 1.51 (m, 4H), 1.49 - 1.30 (m, 8H), 1.19 (s, 6H), 1.13 - 1.04 (m, 3H), 0.91 (d, *J* = 6.5 Hz, 3H), 0.66 (s, 3H).

**Step 2:** (1*R*,3a*S*,3b*S*,7*S*,9a*R*,9b*S*,11a*R*)-1-[(2*R*)-6-Hydroxy-6-methylheptan-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate (**216-1**) (280 mg, 0.63 mmol) was dissolved in acetone (10 mL). Cobalt acetate (1.11 mg, 0.006 mmol), N-hydroxyphthalimide (10.27 mg, 0.063 mmol), and *tert*-butyl hydroperoxide (226.97 mg, 2.52 mmol) were added thereto at room temperature, and the reaction mixture was stirred at room temperature for 40 hours. The reaction was monitored by TLC (petroleum ether: ethyl acetate = 3:1). After the reaction was completed, the reaction mixture was quenched with water (10 mL), extracted with ethyl acetate (10 mL * 3), washed once with brine, dried over anhydrous sodium sulfate, and concentrated to obtain a crude product, which was then purified by column chromatography (petroleum ether: ethyl acetate = 60:1 to 4:1) to obtain (1*R*,3a*S*,3b*S*,7*S*,9a*R*,9b*S*,11a*R*)-1-[(2*R*)-6-hydroxy-6-methylheptan-2-yl]-9a,11a-dimethyl-4-oxo-2,3,3a,3b,4,6,7,8,9,9a,9b,10,11,11a-tetradecahydro-1*H*-cyclopenta[1,2-*i*]phenanthren-7-yl acetate (**216-2**) (180 mg, 0.37 mmol, 59.21%). ¹H NMR (399 MHz, Chloroform-d) δ 5.68 (s, 1H), 4.69 (s, 1H), 2.57 - 2.34 (m, 3H), 2.21 (s, 1H), 2.03 (s, 3H), 1.94 (d, *J=* 13.6 Hz, 3H), 1.86 (t, *J =* 8.9 Hz, 1H), 1.73 - 1.62 (m, 1H), 1.57 (s, 4H), 1.51 (dd, *J=* 13.4, 6.2 Hz, 2H), 1.44 - 1.39 (m, 3H), 1.35 (dd, *J=* 11.9, 6.3 Hz, 3H), 1.28 - 1.25 (m, 2H), 1.19 (s, 9H), 1.06 (dd, *J =* 15.2, 9.2 Hz, 2H), 0.92 (d, *J =* 6.5 Hz, 3H), 0.66 (s, 3H).

**Step 3:** (1*R*,3a*S*,3b*S*,7*S*,9a*R*,9b*S*,11a*R*)-1-[(2*R*)-6-Hydroxy-6-methylheptan-2-yl]-9a,11a-dimethyl-4-oxo-2,3,3a,3b,4,6,7,8,9,9a,9b,10,11,11a-tetradecahydro-1*H-*cyclopenta[1,2-*i*]phenanthren-7-yl acetate (**216-2**) (140 mg, 0.31 mmol) was dissolved in ethyl acetate (10 mL), and Pd/C (30 mg, 0.28 mmol) was added thereto at room temperature. The reaction system was replaced with hydrogen and stirred at room temperature for 1 hour. The reaction was monitored by TLC (petroleum ether: ethyl acetate = 2:1). After the reaction was completed, the reaction mixture was filtered and evaporated to dryness by rotary evaporation to obtain a crude product (150 mg), which was then purified by column chromatography (petroleum ether: ethyl acetate = 60:1 to 5:1) to obtain (1*R*,3a*S*,3b*R*,5a*R*,7*S*,9a*S*,9b*S*,11a*R*)-1-[(2*R*)-6-hydroxy-6-methylheptan-2-yl]-9a,11a-dimethyl-4-oxohexadecahydro-1*H-*cyclopenta[1,2-*i*]phenanthren-7-yl acetate (**216-3**) (100 mg, 0.21 mmol, 67.56%). ¹H NMR (399 MHz, Chloroform-d) δ 4.67 (s, 1H), 2.29 (d, *J =* 15.5 Hz, 2H), 2.16 (s, 1H), 2.00 (s, 3H), 1.94 (s, 1H), 1.88 (d, *J =* 19.1 Hz, 2H), 1.77 (d, *J =* 13.7 Hz, 1H), 1.64 (d, *J =* 16.8 Hz, 1H), 1.58 (s, 4H), 1.56 - 1.44 (m, 5H), 1.44 - 1.39 (m, 2H), 1.36 (d, *J =* 10.8 Hz, 4H), 1.25 (d, *J =* 11.1 Hz, 2H), 1.19 (s, 6H), 1.11 (d, *J =* 7.2 Hz, 1H), 1.08 (s, 3H), 1.03 (d, *J =* 12.5 Hz, 2H), 0.90 (d, *J=* 6.5 Hz, 3H), 0.63 (s, 3H).

**Step 4:** (1*R*,3a*S*,3b*R*,5a*R*,7*S*,9a*S*,9b*S*,11a*R*)-1-[(2*R*)-6-Hydroxy-6-methylheptan-2-yl]-9a,11a-dimethyl-4-oxohexadecahydro-1*H*-cyclopenta[1,2-*i*]phenanthren-7-yl acetate (**216-3**) (30 mg, 0.065 mmol) was dissolved in a mixed solvent of tetrahydrofuran (3 mL) and methanol (1.5 mL), then lithium hydroxide (0.5 mL, 0.5 mmol) was added thereto at room temperature, and the reaction mixture was stirred at room temperature for 2 hours. The reaction was monitored by TLC (petroleum ether: ethyl acetate = 2:1). After the reaction was completed, the reaction mixture was concentrated to obtain a crude product (50 mg), which was then purified by column chromatography (dichloromethane: methanol = 80:1) to obtain 3β,25-dihydroxy-5α-cholest-7-one (**216**) (13.8 mg, 0.031 mmol, 48.09%) as a white solid. ¹H NMR (399 MHz, Chloroform-d) δ 3.63 - 3.54 (m, 1H), 2.40 - 2.26 (m, 2H), 2.18 (s, 1H), 2.03 - 1.94 (m, 2H), 1.85 (d, *J =* 5.9 Hz, 2H), 1.75 (d, *J =* 13.4 Hz, 1H), 1.52 (d, *J =* 11.7 Hz, 7H), 1.48 - 1.39 (m, 6H), 1.37 (d, *J =* 10.2 Hz, 5H), 1.19 (s, 6H), 1.06 (s, 3H), 1.01 - 0.93 (m, 2H), 0.90 (d, *J* = 6.5 Hz, 3H), 0.63 (s, 3H). ¹³C NMR (100 MHz, CDCl₃) δ 212.08, 77.30, 77.17, 76.98, 76.66, 71.08, 70.63, 55.19, 54.91, 49.97, 48.85, 46.80, 46.06, 44.35, 42.48, 38.71, 37.85, 36.36, 35.95, 35.59, 31.02, 29.29, 29.20, 28.40, 21.82, 20.70, 18.75, 11.99. LC-MS: [M-OH]⁺ = 401.35.

### Example 217

### Preparation of 24-[cyclopropyl(hydroxy)methyl]-5a-cholan-3p-ol (compound 217)

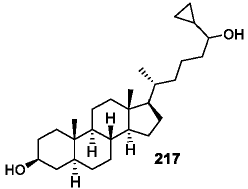

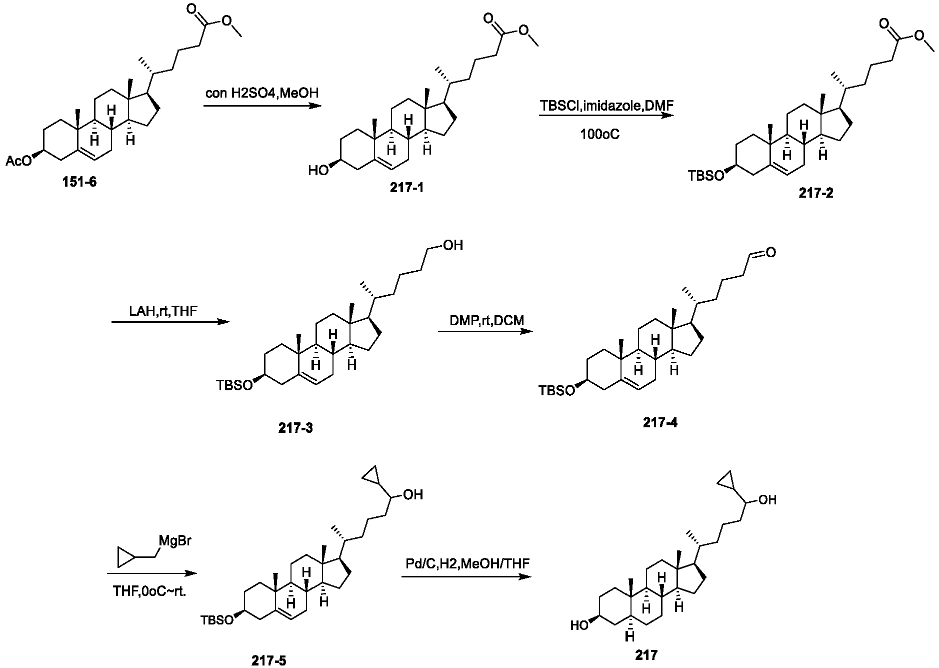

***Step 1*:** The reactant methyl (5*R*)-5-[(1*R*,3a*S*,3b*S*,7*S*,9a*R*,9b*S*,11a*R*)-7-acetoxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate (**151-6**) (2 g, 4.50 mmol, 1.0 *eq*) was dissolved in methanol (50 mL), and concentrated sulfuric acid (98%) (1 mL, 18.76 mmol) was added dropwise thereto. The reaction mixture was then heated to reflux and stirred for 1 hour. The reaction was monitored by TLC (petroleum ether: ethyl acetate = 10:1). After the reaction was completed, the reaction mixture was concentrated to remove most of the methanol, then added with ethyl acetate, and washed twice with saturated sodium bicarbonate and once with saturated brine. The organic phase was dried over anhydrous sodium sulfate, filtered, and the filtrate was concentrated to dryness to obtain a crude product, which was then purified by flash silica gel column chromatography (petroleum ether: ethyl acetate = 90:10 to 80:20, heating with phosphomolybdic acid) to obtain methyl (5*R*)-5-[(1*R*,3a*S*,3b*S*,7*S*,9a*R*,9b*S*,11a*R*)-7-hydroxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate (**217-1**) (1.8 g, 4.024 mmol, 89.46%) as a white solid. ¹H NMR (400 MHz, CDCl₃) δ 5.37 - 5.33 (m, 1H), 3.67 (s, 3H), 3.52 (dd, *J =* 7.7, 3.3 Hz, 1H), 2.35 - 2.20 (m, 4H), 2.03 - 1.93 (m, 2H), 1.84 (ddd, *J=* 12.5, 7.5, 4.3 Hz, 3H), 1.75 - 1.60 (m, 4H), 1.59 - 1.35 (m, 9H), 1.33 - 1.04 (m, 7H), 1.03 - 0.99 (m, 3H), 0.93 (d, *J=* 6.6 Hz, 3H), 0.68 (s, 3H).

***Step 2*:** The reactant methyl (5*R*)-5-[(1*R*,3a*S*,3b*S*,7*S*,9a*R*,9b*S*,11a*R*)-7-hydroxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate (**217-1**) (1.7 g, 4.38 mmol) was dissolved in DMF (50 mL), then TBSCl (3.3 g, 21.9 mmol, 5.0 *eq*) and imidazole (0.30 g, 4.38 mmol, 1.0 *eq*) were sequentially added thereto, and the reaction mixture was heated to 100°C. The reaction was monitored by TLC (petroleum ether: ethyl acetate = 10:1). After the reaction was completed, the reaction mixture was cooled to room temperature, added with ethyl acetate (100 mL), and washed three times with water. The organic phase was then dried over anhydrous sodium sulfate, filtered, and the filtrate was concentrated. The crude product was purified by flash column chromatography (petroleum ether: ethyl acetate = 90:10) to obtain methyl (5R)-5-[(1*R*,3a*R*,3b*S*,7*S*,9a*R*,9b*S*,11a*R*)-7-{[dimethyl(2-methylpropan-2-yl)methylsilyl]oxy}-3b,9a,11a-trimethyl-2,3,3a,3b,4,6,7,8,9,9a,9b,10,11,11a-tetradecahydro-1*H*-cyclopenta[1,2-*a*]phenanthren-1-yl]hexanoate (**217-2**) (1.9 g, 3.40 mmol, 77.73%) as a colorless wax. ¹H NMR (400 MHz, CDCl₃) δ 5.26 - 5.18 (m, 1H), 3.57 (s, 3H), 3.43 - 3.33 (m, 1H), 2.16 (d, *J=* 1.9 Hz, 4H), 1.93 - 1.82 (m, 2H), 1.65 - 1.55 (m, 2H), 1.39 (d, *J=* 3.0 Hz, 9H), 0.99 (s, 7H), 0.90 (s, 5H), 0.85 - 0.79 (m, 21H), 0.57 (s, 3H), - 0.00 (s, 6H).

***Step 3*:** The reactant methyl (5*R*)-5-[(1*R*,3a*R*,3b*S*,7*S*,9a*R*,9b*S*,11a*R*)-7-{[dimethyl(2-methylpropan-2-yl)methylsilyl]oxy}-3b,9a,11a-trimethyl-2,3,3a,3b,4,6,7,8,9,9a,9b,10,11,11a-tetradecahydro-1*H*-cyclopenta[1,2-*a*]phenanthren-1-yl]hexanoate (**217-2**) (1.8 g, 3.58 mmol, 1.0 *eq*) was dissolved in tetrahydrofuran (50 mL), then lithium aluminum hydride (0.168 g, 4.269 mmol, 1.2 *eq*) was added thereto, and the reaction mixture was stirred at room temperature for 30 minutes. The reaction was monitored by TLC (petroleum ether: ethyl acetate = 10:1). After the reaction was completed, the reaction mixture was quenched with sodium sulfate decahydrate, filtered, and the filtrate was concentrated to obtain a crude product, which was then purified by silica gel column chromatography (petroleum ether: ethyl acetate = 80:20 to 70:30) to obtain (5*R*)-5-[(1*R*,3a*R*,3b*S*,7*S*,9a*R*,9b*S*,11a*R*)-7-{[dimethyl(2-methylpropan-2-yl)methylsilyl]oxy}-3b,9a,11a-trimethyl-2,3,3a,3b,4,6,7,8,9,9a,9b,10,11,11a-tetradecahydro-1*H*-cyclopenta[1,2-*a*]phenanthren-1-yl]hexan-1-ol (**217-3**) (1.7 g, 3.22 mmol, 90.01%) as a white solid. ¹H NMR (400 MHz, CDCl₃) δ 5.26 (d, *J* = 5.3 Hz, 1H), 3.59 (t, *J* = 6.6 Hz, 2H), 3.47 - 3.37 (m, 1H), 2.27 - 2.17 (m, 1H), 2.11 (ddd, *J=* 13.4, 5.0, 2.2 Hz, 1H), 1.93 (s, 2H), 1.77 (s, 2H), 1.66 (d, *J =* 12.2 Hz, 1H), 1.47 (ddd, *J* = 13.9, 8.7, 4.5 Hz, 7H), 1.37 - 1.29 (m, 3H), 1.25 - 1.15 (m, 2H), 1.13 - 0.97 (m, 5H), 0.94 (s, 3H), 0.87 (d, *J =* 6.3 Hz, 4H), 0.84 - 0.81 (m, 10H), 0.62 (s, 3H), 0.02 - -0.02 (m, 6H).

***Step 4*:** The compound (5*R*)-5-[(1*R*,3a*R*,3b*S*,7*S*,9a*R*,9b*S*,11a*R*)-7-{[dimethyl(2-methylpropan-2-yl)methylsilyl]oxy}-3b,9a,11a-trimethyl-2,3,3a,3b,4,6,7,8,9,9a,9b,10,11,11a-tetradecahydro-1*H*-cyclopenta[1,2-*a*]phenanthren-1-yl]hexan-1-ol (**217-3**) (1.0 g, 2.11 mmol, 1.0 *eq*) was dissolved in DCM (100 mL), then Dess-Martin periodinane (1.068 g, 2.532 mmol, *1.2 eq*) was added thereto, and the reaction mixture was stirred at room temperature. The reaction was monitored by TLC (petroleum ether: ethyl acetate = 5:1). After the reaction was completed, the reaction mixture was quenched with saturated sodium sulfite and washed with saturated sodium bicarbonate. The organic phase was dried over anhydrous sodium sulfate, concentrated, and the crude product was purified by silica gel column chromatography (petroleum ether: ethyl acetate = 90:10 to 80:20) to obtain (5R)-5-[(1*R*,3a*S*,3b*S*,7*S*,9a*R*,9b*S*,11a*R*)-7-{[dimethyl(2-methylpropan-2-yl)methylsilyl]oxy}-9a,11a-dimethyl-2,3,3a,3b,4,6,7,8,9,9a,9b,10,11,11a-tetradecahydro-1*H*-cyclopenta[1,2-*a*]phenanthren-1-yl]hexanal (**217-4**) (0.6 g, 1.142 mmol, 54.23%) as a white solid. ¹H NMR (400 MHz, CDCl₃) δ 9.71 (t, *J =* 1.8 Hz, 1H), 5.26 (d, *J =* 5.3 Hz, 1H), 3.49 - 3.36 (m, 1H), 2.34 (d, *J=* 6.6 Hz, 2H), 2.25 - 2.07 (m, 2H), 1.93 (s, 2H), 1.81 - 1.60 (m, 4H), 1.48 - 1.30 (m, 8H), 1.03 (ddd, *J =* 14.6, 12.0, 6.3 Hz, 7H), 0.94 (s, 4H), 0.89 (d, *J =* 6.5 Hz, 4H), 0.85 - 0.81 (m, 9H), 0.62 (s, 3H), 0.02 - -0.02 (m, 7H).

***Step 5*:** (5*R*)-5-[(1*R*,3a*S*,3b*S*,7*S*,9a*R*,9b*S*,11a*R*)-7-{[Dimethyl(2-methylpropan-2-yl)methylsilyl]oxy}-9a,11a-dimethyl-2,3,3a,3b,4,6,7,8,9,9a,9b,10,11,11a-tetradecahydro-1*H-*cyclopenta[1,2-*a*]phenanthren-1-yl]hexanal (**217-4**) (100 mg, 0.205 mmol, 1.0 *eq*) was dissolved in tetrahydrofuran (5 mL), and the reaction mixture was cooled to 0°C in an ice bath. After complete dissolution, cyclopropylmagnesium bromide (2.054 mL, 1 mol/L, 2.054 mmol, 10 *eq*) was added to the reaction system. After the addition was completed, the reaction mixture was stirred at room temperature for 2 hours. After the completion of the reaction was monitored by TLC (petroleum ether: ethyl acetate = 5:1), the reaction mixture was quenched with saturated ammonium chloride solution (10 mL) in an ice bath, washed with water (10 mL × 3), and extracted with ethyl acetate (10 mL × 3). The organic phases were combined, dried over anhydrous sodium sulfate, and purified by silica gel column chromatography (petroleum ether: ethyl acetate = 100:0 to 94:6) to obtain (5*R*)-1-cyclopropyl-5-[(1*R*,3a*S*,3b*S*,7*S*,9a*R*,9b*S*,11a*R*)-7-{[dimethyl(2-methylpropan-2-yl)methylsilyl]oxy}-9a,11a-dimethyl-2,3,3a,3b,4,6,7,8,9,9a,9b,10,11,11a-tetradecahydro-1*H*-cyclopenta[1,2-*a*]phenanthren-1-yl]hexan-1-ol (**217-5**) (72 mg, 0.109 mmol, purity: 80.0%, yield: 53.02%). ¹H NMR (400 MHz, CDCl₃) δ 5.31 (d, *J =* 5.3 Hz, 1H), 3.47 (td, *J =* 10.9, 5.4 Hz, 1H), 2.88 - 2.82 (m, 1H), 2.27 (t, *J* = 11.1 Hz, 1H), 2.18 - 2.13 (m, 1H), 1.99 (s, 2H), 1.82 (s, 2H), 1.71 (d, *J =* 12.3 Hz, 1H), 1.61 - 1.51 (m, 11H), 1.41 (dd, *J* = 12.4, 6.8 Hz, 3H), 1.27 (d, *J =* 12.0 Hz, 3H), 1.16 - 1.01 (m, 5H), 0.96 - 0.84 (m, 15H), 0.67 (s, 3H), 0.58 - 0.44 (m, 2H), 0.24 (d, *J* = 4.7 Hz, 2H), 0.02 - -0.02 (m, 5H).

***Step 6*:** (5*R*)-1-Cyclopropyl-5-[(1*R*,3a*S*,3b*S*,7*S*,9a*R*,9b*S*,11a*R*)-7-{[dimethyl(2-methylpropan-2-yl)methylsilyl]oxy}-9a,11a-dimethyl-2,3,3a,3b,4,6,7,8,9,9a,9b,10,11,11a-tetradecahydro-1*H*-cyclopenta[1,2-*a*]phenanthren-1-yl]hexan-1-ol (**217-5**) (72 mg, 0.136 mmol, 1.0 *eq*) was dissolved in methanol (5 mL) and tetrahydrofuran (2 mL), then Pd/C was added thereto, and the reaction system was replaced with hydrogen three times. The reaction was monitored by HNMR. After the reaction was completed, the reaction mixture was filtered through diatomite to remove Pd/C. The organic phases were combined and purified by silica gel column chromatography (petroleum ether: ethyl acetate = 90:10 to 75:25) to obtain 24-[cyclopropyl(hydroxy)methyl]-5α-cholan-3β-ol (**217**) (17.88 mg, 0.122 mmol, purity: 94.86%, yield: 89%) as a white solid. ¹H NMR (400 MHz, CDCl₃) δ 3.63 - 3.54 (m, 1H), 2.85 (td, *J =* 7.8, 4.9 Hz, 1H), 1.96 (dt, *J =* 12.6, 3.4 Hz, 1H), 1.86 - 1.75 (m, 2H), 1.74 - 1.58 (m, 3H), 1.49 - 1.19 (m, 14H), 1.03 (ddd, *J =* 22.5, 17.2, 8.7 Hz, 7H), 0.90 (t, *J* = 7.6 Hz, 5H), 0.80 (s, 3H), 0.65 (s, 4H), 0.57 - 0.44 (m, 2H), 0.24 (d, *J =* 4.7 Hz, 2H). ¹³C NMR (101 MHz, CDCl₃) δ 71.40, 64.92, 63.38, 56.51, 56.09, 54.40, 45.03, 42.62, 39.97, 38.29, 37.56, 36.91, 35.76, 35.39, 32.09, 31.44, 28.75, 28.10, 24.22, 21.26, 19.64, 18.67, 18.01, 12.28, 11.99, 3.35, 2.27. LC-MS: [M-OH-H₂O]⁺ = 381.

### Example 218

### Preparation of 24-(2,2,2-trifluoro-1-hydroxyethyl)-5α-cholan-3β-ol (compound 218)

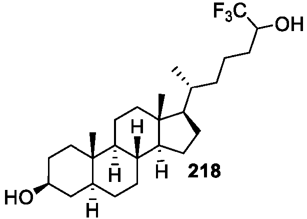

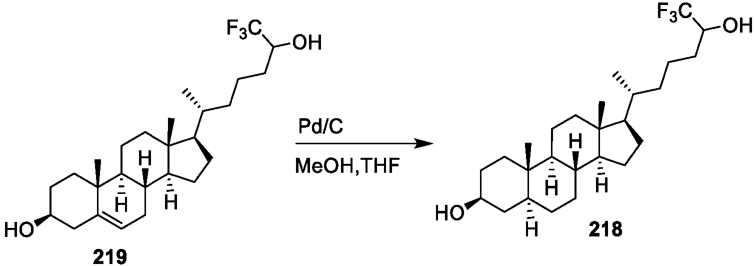

24-(2,2,2-Trifluoro-1-hydroxyethyl)cholan-6(5)-en-3β-ol (**219**) (52 mg, 0.093 mmol, 1.0 *eq*) was dissolved in methanol (5 mL) and tetrahydrofuran (2 mL), then Pd/C was added thereto, and the reaction system was replaced with hydrogen three times. The reaction was monitored by HNMR. After the reaction was completed, the reaction mixture was filtered through diatomite to remove Pd/C. The organic phases were combined and purified by silica gel column chromatography (petroleum ether: ethyl acetate = 90:10 to 75:25) to obtain 24-(2,2,2-trifluoro-1-hydroxyethyl)-5α-cholan-3β-ol (**218**) (32 mg, 0.066 mmol, purity: 94.86%, yield: 89%) as a white solid. ¹H NMR (400 MHz, CDCl₃) δ 3.95 - 3.87 (m, 1H), 3.64 - 3.54 (m, 1H), 1.96 (m, 1H), 1.79 (m, 2H), 1.73 - 1.62 (m, 4H), 1.47 (m, 4H), 1.43 - 1.32 (m, 5H), 1.30 - 1.19 (m, 6H), 1.15 - 0.96 (m, 7H), 0.92 (d, *J =* 6.5 Hz, 3H), 0.85 (m, 1H), 0.80 (s, 3H), 0.65 (s, 3H) ¹⁹F (376 MHz, CDCl₃) δ -80.04.

### Example 219

### Preparation of 24-(2,2,2-trifluoro-1-hydroxyethyl)cholan-6(5)-en-3β-ol (compound 219)

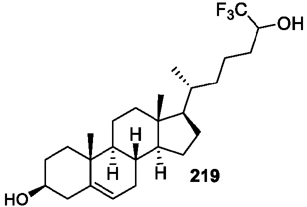

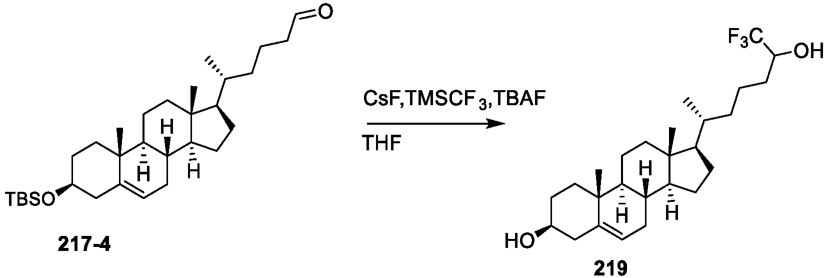

(5*R*)-5-[(1*R*,3a*S*,3b*S*,7*S*,9a*R*,9b*S*,11a*R*)-7-{[Dimethyl(2-methylpropan-2-yl)methylsilyl]oxy}-9a,11a-dimethyl-2,3,3a,3b,4,6,7,8,9,9a,9b,10,11,11a-tetradecahydro-1*H-*cyclopenta[1,2-*a*]phenanthren-1-yl]hexanal (**217-4**) (100 mg, 0.205 mmol, 1.0 *eq*) was dissolved in tetrahydrofuran (5 mL). After complete dissolution, cesium fluoride (15.6 mg, 0.103 mmol, 0.5 *eq*) and (trifluoromethyl)trimethylsilane (146.03 mg, 0.513 mmol, 5.0 *eq*) were sequentially added to the reaction system. After the addition was completed, the reaction mixture was stirred at room temperature for 2 hours. After the appearance of a large polar spot was monitored by TLC (petroleum ether: ethyl acetate = 10:1), the reaction mixture was added with tetrabutylammonium fluoride (2 mL, 1 mol/L, 10.0 *eq*) and stirred at room temperature for 1 hour. After the appearance of a large polar spot was monitored by TLC (petroleum ether: ethyl acetate = 2:1), the reaction mixture was quenched with saturated ammonium chloride solution (50 mL) in an ice bath, washed with water (10 mL × 3), and extracted with ethyl acetate (10 mL × 3). The organic phases were combined, dried over anhydrous sodium sulfate, and purified by silica gel column chromatography (petroleum ether: ethyl acetate = 90:10 to 75:25) to obtain 24-(2,2,2-trifluoro-1-hydroxyethyl)cholan-6(5)-en-3β-ol (**219**) (10.82 mg, 0.075 mmol, purity: 91.82%, yield: 36.37%). ¹H NMR (400 MHz, CDCl₃) δ 5.37 - 5.33 (m, 1H), 3.96 - 3.87 (m, 1H), 3.52 (ddd, *J =* 15.7, 11.0, 4.5 Hz, 1H), 2.27 (tdd, *J =* 12.8, 8.9, 6.6 Hz, 2H), 2.00 (d, *J =* 3.2 Hz, 3H), 1.84 (dd, *J =* 14.1, 3.4 Hz, 3H), 1.61 - 1.42 (m, 15H), 1.35 - 1.19 (m, 5H), 1.10 (dd, *J=* 18.5, 5.5 Hz, 4H), 0.94 (d, *J* = 6.6 Hz, 3H). ¹³C NMR (101 MHz, CDCl₃) δ 155.09, 121.69, 115.18, 71.82, 69.47, 56.76, 55.91, 50.11, 42.35, 37.25, 35.58, 31.90, 28.24, 19.40, 11.86 ¹⁹F (376 MHz, CDCl₃) δ -80.04 LC-MS: [M-OH]⁺ = 425.

### Example 225

### Preparation of 3β,5,25-trihydroxycholest-6-one (compound 225)

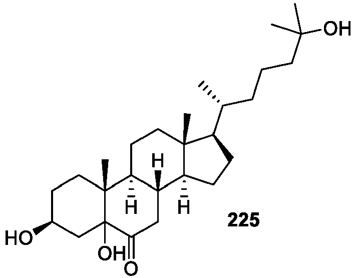

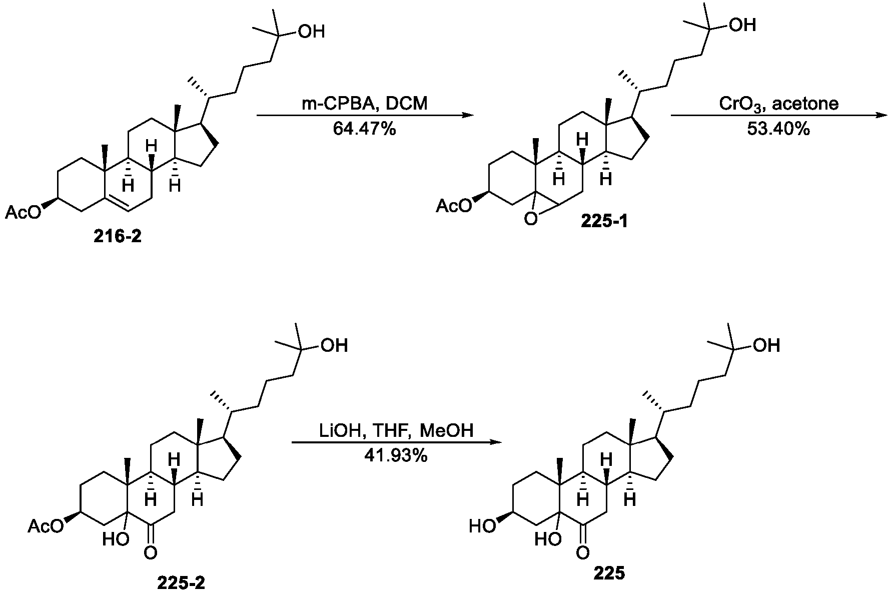

**Step 1:** (3a*S*,3b*S*,7*S*,9a*R*,9b*S*,11a*R*)-1-[(2*R*)-6-Hydroxy-6-methylheptan-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate (**216-2**) (280 mg, 0.63 mmol) was dissolved in dichloromethane (20 mL), then m-chloroperoxybenzoic acid (249.9 mg, 1.45 mmol) was added thereto in batches at room temperature, and the reaction mixture was stirred at room temperature. The reaction was monitored by TLC (petroleum ether: ethyl acetate = 3:1). After the reaction was completed, the reaction mixture was quenched with saturated sodium bicarbonate aqueous solution and extracted with dichloromethane. The organic phase was dried over anhydrous sodium sulfate, and the reaction mixture was concentrated to obtain a crude product, which was then purified by column chromatography (petroleum ether: ethyl acetate = 30:1 to 10:1 to 5:1) to obtain (3*S*,6a*S*,6b*S*,9a*R*,11a*S*,11b*R*)-9-[(2*R*)-6-hydroxy-6-methylheptan-2-yl]-9a,11b-dimethyl-1,2,3,4,5a,6,6a,6b,7,8,9,9a,10,11,11a,11b-hexadecahydrocyclopenta[1,2-*i*]oxazepino[2,3-*m*]phenanthren-3-yl acetate (**225-1**) (220 mg, 0.41 mmol, 64.47%) as a white solid. ¹H NMR (399 MHz, Chloroform-d) δ 4.92 (dd, *J* = 10.8, 5.8 Hz, 1H), 2.87 (d, *J* = 4.3 Hz, 1H), 2.19 - 2.05 (m, 1H), 2.01 (s, 1H), 1.99 (s, 3H), 1.93 (d, *J =* 16.2 Hz, 1H), 1.70 - 1.59 (m, 1H), 1.53 (d, *J =* 15.7 Hz, 3H), 1.47 - 1.40 (m, 3H), 1.38 - 1.30 (m, 7H), 1.29 - 1.20 (m, 4H), 1.19 (s, 6H), 1.05 (s, 3H), 0.98 (s, 1H), 0.96 (s, 1H), 0.89 (d, *J =* 6.5 Hz, 3H), 0.62 (s, 1H), 0.59 (s, 3H).

**Step 2:** (3*S*,6a*S*,6b*S*,9a*R*,11a*S*,11b*R*)-9-[(2*R*)-6-Hydroxy-6-methylheptan-2-yl]-9a,11b-dimethyl-1,2,3,4,5a,6,6a,6b,7,8,9,9a,10,11,11a,11b-hexadecahydrocyclopenta[1,2-*i*]oxazepino[2,3-*m*]phenanthren-3-yl acetate (**225-1**) (100 mg, 0.22 mmol) was dissolved in acetone (10 mL), and the reaction mixture was cooled to 0°C in an ice bath. Chromium trioxide (81.39 mg, 0.81 mmol) was then added thereto. After the addition was completed, the reaction mixture was warmed to 30°C and then stirred for 2 hours. The reaction was monitored by TLC (petroleum ether: ethyl acetate = 5:1). After the reaction was completed, the reaction mixture was quenched with water (10 mL), extracted with ethyl acetate (10 mL * 3), washed once with brine, dried over anhydrous sodium sulfate, and concentrated to obtain the crude product (1*R*,3a*S*,3b*S*,7*S*,9a*R*,9b*S*,11a*R*)-5a-hydroxy-1-[(2*R*)-6-hydroxy-6-methylheptan-2-yl]-9a,11a-dimethyl-5-oxohexadecahydro-1*H*-cyclopenta[1,2-*i*]phenanthren-7-yl acetate (**225-2**) (65 mg, 0.12 mmol, 53.4%). ¹H NMR (399 MHz, Chloroform-d) δ 5.00 (dt, *J =* 11.5, 6.0 Hz, 1H), 2.98 (s, 1H), 2.73 (t, *J =* 12.6 Hz, 1H), 2.08 (dd, *J =* 13.0, 4.5 Hz, 1H), 1.99 (s, 4H), 1.95 - 1.86 (m, 2H), 1.84 - 1.76 (m, 4H), 1.75 - 1.60 (m, 3H), 1.51 (d, *J=* 12.2 Hz, 3H), 1.42 (d, *J =* 9.6 Hz, 3H), 1.37 - 1.29 (m, 4H), 1.26 (d, *J =* 3.0 Hz, 3H), 1.22 (d, *J =* 6.1 Hz, 5H), 1.18 (s, 6H), 1.12 (q, *J =* 9.6 Hz, 2H), 1.03 (dd, *J =* 12.1, 6.5 Hz, 2H), 0.90 (d, *J* = 6.4 Hz, 3H), 0.79 (s, 3H), 0.62 (s, 3H). ¹³C NMR (100 MHz, Chloroform-d) δ 110.00, 80.14, 71.16, 70.74, 56.24, 56.09, 44.26, 44.18, 43.10, 42.45, 41.69, 39.53, 37.26, 36.34, 35.71, 32.29, 29.67, 29.49, 29.29, 29.13, 28.06, 26.24, 23.88, 21.35, 21.31, 20.81, 18.55, 13.85, 11.98.

**Step 3:** (1*R*,3a*S*,3b*S*,7*S*,9a*R*,9b*S*,11a*R*)-5a-Hydroxy-1-[(2*R*)-6-hydroxy-6-methylheptan-2-yl]-9a,11a-dimethyl-5-oxohexadecahydro-1*H*-cyclopenta[1,2-*i*]phenanthren-7-yl acetate (**225-2**) (65 mg, 0.14 mmol) was dissolved in a mixture of methanol (3 mL) and tetrahydrofuran (3 mL), and lithium hydroxide aqueous solution (1 M, 0.21 mL, 0.21 mmol) was added thereto at room temperature. After the addition was completed, the reaction mixture was stirred at room temperature. The reaction was monitored by TLC (petroleum ether: ethyl acetate = 1:1). After the reaction was completed, the reaction mixture was added with water, extracted with dichloromethane (10 mL * 3), washed once with brine, dried over anhydrous sodium sulfate, concentrated, and subjected to column chromatography (petroleum ether: ethyl acetate = 10:1 to 5:1 to 2:1) to obtain 3β,5,25-trihydroxycholest-6-one (**225**) (26 mg, 0.06 mmol, 41.93%). ¹H NMR (400 MHz, Methanol-d₄) δ 3.88 (dt, *J* = 11.4, 6.1 Hz, 1H), 2.74 (t, *J* = 12.5 Hz, 1H), 2.04 (d, *J* = 11.7 Hz, 1H), 1.92 (dtd, *J* = 25.2, 13.0, 5.0 Hz, 3H), 1.81 - 1.59 (m, 5H), 1.54 (d, *J* = 9.2 Hz, 1H), 1.48 - 1.37 (m, 6H), 1.33 - 1.21 (m, 6H), 1.18 (d, *J* = 9.8 Hz, 1H), 1.15 (s, 6H), 1.12 - 1.00 (m, 2H), 0.94 (d, *J* = 6.4 Hz, 3H), 0.77 (s, 3H), 0.67 (s, 3H). ¹³C NMR (101 MHz, CD₃OD) δ 213.88, 79.75, 70.03, 66.42, 56.38, 56.06, 48.19, 47.98, 47.77, 47.55, 47.34, 47.13, 46.92, 44.30, 43.83, 42.89, 42.16, 41.37, 37.48, 36.30, 35.63, 35.13, 29.78, 29.55, 27.82, 23.53, 20.41, 12.92, 10.98. LCMS: [M-OH-H₂O]⁺ = 399.25.

### Example 231

### Preparation of cholest-3β,4β,7,25-tetraol (compound 231)

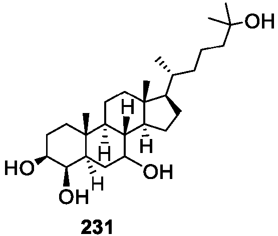

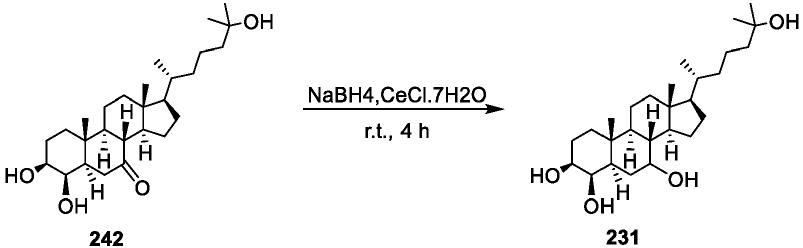

25-Hydroxy-4β-hydroxy-3β-hydroxycholest-7-one (**242**) (40 mg, 0.092 mmol) was dissolved in tetrahydrofuran (2 mL) in a 50 mL round-bottom flask. Cerium(III) chloride heptahydrate (86 mg, 0.23 mmol), sodium borohydride (8.7 mg, 0.23 mmol), and methanol (0.2 mL) were added thereto at room temperature, and the resulting mixture was stirred at room temperature for 2 hours. The completion of the reaction was monitored by TLC (ethyl acetate/petroleum ether = 2/1). After the reaction was completed, the reaction mixture was added with ice water (30 mL) and extracted with ethyl acetate (15 mL × 3). The extracted organic solution was washed with saturated brine (20 mL), dried over anhydrous sodium sulfate, filtered, and concentrated to obtain a crude product. The crude product was purified by silica gel column chromatography (eluent: ethyl acetate/petroleum ether = 2/1) to obtain cholest-3β,4β,7,25-tetraol (**231**) (28 mg, purity: 92.41%, yield: 64.38%) as a white solid. ¹H NMR (400 MHz, DMSO) δ 4.30 - 4.28 (m, 1H), 4.03 (s, 1H), 4.00 - 3.95 (m, 2H), 3.44 - 3.38 (m, 1H), 3.29 - 3.25 (m, 1H), 3.13 - 3.02 (m, 1H), 1.92 - 1.54 (m, 8H), 1.41 - 1.27 (m, 10H), 1.19 - 1.14 (m, 4H), 1.05 (s, 6H), 1.01 - 0.97 (m, 3H), 0.94 - 0.92 (m, 3H), 0.88 (d, *J=* 6.4 Hz, 3H), 0.84 - 0.80 (m, 1H), 0.61 - 0.59 (m, 3H). ¹³C NMR (101 MHz, DMSO) δ 74.55, 73.98, 72.09, 71.90, 69.23, 56.43, 56.22, 55.43, 53.40, 50.54, 46.16, 44.61, 43.41, 43.20, 42.37, 37.47, 37.16, 36.72, 35.75, 35.64, 35.02, 29.89, 29.70, 29.46, 28.83, 27.20, 26.23, 22.57, 20.91, 20.77, 19.17, 19.03, 15.28, 14.20, 12.55, 12.17. LCMS [M+H-3H₂O]⁺ = 401.

### Example 232

### Preparation of 24-(2,2,2-trifluoro-1-hydroxyethyl)-5α-cholan-3β,4β-diol (compound 232)

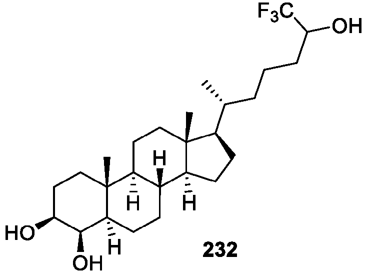

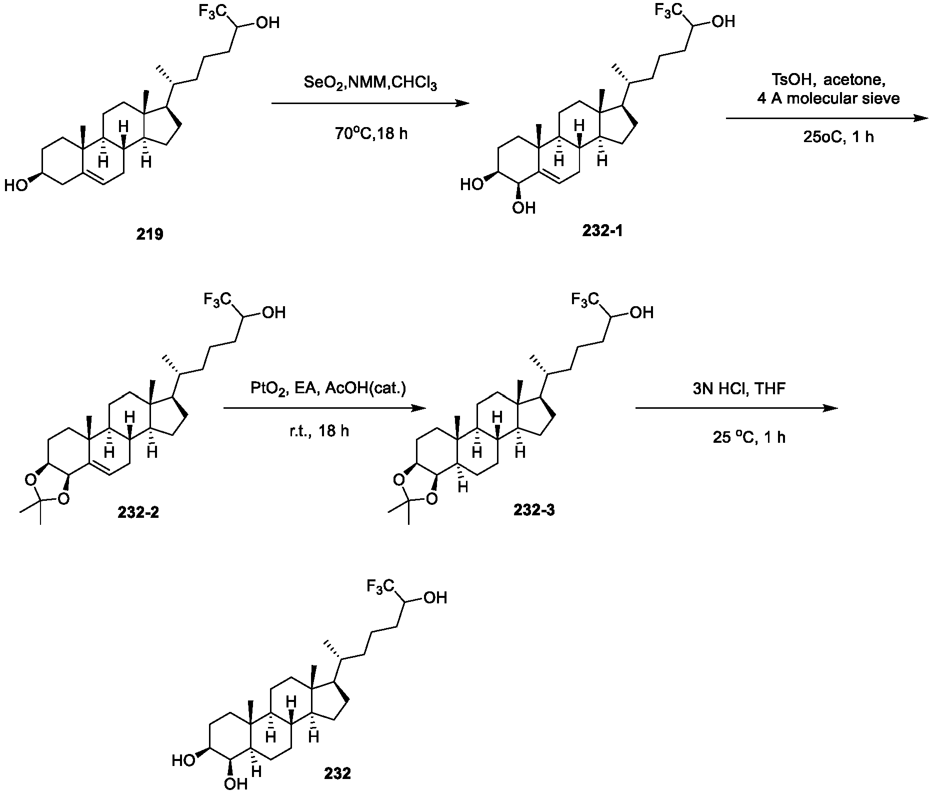

***Step 1*:** The compound 24-(2,2,2-trifluoro-1-hydroxyethyl)cholan-5(6)-en-3β-ol hexanal (**219**) (89 mg, 0.20 mmol, 1 *eq*) was dissolved in chloroform (10 mL), then selenium dioxide (33.46 mg, 0.30 mmol, 1.50 *eq*) and *N*-methylmorpholine (60.99 mg, 0.60 mmol, 3.0 *eq*) were added thereto, and the reaction system was stirred at 80°C for 18 hours. The completion of the reaction was monitored by TLC plate (petroleum ether: ethyl acetate = 5: 1). The reaction system was added with water (20 mL) and extracted with ethyl acetate (50 mL × 3). The organic phase was collected, dried over anhydrous sodium sulfate, and evaporated to dryness by rotary evaporation under vacuum to obtain a crude product. The crude product was purified by silica gel column chromatography (petroleum ether: ethyl acetate = 76:24) to obtain 24-(2,2,2-trifluoro-1-hydroxyethyl)-5α-cholan-3β,4β-diol (**232-1**) (40 mg, purity: 90%, yield: 41%) as a white solid. ¹H NMR (400 MHz, DMSO) δ 6.04 (d, *J =* 6.7 Hz, 1H), 5.49 (s, 1H), 4.42 (d, *J =* 6.2 Hz, 1H), 4.28 (d, *J =* 2.3 Hz, 1H), 3.91 - 3.81 (m, 2H), 3.25 (d, *J =* 9.6 Hz, 1H), 2.03 - 1.91 (m, 3H), 1.74 (dd, *J =* 22.2, 13.5 Hz, 3H), 1.58 - 1.34 (m, 12H), 1.27 - 1.18 (m, 4H), 1.11 (s, 3H), 0.98 (d, *J* = 10.4 Hz, 2H), 0.90 (d, *J* = 6.3 Hz, 3H), 0.86 - 0.79 (m, 1H), 0.65 (s, 3H).

***Step 2*:** The reactant 24-(2,2,2-trifluoro-1-hydroxyethyl)-5α-cholan-3β,4β-diol (**232-1**) (40 mg, 0.087 mmol, 1 *eq*) was dissolved in acetone (8 mL), then *p*-toluenesulfonic acid (11.61 mg, 0.061 mmol, 0.7 *eq*) and 4A molecular sieve were added thereto, and the reaction system was stirred at room temperature for 1 hour. The completion of the reaction was monitored by TLC plate (petroleum ether: ethyl acetate = 10:1), and the reaction was terminated by adding saturated sodium sulfite to quench. The reaction system was added with water (20 mL) and extracted with ethyl acetate (25 mL × 3). The organic phase was collected, dried over anhydrous sodium sulfate, and evaporated to dryness by rotary evaporation under vacuum to obtain a crude product. The crude product was dissolved in ethyl acetate and subjected to column chromatography (petroleum ether: ethyl acetate = 82:18) to obtain the crude product (6*R*)-6-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1,1,1-trifluoroheptan-2-ol (**232-2**) (35 mg, purity: 90%, yield: 72%) as a white solid.

***Step 3*:** The compound (6*R*)-6-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1,1,1-trifluoroheptan-2-ol (**232-2**) (16 mg, 0.032 mmol, 1.0 *eq*) was dissolved in ethyl acetate (5 mL), then platinum dioxide (10 mg, 0.044 mmol, 1.0 *eq*) was added thereto, and the reaction system was replaced with a hydrogen atmosphere and stirred at room temperature for 1 hour. The completion of the reaction was monitored by TLC plate (petroleum ether: ethyl acetate = 5:1). The reaction system was filtered, and the organic phase was collected and concentrated to obtain the crude product (6*R*)-6-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1,1,1-trifluoroheptan-2-ol (**232-3**) (16 mg) as a white solid. The crude product was directly used in the next step.

***Step 4*:** The reactant (6*R*)-6-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1,1,1-trifluoroheptan-2-ol (**232-3**) (16 mg, 0.03 mmol, 1.0 *eq*) was dissolved in tetrahydrofuran (5 mL), then dilute hydrochloric acid (0.2 mL, 3 mol/L) was added thereto, and the reaction system was stirred at room temperature for 40 minutes. The completion of the reaction was monitored by TLC plate (petroleum ether: ethyl acetate = 2:1). The reaction system was added with water (10 mL), and the aqueous phase was extracted with ethyl acetate (25 mL × 3). The ethyl acetate phases were combined and washed with saturated brine (10 mL × 3). The ethyl acetate phase was dried over anhydrous sodium sulfate, filtered, and concentrated to obtain a crude product. The crude product was purified by column chromatography (petroleum ether: ethyl acetate = 73:27) to obtain 24-(2,2,2-trifluoro-1-hydroxyethyl)-5α-cholan-3β,4β-diol (**232**) (12.01 mg, purity: 96.9%, yield: 79.06%) as a white solid.

¹H NMR (400 MHz, DMSO) δ 6.02 (d, *J* = 6.8 Hz, 1H), 4.29 (d, *J* = 6.0 Hz, 1H), 3.90 (d, *J* = 3.1 Hz, 1H), 3.85 (s, 1H), 3.47 (d, *J =* 2.4 Hz, 1H), 3.27 (dd, *J =* 6.2, 4.5 Hz, 1H), 1.91 (d, *J* = 12.2 Hz, 1H), 1.81 - 1.49 (m, 7H), 1.44 - 1.15 (m, 15H), 1.11- 0.99 (m, 4H), 0.94 (s, 3H), 0.88 (d, *J=* 6.4 Hz, 3H), 0.61 (s, 3H), 0.56 (dd, *J =* 15.2, 7.3 Hz, 1H).

¹³C NMR (101 MHz, DMSO) *δ* 124.67, 82.56, 74.28, 71.92, 56.59, 56.14, 56.00, 55.16, 42.61, 37.48, 35.57, 35.55, 35.49, 32.67, 28.22, 26.43, 26.19, 24.29, 21.43, 20.64, 19.54, 18.95, 18.90, 15.18, 12.34, 0.57. LCMS [M+H-2H₂O]⁺ = 425.5.

### Example 233

### Preparation of 24-[cyclopropyl(hydroxy)methyl]-5α-cholan-3β,4β-diol (compound 233)

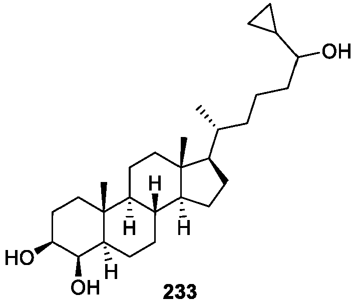

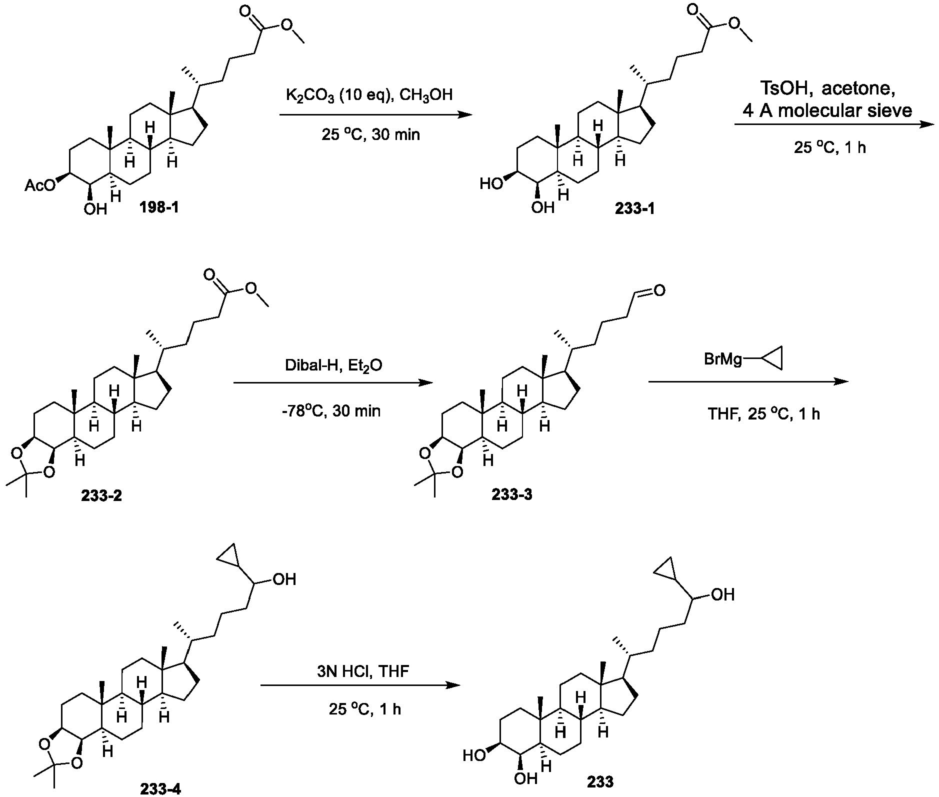

***Step 1*:** The reactant methyl (5*R*)-5-[(1*R*,3a*S*,3b*R*,5a*R*,6*R*,7*S*,9a*R*,9b*S*,11a*R*)-7-acetoxy-6-hydroxy-3a,9a,11a-trimethylhexadecahydro-1*H*-cyclopenta[1,2-*a*]phenanthren-1-yl]hexanoate (**198-1**) (200 mg, 0.43 mmol, 1.0 *eq*) was dissolved in methanol (30 mL), then potassium carbonate (597.45 mg, 4.323 mmol, 10.0 *eq*) was added thereto, and the reaction system was stirred at room temperature for 30 minutes. The completion of the reaction was monitored by TLC plate (petroleum ether: ethyl acetate = 5:1). The reaction system was added with water (10 mL), and the aqueous phase was extracted with ethyl acetate (25 mL × 3). The ethyl acetate phases were combined and washed with saturated brine (10 mL × 3). The ethyl acetate phase was dried over anhydrous sodium sulfate, filtered, and concentrated to obtain a crude product. The crude product was purified by column chromatography (petroleum ether: ethyl acetate = 77:23 to 75:25) to obtain methyl (5*R*)-5-[(1*R*,3a*S*,3b*S*,5a*R*,6*R*,7*S*,9a*R*,9b*S*,11a*R*)-6,7-dihydroxy-9a,11a-dimethylhexadecahydro-1*H-*cyclopenta[1,2-*a*]phenanthren-1-yl]hexanoate (**233-1**) (150 mg, purity: 80%, yield: 66%) as a white solid. ¹H NMR (400 MHz, CDCl₃) δ 3.74 (s, 1H), 3.66 (s, 3H), 3.56 (s, 1H), 2.27 (dd, *J =* 16.8, 8.6 Hz, 2H), 1.95 (d, *J =* 12.5 Hz, 1H), 1.84 - 1.67 (m, 8H), 1.53 (s, 4H), 1.43 - 1.31 (m, 6H), 1.29 -1.19 (m, 3H), 1.08 (dd, *J=* 18.6, 8.9 Hz, 5H), 1.02 (s, 3H), 0.92 (d, *J =* 6.5 Hz, 3H), 0.64 (s, 3H), 0.60 (dd, *J =* 15.1, 7.3 Hz, 1H).

***Step 2*:** The reactant methyl (5*R*)-5-[(1*R*,3a*S*,3b*S*,5a*R*,6*R*,7*S*,9a*R*,9b*S*,11a*R*)-6,7-dihydroxy-9a,11a-dimethylhexadecahydro-1*H*-cyclopenta[1,2-*a*]phenanthren-1-yl]hexanoate (**233-1**) (150 mg, 0.36 mmol, 1 *eq*) was dissolved in acetone (8 mL), then *p*-toluenesulfonic acid (47.48 mg, 0.250 mmol, 0.7 *eq*) and 4A molecular sieve were added thereto, and the reaction system was stirred at room temperature for 1 hour. The completion of the reaction was monitored by TLC plate (petroleum ether: ethyl acetate = 10:1), and the reaction was terminated by adding saturated sodium sulfite to quench. The reaction system was added with water (20 mL) and extracted with ethyl acetate (25 mL × 3). The organic phase was collected, dried over anhydrous sodium sulfate, and evaporated to dryness by rotary evaporation under vacuum to obtain a crude product. The crude product was dissolved in ethyl acetate and subjected to column chromatography (petroleum ether: ethyl acetate = 95:5) to obtain methyl (5*R*)-5-[(3a*S*,5a*R*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]hexanoate (**233-2**) (126 mg, purity: 90%, yield: 69%) as a white solid.

¹H NMR (400 MHz, CDCl₃) δ 3.98 (d, *J* = 15.0 Hz, 2H), 3.67 (s, 3H), 2.27 (dd, *J =* 17.0, 8.5 Hz, 1H), 1.95 (d, *J=* 12.9 Hz, 1H), 1.83 - 1.65 (m, 3H), 1.51 (s, 2H), 1.42 (dd, *J=* 24.1, 13.0 Hz, 3H), 1.30 (s, 1H), 1.24 (t, *J =* 12.4 Hz, 2H), 1.04 (s, 2H), 0.92 (d, *J =* 6.4 Hz, 2H), 0.85 (d, *J =* 12.5 Hz, 1H), 0.66 (s, 1H), 0.59 (s, 1H).

***Step 3*:** The compound methyl (5*R*)-5-[(3a*S*,5a*R*,5b*S*,7a*R,*8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-d][1,3]dioxol-8-yl]hexanoate (**233-2**) (158 mg, 0.34 mmol, 1.0 *eq*) was dissolved in diethyl ether (20 mL), and the reaction system was cooled to -78°C. Diisobutylaluminium hydride (1.029 mL, 1.02 mmol, 1.0 *eq*, 1 mol/L) was then added thereto, and the reaction system was stirred at -78°C for 30 minutes. The completion of the reaction was monitored by TLC plate (petroleum ether: ethyl acetate = 10:1). The reaction system was diluted with ethyl acetate (20 mL), quenched with saturated sodium tartrate (20 mL), and extracted with ethyl acetate (10 mL * 3). The organic phase was collected, washed twice with water (20 mL), dried over anhydrous sodium sulfate, and evaporated to dryness by rotary evaporation under vacuum to obtain a crude product. The crude product was purified by silica gel column chromatography (petroleum ether: ethyl acetate = 96:4 to 95:5) to obtain (5*R*)-5-[(3a*S*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]hexanal (**233-3**) (125 mg, purity: 90%, yield: 76%) as a white solid.

¹H NMR (400 MHz, CDCl₃) δ 9.76 (s, 1H), 4.12 (q, *J* = 7.2 Hz, 0H), 3.99 (d, *J* = 14.3 Hz, 2H), 2.47 - 2.32 (m, 2H), 1.95 (d, *J* = 12.1 Hz, 1H), 1.85 - 1.61 (m, 4H), 1.51 (s, 3H), 1.43 (dd, *J =* 20.7, 12.2 Hz, 3H), 1.30 (s, 3H), 1.28 - 1.20 (m, 2H), 1.09 (dd, *J =* 15.8, 6.7 Hz, 2H), 1.04 (s, 3H), 1.02 (s, 1H), 0.93 (d, *J =* 6.5 Hz, 3H), 0.66 (s, 3H), 0.61 (d, *J =* 9.0 Hz, 1H).

***Step 4*:** The compound (5*R*)-5-[(3a*S*,5a*R*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]hexanal (**233-3**) (50 mg, 0.116 mmol, 1.0 *eq*) was dissolved in tetrahydrofuran (8 mL), and the reaction system was cooled to 0°C. Cyclopropylmagnesium bromide (0.580 mL, 0.580 mmol, 5 *eq*) was then slowly added thereto, and the reaction system was stirred at room temperature for 1 hour. The completion of the reaction was monitored by TLC plate (petroleum ether: ethyl acetate = 5:1). The reaction system was added with water (20 mL) and extracted with ethyl acetate (50 mL × 3). The organic phases were combined, washed with water (30 mL), dried, and concentrated to obtain a crude product. The crude product was subjected to silica gel column chromatography (petroleum ether: ethyl acetate = 92:8) to obtain (5*R*)-5-[(3a*S*,5a*R*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]-1-cyclopropylhexan-1-ol (**233-4**) (49 mg, purity: 90%, yield: 80%) as a white solid. ¹H NMR (400 MHz, CDCl₃) δ 4.04 - 3.93 (m, 2H), 2.89 - 2.82 (m, 1H), 1.96 (d, *J =* 12.4 Hz, 1H), 1.84 - 1.73 (m, 2H), 1.66 (t, *J =* 13.9 Hz, 1H), 1.51 (s, 2H), 1.42 (ddd, *J =* 25.8, 16.4, 9.2 Hz, 3H), 1.31 (s, 1H), 1.29 - 1.20 (m, 4H), 1.14 - 1.06 (m, 2H), 1.04 (s, 2H), 0.91 (d, *J* = 6.5 Hz, 2H), 0.66 (s, 1H), 0.61 (dd, *J* = 15.3, 7.5 Hz, 1H), 0.56 - 0.44 (m, 1H), 0.24 (dtd, *J =* 13.1, 9.0, 4.4 Hz, 1H).

***Step 5*:** The reactant (5*R*)-5-[(3a*S*,5a*R*,5b*S*,7a*R*,8*R*,10a*S*,10b*S*,12a*R*,12b*R*)-2,2,5a,7a - tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]-1-cyclopropylhexan-1-ol (**233-4**) (25 mg, 0.05 mmol, 1.0 *eq*) was dissolved in tetrahydrofuran (5 mL), then dilute hydrochloric acid (0.2 mL, 3 mol/L) was added thereto, and the reaction system was stirred at room temperature for 40 minutes. The completion of the reaction was monitored by TLC plate (petroleum ether: ethyl acetate = 2:1). The reaction system was added with water (10 mL), and the aqueous phase was extracted with ethyl acetate (25 mL × 3). The EA phases were combined and washed with saturated brine (10 mL × 3). The ethyl acetate phase was dried over anhydrous sodium sulfate, filtered, and concentrated to obtain a crude product. The crude product was purified by column chromatography (petroleum ether: ethyl acetate = 73:27) to obtain 24-[cyclopropyl(hydroxy)methyl]-5α-cholan-3β,4β-diol (**233**) (3.38 mg, purity: 95.09%, yield: 14.17%) as a white solid.

¹H NMR (400 MHz, DMSO) δ 4.32 - 4.25 (m, 2H), 3.90 (d, *J =* 2.6 Hz, 1H), 3.47 (s, 1H), 3.29 - 3.27 (m, 1H), 2.78 (dd, *J =* 7.3, 4.0 Hz, 1H), 1.91 (d, *J=* 12.4 Hz, 1H), 1.74 (d, *J* = 15.2 Hz, 1H), 1.69 - 1.53 (m, 5H), 1.40 - 1.22 (m, 17H), 1.08 - 0.98 (m, 4H), 0.94 (s, 3H), 0.87 (d, *J =* 6.5 Hz, 3H), 0.61 (s, 3H), 0.58 - 0.50 (m, 1H), 0.36 - 0.26 (m, 2H), 0.23 - 0.07 (m, 2H). ¹³C NMR (101 MHz, DMSO) *δ* 74.28, 73.66, 71.92, 56.60, 56.19, 55.16, 48.91, 35.57, 35.55, 32.53, 30.29, 28.28, 26.44, 26.19, 24.29, 22.22, 20.64, 20.51, 18.99, 18.97, 18.08, 12.30, 0.64. LCMS [M+H-2H₂O]⁺ = 397.5.

### Example 234

### Preparation of 24-[cyclopropyl(hydroxy)methyl]-5α-cholan-3β,4β-diol (compound 234)

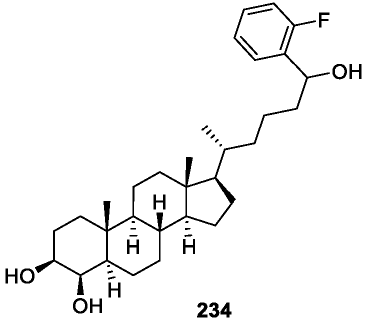

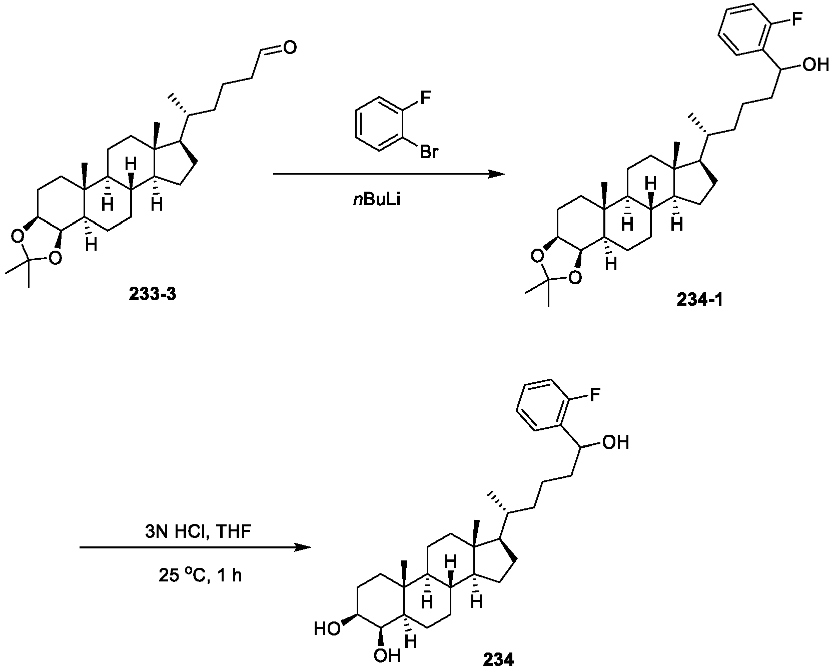

***Step 1*:** The compound 2-bromo-1-fluorobenzene (163 mg, 0.93 mmol, 10 *eq*) was dissolved in tetrahydrofuran (5 mL). The reaction system was replaced with nitrogen three times and cooled to -78°C in a dry ice bath. A 2.5 M solution of *n*-butyllithium in *n*-hexane (0.41 mL, 1.02 mmol, 11 *eq*) was then added dropwise thereto with a syringe, and the reaction mixture was reacted at the same temperature for 1 hour. The compound (5*R*)-5-[(3a*S*,5a*R*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]hexanal (**233-3**) (40 mg, 0.093 mmol, 1.0 *eq*) was dissolved in tetrahydrofuran (1 mL) and added to the above system. The reaction mixture was reacted at the same temperature for 30 minutes and then naturally warmed to room temperature. The completion of the reaction was monitored by TLC plate (petroleum ether: ethyl acetate = 5:1). The reaction system was added with ice water (20 mL) and extracted with ethyl acetate (10 mL × 3). The organic phases were combined, washed with saturated brine (30 mL), dried, and concentrated to obtain a crude product. The crude product was subjected to flash column chromatography (petroleum ether: ethyl acetate = 92:8) to obtain (5*R*)-5-[(3a*S*,5a*R*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]-1-(2-fluorophenyl)hexan-1-ol (**234-1**) (30 mg, purity: 80%, yield: 49%) as a white solid. ¹H NMR (400 MHz, CDCl₃) *δ* 7.45 (d, *J* = 7.3 Hz, 1H), 7.23 (d, *J =* 7.0 Hz, 1H), 7.15 (t, *J =* 7.4 Hz, 1H), 7.02 (m, 1H), 5.00 (d, *J =* 7.0 Hz, 1H), 3.98 (d, *J =* 14.3 Hz, 2H), 1.75 (m, 5H), 1.67 (d, *J=* 15.3 Hz, 3H), 1.51 (s, 4H), 1.41 (dd, *J =* 23.4, 11.9 Hz, 7H), 1.28 (m, 12H), 1.05 (d, *J =* 13.5 Hz, 6H), 0.88 (m, 5H), 0.64 (s, 3H).

***Step 2*:** The reactant (5*R*)-5-[(3a*S*,5a*R*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]-1-(2-fluorophenyl)hexan-1-ol (**234-1**) (25 mg, 0.05 mmol, 1.0 *eq*) was dissolved in tetrahydrofuran (5 mL), then dilute hydrochloric acid (0.3 mL, 3 mol/L) was added thereto, and the reaction system was stirred at room temperature for 60 minutes. The completion of the reaction was monitored by TLC plate (petroleum ether: ethyl acetate = 2:1). The reaction system was added with water (10 mL), and the aqueous phase was extracted with ethyl acetate (25 mL × 3). The ethyl acetate phases were combined and washed with saturated brine (10 mL). The ethyl acetate phase was dried over anhydrous sodium sulfate, filtered, and concentrated to obtain a crude product. The crude product was purified by column chromatography (petroleum ether: ethyl acetate = 73:27) to obtain 24-[cyclopropyl(hydroxy)methyl]-5α-cholan-3β,4β-diol (**234**) (19.5 mg, purity: 93.21%, yield: 78.69%) as a white solid. ¹H NMR (400 MHz, CDCl₃) *δ* 7.47 (t, *J =* 7.5 Hz, 1H), 7.25 (m, 1H), 7.17 (t, *J =* 7.4 Hz, 1H), 7.09 (m, 1H), 5.22 (s, 1H), 4.79 (s, 1H), 4.30 (d, *J* = 5.2 Hz,1H), 3.91 (s, 1H), 3.46 (s, 1H), 3.27 (m, 1H), 1.89 (d, *J =* 12.3 Hz, 1H), 1.64 (s, 3H), 1.54 (s, 5H), 1.33 (s, 6H), 1.22 (t, *J* = 14.2 Hz, 7H), 1.01 (d, *J* = 9.9 Hz, 3H), 0.93 (s, 3H), 0.84 (d, *J =* 3.3 Hz, 4H), 0.74 (d, *J* = 6.2 Hz, 1H), 0.59 (s, 3H). ¹³C NMR (101 MHz, DMSO) δ 160.73, 133.32, 128.00, 124.78, 121.54, 115.12, 74.28, 71.93, 56.58, 56.15, 55.14, 49.99, 48.90, 42.60, 37.47, 35.55, 32.52, 29.43, 28.24, 28.23, 26.43, 26.19, 24.26, 22.04, 21.90, 20.79, 20.64, 18.87, 15.17, 12.28. LCMS [M+H-2H₂O]⁺ = 451.

### Example 235

### Preparation of 25-hydroxy-4β-hydroxy-3β-hydroxy-5α-cholest-6-one (compound 235)

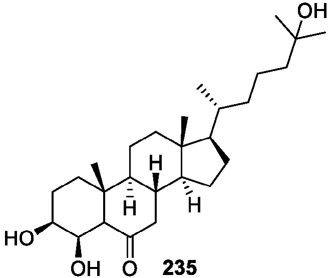

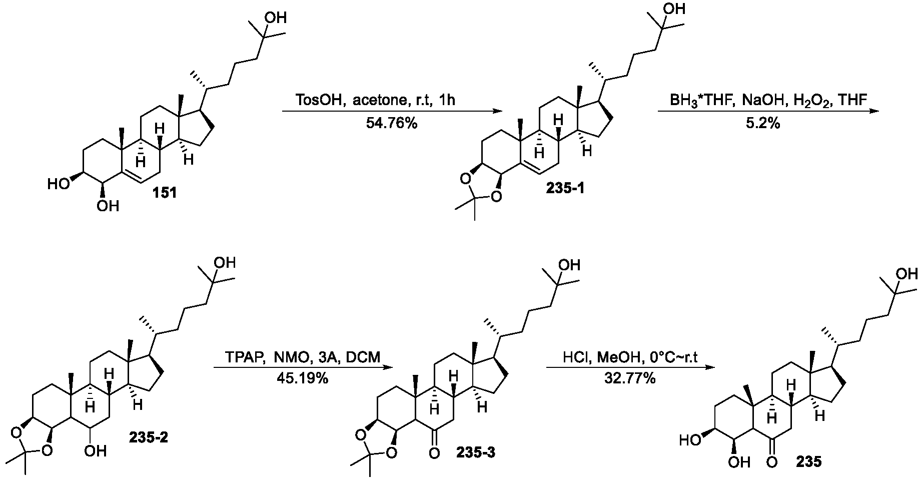

**Step 1:** Cholest-6(5)-en-3β,4β,25-triol (**151**) (300 mg, 0.717 mmol) was dissolved in acetone (30 mL), then 3A molecular sieve (100 mg, 3.583 mmol) and anhydrous p-toluic acid (7.3 mg, 0.054 mmol) were added thereto, and the reaction mixture was stirred at room temperature for 1 hour. The completion of the reaction was monitored by TLC (petroleum ether: ethyl acetate = 2:1). The reaction mixture was added with water, extracted twice with ethyl acetate, dried over anhydrous sodium sulfate, concentrated, and subjected to column chromatography (petroleum ether: ethyl acetate = 100% to 20%) to obtain compound (6R)-6-[(3a*S*,5a*R*,5b*S*,7a*R*,8*R*,10a*S*,10b*S*,12b*R*)-2,2,5a,7a-tetramethyl-4,5,5a,Sb,6,7,7a,8,9,10,10a,10b,11,12b-tetradecahydro-3a*H-*cyclopenta[1',2':1,2]phenanthro[7,8-*d*][1,3]dioxol-8-yl]-2-methylheptan-2-ol (**235-1**) (200 mg, 0.392 mmol, 54.76%) as a white solid. ¹H NMR (399 MHz, Chloroform-d) δ 5.78 (dd, *J* = 4.8, 2.2 Hz, 1H), 4.39 (d, *J =* 5.7 Hz, 1H), 1.98 (dd, *J =* 15.9, 12.6 Hz, 1H), 1.67 - 1.57 (m, 4H), 1.55 (s, 4H), 1.51 (s, 3H), 1.47 - 1.37 (m, 5H), 1.33 (s, 3H), 1.26 (d, *J* = 3.6 Hz, 3H), 1.20 (s, 6H), 1.15 (s, 3H), 1.08 - 1.00 (m, 3H), 0.92 (d, *J =* 6.6 Hz, 3H), 0.82 (d, *J =* 6.7 Hz, 3H), 0.68 (s, 3H).

**Step 2:** (6*R*)-6-[(3a*S*,5a*R*,5b*S*,7a*R*,8*R*,10a*S*,10b*S*,12b*R*)-2,2,5a,7a-Tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-2-methylheptan-2-ol (**235-1**) (50 mg, 0.109 mmol) was dissolved in anhydrous tetrahydrofuran (5 mL). The reaction mixture was cooled to 0°C under a nitrogen atmosphere, added with borane-tetrahydrofuran (49.68 mg, 0.654 mmol), stirred at room temperature for 3 hours, then cooled to 0°C, added with sodium hydroxide (0.436 mL, 1.308 mmol) and hydrogen peroxide (85%) (44.48 mg, 1.308 mmol), and stirred at room temperature for 16 hours. The completion of the reaction was monitored by TLC (petroleum ether: ethyl acetate = 3:1). The reaction mixture was added with water, extracted twice with ethyl acetate, dried over anhydrous sodium sulfate, concentrated, and subjected to column chromatography (petroleum ether: ethyl acetate = 100% to 20%) to obtain (3a*S*,5a*R*,5b*S*,7a*R*,8*R*,10a*S*,10b*S*,12a*S*,12b*R*)-8-[(2*R*)-6-hydroxy-6-methylheptan-2-yl]-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-12-ol (**235-2**) (3 mg, 0.006 mmol, 5.20%). ¹H NMR (399 MHz, Chloroform-d) δ 4.21 (d, *J =* 6.1 Hz, 1H), 4.16 (s, 1H), 3.82 (dd, *J =* 10.5, 5.9 Hz, 1H), 2.03 - 1.93 (m, 1H), 1.82 - 1.65 (m, 4H), 1.47 (s, 4H), 1.43 - 1.33 (m, 6H), 1.31 (s, 5H), 1.27 - 1.22 (m, 3H), 1.19 (s, 6H), 1.13 (s, 3H), 1.06 - 0.99 (m, 3H), 0.90 (d, *J=* 6.5 Hz, 3H), 0.87 - 0.76 (m, 3H), 0.66 (s, 3H).

**Step 3:** (3a*S*,5a*R*,5b*S*,7a*R*,8*R*,10a*S*,10b*S*,12a*S*,12b*R*)-8-[(2*R*)-6-Hydroxy-6-methylheptan-2-yl]-2,2,5a,7a-tetramethyl-4,5,5a,5b,6,7,7a,8,9,10,10a,10b,11,12,12a,12b-hexadecahydro-3a*H*-cyclopenta[1',2':1,2]phenanthro[7,8-*d*][1,3]dioxol-12-ol (**235-2**) (20 mg, 0.042 mmol) was dissolved in dichloromethane (5 mL), and the reaction mixture was cooled to 0°C under a nitrogen atmosphere. Tetrapropylammonium perruthenate (7.37 mg, 0.021 mmol), *N*-methylmorpholine oxide (4.94 mg, 0.042 mmol), and 3A molecular sieve (100 mg, 0.210 mmol) were added thereto, and the reaction mixture was stirred at room temperature for 2 hours. The completion of the reaction was monitored by TLC (petroleum ether: ethyl acetate = 3:1). The reaction mixture was directly filtered, concentrated, and subjected to column chromatography (petroleum ether: ethyl acetate = 0 to 80%) to obtain compound (3a*S*,5a*R*,5b*S*,7a*R*,8*R*,10a*S*,10b*S*,12a*R*,12b*R*)-8-[(2*R*)-6-hydroxy-6-methylheptan-2-yl]-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-12-one (**235-3**) (10 mg, 0.019 mmol, 45.19%). ¹H NMR (399 MHz, CDCl₃) δ 4.40 - 4.22 (m, 2H), 2.31 - 2.21 (m, 2H), 2.11 (d, *J* = 9.5 Hz, ¹H), 2.02 (d, *J =* 12.5 Hz, 1H), 1.89 - 1.79 (m, 1H), 1.72 - 1.55 (m, 2H), 1.50 (s, 5H), 1.42 (s, 3H), 1.36 (dd, *J =* 19.4, 6.7 Hz, 5H), 1.30 (s, 3H), 1.23 (s, 3H), 1.19 (s, 6H), 1.13 - 0.98 (m, 4H), 0.91 (d, *J =* 6.5 Hz, 3H), 0.85 (s, 3H), 0.63 (s, 3H).

**Step 4:** (3a*S*,5a*R*,5b*S*,7a*R*,8*R*,10a*S*,10b*S*,12a*R*,12b*R*)-8-[(2*R*)-6-Hydroxy-6-methylheptan-2-yl]-2,2,5a,7a-tetramethyl-4,5,5a,5b,6,7,7a,8,9,10,10a,10b,11,12,12a,12b-hexadecahydro-3a*H*-cyclopenta[1',2':1,2]phenanthro[7,8-*d*][1,3]dioxol-12-one (**235-3**) (3 mg, 0.006 mmol) was dissolved in methanol (5 mL), then hydrochloric acid (0.063 mL, 0.063 mmol) was added thereto, and the reaction mixture was stirred at room temperature for 1 hour. The completion of the reaction was monitored by TLC (petroleum ether: ethyl acetate = 1:1). The reaction mixture was extracted twice with ethyl acetate, dried over anhydrous sodium sulfate, concentrated, and subjected to column chromatography (petroleum ether: ethyl acetate = 0 to 80%) to obtain compound 25-hydroxy-4β-hydroxy-3β-hydroxy-5α-cholest-6-one (**235**) (10 mg, 0.002 mmol, 32.77%). ¹H NMR (399 MHz, CDCl₃) δ 4.00 (d, *J=* 8.8 Hz, 2H), 2.33 - 2.24 (m, 2H), 2.21 (d, *J =* 12.4 Hz, 1H), 2.03 (d, *J =* 12.4 Hz, 1H), 1.77 (d, *J =* 14.7 Hz, 4H), 1.67 - 1.51 (m, 7H), 1.38 (dt, *J =* 27.8, 12.6 Hz, 9H), 1.19 (s, 3H), 1.14 - 0.99 (m, 4H), 0.91 (d, *J* = 6.5 Hz, 3H), 0.87 (s, 3H), 0.83 (s, 1H), 0.64 (s, 3H). ¹³C NMR (100 MHz, CDCl₃) δ 214.09, 77.30, 76.98, 76.66, 71.11, 56.89, 56.07, 44.30, 43.10, 41.07, 40.80, 39.57, 37.15, 36.30, 35.62, 29.16, 28.08, 25.52, 23.92, 23.52, 21.27, 20.73, 18.58, 11.96, 0.99. ELSD-MS: [M+H]⁺ = 435.0.

### Example 237

### Preparation of (1R,3aS,5aR,7S,9aR,11aR)-1-[(2R)-6-hydroxy-6-methylheptan-2-yl]-3a,6,6,9a,11a-pentamethylhexadecahydro-1H-cyclopenta[1,2-a]phenanthrene-4,7-diol (compound 237)

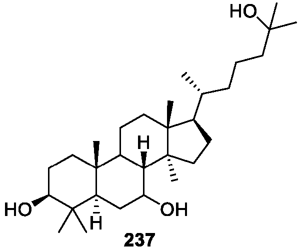

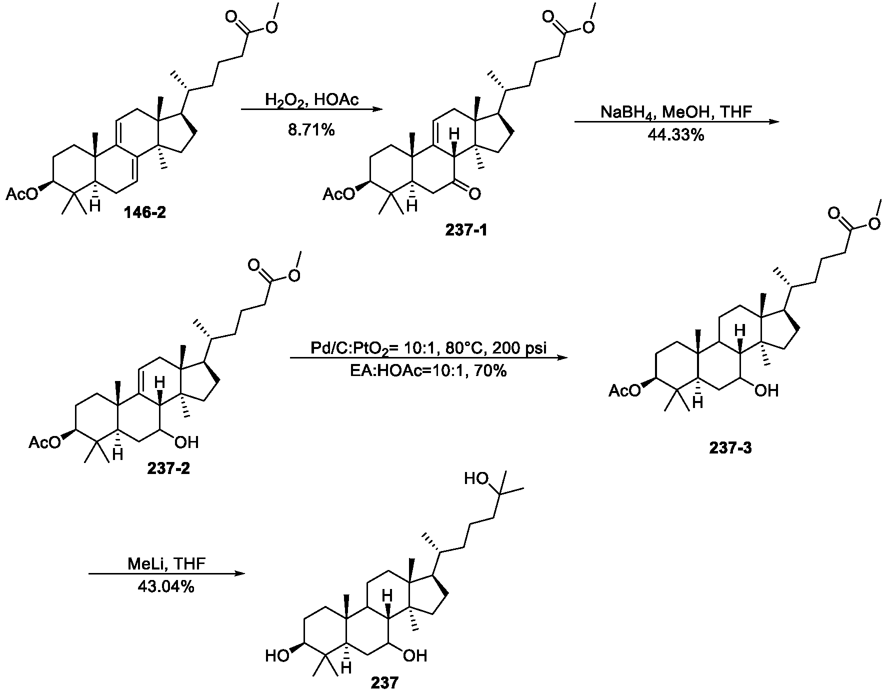

**Step 1:** (1*R*,3a*R*,5a*R*,7*S*,9a*S*,11a*R*)-1-[(2*R*)-6-Methoxy-6-oxohexan-2-yl]-3a,6,6,9a,11a-pentamethyl-2,3,3a,5,5a,6,7,8,9,9a,11,11a-dodecahydro-1*H*-cyclopenta[1,2-i]phenanthren-7-yl acetate (**146-2**) (500 mg, 1.03 mmol) was weighed and dissolved in acetic acid (10 mL) in a 100 mL single-necked flask, and then hydrogen peroxide (1.6 mL, 14.832 mmol) was added dropwise thereto at room temperature. After the addition was completed, the reaction mixture was stirred at room temperature. The completion of the reaction was monitored by TLC (petroleum ether: ethyl acetate = 5:1). The reaction mixture was lyophilized, then added with saturated sodium bicarbonate aqueous solution to adjust the pH to 7, and extracted with ethyl acetate. The organic phase was separated, dried, concentrated, and purified by column chromatography (petroleum ether: ethyl acetate = 50:1 to 20:1) to obtain a crude product mixture (220 mg), which was then slurried with diethyl ether (1 mL) and filtered to obtain (1*R*,3a*S*,5a*R*,7*S*,9a*S*,11a*R*)-1-[(2*R*)-6-methoxy-6-oxohexan-2-yl]-3a,6,6,9a,11a-pentamethyl-4-oxo-2,3,3a,3b,4,5,5a,6,7,8,9,9a,11,11a-tetradecahydro-1*H-*cyclopenta[1,2-i]phenanthren-7-yl acetate (**237-1**) (44.9 mg, 0.09 mmol, 8.71%) as a white solid. ¹H NMR (399 MHz, Chloroform-d) δ 5.52 - 5.46 (m, 1H), 4.58 (dd, *J* = 11.1, 4.9 Hz, 1H), 3.65 (d, *J =* 0.8 Hz, 3H), 3.13 (s, 1H), 3.02 - 2.90 (m, 1H), 2.40 - 2.33 (m, 1H), 2.30 - 2.20 (m, 3H), 2.05 (d, *J =* 2.0 Hz, 4H), 2.00 (d, *J=* 3.5 Hz, 2H), 1.89 (d, *J =* 13.4 Hz, 2H), 1.77 - 1.67 (m, 3H), 1.48 (t, *J* = 5.2 Hz, 1H), 1.46 - 1.34 (m, 4H), 1.24 - 1.15 (m, 1H), 1.09 (d, *J =* 3.8 Hz, 4H), 1.05 - 1.00 (m, 1H), 0.92 (d, *J=* 10.3 Hz, 6H), 0.86 (d, *J =* 6.0 Hz, 3H), 0.81 (d, *J* = 1.0 Hz, 3H), 0.61 (s, 3H). ¹³C NMR (100 MHz, CDCl₃) δ 209.12, 174.29, 170.87, 142.50, 121.71, 80.12, 77.33, 77.01, 76.70, 56.19, 51.42, 50.46, 47.58, 47.15, 47.12, 46.62, 44.57, 42.49, 38.93, 38.54, 38.31, 37.96, 37.83, 37.34, 36.99, 35.83, 35.67, 35.61, 35.57, 35.33, 34.47, 34.34, 28.45, 28.34, 27.52, 27.25, 26.01, 24.77, 24.60, 23.88, 21.70, 21.23, 20.37, 18.36, 18.25, 17.71, 16.99, 16.71, 15.67, 15.14.

**Step 2:** (1*R*,3a*S*,5a*R*,7*S*,9a*S*,11a*R*)-1-[(2*R*)-6-Methoxy-6-oxohexan-2-yl]-3a,6,6,9a,11a-pentamethyl-4-oxo-2,3,3a,3b,4,5,5a,6,7,8,9,9a,11,11a-tetradecahydro-1*H-*cyclopenta[1,2-i]phenanthren-7-yl acetate (**237-1**) (91 mg, 0.182 mmol) was weighed and dissolved in a mixed solvent of methanol (2 mL) and tetrahydrofuran (4 mL), then sodium borohydride (6.88 mg, 0.182 mmol) was added thereto at room temperature, and the reaction mixture was stirred overnight. The completion of the reaction was detected by TLC (petroleum ether: ethyl acetate = 3:1). The reaction mixture was added with saturated sodium bicarbonate aqueous solution and ethyl acetate. The organic phase was separated, dried, concentrated, and purified by column chromatography (petroleum ether: ethyl acetate = 10:1 to 5:1) to obtain (1*R*,3a*S*,5a*R*,7*S*,9a*S*,11a*R*)-4-hydroxy-1-[(2*R*)-6-methoxy-6-oxohexan-2-yl]-3a,6,6,9a,11a-pentamethyl-2,3,3a,3b,4,5,5a,6,7,8,9,9a,11,11a-tetradecahydro-1*H-*cyclopenta[1,2-*i*]phenanthren-7-yl acetate (**237-2**) (45 mg, 0.081 mmol, 44.33%) as a white solid.

¹H NMR (399 MHz, Chloroform-d) δ 5.29 (d, *J* = 5.9 Hz, 1H), 4.46 (dd, *J =* 11.5, 4.2 Hz, 1H), 3.67 (s, 1H), 3.65 (s, 3H), 2.26 (q, *J =* 8.2 Hz, 2H), 2.16 - 2.07 (m, 1H), 2.03 (s, 4H), 1.93 (dd, *J =* 19.6, 8.4 Hz, 3H), 1.82 - 1.59 (m, 6H), 1.49 (d, *J =* 13.7 Hz, 4H), 1.44 - 1.25 (m, 7H), 1.07 (s, 3H), 1.06 - 0.91 (m, 2H), 0.89 (t, *J =* 3.3 Hz, 6H), 0.85 (d, *J J=* 3.0 Hz, 3H), 0.81 (s, 3H), 0.64 (s, 3H).

**Step 3:** (1*R*,3a*S*,5a*R*,7*S*,9a*S*,11a*R*)-4-Hydroxy-1-[(2*R*)-6-methoxy-6-oxohexan-2-yl]-3a,6,6,9a,11a-pentamethyl-2,3,3a,3b,4,5,5a,6,7,8,9,9a,11,11a-tetradecahydro-1*H-*cyclopenta[1,2-*i*]phenanthren-7-yl acetate (**237-2**) (20 mg, 0.040 mmol) was weighed and dissolved in a mixed solvent of ethyl acetate (3 mL) and acetic acid (0.3 mL), and Pd/C (10% Pd, containing 40 to 60% water) (30 mg, 0.282 mmol) and platinum dioxide (3 mg, 0.013 mmol) were added thereto. The reaction system was then replaced with hydrogen, heated to 80°C, and stirred when the pressure was increased to 200 psi. The reaction was monitored by HNMR. After the reaction mixture was stirred overnight (about 48 hours * 3), the heating was stopped. The reaction mixture was cooled to room temperature and filtered. The filtrate was added with saturated sodium bicarbonate aqueous solution and extracted with ethyl acetate. The organic phase was separated, dried, and concentrated to obtain (1*R*,3a*S*,5a*R*,7*S*,9a*R*,11a*R*)-4-hydroxy-1-[(2*R*)-6-methoxy-6-oxohexan-2-yl]-3a,6,6,9a,11a-pentamethylhexadecahydro-1*H*-cyclopenta[1,2-*i*]phenanthren-7-yl acetate (**237-3**) (15.6 mg, 0.029 mmol, 70%). ¹H NMR (400 MHz, Chloroform-d) δ 4.50 - 4.40 (m, 1H), 3.65 (s, 3H), 3.59 (d, *J=* 4.2 Hz, 1H), 2.26 (q, *J=* 8.4 Hz, 2H), 2.03 (s, 3H), 1.90 - 1.82 (m, 1H), 1.63 (d, *J=* 6.7 Hz, 6H), 1.43 (s, 2H), 1.38 (s, 2H), 1.35 (s, 3H), 1.31 (s, 1H), 1.26 (s, 3H), 1.23 (s, 3H), 1.09 - 1.02 (m, 2H), 0.92 (s, 3H), 0.88 (d, *J=* 4.9 Hz, 3H), 0.86 (d, *J=* 1.2 Hz, 6H), 0.84 (s, 3H), 0.81 (s, 1H), 0.75 (s, 3H). ¹³C NMR (100 MHz, Chloroform-d) δ 174.33, 170.98, 110.00, 47.26, 46.18, 45.96, 37.61, 36.57, 35.73, 34.49, 31.71, 31.49, 23.97, 21.71.

**Step 4:** (1*R*,3a*S*,5a*R*,7*S*,9a*R*,11a*R*)-4-Hydroxy-1-[(2*R*)-6-methoxy-6-oxohexan-2-yl]-3a,6,6,9a,11a-pentamethylhexadecahydro-1*H*-cyclopenta[1,2-*i*]phenanthren-7-yl acetate (**237-3**) (25 mg, 0.050 mmol) was dissolved in THF (3 mL). The reaction mixture was cooled in an ice bath, and methyllithium (0.464 mL, 0.743 mmol) was added dropwise thereto. After the dropwise addition was completed, the completion of the reaction was detected by TLC (dichloromethane: methanol = 10:1). The reaction mixture was added with saturated sodium bicarbonate aqueous solution and extracted with ethyl acetate. The organic phase was separated, dried, concentrated, and purified by column chromatography (petroleum ether: ethyl acetate = 10:1 to 1:1) to obtain (1*R*,3a*S*,5a*R*,7*S*,9a*R*,11a*R*)-1-[(2*R*)-6-hydroxy-6-methylheptan-2-yl]-3a,6,6,9a,11a-pentamethylhexadecahydro-1*H*-cyclopenta[1,2-*a*]phenanthrene-4,7-diol (**237**) (12 mg, 0.021 mmol, 43.04%). ¹H NMR (400 MHz, Methanol-d₄) δ 3.45 (td, *J =* 10.4, 4.8 Hz, 1H), 3.16 - 3.08 (m, 1H), 1.94 - 1.78 (m, 2H), 1.68 - 1.49 (m, 8H), 1.41 (d, *J =* 13.4 Hz, 7H), 1.27 (q, *J =* 12.6, 10.3 Hz, 4H), 1.15 (s, 6H), 1.05 - 0.96 (m, 3H), 0.94 (d, *J* = 4.1 Hz, 6H), 0.90 (d, *J* = 5.3 Hz, 3H), 0.87 (s, 3H), 0.86 - 0.82 (m, 1H), 0.79 (d, *J* = 7.6 Hz, 6H). ¹³C NMR (100 MHz, Methanol-d₄) δ 78.12, 72.24, 70.04, 52.10, 49.88, 45.64, 45.57, 43.86, 38.35, 37.32, 36.81, 36.34, 36.00, 35.87, 31.87, 31.78, 28.14, 27.83, 27.67, 27.23, 26.91, 20.59, 19.95, 17.95, 15.39, 14.82, 13.52, 12.97. LCMS: [M-2H₂O-OH]⁺ = 409.5.

### Example 238

### Preparation of 3β,25-dihydroxy-5α-cholest-4-one (compound 238)

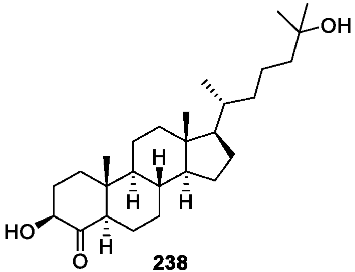

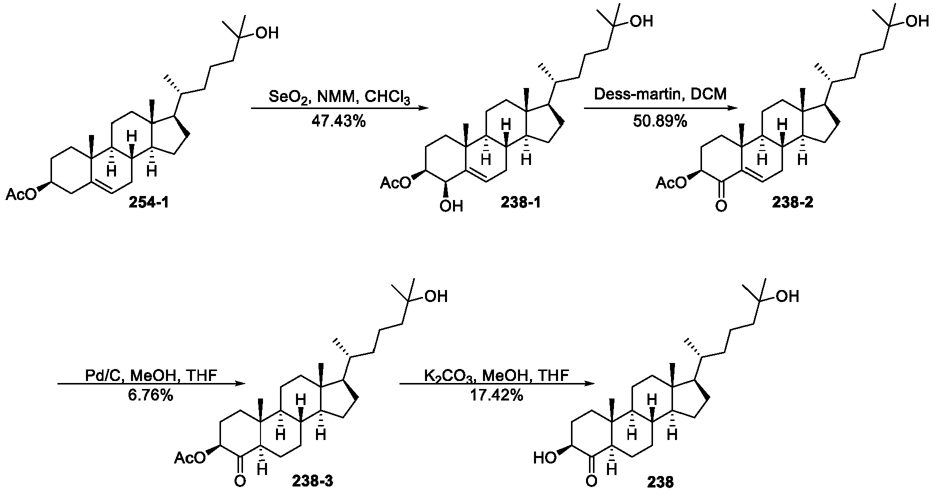

**Step 1:** (1*R*,3a*S*,3b*S*,7*S*,9a*R*,9b*S*,11a*R*)-1-[(2*R*)-6-Hydroxy-6-methylheptan-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate (**254-1**) (1.4 g, 3.261 mmol) was dissolved in chloroform (22 mL), then selenium dioxide (455.37 mg, 4.104 mmol) and *N*-methylmorpholine (989.44 mg, 9.782 mmol) were added thereto, and the reaction system was stirred at 75°C overnight. The reaction was monitored by TLC (petroleum ether: ethyl acetate = 2:1). The reaction mixture was concentrated to obtain a crude product (2 g), which was then purified by column chromatography (petroleum ether: ethyl acetate = 20:1 to 10:1) to obtain (1*R*,3a*S*,3b*S*,6*R*,7*S*,9a*R*,9b*S*,11a*R*)-6-hydroxy-1-[(2*R*)-6-hydroxy-6-methylheptan-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate (**238-1**) (750 mg, 1.547 mmol, 47.43%) as a light yellow solid. ¹H NMR (399 MHz, Chloroform-d) δ 5.68 (d, *J =* 4.7 Hz, 1H), 4.75 - 4.66 (m, 1H), 4.26 - 4.20 (m, 1H), 2.08 (s, 3H), 2.06 - 1.97 (m, 3H), 1.89 - 1.79 (m, 3H), 1.69 - 1.58 (m, 3H), 1.55 (t, *J* = 7.4 Hz, 6H), 1.46 - 1.41 (m, 4H), 1.36 (d, *J =* 12.0 Hz, 5H), 1.25 (d, *J =* 12.0 Hz, 4H), 1.20 (d, *J =* 2.1 Hz, 9H), 1.17 - 1.08 (m, 4H), 1.08 - 0.98 (m, 4H), 0.91 (d, *J =* 6.5 Hz, 3H), 0.90 - 0.80 (m, 2H), 0.66 (s, 3H).

**Step 2:** (1*R*,3a*S*,3b*S*,6*R*,7*S*,9a*R*,9b*S*,11a*R*)-6-Hydroxy-1-[(2*R*)-6-hydroxy-6-methylheptan-2-yl]-9a,11a-dimethyl-2,3,3a,3b,4,6,7,8,9,9a,9b,10,11,11a-tetradecahydro-1*H-*cyclopenta[1,2-*i*]phenanthren-7-yl acetate (**238-1**) (750 mg, 1.628 mmol) was dissolved in dichloromethane (15 mL), then Dess-Martin periodinane (1380.96 mg, 3.256 mmol) was added thereto, and the reaction mixture was stirred at room temperature for 1 hour. The reaction was monitored by TLC (petroleum ether: ethyl acetate = 3:1). After filtration, the filtrate was diluted with water and extracted three times with ethyl acetate. The organic phase was washed once with brine, dried over anhydrous sodium sulfate, and concentrated to obtain a crude product (1 g), which was then purified by column chromatography (petroleum ether: ethyl acetate = 15:1 to 5:1) to obtain (1*R*,3a*S*,3b*S*,7*S*,9a*R*,9b*S*,11a*R*)-1-[(2*R*)-6-hydroxy-6-methylheptan-2-yl]-9a,11a-dimethyl-6-oxo-2,3,3a,3b,4,6,7,8,9,9a,9b,10,11,11a-tetradecahydro-1*H*-cyclopenta[1,2-*i*]phenanthren-7-yl acetate (**238-2**) (400 mg, 0.828 mmol, 50.89%) as a light yellow solid. ¹H NMR (399 MHz, Chloroform-d) δ 6.35 (dd, *J =* 5.0, 2.6 Hz, 1H), 5.19 (dd, *J =* 12.5, 7.1 Hz, 1H), 2.24 - 2.16 (m, 1H), 2.15 (s, 3H), 2.12 (d, *J =* 6.8 Hz, 1H), 2.06 - 2.00 (m, 3H), 1.84 (d, *J =* 11.2 Hz, 1H), 1.70 (dd, *J =* 19.3, 11.2 Hz, 1H), 1.53 - 1.44 (m, 3H), 1.43 - 1.37 (m, 4H), 1.32 (d, *J=* 6.2 Hz, 1H), 1.24 (s, 2H), 1.20 (s, 6H), 1.15 - 1.08 (m, 3H), 1.08 - 1.00 (m, 3H), 0.97 (s, 3H), 0.92 (d, *J=* 6.5 Hz, 3H), 0.67 (s, 3H).

**Step 3:** (1*R*,3a*S*,3b*S*,7*S*,9a*R*,9b*S*,11a*R*)-1-[(2*R*)-6-Hydroxy-6-methylheptan-2-yl]-9a,11a-dimethyl-6-oxo-2,3,3a,3b,4,6,7,8,9,9a,9b,10,11,11a-tetradecahydro-1*H-*cyclopenta[1,2-*i*]phenanthren-7-yl acetate (**238-2**) (420 mg, 0.916 mmol) was dissolved in tetrahydrofuran (20 mL) and methanol (10 mL), then palladium (Pd/C) (200 mg, 1.879 mmol) was added thereto, and the reaction mixture was stirred at room temperature for 1 hour under a hydrogen atmosphere. The reaction was monitored by TLC (petroleum ether: ethyl acetate = 3:1). After filtration, the filter cake was washed with ethyl acetate, and the filtrate was concentrated to obtain a crude product (420 mg), which was then purified by column chromatography (petroleum ether: ethyl acetate = 10:1) to obtain (1*R*,3a*S*,3b*S*,5a*R*,7*S*,9a*R*,9b*S*,11a*R*)-1-[(2*R*)-6-hydroxy-6-methylheptan-2-yl]-9a,11a-dimethyl-6-oxohexadecahydro-1*H*-cyclopenta[1,2-*i*]phenanthren-7-yl acetate (**238-3**) (30 mg, 0.062 mmol, 6.76%) as a white solid. ¹H NMR (399 MHz, Chloroform-d) δ 5.14 (dd, *J =* 12.2, 7.2 Hz, 1H), 2.18 (d, *J =* 10.4 Hz, 2H), 2.13 (s, 3H), 1.94 (d, *J =* 14.8 Hz, 2H), 1.89 - 1.78 (m, 2H), 1.72 (d, *J =* 12.8 Hz, 2H), 1.52 - 1.44 (m, 3H), 1.42 (s, 3H), 1.34 (t, *J =* 7.9 Hz, 3H), 1.25 (d, *J =* 12.1 Hz, 3H), 1.19 (s, 6H), 1.15 - 1.06 (m, 3H), 1.02 - 0.93 (m, 3H), 0.90 (d, *J =* 6.6 Hz, 3H), 0.86 - 0.77 (m, 2H), 0.73 (s, 3H), 0.63 (s, 3H).

**Step 4:** (1*R*,3a*S*,3b*S*,5a*R*,7*S*,9a*R*,9b*S*,11a*R*)-1-[(2*R*)-6-Hydroxy-6-methylheptan-2-yl]-9a,11a-dimethyl-6-oxohexadecahydro-1*H*-cyclopenta[1,2-*i*]phenanthren-7-yl acetate (**238-3**) (30 mg, 0.065 mmol) was dissolved in tetrahydrofuran (1 mL) and methanol (2 mL), then potassium carbonate (45.00 mg, 0.326 mmol) was added thereto, and the reaction mixture was stirred at room temperature for 1 hour. The reaction was monitored by TLC (petroleum ether: ethyl acetate = 3:1). The reaction mixture was filtered, and the filtrate was directly concentrated to obtain a crude product (30 mg), which was then purified by column chromatography (petroleum ether: ethyl acetate = 10:1) to obtain 3β,25-dihydroxy-5α-cholest-4-one (**238**) (5 mg, 0.011 mmol, 17.42%) as a white solid.

¹H NMR (400 MHz, DMSO-d₆) δ 4.79 (d, *J =* 5.1 Hz, 1H), 4.01 (s, 2H), 2.18 (d, *J =* 12.1 Hz, 1H), 2.08 (d, *J =* 9.8 Hz, 1H), 1.94 - 1.87 (m, 1H), 1.72 (d, *J =* 9.1 Hz, 2H), 1.64 - 1.56 (m, 1H), 1.54 - 1.36 (m, 6H), 1.30 - 1.25 (m, 4H), 1.16 - 1.07 (m, 5H), 1.01 (s, 6H), 0.98 - 0.88 (m, 4H), 0.85 (d, *J* = 6.4 Hz, 3H), 0.83 - 0.67 (m, 3H), 0.58 (d, *J* = 3.8 Hz, 6H). ¹³C NMR (101 MHz, DMSO) δ 212.35, 74.51, 69.22, 56.16, 56.11, 56.05, 53.85, 44.54, 42.61, 42.55, 36.57, 35.70, 35.64, 34.86, 32.56, 31.13, 30.38, 29.83, 29.64, 28.23, 24.19, 21.65, 20.68, 20.62, 18.94, 13.81, 12.30. LCMS: [M+H]⁺ = 419.30.

### Example 239

### Preparation of (1R,3aS,3bR,5aR,7S,9aS,9bS,11aR)-4,4-difluoro-1-[(2R)-6-hydroxy-6-methylheptan-2-yl]-9a,11a-dimethylhexadecahydro-1H-cyclopenta[1,2-i]phenanthren-7-yl acetate (compound 239)

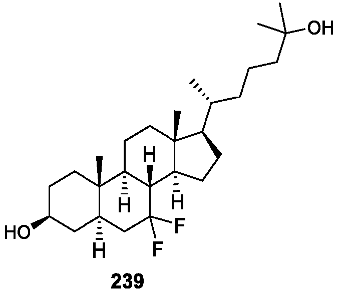

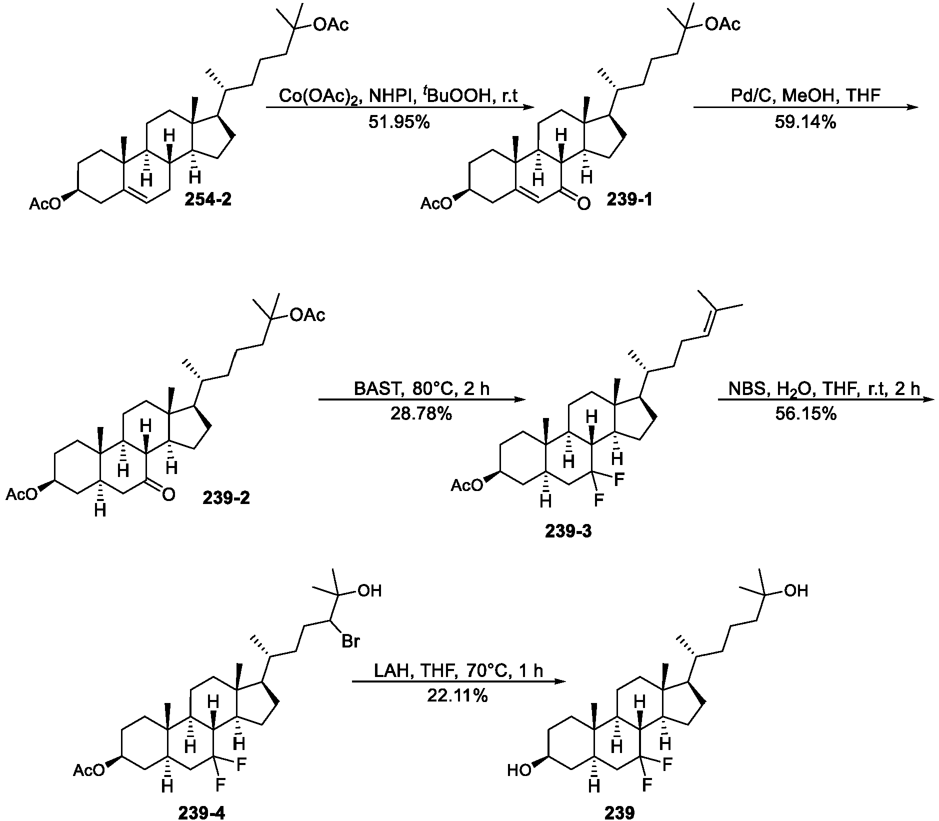

**Step 1:** (1*R*,3a*S*,3b*S*,7*S*,9a*R*,9b*S*,11a*R*)-1-[(2*R*)-6-Acetoxy-6-methylheptan-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate (**254-2**) (800 mg, 1.644 mmol) was dissolved in acetone (16 mL). Cobalt acetate (2.91 mg, 0.016 mmol), N-hydroxyphthalimide (26.81 mg, 0.164 mmol), and *tert*-butyl hydroperoxide (592.48 mg, 6.574 mmol) were added thereto, and the reaction system was stirred at room temperature for 18 hours. The reaction was monitored by TLC (petroleum ether: ethyl acetate = 5:1). After the reaction was completed, the reaction mixture was added with water and extracted three times with ethyl acetate. The organic phase was washed once with brine, dried over anhydrous sodium sulfate, and concentrated to obtain a crude product (1 g). The crude product was purified by column chromatography (petroleum ether: ethyl acetate = 30:1) to obtain (1*R*,3a*S*,3b*S*,7*S*,9a*R*,9b*S*,11a*R*)-1-[(2*R*)-6-acetoxy-6-methylheptan-2-yl]-9a,11a-dimethyl-4-oxo-2,3,3a,3b,4,6,7,8,9,9a,9b,10,11,11a-tetradecahydro-1*H-*cyclopenta[1,2-*i*]phenanthren-7-yl acetate (**239-1**) (450 mg, 0.854 mmol, 51.95%) as a light yellow solid. ¹H NMR (399 MHz, Chloroform-d) δ 5.68 (d, *J* = 1.8 Hz, 1H), 4.76 - 4.62 (m, 1H), 2.56 - 2.34 (m, 3H), 2.21 (s, 1H), 2.03 (s, 3H), 2.01 - 1.95 (m, 2H), 1.94 (d, *J=* 1.5 Hz, 3H), 1.86 (d, *J=* 9.7 Hz, 1H), 1.73 - 1.60 (m, 3H), 1.56 - 1.49 (m, 3H), 1.40 (s, 6H), 1.35 (d, *J =* 8.8 Hz, 3H), 1.29 (d, *J =* 17.6 Hz, 2H), 1.23 (t, *J=* 9.0 Hz, 4H), 1.19 (s, 3H), 1.16 (d, *J =* 5.6 Hz, 3H), 1.09 - 0.96 (m, 2H), 0.91 (d, *J=* 6.5 Hz, 3H), 0.82 (d, *J=* 6.4 Hz, 1H), 0.66 (d, *J* = 2.7 Hz, 3H).

**Step 2:** (1*R*,3a*S*,3b*S*,7*S*,9a*R*,9b*S*,11a*R*)-1-[(2*R*)-6-Acetoxy-6-methylheptan-2-yl]-9a,11a-dimethyl-4-oxo-2,3,3a,3b,4,6,7,8,9,9a,9b,10,11,11a-tetradecahydro-1*H-*cyclopenta[1,2-*i*]phenanthren-7-yl acetate (**239-1**) (400 mg, 0.799 mmol) was dissolved in tetrahydrofuran (12 mL) and methanol (24 mL), and Pd/C (200 mg, 1.879 mmol) was added thereto. The reaction system was stirred at room temperature for 1 hour under a hydrogen atmosphere. The completion of the reaction was monitored by TLC (petroleum ether: ethyl acetate = 5:1). After filtration, the filtrate was concentrated to obtain a crude product (50 g). The crude product was purified by column chromatography (petroleum ether: ethyl acetate = 30:1) to obtain (1*R*,3a*S*,3b*R*,7*S*,9a*S*,9b*S*,11a*R*)-1-[(2*R*)-6-acetoxy-6-methylheptan-2-yl]-9a,11a-dimethyl-4-oxohexadecahydro-1*H*-cyclopenta[1,2-*i*]phenanthren-7-yl acetate (**239-2**) (250 mg, 0.472 mmol, 59.14%) as a light yellow solid. ¹H NMR (399 MHz, Chloroform-d) δ 4.64 (d, *J* = 11.3 Hz, 1H), 2.31 (t, *J=* 12.5 Hz, 2H), 2.18 (d, *J =* 11.9 Hz, 1H), 2.00 (s, 3H), 1.94 (d, *J=* 1.9 Hz, 3H), 1.87 (d, *J=* 9.9 Hz, 2H), 1.77 (d, *J=* 13.8 Hz, 1H), 1.70 (s, 1H), 1.64 - 1.59 (m, 2H), 1.50 (d, *J=* 11.7 Hz, 3H), 1.45 (d, *J=* 10.6 Hz, 1H), 1.40 (s, 6H), 1.35 (d, *J=* 7.2 Hz, 3H), 1.23 (s, 3H), 1.19 - 1.09 (m, 2H), 1.08 (s, 3H), 1.02 (d, *J=* 17.7 Hz, 2H), 0.89 (d, *J =* 6.3 Hz, 2H), 0.81 (d, *J =* 6.7 Hz, 1H), 0.63 (d, *J =* 2.6 Hz, 3H).

**Step 3:** (1*R*,3a*S*,3b*R*,7*S*,9a*S*,9b*S*,11a*R*)-1-[(2*R*)-6-Acetoxy-6-methylheptan-2-yl]-9a,11a-dimethyl-4-oxohexadecahydro-1*H*-cyclopenta[1,2-*i*]phenanthren-7-yl acetate (**239-2**) (250 mg, 0.497 mmol) was dissolved in bis(2-methoxyethyl)aminosulfur trifluoride (10 mL). The reaction mixture was heated to 80°C and stirred for 2 hours. The completion of the reaction was monitored by TLC (petroleum ether: ethyl acetate = 10:1). The reaction mixture was cooled to room temperature, diluted with ethyl acetate, then quenched with water, and extracted three times with ethyl acetate. The organic phase was washed once with brine, dried over anhydrous sodium sulfate, and concentrated to obtain a crude product (300 mg), which was then purified by column chromatography (petroleum ether: ethyl acetate = 100:1) to obtain (1*R*,3a*S*,3b*R*,5a*R*,7*S*,9a*S*,9b*S*,11a*R*)-4,4-difluoro-9a,11a-dimethyl-1-[(2*R*)-6-methylhept-5-en-2-yl]hexadecahydro-1*H*-cyclopenta[1,2-*i*]phenanthren-7-yl acetate (**239-3**) (70 mg, 0.143 mmol, 28.78%) as a light yellow solid. ¹H NMR (400 MHz, DMSO-d₆) δ 5.04 (d, *J=* 7.1 Hz, 1H), 4.55 (dt, *J=* 11.2, 6.1 Hz, 1H), 1.93 (s, 3H), 1.89 (d, *J =* 12.6 Hz, 2H), 1.75 (d, *J=* 15.7 Hz, 2H), 1.70 (s, 3H), 1.63 (d, *J* = 7.3 Hz, 2H), 1.60 (s, 3H), 1.52 (s, 3H), 1.44 (d, *J =* 22.1 Hz, 2H), 1.30 (t, *J =* 11.7 Hz, 4H), 1.21 (d, *J =* 11.5 Hz, 2H), 1.09 (d, *J=* 6.7 Hz, 1H), 1.04 - 0.97 (m, 2H), 0.93 (s, 1H), 0.87 (d, *J=* 6.6 Hz, 3H), 0.79 (s, 3H), 0.63 - 0.53 (m, 3H).

**Step 4:** (1*R*,3a*S*,3b*R*,5a*R*,7*S*,9a*S*,9b*S*,11a*R*)-4,4-Difluoro-9a,11a-dimethyl-1-[(2*R*)-6-methylhept-5-en-2-yl]hexadecahydro-1*H*-cyclopenta[1,2-*i*]phenanthren-7-yl acetate (**239-3**) (70 mg, 0.151 mmol) was dissolved in tetrahydrofuran (3 mL) and water (0.75 mL), then N-bromosuccinimide (26.8 mg, 0.15 mmol) was added thereto, and the reaction mixture was stirred at room temperature for 2 hours. The reaction was monitored by TLC (petroleum ether: ethyl acetate = 5:1). The reaction mixture was diluted with water and extracted three times with ethyl acetate. The organic phase was washed once with brine, dried over anhydrous sodium sulfate, and concentrated to obtain a crude product (100 mg), which was then purified by column chromatography (petroleum ether: ethyl acetate = 20:1) to obtain (1*R*,3a*S*,3b*R*,5a*R*,7*S*,9a*S*,9b*S*,11a*R*)-1-[(2*R*)-5-bromo-6-hydroxy-6-methylheptan-2-yl]-4,4-difluoro-9a,11a-dimethylhexadecahydro-1*H*-cyclopenta[1,2-*i*]phenanthren-7-yl acetate (**239-4**) (50 mg, 0.085 mmol, 56.15%) as a light yellow solid. ¹H NMR (400 MHz, DMSO-d₆) δ 4.74 (s, 1H), 4.55 (dt, *J=* 11.2, 5.9 Hz, 1H), 3.78 (dd, *J* = 22.2, 11.1 Hz, 1H), 1.93 (s, 3H), 1.88 (s, 1H), 1.70 (s, 4H), 1.59 (d, *J=* 22.7 Hz, 2H), 1.49 (d, *J=* 20.7 Hz, 2H), 1.39 (d, *J=* 13.0 Hz, 3H), 1.30 (t, *J* = 11.9 Hz, 5H), 1.22 (s, 3H), 1.15 (d, *J* = 1.5 Hz, 3H), 1.09 (d, *J* = 17.9 Hz, 2H), 1.04 - 0.95 (m, 2H), 0.87 (t, *J=* 7.2 Hz, 3H), 0.79 (s, 3H), 0.61 (d, *J=* 3.8 Hz, 3H).

**Step 5:** (1*R*,3a*S*,3b*R*,5a*R*,7*S*,9a*S*,9b*S*,11a*R*)-1-[(2*R*)-5-Bromo-6-hydroxy-6-methylheptan-2-yl]-4,4-difluoro-9a,11a-dimethylhexadecahydro-1*H*-cyclopenta[1,2-*i*]phenanthren-7-yl acetate (**239-4**) (50 mg, 0.089 mmol) was dissolved in tetrahydrofuran (3 mL), then lithium aluminum hydride (33.79 mg, 0.890 mmol) was added thereto, and the reaction mixture was stirred at 70°C for 1 hour. The reaction was monitored by TLC (petroleum ether: ethyl acetate = 2:1). The reaction mixture was quenched with water and extracted three times with ethyl acetate. The organic phase was washed once with brine, dried over anhydrous sodium sulfate, and concentrated to obtain a crude product (60 mg), which was then purified by column chromatography (petroleum ether: ethyl acetate = 10:1) to obtain (1*R*,3a*S*,3b*R*,5a*R*,7*S*,9a*S*,9b*S*,11a*R*)-4,4-difluoro-1-[(2*R*)-6-hydroxy-6-methylheptan-2-yl]-9a,11a-dimethylhexadecahydro-1*H*-cyclopenta[1,2-*i*]phenanthren-7-yl acetate (**239**) (10 mg, 0.020 mmol, 22.11%) as a white solid.

¹H NMR (400 MHz, DMSO-d₆) δ 4.53 (d, *J* = 5.1 Hz, 1H), 4.03 (s, 1H), 3.30 (s, 1H), 1.92 - 1.85 (m, 1H), 1.73 (d, *J* = 8.5 Hz, 2H), 1.67 - 1.54 (m, 5H), 1.50 - 1.38 (m, 2H), 1.35 - 1.29 (m, 3H), 1.25 (s, 3H), 1.21 (d, *J=* 9.6 Hz, 4H), 1.15 (d, *J=* 8.6 Hz, 3H), 1.12 - 1.03 (m, 3H), 1.01 (s, 6H), 0.93 (d, *J=* 17.4 Hz, 2H), 0.86 (d, *J=* 6.3 Hz, 3H), 0.72 (d, *J=* 20.4 Hz, 3H), 0.58 (d, *J =* 18.8 Hz, 3H). ¹³C NMR (100 MHz, DMSO-d₆) δ 69.22, 55.15, 50.26, 48.68, 44.54, 43.11, 37.19, 36.60, 36.27, 35.60, 35.00, 31.45, 29.82, 28.41, 25.60, 21.16, 20.68, 19.02, 12.07, 11.48. LC-MS: [M-H₂O-HF]⁺ = 403.40.

### Example 240

### Preparation of cholest-3β,6α,25-triol (compound 240)

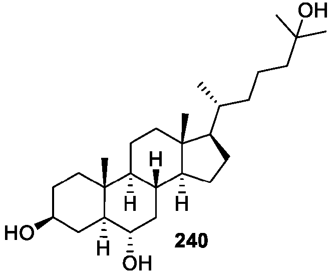

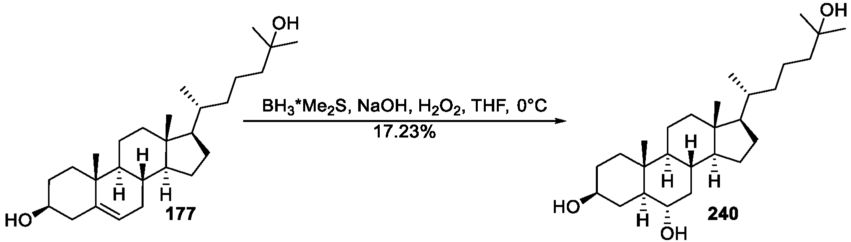

Cholest-6(5)-en-3β,25-diol (**177**) (50 mg, 0.07 mmol) was dissolved in tetrahydrofuran (5 mL), and the reaction mixture was cooled to -10°C under a nitrogen atmosphere. 1 M borane dimethyl sulfide solution (0.37 mL, 0.42 mmol) was added dropwise thereto. The reaction mixture was then naturally warmed to room temperature, stirred for 3 hours, and cooled to 0°C in an ice bath. Sodium hydroxide (10 M, 57.6 mg, 0.84 mmol) was added thereto, followed by the dropwise addition of hydrogen peroxide (31%, 48 mg, 0.84 mmol), and the reaction mixture was stirred at room temperature for 16 hours. The completion of the reaction was monitored by TLC (petroleum ether: ethyl acetate = 1:1). The reaction mixture was quenched with water, extracted twice with ethyl acetate, dried over anhydrous sodium sulfate, concentrated, and purified by column chromatography (petroleum ether: ethyl acetate = 30:1 to 2:1) to obtain compound cholest-3β,6α,25-triol (**240**) (10 mg, 0.021 mmol, 17.23%) as a white solid. ¹H NMR (399 MHz, Chloroform-d) δ 3.56 (s, 1H), 3.40 (s, 1H), 2.18 (d, *J =* 11.5 Hz, 1H), 1.96 (d, *J =* 11.5 Hz, 1H), 1.79 (s, 2H), 1.56 (s, 6H), 1.40 (s, 7H), 1.25 (d, *J=* 11.1 Hz, 6H), 1.19 (s, 6H), 1.03 (s, 2H), 1.03 (s, 4H), 0.90 (d, *J =* 6.6 Hz, 2H), 0.81 (d, *J* = 9.5 Hz, 3H), 0.63 (s, 3H). ¹³C NMR (100 MHz, CDCl₃) δ 71.22, 71.09, 69.40, 56.09, 53.74, 51.64, 43.08, 42.64, 41.64, 37.22, 35.69, 34.25, 31.01, 29.65, 23.99, 20.95, 20.49, 18.18, 0.99. ELSD-MS: [M-H₂O-OH]⁺ = 385.7.

### Example 241

### Preparation of 24-(1-hydroxypropyl)-5α-cholan-3β,4β-diol (compound 241)

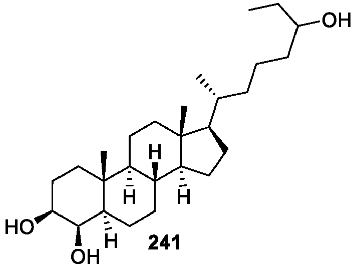

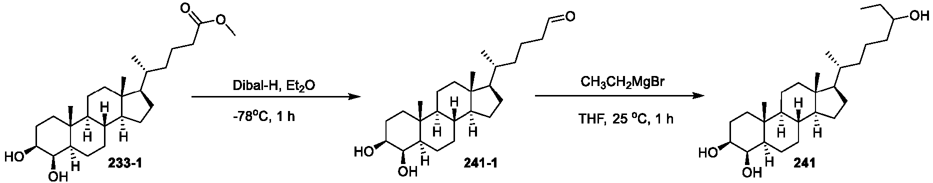

***Step 1*:** The compound methyl (5*R*)-5-[(1*R*,3a*S*,3b*S*,5a*R*,6*R*,7*S*,9a*R*,9b*S*,11a*R*)-6,7-dihydroxy-9a,11a-dimethylhexadecahydro-1*H*-cyclopenta[1,2-*a*]phenanthren-1-yl]hexanoate (**233-1**) (40 mg, 0.08 mmol, 1.0 *eq*) was dissolved in diethyl ether (10 mL), and the reaction system was cooled to -78°C. Diisobutylaluminium hydride (0.5 mL, 0.5 mmol, 6.0 *eq*, 1 mol/L) was then added thereto, and the reaction system was stirred at -78°C for 1 hour. The completion of the reaction was monitored by TLC plate (petroleum ether: ethyl acetate = 1:1). The reaction system was diluted with ethyl acetate (20 mL), quenched with saturated sodium tartrate (20 mL), and extracted with ethyl acetate (10 mL * 3). The organic phase was collected, washed twice with water (20 mL), dried over anhydrous sodium sulfate, and evaporated to dryness by rotary evaporation under vacuum to obtain a crude product. The crude product was purified by silica gel column chromatography (petroleum ether: ethyl acetate = 74:26) to obtain (5*R*)-5-[(1*R*,3a*S*,3b*S*,5a*R*,6*R*,7*S*,9a*R*,9b*S*,11a*R*)-6,7-dihydroxy-9a,11a-dimethylhexadecahydro-1*H*-cyclopenta[1,2-*a*]phenanthren-1-yl]hexanal (**241-1**) (30 mg, purity: 40%, yield: 35%) as a white solid.

***Step 2*:** The compound (5*R*)-5-[(1*R*,3a*S*,3b*S*,5a*R*,6*R*,7*S*,9a*R*,9b*S*,11a*R*)-6,7-dihydroxy-9a,11a-dimethylhexadecahydro-1*H*-cyclopenta[1,2-*a*]phenanthren-1-yl]hexanal (**241-1**) (30 mg, 0.031 mmol, 1.0 *eq*) was dissolved in tetrahydrofuran (8 mL), and the reaction system was cooled to 0°C. Ethylmagnesium bromide (0.077 mL, 0.154 mmol, 5 *eq*) was then slowly added thereto, and the reaction system was stirred at room temperature for 1 hour. The completion of the reaction was detected by TLC plate (petroleum ether: ethyl acetate = 5:1). The reaction system was added with water (20 mL) and extracted with ethyl acetate (50 mL × 3). The organic phases were combined, washed with water (30 mL), dried, and concentrated to obtain a crude product. The crude product was purified by silica gel column chromatography (petroleum ether: ethyl acetate = 74:26) to obtain 24-(1-hydroxypropyl)-5α-cholan-3β,4β-diol (**241**) (10.55 mg, purity: 93.23%, yield: 76%) as a white solid.

¹H NMR (400 MHz, DMSO) *δ* 4.28 (d, *J* = 6.0 Hz, 1H), 4.19 (dd, *J* = 5.2, 3.0 Hz, 1H), 3.88 (d, *J =* 2.9 Hz, 1H), 3.47 (d, *J =* 2.2 Hz, 1H), 3.29 - 3.24 (m, 2H), 1.90 (d, *J =* 12.7 Hz, 1H), 1.70 - 1.54 (m, 5H), 1.43 - 1.33 (m, 4H), 1.30 (s, 3H), 1.25 (d, *J =* 9.8 Hz, 8H), 1.19 (s, 3H), 1.08 - 0.99 (m, 4H), 0.94 (s, 3H), 0.87 (d, *J =* 6.5 Hz, 3H), 0.83 (t, *J =* 7.4 Hz, 4H), 0.61 (s, 3H), 0.54 (d, *J =* 9.5 Hz, 1H). ¹³C NMR (101 MHz, CDCl₃) *δ* 74.86, 73.49, 72.31, 56.57, 56.10, 55.26, 48.84, 42.61, 39.91, 37.50, 36.88, 35.50, 35.40, 32.40, 31.44, 30.20, 29.71, 28.25, 26.00, 25.87, 24.21, 22.10, 20.59, 18.65, 14.68, 12.07, 9.90. LCMS [M+H-2H₂O]⁺ = 385.

### Example 242

### Preparation of 25-hydroxy-4β-hydroxy-3β-hydroxy-5α-cholest-7-one (compound 242)

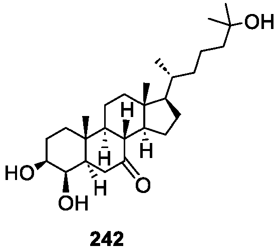

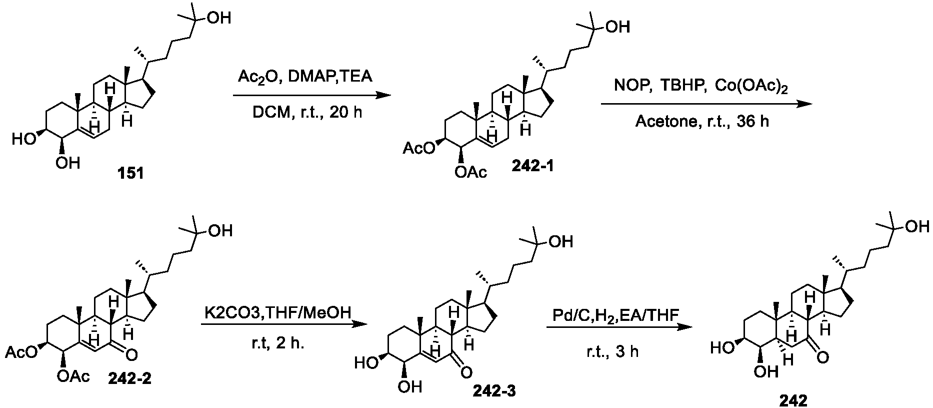

***Step 1*:** Cholest-6(5)-en-3β,4β,25-triol (**151**) (363 mg, 0.87 mmol) was dissolved in dichloromethane (10 mL) in a 50 mL round-bottom flask at room temperature. Triethylamine (702 mg, 6.94 mmol), 4-dimethylaminopyridine (106 mg, 0.87 mmol), and acetic anhydride (265 mg, 2.60 mmol) were added thereto at room temperature, and the reaction mixture was stirred at room temperature for 16 hours. After the completion of the reaction was detected by TLC (petroleum ether: ethyl acetate = 8:1), the reaction mixture was quenched with water (50 mL) and extracted with ethyl acetate (30 mL × 3). The organic phase was washed with saturated brine (50 mL), dried over anhydrous sodium sulfate, concentrated, and purified by silica gel column chromatography (petroleum ether: ethyl acetate = 8:1) to obtain (1*R*,3a*S*,3b*S*,6*R*,7*S*,9a*R*,9b*S*,11a*R*)-7-acetoxy-1-[(2*R*)-6-hydroxy-6-methylheptan-2-yl]-9a,11a-dimethyl-2,3,3a,3b,4,6,7,8,9,9a,9b,10,11,11a-tetradecahydro-1*H*-cyclopenta[1,2-*i*]phenanthren-6-yl acetate (**242-1**) (334 mg, purity: 95%, yield: 72.79%) as a white solid. ¹H NMR (400 MHz, CDCl₃) δ 5.89 - 5.73 (m, 1H), 5.50 (d, *J=* 2.3 Hz, 2H), 4.78 - 4.72 (m, 1H), 2.07 (s, 3H), 2.05 - 2.01 (m, 1H), 2.01 (s, 3H), 1.93 - 1.80 (m, 2H), 1.72 - 1.64 (m, 1H), 1.63 - 1.31 (m, 15H), 1.28 - 1.25 (m, 2H), 1.21 (s, 6H), 1.13 (s, 3H), 1.09 - 0.96 (m, 4H), 0.93 (d, *J=* 6.5 Hz, 3H), 0.90 - 0.81 (m, 1H), 0.67 (s, 3H).

***Step 2*:** (1*R*,3a*S*,3b*S*,6*R*,7*S*,9a*R*,9b*S*,11a*R*)-7-Acetoxy-1-[(2*R*)-6-hydroxy-6-methylheptan-2-yl]-9a,11a-dimethyl-2,3,3a,3b,4,6,7,8,9,9a,9b,10,11,11a-tetradecahydro-1*H-*cyclopenta[1,2-*i*]phenanthren-6-yl acetate (**242-1**) (170 mg, 0.34 mmol) was dissolved in acetone (5 mL) in a 50 mL round-bottom flask at room temperature. *N-*Hydroxyphthalimide (11 mg, 0.068 mmol), cobalt acetate (6 mg, 0.034 mmol), and *tert*-butyl hydroperoxide (122 mg, 1.35 mmol) were added thereto at room temperature, and the reaction mixture was stirred at room temperature for 16 hours. After the completion of the reaction was detected by TLC (petroleum ether: ethyl acetate = 5:1), the reaction mixture was quenched with water (20 mL) and extracted with ethyl acetate (10 mL × 3). The organic phase was washed with saturated brine (20 mL), dried over anhydrous sodium sulfate, concentrated, and purified by silica gel column chromatography (petroleum ether: ethyl acetate = 5:1) to obtain (1*R*,3a*S*,3b*S*,6*R*,7*S*,9a*R*,9b*S*,11a*R*)-7-acetoxy-1-[(2*R*)-6-hydroxy-6-methylheptan-2-yl]-9a,11a-dimethyl-4-oxo-2,3,3a,3b,4,6,7,8,9,9a,9b,10,11,11a-tetradecahydro-1*H-*cyclopenta[1,2-*i*]phenanthren-6-yl acetate (**242-2**) (90 mg, purity: 90%, yield: 46.36%) as a white solid. ¹H NMR (400 MHz, CDCl₃) δ 5.91 (d, *J* = 14.2 Hz, 1H), 5.61 (d, *J* = 2.4 Hz, 1H), 4.80 (dt, *J=* 12.2, 4.0 Hz, 1H), 2.43 - 2.27 (m, 2H), 2.10 (s, 3H), 2.03 (s, 3H), 2.00 - 1.80 (m, 4H), 1.51 - 1.24 (m, 16H), 1.21 (s, 6H), 1.20 - 0.98 (m, 5H), 0.94 (d, *J* = 6.5 Hz, 3H), 0.68 (s, 3H). ¹³C NMR (101 MHz, CDCl₃) δ 202.20, 170.06, 169.47, 158.31, 131.14, 73.61, 71.51, 71.12, 54.68, 50.80, 49.91, 45.95, 44.37, 43.06, 38.53, 37.93, 36.42, 35.80, 35.69, 29.31, 29.27, 28.52, 26.20, 22.21, 21.15, 20.99, 20.78, 18.83, 17.91, 11.92.

***Step 3:*** (1*R*,3a*S*,3b*S*,6*R*,7*S*,9a*R*,9b*S*,11a*R*)-7-Acetoxy-1-[(2*R*)-6-hydroxy-6-methylheptan-2-yl]-9a,11a-dimethyl-4-oxo-2,3,3a,3b,4,6,7,8,9,9a,9b,10,11,11a-tetradecahydro-1*H*-cyclopenta[1,2-*i*]phenanthren-6-yl acetate (**242-2**) (50 mg, 0.097 mmol) was dissolved in tetrahydrofuran (1 mL) and methanol (1 mL) in a 50 mL round-bottom flask. Potassium carbonate (67 mg, 0.48 mmol) and water (0.5 mL) were added thereto at room temperature, and the reaction mixture was stirred at room temperature for 2 hours. After the completion of the reaction was detected by TLC (petroleum ether: ethyl acetate = 3:1), the reaction mixture was diluted with water (20 mL) and extracted with ethyl acetate (10 mL × 3). The organic phase was washed with saturated brine (20 mL), dried over anhydrous sodium sulfate, concentrated, and purified by silica gel column chromatography (petroleum ether: ethyl acetate = 3:1) to obtain 2,5-hydroxy-4β-hydroxy-3β-hydroxycholest-6(5)-en-7-one (**242-3**) (40 mg, purity: 90%, yield: 85.99%) as a white solid. ¹H NMR (400 MHz, CDCl₃) δ 5.83 (s, 1H), 4.28 (t, *J=* 10.2 Hz, 1H), 3.65 (dt, *J=* 11.8, 4.0 Hz, 1H), 2.41 - 2.28 (m, 2H), 2.06 - 1.88 (m, 5H), 1.78 - 1.71 (m, 4H), 1.58 - 1.35 (m, 7H), 1.37 (s, 3H), 1.21 (s, 6H), 1.19 - 1.00 (m, 5H), 0.94 (d, *J=* 6.5 Hz, 3H), 0.69 (s, 3H).

***Step 4*:** 25-Hydroxy-4β-hydroxy-3β-hydroxycholest-6(5)-en-7-one (**242-3**) (30 mg, 0.069 mmol) was dissolved in tetrahydrofuran (1 mL) and ethyl acetate (1 mL) in a 50 mL round-bottom flask. 10% Pd/C (30 mg) was added thereto at room temperature, and the resulting mixture was stirred at room temperature for 3 hours under a hydrogen atmosphere. The completion of the reaction was detected by TLC (ethyl acetate/petroleum ether = 1:1). After the reaction was completed, the reaction mixture was diluted with ethyl acetate (20 mL) and filtered through diatomite. The organic phase was concentrated and purified by silica gel column chromatography (petroleum ether: ethyl acetate = 1:1) to obtain 2,5-hydroxy-4β-hydroxy-3β-hydroxycholest-7-one (**242**) (23 mg, purity: 98.06%, yield: 74.78%) as a white solid.

¹H NMR (400 MHz, DMSO) δ 4.39 (d, *J* = 6.1 Hz, 1H), 4.28 (d, *J* = 3.5 Hz, 1H), 4.03 (s, 1H), 3.43 (s, 1H), 2.80 (t, *J=* 13.4 Hz, 1H), 2.37 (t, *J=* 11.3 Hz, 1H), 2.12 - 2.05 (m, 1H), 1.92 - 1.89 (m, 1H), 1.83 - 1.76 (m, 2H), 1.72 - 1.55 (m, 3H), 1.45 - 1.41 (m, 3H), 1.34 - 1.28 (m, 6H), 1.25 - 1.23 (m, 4H), 1.19 (s, 3H), 1.04 (s, 6H), 1.00 - 0.91 (m, 5H), 0.88 (d, *J =* 6.5 Hz, 3H), 0.60 (s, 3H). ¹³C NMR (101 MHz, DMSO) δ 212.65, 73.62, 71.66, 69.23, 55.58, 55.04, 50.12, 49.56, 49.40, 44.60, 44.39, 42.60, 38.81, 36.68, 36.49, 36.06, 35.60, 29.88, 29.71, 28.49, 26.02, 25.07, 21.12, 20.74, 19.12, 14.50, 12.37. LCMS (M+Na)⁺ = 457.3.

### Example 244

### Preparation of cholest-3β,4β,6,25-tetraol (compound 244)

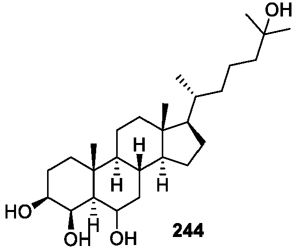

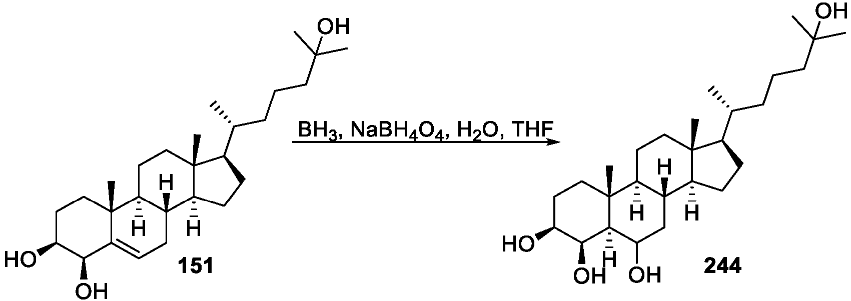

Cholest-6(5)-en-3β,4α,25-triol (**151**) (30 mg, 0.072 mmol) was dissolved in anhydrous tetrahydrofuran (5 mL), and the reaction mixture was cooled to -10°C under a nitrogen atmosphere. Borane (0.717 mL) was added dropwise thereto, and the reaction mixture was naturally warmed to room temperature and stirred for 3 hours. Water (0.2 mL) was added dropwise thereto at 0°C, then sodium perborate (28 mg, 0.717 mmol) was added thereto, and the reaction mixture was stirred at room temperature for 16 hours. The reaction was monitored by TLC (dichloromethane: methanol = 10:1). The reaction mixture was then quenched with water, extracted twice with ethyl acetate, dried over anhydrous sodium sulfate, evaporated to dryness by rotary evaporation, and purified by column chromatography (dichloromethane: methanol = 100% to 20%) to obtain cholest-3β,4β,6,25-tetraol (**244**) (10 mg, 0.005 mmol, 24%). ¹H NMR (399 MHz, Chloroform-d) δ 4.41 (s, 0.77H), δ 4.25 (s, 0.22H), 3.94 (s, 1H), 3.59 - 3.50 (m, 1H), 1.82 (m, 9H),1.66 (m,5H) 1.37 (ddd, *J =* 26.5, 13.5, 6.2 Hz, 8H), 1.23 (s, 2H), 1.19 (s, 4H), 1.14 (s, 3H), 1.08 - 0.96 (m, 4H), 0.90 (d, *J* = 6.6 Hz, 2H), 0.80 (d, *J=* 6.6 Hz, 1H), 0.66 (s, 2H), 0.63 (s, 1H). ¹³C NMR (101 MHz, CDCl₃) δ, 71.14, 56.46, 56.27, 44.37, 42.65, 35.73, 29.33, 29.12, 28.31, 25.77, 20.81, 18.63, 12.07, 1.00. ELSD-MS: [M-OH]⁺ = 419.3, [M+Na]⁺ = 459.4.

Example **245**

### Preparation of (1R,3aS,5aR,6R,7S,9aR,11aR)-1-[(2R)-5-(hydroxycyclopropyl)pentan-2-yl]-9a,11a-dimethylhexadecahydro-1H-cyclopenta[1,2-a]phenanthrene-6,7-diol (compound 245)

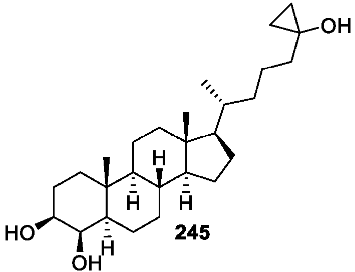

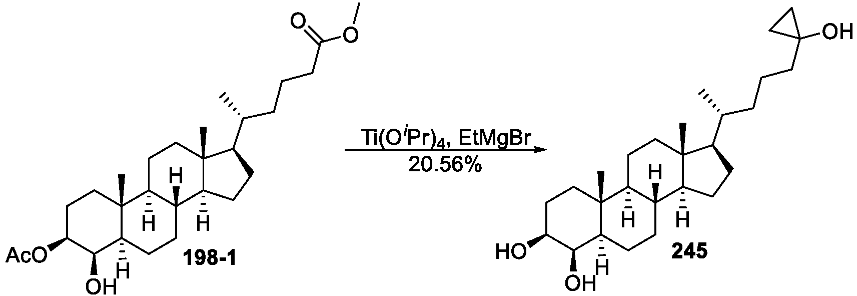

The compound (1*R*,3a*S*,3b*S*,5a*R*,6*R*,7*S*,9a*R*,9b*S*,11a*R*)-6-hydroxy-1-[(2*R*)-6-methoxy-6-oxohexan-2-yl]-9a,11a-dimethylhexadecahydro-1*H*-cyclopenta[1,2-i]phenanthren-7-yl acetate (**198-1**) (50 mg, 0.112 mmol) was dissolved in tetrahydrofuran (5 mL), and titanium isopropoxide (95.6 mg, 0.670 mmol) was added thereto at room temperature. The reaction system was replaced with nitrogen three times, and ethylmagnesium bromide (1.080 mL) was added dropwise thereto at room temperature. The reaction mixture was stirred at room temperature for 1 hour. The completion of the reaction was monitored by TLC (petroleum ether: ethyl acetate = 5:1). The reaction mixture was added with water and extracted with ethyl acetate. The organic phase was separated and dried to obtain a crude product, which was then purified by column chromatography (petroleum ether: ethyl acetate = 60:1 to 1:1) to obtain (1*R*,3a*S*,5a*R*,6*R*,7*S*,9a*R*,11a*R*)-1-[(2*R*)-5-(hydroxycyclopropyl)pentan-2-yl]-9a,11a-dimethylhexadecahydro-1*H*-cyclopenta[1,2-*a*]phenanthrene-6,7-diol (**245**) (10 mg, 0.022 mmol, 20.56%) as a white solid. ¹H NMR (399 MHz, Chloroform-d) δ 3.72 (s, 1H), 3.54 (s, 1H), 1.91 (s, 3H), 1.74 (s, 6H), 1.37 (s, 7H), 1.24 (s, 5H), 1.05 (s, 4H), 1.00 (s, 4H), 0.90 (d, *J =* 6.4 Hz, 3H), 0.84 (d, *J =* 15.5 Hz, 2H), 0.71 (s, 2H), 0.63 (s, 3H), 0.42 (s, 2H). ¹³C NMR (100 MHz, CDCl₃) δ, 76.66, 72.17, 55.93, 42.57, 38.81, 36.86, 35.36, 29.50, 28.37, 25.95, 24.16, 20.28, 13.43, 0.99. LC-MS: [M-H₂O-OH]⁺ = 383.4.

### Example 246

### Preparation of 4-(trifluoromethyl)-5α-cholest-3β,4,25-triol (compound 246)

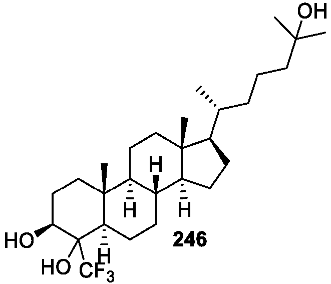

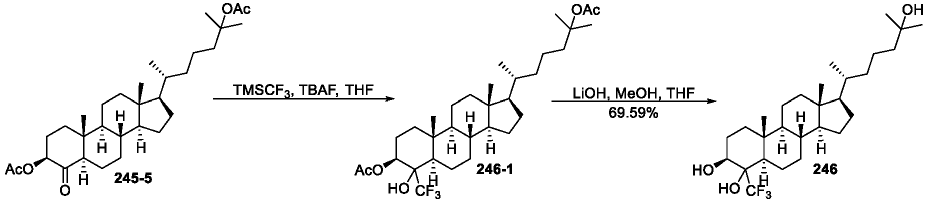

**Step 1:** (1*R*,3a*S*,3b*S*,5a*R*,7*S*,9a*R*,9b*S*,11a*R*)-1-[(2*R*)-6-Acetoxy-6-methylheptan-2-yl]-9a,11a-dimethyl-6-oxohexadecahydro-1*H*-cyclopenta[1,2-*i*]phenanthren-7-yl acetate (**254-5**) (60 mg, 0.12 mmol) was dissolved in tetrahydrofuran (5 mL), then tetrabutylammonium fluoride (3.12 mg, 0.012 mmol) and (trifluoromethyl)trimethylsilane (339.42 mg, 2.387 mmol) were added thereto, and the reaction mixture was stirred at room temperature for 1 hour. The reaction was monitored by TLC (petroleum ether: ethyl acetate = 5:1). The reaction mixture was diluted with water and extracted three times with ethyl acetate. The organic phase was washed once with brine, dried over anhydrous sodium sulfate, and concentrated to obtain a crude product (80 mg). The crude product was purified by column chromatography (petroleum ether: ethyl acetate = 50:1) to obtain (1*R*,3a*S*,3b*S*,5a*R*,7*S*,9a*R*,9b*S*,11a*R*)-1-[(2*R*)-6-acetoxy-6-methylheptan-2-yl]-6-hydroxy-9a,11a-dimethyl-6-(trifluoromethyl)hexadecahydro-1*H*-cyclopenta[1,2-*i*]phenanthren-7-yl acetate (**246-1**) (40 mg, 0.066 mmol, 55.59%). ¹H NMR (400 MHz, DMSO-d₆) δ 4.89 (s, 1H), 4.85 (d, *J =* 5.8 Hz, 1H), 1.96 (s, 1H), 1.86 (s, 3H), 1.56 (td, *J=* 32.5, 29.8, 9.7 Hz, 5H), 1.32 (s, 6H), 1.27 (d, *J=* 10.8 Hz, 3H), 1.16 - 0.99 (m, 4H), 0.97 (s, 3H), 0.89 (s, 1H), 0.84 (d, *J=* 6.2 Hz, 3H), 0.76 (s, 1H), 0.58 (s, 3H).

**Step 2:** (1*R*,3a*S*,3b*S*,5a*R*,7*S*,9a*R*,9b*S*,11a*R*)-1-[(2*R*)-6-Acetoxy-6-methylheptan-2-yl]-6-hydroxy-9a,11a-dimethyl-6-(trifluoromethyl)hexadecahydro-1*H*-cyclopenta[1,2-*i*]phenanthren-7-yl acetate (**246-1**) (40 mg, 0.070 mmol) was dissolved in methanol (1 mL) and tetrahydrofuran (2 mL), then lithium hydroxide (1 mL, 1.000 mmol) was added thereto, and the reaction mixture was stirred at room temperature for 18 hours. The reaction was monitored by TLC (petroleum ether: ethyl acetate = 5:1). The reaction mixture was concentrated to obtain a crude product (50 mg), which was then purified by column chromatography (petroleum ether: ethyl acetate = 15:1) to obtain 4-(trifluoromethyl)-5α-cholest-3β,4,25-triol (**246**) (25 mg, 0.049 mmol, 69.59%) as a white solid. ¹H NMR (400 MHz, DMSO-d₆) δ 4.89 (s, 1H), 4.85 (d, *J=* 6.1 Hz, 1H), 4.00 (s, 1H), 3.52 (d, *J* = 7.0 Hz, 1H), 1.99 - 1.72 (m, 2H), 1.60 (d, *J=* 21.0 Hz, 5H), 1.48 (d, *J=* 12.2 Hz, 2H), 1.38 (d, *J =* 12.8 Hz, 2H), 1.28 (d, *J =* 14.5 Hz, 4H), 1.16 (d, *J =* 11.3 Hz, 4H), 1.09 (d, *J =* 18.4 Hz, 3H), 1.01 (s, 6H), 0.97 (s, 2H), 0.93 (s, 3H), 0.89 (d, *J=* 7.0 Hz, 2H), 0.84 (d, *J=* 6.4 Hz, 3H), 0.81 - 0.68 (m, 2H), 0.58 (s, 3H). ¹³C NMR (101 MHz, DMSO-d₆) δ 69.19, 69.11, 56.32, 56.09, 55.16, 47.34, 44.54, 42.41, 36.58, 36.37, 36.08, 35.64, 34.60, 32.14, 29.85, 29.64, 28.28, 27.51, 24.10, 20.67, 18.92, 14.50, 12.29.

¹⁹F NMR (376 MHz, DMSO-d₆) δ -70.57. ELSD-MS: [M-OH]⁺ = 471.4.
Examples **248 and 249**
Preparation of **cholest-3β,4,5,25-tetraol** isomer 2 (compound **248**)
Preparation of **cholest-3β,4,5,25-tetraol** isomer 1 (compound **249**)

Both compound **248** and compound **249** are single configurational isomers, each being one of the following isomers:

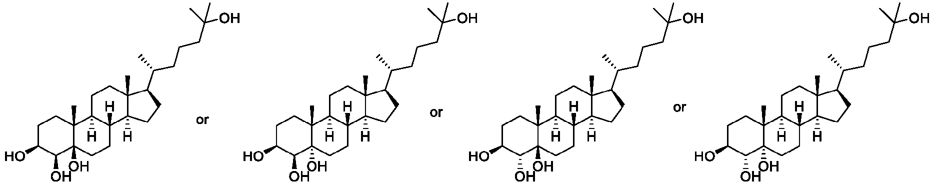

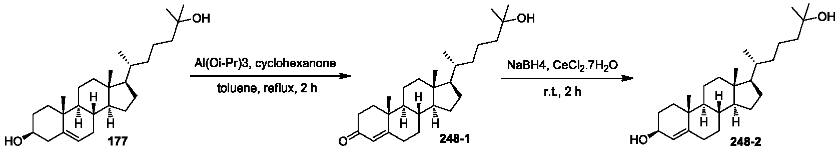

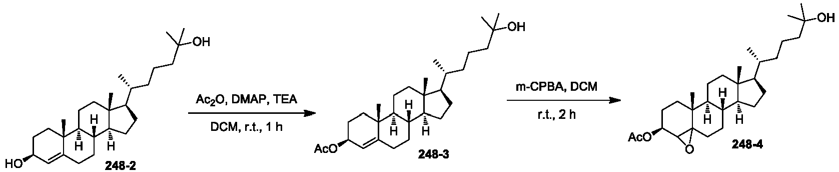

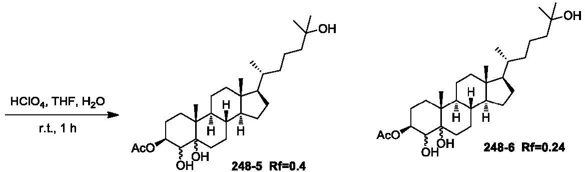

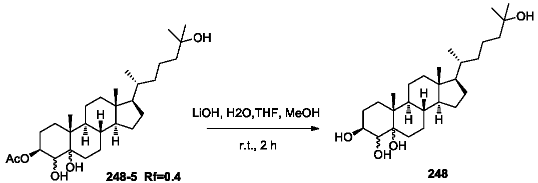

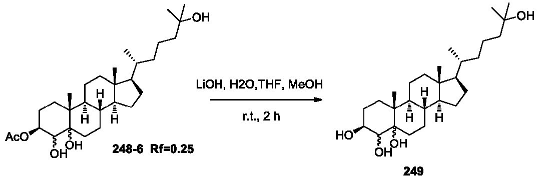

***Step 1*:** (1*R*,3a*S*,7*S*,9a*R*,11a*R*)-1-[(2*R*)-6-Hydroxy-6-methylheptan-2-yl]-9a,11a-dimethyl-2,3,3a,3b,4,6,7,8,9,9a,9b,10,11,11a-tetradecahydro-1*H*-cyclopenta[1,2-*a*]phenanthren-7-ol (**177**) (1.0 g, 2.48 mmol) was dissolved in anhydrous toluene (30 mL) in a 100 mL round-bottom flask. The reaction mixture was heated to 110°C, slowly added dropwise with a solution of cyclohexanone (10 mL) and aluminum isopropoxide (1.7 g, 2.48 mmol) in methanol, and stirred at 110°C for 3 hours. The reaction was monitored by TLC (petroleum ether: ethyl acetate = 5:1). After the reaction was completed, the reaction mixture was quenched with saturated ammonium chloride (50 mL) and extracted with ethyl acetate (40 mL × 3). The organic phase was washed with saturated brine (800 mL), dried over anhydrous sodium sulfate, and purified by silica gel column chromatography (petroleum ether: ethyl acetate = 5:1) to obtain 2,5-hydroxycholest-5(4)-en-3-one (**248-1**) (767 mg, purity: 90%, yield: 69.38%) as a white solid.

¹H NMR (400 MHz, CDCl₃) δ 5.72 (s, 1H), 2.47 - 2.27 (m, 4H), 2.05 - 2.01 (m, 2H), 1.87 - 1.82 (m, 2H), 1.70 - 1.24 (m, 15H), 1.22 (s, 6H), 1.18 (s, 3H), 1.12 - 1.00 (m, 5H), 0.93 (d, *J =* 6.5 Hz, 3H), 0.71 (s, 3H). ¹³C NMR (101 MHz, CDCl₃) δ 199.69, 171.71, 123.76, 71.09, 56.03, 55.87, 53.80, 44.39, 42.41, 39.62, 38.60, 36.38, 35.72, 35.69, 35.61, 33.99, 32.95, 32.04, 29.36, 29.23, 28.20, 24.17, 21.02, 20.77, 18.61, 17.39, 11.96.

***Step 2*:** 25-Hydroxycholest-5(4)-en-3-one (**248-1**) (600 mg, 1.50 mmol) was dissolved in methanol (25 mL) in a 50 mL round-bottom flask. Cerium(III) chloride heptahydrate (1.39 g, 3.75 mmol) and sodium borohydride (141.63 mg, 3.75 mmol) were added thereto at room temperature, and the resulting mixture was stirred at room temperature for 2 hours. The reaction was monitored by TLC (ethyl acetate/petroleum ether = 1/4). After the reaction was completed, the reaction mixture was added with ice water (50 mL) and extracted with dichloromethane (30 mL × 3). The extracted organic solution was washed with saturated brine (60 mL), dried over anhydrous sodium sulfate, filtered, and concentrated to obtain a crude product. The crude product was purified by silica gel column chromatography (eluent: ethyl acetate/petroleum ether = 20%) and concentrated under vacuum to remove the solvent to obtain cholest-5(4)-en-3β,25-diol (**248-2**) (510 mg, purity: 80%, yield: 67.66%) as a white solid. ¹H NMR (400 MHz, CDCl₃) δ 5.27 (d, *J =* 1.5 Hz, 1H), 4.17 - 4.13 (m, 1H), 2.23 - 2.15 (m, 2H), 2.04 - 1.96 (m, 4H), 1.86 - 1.69 (m, 6H), 1.43 - 1.35 (m, 12H), 1.21 (s, 6H), 1.14 - 1.05 (m, 5H), 1.05 (s, 3H), 0.92 (d, *J =* 6.6 Hz, 3H), 0.68 (s, 3H).

***Step 3*:** Cholest-5(4)-en-3β,25-diol (**248-2**) (450 mg, 1.12 mmol) was dissolved in dichloromethane (10 mL) in a 50 mL round-bottom flask at room temperature. Triethylamine (340 mg, 3.36 mmol), 4-dimethylaminopyridine (27.31 mg, 0.224 mmol), and acetic anhydride (171.14 mg, 1.68 mmol) were added thereto at room temperature, and the resulting mixture was stirred at room temperature for 2 hours. The reaction was monitored by TLC (petroleum ether: ethyl acetate = 5:1). After the reaction was completed, the reaction mixture was quenched with water (50 mL) and extracted with ethyl acetate (30 mL × 3). The organic phase was washed with saturated brine (50 mL), dried over anhydrous sodium sulfate, concentrated, and purified by silica gel column chromatography (petroleum ether: ethyl acetate = 5:1) to obtain (1*R*,3a*S*,3b*S*,7*S*,9a*R*,9b*S*,11a*R*)-1-[(2*R*)-6-hydroxy-6-methylheptan-2-yl]-9a,11a-dimethyl-2,3,3a,3b,4,5,7,8,9,9a,9b,10,11,11a-tetradecahydro-1*H*-cyclopenta[1,2-i]phenanthren-7-yl acetate (**248-3**) (440 mg, purity: 90%, yield: 79.68%) as a white solid. ¹H NMR (400 MHz, CDCl₃) δ 5.26 - 5.20 (m, 2H), 2.24 - 2.15 (m, 1H), 2.05 (s, 3H), 2.02 - 1.95 (m, 2H), 1.88 - 1.68 (m, 4H), 1.62 - 1.27 (m, 168H), 1.21 (s, 6H), 1.18 - 1.08 (m, 3H), 1.06 (s, 3H), 1.03 - 0.95 (m, 1H), 0.92 (d, *J=* 6.5 Hz, 3H), 0.68 (s, 3H).

***Step 4*:** (1*R*,3a*S*,3b*S*,7*S*,9a*R*,9b*S*,11a*R*)-1-[(2*R*)-6-Hydroxy-6-methylheptan-2-yl]-9a,11a-dimethyl-2,3,3a,3b,4,5,7,8,9,9a,9b,10,11,11a-tetradecahydro-1*H*-cyclopenta[1,2-*i*]phenanthren-7-yl acetate (**248-3**) (250 mg, 0.562 mmol) was dissolved in dichloromethane (5 mL) in a 50 mL round-bottom flask at room temperature, then *m*-chloroperoxybenzoic acid (145.51 mg, 0.843 mmol) was added thereto at room temperature, and the reaction mixture was stirred at room temperature for 2 hours. The reaction was monitored by TLC (petroleum ether: ethyl acetate = 5:1). After the reaction was completed, the reaction mixture was quenched with saturated sodium sulfite (20 mL) and saturated sodium bicarbonate aqueous solution (20 mL), and extracted with dichloromethane (20 mL × 3). The organic phase was washed with saturated brine (20 mL), dried over anhydrous sodium sulfate, concentrated, and purified by silica gel column chromatography (petroleum ether: ethyl acetate = 5:1) to obtain (2*S*,4a*R*,4b*S*,6a*R*,7*R*,9a*S*,9b*S*)-7-(*R*-6-hydroxy-6-methylheptan-2-yl)-4a,6a-dimethylhexadecahydrocyclopenta[7,8]phenanthro[1,10a-*b*]-2-acetate (**248-4**) (230 mg, purity: 90%, yield: 79.92%) as a white solid. ¹H NMR (400 MHz, CDCl₃) δ 5.14 - 5.10 (m, 0.57H), 4.92 (d, *J =* 3.6 Hz, 1H), 4.31 - 4.26 (m, 1H), 3.16 (d, *J* = 3.3 Hz, 0.5H), 2.13 (s, 3H), 2.10 (s, 1.73H), 2.03 - 1.94 (m, 2H), 1.93 - 1.88 (m, 1H), 1.86 - 1.71 (m, 5H), 1.69 - 1.33 (m, 32H), 1.21 (s, 9.44H), 1.11 (s, 3H), 1.04 (s, 1.72H), 1.03 - 0.97 (m, 4H), 0.92 (d, *J=* 6.4 Hz, 4.74H), 0.68 (s, 1.74H), 0.65 (s, 3H).

***Step 6*:** (2*S*,4a*R*,4b*S*,6a*R*,7*R*,9a*S*,9b*S*)-7-[(2*R*)-6-Hydroxy-6-methylheptan-2-yl]-4a,6a-dimethyl-1a,2,3,4,4a,4b,5,6,6a,7,8,9,9a,9b,10,11-hexadecahydrocyclopenta[1',2':1,2]phenanthro[8,8a-*b*]oxazepin-2-ol (**248-4**) (40 mg, 0.087 mmol) was dissolved in acetone (2 mL) in a 50 mL round-bottom flask at room temperature. The reaction mixture was added dropwise with 10% aqueous solution of perchloric acid (0.087 mL, 0.087 mmol) and stirred at room temperature for 16 hours. The reaction was monitored by TLC (petroleum ether: ethyl acetate = 1:1). After the reaction was completed, the reaction mixture was added with saturated sodium bicarbonate (10 mL) and extracted with dichloromethane (5 mL × 3). The extracted organic solution was washed with saturated brine (10 mL), dried over anhydrous sodium sulfate, and filtered. The organic phase was concentrated and purified by silica gel column chromatography (petroleum ether: ethyl acetate = 1:1) to obtain (1*R*,3a*S*,3b*S*,7*S*,9a*R*,9b*S*,11a*R*)-5a,6-dihydroxy-1-[(2*R*)-6-hydroxy-6-methylheptan-2-yl]-9a,11a-dimethylhexadecahydro-1*H*-cyclopenta[1,2-*i*]phenanthren-7-yl acetate isomer 1 (**248-6**) (Rf = 0.24) (15 mg, purity: 80%, yield: 28.87%) and isomer 2 (**248-5**) (Rf = 0.4) (20 mg, purity: 80%, yield: 38.50%).

**248-5** (Rf = 0.4): ¹H NMR (400 MHz, CDCl₃) δ 5.46 - 5.16 (m, 1H), 3.65 (d, *J* = 3.0 Hz, 1H), 2.30 - 2.18 (m, 1H), 2.08 (s, 3H), 2.02 - 1.96 (m, 2H), 1.88 - 1.77 (m, 3H), 1.76 - 1.70 (m, 2H), 1.63 - 1.52 (m, 7H), 1.47 - 1.35 (m, 10H), 1.21 (s, 6H), 1.18 (s, 3H), 1.11 - 1.01 (m, 5H), 0.92 (d, *J=* 6.4 Hz, 3H), 0.66 (s, 3H).

**248-6** (Rf = 0.24): ¹H NMR (400 MHz, CDCl₃) δ 4.92 (d, *J=* 3.6 Hz, 1H), 4.44 - 3.99 (m, 1H), 2.13 (s, 3H), 1.99 (d, *J =* 12.3 Hz, 1H), 1.92 - 1.80 (m, 2H), 1.75 - 1.50 (m, 11H), 1.47 - 1.31 (m, 12H), 1.21 (s, 6H), 1.11 (s, 3H), 1.07 - 0.98 (m, 4H), 0.92 (d, *J* = 6.5 Hz, 3H), 0.65 (s, 3H).

***Step 7*:** (1*R*,3a*S*,3b*S*,7*S*,9a*R*,9b*S*,11a*R*)-5a,6-Dihydroxy-1-[(2*R*)-6-hydroxy-6-methylheptan-2-yl]-9a,11a-dimethylhexadecahydro-1*H*-cyclopenta[1,2-*i*]phenanthren-7-yl acetate isomer (**248-5**) (Rf = 0.4) (15 mg, 0.031 mmol) was dissolved in tetrahydrofuran (0.5 mL) and methanol (0.5 mL) in a 50 mL round-bottom flask at room temperature, then lithium hydroxide monohydrate (6.57 mg, 0.157 mmol) and water (0.3 mL) were added thereto at room temperature, and the reaction mixture was stirred at room temperature for 2 hours. The reaction was monitored by TLC (petroleum ether: ethyl acetate = 1:2). After the reaction was completed, the reaction mixture was diluted with water (10 mL) and extracted with ethyl acetate (5 mL × 3). The organic phase was washed with saturated brine (10 mL), dried over anhydrous sodium sulfate, concentrated, and purified by silica gel column chromatography (petroleum ether: ethyl acetate = 1:2) to obtain cholest-3β,4,5,25-tetraol isomer 1 (**248**) as a white solid.

**248:** ¹H NMR (400 MHz, CDCl₃) δ 4.14 (s, 1H), 3.54 (s, 1H), 2.24 - 2.16 (m, 1H), 2.05 - 1.93 (m, 2H), 1.80 - 1.58 (m, 10H), 1.51 - 1.33 (m, 13H), 1.21 (s, 6H), 1.15 (s, 3H), 1.11 - 0.99 (m, 5H), 0.92 (d, *J =* 6.4 Hz, 3H), 0.66 (s, 3H). ¹³C NMR (101 MHz, CDCl₃) δ 78.14, 75.68, 71.16, 68.40, 56.28, 56.14, 46.97, 44.41, 42.77, 39.96, 38.40, 36.43, 35.76, 34.56, 31.51, 30.65, 29.71, 29.34, 29.20, 28.27, 25.84, 25.69, 24.10, 20.81, 20.53, 18.62, 15.59, 12.16. LCMS: [M+H-2H₂O]⁺ = 401.

Referring to example 248, (1*R*,3a*S*,3b*S*,7*S*,9a*R*,9b*S*,11a*R*)-5a,6-dihydroxy-1-[(2*R*)-6-hydroxy-6-methylheptan-2-yl]-9a,11a-dimethylhexadecahydro-1*H*-cyclopenta[1,2-*i*]phenanthren-7-yl acetate isomer (**248-5**) (Rf = 0.4) was replaced with (1*R*,3a*S*,3b*S*,7*S*,9a*R*,9b*S*,11a*R*)-5a,6-dihydroxy-1-[(2*R*)-6-hydroxy-6-methylheptan-2-yl]-9a,11a-dimethylhexadecahydro-1*H*-cyclopenta[1,2-*i*]phenanthren-7-yl acetate isomer (**248-6**) (Rf = 0.24) to obtain cholest-3β,4,5,25-tetraol isomer 2 (**249**) as a white solid.

249: ¹H NMR (400 MHz, CDCl₃) δ 4.19 - 4.12 (m, 1H), 3.54 (d, *J* = 3.5 Hz, 1H), 2.24 - 2.17 (m, 1H), 2.04 - 1.94 (m, 2H), 1.83 - 1.58 (m, 10H), 1.49 - 1.33 (m, 13H), 1.21 (s, 6H), 1.15 (s, 3H), 1.10 - 0.99 (m, 5H), 0.92 (d, *J=* 6.5 Hz, 3H), 0.66 (s, 3H). ¹³C NMR (101 MHz, CDCl₃) δ 78.14, 75.67, 71.15, 68.40, 56.28, 56.14, 46.97, 44.41, 42.77, 39.96, 38.40, 36.43, 35.76, 34.56, 31.51, 31.44, 30.65, 30.19, 29.71, 29.34, 29.21, 28.27, 25.84, 25.69, 24.10, 20.81, 20.53, 18.62, 15.59, 12.16. LCMS: [M+H-2H₂O]⁺ = 401.

### Example 250

### Preparation of 5-fluorocholest-3β,4,25-triol (compound 250)

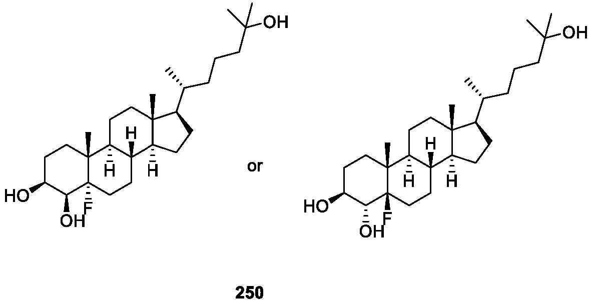

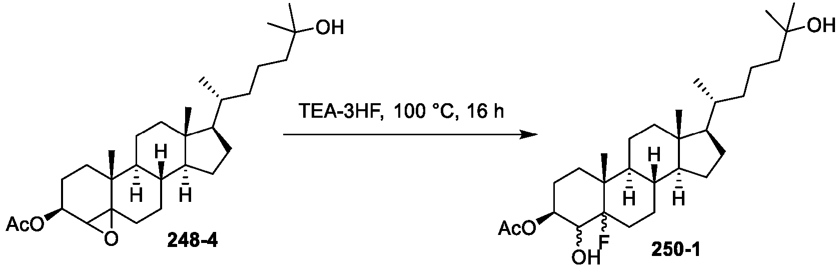

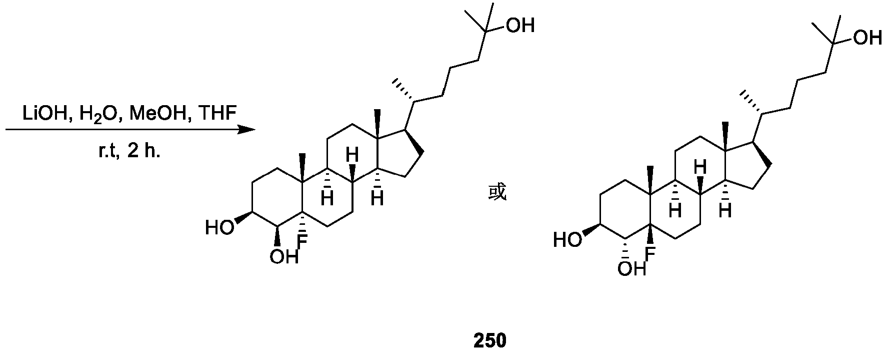

***Step 1*:** (2*S*,4a*R*,4b*S*,6a*R*,7*R*,9a*S*,9b*S*)-7-(*R*-6-Hydroxy-6-methylheptan-2-yl)-4a,6a-dimethylhexadecahydrocyclopenta[7,8]phenanthro[1,10a-*b*]-2-acetate (10 mg, 0.022 mmol) was dissolved in triethylamine trihydrofluoride (0.301 mL, 1.845 mmol) in a 50 mL round-bottom flask at room temperature, and the reaction mixture was stirred at 100°C for 16 hours. The reaction was monitored by TLC (petroleum ether: ethyl acetate = 3 :1). After the reaction was completed, the reaction mixture was diluted with water (10 mL) and extracted with ethyl acetate (5 mL × 3). The organic phase was washed with saturated brine (10 mL), dried over anhydrous sodium sulfate, concentrated, and purified by silica gel column chromatography (petroleum ether: ethyl acetate = 3:1) to obtain (1*R*,3a*S*,3b*S*,7*S*,9a*R*,9b*S*,11a*R*)-5a-fluoro-6-hydroxy-1-[(2*R*)-6-hydroxy-6-methylheptan-2-yl]-9a,11a-dimethylhexadecahydro-1*H-*cyclopenta[1,2-*i*]phenanthren-7-yl acetate (15 mg, purity: 80%, yield: 23.00%) as a white solid. ¹H NMR (400 MHz, CDCl₃) δ 5.09 (dd, *J=* 10.1, 5.3 Hz, 1H), 3.81 (d, *J* = 4.0 Hz, 1H), 2.34 (t, *J =* 7.5 Hz, 1H), 2.26 - 2.20 (m, 1H), 2.09 (s, 4H), 2.05 - 1.98 (m, 4H), 1.82 - 1.46 (m, 22H), 1.21 (s, 6H), 1.15 (s, 4H), 0.92 (d, *J* = 6.4 Hz, 4H), 0.66 (s, 3H). ¹⁹F NMR (376 MHz, CDCl₃) δ -165.13 (s, 1F).

***Step 2*:** (1*R*,3a*S*,3b*S*,7*S*,9a*R*,9b*S*,11a*R*)-5a-Fluoro-6-hydroxy-1-[(2*R*)-6-hydroxy-6-methylheptan-2-yl]-9a,11a-dimethylhexadecahydro-1*H*-cyclopenta[1,2-*i*]phenanthren-7-yl acetate (**250-1**) (12 mg, 0.025 mmol) was dissolved in tetrahydrofuran (1 mL) and methanol (1 mL) in a 50 mL round-bottom flask at room temperature, then lithium hydroxide monohydrate (5 mg, 0.125 mmol) and water (0.5 mL) were added thereto at room temperature, and the reaction mixture was stirred at room temperature for 2 hours. The reaction was monitored by TLC (petroleum ether: ethyl acetate = 1:1). After the reaction was completed, the reaction mixture was diluted with water (10 mL) and extracted with ethyl acetate (5 mL × 3). The organic phase was washed with saturated brine (10 mL), dried over anhydrous sodium sulfate, concentrated, and purified by silica gel column chromatography (petroleum ether: ethyl acetate = 1:1) to obtain 5-fluorocholest-3β,4,25-triol (**250**) (3.5 mg, purity: 100%, yield: 31.96%) as a white solid. ¹H NMR (400 MHz, CDCl₃) δ 4.02 - 3.96 (m, 1H), 3.74 - 3.69 (m, 1H), 2.02 - 1.96 (m, 2H), 1.86 - 1.79 (m, 2H), 1.75 - 1.64 (m, 5H), 1.46 - 1.31(m, 16H), 1.21 (s, 6H), 1.12 (s, 3H), 1.11 - 0.98 (m, 5H), 0.92 (d, *J =* 6.5 Hz, 3H), 0.66 (s, 3H). ¹⁹F NMR (376 MHz, CDCl₃) δ -165.74 (s, 1F). ¹³C NMR (101 MHz, CDCl₃) δ 75.60, 71.14, 69.65, 68.45, 57.66, 56.40, 56.10, 55.98, 46.77, 44.41, 42.67, 39.85, 36.41, 35.75, 34.41, 30.86, 30.84, 30.08, 29.87, 29.70, 29.33, 29.21, 28.26, 27.21, 27.10, 26.44, 25.82, 25.79, 25.26, 24.09, 20.80, 20.36, 18.61, 15.31, 15.24, 12.10. LCMS: (M-H₂O-F)⁺ = 401.

### Example 251

### Preparation of 24-[(2-fluorophenyl)(hydroxy)methyl]-5α-cholan-3β-ol (compound 251)

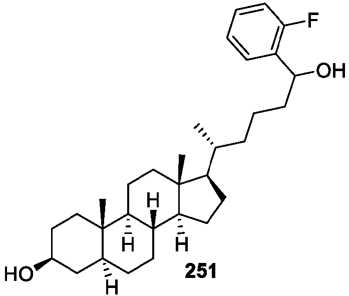

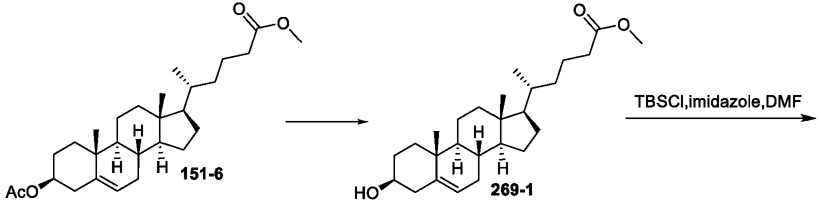

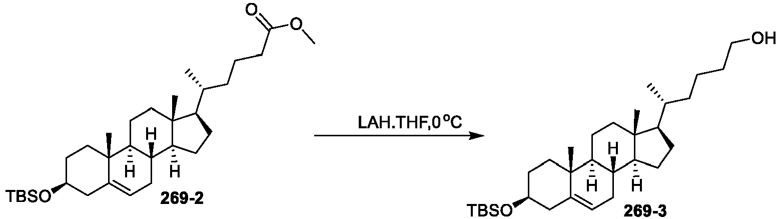

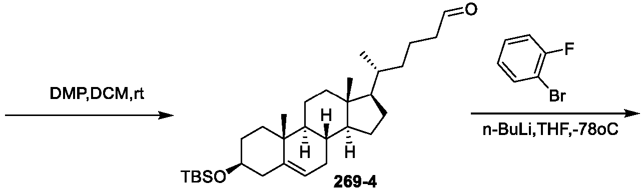

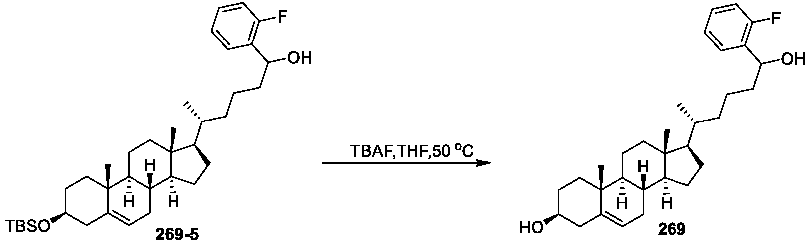

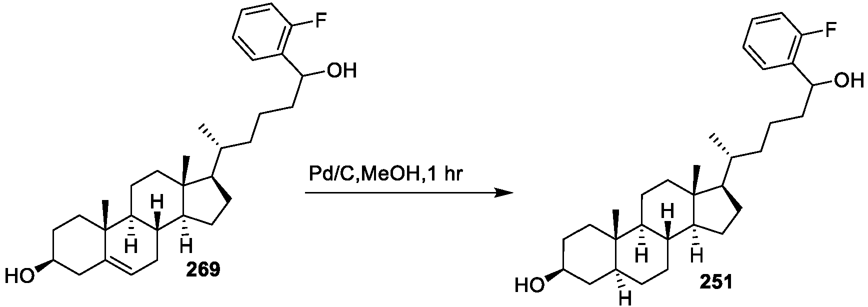

***Step 1:*** The reagent methyl (5*R*)-5-[(1*R*,3a*S*,3b*S*,7*S*,9a*R*,9b*S*,11a*R*)-7-acetoxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate **(151-6)** (2 g, 4.50 mmol) was dissolved in methanol (50 mL), and concentrated sulfuric acid (98%) (1 mL, 18.76 mmol) was added dropwise thereto. The reaction mixture was then heated to reflux and stirred for 1 hour. After the completion of the reaction was monitored by TLC (petroleum ether: ethyl acetate = 10:1), the reaction mixture was concentrated to remove most of the solvent, then added with ethyl acetate (about 100 mL), and washed twice with saturated sodium bicarbonate solution (about 50 mL × 2) and once with saturated brine (about 50 mL). The organic phase was dried over anhydrous sodium sulfate, filtered, and the filtrate was concentrated to dryness to obtain a crude product, which was then purified by flash column chromatography (petroleum ether: ethyl acetate = 90:10 to 80:20) to obtain methyl (5*R*)-5-[(1*R*,3a*S*,3b*S*,7*S*,9a*R*,9b*S*,11a*R*)-7-hydroxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate **(269-1)** (1.8 g, 4.02 mmol, yield: 89.5%) as a white solid. ¹H NMR (400 MHz, CDCl₃) *δ* 5.37 - 5.33 (m, 1H), 3.67 (s, 3H), 3.52 (dd, *J =* 7.7, 3.3 Hz, 1H), 2.35 - 2.20 (m, 4H), 2.03 - 1.93 (m, 2H), 1.84 (ddd, *J =* 12.5, 7.5, 4.3 Hz, 3H), 1.75 - 1.60 (m, 4H), 1.59 - 1.35 (m, 9H), 1.33 - 1.04 (m, 7H), 1.03 - 0.99 (m, 3H), 0.93 (d, *J* = 6.6 Hz, 3H), 0.68(s, 3H).

***Step 2*:** The compound methyl (5*R*)-5-[(1*R*,3a*S*,3b*S*,7*S*,9a*R*,9b*S*,11a*R*)-7-hydroxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate **(269-1)** (1.7 g, 4.38 mmol, 1.0 *eq)* was dissolved in *N,N-*dimethylformamide (50 mL), then *tert*-butyldimethylsilyl chloride (3.3 g, 21.85 mmol, 5.0 *eq*) and imidazole (1.5 g, 21.85 mmol, 5.0 *eq*) were sequentially added thereto, and the reaction mixture was heated to 100°C. After the completion of the reaction was monitored by TLC (petroleum ether: ethyl acetate = 10:1), the reaction mixture was cooled to room temperature and added with ethyl acetate (150 mL). The organic phase was washed three times with water, dried over anhydrous sodium sulfate, filtered, concentrated, and the crude product was purified by flash silica gel column chromatography (petroleum ether: ethyl acetate = 90:10) to obtain (5*R*)-5-[(1*R*,3a*S*,3b*R*,7*S*,9b*S*,11a*R*)-7-{[dimethyl(2-methylpropan-2-yl)methylsilyl]oxy}-11a-methyl-2,3,3a,3b,4,6,7,8,9,9a,9b,10,11,11a-tetradecahydro-1*H*-cyclopenta[1,2-*a*]phenanthren-1-yl]hexanoate **(269-2)** as a colorless wax. ¹H NMR (400 MHz, CDCl₃) *δ* 5.26 - 5.18 (m, 1H), 3.57 (s, 3H), 3.43 - 3.33 (m, 1H), 2.16 (d, *J* = 1.9 Hz, 4H), 1.93 - 1.82 (m, 2H), 1.65 - 1.55 (m, 2H), 1.39 (d, *J =* 3.0 Hz, 9H), 0.99 (s, 7H), 0.90 (s, 5H), 0.85 - 0.79 (m, 21H), 0.57 (s, 3H), -0.00 (s, 6H).

***Step 3:*** The compound (5*R*)-5-[(1*R*,3a*S*,3b*R*,7*S*,9b*S*,11a*R*)-7-{[dimethyl(2-methylpropan-2-yl)methylsilyl]oxy}-11a-methyl-2,3,3a,3b,4,6,7,8,9,9a,9b,10,11,11a-tetradecahydro-1*H*-cyclopenta[1,2-*a*]phenanthren-1-yl]hexanoate **(269-2)** (1.8 g, 3.58 mmol, 1.0 *eq)* was dissolved in tetrahydrofuran (50 mL), then lithium aluminum hydride (0.14 g, 3.580 mmol, 1.0 *eq)* was added thereto, and the reaction mixture was stirred at room temperature for 30 minutes. The reaction was monitored by TLC (petroleum ether: ethyl acetate = 10:1). After the reaction was completed, the reaction mixture was quenched with sodium sulfate decahydrate, filtered, and the filtrate was concentrated to obtain a crude product, which was then purified by flash silica gel column chromatography (petroleum ether: ethyl acetate = 80:20 to 70:30) to obtain (5*R*)-5-[(1*R*,3a*S*,3b*R*,7*S*,9b*S*,11a*R*)-7-{[dimethyl(2-methylpropan-2-yl)methylsilyl]oxy}-11a-methyl-2,3,3a,3b,4,6,7,8,9,9a,9b,10,11,11a-tetradecahydro-1*H-*cyclopenta[1,2-*a*]phenanthren-1-yl]hexan-1-ol **(269-3)** (1.7 g, 3.22 mmol, yield: 90.0%) as a white solid. ¹H NMR (400 MHz, CDCl₃) *δ* 5.26 (d, *J* = 5.3 Hz, 1H), 3.59 (t, *J* = 6.6 Hz, 2H), 3.47 - 3.37 (m, 1H), 2.27 - 2.17 (m, 1H), 2.11 (ddd, *J =* 13.4, 5.0, 2.2 Hz, 1H), 1.93 (s, 2H), 1.77 (s, 2H), 1.66 (d, *J =* 12.2 Hz, 1H), 1.47 (ddd, *J =* 13.9, 8.7, 4.5 Hz, 7H), 1.37 - 1.29 (m, 3H), 1.25 - 1.15 (m, 2H), 1.13 - 0.97 (m, 5H), 0.94 (s, 3H), 0.87 (d, *J =* 6.3 Hz, 4H), 0.84 - 0.81 (m, 10H), 0.62 (s, 3H), 0.02 - -0.02 (m, 6H).

***Step 4:*** The compound (5*R*)-5-[(1*R*,3a*S*,3b*R*,7*S*,9b*S*,11a*R*)-7-{[dimethyl(2-methylpropan-2-yl)methylsilyl]oxy}-11a-methyl-2,3,3a,3b,4,6,7,8,9,9a,9b,10,11,11a-tetradecahydro-1*H*-cyclopenta[1,2-*a*]phenanthren-1-yl]hexan-1-ol **(269-3)** (800 mg, 1.69 mmol, 1.0 *eq*) was dissolved in dichloromethane (40 mL), then Dess-Martin periodinane (833 mg, 1.96 mmol, 1.2 *eq*) was added thereto, and the reaction mixture was stirred at room temperature. The reaction was monitored by TLC (petroleum ether: ethyl acetate = 5:1). After the reaction was completed, the reaction mixture was quenched with saturated sodium sulfite solution and washed with saturated sodium bicarbonate. The organic phase was dried over anhydrous sodium sulfate, filtered, and concentrated to obtain a crude product, which was then purified by flash column chromatography (petroleum ether: ethyl acetate = 90:10) to obtain (5*R*)-5-[(1*R*,3a*S*,3b*S*,7*S*,9a*R*,9b*S*,11a*R*)-7-{[dimethyl(2-methylpropan-2-yl)methylsilyl]oxy}-9a,11a-dimethyl-2,3,3a,3b,4,6,7,8,9,9a,9b,10,11,11a-tetradecahydro-1*H-*cyclopenta[1,2-*a*]phenanthren-1-yl]hexanal **(269-4)** (500 mg, 0.924 mmol, yield: 56.48%) as a white solid. ¹H NMR (400 MHz, CDCl₃) *δ* 9.71 (t, *J* = 1.8 Hz, 1H), 5.26 (d, *J* = 5.2 Hz, 1H), 3.48 - 3.36 (m, 1H), 2.34 (d, *J =* 6.7 Hz, 3H), 2.11 (ddd, *J =* 13.3, 5.0, 2.1 Hz, 1H), 1.93 (s, 2H), 1.75 (d, *J =* 13.3 Hz, 2H), 1.66 (dd, *J =* 12.7, 4.6 Hz, 2H), 1.47 - 1.28 (m, 8H), 1.20 (s, 2H), 1.15 - 0.97 (m, 6H), 0.94 (s, 4H), 0.89 (d, *J =* 6.5 Hz, 4H), 0.85 - 0.82 (m, 10H), 0.62 (s, 3H), 0.01 (m, 6H).

***Step 5:*** The compound 2-bromo-1-fluorobenzene (500 mg, 2.86 mmol) was dissolved in tetrahydrofuran (5 mL). The reaction system was replaced with nitrogen three times and cooled to -78°C in a dry ice bath. A 2.5 M solution of *n*-butyllithium in *n*-hexane (1.26 mL, 3.14 mmol) was then added dropwise thereto with a syringe, and the reaction mixture was reacted at the same temperature for 1 hour and directly used in the next step.

The compound solid (5*R*)-5-[(1*R*,3a*S*,3b*S*,7*S*,9a*R*,9b*S*,11a*R*)-7-{[dimethyl(2-methylpropan-2-yl)methylsilyl]oxy}-9a,11a-dimethyl-2,3,3a,3b,4,6,7,8,9,9a,9b,10,11,11a-tetradecahydro-1*H*-cyclopenta[1,2-*a*]phenanthren-1-yl]hexanal **(269-4)** (500 mg, 1.03 mmol) was dissolved in anhydrous tetrahydrofuran (20 mL). The reaction mixture was cooled to - 10°C in an ice-salt bath under a nitrogen atmosphere. The above prepared (2-fluorophenyl)lithium (6.26 mL) was slowly added thereto, and the reaction mixture was stirred at room temperature for 30 minutes. The reaction was monitored by TLC (petroleum ether: ethyl acetate = 5:1). After the reaction was completed, the reaction mixture was quenched with ice water and extracted twice with ethyl acetate. The organic phase was washed with saturated brine, dried over anhydrous sodium sulfate, filtered, concentrated, and the crude product was purified by silica gel column chromatography (petroleum ether: ethyl acetate = 90:10) to obtain (5*R*)-5-[(1*R*,3a*S*,3b*S*,7*S*,9a*R*,9b*S*,11a*R*)-7-{[dimethyl(2-methylpropan-2-yl)methylsilyl]oxy}-9a,11a-dimethyl-2,3,3a,3b,4,6,7,8,9,9a,9b,10,11,11a-tetradecahydro-1*H-*cyclopenta[1,2-*a*]phenanthren-1-yl]-1-(2-fluorophenyl)hexan-1-ol **(269-5)** (450 mg, 0.58 mmol, yield: 56.37%) as a white solid. ¹H NMR (400 MHz, CDCl₃) δ 7.46 (t, *J* = 7.5 Hz, 1H), 7.26 - 7.19 (m, 1H), 7.15 (t, *J =* 7.5 Hz, 1H), 7.06 - 6.98 (m, 1H), 5.35 (d, *J =* 5.0 Hz, 1H), 5.00 (dd, *J* = 13.2, 6.1 Hz, 1H), 3.52 (dt, *J* = 15.8, 5.6 Hz, 1H), 2.34 - 2.18 (m, 2H), 1.98 (t, *J =* 12.9 Hz, 2H), 1.88 - 1.66 (m, 5H), 1.60 - 1.31 (m, 10H), 1.31 - 1.13 (m, 3H), 1.13 - 1.03 (m, 4H), 1.00 (s, 3H), 0.96 - 0.87 (m, 16H), 0.66 (t, *J =* 6.2 Hz, 3H). 0.01 (m, 6H).

***Step 6*:** The compound (5*R*)-5-[(1*R*,3a*S*,3b*S*,7*S*,9a*R*,9b*S*,11a*R*)-7-{[dimethyl(2-methylpropan-2-yl)methylsilyl]oxy}-9a,11a-dimethyl-2,3,3a,3b,4,6,7,8,9,9a,9b,10,11,11a-tetradecahydro-1*H*-cyclopenta[1,2-*a*]phenanthren-1-yl]-1-(2-fluorophenyl)hexan-1-ol **(269-5)** (250 mg, 0.43 mmol) was dissolved in tetrahydrofuran (10 mL), then 1 mol/L tetrabutylammonium fluoride solution (1 mL) was added thereto, and the reaction mixture was heated to 50°C. The reaction was monitored by TLC (petroleum ether: ethyl acetate = 5:1). After the reaction was completed, the reaction mixture was cooled to room temperature, diluted with ethyl acetate, and washed three times with saturated brine. The organic phase was dried over anhydrous sodium sulfate, filtered, and the filtrate was concentrated to dryness to obtain a crude product. The crude product was purified by flash silica gel column chromatography (petroleum ether: ethyl acetate = 80:20) to obtain a white solid, which was then purified by preparative chiral column chromatography (column: Daicel CHIRALCEL IE, 250 mm/30 mm I.D., 10 µm; mobile phase: CO₂/MeOH [0.2% NH₃ (7 M solution in MeOH)] = 60/40) to obtain 24-[(2-fluorophenyl)(hydroxy)methyl]cholan-6(5)-en-3β-ol **(269)** (50 mg, 0.09 mmol, yield: 21.43%) as a white solid. ¹H NMR (400 MHz, CDCl₃) *δ* 7.46 (t, *J* = 7.5 Hz, 1H), 7.26 - 7.19 (m, 1H), 7.15 (t, *J =* 7.5 Hz, 1H), 7.06 - 6.98 (m, 1H), 5.35 (d, *J =* 5.0 Hz, 1H), 5.00 (dd, *J =* 13.2, 6.1 Hz, 1H), 3.52 (dt, *J =* 15.8, 5.6 Hz, 1H), 2.34 - 2.18 (m, 2H), 1.98 (t, *J =* 12.9 Hz, 2H), 1.88 - 1.66 (m, 5H), 1.60 - 1.31 (m, 10H), 1.31 - 1.13 (m, 3H), 1.13 - 1.03 (m, 4H), 1.00 (s, 3H), 0.96 - 0.87 (m, 4H), 0.66 (t, *J =* 6.2 Hz, 3H). ¹³C NMR (101 MHz, CDCl₃) δ 140.76, 131.84, 128.72, 127.25, 124.28, 121.73, 115.41, 115.15, 71.86, 68.67, 68.44, 68.38, 56.75, 56.06, 50.11, 42.33, 39.77, 38.63, 38.46, 37.25, 36.50, 35.72, 35.59, 31.90, 31.66, 28.24, 24.28, 22.37, 22.26, 21.08, 19.41, 18.61, 11.86. LCMS [M+H-2OH]⁺ = 433.

***Step 7*:** 24-[(2-Fluorophenyl)(hydroxy)methyl]cholan-6(5)-en-3β-ol **(269)** (25 mg, 0.05 mmol) was dissolved in methanol (5 mL). The reaction system was replaced with hydrogen three times and stirred at room temperature for 1 hour. The reaction was monitored by TLC (petroleum ether: ethyl acetate = 5:1). After filtration, the filtrate was concentrated, and the crude product was purified by flash column chromatography (petroleum ether: ethyl acetate = 90:10) to obtain 24-[(2-fluorophenyl)(hydroxy)methyl]-5α-cholan-3β-ol **(251)** (15.62 mg, 0.03 mmol, yield: 49.7%) as a white solid. ¹H NMR (400 MHz, CDCl₃) *δ* 7.46 (t, *J* = 7.5 Hz, 1H), 7.22 (d, *J =* 7.2 Hz, 1H), 7.15 (t, *J =* 7.3 Hz, 1H), 7.05 - 6.98 (m, 1H), 5.00 (dd, *J =* 13.0, 6.0 Hz, 1H), 3.58 (dt, *J =* 15.8, 5.4 Hz, 1H), 1.95 (d, *J =* 12.5 Hz, 1H), 1.80 - 1.62 (m, 8H), 1.59 - 1.47 (m, 4H), 1.4 - 1.43 (m, 1H), 1.39 (dd, *J =* 12.5, 4.4 Hz, 3H), 1.34 - 1.28 (m, 4H), 1.24 - 1.14 (m, 3H), 1.13 - 1.04 (m, 4H), 0.97 (dd, *J =* 11.0, 5.0 Hz, 2H), 0.90 - 0.87 (m, 3H), 0.80 (s, 3H), 0.63 (d, *J* = 1.8 Hz, 3H). ¹³C NMR (101 MHz, CDCl₃) *δ* 128.75, 127.25, 124.29, 124.24, 115.38, 115.16, 71.40, 68.74, 56.49, 56.24, 54.42, 44.86, 42.62, 40.03, 38.58, 38.47, 38.24, 37.01, 35.65, 35.51, 35.47, 32.08, 31.55, 28.74, 28.24, 24.21, 22.37, 22.26, 21.26, 18.56, 12.32, 12.07.

### Example 253

### Preparation of 24-(hydroxycyclopropyl)-5α-cholan-3β-ol (compound 253)

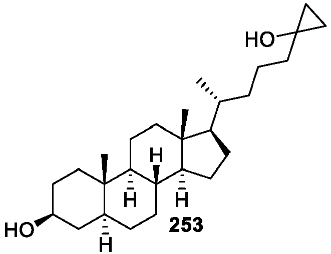

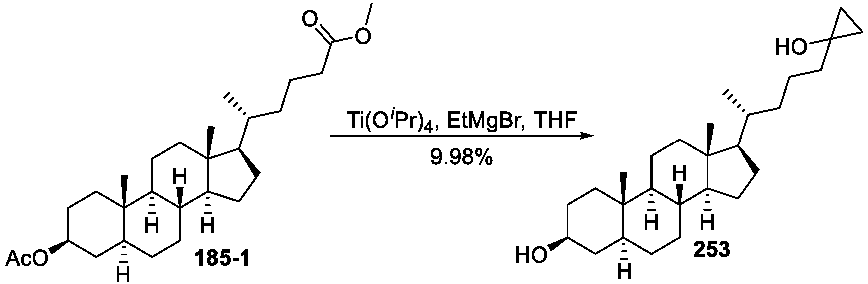

(1*R*,3a*S*,5a*S*,7*S*,9a*S*,11a*R*)-1-[(2*R*)-6-Methoxy-6-oxohexan-2-yl]-9a,11a-dimethylhexadecahydro-1*H*-cyclopenta[1,2-*i*]phenanthren-7-yl acetate **(185-1)** (50 mg, 0.11 mmol) was dissolved in tetrahydrofuran (5 mL), and titanium isopropoxide (95.6 mg, 0.670 mmol) was added thereto at room temperature. The reaction system was replaced with nitrogen three times, added dropwise with ethylmagnesium chloride (0.67 mL) at room temperature, and stirred at room temperature for 1 hour. The reaction was monitored by TLC (petroleum ether: ethyl acetate = 5:1). The reaction mixture was quenched with brine at low temperature, diluted with ethyl acetate, dried over anhydrous sodium sulfate, concentrated, and purified by column chromatography (petroleum ether: ethyl acetate = 50:1 to 1:1) to obtain 24-(hydroxycyclopropyl)-5α-cholan-3β-ol **(253)** (5 mg, 0.006 mmol, 5.55%). ¹H NMR (399 MHz, Chloroform-d) δ 3.57 (dt, *J =* 11.1, 6.0 Hz, 1H), 1.94 (d, *J =* 13.5 Hz, 1H), 1.74 (d, *J =* 26.7 Hz, 5H), 1.53 - 1.42 (m, 6H), 1.35 (d, *J =* 12.5 Hz, 5H), 1.22 (d, *J =* 14.8 Hz, 5H), 1.08 (q, *J =* 9.3, 8.7 Hz, 5H), 0.94 (s, 2H), 0.90 (d, *J =* 6.4 Hz, 3H), 0.81 (d, *J =* 6.1 Hz, 1H), 0.78 (s, 3H), 0.71 (s, 2H), 0.63 (s, 3H), 0.43 (d, *J =* 5.4 Hz, 2H). ¹³C NMR (100 MHz, CDCl₃) δ 71.35, 56.46, 56.08, 55.93, 54.31, 44.82, 42.58, 40.00, 38.81, 38.18, 36.97, 35.91, 35.74, 35.48, 35.43, 32.05, 31.50, 28.70, 28.22, 24.18, 22.29, 21.23, 18.61, 13.58, 13.46, 12.30, 12.05, 0.99.

LC-MS: [M-H₂O-OH]⁺ = 367.5.

### Example 254

### Preparation of 4-methyl-5α-cholest-3β,4,25-triol (compound 254)

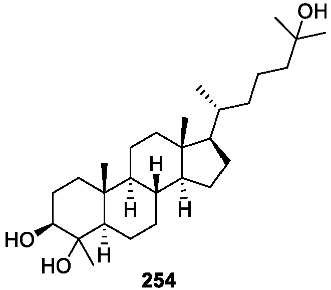

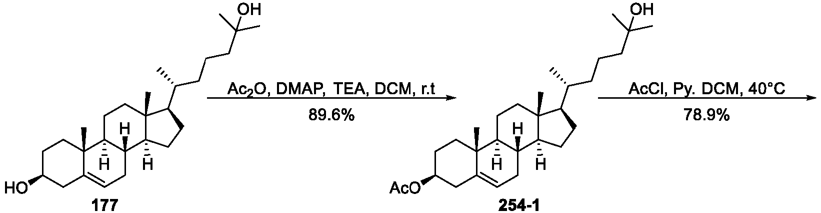

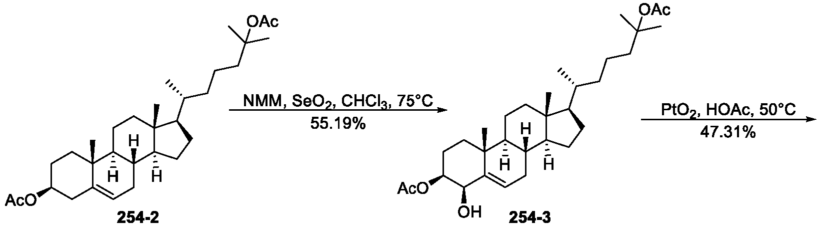

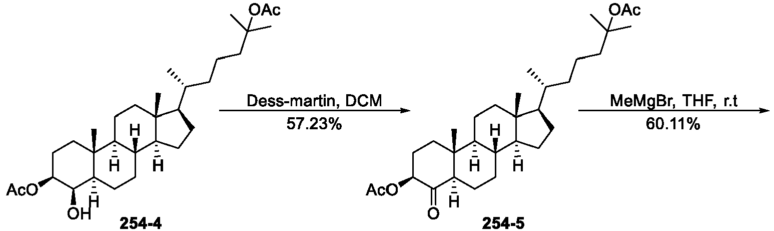

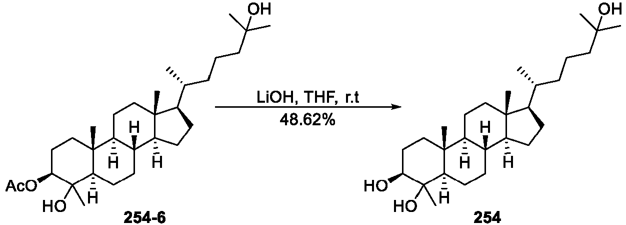

**Step 1:** Cholest-5(6)-en-3β,25-diol (177) (1.6 g, 2.384 mmol) was dissolved in dichloromethane (20 mL). Triethylamine (1.6 mL, 11.92 mmol), 4-dimethylaminopyridine (29.13 mg, 0.24 mmol), and acetic anhydride (0.45 mL, 4.77 mmol) were added thereto, and the reaction mixture was stirred at room temperature for 2 hours. The reaction was monitored by TLC (petroleum ether: ethyl acetate = 3 :1). The reaction mixture was quenched with water, extracted three times with dichloromethane, washed once with brine, dried over anhydrous sodium sulfate, and concentrated to obtain a crude product (1.6 g), which was then purified by column chromatography (petroleum ether: ethyl acetate = 15:1) to obtain (1*R*,3a*S*,3b*S*,7*S*,9a*R*,9b*S*,11a*R*)-1-[(2*R*)-6-hydroxy-6-methylheptan-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate **(254-1)** (1.0 g, 2.1 mmol, 89.60%) as a white solid. ¹H NMR (399 MHz, Chloroform-d) δ 5.38 - 5.32 (m, 1H), 4.57 (dt, *J =* 10.7, 4.8 Hz, 1H), 2.30 (d, *J =* 7.9 Hz, 2H), 2.01 (s, 3H), 1.97 (d, *J =* 2.9 Hz, 2H), 1.83 (t, *J =* 8.5 Hz, 3H), 1.62 - 1.50 (m, 3H), 1.49 - 1.40 (m, 6H), 1.35 (t, *J =* 8.1 Hz, 3H), 1.23 (d, *J =* 3.0 Hz, 2H), 1.18 - 1.13 (m, 2H), 1.13 - 1.02 (m, 4H), 1.00 (s, 3H), 0.91 (d, *J =* 6.5 Hz, 3H), 0.66 (s, 3H).

**Step 2:** (1*R*,3a*S*,3b*S*,7*S*,9a*R*,9b*S*,11a*R*)-1-[(2*R*)-6-Hydroxy-6-methylheptan-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate **(254-1)** (330 mg, 0.7 mmol) and pyridine (0.3 mL, 3.710 mmol) were dissolved in dichloromethane (10 mL), then acetyl chloride (0.16 mL, 2.2 mmol) was added thereto at room temperature, and the reaction system was stirred at 40°C for 3 hours. The reaction was monitored by TLC (petroleum ether: ethyl acetate = 3:1). The reaction mixture was quenched with water, extracted three times with dichloromethane, washed once with brine, dried over anhydrous sodium sulfate, and concentrated to obtain a crude product (500 mg), which was then purified by column chromatography (petroleum ether: ethyl acetate = 60:1) to obtain (1*R*,3a*S*,3b*S*,7*S*,9a*R*,9b*R*,11a*R*)-1-[(2*R*)-6-acetoxy-6-methylheptan-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate **(254-2)** (300 mg, 0.586 mmol, 78.90%) as a light yellow solid. ¹H NMR (399 MHz, Chloroform-d) δ 5.36 (d, *J =* 4.3 Hz, 1H), 4.58 (s, 1H), 2.29 (d, *J =* 8.1 Hz, 2H), 2.02 - 1.99 (m, 3H), 1.94 (d, *J =* 1.5 Hz, 3H), 1.84 (d, *J =* 11.5 Hz, 3H), 1.76 - 1.58 (m, 3H), 1.56 (d, *J* = 6.9 Hz, 2H), 1.50 (d, *J =* 18.8 Hz, 4H), 1.41 - 1.39 (m, 6H), 1.35 (s, 3H), 1.28 - 1.22 (m, 3H), 1.22 - 1.05 (m, 6H), 1.00 (s, 3H), 0.90 (d, *J =* 6.6 Hz, 3H), 0.80 (d, *J =* 7.1 Hz, 1H), 0.67 (d, *J* = 7.3 Hz, 3H).

**Step 3:** (1*R*,3a*S*,3b*S*,7*S*,9a*R*,9b*S*,11a*R*)-1-[(2*R*)-6-Acetoxy-6-methylheptan-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate **(254-2)** (300 mg, 0.62 mmol) was dissolved in chloroform (6 mL), then selenium dioxide (171 mg, 1.54 mmol) and *N*-methylmorpholine (187.03 mg, 1.85 mmol) were added thereto, and the reaction system was stirred at 75°C overnight. The completion of the reaction was monitored by TLC (petroleum ether: ethyl acetate = 3:1). The reaction mixture was concentrated to obtain a crude product (500 mg), which was then purified by column chromatography (petroleum ether: ethyl acetate = 10:1) to obtain (1*R*,3a*S*,3b*S*,6*R*,7*S*,9a*R*,9b*S*,11a*R*)-1-[(2*R*)-6-acetoxy-6-methylheptan-2-yl]-6-hydroxy-9a,11a-dimethyl-2,3,3a,3b,4,6,7,8,9,9a,9b,10,11,11a-tetradecahydro-1*H*-cyclopenta[1,2-*i*]phenanthren-7-yl acetate **(254-3)** (180 mg, 0.34 mmol, 55.19%) as a light yellow solid. ¹H NMR (399 MHz, Chloroform-d) δ 5.68 (d, *J =* 4.7 Hz, 1H), 4.70 (dt, *J =* 12.2, 4.0 Hz, 1H), 4.23 (d, *J =* 3.3 Hz, 1H), 2.08 (s, 3H), 2.00 (d, *J =* 15.1 Hz, 3H), 1.95 (s, 3H), 1.87 - 1.79 (m, 3H), 1.72 - 1.58 (m, 5H), 1.44 (q, *J* = 4.4, 3.5 Hz, 3H), 1.40 (s, 6H), 1.35 (s, 4H), 1.31 (s, 3H), 1.20 (s, 3H), 1.16 (s, 2H), 1.08 (s, 2H), 1.00 (s, 2H), 0.95 (d, *J =* 7.2 Hz, 2H), 0.90 (d, *J =* 6.4 Hz, 3H), 0.87 - 0.84 (m, 3H), 0.66 (s, 3H).

**Step 4:** (1*R*,3a*S*,3b*S*,6*R*,7*S*,9a*R*,9b*S*,11a*R*)-1-[(2*R*)-6-Acetoxy-6-methylheptan-2-yl]-6-hydroxy-9a,11a-dimethyl-2,3,3a,3b,4,6,7,8,9,9a,9b,10,11,11a-tetradecahydro-1*H-*cyclopenta[1,2-*i*]phenanthren-7-yl acetate **(254-3)** (100 mg, 0.2 mmol) was dissolved in acetic acid (3 mL), then platinum dioxide (33 mg, 0.15 mmol) was added thereto, and the reaction mixture was stirred at 50°C for 0.5 hours under a hydrogen atmosphere. The completion of the reaction was monitored by TLC (petroleum ether: ethyl acetate = 3:1). The reaction mixture was filtered and concentrated to obtain a crude product (100 mg), which was then purified by column chromatography (petroleum ether: ethyl acetate = 15:1) to obtain (1*R*,3a*S*,3b*S*,5a*R*,6*R*,7*S*,9a*R*,9b*S*,11a*R*)-1-[(2*R*)-6-acetoxy-6-methylheptan-2-yl]-6-hydroxy-9a,11a-dimethylhexadecahydro-1*H*-cyclopenta[1,2-*i*]phenanthren-7-yl acetate **(254-4)** (50 mg, 0.094 mmol, 47.31%) as a white solid. ¹H NMR (399 MHz, Chloroform-d) δ 4.70 (dt, *J* = 12.3, 4.1 Hz, 1H), 3.80 (s, 1H), 2.06 (s, 3H), 1.94 (s, 3H), 1.93 - 1.79 (m, 2H), 1.77 - 1.70 (m, 4H), 1.64 (d, *J* = 11.8 Hz, 2H), 1.59 - 1.50 (m, 2H), 1.39 (s, 6H), 1.33 (d, *J =* 3.1 Hz, 4H), 1.23 (s, 3H), 1.19 - 1.12 (m, 2H), 1.10 - 1.04 (m, 3H), 1.03 (s, 3H), 0.99 - 0.93 (m, 2H), 0.88 (d, *J* = 6.4 Hz, 3H), 0.86 - 0.77 (m, 2H), 0.63 (s, 3H), 0.57 (d, *J =* 10.8 Hz, 1H).

**Step 5:** (1*R*,3a*S*,3b*S*,5a*R*,6*R*,7*S*,9a*R*,9b*R*,11a*R*)-1-[(2*R*)-6-Acetoxy-6-methylheptan-2-yl]-6-hydroxy-9a,11a-dimethylhexadecahydro-1*H*-cyclopenta[1,2-*i*]phenanthren-7-yl acetate **(254-4)** (550 mg, 1.09 mmol) was dissolved in dichloromethane (15 mL), then Dess-Martin periodinane (693.25 mg, 1.634 mmol) was added thereto, and the reaction mixture was stirred at room temperature for 1 hour. The reaction was monitored by TLC (petroleum ether: ethyl acetate = 5:1). After filtration, the filtrate was diluted with water and extracted three times with dichloromethane. The organic phase was washed once with brine, dried over anhydrous sodium sulfate, and concentrated to obtain a crude product (10 mg), which was then purified by column chromatography (petroleum ether: ethyl acetate = 25:1) to obtain (1*R*,3a*S*,3b*S*,5a*R*,6*R*,7*S*,9a*R*,9b*S*,11a*R*)-1-[(2*R*)-6-acetoxy-6-methylheptan-2-yl]-6-hydroxy-9a,11a-dimethylhexadecahydro-1*H*-cyclopenta[1,2-*i*]phenanthren-7-yl acetate **(254-5)** (330 mg, 0.62 mmol, 57.23%) as a white solid. ¹H NMR (400 MHz, DMSO-d₆) δ 5.15 (dd, *J =* 12.3, 7.2 Hz, 1H), 2.34 (d, *J =* 11.6 Hz, 1H), 2.12 (s, 1H), 2.02 (s, 3H), 1.92 (s, 1H), 1.87 (s, 3H), 1.83 - 1.68 (m, 3H), 1.57 (d, *J* = 11.9 Hz, 4H), 1.47 (d, *J* = 11.3 Hz, 2H), 1.39 (d*, J =* 14.0 Hz, 2H), 1.32 (s, 6H), 1.28 (d, *J* = 13.4 Hz, 3H), 1.20 (s, 3H), 1.14 (d, *J =* 10.8 Hz, 2H), 1.10 - 1.01 (m, 2H), 0.94 (t, *J* = 8.9 Hz, 3H), 0.85 (d, *J =* 6.5 Hz, 3H), 0.77 (d, *J* = 13.2 Hz, 1H), 0.60 (d, *J* = 6.4 Hz, 6H).

**Step 6:** (1*R*,3a*S*,3b*S*,5a*R*,6*R*,7*S*,9a*R*,9b*S*,11a*R*)-1-[(2*R*)-6-Acetoxy-6-methylheptan-2-yl]-6-hydroxy-9a,11a-dimethylhexadecahydro-1*H*-cyclopenta[1,2-*i*]phenanthren-7-yl acetate **(254-5)** (50 mg, 0.1 mmol) was dissolved in tetrahydrofuran (3 mL). The reaction mixture was cooled to 0°C under a nitrogen atmosphere, added with methylmagnesium bromide (0.33 mL, 1 mmol), and stirred at room temperature for 1 hour. The completion of the reaction was monitored by TLC (petroleum ether: ethyl acetate = 3:1). The reaction mixture was quenched with water and extracted three times with ethyl acetate. The organic phase was washed once with brine, dried over anhydrous sodium sulfate, and concentrated to obtain a crude product (60 mg). The crude product was purified by column chromatography (petroleum ether: ethyl acetate = 12:1) to obtain (1*R*,3a*S*,3b*S*,5a*R*,7*S*,9a*R*,9b*S*,11a*R*)-6-hydroxy-1-[(2*R*)-6-hydroxy-6-methylheptan-2-yl]-6,9a,11a-trimethylhexadecahydro-1*H-*cyclopenta[1,2-*i*]phenanthren-7-yl acetate **(254-6)** (30 mg, 0.060 mmol, 60.11%) as a white solid. ¹H NMR (400 MHz, DMSO-d₆) δ 4.23 (d, *J =* 6.5 Hz, 1H), 3.26 (s, 1H), 2.97 (dt, *J =* 10.7, 5.4 Hz, 1H), 1.86 (s, 3H), 1.65 (t, *J =* 12.5 Hz, 3H), 1.55 (t, *J =* 11.8 Hz, 3H), 1.41 (s, 2H), 1.38 (s, 2H), 1.32 (s, 6H), 1.27 (d, *J =* 10.4 Hz, 3H), 1.21 (d, *J* = 9.6 Hz, 4H), 1.15 (s, 2H), 1.12 (s, 2H), 1.02 (s, 3H), 0.99 - 0.93 (m, 2H), 0.91 (s, 3H), 0.88 (s, 2H), 0.84 (d, *J =* 6.7 Hz, 3H), 0.77 - 0.69 (m, 2H), 0.57 (s, 3H), 0.50 (t, *J =* 11.1 Hz, 1H).

**Step 7:** (1*R,*3a*S*,3b*S*,5a*R*,7*S*,9a*R*,9b*R*,11a*R*)-6-hydroxy-1-[(2*R*)-6-hydroxy-6-methylheptan-2-yl]-6,9a,11a-trimethylhexadecahydro-1*H*-cyclopenta[1,2-*i*]phenanthren-7-yl acetate **(254-6)** (30 mg, 0.063 mmol) was dissolved in tetrahydrofuran (3 mL) and methanol (1.5 mL), then lithium hydroxide (1.5 mL, 1.50 mmol) was added thereto, and the reaction mixture was stirred at room temperature for 18 hours. The completion of the reaction was monitored by TLC (petroleum ether: ethyl acetate = 2:1). The reaction mixture was concentrated. The crude product was purified by column chromatography (petroleum ether: ethyl acetate = 8:1) to obtain 4-methyl-5α-cholest-3β,4,25-triol **(254)** (14 mg, 0.031 mmol, 48.62%) as a white solid with a purity of 94.0%. ¹H NMR (400 MHz, DMSO-d₆) δ 4.23 (d, *J =* 6.7 Hz, 1H), 4.01 (s, 1H), 3.26 (s, 1H), 3.01 - 2.93 (m, 1H), 1.97 - 1.85 (m, 1H), 1.64 (d, *J =* 28.8 Hz, 1H), 1.54 (t, *J* = 12.2 Hz, 2H), 1.48 - 1.32 (m, 4H), 1.30 (d, *J =* 6.4 Hz, 3H), 1.24 (d, *J =* 15.8 Hz, 6H), 1.13 (d, *J =* 12.0 Hz, 3H), 1.05 (s, 1H), 1.01 (d, *J =* 3.9 Hz, 9H), 0.94 (s, 2H), 0.91 (s, 3H), 0.88 (s, 1H), 0.84 (d, *J =* 6.5 Hz, 3H), 0.81 (d, *J =* 7.2 Hz, 1H), 0.73 (d, *J =* 12.5 Hz, 2H), 0.57 (s, 3H), 0.51 (d, *J =* 12.7 Hz, 1H). ¹³C NMR (101 MHz, DMSO-d₆) δ 73.43, 73.43, 69.20, 55.46, 42.52, 36.27, 29.85, 29.65, 25.41, 18.95, 14.53, 12.33. LCMS: [M+Na]⁺ = 457.30, [M+ACN+Na]⁺ = 498.30.

### Example 255

### Preparation of 3β,25-dihydroxy-5α-cholest-6-one (compound 255)

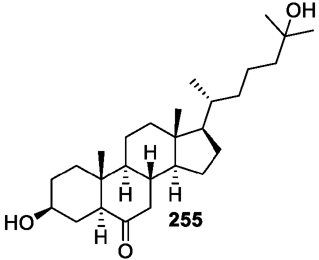

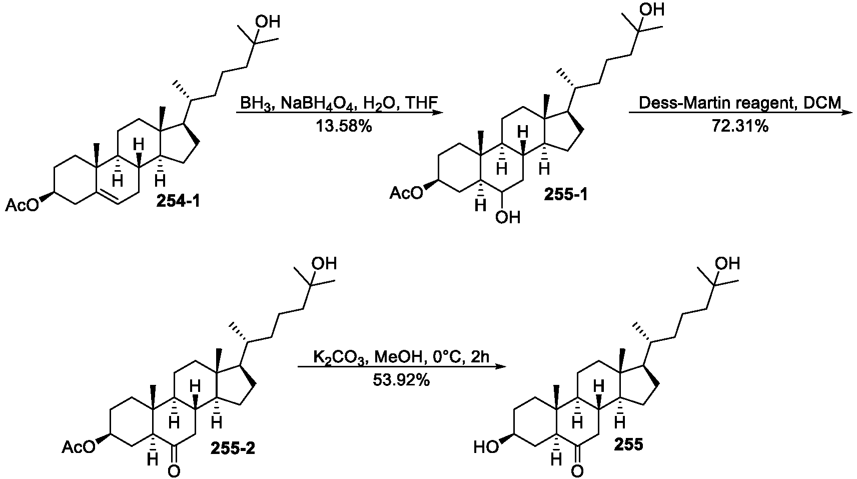

**Step 1:** (1*R*,3a*S*,3b*S*,7*S*,9a*R*,9b*S*,11a*R*)-1-[(2*R*)-6-Hydroxy-6-methylheptan-2-yl]-9a,11a-dimethyl-2,3,3a,3b,4,6,7,8,9,9a,9b,10,11,11a-tetradecahydro-1*H*-cyclopenta[1,2-i]phenanthren-7-yl acetate **(254-1)** (100 mg, 0.23 mmol) was dissolved in anhydrous tetrahydrofuran (5 mL), and the reaction mixture was cooled to -10°C under a nitrogen atmosphere. A 1 M solution of borane in tetrahydrofuran (6.2 mg, 0.07 mmol) was added dropwise thereto, and the reaction mixture was naturally warmed to room temperature and stirred for 3 hours. Water (0.2 mL) was added dropwise thereto at 0°C, then sodium perborate (tetrahydrate) (11.03 mg, 0.07 mmol) was added thereto, and the reaction mixture was stirred at room temperature for 16 hours. The reaction was monitored by TLC (petroleum ether: ethyl acetate = 1:1). The reaction mixture was then quenched with water, extracted twice with ethyl acetate, dried over anhydrous sodium sulfate, evaporated to dryness by rotary evaporation, and subjected to column chromatography (petroleum ether: ethyl acetate = 30:1 to 5:1) to obtain compound (1*R*,3a*S*,3b*S*,7*S*,9a*R*,9b*S*,11a*R*)-5-hydroxy-1-[(2*R*)-6-hydroxy-6-methylheptan-2-yl]-9a,11a-dimethylhexadecahydro-1*H*-cyclopenta[1,2-*i*]phenanthren-7-yl acetate **(255-1)** (5 mg, 0.010 mmol, 13.58%). ¹H NMR (399 MHz, Chloroform-d) δ 4.65 (td, *J =* 11.4, 5.5 Hz, 1H), 3.37 (td*, J =* 10.6, 4.5 Hz, 1H), 2.19 (d, *J* = 12.6 Hz, 1H), 2.04 - 1.93 (m, 4H), 1.81 (d, *J =* 10.6 Hz, 2H), 1.70 (dd, *J =* 13.4, 3.7 Hz, 1H), 1.65 - 1.52 (m, 3H), 1.49 - 1.30 (m, 8H), 1.29 - 1.21 (m, 4H), 1.19 (s, 6H), 1.11 - 0.97 (m, 5H), 0.90 (d, *J =* 6.5 Hz, 3H), 0.81 (s, 3H), 0.63 (s, 3H).

**Step 2:** (1*R*,3a*S*,3b*S*,5a*S*,7*S*,9a*R*,9b*S*,11a*R*)-5-Hydroxy-1-[(2*R*)-6-hydroxy-6-methylheptan-2-yl]-9a,11a-dimethylhexadecahydro-1*H*-cyclopenta[1,2-*i*]phenanthren-7-yl acetate **(255-2)** (15 mg, 0.032 mmol) was dissolved in anhydrous tetrahydrofuran (5 mL). The reaction mixture was cooled to 0°C under a nitrogen atmosphere, added with Dess-Martin periodinane (41.25 mg, 0.1 mmol), and naturally warmed to room temperature and stirred for 3 hours. The completion of the reaction was monitored by TLC (petroleum ether: ethyl acetate = 3 :1). The reaction mixture was then quenched with water, extracted twice with ethyl acetate, dried over anhydrous sodium sulfate, evaporated to dryness by rotary evaporation, and purified by column chromatography (petroleum ether: ethyl acetate = 50:1 to 5:1) to obtain compound (1*R*,3a*S*,3b*S*,5a*S*,7*S*,9a*R*,9b*S*,11a*R*)-1-[(2*R*)-6-hydroxy-6-methylheptan-2-yl]-9a,11a-dimethyl-5-oxohexadecahydro-1*H*-cyclopenta[1,2-*i*]phenanthren-7-yl acetate **(255-2)** (12 mg, 0.023 mmol, 72.31%) as a white solid. ¹H NMR (399 MHz, Chloroform-d) δ 4.65 (dt, *J =* 11.9, 6.8 Hz, 1H), 2.33 - 2.19 (m, 2H), 2.00 (s, 3H), 1.89 (d, *J* = 8.8 Hz, 1H), 1.80 (t, *J =* 15.5 Hz, 3H), 1.42 - 1.30 (m, 6H), 1.19 (s, 6H), 0.91 (d, *J =* 6.4 Hz, 3H), 0.75 (s, 3H), 0.64 (s, 3H).

**Step 3:** (1*R*,3a*S*,3b*S*,5a*S*,7*S*,9a*R*,9b*S*,11a*R*)-1-[(2*R*)-6-Hydroxy-6-methylheptan-2-yl]-9a,11a-dimethyl-5-oxohexadecahydro-1*H*-cyclopenta[1,2-*i*]phenanthren-7-yl acetate **(255-2)** (12 mg, 0.03 mmol) was dissolved in methanol (5 mL), then potassium carbonate (10 mg, 0.065 mmol) was added thereto, and the reaction mixture was stirred at room temperature for 2 hours. The completion of the reaction was monitored by TLC (petroleum ether: ethyl acetate = 2:1). The reaction mixture was then quenched with water, extracted twice with ethyl acetate, dried over anhydrous sodium sulfate, evaporated to dryness by rotary evaporation, and purified by column chromatography (petroleum ether: ethyl acetate = 50:1 to 5:1) to obtain compound 3β,25-dihydroxy-5α-cholest-6-one **(255)** (7.9 mg, 0.014 mmol, 53.92%).

¹H NMR (399 MHz, Chloroform-d) δ 3.55 (dt, *J =* 11.2, 6.2 Hz, 1H), 2.29 (dd, *J* = 13.1, 4.5 Hz, 1H), 2.22 - 2.14 (m, 1H), 1.94 (dd, *J =* 27.4, 14.7 Hz, 2H), 1.87 - 1.73 (m, 4H), 1.60 - 1.45 (m, 4H), 1.44 - 1.30 (m, 7H), 1.28 - 1.20 (m, 6H), 1.19 (s, 6H), 1.11 - 0.99 (m, 3H), 0.91 (d, *J =* 6.5 Hz, 3H), 0.73 (s, 3H), 0.64 (s, 3H). ¹³C NMR (101 MHz, CDCl₃) δ 210.96, 71.08, 70.67, 56.74, 56.70, 56.00, 53.87, 46.69, 44.32, 42.96, 40.92, 39.46, 37.88, 36.62, 36.31, 35.63, 30.66, 30.00, 29.35, 29.17, 28.03, 23.94, 21.48, 20.70, 18.58, 13.11, 11.99, 0.99. LC-MS: [M-H₂O-OH]⁺ = 383.30.

### Example 266

### Preparation of 5-fluorocholest-3β,25-diol (compound 266)

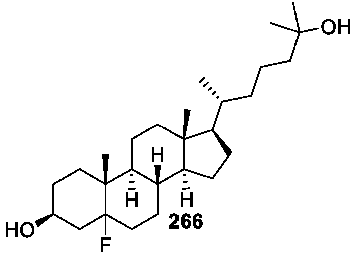

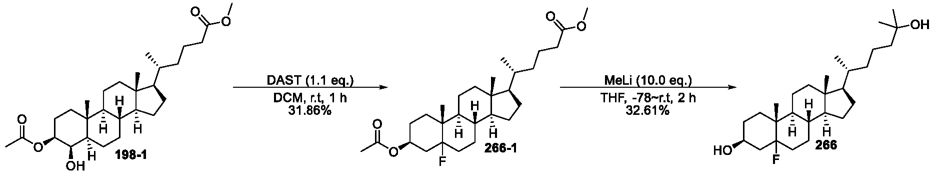

**Step 1:** (1*R*,3a*S*,3b*S*,5a*R*,6*R*,7*S*,9a*R*,9b*S*,11a*R*)-6-Hydroxy-1-[(2*R*)-6-methoxy-6-oxohexan-2-yl]-9a,11a-dimethylhexadecahydro-1*H*-cyclopenta[1,2-*i*]phenanthren-7-yl acetate **(198-1)** (100 mg, 0.216 mmol) was weighed and dissolved in dichloromethane. The reaction mixture was cooled in an ice bath, added dropwise with DAST (38.32 mg, 0.238 mmol), stirred for 10 minutes, then warmed to room temperature, and stirred. The completion of the reaction was monitored by TLC (petroleum ether: ethyl acetate = 5:1). The reaction mixture was quenched with water and extracted with dichloromethane. The organic phase was dried and concentrated to obtain a crude product, which was then subjected to column chromatography (petroleum ether: ethyl acetate = 50:1 to 30:1 to 10:1) to obtain (1*R*,3a*S*,3b*S*,7*S*,9a*R*,9b*S*,11a*R*)-5a-fluoro-1-[(2*R*)-6-methoxy-6-oxohexan-2-yl]-9a,11a-dimethylhexadecahydro-1*H*-cyclopenta[1,2-*i*]phenanthren-7-yl acetate **(266-1)** (40 mg, 0.069 mmol, 31.86%). ¹H NMR (399 MHz, Chloroform-*d*) δ 5.08 - 4.97 (m, 1H), 3.64 (s, 3H), 2.31 - 2.19 (m, 2H), 2.00 (s, 3H), 1.96 - 1.86 (m, 3H), 1.84 - 1.75 (m, 1H), 1.73 - 1.60 (m, 3H), 1.51 (s, 4H), 1.37 (d, *J =* 9.1 Hz, 5H), 1.29 - 1.19 (m, 5H), 1.18 - 1.10 (m, 2H), 1.08 - 0.98 (m, 4H), 0.96 (s, 3H), 0.90 (d, *J* = 6.5 Hz, 3H), 0.63 (s, 3H). ¹⁹F NMR (376 MHz, Chloroform-*d*) δ -162.54 (t, *J* = 42.8 Hz).

**Step 2:** (1*R*,3a*S*,3b*S*,7*S*,9a*R*,9b*S*,11a*R*)-5a-Pluoro-1-[(2*R*)-6-methoxy-6-oxohexan-2-yl]-9a,11a-dimethylhexadecahydro-1*H*-cyclopenta[1,2-*i*]phenanthren-7-yl acetate **(266-1)** (40 mg, 0.086 mmol) was weighed and dissolved in ultra-dry tetrahydrofuran (10 mL). The reaction mixture was cooled to -78°C in a dry ice bath, and methyllithium (0.537 mL, 0.860 mmol) was added dropwise thereto. After the dropwise addition was completed, the reaction mixture was warmed to room temperature and stirred. The completion of the reaction was monitored by TLC (petroleum ether: ethyl acetate = 2:1). The reaction mixture was quenched with water and extracted with ethyl acetate. The organic phase was dried and concentrated to obtain a crude product, which was then subjected to column chromatography (petroleum ether: ethyl acetate = 20:1 to 5:1 to 2:1) to obtain 5-fluorocholest-3β,25-diol **(266)** (15 mg, 0.028 mmol, 32.61%). ¹H NMR (399 MHz, Chloroform-d) δ 3.99 (s, 1H), 1.96 (d, *J =* 12.4 Hz, 1H), 1.83 (d, *J* = 15.1 Hz, 4H), 1.56 (s, 4H), 1.46 - 1.31 (m, 10H), 1.23 (s, 12H), 1.19 (s, 6H), 0.95 (s, 3H), 0.90 (d, *J* = 6.5 Hz, 3H), 0.64 (s, 3H). ¹³C NMR (100 MHz, CDCl₃) δ 100.99, 71.11, 56.09, 55.87, 45.91, 44.38, 42.61, 41.77, 41.56, 39.89, 38.77, 38.58, 36.38, 35.72, 34.54, 32.21, 30.99, 30.54, 29.67, 28.22, 24.07, 21.08, 1.01, 0.97. ¹⁹F NMR (376 MHz, Chloroform-d) δ - 161.61 (d, *J =* 42.2 Hz). LCMS: [M-HF-OH]⁺ = 385.20.

### Example 268

### Preparation of 2-fluoro-N-[(3R)-3-[(1R,3aS,3bR,5aS,7S,9aS,9bS,11aR)-7-hydroxy-9a,11a-dimethylhexadecahydro-1H-cyclopenta[1,2-a]phenanthren-1-yl]butyl]-N-methylbenzamide (compound 268)

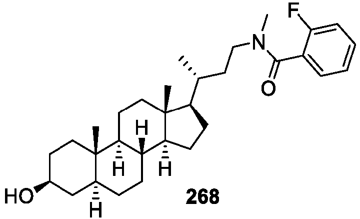

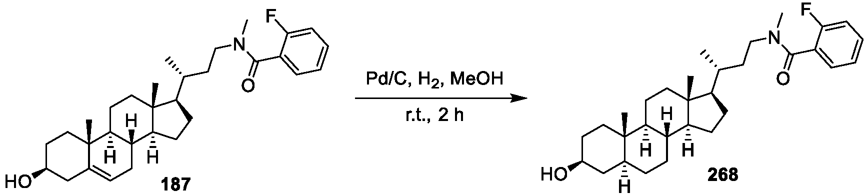

2-Fluoro-*N*-[(3*R*)-3-[(1*R*,3a*S*,3b*S*,7*S*,9a*R*,9b*S*,11a*R*)-7-hydroxy-9a,11a-dimethyl-2,3,3a,3b,4,6,7,8,9,9a,9b,10,11,11a-tetradecahydro-1*H*-cyclopenta[1,2-*a*]phenanthren-1-yl]butyl]-N-methylbenzamide **(187)** (12 mg, 0.025 mmol) was dissolved in methanol (1 mL) in a 50 mL round-bottom flask, then 10% Pd/C (12 mg) was added thereto at room temperature, and the resulting mixture was stirred at room temperature for 3 hours under a hydrogen atmosphere. The reaction was detected by TLC (ethyl acetate/petroleum ether = 2/1) and a new compound was generated. After the reaction was completed, the reaction mixture was diluted with dichloromethane (10 mL), filtered through diatomite, and concentrated to obtain 2-fluoro-*N*-[(3*R*)-3-[(1*R*,3a*S*,3b*R*,5a*S*,7*S*,9a*S*,9b*S*,11a*R*)-7-hydroxy-9a,11a-dimethylhexadecahydro-1*H*-cyclopenta[1,2-*a*]phenanthren-1-yl]butyl]-*N*-methylbenzamide (268) (7.23 mg, purity: 90.57%, yield: 59.59%) as a white solid.

¹H NMR (400 MHz, CDCl₃) δ 7.40 - 7.30 (m, 2H), 7.21 - 7.17 (m, 1H), 7.12 - 7.06 (m, 1H), 3.71 - 3.42 (m, 2H), 3.23 - 3.13 (m, 1H), 3.08 (s, 1.5H), 2.87 (s, 1.5H), 2.02 - 1.33 (m, 19H), 1.19 - 1.07 (m, 4H), 1.05 - 1.01 (m, 3H), 1.00 - 0.86 (m, 4H), 0.79 (d, *J=* 9.2 Hz, 2H), 0.68 (s, 1H), 0.64 (d, *J =* 6.0 Hz, 1H), 0.58 (s, 1H). ¹³C NMR (101 MHz, CDCl₃) δ 166.84, 166.48, 159.34, 159.29, 156.88, 156.82, 132.06, 131.05, 130.97, 130.93, 130.85, 129.93, 129.89, 128.97, 128.93, 128.67, 125.21, 125.03, 124.82, 124.61, 124.58, 124.47, 124.44, 115.92, 115.80, 115.71, 115.59, 71.40, 71.36, 56.49, 56.39, 56.07, 55.70, 54.33, 54.26, 48.61, 44.99, 44.86, 44.83, 42.72, 42.61, 40.03, 39.88, 38.20, 38.17, 37.00, 36.98, 36.16, 36.13, 35.94, 35.52, 35.47, 35.44, 34.11, 33.83, 33.45, 32.80, 32.76, 32.07, 32.02, 31.91, 31.52, 31.48, 29.78, 29.70, 29.62, 29.53, 29.33, 29.23, 28.73, 28.67, 28.32, 28.09, 27.22, 25.53, 24.21, 24.10, 22.69, 21.26, 21.18, 18.77, 18.48, 14.12, 12.33, 12.30, 12.01, 11.93. ¹⁹F NMR (376 MHz, CDCl₃) δ -115.20 (s, 1F). LC-MS: [M+H]⁺ = 484.3.

### Example 271

### Preparation of 24-[(2-fluorophenyl)(hydroxy)methyl]-4β-hydroxy-3β-hydroxy-5α-cholan-7-one (compound 271)

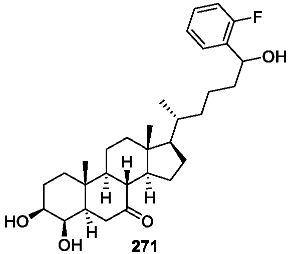

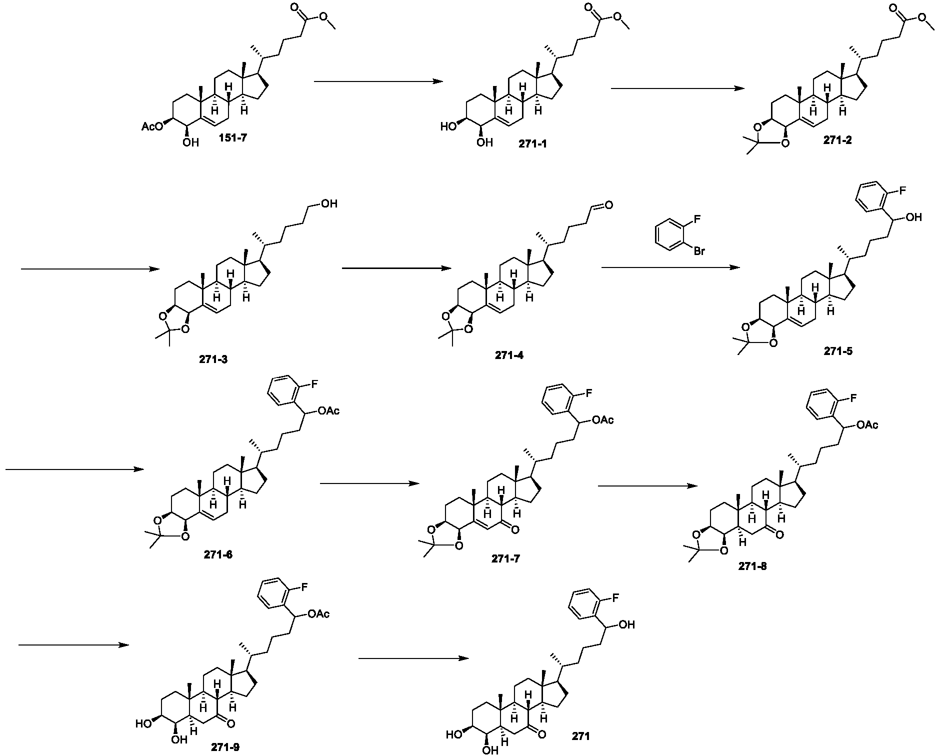

**Step 1:** The reactant methyl (5*R*)-5-[(1*R*,3a*S*,3b*S*,6*R*,7*S*,9a*R*,9b*S*,11a*R*)-7-acetoxy-6-hydroxy-9a, 11a-dimethyl-2,3,3a,3b,4,6,7,8,9,9a,9b,10,11,11a-tetradecahydro-1*H-*cyclopenta[1,2-*a*]phenanthren-1-yl]hexanoate **(151-7)** (1 g, 2.17 mmol, 1.0 *eq)* was dissolved in methanol (50 mL), and potassium carbonate (3.00 g, 21.71 mmol, 10 *eq)* was added thereto. The reaction mixture was reacted for 20 minutes. The reaction was monitored by TLC (petroleum ether: ethyl acetate = 1:1). The reaction system was diluted with water (10 mL) and extracted with ethyl acetate (30 mL * 3). The organic phase was collected, washed with water (30 mL), dried over anhydrous sodium sulfate, and concentrated to obtain a crude product. The crude product was purified by silica gel column chromatography (petroleum ether: ethyl acetate = 73:27) to obtain methyl (5*R*)-5-[(1*R*,3a*S*,3b*S*,6R*,*7*S*,9a*R*,9b*R*,11a*R*)-6,7-dihydroxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate **(271-1)** (760 mg, 1.725 mmol, purity: 95%, yield: 79%) as a white solid.

¹H NMR (400 MHz, CDCl₃) δ 5.71 - 5.63 (m, 1H), 4.14 (d, *J* = 3.2 Hz, 1H), 3.67 (s, 3H), 3.56 (dt, *J =* 11.7, 4.1 Hz, 1H), 2.36 - 2.19 (m, 2H), 2.14 - 1.79 (m, 6H), 1.76 - 1.62 (m, 6H), 1.59 - 1.51 (m, 3H), 1.49 - 1.33 (m, 5H), 1.25 (d, *J* = 7.2 Hz, 1H), 1.18 (s, 3H), 1.08 (ddd, *J* = 17.0, 12.6, 7.8 Hz, 5H), 0.93 (d, *J =* 6.5 Hz, 3H), 0.68 (s, 3H).

**Step 2:** The compound methyl (5*R*)-5-[(1*R*,3a*S*,3b*S*,6*R*,7*S*,9a*R*,9b*S*,11a*R*)-6,7-dihydroxy-9a, 11a-dimethyl-2,3,3a,3b,4,6,7,8,9,9a,9b,10,11,11a-tetradecahydro-1*H-*cyclopenta[1,2-*a*]phenanthren-1-yl]hexanoate **(271-1)** (1.8 g, 4.30 mmol, 1.0 *eq*) was dissolved in acetone (50 mL), then *p*-toluenesulfonic acid (0.57 g, 3.01 mmol, 0.7 *eq)* and 4A molecular sieve were added thereto, and the resulting reaction mixture was stirred at 25°C for 1 hour. The completion of the reaction was monitored by TLC (petroleum ether: ethyl acetate = 5:1). The reaction system was added with water (10 mL), and the aqueous phase was extracted with ethyl acetate (50 mL × 3). The organic phases were combined, washed with saturated brine (50 mL), dried over Na₂SO₄, filtered, and concentrated to obtain a crude product. The crude product was purified by silica gel column chromatography (petroleum ether: ethyl acetate = 90:10) to obtain methyl (5*R*)-5-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]hexanoate **(271-2)** (1.6 g, 3.14 mmol, purity: 90%, yield: 73%) as a white solid. ¹H NMR (400 MHz, CDCl₃) δ 7.33 (d, *J* = 7.3 Hz, 1H), 7.23 (s, 1H), 7.13 (d, *J =* 7.5 Hz, 1H), 7.03 (m, 1H), 6.02 (d, *J =* 5.0 Hz, 1H), 5.68 (d, *J =* 3.4 Hz, 1H), 4.14 (s, 1H), 3.58 (s, 1H), 2.09 (t, *J =* 3.9 Hz, 3H), 2.00 (m, 2H), 1.78 (m, 12H), 1.56 (m, 4H), 1.38 - 1.30 (m, 9H), 1.18 (s, 3H), 1.00 - 0.95 (m, 7H), 0.88 (dd, *J =* 13.7, 7.3 Hz, 4H), 0.66 (d, *J =* 3.4 Hz, 3H).

**Step 3:** The compound methyl (5*R*)-5-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]hexanoate **(271-2)** (1 g, 2.18 mmol, 1.0 *eq*) was dissolved in tetrahydrofuran (50 mL). The reaction system was replaced with nitrogen, then added with lithium aluminum hydride (0.12 g, 3.270 mmol, 1.5 *eq*)*,* and stirred at 25°C for 1 hour. The completion of the reaction was monitored by TLC (petroleum ether: ethyl acetate = 5:1). The reaction mixture was quenched with sodium sulfate decahydrate, filtered, and the filtrate was concentrated to obtain a crude product. The crude product was purified by silica gel column chromatography (petroleum ether: ethyl acetate = 80:20) to obtain (5*R*)-5-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]hexan-1-ol (271-3) (1000 mg, 2.09 mmol, purity: 90%, yield: 96%) as a white solid.

¹H NMR (400 MHz, CDCl₃) δ 5.80 (d, *J* = 2.6 Hz, 1H), 4.41 (d, *J* = 5.8 Hz, 1H), 4.09 (m, 1H), 3.64 (t, *J =* 6.5 Hz, 2H), 2.08 (m, 2H), 1.84 (tt, *J =* 18.5, 7.8 Hz, 2H), 1.67 (m, 8H), 1.41 (td, *J* = 12.7, 6.2 Hz, 4H), 1.35 (s, 3H), 1.24 (m, 2H), 1.17 (s, 4H), 1.08 (m, 5H), 0.93 (d, *J =* 6.5 Hz, 4H), 0.69 (d, *J =* 4.6 Hz, 3H).

**Step 4:** The compound (5*R*)-5-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]hexan-1-ol **(271-3)** (1 g, 2.32 mmol, 1.0 *eq*) was dissolved in dichloromethane (50 mL), then Dess-Martin periodinane (1.18 g, 2.79 mmol, 1.2 *eq*) was added thereto, and the reaction mixture was stirred at 25°C for 1 hour. The completion of the reaction was monitored by TLC (petroleum ether: ethyl acetate = 5:1). The reaction system was quenched with saturated sodium sulfite, added with water (10 mL), and the aqueous phase was extracted with dichloromethane (50 mL × 3). The organic phases were combined, washed with saturated brine (10 mL), dried over anhydrous Na₂SO₄, filtered, and concentrated to obtain a crude product. The crude product was purified by silica gel column chromatography (petroleum ether: ethyl acetate = 90:10) to obtain (5R)-5-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-d][1,3]dioxol-8-yl]hexanal **(271-4)** (750 mg, 1.58 mmol, purity: 90%, yield: 68%) as a white solid.

¹H NMR (400 MHz, CDCl₃) δ 9.76 (s, 1H), 5.80 (d, *J* = 2.7 Hz, 1H), 4.41 (d, *J* = 5.8 Hz, 1H), 4.10 (dd, *J =* 13.6, 6.3 Hz, 1H), 2.39 (m, 2H), 2.12 (m, 1H), 2.01 (m, 1H), 1.83 (m, 1H), 1.66 (m, 10H), 1.53 (s, 4H), 1.42 (dd, *J=* 12.5, 4.2 Hz, 3H), 1.35 (s, 3H), 1.17 (s, 3H), 1.10 (m, 4H), 0.92 (dd, *J* = 18.2, 5.8 Hz, 4H), 0.71 (d, *J* = 12.1 Hz, 3H).

**Step 5:** The compound 1-bromo-2-fluorobenzene (195.96 mg, 1.12 mmol, 1.2 *eq*) was dissolved in tetrahydrofuran (30 mL), and the reaction mixture was cooled to -78°C in a dry ice bath under a nitrogen atmosphere. A 2.5 M solution of n-butyllithium in n-hexane (89.67 mg, 1.400 mmol, 1.5 *eq*) was added thereto, and the reaction mixture was stirred at the same temperature for 30 minutes. (5*R*)-5-[(3a*S*,5a*R*,5b*S*,7a*R*,8*R*,10a*S*,10b*S*,12b*R*)-2,2,5a,7a-Tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]hexanal **(271-4)** (400 mg, 0.933 mmol, 1.0 *eq*) was then added thereto, and the reaction mixture was naturally warmed to room temperature. The reaction was monitored by TLC (petroleum ether: ethyl acetate = 5:1). After the reaction was completed, the reaction mixture was quenched with saturated ammonium chloride, and the aqueous phase was extracted with ethyl acetate (50 mL × 3). The organic phases were combined and washed with saturated brine (15 mL). The ethyl acetate phase was dried over Na₂SO₄, filtered, and concentrated to obtain a crude product. The crude product was purified by silica gel column chromatography (petroleum ether: ethyl acetate = 90:10) to obtain (5*R*)-5-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1-(2-fluorophenyl)hexan-1-ol **(271-5)** (400 mg, 0.610 mmol, purity: 80%, yield: 65%) as a white solid. ¹H NMR (400 MHz, CDCl₃) δ 7.46 (t, *J =* 7.4 Hz, 1H), 7.23 (s, 1H), 7.15 (s, 1H), 7.02 (m, 1H), 5.80 (s, 1H), 5.00 (dd, *J =* 12.0, 7.0 Hz, 1H), 4.41 (d, *J* = 5.8 Hz, 1H), 4.10 (dd, *J* = 13.9, 6.1 Hz, 1H), 2.06 (dd, *J* = 44.2, 10.2 Hz, 3H), 1.65 (dd, *J =* 30.7, 25.0 Hz, 9H), 1.41 (d, *J =* 9.7 Hz, 4H), 1.35 (s, 3H), 1.25 (s, 3H), 1.16 (s, 3H), 1.10 (m, 6H), 0.90 (dd, *J =* 6.4, 4.0 Hz, 3H), 0.68 (m, 3H).

Step 6: The compound (5*R*)-5-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1-(2-fluorophenyl)hexan-1-ol **(271-5)** (400 mg, 0.76 mmol, 1.0 *eq*) was dissolved in dichloromethane (20 mL). Acetic anhydride (0.215 mL, 2.29 mmol, 3.0 *eq*), triethylamine (0.530 mL, 3.81 mmol, 5.0 *eq*)*,* and 4-dimethylaminopyridine (93.09 mg, 0.76 mmol, 1.0 *eq)* were added thereto, and the resulting reaction mixture was stirred at 25°C for 1 hour. The completion of the reaction was monitored by TLC (petroleum ether: ethyl acetate = 5:1). The reaction system was quenched with methanol, added with water (10 mL), and the aqueous phase was extracted with dichloromethane (20 mL × 3). The organic phases were combined, washed with saturated brine (50 mL), dried over Na₂SO₄, filtered, and concentrated to obtain a crude product, which was then purified by silica gel column chromatography (petroleum ether: ethyl acetate = 95:5) to obtain (5*R*)-5-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1-(2-fluorophenyl)hexyl acetate **(271-6)** (400 mg, 0.635 mmol, purity: 90%, yield: 83%) as a white solid. ¹H NMR (400 MHz, CDCl₃) δ 7.34 (t, *J =* 7.4 Hz, 1H), 7.24 (d, *J =* 7.2 Hz, 1H), 7.12 (t, *J =* 7.5 Hz, 1H), 7.03 (m, 1H), 6.01 (m, 1H), 5.80 (d, *J =* 2.8 Hz, 1H), 4.41 (d, *J =* 5.8 Hz, 1H), 4.10 (dd, *J =* 13.6, 6.3 Hz, 1H), 2.08 (d, *J =* 2.4 Hz, 3H), 1.61 (m, 8H), 1.43 (s, 4H), 1.34 (d, *J =* 6.2 Hz, 5H), 1.26 (s, 3H), 1.16 (s, 5H), 1.02 (m, 9H), 0.86 (m, 7H), 0.67 (m, 4H).

**Step 7:** The compound (5*R*)-5-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1-(2-fluorophenyl)hexyl acetate **(271-6)** (120 mg, 0.21 mmol, 1.0 *eq*) was dissolved in acetone (5 mL). *N*-Hydroxyphthalimide (6.92 mg, 0.042 mmol, 0.2 *eq*)*,* tert-butyl hydroperoxide (0.170 mL, 0.848 mmol, 4.0 *eq),* and anhydrous cobalt (II) acetate (1.88 mg, 0.011 mmol, 0.05 *eq*) were added thereto, and the resulting reaction mixture was stirred at 25°C for 18 hours. The completion of the reaction was monitored by TLC (petroleum ether: ethyl acetate = 10:1). The reaction system was quenched with saturated sodium sulfite, added with water (10 mL), and the aqueous phase was extracted with ethyl acetate (15 mL × 3). The ethyl acetate phases were combined, washed with saturated brine (15 mL), dried over Na₂SO₄, filtered, and concentrated to obtain a crude product. The crude product was purified by silica gel column chromatography (petroleum ether: ethyl acetate = 80:20) to obtain (5*R*)-5-[(3a*S*,5a*R*,5b*S*,7a*R*,8*R*,10a*S*,10b*S*,12b*R*)-2,2,5a,7a-tetramethyl-11-oxo-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1-(2-fluorophenyl)hexyl acetate **(271-7)** (60 mg, 0.093 mmol, purity: 90%, yield: 44%). ¹H NMR (400 MHz, CDCl₃) δ 7.34 (t, *J* = 7.3 Hz, 1H), 7.23 (m, 1H), 7.12 (t, *J =* 7.5 Hz, 1H), 7.03 (m, 1H), 6.02 (d, *J* = 5.0 Hz, 1H), 5.92 (s, 1H), 4.52 (d, *J =* 6.4 Hz, 1H), 4.32 (d, *J =* 5.6 Hz, 1H), 2.34 (d, *J =* 10.6 Hz, 2H), 2.08 (s, 3H), 2.02 (m, 1H), 1.82 (dd, *J* = 20.1, 10.2 Hz, 4H), 1.57 (t, *J =* 12.4 Hz, 13H), 1.34 (m, 17H), 1.07 (m, 5H), 0.87 (dd, *J =* 11.7, 6.8 Hz, 4H), 0.80 (d, *J =* 5.9 Hz, 1H), 0.69 (d, *J =* 3.5 Hz, 3H).

**Step 8:** The compound (5*R*)-5-[(3a*S*,5a*R*,5b*S*,7a*R*,8*R*,10a*S*,10b*S*,12b*R*)-2,2,5a,7a-tetramethyl-11-oxo-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1-(2-fluorophenyl)hexyl acetate **(271-7)** (60 mg, 0.10 mmol) was dissolved in methanol (5 mL), and Pd/C (11 mg, 0.103 mmol) was added thereto. The reaction system was replaced with hydrogen three times, and the resulting reaction mixture was stirred at 25°C for 1 hour under a H₂ atmosphere. The completion of the reaction was monitored by TLC (petroleum ether: ethyl acetate = 3:1). The reaction mixture was filtered and concentrated to obtain a crude product. The crude product was purified by silica gel column chromatography (petroleum ether: ethyl acetate = 80:20) to obtain (5*R*)-5-[(3a*S*,5a*R*,5b*S*,7a*R*,8*R*,10a*S*,10b*S*,12a*R*,12b*R*)-2,2,5a,7a-tetramethyl-11-oxo-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]-1-(2-fluorophenyl)hexyl acetate (271-**8)** (40 mg, 0.062 mmol, purity: 90%, yield: 60%) as a white solid. ¹H NMR (400 MHz, CDCl₃) δ 7.34 (s, 1H), 7.23 (s, 1H), 7.12 (s, 1H), 7.03 (s, 1H), 6.01 (dd, *J=* 10.9, 6.4 Hz, 1H), 3.98 (d, *J =* 3.4 Hz, 2H), 2.77 (s, 1H), 2.42 (s, 1H), 2.19 (dd, *J =* 12.7, 2.7 Hz, 2H), 2.08 (s, 3H), 1.74 (m, 9H), 1.53 (s, 3H), 1.38 (dd, *J=* 11.7, 7.8 Hz, 4H), 1.28 (d, *J=* 17.1 Hz, 10H), 0.95 (ddd, *J* = 10.8, 9.5, 6.5 Hz, 10H), 0.64 (d, *J=* 3.2 Hz, 3H).

**Step 9:** The compound (5*R*)-5-[(3a*S*,5a*R*,5b*S*,7a*R*,8*R*,10a*S*,10b*S*,12a*R*,12b*R*)~ 2,2,5a,7a-tetramethyl-11-oxo-4,5,5a,5b,6,7,7a,8,9,10,10a,10b,11,12,12a,12b-hexadecahydro-3a*H*-cyclopenta[1',2':1,2]phenanthro[7,8-*d*][1,3]dioxol-8-yl]-1-(2-fluorophenyl)hexyl acetate **(271-8)** (40 mg, 0.069 mmol) was dissolved in tetrahydrofuran (1 mL), then dilute hydrochloric acid (0.5 mL) was added thereto, and the resulting reaction mixture was stirred at 25°C for 2 hours. The completion of the reaction was monitored by TLC (petroleum ether: ethyl acetate = 1:1). The reaction system was added with water (1 mL), and the aqueous phase was extracted with ethyl acetate (15 mL × 3). The ethyl acetate phases were combined and washed with saturated brine (5 mL). The organic phase was dried over Na₂SO₄, filtered, and concentrated to obtain a crude product. The crude product was purified by silica gel column chromatography (petroleum ether: ethyl acetate = 50:50) to obtain (5*R*)-5-[(1*R*,3a*S*,3b*S*,5a*R*,6*R*,7*S*,9a*R*,9b*S*,11a*R*)-6,7-dihydroxy-9a,11a-dimethyl-4-oxohexadecahydro-1*H*-cyclopenta[1,2-*a*]phenanthren-1-yl]-1-(2-fluorophenyl)hexyl acetate **(271-9)** (25 mg, 0.041 mmol, purity: 90%, yield: 60%) as a white solid. ¹H NMR (400 MHz, CDCl₃) δ 7.34 (s, 1H), 7.23 (s, 1H), 7.13 (d, *J =* 7.5 Hz, 1H), 7.04 (d, *J =* 9.4 Hz, 1H), 6.00 (s, 1H), 3.72 (s, 1H), 3.58 (m, 1H), 2.87 (s, 1H), 2.38 (s, 1H), 2.12 (d, *J =* 2.8 Hz, 2H), 2.08 (s, 4H), 1.94 (s, 1H), 1.80 (m, 6H), 1.70 (m, 2H), 1.42 (m, 8H), 1.26 (m, 7H), 1.04 (dt, *J =* 24.0, 12.1 Hz, 7H), 0.86 (dd, *J =* 6.4, 3.1 Hz, 3H), 0.63 (d, *J =* 3.2 Hz, 3H).

**Step 10:** The compound (5*R*)-5-[(1*R*,3a*S*,3b*S*,5a*R*,6*R*,7*S*,9a*R*,9b*R*,11a*R*)-6,7-dihydroxy-9a,11a-dimethyl-4-oxohexadecahydro-1*H*-cyclopenta[1,2-*a*]phenanthren-1-yl]-1-(2-fluorophenyl)hexyl acetate **(271-9)** (25 mg, 0.05 mmol) was dissolved in a mixed solvent of methanol (5 mL) and tetrahydrofuran (1 mL), then 1 mol of hydroxide solution (0.5 mL) was added thereto, and the resulting mixture was stirred at 25°C for 1 hour. The completion of the reaction was monitored by TLC (petroleum ether: ethyl acetate = 1: 1). The reaction system was diluted with ethyl acetate and washed with saturated brine (10 mL). The organic phase was dried over Na₂SO₄, filtered, and concentrated to obtain a crude product. The crude product was purified by silica gel column chromatography (petroleum ether: ethyl acetate = 1:1) to obtain 24-[(2-fluorophenyl)(hydroxy)methyl]-4β-hydroxy-3β-hydroxy-5α-cholan-7-one **(271)** (18 mg, 0.035 mmol, purity: 96%, yield: 75%) as a white solid. ¹H NMR (400 MHz, CDCl₃) δ 7.45 (t, *J =* 7.5 Hz, 1H), 7.23 (d, *J =* 5.8 Hz, 1H), 7.15 (t, *J =* 7.5 Hz, 1H), 7.02 (m, 1H), 5.00 (dd, *J =* 7.1, 4.5 Hz, 1H), 3.72 (s, 1H), 3.58 (m, 1H), 2.87 (t, *J =* 13.5 Hz, 1H), 2.38 (t, *J =* 11.3 Hz, 1H), 2.19 (dd, *J =* 6.7, 3.0 Hz, 1H), 2.11 (dd, *J =* 12.7, 2.8 Hz, 1H), 1.97 (d, *J* = 13.1 Hz, 1H), 1.73 (m, 4H), 1.41 (m, 8H), 1.28 (s, 3H), 1.23 (d, *J* = 16.5 Hz, 2H), 0.95 (m, 10H), 0.63 (d, *J =* 2.0 Hz, 3H). ¹³C NMR (101 MHz, CDCl₃) δ 215.07, 162.83, 128.71, 127.79, 127.20, 124.30, 122.12, 79.37, 73.82, 71.97, 58.86, 55.78, 54.96, 50.06, 48.98, 43.80, 42.56, 38.63, 36.10, 35.91, 35.49, 28.41, 26.70, 25.62, 25.01, 22.21, 21.12, 18.69, 14.19, 12.06, 11.54. LCMS [M+H-H₂O]⁺ = 483.

### Example 272

### Preparation of 5α-cholest-3β,4β,6α,25-tetraol (compound 272)

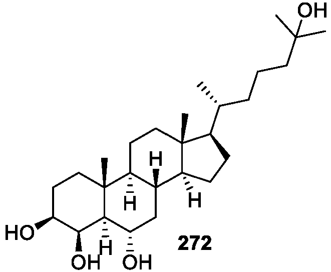

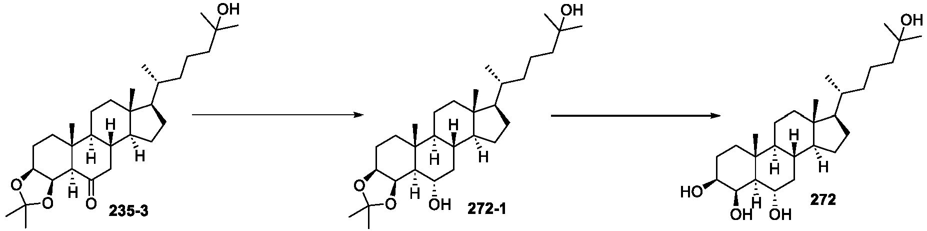

**Step 1:** (3a*S*,5a*R*,5b*S*,7a*R*,8*R*,10a*S*,10b*S*,12a*R*,12b*R*)-8-[(2*R*)-6-Hydroxy-6-methylheptan-2-yl]-2,2,5a,7a-tetramethyl-4,5,5a,5b,6,7,7a,8,9,10,10a,10b,11,12,12a,12b-hexadecahydro-3a*H*-cyclopenta[1',2':1,2]phenanthro[7,8-*d*][1,3]dioxol-12-one **(235-3)** (25 mg, 0.053 mmol) was dissolved in tetrahydrofuran (3 mL) and methanol (3 mL), then sodium borohydride (5.98 mg, 0.158 mmol) was added thereto, and the reaction mixture was stirred at room temperature for 2 hours. The completion of the reaction was monitored by TLC (petroleum ether: ethyl acetate = 3:1). The reaction mixture was diluted with water and ethyl acetate, concentrated, and subjected to column chromatography (petroleum ether: ethyl acetate = 0 to 80%) to obtain compound (3a*S*,5a*R*,5b*S*,7a*R*,8*R*,10a*S*,10b*S*,12a*S*,12b*R*,12*S*)-8-[(2*R*)-6-hydroxy-6-methylheptan-2-yl]-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-12-ol **(272-1)** (15 mg, 0.028 mmol, 53.77%) as a white solid. ¹H NMR (399 MHz, Chloroform-d) δ 4.21 (d, *J =* 5.1 Hz, 1H), 4.16 (s, 1H), 3.82 (dd, *J =* 10.2, 5.8 Hz, 1H), 2.03 - 1.93 (m, 1H), 1.74 (s, 3H), 1.47 (s, 3H), 1.44 (s, 1H), 1.42 - 1.32 (m, 8H), 1.31 (s, 3H), 1.23 (s, 3H), 1.19 (s, 6H), 1.13 (s, 3H), 1.08 (t, *J =* 9.1 Hz, 3H), 0.90 (d, *J =* 6.3 Hz, 3H), 0.84 (d, *J =* 9.9 Hz, 1H), 0.66 (s, 3H).

**Step 2:** (3a*S*,5a*R*,5b*S*,7a*R*,8*R*,10a*S*,10b*S*,12a*S*,12b*R*,12*S*)-8-[(2*R*)-6-Hydroxy-6-methylheptan-2-yl]-2,2,5a,7a-tetramethyl-4,5,5a,5b,6,7,7a,8,9,10,10a,10b,11,12,12a,12b-hexadecahydro-3a*H*-cyclopenta[1',2':1,2]phenanthro[7,8-*d*][1,3]dioxol-12-ol **(272-1)** (10 mg, 0.021 mmol) was dissolved in methanol (5 mL), then hydrochloric acid (0.210 mL, 0.210 mmol) was added thereto, and the reaction mixture was stirred at room temperature for 1 hour. The completion of the reaction was monitored by TLC (petroleum ether: ethyl acetate = 1:1). The reaction mixture was extracted twice with ethyl acetate, dried over anhydrous sodium sulfate, concentrated, and subjected to column chromatography (petroleum ether: ethyl acetate = 100:1 to 1:4) to obtain compound 5α-cholest-3β,4β,6α,25-tetraol (3 mg, 0.006 mmol, 27.03%). ¹H NMR (399 MHz, Chloroform-d) δ 4.39 (s, 1H), 3.95 (s, 1H), 3.56 (d, *J* = 11.3 Hz, 1H), 2.06 - 1.92 (m, 3H), 1.79 (d*, J =* 11.8 Hz, 2H), 1.65 (s, 5H), 1.45 - 1.26 (m, 10H), 1.19 (s, 6H), 1.14 (s, 3H), 1.08 (dd, *J* = 19.0, 9.6 Hz, 6H), 0.90 (d, *J* = 6.3 Hz, 3H), 0.66 (s, 3H). ¹³C NMR (100 MHz, CDCl₃) δ, 69.92, 69.32, 68.05, 44.36, 42.66, 41.93, 40.01, 36.27, 35.70, 34.47, 30.16, 29.36, 28.28, 25.92, 25.77, 20.83, 18.63, 12.06, 0.99. LCMS: [M-OH]⁺ = 419.45.

### Example 273

### Preparation of [(1R,3aS,3bS,5aR,6E,7S,9aR,9bS,11aR)-7-hydroxy-1-[(2R)-6-hydroxy-6-methylheptan-2-yl]-9a,11a-dimethylhexadecahydro-1H-cyclopenta[1,2-a]phenanthren-6-ylidene]hydroxylamine (compound 273)

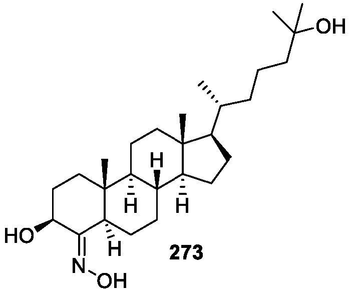

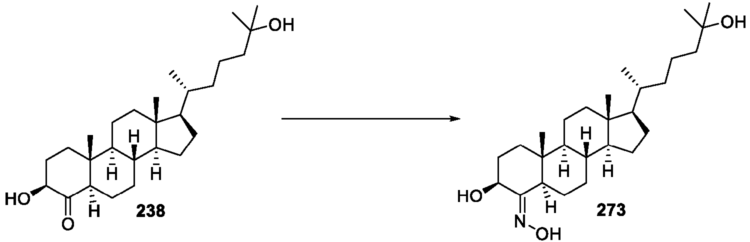

3β,25-Dihydroxy-5α-cholest-4-one **(238)** (30 mg, 0.07 mmol) was dissolved in ethanol (5 mL), then hydroxylamine hydrochloride (9.94 mg, 0.14 mmol) and sodium acetate (23.5 mg, 0.29 mmol) were added thereto, and the reaction system was stirred at 90°C overnight. The reaction was monitored by TLC (petroleum ether: ethyl acetate = 3:1). The reaction mixture was concentrated to obtain a crude product, which was then purified by column chromatography (petroleum ether: ethyl acetate = 0 to 30%) to obtain [(1*R*,3a*S*,3b*S*,5a*R*,6*E*,7*S*,9a*R*,9b*S*,11a*R*)-7-hydroxy-1-[(2*R*)-6-hydroxy-6-methylheptan-2-yl]-9a,11a-dimethylhexadecahydro-1*H*-cyclopenta[1,2-*a*]phenanthren-6-ylidene]hydroxylamine (8 mg, 0.018 mmol, 25.7%) as a white solid with a purity of 83.65%. ¹H NMR (400 MHz, DMSO-d₆) *δ* 11.41 (s, 1H), 6.28 (s, 1H), 4.33 (dd, *J* = 10.5, 5.8 Hz, 1H), 4.01 (s, 1H), 1.93 (dd, *J =* 26.3, 9.9 Hz, 2H), 1.84 - 1.73 (m, 3H), 1.65 - 1.56 (m, 3H), 1.47 (s, 4H), 1.29 (d, *J =* 10.8 Hz, 4H), 1.20 (s, 3H), 1.15 (s, 3H), 1.08 - 1.03 (m, 2H), 1.01 (s, 6H), 0.95 (s, 2H), 0.85 (d,*J* = 6.4 Hz, 3H), 0.75 (d, *J =* 12.5 Hz, 2H), 0.69 (s, 3H), 0.59 (s, 3H). ¹³C NMR (101 MHz, DMSO-d₆) δ 158.63, 74.11, 69.20, 56.18, 56.16, 53.85, 49.87, 44.54, 42.54, 36.58, 35.99, 35.65, 35.14, 32.13, 31.33, 29.84, 29.65, 28.24, 24.18, 23.01, 21.66, 20.69, 18.94, 13.80, 12.30. LCMS: [M+H]⁺ = 434.45.

### Example 274

### Synthesis of (1R,3aS,3bS,5aR,6R,7S,9aR,9bS,11aR)-4,4-difluoro-1-[(2R)-6-hydroxy-6-methylheptan-2-yl]-9a,11a-dimethylhexadecahydro-1H-cyclopenta[1,2-a]phenanthrene-6,7-diol (compound 274)

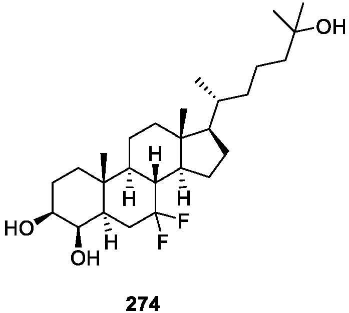

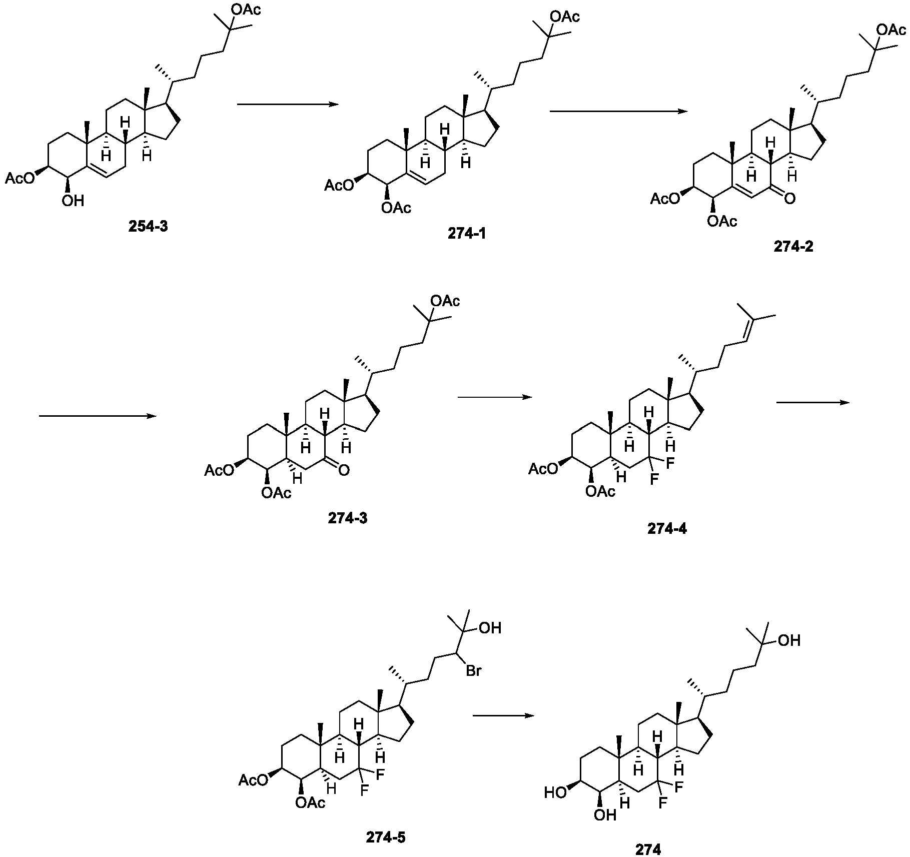

**Step 1:** (1*R*,3a*S*,3b*S*,6*R*,7*S*,9a*R*,9b*S*,11a*R*)-1-[(2*R*)-6-Acetoxy-6-methylheptan-2-yl]-6-hydroxy-9a,11a-dimethyl-2,3,3a,3b,4,6,7,8,9,9a,9b,10,11,11a-tetradecahydro-1*H-*cyclopenta[1,2-i]phenanthren-7-yl acetate **(254-3)** (480 mg, 0.96 mmol) was dissolved in dichloromethane (10 mL). Triethylamine (266 mg, 2.63 mmol), 4-dimethylaminopyridine (10.7 mg, 0.09 mmol), and acetic anhydride (179 mg, 1.75 mmol) were added thereto, and the reaction mixture was stirred at room temperature for 2 hours. The reaction was monitored by TLC (petroleum ether: ethyl acetate = 5:1). The reaction mixture was quenched with water, extracted three times with dichloromethane, washed once with brine, dried over anhydrous sodium sulfate, and concentrated to obtain a crude product, which was then purified by column chromatography (petroleum ether: ethyl acetate = 0% to 10%) to obtain (1*R*,3a*S*,3b*S*,6*R*,7*S*,9a*R*,9b*S*,11a*R*)-6-acetoxy-1-[(2*R*)-6-acetoxy-6-methylheptan-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate **(274-1)** (400 mg, 0.73 mmol, 76.9%) as a white solid. ¹H NMR (399 MHz, Chloroform-d) δ 5.79 (d, *J =* 4.1 Hz, 1H), 5.48 (d, *J =* 3.3 Hz, 1H), 4.72 (dt, *J =* 12.4, 4.1 Hz, 1H), 2.04 (d, *J =* 0.9 Hz, 3H), 2.04 - 2.00 (m, 2H), 1.99 (d, *J =* 0.9 Hz, 3H), 1.95 (d, *J =* 0.9 Hz, 3H), 1.92 - 1.76 (m, 3H), 1.68 (d, *J =* 4.5 Hz, 2H), 1.62 (d, *J =* 12.4 Hz, 2H), 1.52 (s, 2H), 1.50 - 1.42 (m, 3H), 1.40 (s, 6H), 1.35 (s, 3H), 1.24 (d, *J =* 12.1 Hz, 2H), 1.15 (dd, *J =* 11.3, 6.0 Hz, 2H), 1.11 (s, 3H), 1.06 (dd, *J =* 11.4, 6.7 Hz, 2H), 0.99 (d, *J =* 6.9 Hz, 2H), 0.90 (d, *J =* 6.4 Hz, 3H), 0.65 (s, 3H).

**Step 2:** (1*R*,3a*S*,3b*S*,6*R*,7*S*,9a*R*,9b*S*,11a*R*)-6-Acetoxy-1-[(2*R*)-6-acetoxy-6-methylheptan-2-yl]-9a,11a-dimethyl-2,3,3a,3b,4,6,7,8,9,9a,9b,10,11,11a-tetradecahydro-1*H-*cyclopenta[1,2-*i*]phenanthren-7-yl acetate **(274-1)** (350 mg, 0.64 mmol) was dissolved in acetone. Cobalt acetate (1.46 mg, 0.008 mmol), *N*-hydroxyphthalimide (13.5 mg, 0.08 mmol), and *tert*-butyl hydroperoxide (297 mg, 3.3 mmol) were added thereto, and the reaction mixture was stirred at room temperature. The reaction was monitored by TLC (petroleum ether: ethyl acetate = 5:1). After the reaction was completed, the reaction mixture was diluted with water, extracted three times with ethyl acetate, washed once with brine, dried over anhydrous sodium sulfate, and concentrated to obtain a crude product, which was then purified by column chromatography (petroleum ether: ethyl acetate = 0% to 20%) to obtain (1*R*,3a*S*,3b*S*,6*R*,7*S*,9a*R*,9b*S*,11a*R*)-6-acetoxy-1-[(2*R*)-6-acetoxy-6-methylheptan-2-yl]-9a,11a-dimethyl-4-oxo-2,3,3a,3b,4,6,7,8,9,9a,9b,10,11,11a-tetradecahydro-1*H-*cyclopenta[1,2-i]phenanthren-7-yl acetate **(274-2)** (170 mg, 0.3 mmol, 47.4%) as a white solid. ¹H NMR (399 MHz, Chloroform-d) δ 5.90 (s, 1H), 5.59 (d, *J =* 3.2 Hz, 1H), 4.78 (dt, *J =* 12.4, 4.0 Hz, 1H), 2.30 (dt, *J* = 21.6, 10.1 Hz, 2H), 2.08 (s, 3H), 2.03 (s, 1H), 2.01 (s, 3H), 1.97 (t,*J* = 3.6 Hz, 1H), 1.95 (s, 3H), 1.82 (dd, *J* = 30.6, 10.4 Hz, 3H), 1.73 - 1.60 (m, 2H), 1.40 (s, 6H), 1.35 (t, *J =* 8.7 Hz, 4H), 1.30 (s, 3H), 1.24 (d, *J =* 7.0 Hz, 3H), 1.16 (dt, *J =* 17.2, 7.5 Hz, 3H), 1.05 (dt, *J =* 18.9, 8.9 Hz, 3H), 0.91 (d, *J =* 6.4 Hz, 3H), 0.66 (s, 3H).

**Step 3:** (1*R*,3a*S*,3b*S*,6*R*,7*S*,9a*R*,9b*S*,11a*R*)-6-Acetoxy-1-[(2*R*)-6-acetoxy-6-methylheptan-2-yl]-9a,11a-dimethyl-4-oxo-2,3,3a,3b,4,6,7,8,9,9a,9b,10,11,11a-tetradecahydro-1*H*-cyclopenta[1,2-*i*]phenanthren-7-yl acetate **(274-2)** (190 mg, 0.34 mmol) was dissolved in anhydrous methanol (6 mL), and Pd/C (10% w/w) was added thereto. The reaction system was replaced with hydrogen and stirred at room temperature for 0.5 hours. The completion of the reaction was monitored by TLC (petroleum ether: ethyl acetate = 5:1). The reaction mixture was concentrated to obtain the crude product (1*R*,3a*S*,3b*S*,5a*R*,6*R*,7*S*,9a*R*,9b*S*,11a*R*)-6-acetoxy-1-[(2*R*)-6-acetoxy-6-methylheptan-2-yl]-9a,11a-dimethyl-4-oxohexadecahydro-1*H*-cyclopenta[1,2-*i*]phenanthren-7-yl acetate **(274-3)** (120 mg, 0.21 mmol, 62.9%) as a white solid. ¹H NMR (399 MHz, Chloroform-d) δ 5.15 (s, 1H), 4.75 (dd, *J =* 11.9, 3.8 Hz, 1H), 2.49 (t, *J =* 13.5 Hz, 1H), 2.30 (t, *J =* 11.3 Hz, 1H), 2.18 (d, *J =* 10.4 Hz, 1H), 2.09 (s, 3H), 2.08 - 2.03 (m, 1H), 1.99 (s, 1H), 1.95 (d, *J =* 4.3 Hz, 6H), 1.91 - 1.80 (m, 3H), 1.76 - 1.62 (m, 4H), 1.49 (d, *J =* 3.3 Hz, 2H), 1.39 (s, 6H), 1.34 (d, *J =* 7.8 Hz, 4H), 1.26 (s, 3H), 1.23 - 1.14 (m, 2H), 1.12 - 1.04 (m, 4H), 1.03 - 0.91 (m, 2H), 0.89 (d, *J =* 6.4 Hz, 3H), 0.62 (s, 3H).

**Step 4:** (1*R*,3a*S*,3b*S*,6*R*,7*S*,9a*R*,9b*S*,11a*R*)-1-[(2*R*)-6-Acetoxy-6-methylheptan-2-yl]-6-hydroxy-9a,11a-dimethyl-2,3,3a,3b,4,6,7,8,9,9a,9b,10,11,11a-tetradecahydro-1*H-*cyclopenta[1,2-*i*]phenanthren-7-yl acetate **(274-3)** (110 mg, 0.2 mmol) was dissolved in bis(2-methoxyethyl)aminosulfur trifluoride (5 mL). The reaction mixture was heated to 80°C and stirred for 2 hours. The reaction was monitored by TLC (petroleum ether: ethyl acetate = 5:1). After the reaction was completed, the reaction mixture was poured into water to quench. The organic phase was separated, dried, concentrated, and purified by column chromatography (petroleum ether: ethyl acetate = 0% to 20%) to obtain (1*R*,3a*S*,3b*S*,5a*R*,6*R*,7*S*,9a*R*,9b*S*,11a*R*)-6-acetoxy-4,4-difluoro-9a,11a-dimethyl-1-[(2*R*)-6-methylhept-5-en-2-yl]hexadecahydro-1*H-*cyclopenta[1,2-i]phenanthren-7-yl acetate **(274-4)** (50 mg, 0.096 mmol, 48.8%) as a white solid. ¹H NMR (400 MHz, DMSO-d₆) δ 5.08 (d, *J =* 44.2 Hz, 2H), 4.73 (d, *J* = 12.3 Hz, 1H), 2.03 (d, *J =* 8.1 Hz, 3H), 1.92 (s, 2H), 1.86 (d, *J =* 1.1 Hz, 3H), 1.80 (d, *J =* 16.9 Hz, 2H), 1.74 (d, *J* = 11.1 Hz, 3H), 1.65 (d, *J* = 14.8 Hz, 4H), 1.60 (s, 3H), 1.52 (s, 3H), 1.42 (d, *J* = 12.2 Hz, 1H), 1.29 (d, *J =* 13.5 Hz, 4H), 1.26 - 1.13 (m, 4H), 1.12 - 0.99 (m, 3H), 0.97 (s, 3H), 0.93 (s, 1H), 0.87 (d, *J =* 5.8 Hz, 3H), 0.57 (d, *J =* 20.1 Hz, 3H).

**Step 5:** (1*R*,3a*S*,3b*S*,5a*R*,6*R*,7*S*,9a*R*,9b*S*,11a*R*)-6-Acetoxy-4,4-difluoro-9a,11a-dimethyl-1-[(2*R*)-6-methylhept-5-en-2-yl]hexadecahydro-1*H*-cyclopenta[1,2-*i*]phenanthren-7-yl acetate **(274-4)** (50 mg, 0.1 mmol) was dissolved in a mixed solvent of tetrahydrofuran (3 mL) and water (0.75 mL), then *N*-bromosuccinimide (15.2 mg, 0.085 mmol) was added thereto, and the reaction mixture was stirred at room temperature for 1 hour. The reaction was monitored by TLC (petroleum ether: ethyl acetate = 5:1). After the reaction was completed, the reaction mixture was extracted with water and ethyl acetate. The organic phase was separated, dried, and concentrated to obtain a crude product, which was then purified by column chromatography (petroleum ether: ethyl acetate = 0% to 20%) to obtain (1*R*,3a*S*,3b*S*,5a*R*,6*R*,7*S*,9a*R*,9b*S*,11a*R*)-6-acetoxy-1-[(2*R*)-5-bromo-6-hydroxy-6-methylheptan-2-yl]-4,4-difluoro-9a,11a-dimethylhexadecahydro-1*H*-cyclopenta[1,2-*i*]phenanthren-7-yl acetate **(274-5)** (30 mg, 0.049 mmol, 50.7%) as a white solid. ¹H NMR (400 MHz, DMSO-d₆) δ 5.09 (d, *J =* 44.0 Hz, 1H), 4.78 - 4.60 (m, 2H), 3.78 (dd, *J =* 21.3, 10.9 Hz, 1H), 2.04 (d, *J* = 8.1 Hz, 3H), 1.99 - 1.88 (m, 2H), 1.86 (s, 3H), 1.84 - 1.72 (m, 4H), 1.65 (d, *J* = 10.7 Hz, 4H), 1.41 (s, 2H), 1.32 (s, 5H), 1.22 (s, 3H), 1.16 (s, 3H), 1.09 (d, *J=* 18.8 Hz, 4H), 0.97 (s, 3H), 0.88 (d, *J=* 6.5 Hz, 3H), 0.58 (dd, *J* = 20.2, 3.7 Hz, 3H).

**Step 6:** (1*R*,3a*S*,3b*S*,5a*R*,6*R*,7*S*,9a*R*,9b*S*,11a*R*)-6-Acetoxy-1-[(2*R*)-5-bromo-6-hydroxy-6-methylheptan-2-yl]-4,4-difluoro-9a,11a-dimethylhexadecahydro-1*H-*cyclopenta[1,2-*i*]phenanthren-7-yl acetate **(274-5)** (30 mg, 0.05 mmol) was dissolved in tetrahydrofuran (3 mL). The reaction mixture was cooled to 0°C under a nitrogen atmosphere, added with lithium aluminum hydride (31 mg, 0.8 mmol), heated to 70°C, and stirred for 1 hour. The reaction was monitored by TLC (petroleum ether: ethyl acetate = 3:1). After the reaction was completed, the reaction mixture was cooled to room temperature, quenched with water, and extracted three times with ethyl acetate. The organic phase was washed once with brine, dried over anhydrous sodium sulfate, concentrated, and purified by column chromatography (petroleum ether: ethyl acetate = 0 to 30%) to obtain (1*R*,3a*S*,3b*S*,5a*R*,6*R*,7*S*,9a*R*,9b*S*,11a*R*)-4,4-difluoro-1-[(2*R*)-6-hydroxy-6-methylheptan-2-yl]-9a,11a-dimethylhexadecahydro-1*H*-cyclopenta[1,2-*a*]phenanthrene-6,7-diol **(274)** (9.2 mg, 0.02 mmol, 41.6%) as a white solid with a purity of 73.48%. ¹H NMR (400 MHz, DMSO-d₆) δ 4.37 (d, *J =* 6.0 Hz, 1H), 4.21 (d, *J =* 3.4 Hz, 1H), 4.01 (s, 1H), 3.50 (d, *J =* 41.3 Hz, 1H), 3.23 (s, 1H), 2.15 - 1.85 (m, 3H), 1.74 (s, 2H), 1.69 - 1.50 (m, 5H), 1.39 (d, *J* = 11.6 Hz, 3H), 1.29 (d, *J =* 9.6 Hz, 4H), 1.23 (s, 2H), 1.20 (s, 2H), 1.15 (d, *J =* 8.8 Hz, 2H), 1.06 (s, 2H), 1.01 (s, 6H), 0.95 (s, 3H), 0.92 (s, 1H), 0.86 (d, *J =* 6.4 Hz, 3H), 0.57 (d, *J =* 19.5 Hz, 3H). ¹³C NMR (101 MHz, DMSO-d₆) δ 72.94, 71.50, 69.19, 55.15, 51.01, 48.73, 44.54, 43.08, 36.61, 35.60, 35.06, 29.84, 29.66, 28.42, 25.93, 20.67, 20.48, 19.02, 14.10, 12.06. ¹⁹F NMR (376 MHz, DMSO-d₆) δ -86.70, -87.32, -108.70, -109.32. LCMS: [M-OH⁻]⁺ = 439.4.

### Example 275

### Preparation of 24-[(2,4,6-difluorophenyl)(hydroxy)methyl]-5α-cholan-3β,4β-diol (compound 275)

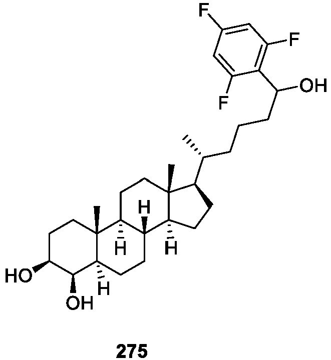

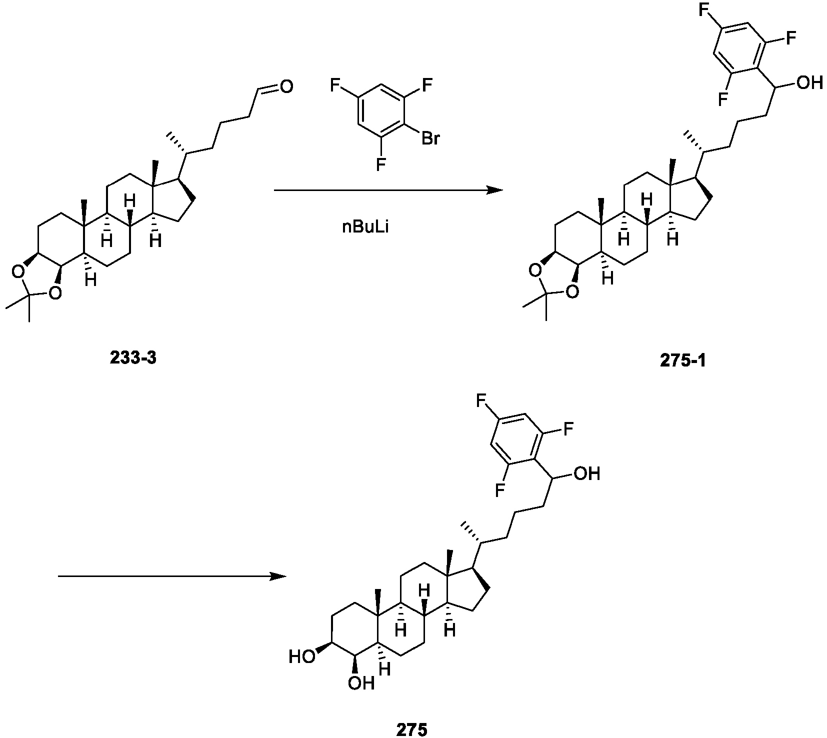

Step 1: The compound (5*R*)-5-[(3a*S*,5a*R*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]hexanal **(233-3)** (50 mg, 0.116 mmol, 1.0 *eq*) was dissolved in tetrahydrofuran (5 mL), and the reaction system was cooled to 0°C. (2,4,6-Trifluorophenyl)lithium (prepared from 2,4,6-trifluorobromobenzene and n-butyllithium) (2.7 mL, 0.580 mmol, 5 *eq*) was then slowly added thereto, and the reaction system was stirred at room temperature for 1 hour. The completion of the reaction was monitored by TLC (petroleum ether: ethyl acetate = 5:1). The reaction system was added with water (20 mL) and extracted with ethyl acetate (50 mL × 3). The organic phases were combined, washed with water (30 mL), dried, and concentrated to obtain a crude product. The crude product was subjected to silica gel column chromatography (petroleum ether: ethyl acetate = 94:6) to obtain (5*R*)-5-[(3a*S*,5a*R*,5b*S*,7a*R*,8*R*,10a*s*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]-1-(2,4,6-trifluorophenyl)hexan-1-ol **(275-1)** (30 mg, purity: 90%, yield: 41.36%) as a white solid. ¹H NMR (400 MHz, CDCl₃) δ 6.65 (t, *J =* 8.6 Hz, 2H), 4.97 (t, *J =* 7.3 Hz, 1H), 3.98 (d, *J =* 15.0 Hz, 2H), 1.94 (d, *J =* 13.4 Hz, 2H), 1.77 (s, 6H), 1.71 - 1.54 (m, 12H), 1.51 (s, 3H), 1.37 (dd, *J =* 25.0, 12.7 Hz, 7H), 1.30 (s, 3H), 1.28 - 1.17 (m, 6H), 1.04 (s, 3H), 1.00 - 0.92 (m, 2H), 0.87 (d, *J=* 6.3 Hz, 3H), 0.64 (t, *J =* 4.0 Hz, 3H), 0.59 (s, 1H).

**Step 2:** The reactant (5*R*)-5-[(3a*S*,5a*R*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]-1-(2,4,6-trifluorophenyl)hexan-1-ol **(275-1)** (30 mg, 0.053 mmol, 1.0 *eq*) was dissolved in tetrahydrofuran (5 mL), then dilute hydrochloric acid (0.5 mL, 3 mol/L) was added thereto, and the reaction system was stirred at room temperature for 40 minutes. The completion of the reaction was monitored by TLC (petroleum ether: ethyl acetate = 2:1). The reaction system was added with water (10 mL), and the aqueous phase was extracted with ethyl acetate (25 mL × 3). The ethyl acetate phases were combined and washed with saturated brine (10 mL × 3). The ethyl acetate phase was dried over anhydrous sodium sulfate, filtered, and concentrated to obtain a crude product. The crude product was purified by column chromatography (petroleum ether: ethyl acetate = 73:27) to obtain 24-[(2,4,6-difluorophenyl)(hydroxy)methyl]-5α-cholan-3β,4β-diol **(275)** (11.07 mg, purity: 92.28%, yield: 36.67%) as a white solid. ¹H NMR (400 MHz, CDCl₃) δ 7.10 (t, *J =* 9.0 Hz, 2H), 5.39 - 5.33 (m, 1H), 4.81 (d, *J =* 4.6 Hz, 1H), 4.28 (d, *J =* 6.0 Hz, 1H), 3.88 (d, *J* = 2.9 Hz, 1H), 3.47 (s, 1H), 3.27 (dd, *J =* 5.9, 4.0 Hz, 1H), 1.88 (d, *J =* 10.9 Hz, 1H), 1.75 - 1.45 (m, 10H), 1.42 - 1.25 (m, 8H), 1.17 (d, *J =* 12.4 Hz, 3H), 1.05 - 0.97 (m, 4H), 0.94 (s, 3H), 0.81 (d, *J* = 7.0 Hz, 3H), 0.75 (s, 1H), 0.59 (s, 3H), 0.54 (s, 1H). ¹³C NMR (101 MHz, CDCl₃) δ 169.92, 168.42, 158.42, 104.02, 99.81, 99.56, 73.84, 72.24, 71.29, 65.37, 65.24, 55.53, 54.21, 47.80, 41.59, 38.87, 36.65, 35.85, 34.46, 34.37, 31.36, 30.91, 28.68, 27.19, 24.98, 24.85, 23.17, 21.58, 19.56, 13.66, 11.03. LCMS: [M+H-2H₂O]⁺ = 488.

### Examples 276 and 277

### Preparation of 3β,5,25-trihydroxycholest-4-one (compound 276)

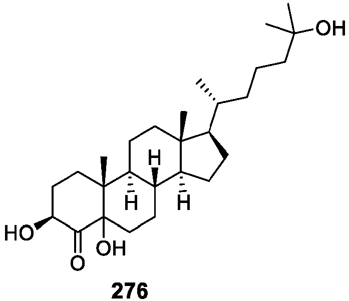

### Preparation of 3β,25-dihydroxycholest-6(5)-en-4-one (compound 277)

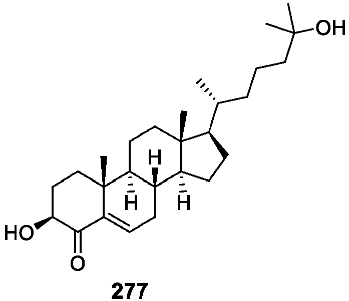

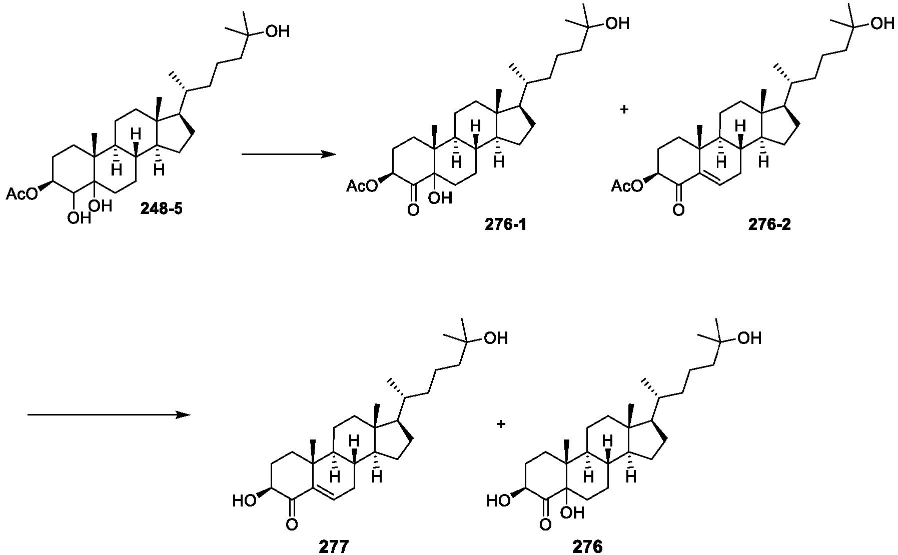

**Step 1:** (1*R*,3a*S*,3b*S*,7*S*,9a*R*,9b*S*,11a*R*)-5a,6-Dihydroxy-1-[(2*R*)-6-hydroxy-6-methylheptan-2-yl]-9a,11a-dimethylhexadecahydro-1*H*-cyclopenta[1,2-*i*]phenanthren-7-yl acetate **(248-5)** (42 mg, 0.088 mmol) was dissolved in dichloromethane (1.5 mL) in a 50 mL round-bottom flask. *N*-Methylmorpholine oxide (15 mg, 0.13 mmol), 4A molecular sieve (21 mg), and tetrapropylammonium perruthenate (6 mg, 0.018 mmol) were added thereto at room temperature, and the resulting mixture was stirred at room temperature for 3 hours. The reaction was monitored by TLC (petroleum ether: ethyl acetate = 3:1). After the reaction was completed, the reaction mixture was added with saturated sodium sulfite aqueous solution (15 mL) and extracted with dichloromethane (10 mL × 3). The extracted organic solution was washed with saturated brine (10 mL), dried over anhydrous sodium sulfate, filtered, and concentrated to obtain a crude product. The crude product was purified by silica gel column chromatography (eluent: petroleum ether/ethyl acetate = 3/1) to obtain a mixture of (1*R*,3a*S*,3b*S*,7*S*,9a*R*,9b*S*,11a*R*)-5a-hydroxy-1-[(2*R*)-6-hydroxy-6-methylheptan-2-yl]-9a,11a-dimethyl-6-oxohexadecahydro-1*H*-cyclopenta[1,2-*i*]phenanthren-7-yl acetate **(276-1)** and (1*R*,3a*S*,3b*S*,7*S*,9a*R*,9b*S*,11a*R*)-1-[(2*R*)-6-hydroxy-6-methylheptan-2-yl]-9a,11a-dimethyl-6-oxo-2,3,3a,3b,4,6,7,8,9,9a,9b,10,11,11a-tetradecahydro-1*H*-cyclopenta[1,2-*i*]phenanthren-7-yl acetate **(276-2)** (35 mg, purity: 90%, yield: 76.90%) as a white solid.

LC-MS **(276-1):** [M+Na]⁺ = 499.4.

LC-MS **(276-2):** [M+Na]⁺ = 481.4.

**Step 2:** The mixture of (1*R*,3a*S*,3b*S*,7*S*,9a*R*,9b*S*,11a*R*)-5a-hydroxy-1-[(2*R*)-6-hydroxy-6-methylheptan-2-yl]-9a,11a-dimethyl-6-oxohexadecahydro-1*H*-cyclopenta[1,2-i]phenanthren-7-yl acetate **(276-1)** and (1*R*,3a*S*,3b*S*,7*S*,9a*R*,9b*S*,11a*R*)-1-[(2*R*)-6-hydroxy-6-methylheptan-2-yl]-9a,11a-dimethyl-6-oxo-2,3,3a,3b,4,6,7,8,9,9a,9b,10,11,11a-tetradecahydro-1*H*-cyclopenta[1,2-*i*]phenanthren-7-yl acetate **(276-2)** (35 mg, 0.074 mmol) was dissolved in tetrahydrofuran (1 mL) and methanol (1 mL) in a 50 mL round-bottom flask. Potassium carbonate (51 mg, 0.37 mmol) and water (0.5 mL) were added thereto at room temperature, and the reaction mixture was stirred at room temperature for 3 hours. The reaction was monitored by TLC (petroleum ether: ethyl acetate = 2:1). After the reaction was completed, the reaction mixture was diluted with water (15 mL) and extracted with ethyl acetate (10 mL × 3). The extracted organic solution was washed with saturated brine (10 mL), dried over anhydrous sodium sulfate, filtered, and concentrated to obtain a crude product. The crude product was purified by silica gel column chromatography (petroleum ether: ethyl acetate = 2:1) to obtain 3β,5,25-trihydroxycholest-4-one **(276)** (11.05 mg, purity: 93.95%, yield: 54.38%) and 3β,25-dihydroxycholest-6(5)-en-4-one **(277)** (6.25 mg, purity: 100%, yield: 48.98%) as white solids.

**276:** ¹H NMR (400 MHz, DMSO) δ 4.21 (d, *J =* 6.1 Hz, 1H), 4.12 - 4.11 (m, 2H), 4.03 (s, 1H), 2.17 - 2.12 (m, 1H), 2.02 - 1.93 (m, 2H), 1.85 - 1.69 (m, 4H), 1.60 - 1.41 (m, 6H), 1.37 - 1.23 (m, 14H), 1.15 (s, 3H), 1.05 (s, 6H), 0.89 (d, *J =* 6.4 Hz, 3H), 0.65 (s, 3H). ¹³C NMR (101 MHz, DMSO) 211.02, 80.19, 77.97, 69.24, 56.28, 45.52, 44.58, 42.62, 36.65, 36.22, 35.74, 34.53, 33.17, 29.88, 29.70, 28.51, 28.36, 26.27, 24.31, 21.09, 20.81, 18.99, 15.50, 12.35. LC-MS-MS [M+H]⁺ = 435.

**277:** ¹H NMR (400 MHz, DMSO) δ 7.71 (s, 1H), 4.03 (s, 1H), 3.18 (s, 0.5H), 3.02 (s, 0.5H), 2.93 - 2.85 (m, 1H), 2.33 - 2.26 (m, 1H), 2.12 - 1.69 (m, 9H), 160 - 1.27 (m, 15H), 1.12 (s, 3H), 1.05 (s, 6H), 0.90 (d, *J* = 6.4 Hz, 3H), 0.68 (s, 3H). ¹³C NMR (101 MHz, DMSO) δ 193.37, 141.85, 139.73, 69.23, 56.09, 56.00, 54.31, 44.60, 42.39, 37.73, 36.62, 35.66, 35.14, 34.78, 32.79, 31.33, 29.88, 29.70, 28.26, 24.27, 22.79, 21.02, 20.69, 18.99, 17.26, 12.25. LC-MS [M+Na]⁺ = 439.3.

### Example 278

### Preparation of 24-[1-(2-fluorophenyl)-1-hydroxyethyl]-5α-cholan-3β,4β-diol (compound 278)

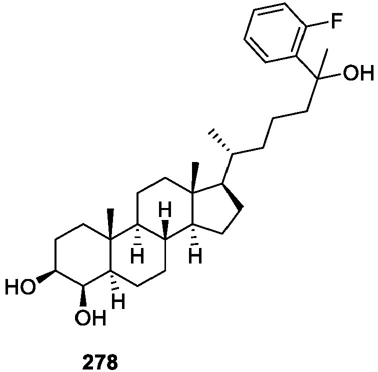

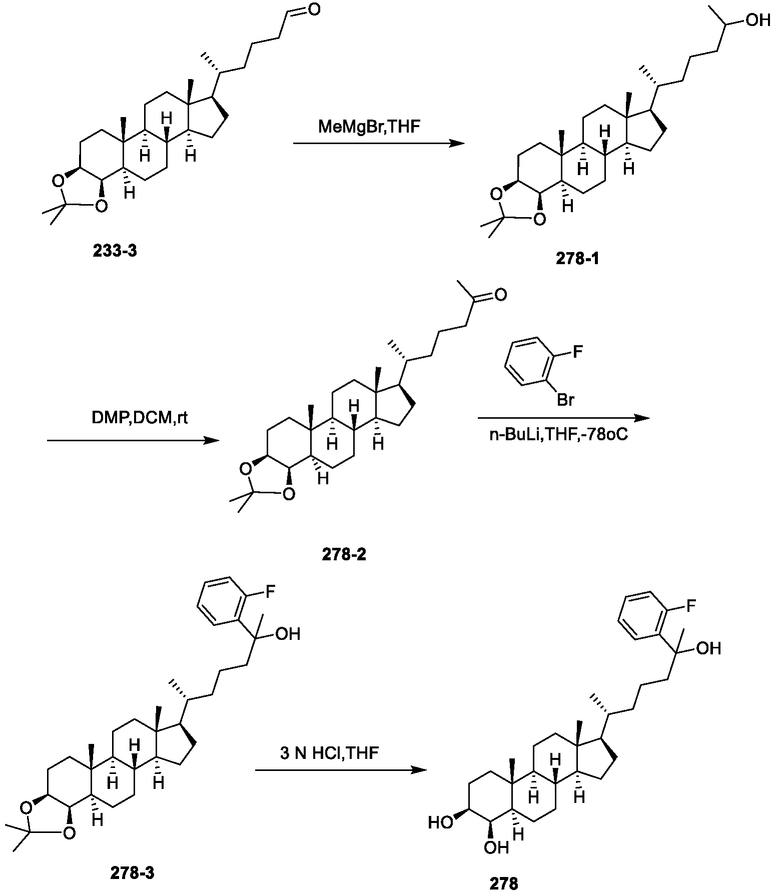

**Step 1:** The reagent (5*R*)-5-[(3a*S*,5a*R*,5b*S*,7a*R*,8*R*,10a*S*,10b*S*,12a*R*,12b*R*)-2,2,5a,7a - tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]hexanal **(233-3)** (30 mg, 0.07 mmol) was dissolved in tetrahydrofuran (1 mL), then methylmagnesium chloride solution (0.279 mL) was added thereto, and the reaction mixture was reacted for about 1 hour. The reaction was monitored by TLC (petroleum ether: ethyl acetate = 5:1) and the starting material basically disappeared. The reaction mixture was quenched with saturated ammonium chloride and extracted with ethyl acetate. The organic phase was dried over anhydrous sodium sulfate, and the crude product was purified by flash column chromatography (petroleum ether: ethyl acetate = 90:10) to obtain (6*R*)-6-[(3a*S*,5a*R*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]heptan-2-ol **(278-1)** (30 mg, 0.054 mmol, purity: 80%, yield: 77.13%) as a white solid. ¹H NMR (400 MHz, CDCl₃) *δ* 5.23 (s, 1H), 3.99 (m, 2H), 1.94 (s, 1H), 1.78 (dd, *J =* 11.0, 7.9 Hz, 2H), 1.51 (s, 2H), 1.41 (m, 5H), 1.30 (s, 2H), 1.24 (m, 3H), 1.19 (d, *J =* 6.2 Hz, 2H), 1.04 (s, 2H), 0.91 (d, *J* = 6.4 Hz, 2H), 0.65 (d, *J =* 4.8 Hz, 3H).

***Step 2*:** The reagent (6*R*)-6-[(3a*S*,5a*R*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]heptan-2-ol **(278-1)** (30 mg, 0.067 mmol, 1.0 *eq*) was dissolved in dichloromethane (2 mL), then Dess-Martin periodinane (34.18 mg, 0.081 mmol, 1.2 *eq*) was added thereto, and the reaction mixture was reacted for about 2 hours. The reaction was monitored by TLC (petroleum ether: ethyl acetate = 5:1) and the starting material basically disappeared. The reaction mixture was quenched with saturated sodium sulfite and washed with saturated sodium bicarbonate. The organic phase was dried over anhydrous sodium sulfate, and the crude product was purified by flash column chromatography (petroleum ether: ethyl acetate = 80:20) to obtain (6*R*)-6-[(3a*S*,5a*R*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]heptan-2-one **(278-2)** (20 mg, 0.036 mmol, purity: 80%, yield: 53.58%) as a white solid. ¹H NMR (400 MHz, CDCl₃) *δ* 7.46 (t, *J* = 7.6 Hz, 1H), 7.22 (d, *J* = 8.0 Hz, 1H), 7.15 (t, *J =* 7.5 Hz, 1H), 7.02 (m, 1H), 5.31 (d, *J =* 5.3 Hz, 1H), 4.95 (m, 1H), 3.48 (m, 1H), 2.22 (dt, *J* = 13.2, 11.8 Hz, 2H), 1.96 (d, *J =* 14.1 Hz, 2H), 1.75 (m, 5H), 1.37 (d, *J =* 26.9 Hz, 3H), 1.14 (m, 9H), 0.99 (s, 5H), 0.90 (m, 13H), 0.66 (d, *J =* 2.1 Hz, 3H), 0.05 (s, 6H).

***Step 3:*** The reagent 1-bromo-2-fluorobenzene (15.75 mg, 0.090 mmol) was dissolved in tetrahydrofuran (0.5 mL), and the reaction mixture was cooled to -78°C in a dry ice bath under a nitrogen atmosphere. A2.5 M solution of n-butyllithium in *n*-hexane (6.34 mg, 0.099 mmol) was then added thereto, and the reaction mixture was reacted for about 0.5 hours. A solution of (6*R*)-6-[(3a*S*,5a*R*,5b*S*,7a*R*,8*R*,10a*R*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]heptan-2-one **(278-2)** (20 mg, 0.045 mmol, 1.0 *eq*) in tetrahydrofuran (0.5 mL) was then added thereto. The reaction was monitored by TLC (petroleum ether: ethyl acetate = 5:1) and the starting material basically disappeared. The reaction mixture was quenched with saturated ammonium chloride and extracted with ethyl acetate. The organic phase was dried over anhydrous sodium sulfate, and the crude product was purified by flash column chromatography (petroleum ether: ethyl acetate = 80:20) to obtain (6*R*)-6-[(3a*S*,5a*R*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]-2-(2-fluorophenyl)heptan-2-ol **(278-3)** (20 mg, 0.03 mmol, purity: 80%, yield: 65.79%) as a white solid. ¹H NMR (400 MHz, CDCl₃) δ 7.54 (m, 1H), 7.24 (s, 1H), 7.12 (s, 1H), 7.00 (dd, *J =* 13.4, 7.1 Hz, 1H), 4.00 (s, 2H), 1.92 (d, *J =* 12.2 Hz, 2H), 1.75 (d, *J =* 12.7 Hz, 4H), 1.67 (dd, *J =* 13.6, 3.0 Hz, 2H), 1.61 (s, 4H), 1.51 (s, 4H), 1.45 (d, *J =* 15.1 Hz, 6H), 1.30 (s, 4H), 1.21 (m, 6H), 1.03 (d, *J =* 3.8 Hz, 4H), 0.95 - 0.80 (m, 10H), 0.64 (m, 3H).

***Step 4:*** The reagent (6*R*)-6-[(3a*S*,5a*R*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]-2-(2-fluorophenyl)heptan-2-ol **(278-3)** (20 mg, 0.037 mmol) was dissolved in tetrahydrofuran (3 mL), then dilute hydrochloric acid (0.012 mL, 0.037 mmol) was added thereto, and the reaction mixture was reacted for about 1 hour. The reaction was monitored by TLC (petroleum ether: ethyl acetate = 3:1) and the starting material basically disappeared. The reaction mixture was quenched with saturated sodium bicarbonate and extracted with ethyl acetate. The organic phase was dried over anhydrous sodium sulfate, and the crude product was purified by flash column chromatography (petroleum ether: ethyl acetate = 70:30) to obtain 24-[1-(2-fluorophenyl)-1-hydroxyethyl]-5α-cholan-3β,4β-diol **(278)** (10.12 mg, 0.017 mmol, yield: 46.33%) as a white solid. ¹H NMR (400 MHz, CDCl₃) δ 7.53 (d, *J =* 7.1 Hz, 1H), 7.22 (m, 1H), 7.12 (t, *J =* 7.2 Hz, 1H), 7.00 (dd, *J =* 12.4, 8.0 Hz, 1H), 3.73 (s, 1H), 3.55 (m, 1H), 1.89 (dd, *J =* 27.5, 9.2 Hz, 3H), 1.64 (d, *J =* 5.5 Hz, 2H), 1.61 (s, 3H), 1.55 (m, 2H), 1.32 (m, 9H), 1.17 (m, 2H), 0.98 (m, 13H), 0.82 (m, 3H), 0.60 (m, 4H). ¹³C NMR (101 MHz, CDCl₃) δ 187.61, 132.72, 127.25, 124.75, 123.95, 115.81, 77.22, 74.86, 72.31, 48.83, 42.59, 39.88, 36.87, 35.48, 35.39, 32.39, 28.21, 26.00, 25.86, 24.19, 20.57, 18.48, 14.67, 12.06. LCMS (ESI) [M+H-3H₂O]⁺ = 447.

### Examples 279 and 280

### Synthesis of 24-[R-(2-fluorophenyl)(hydroxy)methyl]-5α-cholan-3β,4β-diol or 24-[S-(2-fluorophenyl)(hydroxy)methyl]-5α-cholan-3β,4β-diol (compound 279 or 280)

Both compound 279 and compound 280 are single configurational isomers, each being one of the following isomers:

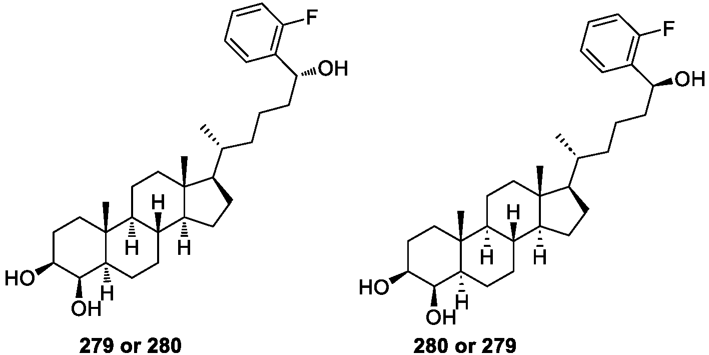

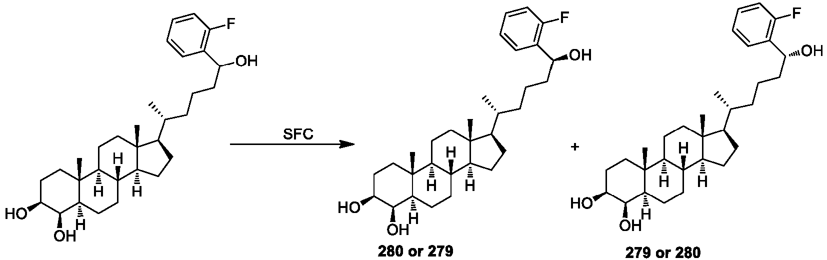

The compound 24-[(2-fluorophenyl)(hydroxy)methyl]-5α-cholan-3β,4β-diol **(234)** (20 mg, 0.041 mmol) was subjected to chiral resolution (instrument: Waters Acquity UPCC; column: Daicel CHIRALPAK OJ_3, 3 * 150 mm, 3 µm; mobile phase: A/B: CO₂/MeOH (0.1% DEA) = 85/15; flow rate: 2.0 mL/min; column temperature: 37°C) to obtain two relative configurations: compound **280** (retention time: tR = 4.837 min, 5.24 mg, 0.010 mmol, yield: 24.16%) and compound **279** (retention time: tR = 5.388 min, 4.00 mg, 0.008 mmol, yield: 19.64%).

280: ¹H NMR (400 MHz, ) δ 7.45 (s, 1H), 7.22 (m, 1H), 7.16 (d, *J* = 7.6 Hz, 1H), 7.02 (m, 1H), 5.00 (d, *J =* 6.5 Hz, 1H), 3.74 (s, 1H), 3.57 (s, 1H), 1.94 (d, *J =* 11.9 Hz, 1H), 1.72 (m, 10H), 1.54 (m, 4H), 1.38 (m, 6H), 1.24 (dd, *J =* 21.6, 11.4 Hz, 4H), 1.06 (d, *J =* 8.4 Hz, 4H), 1.01 (s, 3H), 0.88 (d, *J =* 6.1 Hz, 3H), 0.63 (s, 3H). ¹⁹F NMR (377 MHz, CDCl₃) δ - 119.78 (d, *J* = 10.2 Hz, 1H). LC-MS: [M+H-3H₂O]⁺ = 451.6.

**279:** ¹H NMR (400 MHz) δ 7.46 (t, *J =* 7.1 Hz, 1H), 7.23 (s, 1H), 7.01 (m, 1H), 5.00 (m, 1H), 3.74 (s, 1H), 3.55 (d, *J =* 11.2 Hz, 1H), 1.95 (d, *J =* 12.2 Hz, 1H), 1.75 (d, *J =* 15.6 Hz, 7H), 1.66 (s, 5H), 1.39 (t, *J =* 11.6 Hz, 7H), 1.25 (t, *J =* 14.7 Hz, 4H), 1.05 (d, *J =* 9.9 Hz, 4H), 1.01 (s, 3H), 0.88 (d, *J =* 6.1 Hz, 3H), 0.64 (s, 3H). ¹⁹F NMR (377 MHz, CDCl₃) δ - 119.78 (d, *J* = 10.2 Hz, 1H). LC-MS: [M+H-3H₂O]⁺ = 451.6.

### Example 281

### Preparation of 24-[(3-fluorophenyl)(hydroxy)methyl]-5α-cholan-3β,4β-diol (compound 281)

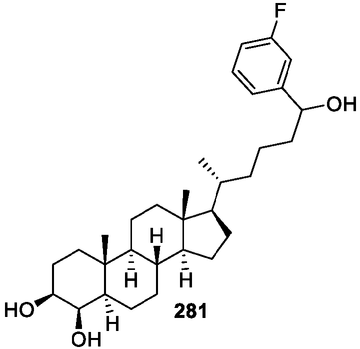

Referring to example **275,** 2,4,6-trifluorobromobenzene was replaced with *m-*fluorobromobenzene to obtain compound 24-[(3-fluorophenyl)(hydroxy)methyl]-5α-cholan-3β,4β-diol **(281).** ¹H NMR (400 MHz, DMSO) δ 7.37 - 7.28 (m, 1H), 7.11 (t, *J =* 9.2 Hz, 2H), 7.02 (t, *J =* 8.6 Hz, 1H), 5.21 (d, *J =* 3.3 Hz, 1H), 4.51 (s, 1H), 4.29 (d, *J =* 5.9 Hz, 1H), 3.89 (d, *J =* 3.0 Hz, 1H), 3.47 (d, *J =* 2.1 Hz, 1H), 3.28 (dd, *J =* 7.7, 3.7 Hz, 1H), 1.89 (d, *J =* 12.6 Hz, 1H), 1.75 - 1.46 (m, 8H), 1.41 - 1.13 (m, 12H), 1.09 - 0.95 (m, 5H), 0.94 (s, 3H), 0.89 (s, 2H), 0.83 (dd, *J* = 6.4, 2.8 Hz, 3H), 0.59 (t, *J* = 3.8 Hz, 3H), 0.58 - 0.51 (m, 1H). ¹³C NMR (101 MHz, DMSO) δ 177.61, 140.43, 130.09, 127.31, 126.70, 126.09, 92.16, 73.21, 71.13, 70.84, 55.51, 55.07, 54.08, 47.83, 41.52, 36.41, 34.58, 34.49, 34.47, 31.44, 27.79, 27.17, 25.35, 25.11, 23.21, 19.91, 19.60, 17.85, 14.58, 14.09, 11.26. LC-MS: [M+H-2H₂O]⁺ = 452.

### Example 282

### Preparation of 24-[(3,5-difluorophenyl)(hydroxy)methyl]-5α-cholan-3β,4β-diol (compound 282)

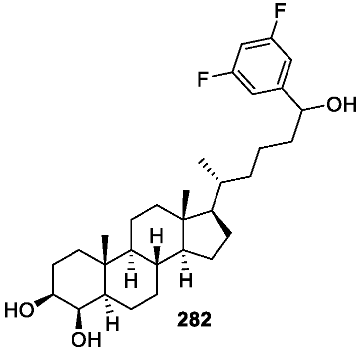

Referring to example **275,** 2,4,6-trifluorobromobenzene was replaced with 3,5-difluorobromobenzene to obtain compound 24-[(3,5-difluorophenyl)(hydroxy)methyl]-5α-cholan-3β,4β-diol **(282).** ¹H NMR (400 MHz, CDCl₃) δ 7.24 - 7.18 (m, 1H), 6.87 (t, *J =* 7.7 Hz, 2H), 5.02 (d, *J* = 6.1 Hz, 1H), 3.74 (s, 1H), 3.55 (d, *J =* 10.8 Hz, 1H), 1.94 (d, *J =* 12.3 Hz, 3H), 1.73 (s, 5H), 1.70 (s, 3H), 1.40 (d, *J =* 17.9 Hz, 4H), 1.31 (d, *J =* 19.6 Hz, 5H), 1.26 (s, 3H), 1.05 (d, *J =* 10.9 Hz, 5H), 1.01 (s, 3H), 0.87 (d, *J =* 5.1 Hz, 3H), 0.63 (s, 3H), 0.59 (s, 1H). ¹³C NMR (101 MHz, CDCl₃) δ 171.25, 165.79, 140.88, 111.85, 111.59, 99.10, 74.85, 73.85, 72.31, 56.56, 56.15, 55.24, 48.84, 47.81, 42.09, 39.90, 36.87, 35.69, 35.49, 35.40, 32.39, 30.20, 29.71, 26.01, 25.87, 24.21, 20.59, 18.51, 14.67, 12.06, 9.04, 7.99.

### Example 284

### Preparation of 24-[(2,6-difluorophenyl)(hydroxy)methyl]-5α-cholan-3β,4β-diol (compound 284)

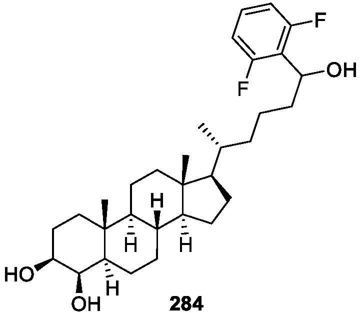

Referring to example **275,** 2,4,6-trifluorobromobenzene was replaced with 2,6-difluorobromobenzene to obtain compound 24-[(2,6-difluorophenyl)(hydroxy)methyl]-5α-cholan-3β,4β-diol **(284).** ¹H NMR (400 MHz, DMSO) δ 7.38 - 7.28 (m, 1H), 7.02 (t, *J* = 8.5 Hz, 2H), 5.34 (t, *J =* 3.9 Hz, 1H), 4.86 (d, *J =* 4.8 Hz, 1H), 4.30 (d, *J =* 5.9 Hz, 1H), 3.91 (d, *J* = 2.8 Hz, 1H), 3.47 (d, *J=* 1.5 Hz, 1H), 3.29 - 3.24 (m, 1H), 1.87 (t, *J =* 11.2 Hz, 2H), 1.71 (d, *J =* 4.4 Hz, 2H), 1.69 - 1.47 (m, 6H), 1.43 - 1.11 (m, 12H), 1.02 (dd, *J =* 19.7, 10.2 Hz, 4H), 0.94 (s, 3H), 0.89 (s, 1H), 0.84 - 0.79 (m, 3H), 0.59 (s, 3H), 0.52 (d, *J* = 10.4 Hz, 1H). ¹³C NMR (101 MHz, DMSO) δ 174.24, 114.01, 112.37, 111.16, 76.13, 75.51, 74.28, 56.11, 55.82, 48.89, 42.60, 41.02, 36.58, 35.54, 35.35, 26.19, 21.37, 20.62, 18.77, 15.22, 15.17, 12.30, 9.42, 8.54, 5.15, 4.82, 4.45, 0.58. LC-MS: [M+H-2H₂O]⁺ = 469.

### Example 285

### Preparation of [(1R,3aS,3bR,4E,5aS,7S,9aS,9bS,11aR)-7-hydroxy-1-[(2R)-6-hydroxy-6-methylheptan-2-yl]-9a,11a-dimethylhexadecahydro-1H-cyclopenta11.2-a]phenanthren-4-ylidene]hydroxylamine (compound 285)

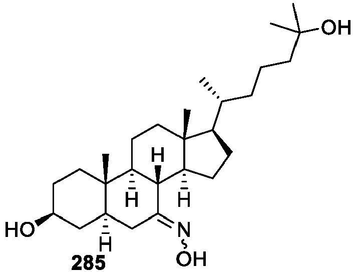

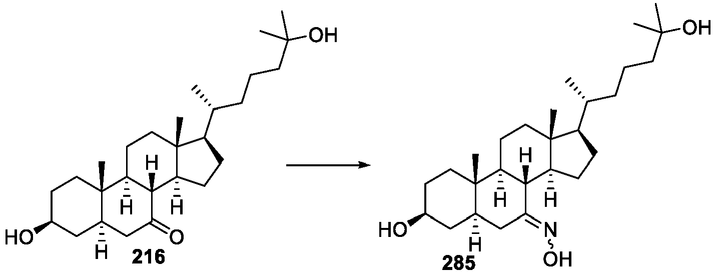

3β,25-Dihydroxy-5α-cholest-7-one (45 mg, 0.107 mmol) was dissolved in ethanol (10 mL), then sodium acetate (35.27 mg, 0.430 mmol) and hydroxylamine hydrochloride (14.94 mg, 0.215 mmol) were added thereto, and the reaction mixture was stirred at 90°C for 2 hours. The reaction was monitored by TLC (dichloromethane: methanol = 20:1). The reaction mixture was concentrated to obtain a crude product (10 mg), which was then purified by column chromatography (dichloromethane: methanol = 10:1) to obtain [(1*R*,3a*S*,3b*R*,4*E*,5a*S*,7*S*,9a*S*,9b*S*,11a*R*)-7-hydroxy-1-[(2*R*)-6-hydroxy-6-methylheptan-2-yl]-9a,11a-dimethylhexadecahydro-1*H*-cyclopenta[1,2-*a*]phenanthren-4-ylidene]hydroxylamine (30 mg, 0.066 mmol, 61.14%) as a white solid with a purity of 100%.

¹H NMR (400 MHz, DMSO-d₆) δ 10.08 (d, *J* = 4.7 Hz, 1H), 4.48 (d, *J =* 4.8 Hz, 1H), 4.01 (s, 1H), 3.37 (s, 1H), 2.88 (dd, *J =* 12.7, 3.1 Hz, 1H), 2.09 (dd, *J =* 24.2, 13.3 Hz, 2H), 1.97 - 1.82 (m, 1H), 1.69 (d, *J* = 9.1 Hz, 1H), 1.56 (d, *J* = 14.1 Hz, 2H), 1.49 - 1.38 (m, 3H), 1.32 (d, *J* = 17.6 Hz, 4H), 1.25 (d, *J* = 15.5 Hz, 3H), 1.22 - 1.12 (m, 4H), 1.06 (d, *J* = 12.7 Hz, 3H), 1.01 (s, 6H), 0.98 - 0.89 (m, 2H), 0.88 - 0.84 (m, 6H), 0.76 (d, *J* = 6.7 Hz, 2H), 0.60 (s, 3H). ¹³C NMR (101 MHz, DMSO) δ 159.97, 69.50, 69.20, 55.38, 55.29, 49.83, 45.31, 44.54, 42.61, 42.56, 41.89, 38.41, 36.66, 36.42, 36.23, 35.68, 34.97, 31.51, 29.82, 29.74, 29.72, 29.66, 28.35, 28.05, 25.68, 21.33, 20.83, 19.13, 12.65, 12.42, 11.95.

LC-MS: [M+H]⁺ = 434.0.

### Example 286

### Preparation of 24-{hydroxy[2-(trifluoromethyl)phenyl]methyl}-5α-cholan-3β,4β-diol (compound 286)

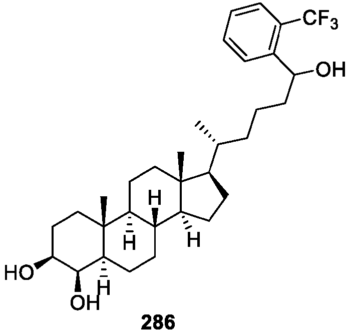

Referring to example **275,** 2,4,6-trifluorobromobenzene was replaced with *o-*trifluoromethylbromobenzene to obtain compound 24-{hydroxy [2-(trifluoromethyl)phenyl]methyl}-5α-cholan-3β,4β-diol **(286).** ¹H NMR (400 MHz) δ 7.77 (d, *J =* 7.9 Hz, 1H), 7.73 - 7.58 (m, 2H), 7.46 - 7.36 (m, 1H), 5.41 (m, 1H), 4.81 (s, 1H), 4.30 (d, *J =* 5.9 Hz, 1H), 3.90 (d, *J =* 2.8 Hz,1H), 3.47 (s, 1H), 3.29 (d, *J =* 4.0 Hz, 1H), 1.89 (d, *J =* 10.7 Hz, 1H), 1.68 (m, 3H), 1.53 (m, 4H), 1.29 (m, 15H), 1.03 (dd, *J =* 22.0, 12.4 Hz, 3H), 0.94 (s, 5H), 0.85 (t, *J* = 5.4 Hz, 3H), 0.60 (d, *J* = 3.3 Hz, 3H). ¹³C NMR (101 MHz, DMSO) δ 133.03, 128.43, 128.38, 127.57, 126.37, 125.41, 125.33, 74.28, 71.93, 68.23, 56.57, 56.19, 55.15, 48.90, 42.60, 37.47, 35.64, 35.55, 35.40, 32.46, 31.61, 30.28, 28.23, 28.18, 26.43, 26.20, 24.28, 22.55, 20.63, 18.81, 15.17, 12.33.

LC-MS: [M+H-3H₂O]⁺ = 483.

### Example 287

### Preparation of 24-[hydroxy(2-methoxyphenyl)methyl]-5α-cholan-3β,4β-diol (compound 287)

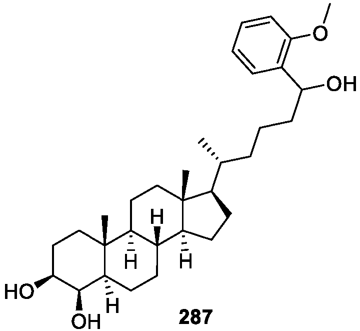

Referring to example **275,** 2,4,6-trifluorobromobenzene was replaced with 2-methoxybromobenzene to obtain compound 24-[hydroxy(2-methoxyphenyl)methyl]-5α-cholan-3β,4β-diol **(287).** ¹H NMR (400 MHz, DMSO) δ 7.38 (d, *J =* 7.8 Hz, 1H), 7.17 (t, *J* = 7.6 Hz, 1H), 6.94 - 6.88 (m, 2H), 4.84 (d, *J =* 9.9 Hz, 2H), 4.30 (d, *J =* 5.9 Hz, 1H), 3.91 (d, *J =* 2.8 Hz, 1H), 3.75 (s, 3H), 3.47 (s, 1H), 3.29 (d, *J =* 4.5 Hz, 1H), 1.90 (d, *J* = 11.7 Hz, 1H), 1.78 - 1.48 (m, 7H), 1.44 - 1.12 (m, 16H), 1.02 (dd, *J =* 18.3, 8.9 Hz, 3H), 0.94 (s, 3H), 0.84 (d, *J* = 6.4 Hz, 3H), 0.60 (d, *J* = 2.2 Hz, 3H), 0.54 (t, *J* = 11.4 Hz, 1H). ¹³C NMR (101 MHz, DMSO) δ 155.81, 135.07, 127.81, 126.52, 120.61, 110.78, 74.28, 72.96, 71.93, 56.59, 56.24, 55.71, 55.15, 48.90, 42.61, 42.60, 37.47, 35.71, 35.55, 35.47, 32.52, 28.27, 26.44, 26.19, 24.29, 22.39, 22.14, 20.64, 18.93, 18.84, 15.17, 12.36. LC-MS: [M+H-2H₂O]⁺ = 464.

### Example 288

### Preparation of (1R,3aS,5aR,7S,9aS,11aR)-4,4-difluoro-1-[(2R)-5-(hydroxycyclopropyl)pentan-2-yl]-9a,11a-dimethylhexadecahydro-1H-cyclopenta[1,2-a]phenanthrene-7-ol (compound 288)

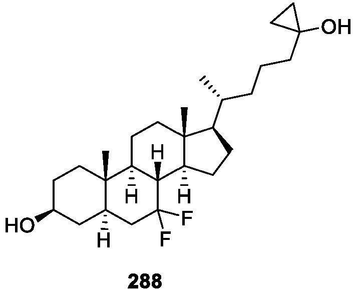

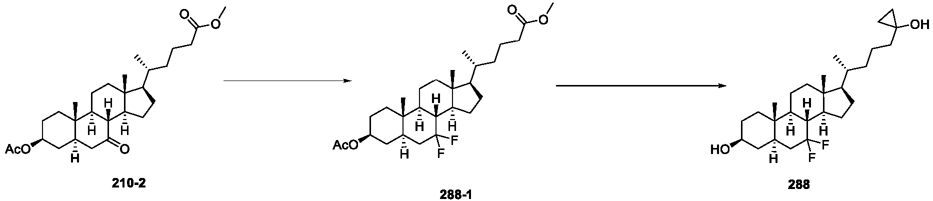

**Step 1:** Methyl (5*R*)-5-[(1*R*,3a*S,*3b*R*,5a*R*,7*S*,9a*S*,9b*S*,11a*R*)-7-acetoxy-9a,11a-dimethyl-4-oxohexadecahydro-1*H*-cyclopenta[1,2-*a*]phenanthren-1-yl]hexanoate **(210-2)** (50 mg, 0.109 mmol) was dissolved in bis(2-methoxyethyl)aminosulfur trifluoride (5 mL). The reaction mixture was heated to 90°C and reacted for 1 hour. The reaction was monitored by TLC (petroleum ether: ethyl acetate = 5:1). After the reaction was completed, the reaction mixture was poured into ice water, extracted with ethyl acetate, dried over anhydrous sodium sulfate, concentrated, and subjected to column chromatography (petroleum ether: ethyl acetate = 100% to 20%) to obtain methyl (5*R*)-5-[(1*R*,3a*S*,3b*R*,5a*R*,7*S*,9a*S*,9b*S*,11a*R*)-7-acetoxy-4,4-difluoro-9a,11a-dimethylhexadecahydro-1*H*-cyclopenta[1,2-*a*]phenanthren-1-yl]hexanoate **(288-1)** (25 mg, 0.047 mmol, 42.95%) as a white solid. ¹H NMR (399 MHz, Chloroform-d) δ 4.71 - 4.62 (m, 1H), 3.65 (s, 3H), 2.25 (h, *J* = 8.4 Hz, 2H), 2.01 (d, *J =* 2.3 Hz, 3H), 1.99 - 1.59 (m, 10H), 1.49 - 1.27 (m, 7H), 1.24 (s, 2H), 1.06 (dd, *J* = 27.7, 13.2 Hz, 4H), 0.91 (d,*J* = 6.4 Hz, 3H), 0.83 (d, *J =* 10.5 Hz, 3H), 0.72 - 0.58 (m, 3H).

**Step 2:** The compound methyl R1(5*R*)-5-[(1*R*,3a*S*,3b*R*,5a*R*,7*S*,9a*S*,9b*S*,11a*R*)-7-acetoxy-4,4-difluoro-9a,11a-dimethylhexadecahydro-1*H*-cyclopenta[1,2-*a*]phenanthren-1-yl]hexanoate **(288-1)** (30 mg, 0.062 mmol) was dissolved in tetrahydrofuran (5 mL), and titanium isopropoxide (95.6 mg, 0.670 mmol) was added thereto at room temperature. The reaction system was replaced with nitrogen three times, cooled to 0°C, and ethylmagnesium chloride (0.622 mL) was added dropwise thereto. The reaction mixture was stirred at room temperature for 1 hour. The reaction was monitored by TLC (petroleum ether: ethyl acetate = 3:1). The reaction mixture was quenched with brine at low temperature, diluted with ethyl acetate, dried over anhydrous sodium sulfate, concentrated, and subjected to column chromatography (petroleum ether: ethyl acetate = 100% to 50%) to obtain (1*R*,3a*S*,5a*R*,7*S*,9a*S*,11a*R*)-4,4-difluoro-1-[(2*R*)-5-(hydroxycyclopropyl)pentan-2-yl]-9a,11a-dimethylhexadecahydro-1*H*-cyclopenta[1,2-*a*]phenanthrene-7-ol **(288)** (5 mg, 0.011 mmol, 17.79%) as a white solid with a purity of 97%. ¹H NMR (400 MHz, Chloroform-d) δ 3.68 - 3.53 (m, 1H), 1.97 (d, *J* = 13.6 Hz, 1H), 1.82 (d, *J* = 9.5 Hz, 3H), 1.73 (d, *J* = 6.6 Hz, 3H), 1.61 - 1.57 (m, 1H), 1.51 (d, *J =* 10.9 Hz, 2H), 1.47 - 1.34 (m, 8H), 1.33 - 1.17 (m, 5H), 1.16 - 0.96 (m, 5H), 0.92 (d, *J =* 6.0 Hz, 3H), 0.82 (d, *J =* 10.3 Hz, 3H), 0.70 (d, *J =* 6.3 Hz, 2H), 0.64 (d, *J =* 12.0 Hz, 3H), 0.42 (s, 2H). ¹³C NMR (100 MHz, Chloroform-d) δ 124.41, 109.99, 70.71, 55.90, 55.11, 54.22, 52.52, 50.12, 50.03, 48.56, 48.53, 43.49, 43.08, 42.21, 42.02, 41.98, 41.79, 40.18, 40.08, 39.59, 39.34, 38.81, 37.98, 37.75, 37.50, 37.34, 36.93, 36.22, 36.21, 35.95, 35.90, 35.70, 34.91, 34.90, 31.23, 31.15, 31.00, 29.67, 28.45, 28.43, 25.38, 25.34, 22.30, 21.60, 21.18, 18.94, 18.70, 13.59, 13.57, 13.46, 12.89, 12.55, 11.83, 11.31. ¹⁹F NMR (376 MHz, Chloroform-d) δ -89.00, -89.63, -110.52, -111.10 (m), -111.51 (dd, *J =* 59.6, 13.9 Hz), -111.52.

LC-MS: [M+Na]⁺ = 461.4.

### Example 289

### Preparation of 24-[(2,6-difluorophenyl)(hydroxy)methyl]-4β-hydroxy-3β-hydroxy-5α-cholan-7-one (compound 289)

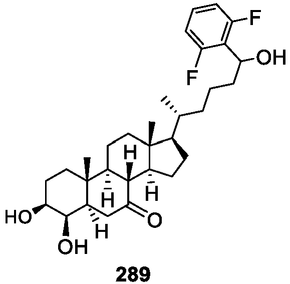

Referring to example **271,** *o*-fluorobromobenzene was replaced with 2,6-difluorobromobenzene to obtain compound 24-[(2,6-difluorophenyl)(hydroxy)methyl]-4β-hydroxy-3β-hydroxy-5α-cholan-7-one **(289).** ¹H NMR (400 MHz, CDCl₃) δ 7.21 (td, *J* = 8.3, 4.1 Hz, 1H), 6.88 (m, 2H), 5.03 (t, *J =* 7.2 Hz, 1H), 3.71 (s, 1H), 3.58 (dt, *J* = 11.3, 4.1 Hz, 1H), 2.87 (t, *J =* 13.5 Hz, 1H), 2.39 (d, *J =* 11.1 Hz, 1H), 2.20 (m, 1H), 2.11 (dd, *J =* 12.7, 2.8 Hz, 1H), 1.97 (dd, *J =* 20.3, 7.8 Hz, 3H), 1.85 (m, 2H), 1.77 (dd, *J =* 9.7, 5.9 Hz, 2H), 1.70 (dd, *J* = 10.6, 5.7 Hz, 1H), 1.47 (m, 3H), 1.38 (dd, *J =* 14.5, 6.6 Hz, 3H), 1.28 (s, 3H), 1.25 (s, 2H), 1.03 (ddd, *J =* 16.6, 15.5, 5.2 Hz, 6H), 0.88 (t, *J =* 6.0 Hz, 3H), 0.63 (d, *J =* 3.5 Hz, 3H). ¹³C NMR (101 MHz, CDCl₃) δ 212.82, 179.17, 151.11, 140.28, 128.96, 111.87, 111.61, 73.82, 71.97, 55.78, 54.94, 50.06, 48.98, 43.80, 42.56, 38.62, 37.89, 36.52, 36.10, 35.87, 35.59, 35.46, 29.71, 28.40, 25.58, 25.01, 22.46, 21.12, 18.64, 14.19, 12.06. LC-MS: [M+H-H₂O]⁺ = 501.5.

### Example 290

### Preparation of 3-(1-hydroxyethyl)-5α-cholest-25-ol (compound 290)

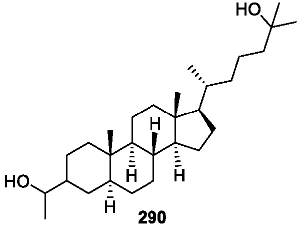

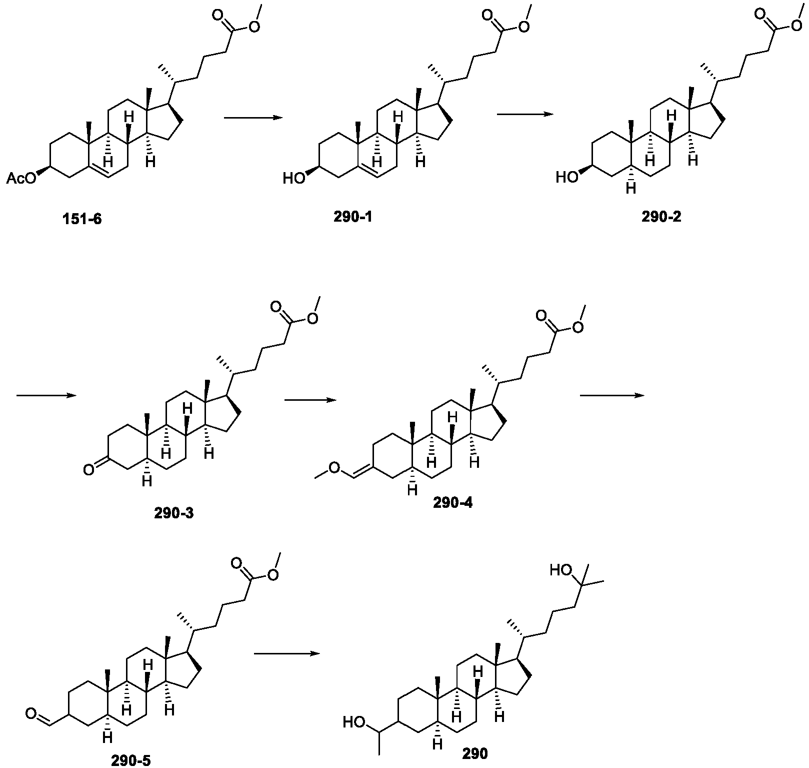

**Step 1:** (1*R*,3a*S*,3b*S*,7*S*,9a*R*,9b*S*,11a*R*)-1-[(2*R*)-6-Methoxy-6-oxohexan-2-yl]-9a,11a-dimethyl-2,3,3a,3b,4,6,7,8,9,9a,9b,10,11,11a-tetradecahydro-1*H*-cyclopenta[1,2-i]phenanthren-7-yl acetate **(151-6)** (1.0 g, 2.2 mmol, 1.0 *eq.*) was weighed and dissolved in tetrahydrofuran (5 mL) and anhydrous methanol (5 mL), and potassium carbonate (0.9 g, 6.5 mmol, 3.0 *eq.*) was added thereto. The reaction mixture was then heated to 50°C and stirred for 1 hour. The reaction was monitored by TLC (petroleum ether: ethyl acetate = 10:1). After the reaction was completed, the reaction mixture was concentrated to obtain a crude product, which was then extracted with water and ethyl acetate. The organic phase was separated, dried, concentrated, and purified by column chromatography (petroleum ether: ethyl acetate = 0 to 40%) to obtain methyl (5*R*)-5-[(1*R*,3a*S*,3b*S*,7*S*,9a*R*,9b*S*,11a*R*)-7-hydroxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate **(290-1)** (800 mg, 1.9 mmol, 88.4%) as a white solid. ¹H NMR (399 MHz, Chloroform-d) δ 5.33 (d, *J =* 5.1 Hz, 1H), 3.65 (s, 3H), 3.51 (tt, *J =* 10.9, 4.9 Hz, 1H), 2.25 (tq, *J =* 15.7, 9.8, 8.3 Hz, 5H), 1.97 (tt, *J =* 12.7, 3.3 Hz, 2H), 1.83 (dt, *J =* 12.7, 4.2 Hz, 3H), 1.73 - 1.65 (m, 1H), 1.46 (d, *J =* 12.6 Hz, 4H), 1.40 (dd, *J* = 17.9, 7.2 Hz, 3H), 1.26 - 1.20 (m, 2H), 1.15 (dq, *J =* 12.8, 6.8 Hz, 2H), 1.06 (dd, *J =* 7.4, 4.0 Hz, 3H), 0.99 (s, 4H), 0.92 (d, *J =* 6.6 Hz, 3H), 0.85 (dd, *J =* 24.0, 6.0 Hz, 1H), 0.66 (s, 3H).

**Step 2:** Methyl (5*R*)-5-[(1*R*,3a*S*,3b*S*,7*S*,9a*R*,9b*S*,11a*R*)-7-hydroxy-9a,11a-dimethyl-2,3,3a,3b,4,6,7,8,9,9a,9b,10,11,11a-tetradecahydro-1*H*-cyclopenta[1,2-a]phenanthren-1-yl]hexanoate **(290-1)** (0.8 g, 1.99 mmol) was weighed and dissolved in tetrahydrofuran (5 mL) and anhydrous methanol (2.5 mL), then Pd/C (10%, 500 mg) was added thereto at room temperature, and the reaction system was replaced with hydrogen. The reaction was monitored by TLC (petroleum ether: ethyl acetate = 3:1). After the reaction was completed, the reaction mixture was filtered to remove Pd/C. The filtrate was concentrated, and the crude product was purified by column chromatography (petroleum ether: ethyl acetate = 0 to 40%) to obtain methyl (5*R*)-5-[(1*R*,3a*S*,3b*R*,5a*S*,7*S*,9a*S*,9b*S*,11a*R*)-7-hydroxy-9a,11a-dimethylhexadecahydro-1*H*-cyclopenta[1,2-*a*]phenanthren-1-yl]hexanoate **(290-2)** (0.65 g, 1.6 mmol, 80.8%) as a white solid. ¹H NMR (399 MHz, Chloroform-d) δ 3.64 (s, 3H), 3.57 (dt, *J* = 11.1, 5.9 Hz, 1H), 2.30 - 2.20 (m, 2H), 1.93 (dt, *J =* 12.6, 3.4 Hz, 1H), 1.82 - 1.75 (m, 2H), 1.66 (ddt, *J =* 19.4, 12.8, 3.7 Hz, 4H), 1.49 - 1.43 (m, 2H), 1.41 - 1.29 (m, 5H), 1.29 - 1.25 (m, 2H), 1.21 (d, *J =* 4.0 Hz, 1H), 1.20 - 1.15 (m, 1H), 1.13 - 1.00 (m, 6H), 0.96 (dd, *J =* 13.3, 4.1 Hz, 2H), 0.90 (d, *J =* 6.5 Hz, 3H), 0.86 - 0.80 (m, 1H), 0.78 (s, 3H), 0.62 (s, 3H), 0.61 - 0.55 (m, 1H).

**Step 3:** Methyl (5*R*)-5-[(1*R*,3a*S*,3b*R*,5a*S*,7*S*,9a*S*,9b*S*,11a*R*)-7-hydroxy-9a,11a-dimethylhexadecahydro-1*H*-cyclopenta[1,2-*a*]phenanthren-1-yl]hexanoate **(290-2)** (650 mg, 1.6 mmol) was weighed and dissolved in dichloromethane (13 mL), then Dess-Martin periodinane (1.37 g, 3.2 mmol) was added thereto with stirring at room temperature, and the reaction mixture was stirred at room temperature for another 1 hour. The completion of the reaction was monitored by TLC (petroleum ether: ethyl acetate = 3:1). The reaction mixture was then extracted with water and ethyl acetate, dried, concentrated, and the crude product was purified by column chromatography (petroleum ether: ethyl acetate = 0 to 30%) to obtain methyl (5*R*)-5-[(1*R*,3a*S*,3b*R*,5a*S*,9a*S*,9b*S*,11a*R*)-9a,11a-dimethyl-7-oxohexadecahydro-1*H-*cyclopenta[1,2-*a*]phenanthren-1-yl]hexanoate **(290-3)** (0.57 g, 1.4 mmol, 88.1%) as a white solid. ¹H NMR (399 MHz, Chloroform-d) δ 3.65 (s, 3H), 2.37 (td, *J* = 14.3, 13.5, 6.3 Hz, 1H), 2.30 - 2.20 (m, 4H), 2.06 - 1.92 (m, 3H), 1.86 - 1.75 (m, 1H), 1.68 (dt, *J =* 13.3, 3.5 Hz, 2H), 1.50 (tt, *J* = 9.2, 4.7 Hz, 4H), 1.37 (q, *J* = 5.8 Hz, 3H), 1.34 - 1.29 (m, 3H), 1.27 - 1.19 (m, 2H), 1.17 - 1.01 (m, 5H), 0.99 (s, 3H), 0.91 (d, *J* = 6.4 Hz, 3H), 0.84 (dd, *J =* 18.0, 6.1 Hz, 1H), 0.75 - 0.67 (m, 1H), 0.66 (s, 3H).

**Step 4:** (Methoxymethyl)triphenylphosphonium chloride (1.22 g, 3.6 mmol) was weighed and dissolved in tetrahydrofuran (11 mL). The reaction system was replaced with nitrogen three times, cooled to 0°C, and added dropwise with sodium bis(trimethylsilyl)amide (1.77 mL, 3.6 mmol). After the dropwise addition was completed, the reaction mixture was stirred at 0°C for 0.5 hours, then added with a solution of methyl (5R)-5-[(1*R*,3a*S*,3b*R*,5a*S*,9a*S*,9b*S*,11a*R*)-9a,11a-dimethyl-7-oxohexadecahydro-1*H*-cyclopenta[1,2-*a*]phenanthren-1-yl]hexanoate **(290-3)** (0.57 g, 1.4 mmol) in tetrahydrofuran (11.5 mL), warmed to room temperature, and stirred for 1 hour. The completion of the reaction was monitored by TLC (petroleum ether: ethyl acetate = 10:1). The reaction mixture was then extracted with water and ethyl acetate, dried, concentrated, and the crude product was purified by column chromatography (petroleum ether: ethyl acetate = 0 to 20%) to obtain methyl (5R)-5-[(1*R*,3a*S*,3b*R*,5a*S*,9a*S*,9b*S*,11a*R*)-7-[(*E*)-methoxymethylene]-9a,11a-dimethylhexadecahydro-1*H*-cyclopenta[1,2-*a*]phenanthren-1-yl]hexanoate **(290-4)** (0.51 g, 1.2 mmol, 83.6%) as a white solid. ¹H NMR (399 MHz, Chloroform-d) δ 5.71 (s, 1H), 3.64 (s, 3H), 3.50 (d, *J* = 2.0 Hz, 3H), 2.58 (dd, *J* = 13.5, 4.4 Hz, 1H), 2.35 (dd, *J* = 14.1, 3.8 Hz, 1H), 2.24 (h, *J =* 6.7 Hz, 2H), 1.93 (dt, *J* = 12.7, 3.4 Hz, 1H), 1.87 - 1.67 (m, 5H), 1.55 - 1.43 (m, 4H), 1.34 (dt, *J* = 17.6, 7.0 Hz, 3H), 1.26 - 1.18 (m, 4H), 1.05 (dd, *J =* 12.1, 7.7 Hz, 4H), 1.00 - 0.93 (m, 2H), 0.90 (d, *J =* 6.7 Hz, 3H), 0.87 - 0.84 (m, 1H), 0.81 (s, 3H), 0.80 - 0.74 (m, 1H), 0.63 (s, 3H), 0.61 - 0.56 (m, 1H).

**Step 5:** Methyl (5*R*)-5-[(1*R*,3a*S*,3b*R*,5a*S*,9a*S*,9b*S*,11a*R*)-7-[(*E*)-methoxymethylene]-9a,11a-dimethylhexadecahydro-1*H*-cyclopenta[1,2-*a*]phenanthren-1-yl]hexanoate **(290-4)** (0.51 g, 1.2 mmol) was weighed and dissolved in tetrahydrofuran (11 mL), and 5 N hydrochloric acid (1.5 mL, 7.5 mmol) was added thereto. The reaction mixture was then heated to 50°C and stirred for 1 hour. The completion of the reaction was monitored by TLC (petroleum ether: ethyl acetate = 10:1). The reaction mixture was then added with water (15 mL), extracted with ethyl acetate (10 mL * 3), dried, concentrated, and the crude product was purified by column chromatography (petroleum ether: ethyl acetate = 0 to 20%) to obtain methyl (5*R*)-5-[(1*R*,3a*S*,3b*R*,5a*S*,9a*S*,9b*S*,11a*R*)-7-formyl-9a,11a-dimethylhexadecahydro-1*H*-cyclopenta[1,2-*a*]phenanthren-1-yl]hexanoate **(290-5)** (0.4 g, 1.0 mmol, 81%) as a white solid. ¹H NMR (399 MHz, Chloroform-d) δ 9.59 (d, *J* = 1.6 Hz, 1H), 3.65 (s, 3H), 3.47 (s, 1H), 2.30 - 2.18 (m, 3H), 1.94 (dt, *J* = 12.6, 3.4 Hz, 1H), 1.78 (dtd, *J* = 18.1, 12.1, 10.9, 5.1 Hz, 3H), 1.65 (dq, *J =* 12.7, 3.8 Hz, 2H), 1.59 - 1.43 (m, 7H), 1.42 - 1.17 (m, 10H), 1.09 (dq, *J* = 17.4, 8.0, 5.6 Hz, 4H), 0.98 (dd, *J =* 12.9, 4.4 Hz, 2H), 0.90 (d, *J =* 6.4 Hz, 3H), 0.88 - 0.79 (m, 2H), 0.77 (d, *J =* 10.3 Hz, 3H), 0.67 (dd, *J =* 11.0, 3.9 Hz, 1H), 0.62 (d, *J =* 6.4 Hz, 3H).

**Step 6:** Methyl (5*R*)-5-[(1*R*,3a*S*,3b*R*,5a*S*,9a*S*,9b*S*,11a*R*)-7-formyl-9a,11a-dimethylhexadecahydro-1*H*-cyclopenta[1,2-*a*]phenanthren-1-yl]hexanoate **(290-5)** (40 mg, 0.1 mmol) was weighed and dissolved in tetrahydrofuran (5.0 mL). The reaction mixture was cooled to -78°C, and methyllithium (1.6 M, 0.6 mL) was added dropwise thereto. After the dropwise addition was completed, the reaction mixture was slowly warmed to room temperature and stirred for 2 hours. The reaction was monitored by TLC (petroleum ether: ethyl acetate = 3:1). After the reaction was completed, the reaction mixture was quenched with water and extracted with ethyl acetate. The organic phase was separated, dried, concentrated, and purified by column chromatography (petroleum ether: ethyl acetate = 0 to 30%) to obtain 3-(1-hydroxyethyl)-5α-cholest-25-ol **(290)** (18 mg, 0.04 mmol, 43.4%) as a white solid. ¹H NMR (399 MHz, Chloroform-d) δ 3.53 (p, *J* = 6.2 Hz, 1H), 1.97 - 1.92 (m, 1H), 1.81 - 1.70 (m, 2H), 1.63 (dt, *J =* 10.5, 3.3 Hz, 2H), 1.57 - 1.51 (m, 2H), 1.46 - 1.41 (m, 4H), 1.35 (dd, *J* = 6.5, 3.9 Hz, 4H), 1.30 - 1.24 (m, 5H), 1.19 (s, 6H), 1.14 (d, *J =* 6.3 Hz, 3H), 1.08 (d, *J* = 9.7 Hz, 3H), 1.06 - 1.02 (m, 3H), 1.01 - 0.96 (m, 3H), 0.90 (d, *J* = 6.4 Hz, 3H), 0.84 (dd, *J =* 11.9, 7.0 Hz, 2H), 0.73 (s, 3H), 0.63 (s, 3H).

¹³C NMR (100 MHz, CDCl₃) δ 72.27, 71.11, 56.53, 56.19, 54.78, 54.53, 46.35, 46.22, 45.39, 45.34, 44.40, 42.59, 41.73, 41.15, 40.05, 38.26, 38.15, 36.41, 35.93, 35.74, 35.52, 32.11, 31.04, 30.55, 29.32, 29.17, 29.07, 28.24, 24.22, 24.17, 23.82, 23.47, 21.98, 21.01, 20.77, 20.61, 20.55, 18.62, 12.25, 12.06. LCMS: [M-OH]⁺ = 415.15.

### Example 291

### Preparation of (1R,3aS,3bS,5aR,7S,9aR,9bS,11aR)-6,6-difluoro-1-[(2R)-6-hydroxy-6-methylheptan-2-yl]-9a,11a-dimethylhexadecahydro-1H-cyclopenta[1,2-a]phenanthrene-7-ol (compound 291)

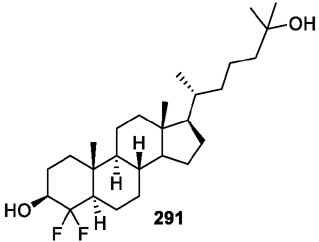

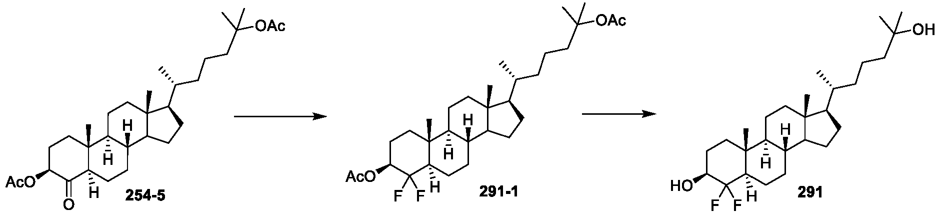

**Step 1:** To the compound (6*R*)-6-[(1*R*,3a*S*,3b*S*,5a*S*,6*R*,7*S*,9a*R*,9b*S*,11a*R*)-7-acetoxy-6-hydroxy-9a,11a-dimethylhexadecahydro-1*H*-cyclopenta[1,2-*a*]phenanthren-1-yl]-2-methylheptan-2-yl acetate **(254-5)** (100 mg, 0.199 mmol, 1 *eq)* was added diethylaminosulfur trifluoride (3 mL, 0.596 mmol). The reaction system was stirred at 80°C for 40 minutes. The reaction was monitored by TLC (petroleum ether: ethyl acetate = 10:1). The reaction system was diluted with dichloromethane and then quenched with water. The aqueous phase was extracted with dichloromethane (25 mL × 3). The dichloromethane phases were combined and washed with saturated brine (10 mL × 3). The dichloromethane phase was dried over anhydrous sodium sulfate, filtered, and concentrated to obtain a crude product. The crude product was purified by flash chromatography (petroleum ether: ethyl acetate = 81:19) to obtain (6*R*)-6-[(1*R*,3a*S*,3b*S*,5a*R*,6*Z*,7*S*,9a*R*,9b*S*,11a*R*)-7-hydroxy-6-(hydroxyimino)-9a,11a-dimethylhexadecahydro-1*H*-cyclopenta[1,2-*a*]phenanthren-1-yl]-2-methylheptan-2-yl acetate **(291-1)** (15 mg, purity: 80%, yield: 11.5%) as a white solid. ¹H NMR (400 MHz, MeOD) δ 4.89 - 4.80 (m, 1H), 1.98 (s, 3H), 1.85 (s, 3H), 1.75 - 1.46 (m, 14H), 1.39 (d, *J* = 5.4 Hz, 4H), 1.32 (s, 6H), 1.23 - 0.90 (m, 19H), 0.86 (d, *J =* 1.9 Hz, 3H), 0.84 (d, *J =* 6.6 Hz, 3H), 0.80 - 0.68 (m, 4H), 0.60 (s, 3H).

**Step 2:** The starting material (6*R*)-6-[(1*R*,3a*S*,3b*S*,5a*R*,6*Z*,7*S*,9a*R*,9b*S*,11a*R*)-7-hydroxy-6-(hydroxyimino)-9a,11a-dimethylhexadecahydro-1*H*-cyclopenta[1,2-*a*]phenanthren-1-yl]-2-methylheptan-2-yl acetate **(291-1)** (15 mg, 0.03 mmol, 1 *eq*) (white solid) was dissolved in tetrahydrofuran (0.5 mL) and methanol (0.5 mL), and the reaction mixture was reacted at room temperature for 24 hours. The completion of the reaction was monitored by TLC (petroleum ether: ethyl acetate = 3:1). The reaction system was added with water (10 mL), and the aqueous phase was extracted with ethyl acetate (25 mL × 3). The ethyl acetate phases were combined and washed with saturated brine (10 mL × 3). The ethyl acetate phase was dried over anhydrous sodium sulfate, filtered, and concentrated to obtain a crude product. After the reaction was completed, the reaction mixture was filtered, and the filtrate was washed with water. The organic phase was dried, concentrated, and the crude product was purified by flash column chromatography (petroleum ether: ethyl acetate = 83:17) to obtain (1*R*,3a*S*,3b*S*,5a*R*,7*S*,9a*R*,9b*S*,11a*R*)-6,6-difluoro-1-[(2*R*)-6-hydroxy-6-methylheptan-2-yl]-9a,11a-dimethylhexadecahydro-1*H*-cyclopenta[1,2-*a*]phenanthrene-7-ol **(291)** (2.09 mg, purity: 97.76%, yield: 16.2%) as a white solid.

¹H NMR (400 MHz, DMSO) δ 5.22 (s, 1H), 4.04 (s, 1H), 3.53 (dd, *J* = 20.7, 5.9 Hz, 1H), 1.93 (d, *J* = 12.4 Hz, 1H), 1.75 - 1.65 (m, 4H), 1.60 - 1.45 (m, 4H), 1.41 - 1.17 (m, 15H), 1.05 (s, 6H), 0.97 (dd, *J =* 20.4, 13.0 Hz, 3H), 0.88 (d, *J =* 6.5 Hz, 3H), 0.82 (s, 3H), 0.72 (dd, *J =* 15.2, 7.4 Hz, 1H), 0.62 (s, 3H). ¹³C NMR (101 MHz, DMSO) δ 70.58, 69.23, 56.26, 56.15, 54.24, 44.59, 42.55, 37.35, 36.62, 35.67, 35.45, 34.83, 31.15, 29.88, 29.70, 28.26, 27.85, 27.77, 24.22, 20.83, 20.71, 18.98, 18.30, 13.31, 13.25, 12.33.

### Example 292

### Preparation of (1R,3aS,3bR,5aS,7S,9aS,9bS,11aR)-1-[(2R)-6-hydroxy-6-methylheptan-2-yl]-9a,11a-dimethylhexadecahydro-1H-cyclopenta[1,2-i]phenanthren-7-yl hydroxyacetate (compound 292)

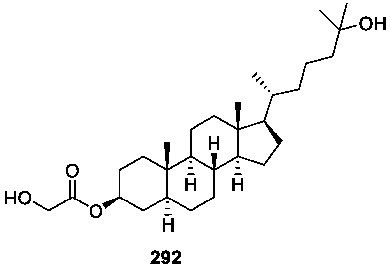

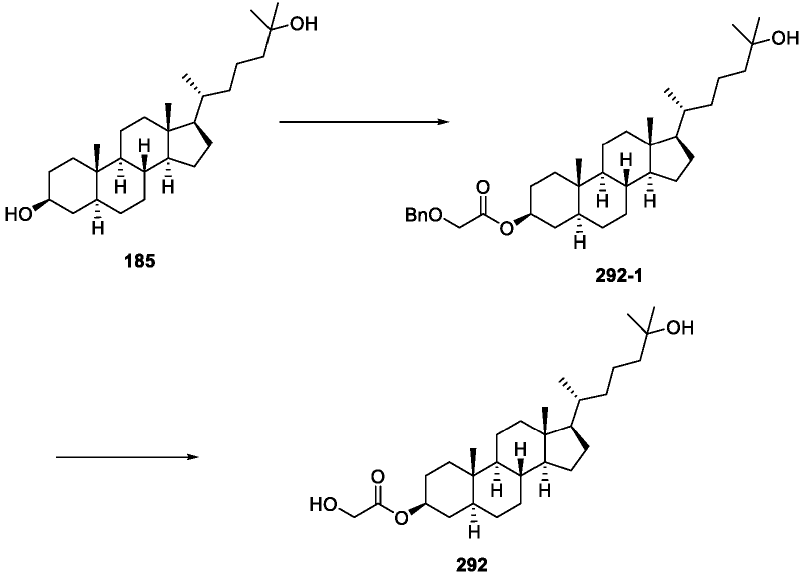

**Step 1:** 5α-Cholest-3β,25-diol **(185)** (100 mg, 0.256 mmol) was dissolved in tetrahydrofuran (5 mL), and diisopropylethylamine (0.085 mL, 0.512 mmol) was added thereto. The reaction mixture was cooled in an ice bath, and (benzyloxy)acetyl chloride (51.99 mg, 0.282 mmol) was added dropwise thereto. After the dropwise addition was completed, the reaction mixture was warmed to room temperature, stirred for 30 minutes, then heated to 40°C, and stirred for 18 hours. The reaction was monitored by TLC (petroleum ether: ethyl acetate = 10:1). After the reaction was completed, the reaction mixture was concentrated to remove the solvent, and extracted with water and ethyl acetate. The organic phase was separated, dried, concentrated, and purified by column chromatography (petroleum ether: ethyl acetate = 0% to 30%) to obtain (1*S*,3a*S*,3b*S*,5a*S*,7*S*,9a*S*,9b*S*,11a*R*)-1-(5-hydroxy-5-methylhexyl)-9a, 11a-dimethylhexadecahydro-1*H*-cyclopenta[1,2-*i*]phenanthren-7-yl (benzyloxy)acetate **(292-1)** (80 mg, 0.134 mmol, 52.20%) as a white solid. ¹H NMR (399 MHz, Chloroform-d) δ 7.38 - 7.26 (m, 5H), 4.79 (dt, *J* = 11.3, 6.1 Hz, 1H), 4.61 (s, 2H), 4.03 (s, 2H), 1.94 (dt, *J* = 12.6, 3.4 Hz, 1H), 1.86 - 1.70 (m, 3H), 1.67 - 1.30 (m, 15H), 1.24 (q, *J =* 6.4, 4.4 Hz, 4H), 1.19 (s, 6H), 1.12 - 0.96 (m, 6H), 0.90 (d, *J* = 6.4 Hz, 3H), 0.87 - 0.83 (m, 1H), 0.79 (s, 3H), 0.63 (s, 4H).

**Step 2:** (1*S*,3a*S*,3b*S*,5a*S*,7*S*,9a*S*,9b*S*,11a*R*)-1-(5-Hydroxy-5-methylhexyl)-9a,11a-dimethylhexadecahydro-1*H*-cyclopenta[1,2-*i*]phenanthren-7-yl (benzyloxy)acetate **(292-1)** (80 mg, 0.148 mmol) was dissolved in methanol (5 mL), and Pd/C (10% Pd, containing 40 to 60% water) (10 mg, 0.094 mmol) was added thereto. The reaction system was then replaced with hydrogen three times and heated to 40°C. The reaction was monitored by TLC (petroleum ether: ethyl acetate = 5:1). After the reaction was completed, the reaction mixture was filtered to remove Pd/C, concentrated, and purified by column chromatography (petroleum ether: ethyl acetate = 0% to 30%) to obtain (1*R*,3a*S*,3b*R*,5a*S*,7*S*,9a*S*,9b*S*,11a*R*)-1-[(2*R*)-6-hydroxy-6-methylheptan-2-yl]-9a,11a-dimethylhexadecahydro-1*H*-cyclopenta[1,2-*i*]phenanthren-7-yl hydroxyacetate **(292)** (55 mg, 0.101 mmol, 69.62%) as a white solid with a purity of 84.75%. ¹H NMR (399 MHz, Chloroform-d) δ 4.80 (dt, *J =* 11.5, 6.1 Hz, 1H), 4.09 (s, 2H), 1.95 (d, *J =* 12.6 Hz, 1H), 1.86 - 1.71 (m, 3H), 1.68 - 1.63 (m, 2H), 1.60 - 1.46 (m, 4H), 1.45 - 1.28 (m, 8H), 1.26 (d, *J* = 10.5 Hz, 2H), 1.19 (s, 6H), 1.15 - 0.95 (m, 9H), 0.90 (d, *J* = 6.4 Hz, 3H), 0.85 (d, *J* = 10.4 Hz, 1H), 0.80 (s, 3H), 0.63 (s, 3H). ¹³C NMR (101 MHz, Chloroform-d) δ 75.43, 71.09, 60.74, 56.36, 56.16, 54.14, 44.58, 44.39, 42.57, 39.92, 36.63, 36.39, 35.73, 35.41, 33.89, 31.92, 29.31, 29.18, 28.53, 28.22, 27.39, 24.16, 21.17, 20.76, 18.61, 12.18, 12.04. ELSD-MS: [M+MeCN+Na⁺]⁺ = 526.3.

### Example 293

### Preparation of 3-{[(1R,3aS,3bR,5aS,7S,9aS,9bS,11aR)-1-[(2R)-6-hydroxy-6-methylheptan-2-yl]-9a,11a-dimethylhexadecahydro-1H-cyclopenta[1,2-a]phenanthren-7-yl]oxy}-3-oxopropanoic acid (compound 293)

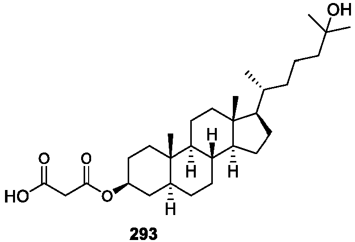

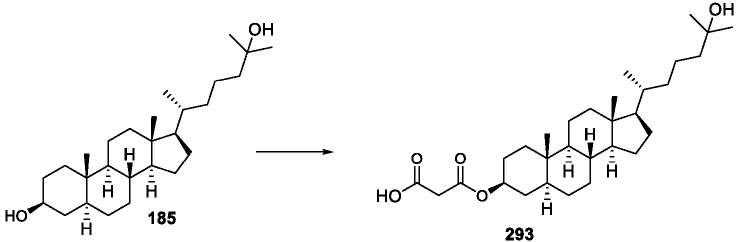

5α-Cholest-3β,25-diol **(185)** (100 mg, 0.25 mmol) was dissolved in toluene (5 mL). Pyridine (0.4 mL, 3.0 *eq.*) and Meldrum's acid (36 mg, 0.25 mmol) were added thereto. The reaction system was replaced with nitrogen three times and stirred at 80°C for 16 hours. The reaction was monitored by TLC (petroleum ether: ethyl acetate = 3:1). The reaction mixture was cooled to room temperature and extracted with water and ethyl acetate. The organic phase was separated, dried, concentrated, and purified by column chromatography (petroleum ether: ethyl acetate = 0% to 80%) to obtain 3-{[(1*R*,3a*S*,3b*R*,5a*S*,7*S*,9a*S*,9b*S*,11a*R*)-1-[(2R)-6-hydroxy-6-methylheptan-2-yl]-9a,11a-dimethylhexadecahydro-1*H*-cyclopenta[1,2-*a*]phenanthren-7-yl]oxy}-3-oxopropanoic acid **(293)** (15 mg, 0.03 mmol, 12.4%) as an off-white solid with a purity of 97.8%. ¹H NMR (400 MHz, DMSO-d₆) δ 4.54 (s, 1H), 3.12 (s, 2H), 1.99 - 1.87 (m, 1H), 1.71 (s, 2H), 1.48 (s, 4H), 1.30 - 1.08 (m, 15H), 1.01 (s, 6H), 0.95 (s, 4H), 0.84 (t, *J* = 8.7 Hz, 3H), 0.74 (s, 3H), 0.59 (s, 3H). ¹³C NMR (100 MHz, DMSO-d₆) δ 73.69, 69.21, 56.37, 56.21, 53.92, 44.55, 44.39, 42.58, 36.55, 35.68, 35.44, 35.39, 34.02, 31.96, 29.83, 29.66, 28.55, 28.24, 27.47, 24.24, 21.19, 20.74, 18.96, 12.33. ELSD-MS: [M+NH₄⁺]⁺ = 508.3.

### Example 294

### Preparation of 4-{[(1R,3aS,3bR,5aS,7S,9aS,9bS,11aR)-1-[(2R)-6-hydroxy-6-methylheptan-2-yl]-9a,11a-dimethylhexadecahydro-1H-cyclopenta[1,2-a]phenanthren-7-yl]oxy}-4-oxobutanoic acid (compound 294)

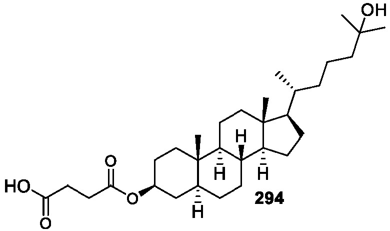

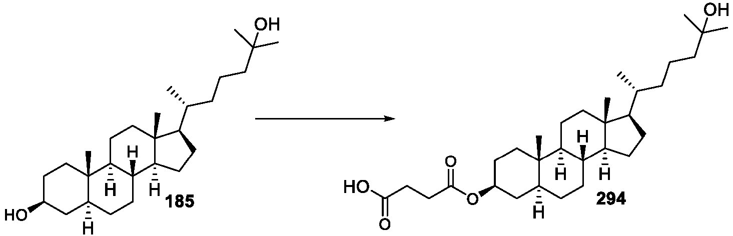

5α-Cholest-3β,25-diol **(185)** (100 mg, 0.25 mmol) was dissolved in pyridine (3 mL), and succinic anhydride (25 mg, 0.25 mmol) was added thereto. The reaction system was replaced with nitrogen three times and stirred at 120°C for 5 hours. The reaction was monitored by TLC (petroleum ether: ethyl acetate = 3:1). The reaction mixture was cooled to room temperature and extracted with water and ethyl acetate. The organic phase was separated, dried, concentrated, and purified by column chromatography (petroleum ether: ethyl acetate = 0% to 80%) to obtain 4-{[(1*R*,3a*S*,3b*R*,5a*S*,7*S*,9a*S*,9b*S*,11a*R*)-1-[(2R)-6-hydroxy-6-methylheptan-2-yl]-9a,11a-dimethylhexadecahydro-1*H*-cyclopenta[1,2-*a*]phenanthren-7-yl]oxy}-4-oxobutanoic acid **(294)** (20 mg, 0.04 mmol, 16%) as an off-white solid with a purity of 90.66%. ¹H NMR (399 MHz, Chloroform-d) δ 4.69 (tt, *J* = 11.3, 5.0 Hz, 1H), 2.67 - 2.60 (m, 2H), 2.58 (d, *J =* 6.4 Hz, 2H), 1.98 - 1.91 (m, 1H), 1.83 - 1.45 (m, 7H), 1.45 - 1.28 (m, 8H), 1.24 (d, *J* = 3.9 Hz, 5H), 1.19 (s, 6H), 1.17 - 0.93 (m, 9H), 0.89 (d, *J* = 6.4 Hz, 3H), 0.79 (s, 3H), 0.63 (s, 3H). ¹³C NMR (101 MHz, Chloroform-d) δ 176.65, 71.25, 56.37, 56.19, 54.16, 44.61, 44.35, 42.57, 39.94, 36.68, 36.39, 35.75, 35.42, 33.89, 31.94, 29.29, 29.12, 28.85, 28.55, 28.23, 27.36, 24.17, 21.17, 20.79, 18.60, 12.19, 12.05. LCMS: [M-H]⁻ = 503.3.

### Example 295

### Preparation of 6-amino-5α-cholest-3β,4β,25-triol (compound 295)

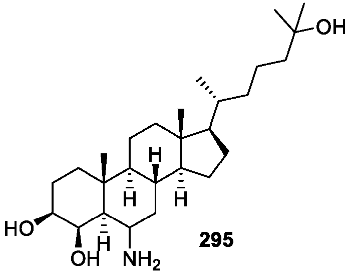

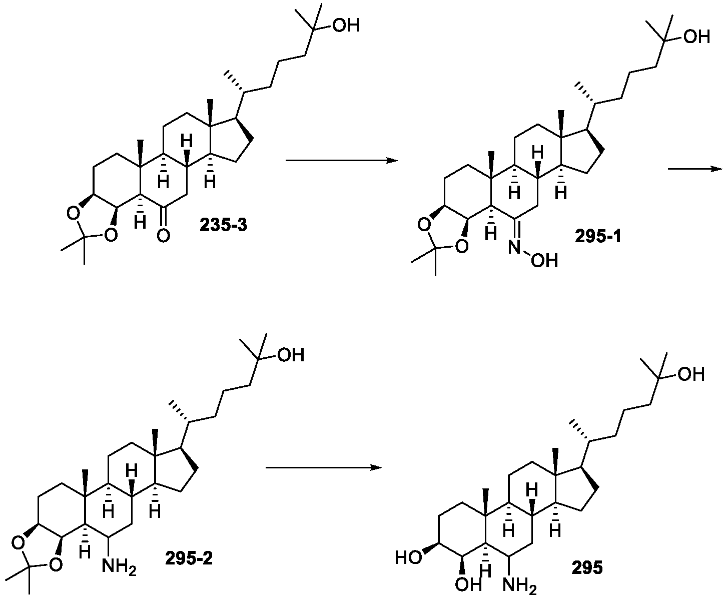

**Step 1:** (3a*S*,5a*R*,5b*S*,7a*R*,8*R*,10a*S*,10b*S*,12a*R*,12b*R*)-8-[(2*R*)-6-Hydroxy-6-methylheptan-2-yl]-2,2,5a,7a-tetramethyl-4,5,5a,5b,6,7,7a,8,9,10,10a,10b,11,12,12a,12b-hexadecahydro-3a*H*-cyclopenta[1',2':1,2]phenanthro[7,8-*d*][1,3]dioxol-12-one **(235-3)** (50 mg, 0.105 mmol) was dissolved in ethanol (5 mL). Ammonium acetate (24.3 mg, 0.316 mmol) and hydroxylamine hydrochloride (21.96 mg, 0.316 mmol) were added thereto, and the reaction mixture was heated to 90°C and stirred for 1 hour. The completion of the reaction was monitored by TLC (petroleum ether: ethyl acetate = 1:1). The reaction mixture was concentrated and subjected to column chromatography (petroleum ether: ethyl acetate = 100 to 20%) to obtain compound [(3a*S*,5a*R*,5b*S*,7a*R*,8*R*,10a*S*,10b*S*,12a*S*,12b*R*,12*E*)-8-[(2*R*)-6-hydroxy-6-methylheptan-2-yl]-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-12-ylidene]hydroxylamine **(295-1)** (30 mg, 0.058 mmol, 55.25%) as a white solid. ¹H NMR (399 MHz, Chloroform-d) δ 4.28 (s, 1H), 3.33 - 3.23 (m, 1H), 2.07 - 1.86 (m, 3H), 1.52 (d, *J =* 25.5 Hz, 7H), 1.40 (q, *J* = 13.2, 12.3 Hz, 6H), 1.32 (s, 4H), 1.24 (s, 4H), 1.19 (s, 6H), 1.13 (d, *J =* 8.8 Hz, 3H), 0.93 - 0.87 (m, 3H), 0.84 (s, 3H), 0.63 (d, *J =* 2.7 Hz, 3H).

**Step 2:** [(3a*S*,5a*R*,5b*S*,7a*R*,8*R*,10a*S*,10b*S*,12a*S*,12b*R*,12*E*)-8-[(2*R*)-6-Hydroxy-6-methylheptan-2-yl]-2,2,5a,7a-tetramethyl-4,5,5a,5b,6,7,7a,8,9,10,10a,10b,11,12,12a,12b-hexadecahydro-3a*H*-cyclopenta[1',2':1,2]phenanthro[7,8-*d*][1,3]dioxol-12-ylidene]hydroxylamine **(295-1)** (10 mg, 0.020 mmol) was dissolved in tetrahydrofuran (5 mL). Lithium aluminum hydride (7.75 mg, 0.204 mmol) was added thereto, and the reaction mixture was heated to 85°C and stirred for 4 hours. The completion of the reaction was monitored by TLC (dichloromethane: methanol = 10:1). The reaction mixture was cooled to 0°C, quenched with ammonium chloride, and extracted three times with ethyl acetate. The organic phases were combined, concentrated, and purified by column chromatography (petroleum ether: ethyl acetate = 0 to 80%) to obtain compound (6*R*)-6-[(3a*S*,5a*R*,5b*S*,7a*R*,8*R*,10a*S*,10b*S*,12a*S*,12b*R*)-12-amino-2,2,5a,7a-tetramethyl-4,5,5a,5b,6,7,7a,8,9,10,10a,10b,11,12,12a,12b-hexadecahydro-3a*H*-cyclopenta[1',2':1,2]phenanthro[7,8-*d*][1,3]dioxol-8-yl]-2-methylheptan-2-ol **(295-2)** (3 mg, 0.006 mmol, 27.79%).

**Step 3:** (6*R*)-6-[(3a*S*,5a*R*,5b*S*,7a*R*,8*R*,10a*S*,10b*S*,12a*S*,12b*R*)-12-Amino-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]-2-methylheptan-2-ol **(295-2)** (20 mg, 0.042 mmol) was dissolved in methanol (5 mL), then hydrochloric acid (0.420 mL, 0.840 mmol) was added thereto, and the reaction mixture was stirred at room temperature for 1 hour. The completion of the reaction was monitored by TLC (petroleum ether: ethyl acetate = 1:1). The reaction mixture was extracted twice with ethyl acetate, dried over anhydrous sodium sulfate, concentrated, and subjected to column chromatography (petroleum ether: ethyl acetate = 0 to 80%) to obtain compound 6-amino-5α-cholest-3β,4β,25-triol **(295)** (10 mg, 0.021 mmol, 48.91%) with a purity of 89.59%. ¹H NMR (399 MHz, Methanol-d₄) δ 3.87 (s, 1H), 3.74 (s, 1H), 3.59 (dd, *J =* 11.1, 3.3 Hz, 1H), 2.02 (d, *J* = 11.7 Hz, 1H), 1.87 (s, 2H), 1.69 (d, *J =* 11.1 Hz, 2H), 1.53 (d, *J =* 11.1 Hz, 2H), 1.41 (s, 6H), 1.28 (d, *J =* 11.3 Hz, 6H), 1.18 (s, 2H), 1.15 (s, 6H), 1.13 (s, 2H), 1.08 (s, 3H), 1.01 (s, 1H), 0.94 (d, *J* = 6.4 Hz, 3H), 0.88 (t, *J* = 6.7 Hz, 1H), 0.73 (s, 3H). ¹³C NMR (101 MHz, CD₃OD) δ 70.01, 68.73, 68.62, 56.15, 55.91, 43.83, 42.45, 41.29, 39.74, 36.32, 35.79, 35.67, 31.00, 29.83, 29.41, 29.32, 27.91, 27.81, 27.68, 25.56, 24.62, 23.74, 22.77, 20.49, 20.41, 17.73, 11.12. ELSD-MS: [M+H]⁺ = 436.3.

### Example 296

### Preparation of 24-[(2-fluorophenyl)(hydroxy)methyl]-3β-hydroxy-5α-cholan-4-one (compound 296)

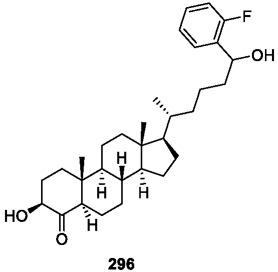

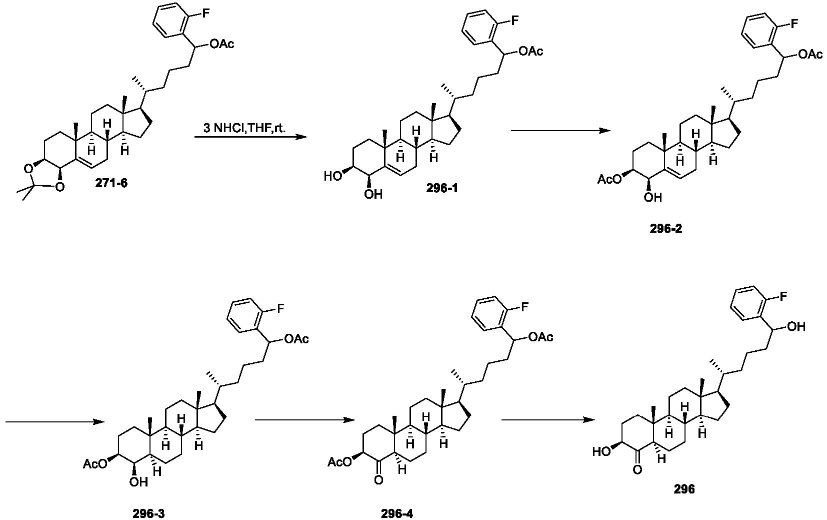

**Step 1:** The compound (5*R*)-5-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1-(2-fluorophenyl)hexyl acetate **(271-6)** (200 mg, 0.353 mmol) was dissolved in tetrahydrofuran (5 mL), then dilute hydrochloric acid (0.5 mL) was added thereto, and the resulting mixture was stirred at 25°C for 1 hour. The completion of the reaction was monitored by TLC (petroleum ether: ethyl acetate = 1: 1). The reaction mixture was quenched with saturated sodium bicarbonate, and the aqueous phase was extracted with ethyl acetate (15 mL × 3). The ethyl acetate phases were combined, washed with saturated brine (15 mL), dried over anhydrous Na₂SO₄, filtered, and concentrated to obtain a crude product. The crude product was purified by silica gel column chromatography (petroleum ether: ethyl acetate = 70:30) to obtain (5*R*)-5-[(1*R*,3a*S*,3b*S*,6*R*,7*S*,9a*R*,9b*S*,11a*R*)-6,7-dihydroxy-9a,11a-dimethyl-2,3,3a,3b,4,6,7,8,9,9a,9b,10,11,11a-tetradecahydro-1*H-*cyclopenta[1,2-*a*]phenanthren-1-yl]-1-(2-fluorophenyl)hexyl acetate **(296-1)** (150 mg, 0.256 mmol, purity: 90%, yield: 72.63%) as a white solid. ¹H NMR (400 MHz, CDCl₃) δ 7.33 (d, *J* = 7.3 Hz, 1H), 7.23 (s, 1H), 7.13 (d, *J* = 7.5 Hz, 1H), 7.03 (m, 1H), 6.02 (d, *J* = 5.0 Hz, 1H), 5.68 (d, *J* = 3.4 Hz, 1H), 4.14 (s, 1H), 3.58 (s, 1H), 2.09 (t, *J=* 3.9 Hz, 3H), 2.00 (m, 2H), 1.78 (m, 12H), 1.56 (m, 4H), 1.35 (ddd, *J =* 31.4, 21.1, 11.7 Hz, 9H), 1.18 (s, 3H), 1.05 (ddd, *J =* 42.3, 19.6, 13.2 Hz, 7H), 0.88 (dd, *J* = 13.7, 7.3 Hz, 4H), 0.66 (d, *J =* 3.4 Hz, 3H).

**Step 2:** The compound (5*R*)-5-[(1*R*,3a*S*,3b*S*,6*R*,7*S*,9a*R*,9b*S*,11a*R*)-6,7-dihydroxy-9a,11a-dimethyl-2,3,3a,3b,4,6,7,8,9,9a,9b,10,11,11a-tetradecahydro-1*H*-cyclopenta[1,2-*a*]phenanthren-1-yl]-1-(2-fluorophenyl)hexyl acetate **(296-1)** (100 mg, 0.189 mmol) was dissolved in dichloromethane (5 mL). Acetic anhydride (0.020 mL, 0.208 mmol, 1.1 *eq*), triethylamine (0.053 mL, 0.378 mmol, 2.0 *eq*), and 4-dimethylaminopyridine (4.62 mg, 0.038 mmol, 0.2 *eq*) were added thereto, and the resulting reaction mixture was stirred at 25°C for 3 hours under a N₂ atmosphere. The completion of the reaction was monitored by TLC (petroleum ether: ethyl acetate = 3:1). The reaction system was quenched with methanol, added with water (10 mL), and the aqueous phase was extracted with dichloromethane (15 mL × 3). The organic phases were combined, washed with saturated brine (5 mL), dried over anhydrous Na₂SO₄, filtered, and concentrated to obtain a crude product. The crude product was purified by silica gel column chromatography (petroleum ether: ethyl acetate = 70:30) to obtain (5*R*)-5-[(1*R*,3a*S*,3b*S*,5a*R*,6*R*,7*S*,9a*R*,9b*S*,11a*R*)-7-acetoxy-6-hydroxy-9a,11a-dimethylhexadecahydro-1*H*-cyclopenta[1,2-*a*]phenanthren-1-yl]-1-(2-fluorophenyl)hexyl acetate **(296-2)** (90 mg, 0.142 mmol, purity: 90%, yield: 75.03%) as a white solid. ¹H NMR (400 MHz, CDCl₃) δ 7.34 (t, *J =* 7.4 Hz, 1H), 7.24 (d, *J =* 8.0 Hz, 1H), 7.12 (t, *J =* 7.5 Hz, 1H), 7.03 (m, 1H), 6.02 (m, 1H), 5.70 (d, *J* = 3.6 Hz, 1H), 4.72 (d, *J* = 12.0 Hz, 1H), 4.24 (d, *J* = 2.7 Hz, 1H), 2.10 (s, 3H), 2.08 (d, *J* = 2.2 Hz, 3H), 2.06 (d, *J* = 8.1 Hz, 2H), 1.84 (m, 5H), 1.68 (m, 2H), 1.36 (m, 10H), 1.21 (s, 4H), 1.08 (m, 8H), 0.87 (t, *J* = 6.0 Hz, 4H), 0.66 (d, *J=* 3.3 Hz, 3H).

**Step 3:** The compound (5*R*)-5-[(1*R*,3a*S*,3b*S*,5a*R*,6*R*,7*S*,9a*R*,9b*S*,11a*R*)-7-acetoxy-6-hydroxy-9a, 11a-dimethylhexadecahydro-1*H*-cyclopenta[1,2-*a*]phenanthren-1-yl]-1-(2-fluorophenyl)hexyl acetate **(296-2)** (90 mg, 0.158 mmol) was dissolved in ethyl acetate (5 mL), then platinum dioxide (45 mg, 0.198 mmol) was added thereto, and the resulting reaction mixture was stirred at 25°C for 3 hours under a H₂ atmosphere. The completion of the reaction was monitored by TLC (petroleum ether: ethyl acetate = 5:1). After filtration, the filtrate was concentrated to dryness to obtain a crude product, which was then purified by silica gel column chromatography (petroleum ether: ethyl acetate = 70:30) to obtain (1*R*,3a*S*,3b*S*,5a*R*,6*R*,7*S*,9a*R*,9b*S,*11a*R*)-1-[(2*R*)-6-(2-fluorophenyl)-6-[(1-hydroxyethyl)oxy]hexan-2-yl]-6-hydroxy-9a,11a-dimethylhexadecahydro-1*H-*cyclopenta[1,2-*i*]phenanthren-7-yl acetate **(296-3)** (60 mg, 0.094 mmol, purity: 90%, yield: 59.58%) as a white solid.

¹H NMR (400 MHz, CDCl₃) δ 7.34 (t, *J* = 7.4 Hz, 1H), 7.23 (s, 1H), 7.12 (s, 1H), 7.03 (m, 1H), 6.01 (t, *J* = 8.8 Hz, 1H), 4.72 (d, *J* = 11.0 Hz, 1H), 3.82 (s, 1H), 2.09 (d, *J* = 8.3 Hz, 6H), 1.92 (s, 3H), 1.75 (m, 5H), 1.36 (dd, *J* = 20.7, 11.3 Hz, 7H), 1.23 (d, *J* = 14.2 Hz, 4H), 1.10 (m, 5H), 1.04 (s, 4H), 1.00 (s, 3H), 0.87 (m,5H), 0.63 (d, *J* = 3.3 Hz, 3H).

**Step 4:** The white solid (1*R*,3a*S*,3b*S*,5a*R*,6*R*,7*S*,9a*R*,9b*S*,11a*R*)-1-[(2*R*)-6-(2-fluorophenyl)-6-[(1-hydroxyethyl)oxy]hexan-2-yl]-6-hydroxy-9a,11 a-dimethylhexadecahydro-1*H*-cyclopenta[1,2-*i*]phenanthren-7-yl acetate **(296-3)** (60 mg, 0.105 mmol) was dissolved in dichloromethane (3 mL), then Dess-Martin periodinane (66.64 mg, 0.157 mmol, 1.5 *eq*) was added thereto, and the resulting reaction mixture was stirred at 25°C for 1 hour under a N₂ atmosphere. The completion of the reaction was monitored by TLC (petroleum ether: ethyl acetate = 3:1). The reaction mixture was diluted with dichloromethane (10 mL), and sequentially washed with saturated sodium sulfite solution and saturated brine. The organic phase was dried over Na₂SO₄, filtered, and concentrated to obtain a crude product. The crude product was purified by silica gel column chromatography (petroleum ether: ethyl acetate = 70:30) to obtain (1*R*,3a*S*,3b*S*,5a*R*,7*S*,9a*R*,9b*S*,11a*R*)-1-[(2*R*)-6-(2-fluorophenyl)-6-[(1-hydroxyethyl)oxy]hexan-2-yl]-9a,11a-dimethyl-6-oxohexadecahydro-1*H*-cyclopenta[1,2-*i*]phenanthren-7-yl acetate **(296-4)** (40 mg, 0.063 mmol, purity: 40%, yield: 60.21%) as a white solid. ¹H NMR (400 MHz, CDCl₃) δ 7.33 (d, *J* = 7.7 Hz, 2H), 7.13 (d, *J* = 7.3 Hz, 1H), 7.04 (d, *J* = 9.5 Hz, 1H), 6.01 (s, 1H), 5.16 (s, 1H), 2.19 (d, *J =* 11.6 Hz, 3H), 2.15 (s, 3H), 2.08 (d, *J* = 1.7 Hz, 3H), 1.83 (m, 10H), 1.06 (m, 10H), 0.85 (m, 9H), 0.74 (s, 3H), 0.63 (d, *J =* 3.3 Hz, 3H).

**Step 5:** The white solid (1*R*,3a*S*,3b*S*,5a*R*,7*S*,9a*R*,9b*S*,11a*R*)-1-[(2*R*)-6-(2-fluorophenyl)-6-[(1-hydroxyethyl)oxy]hexan-2-yl]-9a,11 a-dimethyl-6-oxohexadecahydro-1*H*-cyclopenta[1,2-*i*]phenanthren-7-yl acetate **(296-4)** (40 mg, 0.070 mmol, 1.0 *eq*) was dissolved in a mixed solvent of methanol (4 mL) and tetrahydrofuran (2 mL), then potassium carbonate (48.42 mg, 0.350 mmol, 5.0 *eq*) was added thereto, and the resulting reaction mixture was stirred at 25°C for 1 hour. The completion of the reaction was monitored by TLC (petroleum ether: ethyl acetate = 10:1). The reaction mixture was filtered and concentrated to obtain a crude product, which was then purified by silica gel column chromatography (petroleum ether: ethyl acetate = 80:20) to obtain 24-[(2-fluorophenyl)(hydroxy)methyl]-3β-hydroxy-5α-cholan-4-one **(296)** (10.21 mg, 0.020 mmol, purity: 96.91%, yield: 29.13%) as a white solid. ¹H NMR (400 MHz, CDCl₃) δ 7.46 (t, *J* = 7.4 Hz, 1H), 7.23 (d, *J* = 6.3 Hz, 1H), 7.15 (t, *J =* 7.5 Hz, 1H), 7.02 (m, 1H), 5.00 (dd, *J* = 12.8, 5.9 Hz, 1H), 4.10 (dd, *J =* 11.0, 8.1 Hz, 1H), 2.39 (m, 1H), 2.17 (d, *J* = 11.9 Hz, 1H), 1.98 (d, *J* = 12.3 Hz, 1H), 1.88 (d, *J* = 13.5 Hz, 1H), 1.75 (m, 3H), 1.60 (m, 4H), 1.41 (ddd, *J* = 30.8, 16.4, 10.1 Hz, 5H), 1.20 (m, 6H), 1.03 (ddd, *J* = 17.8, 15.0, 8.5 Hz, 5H), 0.89 (m, 3H), 0.80 (m, 2H), 0.72 (s, 3H), 0.64 (d, *J* = 1.9 Hz, 3H). ¹³C NMR (101 MHz, CDCl₃) δ 212.67, 127.26, 127.18, 124.30, 124.26, 115.39, 115.19, 74.62, 68.65, 56.81, 56.17, 54.36, 43.27, 42.47, 39.80, 38.67, 38.44, 35.71, 35.68, 35.46, 34.90, 32.51, 30.33, 28.19, 24.11, 22.33, 22.24, 21.68, 20.43, 20.17, 18.72, 18.51, 16.78, 13.85, 12.08. ¹⁹F NMR (376 MHz, CDCl₃) δ -119.78, -119.81.

### Example 298

### Preparation of 24-[(2,6-difluorophenyl)(hydroxy)methyl]-5α-cholan-3β,4β-diol (compound 298)

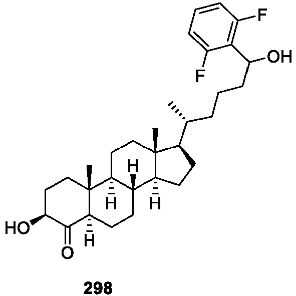

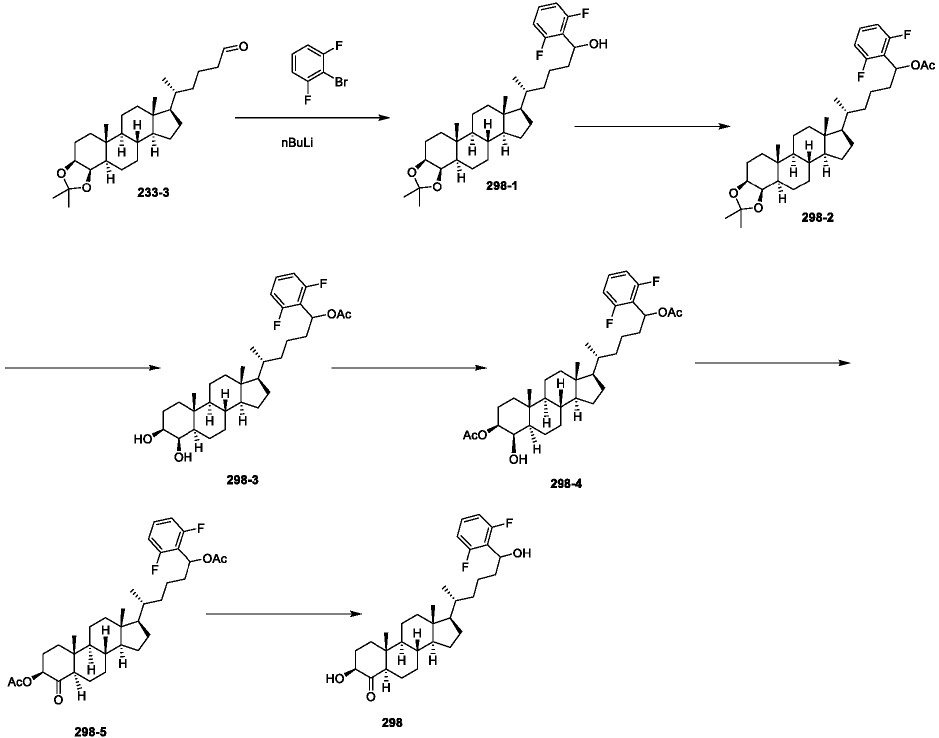

**Step 1:** The compound (5R)-5-[(3aS,5aR,5bS,7aR,SR,10aS,10bS,12aR,12bR)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]hexanal **(233-3)** (50 mg, 0.116 mmol, 1.0 *eq*) was dissolved in tetrahydrofuran (8 mL), and the reaction system was cooled to 0°C. (2,6-Difluorophenyl)lithium (2.5 mL, 0.580 mmol, 5 *eq*) was then slowly added thereto, and the reaction system was stirred at room temperature for 1 hour. The completion of the reaction was monitored by TLC (petroleum ether: ethyl acetate = 5:1, heating with phosphomolybdic acid). The reaction system was added with water (20 mL) and extracted with ethyl acetate (50 mL × 3). The organic phases were combined, washed with water (30 mL), dried, and concentrated to obtain a crude product. The crude product was subjected to silica gel column chromatography (petroleum ether: ethyl acetate = 94:6) to obtain (5*R*)-5-[(3a*S*,5a*R*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]-1-(2,6-difluorophenyl)hexan-1-ol **(298-1)** (25 mg, purity: 90%, yield: 35.6%) as a white solid. ¹H NMR (400 MHz, CDCl₃) δ 7.20 (td, *J =* 8.3, 4.1 Hz, 1H), 6.87 (t, *J =* 8.4 Hz, 2H), 5.03 (t, *J =* 7.2 Hz, 1H), 4.06 - 3.92 (m, 2H), 1.97 (dd, *J* = 23.5, 9.5 Hz, 2H), 1.81 - 1.62 (m, 7H), 1.51 (s, 3H), 1.43 - 1.33 (m, 5H), 1.30 (s, 2H), 1.27 - 1.19 (m, 4H), 1.10 (dd, *J* = 18.7, 6.3 Hz, 3H), 1.04 (s, 3H), 1.01 - 0.92 (m, 2H), 0.87 (t, *J =* 6.2 Hz, 4H), 0.65 (d, *J =* 3.8 Hz, 3H), 0.58 (dd, *J =* 15.5, 7.2 Hz, 1H).

**Step 2:** (5*R*)-5-[(3a*S*,5a*R*,5b*S*,7a*R*,8*R*,10a*S*,10b*S*,12a*R*,12b*R*)-2,2,5a,7a-Tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]-1-(2,6-difluorophenyl)hexan-1-ol (298-1) (120 mg, 0.22 mmol, 1.0 *eq*) was dissolved in dichloromethane (10 mL), and the reaction system was cooled to about 5°C in an ice-water bath. Acetic anhydride (0.06 mL, 0.66 mmol, 3.0 *eq*), 4-dimethylaminopyridine (5.38 mg, 0.044 mmol, 0.2 *eq*), and triethylamine (0.09 mL, 0.66 mmol, 3.0 *eq*) were sequentially added thereto, and the reaction system was stirred at room temperature for 1 hour. The completion of the reaction was monitored by TLC (petroleum ether: ethyl acetate = 5:1). The reaction system was added with water (20 mL) and extracted with ethyl acetate (50 mL × 3). The organic phases were combined, washed with water (30 mL), dried, and concentrated to obtain a crude product. The crude product was subjected to silica gel column chromatography (petroleum ether: ethyl acetate = 95:5) to obtain (5*R*)-5-[(3a*S*,5a*R*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]-1-(2,6-difluorophenyl)hexyl acetate **(298-2)** (110 mg, purity: 90.0%, yield: 76.6%) as a white solid. ¹H NMR (400 MHz, DMSO) δ 7.41 (dd, *J* = 14.6, 6.8 Hz, 1H), 7.10 (t, *J* = 8.4 Hz, 2H), 5.92 (t, *J* = 7.4 Hz, 1H), 3.93 (s, 2H), 2.00 (s, 3H), 1.89 (d, *J =* 10.9 Hz, 2H), 1.73 - 1.45 (m, 7H), 1.39 (s, 3H), 1.36 - 1.22 (m, 6H), 1.19 (s, 3H), 1.09 - 0.99 (m, 4H), 0.97 (s, 3H), 0.81 (dd, *J =* 9.5, 6.7 Hz, 3H), 0.60 (s, 3H).

**Step 3:** The reactant (5*R*)-5-[(3a*S*,5a*R*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]-1-(2,6-difluorophenyl)hexyl acetate **(298-2)** (110 mg, 0.187 mmol, 1.0 *eq*) was dissolved in tetrahydrofuran (8 mL), then dilute hydrochloric acid (0.8 mL, 3 mol/L) was added thereto, and the reaction system was stirred at room temperature for 4 hours. The completion of the reaction was monitored by TLC (petroleum ether: ethyl acetate = 2:1). The reaction system was added with water (10 mL), and the aqueous phase was extracted with ethyl acetate (25 mL × 3). The ethyl acetate phases were combined and washed with saturated brine (10 mL × 3). The ethyl acetate phase was dried over anhydrous sodium sulfate, filtered, and concentrated to obtain a crude product. The crude product was purified by column chromatography (petroleum ether: ethyl acetate = 68:32) to obtain (5*R*)-1-(2,6-difluorophenyl)-5-[(1*R*,3a*S*,3b*S*,5a*R*,6*R*,7*S*,9a*R*,9b*S*,11a*R*)-6,7-dihydroxy-9a,11a-dimethylhexadecahydro-1*H*-cyclopenta[1,2-*a*]phenanthren-1-yl]hexyl acetate **(298-3)** (100 mg, purity: 90%, yield: 87.8%) as a white solid. ¹H NMR (400 MHz, CDCl₃) δ 7.22 (dt, *J =* 13.3, 6.6 Hz, 1H), 6.86 (t, *J =* 8.2 Hz, 2H), 6.04 (t, *J =* 7.5 Hz, 1H), 3.73 (s, 1H), 3.58 - 3.51 (m, 1H), 2.06 (s, 3H), 1.93 (d, *J =* 11.9 Hz, 1H), 1.82 - 1.52 (m, 12H), 1.36 (dd, *J* = 23.9, 13.6 Hz, 6H), 1.11 - 1.02 (m, 5H), 1.01 (s, 4H), 0.98 - 0.89 (m, 3H), 0.88 - 0.82 (m, 4H), 0.63 (d, *J* = 3.9 Hz, 3H), 0.61 - 0.54 (m, 1H).

**Step 4:** (5*R*)-1-(2,6-Difluorophenyl)-5-[(1*R*,3a*S*,3b*S*,5a*R*,6*R*,7*S*,9a*R*,9b*S*,11a*R*)-6,7-dihydroxy-9a,11a-dimethylhexadecahydro-1*H*-cyclopenta[1,2-*a*]phenanthren-1-yl]hexyl acetate **(298-3)** (100 mg, 0.18 mmol, 1.0 *eq*) was dissolved in dichloromethane (6 mL), and the reaction system was cooled to about 5°C in an ice-water bath. Acetic anhydride (0.017 mL, 0.183 mmol, 1.0 *eq*), 4-dimethylaminopyridine (4 mg, 0.04 mmol, 0.2 *eq*), and triethylamine (0.05 mL, 0.366 mmol, 2.0 *eq*) were sequentially added thereto, and the reaction system was stirred at room temperature for 1 hour. The completion of the reaction was monitored by TLC (petroleum ether: ethyl acetate = 5:1). The reaction system was added with water (20 mL) and extracted with ethyl acetate (50 mL × 3). The organic phases were combined, washed with water (30 mL), dried, and concentrated to obtain a crude product. The crude product was subjected to silica gel column chromatography (petroleum ether: ethyl acetate = 80:20) to obtain (5*R*)-5-[(1*R*,3a*S*,3b*S*,5a*R*,6*R*,7*S*,9a*R*,9b*S*,11a*R*)-7-acetoxy-6-hydroxy-9a,11a-dimethylhexadecahydro-1*H*-cyclopenta[1,2-*a*]phenanthren-1-yl]-1-(2,6-difluorophenyl)hexyl acetate **(298-4)** (90 mg, purity: 95.0%, yield: 79.4%) as a white solid. ¹H NMR (400 MHz, CDCl₃) δ 7.22 (dd, *J =* 14.5, 7.6 Hz, 1H), 6.86 (t, *J =* 8.2 Hz, 2H), 6.04 (t, *J =* 7.4 Hz, 1H), 4.75 - 4.68 (m, 1H), 3.82 (s, 1H), 2.08 (s, 3H), 2.06 (s, 3H), 1.97 - 1.62 (m, 12H), 1.43 - 1.08 (m, 16H), 1.04 (s, 4H), 1.02 - 0.94 (m, 3H), 0.85 (t, *J =* 6.9 Hz, 3H), 0.63 (d, *J =* 3.8 Hz, 3H), 0.59 (d, *J =* 11.3 Hz, 1H).

**Step 5:** The compound (5*R*)-5-[(3a*S*,5a*R*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]-1-(2,6-difluorophenyl)hexyl acetate **(298-4)** (90 mg, 0.15 mmol) was dissolved in dichloromethane (6 mL). Tetrapropylammonium perruthenate (16 mg, 0.05 mmol, 0.3 *eq*), *N*-methylmorpholine oxide (27 mg, 0.23 mmol, 1.5 *eq*), and 4A molecular sieve were sequentially added thereto under a nitrogen atmosphere, and the reaction mixture was stirred at room temperature for 3 hours. The reaction was monitored by TLC (petroleum ether: ethyl acetate = 5:1). The reaction system was added with water (10 mL), and the aqueous phase was extracted with ethyl acetate (25 mL × 3). The ethyl acetate phases were combined and washed with saturated brine (10 mL × 3). The ethyl acetate phase was dried over anhydrous sodium sulfate, filtered, and concentrated to obtain a crude product. After the reaction was completed, the reaction mixture was filtered, and the filtrate was washed with water. The organic phase was dried, concentrated, and the crude product was purified by flash column chromatography (petroleum ether: ethyl acetate = 84:16) to obtain (6*R*)-6-[(1*R*,3a*S*,3b*S*,5a*R*,7*S*,9a*R*,9b*S*,11a*R*)-7-acetoxy-9a,11a-dimethyl-6-oxohexadecahydro-1*H*-cyclopenta[1,2-*a*]phenanthren-1-yl]-2-methylheptan-2-yl acetate **(298-5)** (85 mg, purity: 95%, yield: 90%) as a white solid.

¹H NMR (400 MHz, CDCl₃) δ 7.22 (dd, *J =* 14.7, 7.5 Hz, 1H), 6.86 (t, *J =* 8.2 Hz, 2H), 6.04 (t, *J* = 7.5 Hz, 1H), 5.16 (dd, *J =* 12.3, 7.3 Hz, 1H), 2.26 - 2.17 (m, 2H), 2.15 (s, 3H), 2.06 (s, 3H), 2.00 - 1.70 (m, 7H), 1.61 (d, *J =* 14.3 Hz, 1H), 1.53 - 1.39 (m, 4H), 1.35 - 1.13 (m, 7H), 1.08 - 0.89 (m, 5H), 0.86 (t, *J =* 6.9 Hz, 3H), 0.80 (dd, *J =* 12.1, 4.0 Hz, 1H), 0.74 (s, 3H), 0.63 (d, *J =* 3.8 Hz, 3H).

**Step 6:** The reactant (6*R*)-6-[(1*R*,3a*S*,3b*S*,5a*R*,7*S,*9a*R*,9b*S*,11a*R*)-7-acetoxy-9a,11a-dimethyl-6-oxohexadecahydro-1*H*-cyclopenta[1,2-*a*]phenanthren-1-yl]-2-methylheptan-2-yl acetate **(298-5)** (40 mg, 0.07 mmol, 1.0 *eq*) was dissolved in methanol (2 mL) and tetrahydrofuran (2 mL), then potassium carbonate (242 mg, 0.73 mmol, 10.0 *eq*) was added thereto, and the reaction system was stirred at room temperature for 10 minutes. The completion of the reaction was monitored by TLC plate (petroleum ether: ethyl acetate = 5:1). The reaction system was added with water (10 mL), and the aqueous phase was extracted with ethyl acetate (25 mL × 3). The ethyl acetate phases were combined and washed with saturated brine (10 mL × 3). The ethyl acetate phase was dried over anhydrous sodium sulfate, filtered, and concentrated to obtain a crude product. The crude product was purified by column chromatography (petroleum ether: ethyl acetate = 80:20) to obtain 24-[(2,6-difluorophenyl)(hydroxy)methyl]-5α-cholan-3β,4β-diol **(298)** (19.15 mg, purity: 100%, yield: 51.86%) as a white solid. ¹H NMR (400 MHz, CDCl₃) δ 7.20 (dd, *J* = 14.7, 6.5 Hz, 1H), 6.91 - 6.83 (m, 2H), 5.03 (t, *J* = 6.4 Hz, 1H), 4.10 (dd, *J* = 10.9, 8.1 Hz, 1H), 2.43 - 2.35 (m, 1H), 2.17 (d, *J* = 11.0 Hz, 1H), 2.03 - 1.72 (m, 8H), 1.68 - 1.53 (m, 9H), 1.41 - 1.32 (m, 3H), 1.06 (ddd, *J* = 16.7, 16.1, 8.6 Hz, 5H), 0.88 (t, *J =* 6.1 Hz, 3H), 0.81 (dd, *J =* 12.2, 4.0 Hz, 1H), 0.72 (s, 3H), 0.64 (d, *J* = 3.6 Hz, 3H). ¹³C NMR (101 MHz, CDCl₃) δ 212.70, 128.97, 119.81, 111.86, 111.79, 111.67, 74.62, 66.74, 56.81, 56.16, 56.09, 54.34, 43.37, 42.58, 39.83, 37.83, 37.73, 35.63, 35.57, 35.46, 35.40, 34.90, 32.51, 30.28, 28.17, 24.11, 22.58, 22.49, 21.68, 20.17, 18.54, 18.50, 13.85, 12.06. LCMS [M+H]⁺ = 503.70.

### Example 299

### Preparation of 7-amino-24-[(2,6-difluorophenyl)(hydroxy)methyl]-5α-cholan-3β,4β-diol (compound 299)

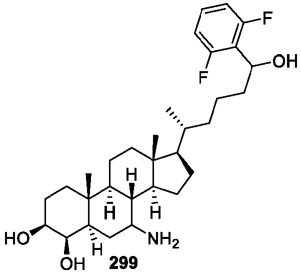

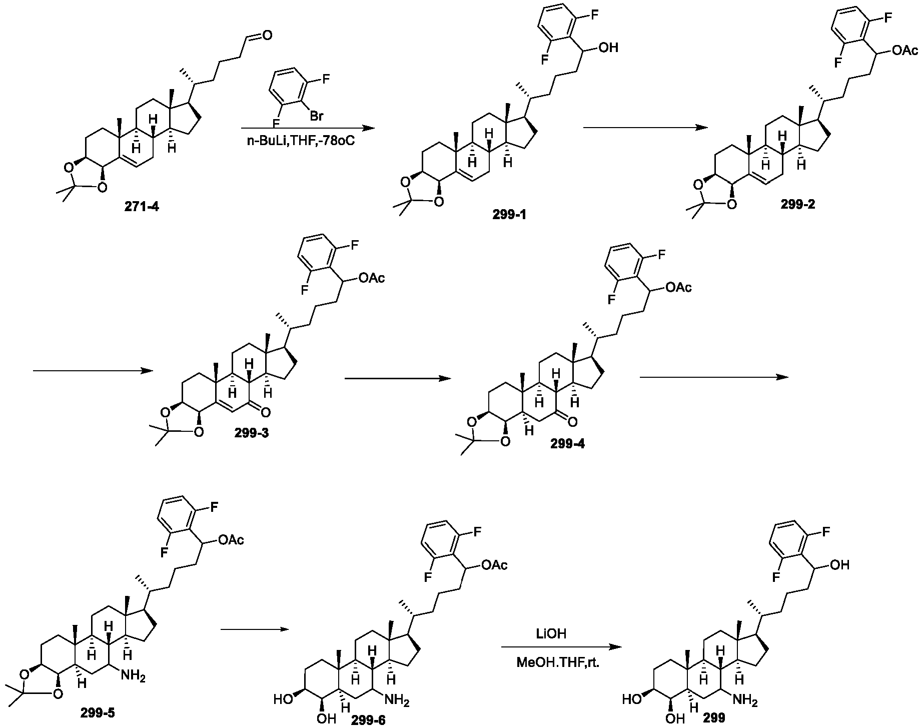

**Step 1:** The reagent 2-bromo-1,3-difluorobenzene (270 mg, 1.40 mmol, 3.0 *eq*) was dissolved in tetrahydrofuran (5 mL), and the reaction mixture was cooled to -78°C in a dry ice bath under a nitrogen atmosphere. A 2.5 M solution of *n*-butyllithium in *n*-hexane (0.67 mL, 1.680 mmol, 3.6 *eq*) was then added thereto, and the reaction mixture was reacted for 0.5 hours. A solution of (5*R*)-5-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]hexanal **(271-4)** (200 mg, 0.467 mmol, 1.0 *eq*) in tetrahydrofuran (10 mL) was then added thereto. The completion of the reaction was monitored by TLC (petroleum ether: ethyl acetate = 5:1). The reaction mixture was quenched with saturated ammonium chloride and extracted with ethyl acetate. The organic phase was dried over anhydrous sodium sulfate, and the crude product was purified by flash column chromatography (petroleum ether: ethyl acetate = 80:20) to obtain (5R)-5-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1-(2,6-difluorophenyl)hexan-1-ol **(299-1)** (180 mg, 0.03 mmol, purity: 90%, yield: 63.97%) as a white solid. ¹H NMR (400 MHz, ) δ 7.21 (s, 1H), 6.87 (t, *J =* 8.2 Hz, 2H), 5.80 (d, *J =* 2.7 Hz, 1H), 5.03 (s, 1H), 4.40 (d, *J* = 5.8 Hz, 1H), 4.10 (d, *J* = 7.4 Hz, 1H), 2.12 (m,1H), 2.00 (d, *J* = 13.4 Hz, 2H), 1.69 (m, 10H), 1.39 (m, 8H), 1.08 (m, 10H), 0.90 (dd, *J =* 14.8, 8.6 Hz, 5H), 0.68 (d, *J =* 3.9 Hz, 3H).

**Step 2:** The compound (5*R*)-5-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1-(2,6-difluorophenyl)hexan-1-ol **(299-1)** (180 mg, 0.332 mmol, 1.0 *eq*) was dissolved in dichloromethane (10 mL). Acetic anhydride (0.093 mL, 0.995 mmol, 3.0 *eq*), triethylamine (0.230 mL, 1.658 mmol, 5.0 *eq*), and DMAP (8.10 mg, 0.066 mmol, 0.2 *eq*) were added thereto, and the resulting reaction mixture was stirred at 25°C for 1 hour. The completion of the reaction was monitored by TLC (petroleum ether: ethyl acetate = 5:1). The reaction mixture was quenched with methanol, washed with saturated sodium bicarbonate and saturated brine (15 mL), dried over Na₂SO₄, filtered, and concentrated to obtain a crude product, which was then purified by silica gel column chromatography (petroleum ether: ethyl acetate = 90:10) to obtain (5R)-5-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1-(2,6-difluorophenyl)hexyl acetate **(299-2)** (150 mg, 0.231 mmol, purity: 90%, yield: 69.61%) as a white solid. ¹H NMR (400 MHz, CDCl₃) δ 7.23 (m, 1H), 6.86 (t, *J =* 8.0 Hz, 2H), 6.05 (t, *J =* 7.5 Hz, 1H), 5.80 (d, *J =* 2.6 Hz, 1H), 4.40 (d, *J =* 5.8 Hz, 1H), 4.10 (dd, *J* = 13.7, 6.2 Hz, 1H), 2.11 (dd, *J* = 12.6, 4.2 Hz, 1H), 2.06 (s, 3H), 2.01 (m, 2H), 1.77 (m, 3H), 1.61 (m, 6H), 1.41 (d, *J=* 12.5 Hz, 4H), 1.30 (m, 6H), 1.06 (m, 11H), 0.87 (m, 5H), 0.67 (d, *J* = 4.0 Hz, 3H).

**Step 3:** The compound (5*R*)-5-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1-(2,6-difluorophenyl)hexyl acetate (299-2) (150 mg, 0.257 mmol, 1.0 *eq)* was dissolved in acetone (5 mL). *N-*Hydroxyphthalimide (8.37 mg, 0.051 mmol, 0.2 *eq*), *tert*-butyl hydroperoxide (0.205 mL, 1.026 mmol, 4.0 *eq*), and anhydrous cobalt (II) acetate (2.27 mg, 0.013 mmol, 0.05 *eq*) were added thereto, and the resulting reaction mixture was stirred at 25°C for 18 hours under a N₂ atmosphere. The completion of the reaction was monitored by TLC (petroleum ether: ethyl acetate = 10:1). The reaction system was quenched with saturated sodium sulfite, added with water (10 mL), and the aqueous phase was extracted with ethyl acetate (15 mL × 3). The organic phases were combined, washed with saturated brine (15 mL), dried over Na₂SO₄, filtered, and concentrated to obtain a crude product. The crude product was purified by silica gel column chromatography (petroleum ether: ethyl acetate = 80:20) to obtain (5R)-5-[(3a*S*,5a*R*,5b*S*,7a*R*,8*R*,10a*S*,10b*S*,12b*R*)-2,2,5a,7a-tetramethyl-11-oxo-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1-(2,6-difluorophenyl)hexyl acetate **(299-3)** (90 mg, 0.135 mmol, purity: 90%, yield: 52.74%) as a white solid. ¹H NMR (400 MHz, CDCl₃) δ 7.23 (s, 1H), 6.86 (t, *J* = 8.2 Hz, 2H), 6.04 (t, *J* = 7.4 Hz, 1H), 5.92 (s, 1H), 4.52 (d, *J =* 6.4 Hz, 1H), 4.33 (d, *J =* 5.6 Hz, 1H), 2.34 (d, *J =* 10.5 Hz, 2H), 2.06 (s, 3H), 1.82 (m, 3H), 1.63 (dd, *J* = 15.0, 9.3 Hz, 3H), 1.56 (d, *J* = 5.4 Hz, 8H), 1.37 (s, 4H), 1.28 (m, 9H), 1.07 (m, 4H), 0.87 (dd, *J* = 12.8, 6.4 Hz, 4H), 0.69 (d, *J* = 3.9 Hz, 3H).

**Step 4:** The compound (5*R*)-5-[(3a*S*,5a*R*,5b*S*,7a*R*,8*R*,10a*S*,10b*S*,12b*R*)-2,2,5a,7a-tetramethyl-11-oxo-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1-(2-fluorophenyl)hexyl acetate **(299-3)** (90 mg, 0.150 mmol) was dissolved in methanol (5 mL), and Pd/C (10.99 mg) was added thereto. The reaction system was replaced with hydrogen three times, and the resulting reaction mixture was stirred at 25°C for 1 hour under a H₂ atmosphere. The completion of the reaction was monitored by TLC (petroleum ether: ethyl acetate = 3:1). The reaction mixture was filtered and concentrated to obtain a crude product. The crude product was purified by silica gel column chromatography (petroleum ether: ethyl acetate = 80:20) to obtain (5*R*)-5-[(3a*S*,5a*R*,5b*S*,7a*R*,4*R*,10a*S*,10b*S*,12a*R*,12b*R*)-2,2,5a,7a-tetramethyl-11-oxo-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]-1-(2,6-difluorophenyl)hexyl acetate **(299-4)** (50 mg, 0.067 mmol, purity: 80%, yield: 44.30%). ¹H NMR (400 MHz, CDCl₃) δ 7.34 (s, 1H), 7.23 (s, 1H), 7.12 (s, 1H), 7.03 (s, 1H), 6.01 (dd, *J =* 10.9, 6.4 Hz, 1H), 3.98 (d, *J* = 3.4 Hz, 2H), 2.77 (s, 1H), 2.42 (s, 1H), 2.19 (dd, *J =* 12.7, 2.7 Hz, 2H), 2.08 (s, 3H), 1.74 (m, 9H), 1.53 (s, 3H), 1.38 (dd, *J* = 11.7, 7.8 Hz, 4H), 1.28 (d, *J* = 17.1 Hz, 10H), 0.95 (ddd, *J* = 10.8, 9.5, 6.5 Hz, 10H), 0.64 (d, *J =* 3.2 Hz, 3H).

**Step 5:** The compound (5*R*)-5-[(3a*S*,5a*R*,5b*S*,7a*R*,4*R*,10a*S*,10b*S*,12a*R*,12b*R*)-2,2,5a,7a-tetramethyl-11-oxo-4,5,5a,5b,6,7,7a,8,9,10,10a,10b,11,12,12a,12b-hexadecahydro-3a*H*-cyclopenta[1',2':1,2]phenanthro[7,8-*d*][1,3]dioxol-8-yl]-1-(2,6-difluorophenyl)hexyl acetate **(299-4)** (85 mg, 0.141 mmol, 1.0 *eq*) was dissolved in a mixed solvent of tetrahydrofuran (2 mL) and methanol (1 mL). A catalytic amount of acetic acid (0.001 mL, 0.014 mmol) and acetamide (83.29 mg, 1.410 mmol, 10 *eq*) were added thereto, and the resulting reaction mixture was stirred at 25°C for 1 hour under a N₂ atmosphere. Sodium cyanoborohydride (88.60 mg, 1.410 mmol, 10 *eq*) was then added thereto, and the reaction mixture was stirred for another 2 hours The completion of the reaction was monitored by TLC (petroleum ether: ethyl acetate = 5:1). The reaction mixture was quenched with water, and the aqueous phase was extracted with dichloromethane (10 mL × 3). The dichloromethane phases were combined, washed with saturated brine (5 mL), dried over anhydrous Na₂SO₄, filtered, and concentrated to obtain a crude product, which was then purified by silica gel column chromatography (dichloromethane: methanol = 10:1) to obtain (5R)-5-[(3a*S*,5a*R*,5b*S*,7a*R*,8*R*,10a*S*,10b*S*,12a*R*,12b*R*)-11-amino-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]-1-(2,6-difluorophenyl)hexyl acetate **(299-5)** (40 mg, 0.047 mmol, purity: 70%, yield: 32.88%) as a white solid.

**Step 6:** The compound (5*R*)-5-[(3a*S*,5a*R*,5b*S*,7a*R*,8*R*,10a*S*,10b*S*,12a*R*,12b*R*)-11-amino-2,2,5a,7a-tetramethyl-4,5,5a,5b,6,7,7a,8,9,10,10a,10b,11,12,12a,12b-hexadecahydro-3a*H*-cyclopenta[1',2':1,2]phenanthro[7,8-*d*][1,3]dioxol-8-yl]-1-(2,6-difluorophenyl)hexyl acetate **(299-5)** (40 mg, 0.066 mmol) was dissolved in tetrahydrofuran (3 mL), then 3 N dilute hydrochloric acid (0.5 mL) was added thereto, and the resulting reaction mixture was stirred at 25°C for 1 hour. The completion of the reaction was monitored by TLC (dichloromethane: methanol = 10:1). The reaction system was added with water (3 mL), and the aqueous phase was extracted with dichloromethane (15 mL × 3). The dichloromethane phases were combined, washed with saturated brine (15 mL), dried over anhydrous Na₂SO₄, filtered, and concentrated to obtain a crude product. The crude product was subjected to silica gel column chromatography (dichloromethane: methanol = 10:1) to obtain (5R)-5-[(1*R*,3a*S*,3b*S*,5a*R*,6*R*,7*S*,9a*R*,9b*S*,11a*R*)-4-amino-6,7-dihydroxy-9a,11a-dimethylhexadecahydro-1*H*-cyclopenta[1,2-*a*]phenanthren-1-yl]-1-(2,6-difluorophenyl)hexyl acetate **(299-6)** (30 mg, 0.048 mmol, purity: 90%, yield: 72.31%) as a white solid. LCMS: [M+H]⁺ = 562.3, tR = 1.20 min.

**Step 7:** The compound (5*R*)-5-[(1*R*,3a*S*,3b*S*,5a*R*,6*R,*7*S,*9a*R,*9b*S*,11a*R*)-4-amino-6,7-dihydroxy-9a,11a-dimethylhexadecahydro-1*H*-cyclopenta[1,2-*a*]phenanthren-1-yl]-1-(2,6-difluorophenyl)hexyl acetate **(299-6)** (30 mg, 0.053 mmol) was dissolved in methanol (1 mL) and tetrahydrofuran (1 mL), then lithium hydroxide solution (0.5 mL, 0.500 mmol) was added thereto, and the resulting reaction mixture was stirred at 25°C for 1 hour. The completion of the reaction was monitored by TLC (dichloromethane: methanol = 10:1). The reaction system was added with water (10 mL), and the aqueous phase was extracted with ethyl acetate (10 mL × 3). The ethyl acetate phases were combined and washed with saturated brine (5 mL). The ethyl acetate phase was dried over Na₂SO₄, filtered, and concentrated to obtain a crude product. The crude product was purified by silica gel column chromatography (dichloromethane: methanol = 10:1) to obtain 7-amino-24-[(2,6-difluorophenyl)(hydroxy)methyl]-5α-cholan-3β,4β-diol **(299)** (20 mg, 0.037 mmol, 69.83%) as a white solid. ¹H NMR (400 MHz, MeOD) δ 7.29 (m, 1H), 6.93 (t, *J* = 8.4 Hz, 2H), 5.02 (t, *J* = 7.3 Hz, 1H), 3.60 (s, 1H), 3.50 (d, *J* = 15.7 Hz, 1H), 3.15 (s, 1H), 2.12 (dd, *J* = 14.3, 10.4 Hz, 1H), 1.93 (m, 4H), 1.72 (m, 4H), 1.36 (m, 10H), 1.06 (s, 10H), 0.89 (dd, *J =* 9.5, 6.5 Hz, 3H), 0.69 (d, *J =* 1.5 Hz, 3H). ¹³C NMR (101 MHz, MeOD) δ 167.28, 162.44, 123.23, 116.95, 113.96, 107.29, 72.66, 67.84, 55.83, 53.51, 50.45, 45.79, 44.06, 43.63, 40.15, 35.74, 31.64, 26.83, 26.25, 23.70, 22.62, 20.44, 19.48, 18.07, 17.55, 14.22, 11.16, 8.80, 7.48, 5.75, 5.53, 3.80.

¹⁹F NMR (377 MHz, MeOD) δ -116.32 (d, *J* = 18.4 Hz, 1H). LCMS: [M+H]⁺ = 520.3.

### Example 300

### Preparation of (1R,3aS,3bS,5aR,6R,7S,9aR,9bS,11aR)-1-[(2R)-6-(2,6-difluorophenyl)-6-hydroxyhexan-2-yl]-4,4-difluoro-9a,11a-dimethylhexadecahydro-1H-cyclopenta[1,2-a]phenanthrene-6,7-diol (compound 300)

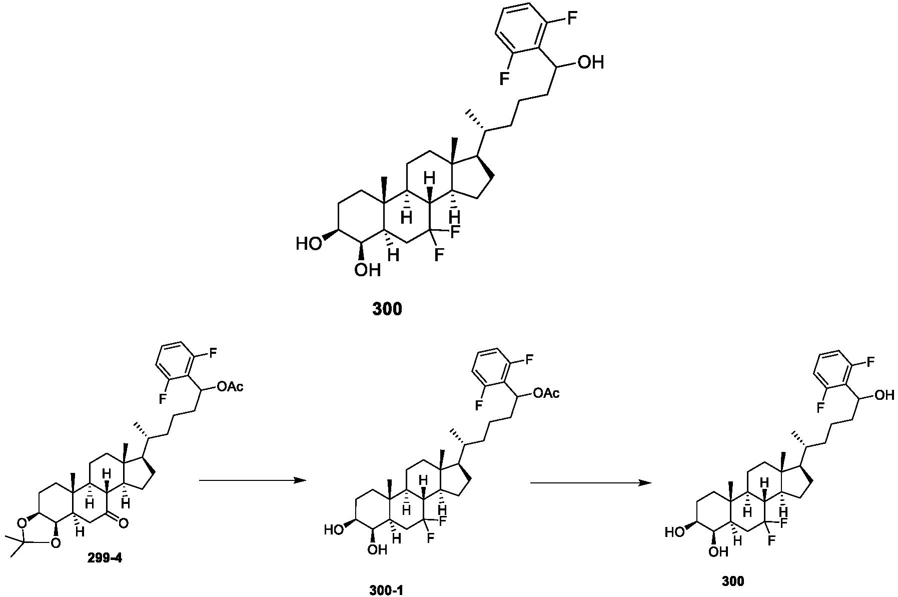

**Step 1:** The compound (5*R*)-5-[(3a*S*,5a*R*,5b*S*,7a*R*,4*R*,10a*S*,10b*S*,12a*R*,12b*R*)-2,2,5a,7a-tetramethyl-11-oxo-4,5,5a,5b,6,7,7a,8,9,10,10a,10b,11,12,12a,12b-hexadecahydro-3a*H*-cyclopenta[1',2':1,2]phenanthro[7,8-*d*][1,3]dioxol-8-yl]-1-(2,6-difluorophenyl)hexyl acetate **(299-4)** (200 mg, 0.333 mmol) was dissolved in DAST (0.5 mL), and the resulting reaction mixture was stirred at 80°C for 3 hours under a N₂ atmosphere. The completion of the reaction was monitored by TLC plate (petroleum ether: ethyl acetate = 5:1). The reaction mixture was cooled to room temperature, carefully added dropwise to an ice-water mixture, and the aqueous phase was extracted with ethyl acetate (10 mL × 3). The ethyl acetate phases were combined and washed with saturated brine (15 mL). The organic phase was dried over Na₂SO₄, filtered, and concentrated to obtain a crude product, whose TLC plate (petroleum ether: ethyl acetate = 1:1) showed that the small polar spot changed to a large polar spot, indicating that the propylidene protecting group was removed. The crude product was subj ected to silica gel column chromatography (petroleum ether: ethyl acetate = 1:1) to obtain (5R)-5-[(1*R*,3a*S*,3b*S,*5a*R*,6*R*,7*S*,9a*R*,9b*S*,11a*R*)-4,4-difluoro-6,7-dihydroxy-9a,11a-dimethylhexadecahydro-1*H*-cyclopenta[1,2-*a*]phenanthren-1-yl]-1-(2,6-difluorophenyl)hexyl acetate **(300-1)** (120 mg, 0.185 mmol, purity: 90%, yield: 55.66%) as a colorless transparent solid.

**Step 2:** The compound (5*R*)-5-[(1*R*,3a*S*,3b*S*,5a*R*,6*R*,7*S*,9a*R*,9b*S,*11a*R*)-4,4-difluoro-6,7-dihydroxy-9a,11a-dimethylhexadecahydro-1*H*-cyclopenta[1,2-*a*]phenanthren-1-yl]-1-(2,6-difluorophenyl)hexyl acetate **(300-1)** (40 mg, 0.069 mmol) was dissolved in a mixed solvent of tetrahydrofuran (3 mL) and methanol (3 mL), then 1 mol/L lithium hydroxide solution (0.5 mL) was added thereto, and the resulting mixture was stirred at 25°C for 1 hour. The completion of the reaction was monitored by TLC (petroleum ether: ethyl acetate = 1:1). The reaction mixture was diluted with ethyl acetate (20 mL) and washed with saturated brine. The organic phase was dried over anhydrous sodium sulfate, filtered, and the filtrate was concentrated to obtain a crude product, which was then purified by silica gel column chromatography (petroleum ether: ethyl acetate = 0:40) to obtain (1*R*,3a*S*,3b*S*,5a*R*,6*R*,7*S*,9a*R*,9b*S*,11a*R*)-1-[(2*R*)-6-(2,6-difluorophenyl)-6-hydroxyhexan-2-yl]-4,4-difluoro-9a,11a-dimethylhexadecahydro-1*H*-cyclopenta[1,2-*a*]phenanthrene-6,7-diol (300) (17.9 mg, 0.033 mmol, purity: 100%, yield: 48.23%) as a white solid. ¹H NMR (400 MHz, CDCl₃) δ 7.21 (m, 1H), 6.87 (t, *J =* 8.3 Hz, 2H), 5.04 (d, *J =* 7.2 Hz, 1H), 3.74 (s, 1H), 3.60 (d, *J =* 11.3 Hz, 1H), 2.18 (m, 1H), 1.98 (m, 2H), 1.81 (m, 6H), 1.68 (m, 3H), 1.37 (ddd, *J =* 42.4, 21.9, 12.6 Hz, 10H), 1.11 (dd, *J* = 13.2, 4.6 Hz, 2H), 1.06 (s, 3H), 0.98 (m, 2H), 0.89 (t, *J=* 6.0 Hz, 3H), 0.65 (d, *J* = 3.7 Hz, 3H). ¹³C NMR (101 MHz, CDCl₃) δ 164.65, 163.79, 137.73, 131.82, 130.78, 111.87, 111.61, 73.43, 71.94, 70.56, 55.18, 50.84, 43.10, 42.13, 41.93, 39.24, 37.87, 37.75, 36.23, 35.59, 35.39, 34.94, 28.98, 28.44, 25.63, 20.52, 18.65, 18.61, 18.47, 13.78, 11.84. ¹⁹F NMR (377 MHz, CDCl₃) δ -88.64, -89.27, -89.29, -110.63, -111.26, -115.38, - 115.40, -115.46.

### Example 301

### Preparation of (1R,3aS,3bS,5aR,7S,9aR,9bS,11aR)-1-[(2R)-6-(2,6-difluorophenyl)-6-hydroxyhexan-2-yl]-4,4-difluoro-7-hydroxy-9a,11a-dimethylhexadecahydro-1H-cyclopenta[1,2-a]phenanthren-6-one (compound 301)

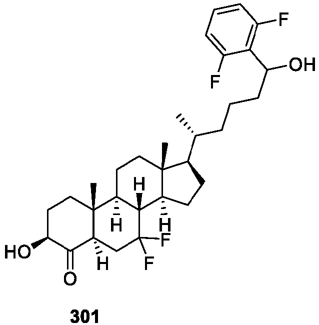

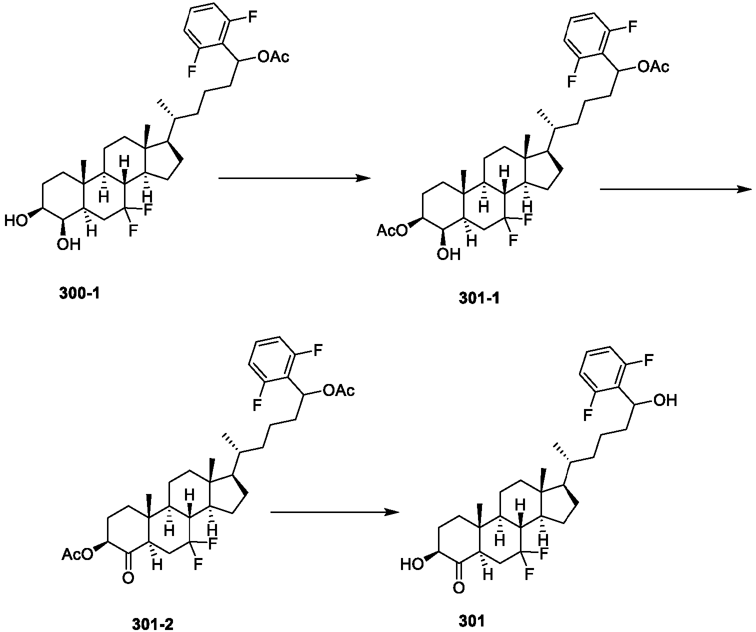

**Step 1:** The compound (5*R*)-5-[(1*R*,3a*S*,3b*S*,5a*R*,6*R*,7*S*,9a*R*,9b*R*,11a*S*)-4,4-difluoro-6,7-dihydroxy-9a,11a-dimethylhexadecahydro-1*H*-cyclopenta[1,2-*a*]phenanthren-1-yl]-1-(2-fluoro-6-methylphenyl)hexyl acetate **(300-1)** (90 mg, 0.154 mmol, 1.0 *eq)* was dissolved in dichloromethane (5 mL). Acetic anhydride (0.016 mL, 0.170 mmol, 1.1 *eq*), triethylamine (0.043 mL, 0.309 mmol, 2.0 *eq*), and 4-dimethylaminopyridine (3.77 mg, 0.031 mmol, 0.2 *eq*) were added thereto, and the resulting reaction mixture was stirred at 25°C for 3 hours. The completion of the reaction was monitored by TLC (petroleum ether: ethyl acetate = 5:1). The reaction system was quenched with methanol, added with water (10 mL), and the aqueous phase was extracted with dichloromethane (15 mL × 3). The organic phases were combined, washed with saturated brine (5 mL), dried over Na₂SO₄, filtered, and concentrated to obtain a crude product. The crude product was purified by silica gel column chromatography (petroleum ether: ethyl acetate = 70:30) to obtain (5*R*)-5-[(1*R*,3a*S*,3b*S*,5a*R*,6*R*,7*S*,9a*R*,9b*S*,11a*R*)-7-acetoxy-4,4-difluoro-6-hydroxy-9a,11a-dimethylhexadecahydro-1*H*-cyclopenta[1,2-*a*]phenanthren-1-yl]-1-(2,6-difluorophenyl)hexyl acetate **(301-1)** (50 mg, 0.072 mmol, purity: 90%, yield: 46.64%) as a white solid. ¹H NMR (400 MHz, CDCl₃) δ 7.24 (m, 1H), 6.86 (t, *J* = 8.3 Hz, 2H), 6.04 (s, 1H), 4.72 (m, 1H), 3.83 (s, 1H), 2.09 (s, 3H), 2.05 (d, *J =* 5.1 Hz, 3H), 1.82 (m, 10H), 1.43 (m, 5H), 1.29 (m, 6H), 1.08 (m, 9H), 0.87 (dd, *J =* 11.9, 5.6 Hz, 4H), 0.63 (dd, *J =* 12.3, 3.9 Hz, 3H).

**Step 2:** The compound (5*R*)-5-[(1*R*,3a*S*,3b*S*,5a*R*,6*R*,7*S*,9a*R*,9b*S*,11a*R*)-7-acetoxy-4,4-difluoro-6-hydroxy-9a,11a-dimethylhexadecahydro-1*H*-cyclopenta[1,2-*a*]phenanthren-1-yl]-1-(2,6-difluorophenyl)hexyl acetate **(301-1)** (80 mg, 0.133 mmol, 1.0 *eq*) was dissolved in dichloromethane (5 mL), then Dess-Martin periodinane (84.72 mg, 0.200 mmol, 1.5 *eq*) was added thereto, and the resulting reaction mixture was stirred at 25°C for 1 hour. The completion of the reaction was monitored by TLC (petroleum ether: ethyl acetate = 5:1). The reaction mixture was quenched with saturated sodium sulfite, and the aqueous phase was extracted with dichloromethane (5 mL × 3). The organic phases were combined, washed with saturated brine (5 mL), dried over anhydrous Na₂SO₄, filtered, and concentrated to obtain a crude product. The crude product was purified by silica gel column chromatography (petroleum ether: ethyl acetate = 70:30) to obtain (5*R*)-5-[(1*R*,3a*S*,3b*S*,5a*R*,7*S*,9a*R*,9b*S*,11a*R*)-7-acetoxy-4,4-difluoro-9a,11a-dimethyl-6-oxohexadecahydro-1*H*-cyclopenta[1,2-*a*]phenanthren-1-yl]-1-(2,6-difluorophenyl)hexyl acetate **(301-2)** (40 mg, 0.058 mmol, purity: 90%, yield: 43.41%) as a white solid. ¹H NMR (400 MHz, CDCl₃) δ 6.86 (t, *J* = 8.2 Hz, 2H), 6.04 (t, *J* = 7.4 Hz, 1H), 5.18 (dd, *J* = 12.2, 7.4 Hz, 1H), 2.54 (d, *J* = 12.1 Hz, 1H), 2.27 (s, 1H), 2.15 (s, 3H), 2.06 (s, 3H), 1.91 (ddd, *J* = 48.9, 25.3, 10.2 Hz, 9H), 1.64 (m, 6H), 1.32 (m, 18H), 1.08 (s, 4H), 0.77 (d, *J* = 10.9 Hz, 3H), 0.63 (dd, *J* = 15.2, 3.8 Hz, 3H).

**Step 3:** The compound (5*R*)-5-[(1*R*,3a*S*,3b*S*,5a*R*,7*S*,9a*R*,9b*S*,11a*R*)-7-acetoxy-4,4-difluoro-9a,11a-dimethyl-6-oxohexadecahydro-1*H*-cyclopenta[1,2-*a*]phenanthren-1-yl]-1-(2,6-difluorophenyl)hexyl acetate **(301-2)** (40 mg, 0.064 mmol) was dissolved in a mixed solvent of tetrahydrofuran (1 mL) and methanol (2 mL), then potassium carbonate (44 mg, 0.32 mmol, 5.0 *eq*) was added thereto, and the resulting reaction mixture was stirred at 25°C for 1 hour. The completion of the reaction was monitored by TLC (petroleum ether: ethyl acetate = 3:1). The reaction system was added with water (10 mL), and the aqueous phase was extracted with ethyl acetate (15 mL × 3). The ethyl acetate phases were combined and washed with saturated brine (15 mL). The ethyl acetate phase was dried over anhydrous Na₂SO₄, filtered, and concentrated to obtain a crude product. The crude product was purified by silica gel column chromatography (petroleum ether: ethyl acetate = 50:50) to obtain (1*R*,3a*S*,3b*S*,5a*R*,7S,9a*R*,9b*S*,11a*R*)-1-[(2*R*)-6-(2,6-difluorophenyl)-6-hydroxyhexan-2-yl]-4,4-difluoro-7-hydroxy-9a,11a-dimethylhexadecahydro-1*H*-cyclopenta[1,2-*a*]phenanthren-6-one **(301)** (4 mg, 0.007 mmol, purity: 94.57%, yield: 10.93%) as a white solid. ¹H NMR (400 MHz, CDCl₃) δ 7.20 (d, *J* = 7.9 Hz, 1H), 6.87 (t, *J* = 8.2 Hz, 2H), 5.03 (s, 1H), 4.14 (s, 1H), 2.51 (d, *J =* 13.2 Hz, 2H), 1.91 (m, 8H), 1.65 (m,10H), 1.40 (m, 3H), 1.11 (m, 4H), 0.89 (t,*J* = 5.9 Hz, 3H), 0.76 (s, 2H), 0.66 (d, *J* = 3.5 Hz, 3H). ¹⁹F NMR (377 MHz, CDCl₃) δ -90.21, - 90.85, -111.49, -112.13, -115.41, -115.48. LC-MS: [M+H-H₂O-2F]⁺ = 485.

### Example 302

### Preparation of (1R,3aS,3bS,5aR,7S,9aR,9bS,11aR)-4,4-difluoro-7-hydroxy-1-[(2R)-6-hydroxy-6-methylheptan-2-yl]-9a,11a-dimethylhexadecahydro-1H-cyclopenta[1,2-a]phenanthren-6-one (compound 302)

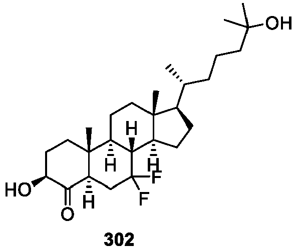

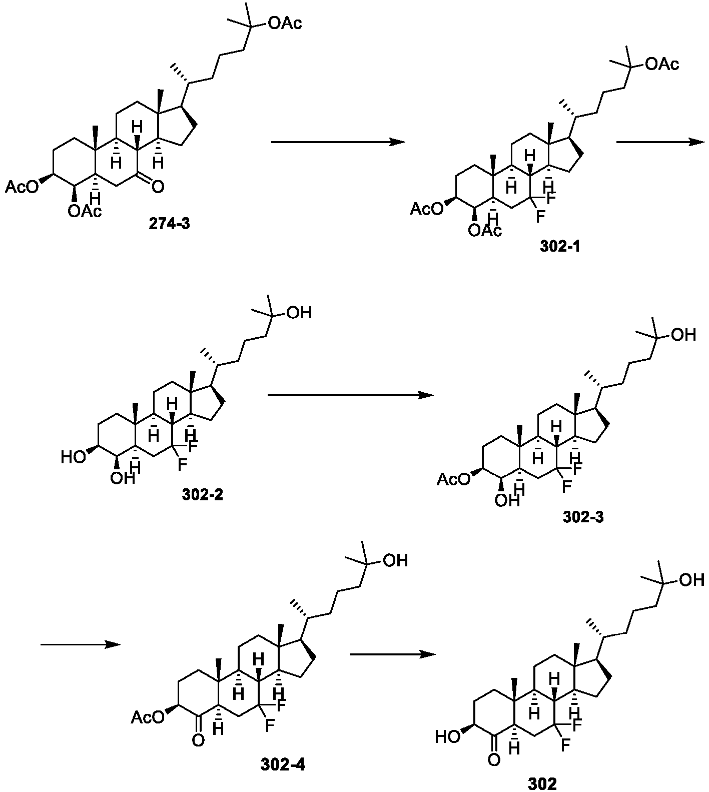

**Step 1:** (6*R*)-6-[(1*R*,3a*S*,3b*S*,5a*R*,6*R*,7*S*,9a*R*,9b*S*,11a*R*)-6,7-Diacetoxy-9a,11a-dimethyl-4-oxohexadecahydro-1*H-*cyclopenta[1,2-*a*]phenanthren-1-yl]-2-methylheptan-2-yl acetate **(274-3)** (200 mg, 0.36 mmol) was dissolved in diethylaminosulfur trifluoride (3 mL) in a 50 mL round-bottom flask at room temperature, and the reaction mixture was stirred at 40°C for 6 hours. The reaction was monitored by TLC (petroleum ether: ethyl acetate = 10:1). After the reaction was completed, the reaction mixture was quenched with ice water (30 mL) and extracted with ethyl acetate (20 mL × 3). The organic phase was washed with saturated brine (30 mL), dried over anhydrous sodium sulfate, concentrated, and purified by silica gel column chromatography (petroleum ether: ethyl acetate = 10:1) to obtain (6R)-6-[(1*R*,3a*S*,3b*S*,5a*R*,6*R*,7*S*,9a*R*,9b*S*,11a*R*)-6,7-diacetoxy-4,4-difluoro-9a,11a-dimethylhexadecahydro-1*H*-cyclopenta[1,2-*a*]phenanthren-1-yl]-2-methylheptan-2-yl acetate **(302-1)** (120 mg, purity: 90%, yield: 51.55%) as a white solid. ¹H NMR (400 MHz, CDCl₃) δ 5.20 (s, 1H), 4.79 (dd, *J =* 8.3, 4.0 Hz, 1H), 2.10 (s, 3H), 1.97 (d, *J* = 3.6 Hz, 6H), 1.92 - 1.59 (m, 21H), 1.42 (s, 6H), 1.19 - 1.09 (m, 6H), 1.05 (s, 3H), 0.91 (d, *J* = 6.5 Hz, 3H), 0.66 (s, 3H). ¹³C NMR (101 MHz, CDCl₃) δ 170.25, 82.53, 72.01, 71.84, 55.25, 43.05, 41.15, 39.11, 36.14, 35.61, 29.70, 26.05, 22.67, 22.50, 20.98, 20.92, 20.55, 20.36, 18.66, 13.25, 11.83. ¹⁹F NMR (376 MHz, CDCl₃) δ = -89.24 (d, *J =* 237.9, 1F), -110.61 (d, *J* = 237.8, 1F).

**Step 2:** (6*R*)-6-[(1*R*,3a*S*,3b*S*,7*S,*9a*R*,9b*S*,11a*R*)-6,7-Diacetoxy-4,4-difluoro-9a,11a-dimethylhexadecahydro-1*H*-cyclopenta[1,2-*a*]phenanthren-1-yl]-2-methylheptan-2-yl acetate **(302-1)** (120 mg, 0.21 mmol) was dissolved in tetrahydrofuran (2 mL) and methanol (2 mL) in a 50 mL round-bottom flask at room temperature, then lithium hydroxide monohydrate (88 mg, 2.1 mmol) and water (1 mL) were added thereto at room temperature, and the reaction mixture was stirred at 40°C for 30 hours. The reaction was monitored by TLC (petroleum ether: ethyl acetate = 2:1). After the reaction was completed, the reaction mixture was diluted with water (20 mL) and extracted with ethyl acetate (15 mL × 3). The organic phase was washed with saturated brine (20 mL), dried over anhydrous sodium sulfate, concentrated, and purified by silica gel column chromatography (petroleum ether: ethyl acetate = 2:1) to obtain (1*R*,3a*S*,3b*S*,7*S*,9a*R*,9b*S*,11a*R*)-4,4-difluoro-1-[(2*R*)-6-hydroxy-6-methylheptan-2-yl]-9a,11a-dimethylhexadecahydro-1*H*-cyclopenta[1,2-*a*]phenanthrene-6,7-diol **(302-2)** (90 mg, purity: 90%, yield: 84.64%) as a white solid. ¹H NMR (400 MHz, CDCl₃) δ = 3.74 (s, 1H), 3.65 - 3.56 (m, 1H), 2.26 - 2.15 (m, 1H), 2.07 - 1.94 (m, 2H), 1.92 - 1.56 (m, 14H), 1.52 - 1.31 (m, 12H), 1.21 (s, 6H), 1.06 (s, 3H), 0.93 (d, *J* = 6.5, 3H), 0.67 (s, 3H). ¹⁹F NMR (376 MHz, CDCl₃) δ = -88.94 (d, *J* = 235.9, 1F), -110.94 (d, *J* = 235.8, 1F).

**Step 3:** (1*R*,3a*S*,3b*S*,6*R*,7*S*,9a*R*,9b*S*,11a*R*)-4,4-Difluoro-1-[(2*R*)-6-hydroxy-6-methylheptan-2-yl]-9a,11a-dimethylhexadecahydro-1*H*-cyclopenta[1,2-*a*]phenanthrene-6,7-diol **(302-2)** (110 mg, 0.24 mmol) was dissolved in dichloromethane (2 mL) and tetrahydrofuran (2 mL) in a 50 mL round-bottom flask at room temperature. Triethylamine (73 mg, 0.72 mmol), 4-dimethylaminopyridine (3 mg, 0.024 mmol), and acetic anhydride (25 mg, 0.24 mmol) were added thereto at room temperature, and the reaction mixture was stirred at room temperature for 0.5 hours. The reaction was monitored by TLC (petroleum ether: ethyl acetate = 5:1). After the reaction was completed, the reaction mixture was quenched with water (20 mL) and extracted with ethyl acetate (15 mL × 3). The organic phase was washed with saturated brine (20 mL), dried over anhydrous sodium sulfate, concentrated, and purified by silica gel column chromatography (petroleum ether: ethyl acetate = 5:1) to obtain (1*R*,3a*S*,3b*S*,6*R*,7*S*,9a*R*,9b*S*,11a*R*)-4,4-difluoro-6-hydroxy-1-[(2*R*)-6-hydroxy-6-methylheptan-2-yl]-9a,11a-dimethylhexadecahydro-1*H*-cyclopenta[1,2-*i*]phenanthren-7-yl acetate **(302-3)** (112 mg, purity: 90%, yield: 84.35%) as a white solid. ¹H NMR (400 MHz, CDCl₃) δ = 4.75 - 4.69 (m, 1H), 3.83 (s, 1H), 2.09 (s, 3H), 2.06 - 1.59 (m, 10H), 1.57 - 1.26 (m, 15H), 1.21 (s, 6H), 1.20 - 1.10 (m, 3H), 1.09 (s, 3H), 0.93 (d, *J* = 6.5, 3H), 0.67 (s, 3H). ¹⁹F NMR (376 MHz, CDCl₃) δ = -89.03 (d, *J =* 236.4, 1F), -110.86 (d, *J =* 236.3, 1F).

**Step 4:** (1*R*,3a*S*,3b*S*,7*R*,9a*S*,9b*S,*11a*R*)-4,4-Difluoro-6-hydroxy-1-[(2*R*)-6-hydroxy-6-methylheptan-2-yl]-9a,11a-dimethylhexadecahydro-1*H*-cyclopenta[1,2-*i*]phenanthren-7-yl acetate **(302-3)** (110 mg, 0.22 mmol) was dissolved in dichloromethane (4 mL) in a 50 mL round-bottom flask at room temperature, then Dess-Martin periodinane (140 mg, 0.33 mmol) was added thereto at room temperature, and the reaction mixture was stirred at room temperature for 2 hours. The reaction was detected by TLC (ethyl acetate/petroleum ether = 8:1). After the reaction was completed, the reaction mixture was diluted with dichloromethane (10 mL), quenched with saturated sodium sulfite aqueous solution (10 mL) and saturated sodium bicarbonate aqueous solution (10 mL), and extracted with dichloromethane (15 mL × 3). The organic phase was washed with saturated brine (20 mL), dried over anhydrous sodium sulfate, concentrated, and purified by silica gel column chromatography (petroleum ether: ethyl acetate = 8:1) to obtain (1*R*,3a*S*,3b*S*,5a*R*,7*S*,9a*R*,9b*S*,11a*R*)-4,4-difluoro-1-[(2*R*)-6-hydroxy-6-methylheptan-2-yl]-9a,11a-dimethyl-6-oxohexadecahydro-1*H*-cyclopenta[1,2-*i*]phenanthren-7-yl acetate **(302-4)** (98 mg, purity: 90%, yield: 80.92%) as a white solid. ¹H NMR (400 MHz, CDCl₃) δ = 5.18 (dd, *J =* 12.3, 7.3, 1H), 2.58 - 2.49 (m, 1H), 2.32 - 2.22 (m, 1H), 2.15 (s, 3H), 2.12 - 1.78 (m, 8H), 1.72 - 1.30 (m, 15H), 1.21 (s, 6H), 1.14 - 1.03 (m, 2H), 0.93 (d, *J* = 6.5, 3H), 0.79 (s, 3H), 0.67 (s, 3H). ¹³C NMR (101 MHz, CDCl₃) δ = 203.23, 170.00, 75.81, 71.08, 55.18, 53.11, 53.01, 52.47, 50.32, 50.23, 48.42, 44.37, 43.11, 41.94, 41.48, 39.19, 36.37, 35.67, 35.25, 31.93, 30.31, 29.70, 29.34, 29.26, 28.44, 28.28, 25.27, 22.69, 21.66, 20.74, 20.68, 18.72, 14.86, 14.11, 12.92, 11.87. ¹⁹F NMR (376 MHz, CDCl₃) δ = -90.61 (d, *J* = 240.1, 1F), -111.79 (d, *J=* 240.0, 1F).

**Step 5:** (1*R*,3a*S*,3b*S*,5a*R*,7*S*,9a*R*,9b*S*,11a*R*)-4,4-Difluoro-1-[(2*R*)-6-hydroxy-6-methylheptan-2-yl]-9a,11a-dimethyl-6-oxohexadecahydro-1*H*-cyclopenta[1,2-*i*]phenanthren-7-yl acetate **(302-4)** (98 mg, 0.20 mmol) was dissolved in tetrahydrofuran (2 mL) and methanol (2 mL) in a 50 mL round-bottom flask at room temperature, then potassium carbonate (138 mg, 1.00 mmol) and water (1 mL) were added thereto at room temperature, and the reaction mixture was stirred at room temperature for 1 hour. The reaction was monitored by TLC (petroleum ether: ethyl acetate = 3:1). After the reaction was completed, the reaction mixture was diluted with water (20 mL) and extracted with ethyl acetate (10 mL × 3). The organic phase was washed with saturated brine (20 mL), dried over anhydrous sodium sulfate, concentrated, and purified by silica gel column chromatography (petroleum ether: ethyl acetate = 3:1) to obtain (1*R*,3a*S*,3b*S*,5a*R*,7*S*,9a*R*,9b*S*,11a*R*)-4,4-difluoro-7-hydroxy-1-[(2*R*)-6-hydroxy-6-methylheptan-2-yl]-9a,11a-dimethylhexadecahydro-1*H*-cyclopenta[1,2-*a*]phenanthren-6-one **(302)** (48.14 mg, purity: 88.47%, yield: 46.90%) as a white solid. ¹H NMR (400 MHz, CDCl₃) δ 4.19 - 4.10 (m, 1H), 2.56 - 2.39 (m, 2H), 2.25 - 1.79 (m, 8H), 1.78 - 1.24 (m, 18H), 1.21 (s, 6H), 0.93 (d, *J* = 6.5 Hz, 3H), 0.77 (s, 3H), 0.68 (s, 3H). ¹³C NMR (101 MHz, CDCl₃) δ = 210.12, 74.56, 71.08, 55.19, 52.47, 50.44, 48.45, 44.37, 43.14, 42.57, 39.22, 36.38, 35.66, 34.83, 32.19, 29.35, 29.25, 28.44, 25.27, 21.63, 20.73, 18.72, 13.07, 11.88. ¹⁹F NMR (376 MHz, CDCl₃) δ -90.51 (d, *J* = 240.1 Hz, 1F), -111.81 (d, *J* = 240.0 Hz, 1F). LC-MS [M+Na]⁺ = 477.3.

### Example 303

### Preparation of 4-amino-5α-cholest-3β,25-diol (compound 303)

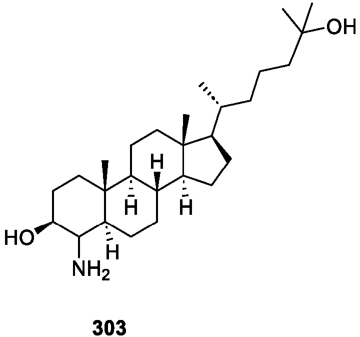

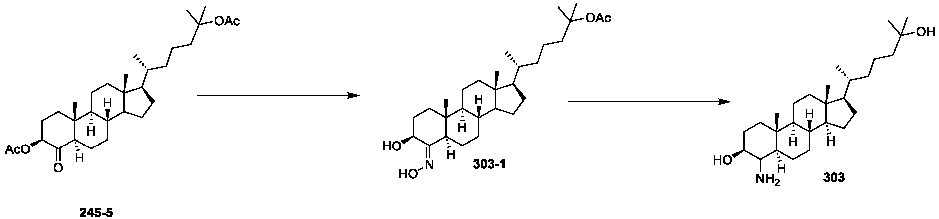

**Step 1:** The starting material (6*R*)-6-[(1*R*,3a*S*,3b*S*,5a*R*,7*S*,9a*R*,9b*S*,11a*R*)-7-acetoxy-9a,11 a-dimethyl-6-oxohexadecahydro-1*H*-cyclopenta[1,2-*a*]phenanthren-1-yl]-2-methylheptan-2-yl acetate **(245-5)** (100 mg, 0.2 mmol, 1.0 *eq*) and sodium acetate (81 mg, 0.6 mmol, 3.0 *eq*) were dissolved in ethanol (20 mL), then hydroxylamine hydrochloride (41.5 mg, 0.6 mmol, 3.0 *eq)* was added thereto, and the reaction mixture was stirred at 90°C for 5 hours under a nitrogen atmosphere. The completion of the reaction was monitored by TLC (petroleum ether: ethyl acetate = 3:1). The reaction system was diluted with water (40 mL) and extracted with ethyl acetate (30 mL × 3). The combined organic phases were washed with saturated brine (50 mL), then dried over anhydrous sodium sulfate, filtered, and concentrated to obtain a crude product. The crude product was purified by flash chromatography (petroleum ether: ethyl acetate = 81:19) to obtain (6R)-6-[(1*R*,3a*S*,3b*S*,5a*R*,6*Z*,7*S*,9a*R*,9b*S*,11a*R*)-7-hydroxy-6-(hydroxyimino)-9a,11a-dimethylhexadecahydro-1*H*-cyclopenta[1,2-*a*]phenanthren-1-yl]-2-methylheptan-2-yl acetate **(303-1)** (36 mg, purity: 90%, yield: 34%) as a white solid. ¹H NMR (400 MHz, CDCl₃) δ 4.48 (dd, *J* = 11.0, 6.1 Hz, 1H), 4.24 (s, 1H), 1.97 (s, 7H), 1.86 - 1.68 (m, 10H), 1.56 (ddd, *J* = 21.5, 13.7, 8.8 Hz, 6H), 1.42 (s, 13H), 1.39 - 1.15 (m, 22H), 1.01 (ddd, *J* = 23.9, 20.2, 10.5 Hz, 9H), 0.93 - 0.89 (m, 9H), 0.82 (s, 4H), 0.66 (d, *J* = 4.9 Hz, 7H).

**Step 2:** The starting material (6*R*)-6-[(1*R*,3a*S*,3b*S*,5a*R*,6*Z*,7*S*,9a*R*,9b*S*,11a*R*)-7-hydroxy-6-(hydroxyimino)-9a,11a-dimethylhexadecahydro-1*H*-cyclopenta[1,2-*a*]phenanthren-1-yl]-2-methylheptan-2-yl acetate **(303-1)** (18 mg, 0.04 mmol, 1 *eq)* was dissolved in tetrahydrofuran (5 mL), and the reaction system was cooled to 0°C. Lithium aluminum hydride (15 mg, 0.39 mmol, 5.0 *eq*) was slowly added thereto, and the reaction mixture was reacted at 80°C for 18 hours under a nitrogen atmosphere. After the completion of the reaction was monitored by TLC (dichloromethane: methanol = 10:1, heating with phosphomolybdic acid), the reaction was terminated. The reaction system was cooled to room temperature and filtered to collect a filtrate, which was concentrated to obtain a crude product. The crude product was purified by flash chromatography (dichloromethane: methanol = 80:20) to obtain 4-amino-5α-cholest-3β,25-diol **(303)** (2.1 mg, purity: 97.33%, yield: 12.8%) as a white solid. ¹H NMR (400 MHz, MeOD) δ 3.83 - 3.75 (m, 1H), 3.24 (s, 1H), 2.02 (d, *J =* 12.3 Hz, 1H), 1.72 (ddd, *J =* 39.4, 25.2, 9.1 Hz, 7H), 1.39 (ddd, *J =* 29.3, 24.8, 15.6 Hz, 14H), 1.16 (s, 6H), 1.13 - 0.99 (m, 6H), 0.94 (d, *J* = 6.5 Hz, 3H), 0.91 (s, 3H), 0.69 (s, 3H). ¹³C NMR (101 MHz, MeOD) δ 70.06, 67.59, 64.53, 56.26, 56.16, 55.08, 43.86, 42.46, 42.25, 39.60, 36.35, 35.69, 35.31, 31.91, 27.85, 27.71, 24.94, 23.82, 20.43, 17.75, 12.51, 10.98. LC-MS: [M+H]⁺ = 420.75.

### Example 304

### Preparation of 24-(1-hydroxyethyl)-5α-cholan-3β,4β-diol (compound 304)

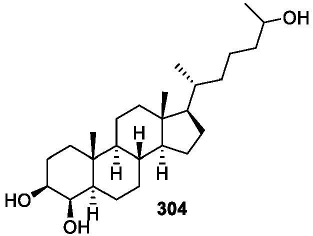

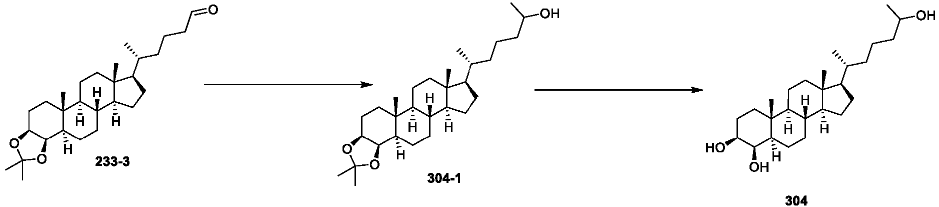

**Step 1:** The compound (5R)-5-[(3aS,5aR,5bS,7aR,SR,10aS,10bS,12aR,12bR)~ 2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]hexanal **(233-3)** (30 mg, 0.07 mmol, 1.0 *eq*) was dissolved in tetrahydrofuran (5 mL), and the reaction system was cooled to 0°C. Methylmagnesium bromide (0.12 mL, 0.35 mmol, 5 *eq*) was then slowly added thereto, and the reaction system was stirred at room temperature for 1 hour. The completion of the reaction was monitored by TLC (petroleum ether: ethyl acetate = 5:1). The reaction system was added with water (20 mL) and extracted with ethyl acetate (50 mL × 3). The organic phases were combined, washed with water (30 mL), dried, and concentrated to obtain a crude product. The crude product was subjected to silica gel column chromatography (petroleum ether: ethyl acetate = 88:12) to obtain (6*R*)-6-[(3a*S*,5a*R*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]heptan-2-ol **(304-1)** (30 mg, purity: 90%, yield: 86.77%) as a white solid.

¹H NMR (400 MHz, CDCl₃) δ 4.00 (s, 2H), 3.80 (s, 1H), 2.18 (d, *J* = 3.3 Hz, 1H), 1.96 (d, *J =* 11.3 Hz, 2H), 1.70 (dd, *J =* 36.8, 23.3 Hz, 9H), 1.51 - 1.48 (m, 4H), 1.45 - 1.32 (m, 12H), 1.31 (s, 3H), 1.19 (d, *J =* 5.2 Hz, 4H), 1.08 (d, *J* = 11.1 Hz, 4H), 1.05 (s, 3H), 0.91 (d, *J =* 4.2 Hz, 4H), 0.66 (s, 3H), 0.60 (s, 1H).

**Step 2:** The reactant (6*R*)-6-[(3a*S*,5a*R*,5b*S*,7a*R*,8*R*,10a*S*,10b*S*,12a*R*,12b*R*)-2,2,5a,7a-tetramethyl-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]heptan-2-ol **(304-1)** (30 mg, 0.067 mmol, 1.0 *eq*) was dissolved in tetrahydrofuran (5 mL), then dilute hydrochloric acid (0.5 mL, 3 mol/L) was added thereto, and the reaction system was stirred at room temperature for 40 minutes. The completion of the reaction was monitored by TLC (petroleum ether: ethyl acetate = 2:1). The reaction system was added with water (10 mL), and the aqueous phase was extracted with ethyl acetate (25 mL × 3). The ethyl acetate phases were combined and washed with saturated brine (10 mL × 3). The ethyl acetate phase was dried over anhydrous sodium sulfate, filtered, and concentrated to obtain a crude product. The crude product was purified by column chromatography (petroleum ether: ethyl acetate = 70:30) to obtain 24-(1-hydroxyethyl)-5α-cholan-3β,4β-diol **(304)** (9.87 mg, purity: 100%, yield: 36.14%) as a white solid. ¹H NMR (400 MHz, CDCl₃) δ 3.81 (d, *J* = 6.2 Hz, 1H), 3.76 (s, 1H), 3.58 (dd, *J* = 11.3, 3.8 Hz, 1H), 1.98 (d, *J =* 12.4 Hz, 1H), 1.75 (d, *J =* 2.3 Hz, 5H), 1.47 - 1.23 (m, 14H), 1.21 (d, *J =* 6.2 Hz, 3H), 1.16 - 1.05 (m, 5H), 1.04 (s, 3H), 0.97 (dd, *J =* 14.7, 5.3 Hz, 2H), 0.93 (d, *J =* 6.5 Hz, 3H), 0.67 (s, 3H), 0.65 - 0.57 (m, 1H). ¹³C NMR (101 MHz, CDCl₃) δ 74.85, 72.31, 56.58, 56.12, 55.27, 48.85, 42.61, 39.91, 36.89, 35.71, 35.50, 35.40, 32.40, 29.69, 28.24, 26.00, 25.87, 24.21, 23.58, 23.50, 22.18, 20.59, 18.62, 14.67, 12.07. LC-MS: [M+H-2H₂O]⁺ = 371.

### Example 305

### Preparation of 24-(hydroxyphenylmethyl)-5α-cholan-3β,4β-diol (compound 305)

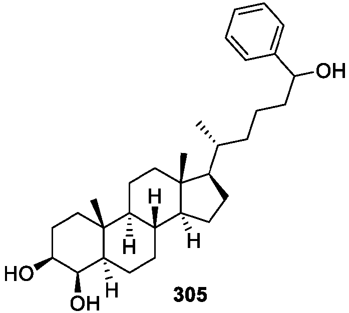

Referring to example **275,** 2,4,6-trifluorobromobenzene was replaced with bromobenzene to obtain compound 24-(hydroxyphenylmethyl)-5α-cholan-3β,4β-diol **(305).** ¹H NMR (400 MHz, CDCl₃) δ 7.35 (d, *J* = 4.4 Hz, 4H), 7.30 - 7.27 (m, 1H), 4.70 - 4.63 (m, 1H), 3.74 (s, 1H), 3.55 (d, *J* = 11.0 Hz, 1H), 1.94 (d, *J* = 12.6 Hz, 1H), 1.84 - 1.75 (m, 4H), 1.67 - 1.51 (m, 3H), 1.43 - 1.16 (m, 11H), 1.12 - 0.91 (m, 11H), 0.87 (t, *J =* 5.9 Hz, 3H), 0.63 (d, *J =* 2.8 Hz, 3H), 0.61 - 0.54 (m, 1H).

¹³C NMR (101 MHz, CDCl₃) δ 145.02, 128.45, 127.53, 127.48, 125.93, 125.85, 74.86, 74.68, 72.32, 56.56, 56.19, 55.25, 48.84, 42.61, 39.90, 39.56, 36.88, 35.67, 35.49, 35.40, 32.40, 28.25, 26.01, 25.88, 24.20, 22.40, 20.58, 18.57, 14.69, 12.07. LCMS (ESI) [M+H-2H₂O]⁺ = 482.

### Example 306

### Preparation of 24-[(4-fluoro-2-methoxyphenyl)(hydroxy)methyl]-5α-cholan-3β,4β-diol (compound 306)

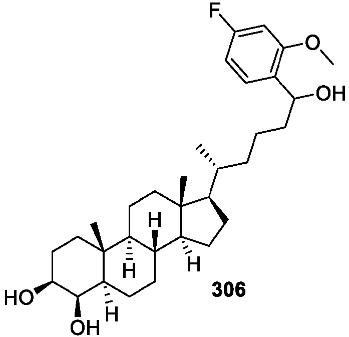

Referring to example **275,** 2,4,6-trifluorobromobenzene was replaced with 1-bromo-4-fluoro-2-methoxybenzene to obtain compound 24-[(4-fluoro-2-methoxyphenyl)(hydroxy)methyl]-5α-cholan-3β,4β-diol **(306).** ¹H NMR (400 MHz, CDCl₃) δ 7.23 (s, 1H), 6.63 (dd, *J* = 19.4, 9.7 Hz, 2H), 4.84 (dd, *J* = 13.6, 7.6 Hz, 1H), 3.83 (s, 3H), 3.74 (s, 1H), 3.56 (dd, *J* = 9.9, 5.4 Hz, 1H), 1.95 (d, *J* = 13.0 Hz, 1H), 1.74 (d, *J* = 10.2 Hz, 4H), 1.54 (s, 2H), 1.37 (d, *J =* 9.4 Hz, 5H), 1.33 (s, 2H), 1.28 (s, 2H), 1.26 (s, 2H), 1.06 (d, *J =* 11.9 Hz, 4H), 1.01 (s, 3H), 0.95 (d, *J* = 10.9 Hz, 2H), 0.88 (dd, *J* = 6.2, 3.9 Hz, 4H), 0.64 (s, 3H), 0.63 - 0.53 (m, 2H). ¹³C NMR (101 MHz, CDCl₃) δ 154.93, 131.43, 127.93, 127.13, 110.88, 106.17, 77.34, 77.23, 77.02, 76.70, 74.87, 74.85, 72.31, 70.41, 70.05, 68.93, 68.68, 68.66, 65.14, 62.98, 56.57, 55.37, 55.25, 48.89, 42.65, 39.99, 36.87, 35.57, 35.40, 32.40, 31.41, 29.78, 28.21, 25.86, 20.51, 18.53, 14.64, 12.05, 1.02, 1.00. LCMS: [M+H-2H₂O]⁺ = 482.

### Example 307

### Preparation of 3-methylcholest-3,25-diol (compound 307)

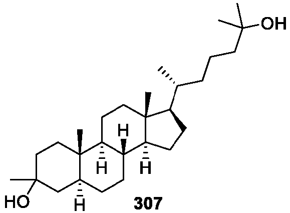

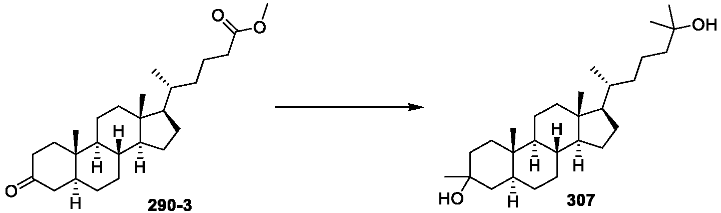

Methyl (5*R*)-5-[(1*R*,3a*S*,3b*R*,5a*S*,9a*S*,9b*S*,11a*R*)-9a,11a-dimethyl-7-oxohexadecahydro-1*H*-cyclopenta[1,2-*a*]phenanthren-1-yl]hexanoate **(290-3)** (50 mg, 0.12 mmol) was dissolved in anhydrous tetrahydrofuran (2 mL) in a 50 mL round-bottom flask at room temperature, and the reaction mixture was cooled to 0°C under a nitrogen atmosphere. A 3 mol/L solution of methylmagnesium bromide in tetrahydrofuran (0.40 mL, 1.20 mmol) was added dropwise thereto, and the reaction mixture was stirred at room temperature for 2 hours. The reaction was monitored by TLC (petroleum ether: ethyl acetate = 5:1). After the reaction was completed, the reaction system was cooled to 0°C, quenched with saturated ammonium chloride aqueous solution (15 mL), and extracted with ethyl acetate (10 mL × 3). The organic phase was washed with saturated brine (20 mL), dried over anhydrous sodium sulfate, and purified by silica gel column chromatography (petroleum ether: ethyl acetate = 5:1) to obtain 3-methylcholest-3,25-diol **(307)** (24.16 mg, purity: 80.12%, yield: 46.46%) as a white solid.

¹H NMR (400 MHz, CDCl₃) δ = 2.01 - 1.92 (m, 1H), 1.86 - 1.75 (m, 1H), 1.71 - 1.24 (m, 23H), 1.21 (s, 6H), 1.19 (s, 3H), 1.18 - 0.94 (m, 7H), 0.92 (d, *J =* 6.5, 3H), 0.75 (s, 3H), 0.65 (s, 3H). ¹³C NMR (101 MHz, CDCl₃) δ = 99.99, 71.52, 71.11, 69.84, 56.55, 56.20, 54.52, 54.25, 44.44, 44.28, 43.39, 42.63, 41.88, 41.17, 40.07, 36.60, 36.45, 36.04, 35.77, 35.56, 34.94, 34.06, 32.02, 31.57, 29.34, 29.21, 28.72, 28.51, 28.26, 26.66, 24.20, 21.24, 21.01, 20.80, 18.65, 12.09, 11.89, 11.21. LC-MS: (M-2OH)⁺ = 384.

### Example 308

### Preparation of 3-(hydroxymethyl)-5α-cholest-25-ol (compound 308)

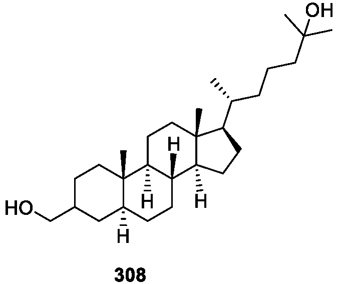

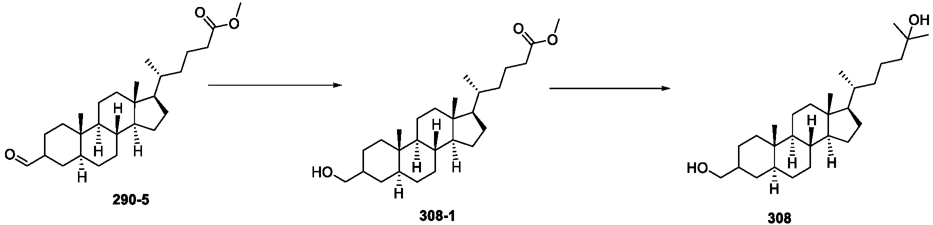

**Step 1:** Methyl (5*R*)-5-[(1*R*,3a*S*,3b*R*,5a*S*,9a*S*,9b*S*,11a*R*)-7-formyl-9a,11a-dimethylhexadecahydro-1*H*-cyclopenta[1,2-*a*]phenanthren-1-yl]hexanoate **(290-5)** (400 mg, 1.0 mmol, 1.0 *eq.*) was weighed and dissolved in tetrahydrofuran (8 mL) and anhydrous methanol (4 mL), and sodium borohydride (75 mg, 2.0 mmol, 2.0 *eq.*) was added thereto in an ice bath. After the addition was completed, the reaction mixture was warmed to room temperature. The reaction was monitored by TLC (petroleum ether: ethyl acetate = 3:1). After the reaction was completed, the reaction mixture was concentrated, and the resulting crude product was extracted with water and ethyl acetate. The organic phase was separated, dried, concentrated, and purified by column chromatography (petroleum ether: ethyl acetate = 0 to 30%) to obtain methyl (5*R*)-5-[(1*R*,3a*S*,3b*R*,5a*S*,9a*S*,9b*S*,11a*R*)-7-(hydroxymethyl)-9a,11a-dimethylhexadecahydro-1*H*-cyclopenta[1,2-*a*]phenanthren-1-yl]hexanoate **(308-1)** (400 mg, 0.95 mmol, 95%) as a white solid. ¹H NMR (399 MHz, Chloroform-d) δ 3.65 (s, 3H), 3.44 (d, *J =* 6.1 Hz, 2H), 2.30 - 2.21 (m, 2H), 1.93 (dt, *J =* 12.7, 3.3 Hz, 1H), 1.77 (dd, *J* = 8.8, 4.6 Hz, 1H), 1.73 - 1.63 (m, 3H), 1.56 - 1.48 (m, 7H), 1.47 - 1.41 (m, 3H), 1.34 (q, *J =* 12.6, 11.1 Hz, 5H), 1.24 (td, *J* = 7.1, 2.4 Hz, 4H), 1.20 - 1.09 (m, 5H), 1.07 (d, *J* = 9.7 Hz, 4H), 1.01 - 0.92 (m, 4H), 0.90 (d, *J =* 6.5 Hz, 3H), 0.88 - 0.80 (m, 2H), 0.74 (s, 3H), 0.68 - 0.63 (m, 1H), 0.62 (s, 4H).

**Step 2:** Methyl (5*R*)-5-[(1*R*,3a*S*,3b*R*,5a*S*,9a*S*,9b*S*,11a*R*)-7-(hydroxymethyl)-9a,11a-dimethylhexadecahydro-1*H*-cyclopenta[1,2-*a*]phenanthren-1-yl]hexanoate **(308-1)** (40 mg, 0.1 mmol) was weighed and dissolved in tetrahydrofuran (5.0 mL). The reaction mixture was cooled to -78°C, and methyllithium (1.6 M, 0.6 mL) was added dropwise thereto. After the dropwise addition was completed, the reaction mixture was slowly warmed to room temperature and stirred for 2 hours. The completion of the reaction was monitored by TLC (petroleum ether: ethyl acetate = 3:1). The reaction mixture was quenched with water and extracted with ethyl acetate. The organic phase was separated, dried, concentrated, and purified by column chromatography (petroleum ether: ethyl acetate = 0 to 30%) to obtain 3-(hydroxymethyl)-5α-cholest-25-ol **(308)** (10 mg, 0.024 mmol, 25%) as a white solid with a purity of 82.03%. ¹H NMR (400 MHz, DMSO-d₆) δ 4.30 (dt, *J* = 10.7, 5.3 Hz, 1H), 4.02 (s, 1H), 3.15 (q, *J =* 5.8 Hz, 2H), 2.00 - 1.84 (m, 2H), 1.81 - 1.61 (m, 2H), 1.55 (d, *J =* 12.5 Hz, 2H), 1.44 (d, *J =* 12.0 Hz, 4H), 1.29 (d, *J =* 11.8 Hz, 4H), 1.25 (s, 2H), 1.22 (s, 2H), 1.15 (d, *J* = 5.4 Hz, 2H), 1.09 (d, *J =* 9.6 Hz, 3H), 1.01 (s, 6H), 0.93 (d, *J =* 11.6 Hz, 4H), 0.85 (d, *J =* 6.6 Hz, 3H), 0.83 - 0.74 (m, 2H), 0.70 (d, *J* = 17.8 Hz, 3H), 0.62 (s, 1H), 0.59 (s, 3H). ¹³C NMR (100 MHz, DMSO) δ 69.21, 67.02, 57.96, 56.50, 56.19, 55.34, 54.44, 46.22, 44.56, 42.58, 38.16, 36.61, 36.30, 36.23, 35.68, 35.47, 32.28, 32.14, 29.84, 29.66, 29.01, 28.24, 25.36, 21.05, 20.71, 18.96, 12.52, 12.34, 11.84. LC-MS: [M-OH⁻]⁺ = 401.3.

### Example 309

### Preparation of 25-hydroxy-5α-cholest-3-carboxylic acid (compound 309)

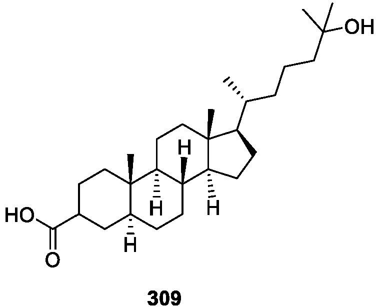

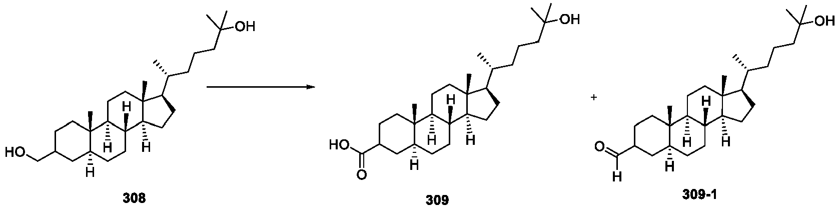

3-(Hydroxymethyl)-5α-cholest-25-ol **(308)** (50 mg, 0.12 mmol, 1.0 *eq.*) was weighed and dissolved in dichloromethane (5 mL), and Dess-Martin periodinane (102 mg, 0.24 mmol, *2.0 eq.*) was added thereto in an ice bath. After the addition was completed, the reaction mixture was warmed to room temperature. The reaction was monitored by TLC (petroleum ether: ethyl acetate = 3:1). After the reaction was completed, the reaction mixture was concentrated, and the resulting crude product was extracted with water and ethyl acetate. The organic phase was separated, dried, concentrated, and purified by column chromatography (petroleum ether: ethyl acetate = 0 to 30%) to obtain 25-hydroxy-5α-cholest-3-carbaldehyde **(309-1)** (15 mg, 0.036 mmol, 30%) as a white solid, which was further purified as the ratio of ethyl acetate increased to (petroleum ether: ethyl acetate = 30 to 50%) to obtain 25-hydroxy-5α-cholest-3-carboxylic acid (20 mg, 0.046 mmol, 38.7%, purity: 92.76%) as a white solid. ¹H NMR (399 MHz, Chloroform-d) δ 2.75 - 2.28 (m, 1H), 1.97 (dt, *J =* 23.3, 7.8 Hz, 2H), 1.77 (p, *J* = 11.2, 10.7 Hz, 3H), 1.67 -1.51 (m, 6H), 1.50 - 1.40 (m, 4H), 1.36 (dt, *J* = 13.9, 5.8 Hz, 4H), 1.26 (s, 3H), 1.20 (s, 6H), 1.08 (qd, *J =* 9.5, 8.4, 4.6 Hz, 4H), 1.02 - 0.93 (m, 3H), 0.92 - 0.88 (m, 3H), 0.85 (s, 1H), 0.78 (d, *J =* 3.9 Hz, 3H), 0.63 (d, *J =* 2.9 Hz, 3H). ¹³C NMR (100 MHz, CDCl₃) δ 180.42, 71.16, 56.47, 56.15, 54.29, 54.20, 45.90, 44.38, 43.18, 42.69, 42.56, 39.97, 39.13, 37.62, 36.40, 35.81, 35.73, 35.66, 35.43, 35.38, 34.98, 31.96, 31.80, 31.00, 29.68, 29.47, 29.32, 29.16, 28.63, 28.23, 24.41, 24.14, 22.60, 20.92, 20.78, 20.74, 18.62, 12.17, 12.05, 11.68. LCMS: [M-H]⁻ = 431.25.

### Example 310

### Preparation of N-[(1R,3aS,3bR,9aS,9bS,11aR)-1-[(2R)-6-hydroxy-6-methylheptan-2-yl]-9a,11a-dimethylhexadecahydro-1H-cyclopenta[1,2-a]phenanthrene-7-yl]methanesulfonamide (compound 310)

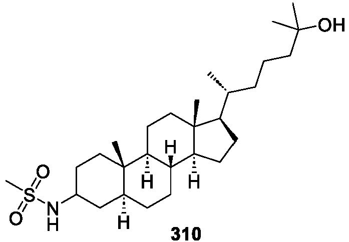

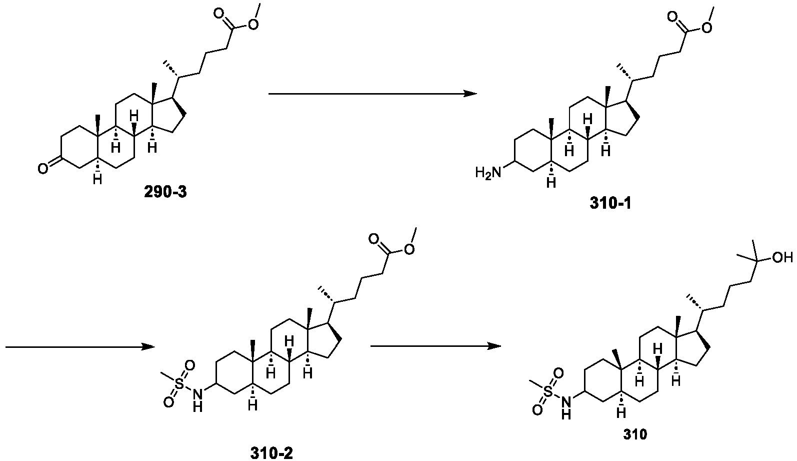

***Step 1*:** Methyl (5*R*)-5-[(1*R*,3a*S*,3b*R*,5a*S*,9a*S*,9b*S*,11a*R*)-9a,11a-dimethyl-7-oxohexadecahydro-1*H*-cyclopenta[1,2-*a*]phenanthren-1-yl]hexanoate **(290-3)** (190 mg, 0.47 mmol) was dissolved in tetrahydrofuran (3 mL) and methanol (6 mL) in a 50 mL round-bottom flask at room temperature. Ammonium acetate (363 mg, 4.70 mmol) and a catalytic amount of acetic acid (0.20 mL) were added thereto at room temperature, and the reaction mixture was stirred at room temperature for 2 hours. Sodium cyanoborohydride (297 mg, 4.70 mmol) was then added thereto, and the reaction mixture was stirred at room temperature for 14 hours. The reaction was detected by TLC (dichloromethane/methanol = 10:1). After the reaction was completed, the reaction mixture was diluted with dichloromethane (20 mL), quenched with saturated sodium bicarbonate aqueous solution (30 mL), and extracted with dichloromethane (20 mL × 3). The organic phase was washed with saturated brine (30 mL), dried over anhydrous sodium sulfate, concentrated, and purified by silica gel column chromatography (dichloromethane/methanol = 10:1) to obtain methyl (5*R*)-5-[(1*R*,3a*S*,3b*R*,5a*S*,9a*S*,9b*S*,11a*R*)-7-amino-9a,11a-dimethylhexadecahydro-1*H*-cyclopenta[1,2-*a*]phenanthren-1-yl]hexanoate **(310-1)** (160 mg, purity: 90%, yield: 75.59%) as a white solid. ¹H NMR (400 MHz, DMSO) δ 7.68 (s, 2H), 3.57 (s, 3H), 2.94 (dd, *J =* 25.9, 13.9 Hz, 1H), 2.32 - 2.16 (m, 2H), 1.98 - 1.88 (m, 1H), 1.80 - 0.95 (m, 27H), 0.88 (d, *J =* 6.4 Hz, 3H), 0.76 (s, 3H), 0.62 (s, 3H).

***Step 2*:** Methyl (5*R*)-5-[(1*R*,3a*S*,3b*R*,5a*S*,9a*S*,9b*S*,11a*R*)-7-amino-9a,11a-dimethylhexadecahydro-1*H*-cyclopenta[1,2-*a*]phenanthren-1-yl]hexanoate **(310-1)** (30 mg, 0.074 mmol) was dissolved in dichloromethane (1 mL) in a 50 mL round-bottom flask at room temperature. Triethylamine (37 mg, 0.37 mmol) was added thereto at room temperature, and methanesulfonyl chloride (13 mg, 0.11 mmol) was added dropwise thereto at 0°C. The ice bath was then removed, and the reaction mixture was stirred at room temperature for 2 hours. The reaction was monitored by TLC (petroleum ether/ethyl acetate = 5:1). After the reaction was completed, the reaction mixture was diluted with dichloromethane (5 mL), quenched with water (10 mL), and extracted with dichloromethane (5 mL × 3). The organic phase was washed with saturated brine (10 mL), dried over anhydrous sodium sulfate, concentrated, and purified by silica gel column chromatography (petroleum ether: ethyl acetate = 5:1) to obtain methyl (5*R*)-5-[(1*R*,3a*S*,3b*R*,9a*S*,9b*S*,11a*R*)-9a,11a-dimethyl-7-[(methyldioxo-λ6-sulfanyl)amino]hexadecahydro-1*H*-cyclopenta[1,2-*a*]phenanthren-1-yl]hexanoate **(310-2)** (22 mg, purity: 80%, yield: 49.16%) as a white solid. ¹H NMR (400 MHz, DMSO) δ = 6.96 - 6.88 (m, 1H), 3.57 (s, 3H), 3.13 - 2.99 (m, 1H), 2.87 (d, *J =* 5.4, 3H), 2.30 - 2.19 (m, 2H), 1.94 - 1.88 (m, 1H), 1.80 - 1.67 (m, 2H), 1.63 - 1.06 (m, 25H), 0.88 (d, *J =* 6.5, 3H), 0.73 (s, 3H), 0.62 (s, 3H).

***Step 3:*** Methyl (5*R*)-5-[(1*R*,3a*S*,3b*R*,9a*S*,9b*S*,11a*R*)-9a,11a-dimethyl-7-[(methyldioxo-λ6-sulfanyl)amino]hexadecahydro-1*H*-cyclopenta[1,2-*a*]phenanthren-1-yl]hexanoate **(310-2)** (22 mg, 0.046 mmol) was dissolved in anhydrous tetrahydrofuran (1 mL) in a 50 mL round-bottom flask at room temperature, and the reaction mixture was cooled to 0°C under a nitrogen atmosphere. A 3 mol/L solution of methylmagnesium bromide in tetrahydrofuran (0.092 mL, 0.28 mmol) was added dropwise thereto, and the reaction mixture was stirred at room temperature for 2 hours. The reaction was monitored by TLC (petroleum ether: ethyl acetate = 4:1). After the reaction was completed, the reaction system was cooled to 0°C, quenched with saturated ammonium chloride aqueous solution (10 mL), and extracted with ethyl acetate (5 mL × 3). The organic phase was washed with saturated brine (10 mL), dried over anhydrous sodium sulfate, and purified by silica gel column chromatography (petroleum ether: ethyl acetate = 4:1) to obtain *N*-[(1*R*,3a*S*,3b*R*,9a*S*,9b*S*,11a*R*)-1-[(2*R*)-6-hydroxy-6-methylheptan-2-yl]-9a,11a-dimethylhexadecahydro-1*H*-cyclopenta[1,2-*a*]phenanthrene-7-yl]methanesulfonamide **(310)** (11 mg, purity: 82.27%, yield: 41.13%) as a white solid. ¹H NMR (400 MHz, DMSO) δ 6.92 (m, 1H), 4.03 (s, 1H), 3.07 (s, 1H), 2.87 (d, *J =* 4.8 Hz, 3H), 1.96 (d, *J =* 22.2 Hz, 2H), 1.72 (m, 2H), 1.60 (d, *J =* 12.2 Hz, 3H), 1.45 (s, 4H), 1.31 (s, 7H), 1.18 (d, *J* = 46.9 Hz, 10H), 1.05 (s, 6H), 0.98 (s, 2H), 0.89 (s, 3H), 0.74 (s, 3H), 0.62 (s, 3H). ¹³C NMR (101 MHz, DMSO) δ 69.23, 56.64, 56.43, 56.20, 54.08, 52.83, 45.42, 44.60, 42.62, 41.70, 37.51, 36.64, 35.79, 35.70, 35.46, 35.28, 32.05, 29.91, 29.87, 29.70, 28.63, 28.27, 24.26, 21.16, 20.74, 18.99, 12.36, 11.82. LC-MS: [M-2OH]⁺ = 504.3.

### Example 311

### Preparation of 24-[hydroxy(2-methoxyphenyl)methyl]cholan-6(5)-en-3β-ol (compound 311)

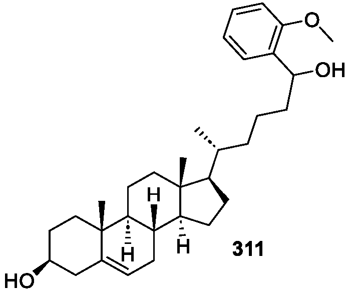

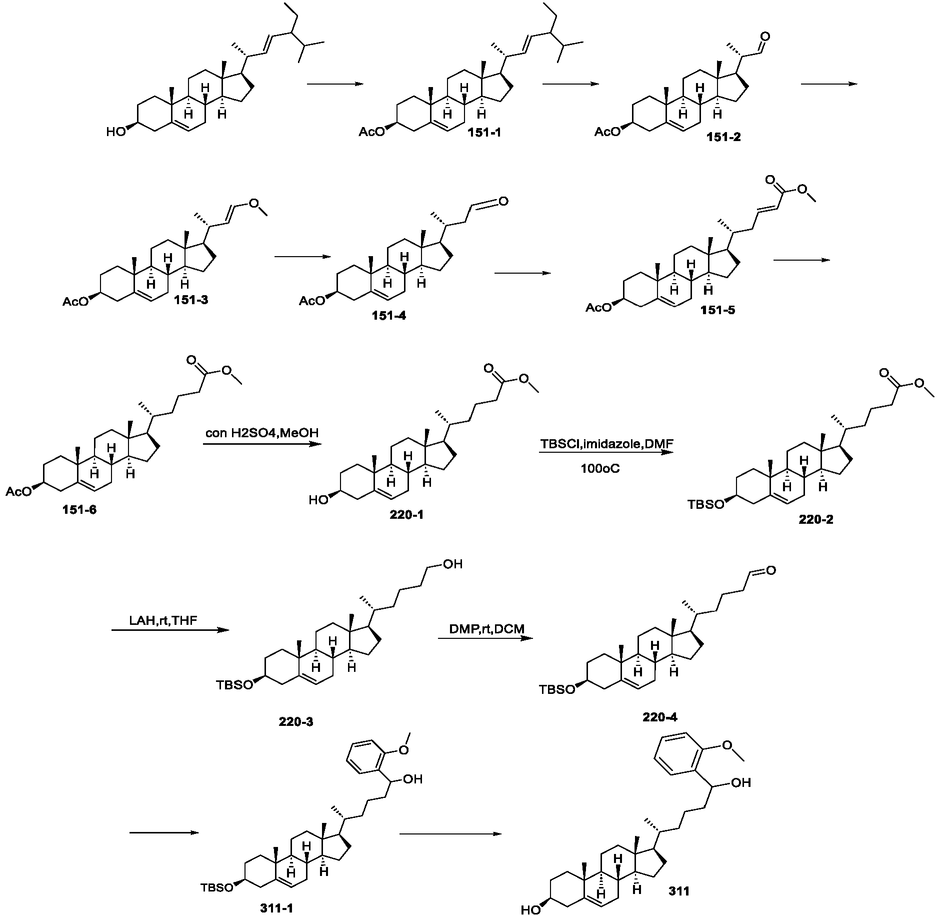

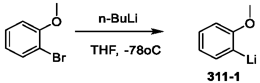

**Step 1:** (22*E*,24*S*)-Stigmasta-6(5),22(23)-dien-3β-ol (50.0 g, 0.12 mol, 1.0 *eq.*) and 4-dimethylaminopyridine (2.96 g, 24.23 mmol) were weighed and dissolved in dichloromethane (500 mL). Triethylamine (61.90 g, 605.77 mmol) was added thereto, then acetic anhydride (37.11 g, 0.36 mol) was added thereto with stirring at room temperature, and the reaction mixture was stirred at room temperature for 2 hours. The reaction was monitored by TLC (petroleum ether: ethyl acetate = 10:1). After the reaction was completed, the reaction mixture was concentrated. The resulting crude product was added with methanol (300 mL), stirred, and filtered. The cake was washed with methanol (20 mL * 2) and dried to obtain (1*R*,3a*S*,3b*S*,7*S*,9a*R*,9b*S*,11a*R*)-1-[(2*R*,3*E*)-5-ethyl-6-methylhept-3-en-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate **(151-1)** (53.8 g, 112.4 mmol, 92.77%) as a white solid. ¹H NMR (399 MHz, Chloroform-d) δ 5.35 (d, *J =* 4.9 Hz, 1H), 5.14 (dd, *J* = 15.1, 8.5 Hz, 1H), 4.99 (dd, *J =* 15.2, 8.5 Hz, 1H), 4.67 - 4.48 (m, 1H), 2.30 (d, *J =* 7.8 Hz, 2H), 2.01 (s, 3H), 2.00 - 1.91 (m, 2H), 1.84 (d, *J =* 11.3 Hz, 2H), 1.68 (dd, *J =* 9.4, 4.8 Hz, 1H), 1.48 (dt, *J =* 24.5, 9.2 Hz, 7H), 1.28 - 1.07 (m, 6H), 1.00 (d, *J =* 3.2 Hz, 6H), 0.80 (dd, *J =* 20.1, 6.4 Hz, 9H), 0.68 (s, 3H).

**Step 2:** (1*R*,3a*S*,3b*S*,7*S*,9a*R*,9b*S*,11a*R*)-1-[(2*R*,3*E*)-5-Ethyl-6-methylhept-3-en-2-yl 9a,11a-dimethyl-2,3,3a,3b,4,6,7,8,9,9a,9b,10,11,11a-tetradecahydro-1*H*-cyclopenta[1,2-i]phenanthren-7-yl acetate **(151-1)** (5.0 g, 11 mmol) was weighed and dissolved in tetrahydrofuran (200 mL) and water (20.0 mL). Pyridine (2.12 mL, 27.5 mmol), N-methylmorpholinium oxide (5.15 g, 43.98 mmol), and potassium osmate (7.3 mL, 1.1 mmol) were added thereto at room temperature, and the reaction mixture was stirred at room temperature overnight. TLC (petroleum ether: ethyl acetate = 3:1) detected that an intermediate (vicinal diol) was generated. The reaction mixture was then added with sodium periodate (9.41 g, 43.98 mmol) at 0°C and stirred at room temperature for 1 hour. TLC (petroleum ether: ethyl acetate = 3:1) detected that the intermediate was converted into a product. The reaction mixture was then added with water (50 mL), extracted with ethyl acetate (50 mL * 3), dried, and concentrated to obtain a crude product, which was then purified by column chromatography (petroleum ether: ethyl acetate = 60:1) to obtain (1*R*,3a*S*,3b*S*,7*S*,9a*R*,9b*S*,11a*R*)-1-[(1*S*)-1-formylethyl]-9a,11a-dimethyl-2,3,3a,3b,4,6,7,8,9,9a,9b,10,11,11a-tetradecahydro-1*H*-cyclopenta[1,2-*i*]phenanthren-7-yl acetate **(151-2)** (0.64 g, 1.6 mmol, 14.73%) as a white solid. ¹H NMR (399 MHz, Chloroform-d) δ 9.55 (s, 1H), 5.36 (s, 1H), 4.60 (s, 1H), 2.31 (s, 3H), 2.02 (s, 3H), 1.93 (s, 2H), 1.84 (d, *J =* 11.2 Hz, 3H), 1.63 (s, 3H), 1.49 (d, *J =* 10.9 Hz, 4H), 1.42 - 1.19 (m, 3H), 1.15 (s, 1H), 1.11 (d, *J* = 6.8 Hz, 3H), 1.06 (s, 1H), 1.01 (s, 3H), 0.98 (d, *J* = 5.8 Hz, 1H), 0.71 (s, 3H).

**Step 3:** (Methoxymethyl)triphenylphosphonium chloride (8.7 g, 25.0 mmol) was weighed and dissolved in tetrahydrofuran (38 mL). The reaction system was replaced with nitrogen three times, cooled to 0°C, and added dropwise with sodium bis(trimethylsilyl)amide (12.8 mL, 25.0 mmol). After the dropwise addition was completed, the reaction mixture was stirred at 0°C for 0.5 hours, then added with a solution of (1*R*,3a*S*,3b*S*,7*S*,9a*R*,9b*S*,11a*R*)-1-[(1*S*)-1-formylethyl]-9a,11a-dimethyl-2,3,3a,3b,4,6,7,8,9,9a,9b,10,11,11a-tetradecahydro-1*H-*cyclopenta[1,2-i]phenanthren-7-yl acetate **(151-2)** (1.9 g, 5.1 mmol) in tetrahydrofuran (18 mL), warmed to room temperature, and stirred for 1 hour. The completion of the reaction was monitored by TLC (petroleum ether: ethyl acetate = 10:1). The reaction mixture was then added with water (30 mL), extracted with ethyl acetate (15 mL * 3), dried, and concentrated to obtain a crude product, which was then purified by column chromatography (petroleum ether: ethyl acetate = 100:1 to 30:1) to obtain (1*R*,3a*S*,3b*S*,7*S*,9a*R*,9b*S*,11a*R*)-1-[(2*S*,3*E*)-4-methoxybut-3-en-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate **(151-3)** (0.75 g, 1.8 mmol, 34.87%) as a white solid. ¹H NMR (399 MHz, Chloroform-d) δ 6.22 (d, *J* = 12.6 Hz, 1H), 5.71 (d, *J* = 6.4 Hz, 0H), 5.35 (d, *J =* 4.1 Hz, 1H), 4.57 (m, 2H), 4.15 (m, 0H), 3.52 (d, *J =* 10.6 Hz, 1H), 3.46 (d, *J* = 8.7 Hz, 3H), 2.59 (d, *J =* 6.6 Hz, 0H), 2.30(d, *J =* 7.6 Hz, 3H), 2.01 (s, 3H), 1.95 (d, *J =* 5.8 Hz, 3H), 1.84 (d, *J =* 10.9 Hz, 3H), 1.69 (d, *J =* 4.6 Hz, 1H), 1.52 (s, 2H), 1.48 (s, 2H), 1.14 (m, 3H), 1.03 (s, 3H), 1.00 (s, 3H), 0.67 (s, 3H).

**Step 4:** (1*R*,3a*S*,3b*S*,7*S*,9a*R*,9b*S*,11a*R*)-1-[(2*S*,3*E*)-4-Methoxybut-3-en-2-yl]-9a,11a-dimethyl-2,3,3a,3b,4,6,7,8,9,9a,9b,10,11,11a-tetradecahydro-1*H*-cyclopenta[1,2-i]phenanthren-7-yl acetate **(151-3)** (0.75 g, 1.87 mmol) was weighed and dissolved in tetrahydrofuran (7.5 mL), and 5 N hydrochloric acid (2.25 mL, 11.2 mmol) was added thereto. The reaction mixture was then heated to 50°C and stirred for 1 hour. TLC (petroleum ether: ethyl acetate = 10:1) monitored that the starting material was completely reacted. The reaction mixture was then added with water (15 mL), extracted with ethyl acetate (10 mL * 3), dried, and concentrated to obtain a crude product, which was then purified by column chromatography (petroleum ether: ethyl acetate = 50:1 to 20:1) to obtain (1*R*,3a*S*,3b*S*,7*S*,9a*R*,9b*S*,11a*R*)-1-[(2*R*)-1-formylpropan-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate **(151-4)** (0.52 g, 1.3 mmol, 68.26%) as a white solid. ¹H NMR (399 MHz, Chloroform-d) δ 9.74 (s, 1H), 5.36 (d, *J =* 4.4 Hz, 1H), 4.60 (s, 1H), 2.44 (d, *J =* 16.5 Hz, 1H), 2.30 (d, *J* = 6.7 Hz, 2H), 2.20 - 2.10 (m, 1H), 2.01 (s, 3H), 1.95 (d, *J =* 14.4 Hz, 2H), 1.84 (d, *J* = 11.5 Hz, 3H), 1.57 (dd, *J =* 19.7, 8.6 Hz, 4H), 1.46 (dd, *J* = 14.2, 10.3 Hz, 3H), 1.29 - 1.16 (m, 3H), 1.15 - 1.03 (m, 3H), 1.01 (d, *J =* 4.5 Hz, 6H), 0.96 - 0.89 (m, 1H), 0.70 (d, *J =* 12.6 Hz, 3H).

**Step 5:** (1*R*,3a*S*,3b*S*,7*S*,9a*R*,9b*S*,11a*R*)-1-[(2*R*)-1-Formylpropan-2-yl]-9a,11a-dimethyl-2,3,3a,3b,4,6,7,8,9,9a,9b,10,11,11a-tetradecahydro-1*H*-cyclopenta[1,2-*i*]phenanthren-7-yl acetate **(151-4)** (0.52 g, 1.34 mmol) was weighed and dissolved in tetrahydrofuran (30 mL), and (triphenyl-λ5-phosphanylidene)methyl acetate (2.25 g, 6.73 mmol) was added thereto. The reaction system was heated to 90°C and stirred for 3 hours. HPLC detected that there was no starting material remaining. The reaction mixture was then cooled to room temperature, added with water (15 mL), extracted with ethyl acetate (10 mL * 3), dried, and concentrated to obtain a crude product, which was then purified by column chromatography (petroleum ether: ethyl acetate = 50:1 to 20:1) to obtain methyl (2E,5R)-5-[(1*R*,3a*S*,3b*S*,7*S*,9a*R*,9b*S*,11a*R*)-7-acetoxy-9a,11a-dimethyl-2,3,3a,3b,4,6,7,8,9,9a,9b,10,11,11a-tetradecahydro-1*H*-cyclopenta[1,2-a]phenanthren-1-yl]hex-2-enoate **(151-5)** (0.44 g, 0.94 mmol, 70.20%) as a white solid. ¹H NMR (399 MHz, Chloroform-d) δ 6.99 - 6.86 (m, 1H), 5.80 (d, *J =* 15.6 Hz, 1H), 5.36 (s, 1H), 4.59 (s, 1H), 3.71 (s, 3H), 2.30 (d, *J =* 6.7 Hz, 3H), 2.01 (s, 3H), 2.00 - 1.90 (m, 3H), 1.84 (d, *J =* 10.9 Hz, 3H), 1.54 (d, *J* = 16.6 Hz, 5H), 1.50 - 1.37 (m, 3H), 1.33 - 1.17 (m, 2H), 1.16 - 1.02 (m, 4H), 1.00 (s, 3H), 0.93 (d, *J* = 6.6 Hz, 3H), 0.67 (s, 3H).

**Step 6:** Methyl (2*E*,5*R*)-5-[(1*R*,3a*S*,3b*S*,7*S*,9a*R*,9b*S*,11a*R*)-7-acetoxy-9a,11a-dimethyl-2,3,3a,3b,4,6,7,8,9,9a,9b,10,11,11a-tetradecahydro-1*H*-cyclopenta[1,2-*a*]phenanthren-1-yl]hex-2-enoate **(151-5)** (0.44 g, 1.0 mmol) was weighed and dissolved in a mixed solvent of tetrahydrofuran (9.0 mL) and methanol (4.5 mL). The reaction mixture was added with nickel chloride (130 mg, 1.0 mmol), then added with sodium borohydride (56.92 mg, 1.5 mmol) with stirring at room temperature to generate a large number of bubbles, and stirred at room temperature for 1 hour. A sample was taken and concentrated. NMR monitored that the starting material was completely reacted. The reaction mixture was added with water (15 mL), extracted with ethyl acetate (10 mL * 3), dried, and concentrated to obtain a crude product, which was then purified by column chromatography (petroleum ether: ethyl acetate = 60:1 to 20:1) to obtain methyl (5*R*)-5-[(1*R*,3a*S*,3b*S*,7*S*,9a*R*,9b*S*,11a*R*)-7-acetoxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate **(151-6)** (0.36 g, 0.78 mmol, 77.74%) as a white solid. ¹H NMR (399 MHz, Chloroform-d) δ 5.36 (s, 1H), 4.59 (s, 1H), 3.65 (s, 3H), 2.35 - 2.18 (m, 4H), 2.01 (s, 3H), 1.94 (d, *J =* 15.3 Hz, 2H), 1.84 (d, *J =* 10.9 Hz, 3H), 1.68 (s, 1H), 1.55 (s, 2H), 1.53 - 1.44 (m, 4H), 1.39 (d, *J =* 10.2 Hz, 3H), 1.22 (dd, *J* = 22.8, 12.0 Hz, 2H), 1.15 - 1.09 (m, 2H), 1.08 - 1.03 (m, 2H), 1.00 (s, 3H), 0.96 (s, 1H), 0.91 (d, *J =* 6.3 Hz, 3H), 0.89 - 0.79 (m, 1H), 0.64 (d, *J* = 12.6 Hz, 3H).

***Step 7*:** The reactant Methyl (5*R*)-5-[(1*R*,3a*S*,3b*S*,7*S*,9a*R*,9b*S*,11a*R*)-7-acetoxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate **(151-6)** (2 g, 4.50 mmol, 1.0 *eq*) was dissolved in methanol (50 mL), and concentrated sulfuric acid (98%) (1 mL, 18.76 mmol) was added dropwise thereto. The reaction mixture was then heated to reflux and stirred for 1 hour. The reaction was monitored by TLC (petroleum ether: EtOAc = 10:1). After the reaction was completed, the reaction mixture was concentrated to remove most of the methanol, then added with ethyl acetate, and washed twice with saturated sodium bicarbonate and once with saturated brine. The organic phase was dried over anhydrous sodium sulfate, filtered, and the filtrate was concentrated to dryness to obtain a crude product, which was then purified by flash silica gel column chromatography (petroleum ether: EtOAc = 90:10 to 80:20) to obtain methyl (5R)-5-[(1*R*,3a*S*,3b*S*,7*S*,9a*R*,9b*S*,11a*R*)-7-hydroxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate **(220-1)** (1.8 g, 4.024 mmol, 89.46%) as a white solid. ¹H NMR (400 MHz, Chloroform-d) δ 5.37 - 5.33 (m, 1H), 3.67 (s, 3H), 3.52 (dd, *J* = 7.7, 3.3 Hz, 1H), 2.35 - 2.20 (m, 4H), 2.03 - 1.93 (m, 2H), 1.84 (ddd, *J =* 12.5, 7.5, 4.3 Hz, 3H), 1.75 - 1.60 (m, 4H), 1.59 - 1.35 (m, 9H), 1.33 - 1.04 (m, 7H), 1.03 - 0.99 (m, 3H), 0.93 (d, *J* = 6.6 Hz, 3H), 0.68(s, 3H).

***Step 8:*** The reactant methyl (5*R*)-5-[(1*R*,3a*S*,3b*S*,7*S*,9a*S*,9b*S*,11a*R*)-7-hydroxy-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate **(220-1)** (1.7 g, 4.38 mmol) was dissolved in DMF (50 mL), then TBSCl (3.3 g, 21.9 mmol, 5.0 *eq*) and imidazole (0.30 g, 4.38 mmol, 1.0 *eq*) were sequentially added thereto, and the reaction mixture was heated to 100°C. The reaction was monitored by TLC (petroleum ether: ethyl acetate = 10:1). After the reaction was completed, the reaction mixture was cooled to room temperature, added with ethyl acetate (100 mL), and washed three times with water. The organic phase was then dried over anhydrous sodium sulfate, filtered, and the filtrate was concentrated. The crude product was purified by flash column chromatography (petroleum ether: ethyl acetate = 90:10) to obtain methyl (5R)-5-[(1*R*,3a*S*,3b*S*,7*S,*9a*R*,9b*S*,11a*R*)-7-{[dimethyl(2-methylpropan-2-yl)methylsilyl]oxy}-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate **(220-2)** (1.9 g, 3.40 mmol, 77.73%) as a colorless wax. ¹H NMR (400 MHz, Chloroform-d) δ 5.26 - 5.18 (m, 1H), 3.57 (s, 3H), 3.43 - 3.33 (m, 1H), 2.16 (d, *J=* 1.9 Hz, 4H), 1.93 - 1.82 (m, 2H), 1.65 - 1.55 (m, 2H), 1.39 (d, *J* = 3.0 Hz, 9H), 0.99 (s, 7H), 0.90 (s, 5H), 0.85 - 0.79 (m, 21H), 0.57 (s, 3H), -0.00 (s, 6H).

***Step 9*:** The reactant methyl (5*R*)-5-[(1*R*,3a*S*,3b*S*,7*S*,9a*R*,9b*S*,11a*R*)-7-{[dimethyl(2-methylpropan-2-yl)methylsilyl]oxy}-9a,11a-dimethyl-2,3,3a,3b,4,6,7,8,9,9a,9b,10,11,11a-tetradecahydro-1*H*-cyclopenta[1,2-*a*]phenanthren-1-yl]hexanoate **(220-2)** (1.8 g, 3.58 mmol, 1.0 *eq*) was dissolved in tetrahydrofuran (50 mL), then lithium aluminum hydride (0.168 g, 4.269 mmol, 1.2 *eq*) was added thereto, and the reaction mixture was stirred at room temperature for 30 minutes. The reaction was monitored by TLC (petroleum ether: EtOAc = 10:1). After the reaction was completed, the reaction mixture was quenched with sodium sulfate decahydrate, filtered, and the filtrate was concentrated to obtain a crude product, which was then purified by silica gel column chromatography (petroleum ether: ethyl acetate = 80:20 to 70:30) to obtain (5*R*)-5-[(1*R*,3a*S*,3b*S*,7*S*,9a*R*,9b*S*,11a*R*)-7-{[dimethyl(2-methylpropan-2-yl)methylsilyl]oxy}-9a,11a-dimethyl-2,3,3a,3b,4,6,7,8,9,9a,9b,10,11,11a-tetradecahydro-1*H-*cyclopenta[1,2-*a*]phenanthren-1-yl]hexan-1-ol **(220-3)** (1.7 g, 3.22 mmol, 90.01%) as a white solid. ¹H NMR (400 MHz, Chloroform-d) δ 5.26 (d, *J* = 5.3 Hz, 1H), 3.59 (t, *J* = 6.6 Hz, 2H), 3.47 - 3.37 (m, 1H), 2.27 - 2.17 (m, 1H), 2.11 (ddd, *J* = 13.4, 5.0, 2.2 Hz, 1H), 1.93 (s, 2H), 1.77 (s, 2H), 1.66 (d, *J* = 12.2 Hz, 1H), 1.47 (ddd, *J* = 13.9, 8.7, 4.5 Hz, 7H), 1.37 - 1.29 (m, 3H), 1.25 - 1.15 (m, 2H), 1.13 - 0.97 (m, 5H), 0.94 (s, 3H), 0.87 (d, *J* = 6.3 Hz, 4H), 0.84 - 0.81 (m, 10H), 0.62 (s, 3H), 0.02 - -0.02 (m, 6H).

***Step 10*:** The compound (5*R*)-5-[(1*R*,3a*S*,3b*S*,7*S,*9a*R*,9b*S*,11a*R*)-7-{[dimethyl(2-methylpropan-2-yl)methylsilyl]oxy}-9a,11a-dimethyl-2,3,3a,3b,4,6,7,8,9,9a,9b,10,11,11a-tetradecahydro-1*H*-cyclopenta[1,2-*a*]phenanthren-1-yl]hexan-1-ol **(220-3)** (1.0 g, 2.11 mmol, 1.0 *eq*) was dissolved in dichloromethane (100 mL), then Dess-Martin periodinane (1.068 g, 2.532 mmol, 1.2 *eq*) was added thereto, and the reaction mixture was stirred at room temperature. The reaction was monitored by TLC (petroleum ether: ethyl acetate = 5:1). After the reaction was completed, the reaction mixture was quenched with saturated sodium sulfite and washed with saturated sodium bicarbonate. The organic phase was dried over anhydrous sodium sulfate, concentrated, and the crude product was purified by silica gel column chromatography (petroleum ether: ethyl acetate = 90:10 to 80:20) to obtain (5*R*)-5-[(1*R*,3a*S*,3b*S*,7*S*,9a*R*,9b*S*,11a*R*)-7-{[dimethyl(2-methylpropan-2-yl)methylsilyl]oxy}-9a,11a-dimethyl-2,3,3a,3b,4,6,7,8,9,9a,9b,10,11,11a-tetradecahydro-1*H*-cyclopenta[1,2-*a*]phenanthren-1-yl]hexanal **(220-4)** (0.6 g, 1.142 mmol, 54.23%) as a white solid. ¹H NMR (400 MHz, Chloroform-d) δ 9.71 (t, *J =* 1.8 Hz, 1H), 5.26 (d, *J =* 5.3 Hz, 1H), 3.49 - 3.36 (m, 1H), 2.34 (d, *J =* 6.6 Hz, 2H), 2.25 - 2.07 (m, 2H), 1.93 (s, 2H), 1.81 - 1.60 (m, 4H), 1.48-1.30 (m, 8H), 1.03 (ddd, *J =* 14.6, 12.0, 6.3 Hz, 7H), 0.94 (s, 4H), 0.89 (d, *J =* 6.5 Hz, 4H), 0.85 - 0.81 (m, 9H), 0.62 (s, 3H), 0.02 - -0.02 (m, 7H).

***Step 11**:* The starting material 1-bromo-2-methoxybenzene (500 mg, 2.67 mmol, 1.0 *eq)* was dissolved in anhydrous tetrahydrofuran (10 mL). The reaction system was cooled to -78°C, added with *n*-butyllithium (1.17 mL, 2.94 mmol, 1.1 *eq),* and stirred at the same temperature for 2 hours to obtain a solution of (2-methoxyphenyl)lithium in tetrahydrofuran (0.25 M) **(311-2).**

***Step 12:*** The compound (5*R*)-5-[(1*R*,3a*S*,3b*S*,7*S*,9a*R*,9b*S*,11a*R*)-7-{[dimethyl(2-methylpropan-2-yl)methylsilyl]oxy}-9a,11a-dimethyl-2,3,3a,3b,4,6,7,8,9,9a,9b,10,11,11a-tetradecahydro-1*H*-cyclopenta[1,2-*a*]phenanthren-1-yl]hexanal (200 mg, 0.411 mmol, 1.0 *eq)* was dissolved in tetrahydrofuran (5 mL), and the reaction system was cooled to 0°C. (2-Methoxyphenyl)lithium **(311-2)** (2.46 mL, 0.616 mmol, 1.5 *eq)* prepared in the previous step was then slowly added thereto, and the reaction system was stirred at room temperature for 1 hour. The completion of the reaction was monitored by TLC plate (petroleum ether: ethyl acetate = 5:1), and the starting material was consumed. The reaction system was added with water (20 mL) and extracted with ethyl acetate (50 mL × 3). The organic phases were combined, washed with water (30 mL), dried, and concentrated to obtain a crude product. The crude product was subjected to silica gel column chromatography (petroleum ether: ethyl acetate = 88:12 to 85:15) to obtain (5*R*)-5-[(1*R*,3a*S*,3b*S*,7*S*,9a*R*,9b*S*,11a*R*)-7-{[dimethyl(2-methylpropan-2-yl)methylsilyl]oxy}-9a,11a-dimethyl-2,3,3a,3b,4,6,7,8,9,9a,9b,10,11,11a-tetradecahydro-1*H*-cyclopenta[1,2-*a*]phenanthren-1-yl]-1-(2-methoxyphenyl)hexan-1-ol **(311-1)** (130 mg, purity: 90%, yield: 47.87%) as a white solid. ¹H NMR (400 MHz, CDCl₃) δ 7.27 - 7.22 (m, 1H), 7.17 (d, *J =* 7.4 Hz, 1H), 6.92 - 6.86 (m, 1H), 6.82 (dd, *J* = 8.4, 5.0 Hz, 2H), 5.26 (d, *J =* 5.0 Hz, 1H), 4.79 (dt, *J =* 9.8, 5.8 Hz, 1H), 3.84 (s, 1H), 3.80 (s, 3H), 3.48-3.34 (m, 1H), 2.21 (t, *J =* 11.3 Hz, 1H), 2.11 (dt, *J* = 13.2, 6.5 Hz, 1H), 1.91 (d, *J =* 15.3 Hz, 2H), 1.70 (ddd, *J =* 21.4, 13.7, 5.2 Hz, 5H), 1.55 - 1.27 (m, 10H), 1.13 - 0.91 (m, 10H), 0.85 - 0.79 (m, 12H), 0.59 (dd, *J =* 13.1, 2.6 Hz, 3H), 0.03 - -0.02 (m, 6H).

***Step 13:*** The reactant (5*R*)-5-[(1*R*,3a*S*,3b*S*,7*S*,9a*R*,9b*S*,11a*R*)-7-{[dimethyl(2-methylpropan-2-yl)methylsilyl]oxy}-9a,11a-dimethyl-2,3,3a,3b,4,6,7,8,9,9a,9b,10,11,11a-tetradecahydro-1*H*-cyclopenta[1,2-*a*]phenanthren-1-yl]-1-(2-methoxyphenyl)hexan-1-ol **(311-1)** (60 mg, 1.101 mmol) was dissolved in tetrahydrofuran (5 mL), then a 1.0 M solution of TBAF in tetrahydrofuran (0.5 mL, 0.5 mmol) was added thereto, and the reaction system was stirred at 50°C for 1 hour. The reaction was monitored by TLC plate (petroleum ether: ethyl acetate = 3:1). After the reaction was completed, the reaction system was added with water (20 mL), and the aqueous phase was extracted with ethyl acetate (25 mL × 3). The ethyl acetate phases were combined and washed with saturated brine (10 mL × 3). The ethyl acetate phase was dried over anhydrous sodium sulfate, filtered, and concentrated to obtain a crude product. The crude product was purified by column chromatography (petroleum ether: ethyl acetate = 95:5 to 60:40) to obtain 24-[hydroxy(2-methoxyphenyl)methyl]cholan-6(5)-en-3β-ol **(311)** (40 mg, purity: 98.78%, yield: 78.38%) as a white solid. ¹H NMR (399 MHz, Chloroform-d) δ 7.23 - 7.25 (s, 3H), 6.96 (d, *J =* 8.3 Hz, 1H), 6.86 (d, *J =* 8.3 Hz, 1H), 4.82 (d, *J* = 9.8 Hz, 1H), 3.84 (s, 3H), 3.57 (s, 1H), 1.94 (d, *J* = 12.4 Hz, 1H), 1.85 - 1.59 (m, 6H), 1.37 (s, 6H), 1.30 (d, *J* = 19.2 Hz, 3H), 1.24 (s, 6H), 1.06 (d, *J* = 11.7 Hz, 7H), 0.87 (s, 3H), 0.79 (s, 3H), 0.62 (s, 3H). ¹³C NMR (101 MHz, Chloroform-d) δ 128.15, 127.00, 120.70, 110.47, 71.36, 56.46, 55.24, 54.31, 44.81, 42.57, 40.00, 38.18, 37.79, 36.96, 35.43, 32.06, 31.50, 31.42, 30.16, 29.66, 28.71, 28.23, 24.18, 21.22, 18.55, 12.30, 12.05, 1.00. LCMS: [Ms-OH]⁺ = 465.5.

### Example 312

### Preparation of compound 24-[hydroxy(2-methoxyphenyl)methyl]-5α-cholan-3β-ol

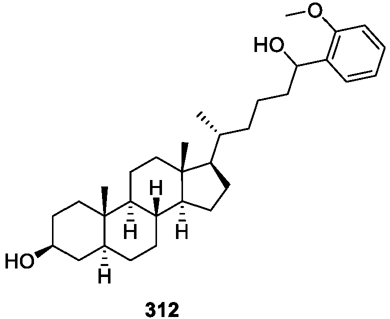

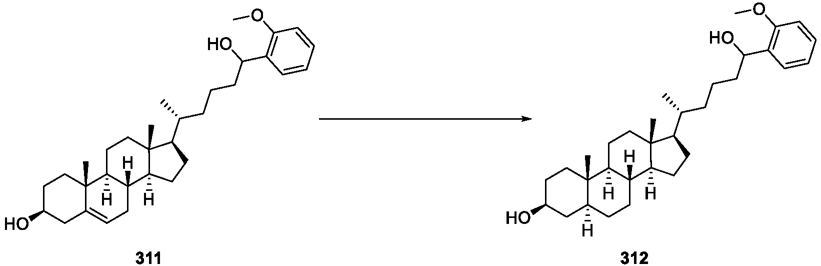

24-[Hydroxy(2-methoxyphenyl)methyl]cholan-6(5)-en-3β-ol **(311)** (15 mg, 0.03 mmol) was weighed and dissolved in methanol (1.5 mL), and Pd/C (3.0 mg) was added thereto. The reaction system was then replaced with hydrogen and stirred at room temperature. The reaction was detected by NMR. After the reaction was completed, the reaction mixture was filtered to remove Pd/C, and the filtrate was concentrated to obtain a crude product, which was then purified by column chromatography (petroleum ether: ethyl acetate = 0 to 30%) to obtain 24-[hydroxy(2-methoxyphenyl)methyl]-5α-cholan-3β-ol **(312)** (6.3 mg, 0.013 mmol, 42%) as a white solid with a purity of 95.27%. ¹H NMR (399 MHz, Chloroform-d) δ 7.23 - 7.25 (s, 3H), 6.96 (d, *J =* 8.3 Hz, 1H), 6.86(d, *J =* 8.3 Hz, 1H), 4.82 (d, *J =* 9.8 Hz, 1H), 3.84 (s, 3H), 3.57 (s, 1H), 1.94 (d, *J =* 12.4 Hz, 1H), 1.85 - 1.59 (m, 6H), 1.37 (s, 6H), 1.30 (d, *J =* 19.2 Hz, 3H), 1.24 (s, 6H), 1.06 (d, *J* = 11.7 Hz, 7H), 0.87 (s, 3H), 0.79 (s, 3H), 0.62 (s, 3H). ¹³C NMR (101 MHz, Chloroform-d) δ 128.15, 127.00, 120.70, 110.47, 71.36, 56.46, 55.24, 54.31, 44.81, 42.57, 40.00, 38.18, 37.79, 36.96, 35.43, 32.06, 31.50, 31.42, 30.16, 29.66, 28.71, 28.23, 24.18, 21.22, 18.55, 12.30, 12.05, 1.00. LCMS: [M-OH]⁺ = 465.5.

### Example 313

### Preparation of (1R,3aS,3bS,5aR,6R,7S,9aR,9bS,11aR)-6-hydroxy-1-[(2R)-6-hydroxy-6-methylheptan-2-yl]-9a,11a-dimethylhexadecahydro-1H-cyclopenta[1,2-i]phenanthren-7-yl hydroxyacetate (compound 313)

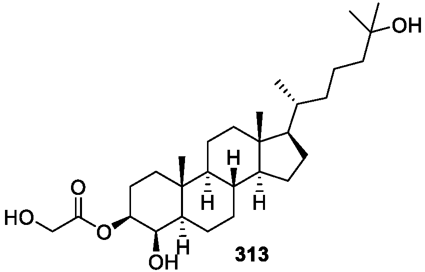

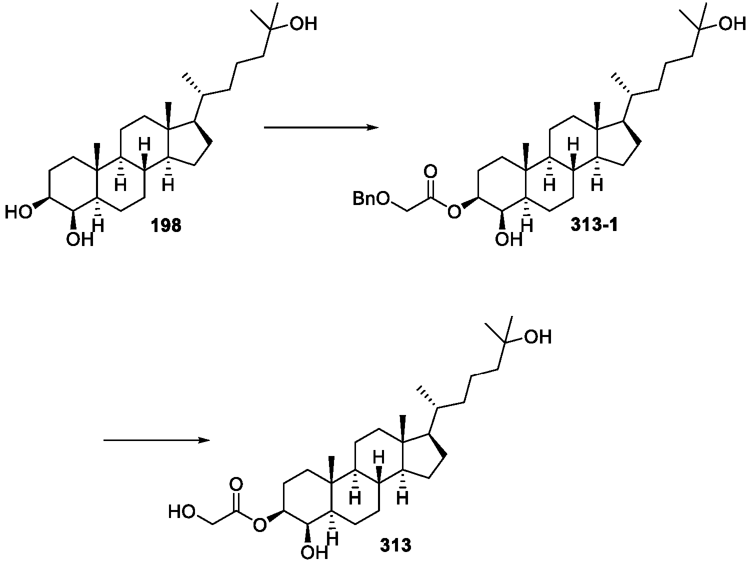

**Step 1:** 5α-Cholest-3β,4β,25-triol **(198)** (90 mg, 0.21 mmol) was dissolved in tetrahydrofuran (5 mL), and diisopropylethylamine (0.07 mL, 0.42 mmol) was added thereto. The reaction mixture was cooled in an ice bath, and (benzyloxy)acetyl chloride (42.7 mg, 0.23 mmol) was added dropwise thereto. After the dropwise addition was completed, the reaction mixture was warmed to room temperature, stirred for 30 minutes, then heated to 40°C, and stirred for 18 hours. The reaction was monitored by TLC (petroleum ether: ethyl acetate = 5:1). After the reaction was completed, the reaction mixture was concentrated to remove the solvent, and extracted with water and ethyl acetate. The organic phase was separated, dried, concentrated, and purified by column chromatography (petroleum ether: ethyl acetate = 0% to 30%) to obtain (1*R,*3a*S*,3b*S*,5a*R*,6*R*,7*S*,9a*R*,9b*S*,11a*R*)-6-hydroxy-1-[(2*R*)-6-hydroxy-6-methylheptan-2-yl]-9a,11a-dimethylhexadecahydro-1*H*-cyclopenta[1,2-*i*]phenanthren-7-yl (benzyloxy)acetate **(313-1)** (40 mg, 0.07 mmol, 32.87%) as a white solid. ¹H NMR (399 MHz, Chloroform-d) δ 7.35 (d, *J =* 4.3 Hz, 4H), 7.31 (dd, *J* = 7.6, 3.7 Hz, 1H), 4.81 (dt, *J* = 12.2, 3.9 Hz, 1H), 4.62 (s, 2H), 4.10 (s, 2H), 1.92 (ddd, *J =* 16.8, 11.2, 3.4 Hz, 2H), 1.76 (td, *J =* 13.6, 3.6 Hz, 4H), 1.70-1.51 (m, 8H), 1.44 - 1.28 (m, 10H), 1.24 (q, *J =* 4.1 Hz, 3H), 1.19 (s, 6H), 1.14 - 1.06 (m, 4H), 1.03 (s, 5H), 0.90 (d, *J =* 6.4 Hz, 3H), 0.85 - 0.80 (m, 1H), 0.63 (s, 3H), 0.58 (dd, *J =* 11.3, 4.0 Hz, 1H).

**Step 2:** (1*R*,3a*S*,3b*S*,5a*R*,6*R*,7*S*,9a*R*,9b*S*,11a*R*)-6-Hydroxy-1-[(2*R*)-6-hydroxy-6-methylheptan-2-yl]-9a,11a-dimethylhexadecahydro-1*H*-cyclopenta[1,2-*i*]phenanthren-7-yl (benzyloxy)acetate **(313-1)** (40 mg, 0.07 mmol) was dissolved in methanol (5 mL), and Pd/C (10% Pd, containing 40 to 60% water) (10 mg, 0.094 mmol) was added thereto. The reaction system was then replaced with hydrogen three times and heated to 40°C. The reaction was monitored by TLC (petroleum ether: ethyl acetate = 3:1). After the reaction was completed, the reaction mixture was filtered to remove Pd/C, concentrated, and purified by column chromatography (petroleum ether: ethyl acetate = 0% to 30%) to obtain (1*R*,3a*S*,3b*S*,5a*R*,6*R,*7*S*,9a*R*,9b*S*,11a*R*)-6-hydroxy-1-[(2*R*)-6-hydroxy-6-methylheptan-2-yl]-9a,11a-dimethylhexadecahydro-1*H*-cyclopenta[1,2-*i*]phenanthren-7-yl hydroxyacetate **(313)** (22 mg, 0.046 mmol, 65.4%) as a white solid with a purity of 80.69%. ¹H NMR (399 MHz, Chloroform-d) δ 4.83 (d, *J =* 12.0 Hz, 1H), 4.17 (s, 2H), 3.86 (s, 1H), 1.94 (d, *J =* 13.0 Hz, 2H), 1.81 - 1.69 (m, 5H), 1.55 (d, *J =* 9.0 Hz, 3H), 1.47 - 1.29 (m, 9H), 1.20 (s, 6H), 1.16 - 1.06 (m, 4H), 1.03 (s, 3H), 0.97 (dt, *J =* 15.7, 5.3 Hz, 2H), 0.90 (d, *J =* 6.5 Hz, 3H), 0.88 - 0.78 (m, 1H), 0.63 (s, 3H), 0.60 (s, 1H). ¹³C NMR (100 MHz, Chloroform-d) δ 72.82, 71.10, 60.74, 56.46, 56.12, 55.18, 48.72, 44.39, 42.57, 39.81, 36.79, 36.39, 35.72, 35.54, 35.40, 32.24, 29.33, 29.19, 28.22, 25.67, 24.17, 22.17, 20.74, 20.53, 18.61, 14.68, 12.05. LCMS: [M+MeCN+Na]⁺ = 542.4.

### Example 316

### Preparation of 24-[(4-fluorophenyl)(hydroxy)methyl]-5α-cholan-3β,4β-diol (compound 316)

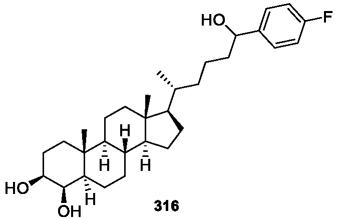

Referring to example **275,** 2,4,6-trifluorobromobenzene was replaced with *p-*fluorobrombenzene to obtain compound 24-[(4-fluorophenyl)(hydroxy)methyl]-5α-cholan-3β,4β-diol **(316).** ¹H NMR (399 MHz, Chloroform-d) δ 7.30 (dd, *J =* 8.4, 5.5 Hz, 2H), 7.02 (t, *J =* 8.6 Hz, 2H), 4.63 (t, *J =* 6.4 Hz, 1H), 3.72 (s, 1H), 3.53 (s, 1H), 1.93 (d, *J =* 12.6 Hz, 2H), 1.72 (tdd, *J =* 21.2, 12.4, 5.4 Hz, 8H), 1.39 - 1.23 (m, 9H), 1.22 - 1.06 (m, 2H), 1.07-1.01 (m, 3H), 1.00 (s, 3H), 0.98 - 0.88 (m, 3H), 0.86 (d, *J =* 5.9 Hz, 3H), 0.62 (d, *J =* 2.5 Hz, 3H), 0.56 (t, *J* = 7.6 Hz, 1H). ¹³C NMR (100 MHz, CDCl₃) δ 160.88, 140.67, 127.55, 127.47, 127.39, 115.31, 115.30, 115.08, 74.80, 74.17, 74.00, 72.27, 56.52, 56.09, 55.19, 48.78, 42.57, 39.86, 39.65, 39.63, 36.83, 35.75, 35.66, 35.45, 35.36, 32.36, 29.68, 29.31, 29.23, 29.10, 28.24, 25.96, 25.83, 24.17, 22.38, 22.29, 20.55, 18.56, 18.54, 14.65, 14.12, 12.05, 1.01. ¹⁹F NMR (376 MHz, Chloroform-d) δ -115.23, -115.24, -115.25, -115.27, -115.27, -115.29, -115.30,-115.31, -115.33, -115.34, -115.35. LCMS: [M-H₂O]⁺ = 467.45.

### Example 317

### Preparation of compound 24-(hydroxy{2-[(trifluoromethyl)oxy]phenyl}methyl)-5α-cholan-3β,4β-diol

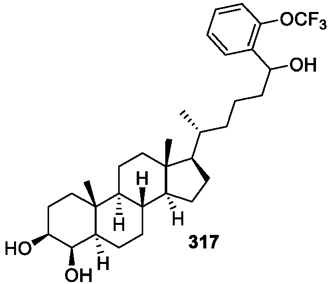

Referring to example **275,** 2,4,6-trifluorobromobenzene was replaced with *o-*trifluoromethoxybromobenzene to obtain compound 24-(hydroxy{2-[(trifluoromethyl)oxy]phenyllmethyl)-5α-cholan-3β,4β-diol **(317).** ¹H NMR (399 MHz, Chloroform-d) δ 7.58 (d, *J=* 6.9 Hz, 1H), 7.35 - 7.27 (m, 2H), 7.22 (s, 1H), 3.73 (s, 1H), 5.04 (s, 1H), 3.55 (s, 1H), 1.90 (s, 4H), 1.73 (d, *J =* 13.8 Hz, 5H), 1.66 (d, *J =* 7.2 Hz, 3H), 1.41-1.32 (m, 6H), 1.28 (s, 2H), 1.20 (s, 1H), 1.05 (s, 4H), 1.00 (s, 3H), 0.94 (d, *J =* 11.2 Hz, 2H), 0.90 - 0.86 (m, 3H), 0.63 (s, 3H), 0.58 - 0.55 (m, 1H). ¹³C NMR (100 MHz, CDCl₃) δ 137.24, 128.43, 127.41, 127.39, 127.00, 119.93, 74.81, 72.27, 68.13, 67.94, 56.52, 56.15, 55.20, 48.79, 42.58, 39.87, 38.54, 38.42, 36.83, 35.66, 35.64, 35.50, 35.45, 35.37, 32.36, 28.18, 25.97, 25.83, 24.18, 22.37, 22.23, 20.56, 18.51, 18.47, 14.65, 12.04. ¹⁹F NMR (376 MHz, Chloroform-d) δ -56.83 (d, *J* = 4.2 Hz). LCMS: [M-H₂O-OH]⁺ = 517.5.

### Example 320

### Preparation of 4β-hydroxy-3β-hydroxy-24-[hydroxy(2-methoxyphenyl)methyl]-5α-cholan-7-one (compound 320)

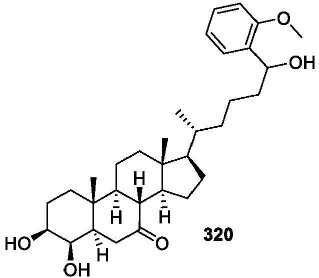

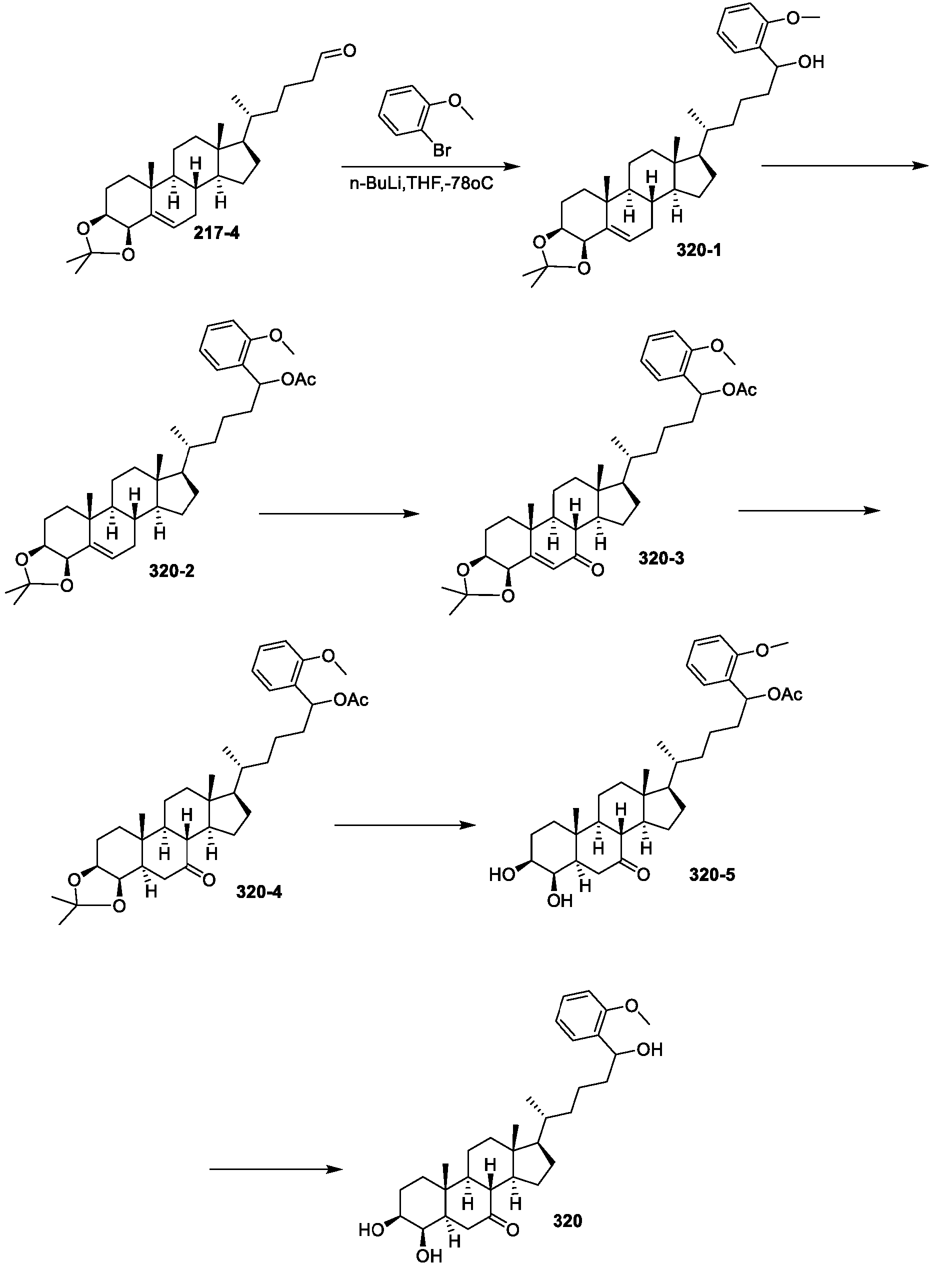

***Step 1:*** The reagent 1-bromo-2-methoxybenzene (1.3 g, 1.40 mmol, 3.0 *eq)* was dissolved in tetrahydrofuran (5 mL), and the reaction mixture was cooled to -78°C in a dry ice bath under a nitrogen atmosphere. A 2.5 M solution of *n*-butyllithium in *n*-hexane (3.34 mL, 1.680 mmol, 3.6 *eq)* was then added thereto, and the reaction mixture was reacted for 0.5 hours. A solution of (5*R*)-5-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]hexanal **(217-4)** (1000 mg, 0.467 mmol, 1.0 *eq)* in tetrahydrofuran (10 mL) was then added thereto. The completion of the reaction was monitored by TLC (petroleum ether: ethyl acetate = 5:1). The reaction mixture was quenched with saturated ammonium chloride and extracted with ethyl acetate. The organic phase was dried over anhydrous sodium sulfate, and the crude product was purified by flash column chromatography (petroleum ether: ethyl acetate = 80:20) to obtain (5R)-5-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1-(2,6-difluorophenyl)hexan-1-ol **(320-1)** (750 mg, 1.257 mmol, purity: 90%, yield: 53.9%) as a white solid. ¹H NMR (400 MHz, CDCl₃) δ 7.30 (ddd, *J =* 7.4, 3.9, 1.6 Hz, 1H), 7.23 (m, 1H), 6.95 (t, *J =* 7.4 Hz, 1H), 6.88 (d, *J =* 8.2 Hz, 1H), 5.74 (dd, *J =* 49.3, 2.9 Hz, 1H), 4.85 (d, *J =* 7.3 Hz, 1H), 4.40 (d, *J =* 5.8 Hz, 1H), 4.14 (d, *J =* 3.6 Hz, 0H), 3.85 (s, 3H), 3.56 (dd, *J =* 7.3, 3.8 Hz, 1H), 2.05 (m, 3H), 1.71 (m, 10H), 1.42 (d, *J* = 8.4 Hz, 4H), 1.17 (d, *J =* 6.5 Hz, 4H), 1.06 (ddd, *J =* 25.8, 14.6, 5.0 Hz, 6H), 0.90 (dt, *J =* 7.5, 3.7 Hz, 4H), 0.68 (dd, *J =* 4.6, 2.7 Hz, 3H).

***Step* 2:** The compound (5*R*)-5-[(3a*S*,5a*R*,5b*S*,7a*R,*8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1-(2-methoxyphenyl)hexan-1-ol (320-1) (750 mg, 1.397 mmol, 1.0 *eq)* was dissolved in dichloromethane (50 mL). Acetic anhydride (0.197 mL, 2.096 mmol, 1.2 *eq),* triethylamine (0.388 mL, 2.794 mmol, 2.0 *eq),* and 4-dimethylaminopyridine (34.14 mg, 0.279 mmol, 0.2 *eq)* were added thereto, and the resulting reaction mixture was stirred at 25°C for 3 hours under a N₂ atmosphere. The reaction was monitored by TLC plate (petroleum ether: ethyl acetate = 5:1). After the reaction was completed, the reaction system was quenched with methanol, added with water (10 mL), and the aqueous phase was extracted with dichloromethane (15 mL × 3). The dichloromethane phases were combined and washed with saturated brine (5 mL). The dichloromethane phase was dried over Na₂SO₄, filtered, and concentrated to obtain a crude product. The crude product was purified by silica gel column chromatography (petroleum ether: ethyl acetate = 90:10) to obtain (5*R*)-5-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1-(2-methoxyphenyl)hexyl acetate **(320-2)** (750 mg, 1.166 mmol, 83.46%) as a white solid. ¹H NMR (400 MHz, CDCl₃) δ 7.23 (m, 1H), 6.86 (t, *J =* 8.0 Hz, 2H), 6.05 (t, *J =* 7.5 Hz, 1H), 5.80 (d, *J =* 2.6 Hz, 1H), 4.40 (d, *J* = 5.8 Hz, 1H), 4.10 (dd, *J =* 13.7, 6.2 Hz, 1H), 2.11 (dd, *J =* 12.6, 4.2 Hz, 1H), 2.06 (s, 3H), 2.01 (m, 2H), 1.77 (m, 3H), 1.61 (m, 6H), 1.41 (d, *J =* 12.5 Hz, 4H), 1.30 (m, 6H), 1.06 (m, 11H), 0.87 (m, 5H), 0.67 (d, *J =* 4.0 Hz, 3H).

***Step 3:*** The compound (5*R*)-5-[(3a*S*,5a*R*,5b*S*,7a*R*,8*R*,10a*S*,10b*S*,12b*R*)-2,2,5a,7a-tetramethyl-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1-(2-methoxyphenyl)hexyl acetate **(320-2)** (750 mg, 1.296 mmol) was dissolved in acetone (30 mL). *N*-Hydroxyphthalimide (42.27 mg, 0.259 mmol), *tert*-butyl hydroperoxide (1.037 mL, 5.183 mmol), and anhydrous cobalt (II) acetate (11.47 mg, 0.065 mmol) were added thereto, and the resulting reaction mixture was stirred at 25°C for 18 hours under a N₂ atmosphere. The reaction was monitored by TLC (petroleum ether: ethyl acetate = 5:1). After the reaction was completed, the reaction mixture was diluted with ethyl acetate and quenched with saturated sodium sulfite, and the aqueous phase was extracted with ethyl acetate (10 mL × 3). The ethyl acetate phases were combined and washed with saturated brine (15 mL). The ethyl acetate phase was dried over Na₂SO₄, filtered, and concentrated to obtain a crude product. The crude product was purified by silica gel column chromatography (petroleum ether: ethyl acetate = 80:20) to obtain (SR)-5-[(3a*S*,5a*R*,5b*S*,7a*R,*8*R*,10a*S*,10b*S*,12b*R*)-2,2,5a,7a-tetramethyl-11-oxo-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1-(2-methoxyphenyl)hexyl acetate **(320-3)** (340 mg, 0.516 mmol, purity: 90%, yield: 39.84%) as a white solid. ¹H NMR (400 MHz, CDCl₃) δ 7.30 (d, *J =* 7.5 Hz, 1H), 7.23 (d, *J =* 8.3 Hz, 1H), 6.94 (t, *J =* 7.5 Hz, 1H), 6.86 (d, *J =* 8.2 Hz, 1H), 6.16 (s, 1H), 5.92 (s, 1H), 4.52 (d, *J =* 6.4 Hz, 1H), 4.33 (d, *J =* 5.7 Hz, 1H), 3.84 (s, 3H), 2.35 (d, *J =* 10.3 Hz, 2H), 2.08 (d, *J =* 1.8 Hz, 3H), 2.05 (s, 1H), 1.80 (m, 4H), 1.63 (dd, *J =* 16.1, 10.4 Hz, 3H), 1.57 (s, 4H), 1.42 (m, 2H), 1.37 (s, 3H), 1.33 (s, 4H), 1.26 (m, 2H), 1.09 (dd, *J =* 38.3, 9.0 Hz, 4H), 0.88 (dt, *J =* 7.8, 3.9 Hz, 3H), 0.69 (d, *J =* 1.7 Hz, 3H).

***Step 4:*** The compound (5*R*)-5-[(3a*S*,5a*R*,5b*S*,7a*R*,8*R*,10a*S*,10b*S*,12b*R*)-2,2,5a,7a-tetramethyl-11-oxo-4,5,5a,5b,6,7,7a,8,9,10,10a,10b,11,12b-tetradecahydro-3a*H-*cyclopenta[1',2':1,2]phenanthro[7,8-*d*][1,3]dioxol-8-yl]-1-(2-methoxyphenyl)hexyl acetate **(320-3)** (340 mg, 0.574 mmol) was dissolved in methanol (20 mL), then Pd/C (100 mg, 0.940 mmol) was added thereto, and the resulting reaction mixture was stirred at 25°C for 1 hour under a H₂ atmosphere. The reaction was monitored by TLC (petroleum ether: ethyl acetate = 5:1). After the reaction was completed, the reaction mixture was filtered and concentrated to obtain a crude product. The crude product was purified by silica gel column chromatography (petroleum ether: ethyl acetate = 80:20) to obtain (5*R*)-5-[(3a*S*,5a*R*,5b*S*,7a*R*,4*R*,10a*S*,10b*S*,12a*R*,12b*R*)-2,2,5a,7a-tetramethyl-11-oxo-4,5,5a,5b,6,7,7a,8,9,10,10a,10b,11,12,12a,12b-hexadecahydro-3a*H-*cyclopenta[1',2':1,2]phenanthro[7,8-*d*][1,3]dioxol-8-yl]-1-(2-methoxyphenyl)hexyl acetate (320-4) (250 mg, 0.378 mmol, purity: 90%, yield: 56.06%) as a white solid. ¹H NMR (400 MHz, CDCl₃) δ 7.29 (d, *J =* 7.6 Hz, 1H), 7.23 (d, *J =* 8.2 Hz, 1H), 6.94 (t, *J =* 7.5 Hz, 1H), 6.86 (d, *J =* 8.2 Hz, 1H), 6.16 (t, *J =* 6.3 Hz, 1H), 3.99 (dt, *J =* 8.4, 7.3 Hz, 2H), 3.84 (d, *J =* 6.9 Hz, 3H), 2.77 (t, *J =* 13.5 Hz, 1H), 2.42 (t, *J =* 11.2 Hz, 1H), 2.21 (d, *J=* 2.7 Hz, 1H), 2.08 (d, *J =* 0.9 Hz, 3H), 1.97 (d, *J =* 12.5 Hz, 1H), 1.87 (m, 2H), 1.74 (m, 3H), 1.63 (d, *J =* 14.5 Hz, 2H), 1.41 (m, 5H), 1.30 (s, 6H), 1.25 (m, 3H), 1.02 (m, 6H), 0.86 (m, 7H), 0.64 (d, *J =* 1.5 Hz, 3H).

***Step 5:*** The compound (5*R*)-5-[(3a*S*,5a*R*,5b*S*,7a*R*,4*R,*10a*S*,10b*S*,12a*R*,12b*R*)-2,2,5a,7a-tetramethyl-11-oxo-4,5,5a,5b,6,7,7a,8,9,10,10a,10b,11,12,12a,12b-hexadecahydro-3a*H*-cyclopenta[1',2':1,2]phenanthro[7,8-*d*][1,3]dioxol-8-yl]-1-(2-methoxyphenyl)hexyl acetate **(320-4)** (50 mg, 0.084 mmol) was dissolved in tetrahydrofuran (5 mL), then dilute hydrochloric acid (0.028 mL) was added thereto, and the resulting reaction mixture was stirred at 25°C for 3 hours under a N₂ atmosphere. The reaction was monitored by TLC (petroleum ether: ethyl acetate = 5:1). After the reaction was completed, the reaction mixture was cooled to room temperature, carefully added dropwise to an ice-water mixture, and the aqueous phase was extracted with ethyl acetate (25 mL × 3). The ethyl acetate phases were combined and washed with saturated brine (15 mL). The EA phase was dried over Na₂SO₄, filtered, and concentrated to obtain a crude product. The crude product was purified by silica gel column chromatography (petroleum ether: ethyl acetate = 50:50) to obtain (5*R*)-5-[(1*R*,3a*S*,3b*S,*5a*R*,6*R*,7*S*,9a*R*,9b*S*,11a*R*)-6,7-dihydroxy-9a,11a-dimethyl-4-oxohexadecahydro-1*H*-cyclopenta[1,2-*a*]phenanthren-1-yl]-1-(2-methoxyphenyl)hexyl acetate **(320-5)** (30 mg, 0.049 mmol, purity: 90%, yield: 57.90%) as a white solid. ¹H NMR (400 MHz, CDCl₃) δ 7.33 - 7.27 (m, 1H), 7.23 (s, 1H), 6.95 (d, *J =* 7.5 Hz, 1H), 6.86 (d, *J =* 8.2 Hz, 1H), 6.15 (s, 1H), 3.83 (s, 3H), 3.72 (s, 1H), 3.62 - 3.55 (m, 1H), 2.87 (s, 1H), 2.38 (s, 1H), 2.13 (s, 2H), 1.97 (d, *J =* 13.9 Hz, 2H), 1.76 (ddd, *J =* 30.5, 18.7, 7.0 Hz, 12H), 1.54-1.44 (m, 4H), 1.42 - 1.32 (m, 5H), 1.30 - 1.16 (m, 12H), 1.03 (dt, *J =* 19.0, 8.2 Hz, 8H), 0.92 - 0.82 (m, 6H), 0.63 (d, *J* = 1.7 Hz, 3H).

***Step 6***: The compound (5*R*)-1-(2,6-difluorophenyl)-5-[(1*R*,3a*S*,3b*S*,5a*R*,6*R,*7*S*,9a*R*,9b*S*,11a*R*)-6,7-dihydroxy-9a,11a-dimethyl-4-oxohexadecahydro-1*H*-cyclopenta[1,2-*a*]phenanthren-1-yl]hexyl acetate **(320-5)** (25 mg, 0.036 mmol) was dissolved in a mixed solvent of tetrahydrofuran (3 mL) and methanol (3 mL), then 1 mol/L lithium hydroxide solution (0.5 mL) was added thereto, and the resulting mixture was stirred at 25°C for 1 hour. The reaction was monitored by TLC (petroleum ether: ethyl acetate = 1:1). After the reaction was completed, the reaction mixture was diluted with ethyl acetate (20 mL) and washed with saturated brine. The organic phase was dried over anhydrous sodium sulfate, filtered, and the filtrate was concentrated to obtain a crude product, which was then purified by silica gel column chromatography (petroleum ether: ethyl acetate = 60:40) to obtain 24-[(2,6-difluorophenyl)(hydroxy)methyl]-4β-hydroxy-3β-hydroxy-5α-cholan-7-one **(320)** (9.64 mg, 0.018 mmol, purity: 96.86%, yield: 50.47%) as a white solid. ¹H NMR (400 MHz, CDCl₃) δ 7.29 (dd, *J* = 7.2, 4.8 Hz, 1H), 7.23 (m, 1H), 6.95 (t, *J =* 7.4 Hz, 1H), 6.88 (d, *J =* 8.2 Hz, 1H), 4.85 (m, 1H), 3.85 (s, 3H), 3.71 (s, 1H), 3.58 (m, 1H), 2.87 (t, *J* = 13.5 Hz, 1H), 2.38 (t, *J =* 11.3 Hz, 1H), 2.20 (m, 1H), 2.11 (dd, *J =* 12.7, 2.8 Hz, 1H), 1.97 (d, *J* = 12.9 Hz, 1H), 1.76 (m, 8H), 1.47 (m, 3H), 1.39 (m, 4H), 1.28 (s, 3H), 1.03 (m, 6H), 0.90 (dd, *J =* 6.3, 4.7 Hz, 3H), 0.64 (d, *J =* 2.2 Hz, 3H). ¹³C NMR (101 MHz, CDCl₃) δ 212.76, 156.64, 132.59, 128.24, 127.04, 126.91, 120.75, 110.67, 73.83, 71.97, 55.79, 55.28, 55.02, 50.07, 49.04, 43.81, 42.56, 38.64, 37.76, 37.61, 36.07, 35.91, 35.85, 35.68, 35.65, 35.55, 28.44, 25.63, 25.03, 22.76, 22.58, 21.13, 18.71, 14.19, 12.08.

LCMS: [M+H-H₂O]⁺ = 495.5.

### Example 321

### Preparation of methyl 25-hydroxy-5α-cholest-3-carboxylate (compound 321)

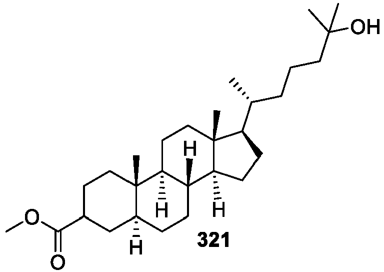

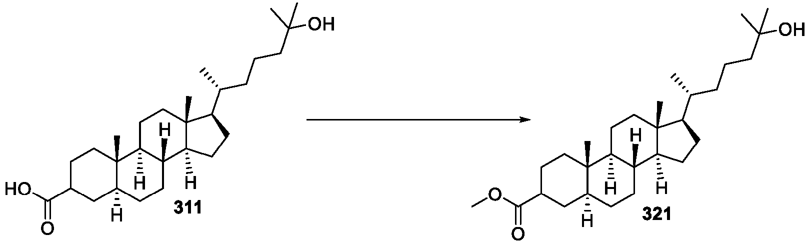

25-Hydroxy-5α-cholest-3-carboxylic acid **(311)** (20 mg, 0.046 mmol, 1.0 *eq.*) was weighed and dissolved in anhydrous methanol (10 mL), then concentrated sulfuric acid (0.5 drops) was added thereto with stirring at room temperature, and the reaction mixture was heated to 60°C. The reaction was monitored by TLC (petroleum ether: ethyl acetate = 3:1). After the reaction was completed, the reaction mixture was concentrated, and the resulting crude product was extracted with sodium bicarbonate aqueous solution and ethyl acetate. The organic phase was separated, dried, concentrated, and purified by column chromatography (petroleum ether: ethyl acetate = 0 to 30%) to obtain methyl 25-hydroxy-5α-cholest-3-carboxylate **(321)** (10 mg, 0.022 mmol, 48.7%) as a white solid with a purity of 85.17%. ¹H NMR (400 MHz, Chloroform-d) δ 3.65 (d, *J =* 11.7 Hz, 3H), 2.35 - 2.16 (m, 1H), 2.02 - 1.89 (m, 2H), 1.85 - 1.68 (m, 3H), 1.62 (ddd, *J =* 13.5, 10.7, 3.6 Hz, 3H), 1.54 - 1.46 (m, 3H), 1.44 - 1.41 (m, 2H), 1.38 - 1.33 (m, 3H), 1.30 (d, *J =* 8.2 Hz, 3H), 1.19 (s, 6H), 1.16 - 1.08 (m, 3H), 1.08 - 1.01 (m, 3H), 1.00 - 0.91 (m, 3H), 0.89 (dd, *J =* 6.6, 2.3 Hz, 3H), 0.86 - 0.79 (m, 2H), 0.77 (d, *J =* 5.2 Hz, 3H), 0.62 (d, *J =* 3.1 Hz, 3H). ¹³C NMR (100 MHz, CDCl₃) δ 176.64, 176.10, 71.11, 56.52, 56.48, 56.34, 56.25, 56.16, 56.13, 55.72, 54.32, 54.25, 54.22, 51.51, 51.48, 45.98, 44.40, 44.30, 43.67, 42.63, 42.60, 42.57, 42.55, 39.98, 39.96, 39.88, 39.38, 37.71, 36.40, 35.91, 35.80, 35.76, 35.74, 35.70, 35.43, 35.39, 35.14, 35.04, 31.98, 31.92, 31.90, 31.81, 31.42, 31.22, 30.16, 29.76, 29.69, 29.66, 29.59, 29.51, 29.47, 29.36, 29.34, 29.32, 29.26, 29.23, 29.18, 29.10, 28.65, 28.24, 28.06, 27.20, 27.15, 25.50, 24.60, 24.15, 24.13, 24.06, 22.79, 22.68, 20.94, 20.92, 20.83, 20.78, 20.76, 20.70, 18.67, 18.62, 18.61, 14.13, 12.29, 12.21, 12.05, 12.01, 11.70. LCMS: [M-OH]⁺ = 429.4.

### Example 322

### Preparation of 24-[(2-chlorophenyl)(hydroxy)methyl]-5α-cholan-3β,4β-diol (compound 322)

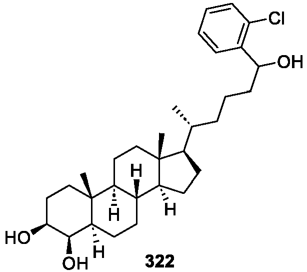

Referring to example **275,** 2,4,6-trifluorobromobenzene was replaced with *o-*chlorobromobenzene to obtain compound 24-[(2-chlorophenyl)(hydroxy)methyl]-5α-cholan-3β,4β-diol **(322)** with a purity of 93.74%. ¹H NMR (399 MHz, Chloroform-d) δ 7.54 (dt, *J* = 7.7, 2.2 Hz, 1H), 7.34 - 7.26 (m, 2H), 7.19 (td, *J =* 7.7, 1.7 Hz, 1H), 5.11 (t, *J* = 6.2 Hz, 1H), 3.73 (s, 1H), 3.59 - 3.48 (m, 1H), 1.94 (d, *J =* 13.1 Hz, 2H), 1.76 (d, *J =* 13.5 Hz, 3H), 1.74-1.67 (m, 4H), 1.66 - 1.60 (m, 3H), 1.35 (d, *J =* 14.8 Hz, 6H), 1.25 (s, 3H), 1.06 (dd, *J =* 10.7, 5.4 Hz, 4H), 1.00 (s, 3H), 0.94 (d, *J =* 12.0 Hz, 2H), 0.90 (q, *J =* 4.2, 3.6 Hz, 3H), 0.63 (s, 3H), 0.59 (d, *J =* 7.8 Hz, 1H). ¹³C NMR (100 MHz, Chloroform-d) δ 142.37, 142.30, 131.84, 131.77, 129.35, 129.33, 128.32, 128.30, 127.06, 127.00, 126.97, 109.98, 74.82, 72.27, 70.91, 70.66, 56.52, 56.19, 56.14, 55.18, 48.77, 42.58, 39.87, 38.15, 37.91, 36.82, 35.73, 35.71, 35.60, 35.50, 35.45, 35.36, 32.36, 30.30, 29.70, 28.23, 25.96, 25.83, 24.19, 22.45, 22.28, 20.56, 18.55, 18.53, 14.66, 12.06. LCMS: [M+Na]⁺ = 525.45.

### Example 323

### Preparation of 24-[hydroxy(2-methylphenyl)methyl]-5α-cholan-3β,4β-diol (compound 323)

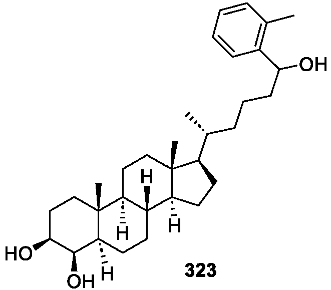

Referring to example **275,** 2,4,6-trifluorobromobenzene was replaced with *o-*methylbromobenzene to obtain compound 24-[hydroxy(2-methylphenyl)methyl]-5α-cholan-3β,4β-diol **(323)** with a purity of 96.3%. ¹H NMR (399 MHz, Chloroform-d) δ 7.46 (d, *J* = 7.8 Hz, 1H), 7.21 (d, *J =* 7.7 Hz, 1H), 7.18 - 7.09 (m, 2H), 4.95 - 4.89 (m, 1H), 3.73 (s, 1H), 3.55 (dd, *J =* 11.6, 4.9 Hz, 1H), 2.33 (d, *J =* 1.4 Hz, 3H), 1.94 (d, *J =* 12.5 Hz, 1H), 1.78 (s, 14H), 1.36 (d, J= 11.8 Hz, 5H), 1.26 (d, *J =* 12.3 Hz, 2H), 1.12 - 1.03 (m, 3H), 1.00 (s, 3H), 0.94 (d, *J =* 11.3 Hz, 2H), 0.89 (dd, *J =* 6.5, 4.2 Hz, 3H), 0.63 (d, *J =* 1.6 Hz, 3H), 0.57 (dt, *J =* 11.3, 5.6 Hz, 1H). ¹³C NMR (101 MHz, Chloroform-d) δ 140.40, 140.16, 130.32, 127.08, 126.25, 125.01, 72.26, 56.51, 55.18, 48.77, 42.57, 39.86, 38.65, 36.82, 35.68, 35.44, 35.35, 30.94, 28.23, 25.95, 25.83, 24.17, 20.55, 19.09, 18.54, 14.65, 12.05, 1.00. LCMS: [M+CH₃CN+Na]⁺ = 546.45.

### Examples 324 and 325

### Preparation of 24-[S-hydroxy(2-methoxyphenyl)methyl]-5α-cholan-3β,4β-diol or 24-[R-hydroxy(2-methoxyphenyl)methyl]-5α-cholan-3β,4β-diol (compound 324 or 325)

Both compound 324 and compound 325 are single configurational isomers, each being one of the following isomers:

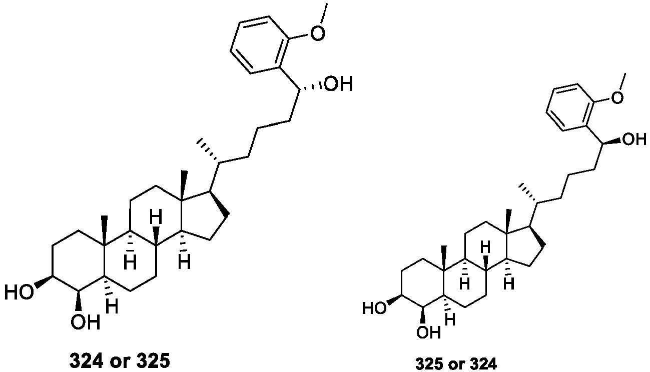

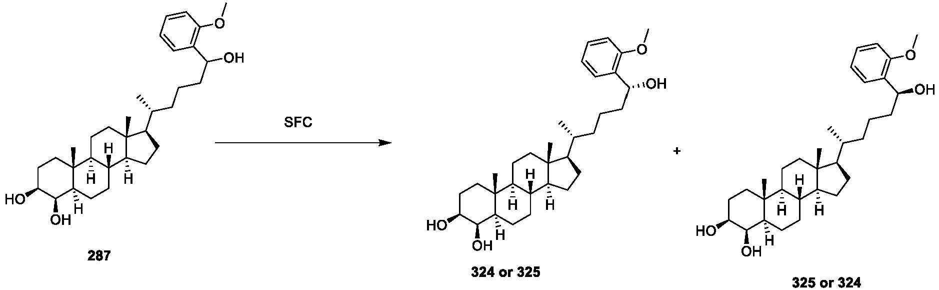

24-[Hydroxy(2-methoxyphenyl)methyl]-5α-cholan-3β,4β-diol **(287)** (50 mg) was subjected to preparative chiral column chromatography (separation conditions: apparatus: SFC 80; column: Daicel CHIRALCELID, 250 mm × 30 mm I.D., 10 µm; mobile phase: CO₂/MeOH [0.2% NH₃ (7 M solution in MeOH)] = 50/50; flow rate: 70 g/min; wave length: UV 214 nm; temperature: 35°C) to obtain two relative configurations: compound **324** (retention time: tR = 2.146 min, 11.26 mg, purity: 100%) and compound **325** (retention time: tR = 2.538 min, 9.37 mg, purity: 98.62%).

**324** (tR = 2.146 min): ¹H NMR (400 MHz, CDCl₃) δ 7.30 (d, *J =* 7.4 Hz, 1H), 7.25-7.21 (m, 1H), 6.95 (t, *J =* 7.5 Hz, 1H), 6.88 (d, *J =* 8.1 Hz, 1H), 4.86 (dd, *J =* 8.1, 5.1 Hz, 1H), 3.74 (s, 1H), 3.59 - 3.52 (m, 1H), 1.98 - 1.87 (m, 5H), 1.70 - 1.62 (m, 3H), 1.60 - 1.51 (m, 1H), 1.44 - 1.32 (m, 9H), 1.29 - 1.18 (m, 4H), 1.11 - 1.03 (m, 4H), 1.01 (s, 3H), 0.96 (dd, *J =* 10.3, 7.0 Hz, 2H), 0.89 (d, *J* = 6.5 Hz, 3H), 0.64 (s, 3H), 0.63 - 0.55 (m, 1H). ¹³C NMR (101 MHz, CDCl₃) δ 155.56, 131.74, 127.16, 125.88, 119.72, 109.50, 73.83, 71.28, 69.95, 55.55, 55.22, 54.25, 47.81, 41.58, 38.89, 36.60, 35.85, 34.65, 34.47, 34.37, 31.38, 29.17, 27.22, 24.97, 24.84, 23.20, 21.59, 19.56, 17.56, 13.65, 11.05.

LCMS: [M+H-2H₂O]⁺ = 463.65.

**325** (tR = 2.538 min): ¹H NMR (400 MHz, CDCl₃) δ 7.32 - 7.27 (m, 1H), 7.25 - 7.21 (m, 1H), 6.95 (t, *J =* 7.4 Hz, 1H), 6.88 (d, *J =* 8.2 Hz, 1H), 4.84 (t, *J =* 6.7 Hz, 1H), 3.86 (s, 3H), 3.74 (s, 1H), 3.55 (d, *J* = 11.2 Hz, 1H), 1.95 (d, *J =* 12.6 Hz, 1H), 1.82 (s, 2H), 1.71 (d, *J* = 13.5 Hz, 4H), 1.60 (ddd, *J* = 19.6, 9.3, 4.7 Hz, 4H), 1.44 - 1.16 (m, 10H), 1.06 (dd, *J =* 11.0, 6.2 Hz, 4H), 1.01 (s, 3H), 0.98 - 0.91 (m, 2H), 0.88 (d, *J =* 6.5 Hz, 3H), 0.63 (s, 3H), 0.62-0.55 (m, 1H). ¹³C NMR (101 MHz, CDCl₃) δ 155.62, 131.61, 127.20, 126.01, 119.72, 109.54, 73.83, 71.28, 70.36, 55.54, 55.18, 54.24, 47.81, 41.58, 38.88, 36.80, 35.84, 34.84, 34.71, 34.47, 34.37, 31.37, 27.21, 24.84, 23.18, 21.73, 19.56, 17.56, 13.64, 11.05. LCMS: [M+H-2H₂O]⁺ = 463.65.

## Claims

1. A compound or a pharmaceutically acceptable salt thereof, wherein the compound has a structure of formula XXI, XVI, XIX, XXIII, XXIV, or XXVI: 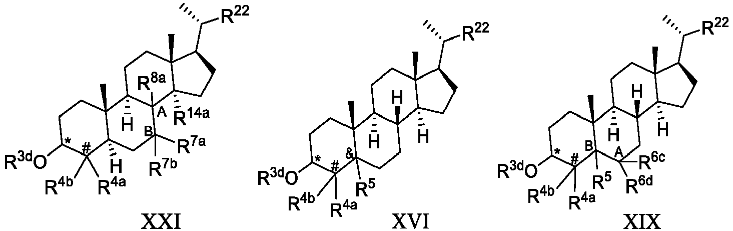 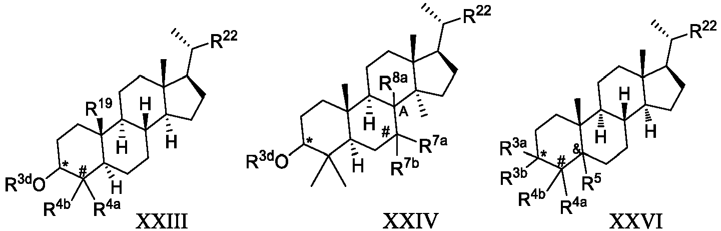 wherein R^{3d} is H or R^{3c};
R^{3c} is -S(O)₂OH, -C(O)-CH₂CH₂COOH, -C(O)-CH₂COOH, -C(O)-CH₂OH, -C(O)-COOH, -CH₂CH₂OH, or -CH₂COOH;
R^{3a} and R^{3b} are as defined in any one of the following schemes:
(i) R^{3a} is H, and R^{3b} is -NH₂, -NHS(O)₂CH₃, -NHS(O)₂CH₂CH₃, -NHS(O)₂CH₂CH₂CH₃, -NHS(O)₂CF₃, -NH-C(O)-CH₂OH, -CH₂OH, -COOH, -C(O)-OCH₃, -CH(CH₃)OH, -CH₂-C(O)-CH₂OH, or -CH(OH)-CH₂CH₂OH;
(ii) R^{3a} is CH₃, and R^{3b} is -OH; and
(iii) R^{3a} and R^{3b} together with the carbon atom to which they are attached form 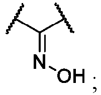
R^{4a} is H, F, CH₃, CF₃, or OH;
R^{4b} is H, F, NH₂, OH, or CF₃;
R^{4a} and R^{4b} together with the carbon atom to which they are attached form 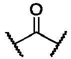
or 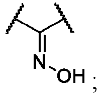
R⁵ is H or R^{5c}; R^{5c} is OH or F;
R^{6c} is H, F, CH₃, or CF₃;
R^{6d} is CN, NH₂, F, COOH, C₁₋₄ alkoxy, or OH;
or, R^{6c} and R^{6d} together with the carbon atom to which they are attached form 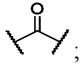
R^{7a} is H, F, NH₂, CH₃, or CF₃;
R^{7b} is F or OH;
or, R^{7a} and R^{7b} together with the carbon atom to which they are attached form 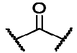
or 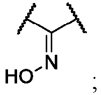
R^{8a} is H or F;
R^{14a} is H;
R¹⁹ is CH₂OH, CH₂CN, CH₂COOH, CH₂F, or CHF₂;
R²² is 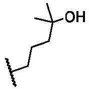
or R21
R²¹ is -L¹-C(O)R^{A}, -L¹-S(O)₂R^{A}, -L¹-R^{B}, 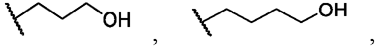 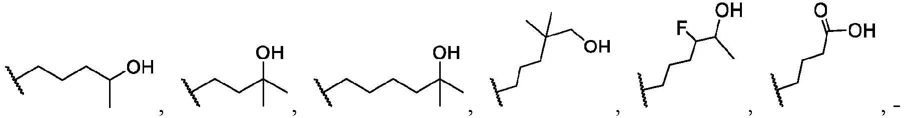 L²-R^{C}, or -L³-R^{D};
L¹ and L² are each independently a single bond, -CH₂-, -(CH₂)₂-, -(CH₂)₃-, -(CH₂)₄-,-(CH₂)₅-, or -(CH₂)₆-, wherein one -CH₂- moiety of the -CH₂-, -(CH₂)₂-, -(CH₂)₃-, -(CH₂)₄-,-(CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 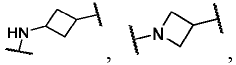 -CH=CH-, 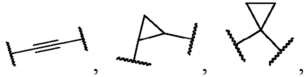 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)₂R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 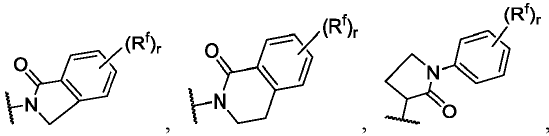 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 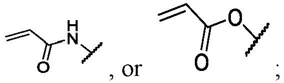
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, C₁₋₄ alkoxy, or fluoro-C₁₋₄ alkoxy;
the number of heteroatoms in the heterocycloalkyl and heteroaryl is independently 1, 2, 3, or 4, and each heteroatom is independently N, O, or S;
the carbon atom marked with * is in *R* configuration, *S* configuration, or a mixture of both;
the carbon atom marked with # is in *R* configuration, *S* configuration, or a mixture of both when it is a chiral carbon atom; the carbon atom marked with & is in *R* configuration, *S* configuration, or a mixture of both when it is a chiral carbon atom; the carbon atom marked with A is in *R* configuration, *S* configuration, or a mixture of both when it is a chiral carbon atom; the carbon atom marked with B is in *R* configuration, *S* configuration, or a mixture of both when it is a chiral carbon atom.

2. The compound or the pharmaceutically acceptable salt thereof according to claim 1, which satisfies one or more of the following conditions:
(1) in the compound of formula XXI, R^{3d} is H or R^{3c}; R^{3c} is -S(O)₂OH, -C(O)-CH₂COOH, -C(O)-CH₂OH, -C(O)-COOH, -CH₂CH₂OH, or -CH₂COOH;
R^{4a} is H, F, CH₃, CF₃, or OH;
R^{4b} is H, F, OH, CH₂OH, CN, COOH, CF₃, or cyclopropyl;
or, R^{4a} and R^{4b} together with the carbon atom to which they are attached form 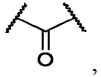 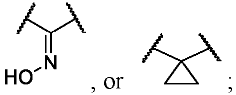
R^{7a} is H, F, CH₃, or CF₃; R^{7b} is F or OH; or, R^{7a} and R^{7b} together with the carbon atom to which they are attached form 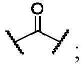
R^{8a} is H or F;
R^{14a} is H;
R²² is 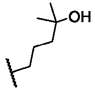
or R²¹;
R²¹ is -L¹-C(O)R^{A}, -L¹-S(O)₂R^{A}, -L¹-R^{B}, 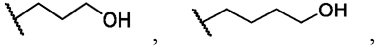 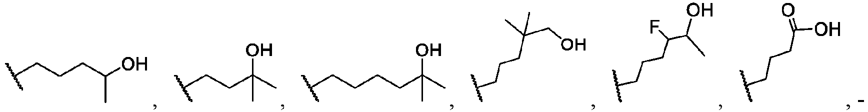 L²-R^{C}, or -L³-R^{D};
L¹ and L² are each independently a single bond, -CH₂-, -(CH₂)₂-, -(CH₂)₃-, -(CH₂)₄-,-(CH₂)₅-, or -(CH₂)₆-, wherein one -CH₂- moiety of the -CH₂-, -(CH₂)₂-, -(CH₂)₃-, -(CH₂)₄-,-(CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 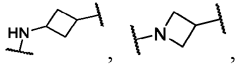 -CH=CH-, 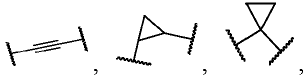 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)₂R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 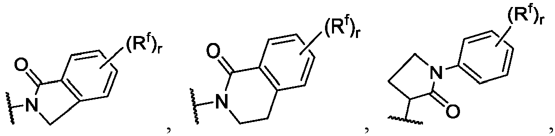 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 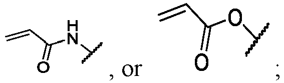
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, or C₁₋₄ alkoxy;
the number of heteroatoms in the heterocycloalkyl and heteroaryl is independently 1, 2, 3, or 4, and each heteroatom is independently N, O, or S;
in formula XXI, the carbon atom marked with * is in *R* configuration, *S* configuration, or a mixture of both; the carbon atom marked with # is in *R* configuration, *S* configuration, or a mixture of both when it is a chiral carbon atom; the carbon atom marked with A is in *R* configuration, *S* configuration, or a mixture of both when it is a chiral carbon atom; the carbon atom marked with B is in *R* configuration, *S* configuration, or a mixture of both when it is a chiral carbon atom;
(2) in the compound of formula XVI, R^{3d} is H or R^{3c};
R^{3c} is -S(O)₂OH, -C(O)-CH₂COOH, -C(O)-CH₂OH, -C(O)-COOH, -CH₂CH₂OH, or-CH₂COOH;
R^{4a} is H, F, CH₃, CF₃, or OH;
R^{4b} is H, F, OH, or CF₃;
or, R^{4a} and R^{4b} together with the carbon atom to which they are attached form 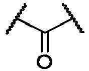 or 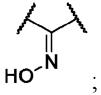
R⁵ is H or R^{5c}; R^{5c} is OH or F;
R²² is 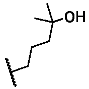 or R²¹;
R²¹ is -L¹-C(O)R^{A}, -L¹-S(O)₂R^{A}, -L¹-R^{B}, 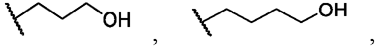 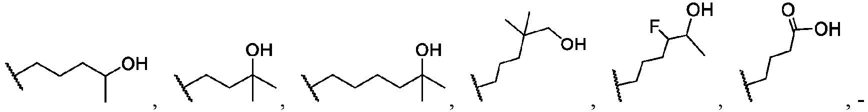 L²-R^{C}, or -L³-R^{D};
L¹ and L² are each independently a single bond, -CH₂-, -(CH₂)₂-, -(CH₂)₃-, -(CH₂)₄-, - (CH₂)₅-, or -(CH₂)₆-, wherein one -CH₂- moiety of the -CH₂-, -(CH₂)₂-, -(CH₂)₃-, -(CH₂)₄-,-(CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 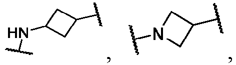 -CH=CH-, 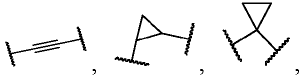 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)₂R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 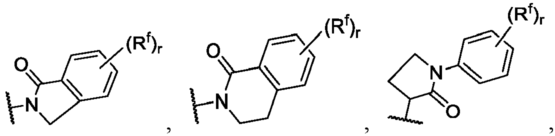 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 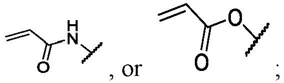
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, or C₁₋₄ alkoxy;
the number of heteroatoms in the heterocycloalkyl and heteroaryl is independently 1, 2, 3, or 4, and each heteroatom is independently N, O, or S;
in formula XVI, the carbon atom marked with * is in *R* configuration, *S* configuration, or a mixture of both; the carbon atom marked with # is in *R* configuration, *S* configuration, or a mixture of both when it is a chiral carbon atom; the carbon atom marked with & is in *R* configuration, S configuration, or a mixture of both when it is a chiral carbon atom;
(3) in the compound of formula XIX, R^{3d} is H or R^{3c}; R^{3c} is -S(O)₂OH, -C(O)-CH₂COOH, -C(O)-CH₂OH, -C(O)-COOH, -CH₂CH₂OH, or -CH₂COOH;
R^{4a} is H, F, CH₃, CF₃, or OH;
R^{4b} is H, F, OH, or CF₃;
or, R^{4a} and R^{4b} together with the carbon atom to which they are attached form 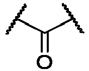 or 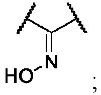
R⁵ is H or R^{5c}; R^{5c} is OH or F;
R^{6c} is H, F, CH₃, or CF₃;
R^{6d} is CN, F, COOH, C₁₋₄ alkoxy (e.g., methoxy), or OH;
or, R^{6c} and R^{6d} together with the carbon atom to which they are attached form 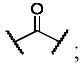
R²² is 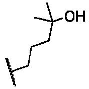 or R²¹;
R²¹ is -L¹-C(O)R^{A}, -L¹-S(O)₂R^{A}, -L¹-R^{B}, 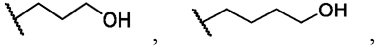 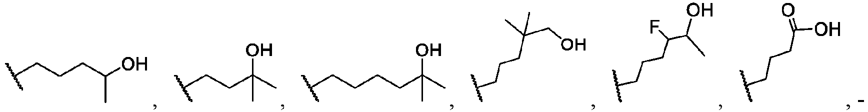 L²-R^{C}, or -L³-R^{D};
L¹ and L² are each independently a single bond, -CH₂-, -(CH₂)₂-, -(CH₂)₃-, -(CH₂)₄-,-(CH₂)₅-, or -(CH₂)₆-, wherein one -CH₂- moiety of the -CH₂-, -(CH₂)₂-, -(CH₂)₃-, -(CH₂)₄-,-(CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 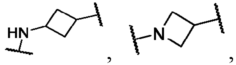 -CH=CH-, 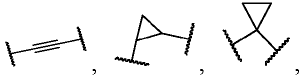 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)₂R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 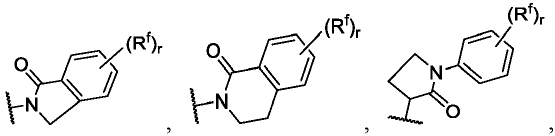 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 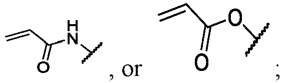
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, or C₁₋₄ alkoxy;
the number of heteroatoms in the heterocycloalkyl and heteroaryl is independently 1, 2, 3, or 4, and each heteroatom is independently N, O, or S;
in formula XIX, the carbon atom marked with * is in *R* configuration, *S* configuration, or a mixture of both; the carbon atom marked with # is in *R* configuration, *S* configuration, or a mixture of both when it is a chiral carbon atom; the carbon atom marked with A is in *R* configuration, S configuration, or a mixture of both when it is a chiral carbon atom; the carbon atom marked with B is in *R* configuration, *S* configuration, or a mixture of both when it is a chiral carbon atom;
(4) in the compound of formula XXIII, R^{3d} is H or R^{3c}; R^{3c} is -S(O)₂OH, -C(O)-CH₂COOH, -C(O)-CH₂OH, -C(O)-COOH, -CH₂CH₂OH, or -CH₂COOH;
R^{4a} is H, F, CH₃, CF₃, or OH;
R^{4b} is H, F, OH, or CF₃;
or, R^{4a} and R^{4b} together with the carbon atom to which they are attached form 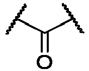 or 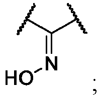
R¹⁹ is CH₂OH, CH₂CN, CH₂COOH, CH₂F, or CHF₂;
R²² is 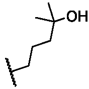 or R²¹;
R²¹ is -L¹-C(O)R^{A}, -L¹-S(O)₂R^{A}, -L¹-R^{B}, 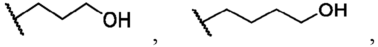 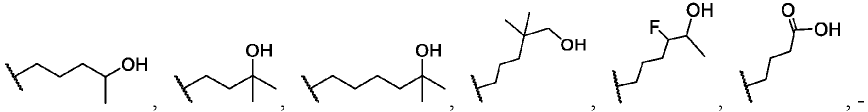 L²-R^{C}, or -L³-R^{D};
L¹ and L² are each independently a single bond, -CH₂-, -(CH₂)₂-, -(CH₂)₃-, -(CH₂)₄-,-(CH₂)₅-, or -(CH₂)₆-, wherein one -CH₂- moiety of the -CH₂-, -(CH₂)₂-, -(CH₂)₃-, -(CH₂)₄-,-(CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 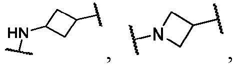 -CH=CH-, 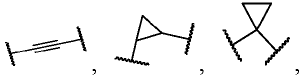 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)2R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 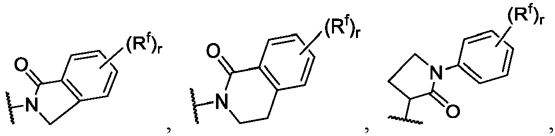 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 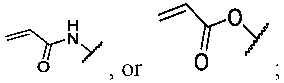
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, or C₁₋₄ alkoxy;
the number of heteroatoms in the heterocycloalkyl and heteroaryl is independently 1, 2, 3, or 4, and each heteroatom is independently N, O, or S;
in formula XXIII, the carbon atom marked with * is in R configuration, S configuration, or a mixture of both; the carbon atom marked with # is in R configuration, S configuration, or a mixture of both when it is a chiral carbon atom;
(5) in the compound of formula XXIV, R^{3d} is H or R^{3c}; R^{3c} is -S(O)₂OH, -C(O)-CH₂COOH, -C(O)-CH₂OH, -C(O)-COOH, -CH₂CH₂OH, or -CH₂COOH;
R^{7a} is H, F, CH₃, or CF₃; R^{7b} is F or OH; or, R^{7a} and R^{7b} together with the carbon atom to which they are attached form 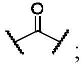
R^{8a} is H or F;
R²² is 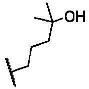 or R²¹;
R²¹ is -L¹-C(O)R^{A}, -L¹-S(O)₂R^{A}, -L¹-R^{B}, 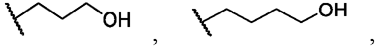 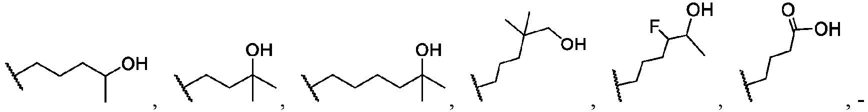 L²-R^{C}, or -L³-R^{D};
L¹ and L² are each independently a single bond, -CH₂-, -(CH₂)₂-, -(CH₂)₃-, -(CH₂)₄-, - (CH₂)₅-, or -(CH₂)₆-, wherein one -CH₂- moiety of the -CH₂-, -(CH₂)₂-, -(CH₂)₃-, -(CH₂)₄-, - (CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 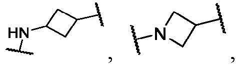 -CH=CH-, 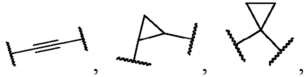 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)₂R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 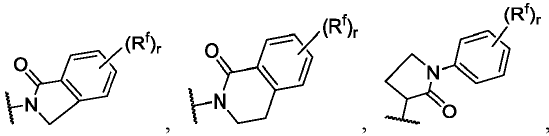 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 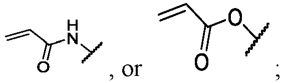
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, or C₁₋₄ alkoxy;
the number of heteroatoms in the heterocycloalkyl and heteroaryl is independently 1, 2, 3, or 4, and each heteroatom is independently N, O, or S;
in formula XXIV, the carbon atom marked with * is in R configuration, S configuration, or a mixture of both; the carbon atom marked with # is in R configuration, S configuration, or a mixture of both when it is a chiral carbon atom; the carbon atom marked with A is in R configuration, S configuration, or a mixture of both when it is a chiral carbon atom; and
(6) in the compound of formula XXVI, R^{3a} and R^{3b} are as defined in any one of the following schemes:
(i) R^{3a} is H, and R^{3b} is -NH₂, -NHS(O)₂CH₃, -NHS(O)₂CH₂CH₃, -NHS(O)₂CH₂CH₂CH₃, -NHS(O)₂CF₃, -NH-C(O)-CH₂OH, -CH₂OH, -COOH, -C(O)-OCH₃, -CH(CH₃)OH, -CH₂-C(O)-CH₂OH, or -CH(OH)-CH₂CH₂OH;
(ii) R^{3a} is CH₃, and R^{3b} is -OH; and
(iii) R^{3a} and R^{3b} together with the carbon atom to which they are attached form 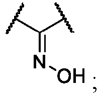
R^{4a} is H, F, CH₃, CF₃, or OH;
R^{4b} is H, F, OH, or CF₃;
or, R^{4a} and R^{4b} together with the carbon atom to which they are attached form 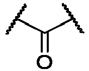 or 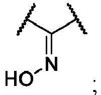
R⁵ is H or R^{5c}; R^{5c} is OH or F;
R²² is 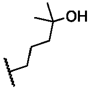 or R²¹;
R²¹ is -L¹-C(O)R^{A}, -L¹-S(O)₂R^{A}, -L¹-R^{B}, 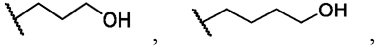 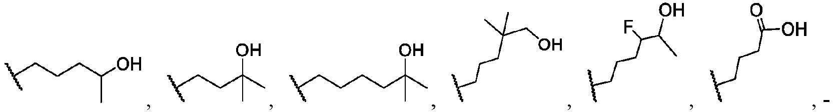 L²-R^{C}, or -L³-R^{D};
L¹ and L² are each independently a single bond, -CH₂-, -(CH₂)₂-, -(CH₂)₃-, -(CH₂)₄-, - (CH₂)₅-, or -(CH₂)₆-, wherein one -CH₂- moiety of the -CH₂-, -(CH₂)₂-, -(CH₂)₃-, -(CH₂)₄-, - (CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 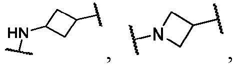 -CH=CH-, 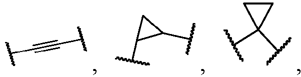 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)₂R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 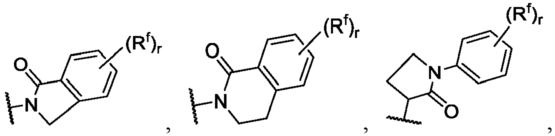 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 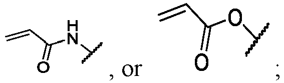
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, or C₁₋₄ alkoxy;
the number of heteroatoms in the heterocycloalkyl and heteroaryl is independently 1, 2, 3, or 4, and each heteroatom is independently N, O, or S;
in formula XXVI, the carbon atom marked with * is in R configuration, S configuration, or a mixture of both; the carbon atom marked with # is in R configuration, S configuration, or a mixture of both when it is a chiral carbon atom; the carbon atom marked with & is in R configuration, S configuration, or a mixture of both when it is a chiral carbon atom.

3. The compound or the pharmaceutically acceptable salt thereof according to claim 1 or 2, which satisfies one or more of the following conditions:
(1) in the compound of formula XXI, R^{3d} is H;
(2) in the compound of formula XXI, R^{4a} is H; R^{4b} is H or OH; or, R^{4a} and R^{4b} together with the carbon atom to which they are attached form 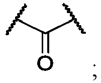 preferably, R^{4a} and R^{4b} are as defined in any one of the following schemes:
a) R^{4a} is H; R^{4b} is H;
b) R^{4a} is H; R^{4b} is OH; and
c) R^{4a} and R^{4b} together with the carbon atom to which they are attached form 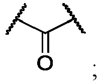
(3) in the compound of formula XXI, R^{8a} is H;
(4) in the compound of formula XXI, R^{7a} and R^{7b} are as defined in any one of the following schemes:
a) R^{7a} is H; R^{7b} is F;
b) R^{7a} is F; R^{7b} is F;
c) R^{7a} is H; R^{7b} is OH;
d) R^{7a} and R^{7b} together with the carbon atom to which they are attached form 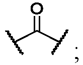
e) R^{7a} and R^{7b} together with the carbon atom to which they are attached form 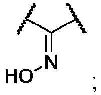 and
f) R^{7a} is H; R^{7b} is NH₂;
(5) in the compound of formula XXI, R²¹ is -L²-R^{C};
(6) in the compound of formula XXI, L² is -(CH₂)₃-;
(7) in the compound of formula XXI, each R^{C} is independently -C(R^{21c})(R^{21d})-OH;
(8) in the compound of formula XXI, in R^{C}, -C(R^{21c})(R^{21d})-OH is 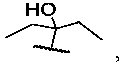 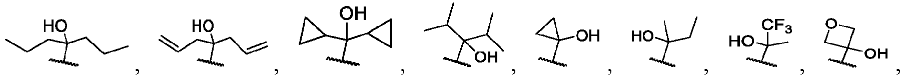 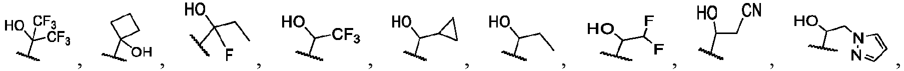 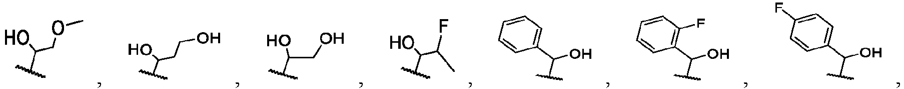 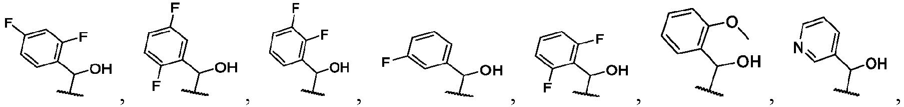 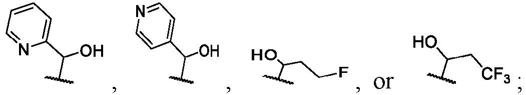 preferably, in R^{C}, -C(R^{21c})(R^{21d})-OH is 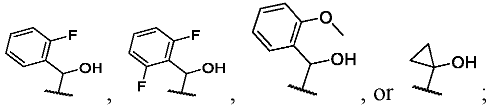
(9) in the compound of formula XVI, R^{3d} is H, -C(O)-CH₂OH, -C(O)-CH₂COOH, or - C(O)-CH₂CH₂COOH;
(10) in the compound of formula XVI, R^{4a} and R^{4b} are as defined in any one of the following schemes:
a) R^{4a} is H, R^{4b} is OH;
b) R^{4a} is CH₃, R^{4b} is F;
c) R^{4a} is F, R^{4b} is F;
d) R^{4a} is H, R^{4b} is CF₃;
e) R^{4a} is CH₃, R^{4b} is OH;
f) R^{4a} is OH, R^{4b} is CF₃;
g) R^{4a} and R^{4b} together with the carbon atom to which they are attached form 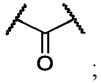
h) R^{4a} is H, R^{4b} is F;
i) R^{4a} is CF₃, R^{4b} is CF₃;
j) R^{4a} and R^{4b} together with the carbon atom to which they are attached form 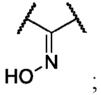
k) R^{4a} is H, R^{4b} is H; and
l) R^{4a} is H, R^{4b} is NH₂;
(11) in the compound of formula XVI, R²¹ is -L¹-C(O)R^{A}, -L²-R^{C}, or 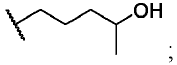
(12) in the compound of formula XVI, L¹ is -(CH₂)₂- or -CH₂-CHR^{5a}-; preferably, L¹ is - (CH₂)₂-;
(13) in the compound of formula XVI, each R^{21a} is independently methyl;
(14) in the compound of formula XVI, each R^{21b} is independently 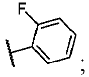
(15) in the compound of formula XVI, L² is -(CH₂)₂- or -(CH₂)₃;
(16) in the compound of formula XVI, each R^{C} is independently -C(R^{21c})(R^{21d})-OH or 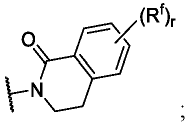
(17) in the compound of formula XVI, in R^{C}, -C(R^{21c})(R^{21d})-OH is 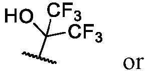 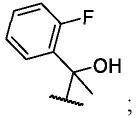
(18) in the compound of formula XVI, r is 0;
(19) in the compound of formula XIX, R^{3d} is H;
(20) in the compound of formula XIX, R^{4a} is H, R^{4b} is OH; or, R^{4a} is H, R^{4b} is H;
(21) in the compound of formula XIX, R⁵ is OH; or, R⁵ is H;
(22) in the compound of formula XIX, R^{6c} and R^{6d} are as defined in any one of the following schemes:
a) R^{6c} is H, R^{6d} is CN;
b) R^{6c} is H, R^{6d} is COOH;
c) R^{6c} is H, R^{6d} is OH;
d) R^{6c} is H, R^{6d} is OCH₃; and
e) R^{6c} and R^{6d} together with the carbon atom to which they are attached form 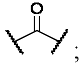
(23) in the compound of formula XIX, R²² is 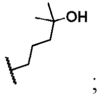
(24) in the compound of formula XXIII, R^{3d} is H;
(25) in the compound of formula XXIII, R^{4a} is H; R^{4b} is H;
(26) in the compound of formula XXIII, R²² is 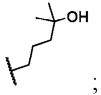
(27) in the compound of formula XXIV, R^{3d} is H;
(28) in the compound of formula XXIV, R^{7a} is H; R^{7b} is OH; or, R^{7a} and R^{7b} together with the carbon atom to which they are attached form 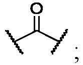
(29) in the compound of formula XXIV, R^{8a} is H;
(30) in the compound of formula XXIV, R²² is 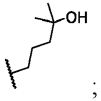
(31) in the compound of formula XXVI, R^{3a} and R^{3b} are as defined in any one of the following schemes:
a) R^{3a} is H, and R^{3b} is -NHS(O)₂CH₃, -CH₂OH, -COOH, -C(O)-OCH₃, or -CH(CH₃)OH; and
b) R^{3a} is CH₃; R^{3b} is -OH;
(32) in the compound of formula XIX, R^{4a} is H, R^{4b} is H;
(33) in the compound of formula XXVI, R⁵ is H; and
(34) in the compound of formula XXVI, R²² is

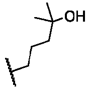

4. The compound or the pharmaceutically acceptable salt thereof according to claim 1 or 2, which satisfies one or more of the following conditions:
(1) in the compound of formula XXI, when R^{C} is -C(R^{21c})(R^{21d})-OH, R^{21c} is H, and R^{21d} is C₂₋₄ alkyl, fluoro-C₁₋₄ alkyl, C₃₋₆ cycloalkyl, phenyl, 5- to 6-membered heteroaryl, or -L⁴-R^{21e}, wherein the phenyl is optionally substituted by 1 or 2 R^{d}, and each R^{d} is independently F, OH, CN, or C₁₋₄ alkoxy;
(2) each R^{21a} is independently H, C₁₋₄ alkyl, -C₁₋₄ alkylene-OH, -C₁₋₄ alkylene-OC₁₋₄ alkyl, fluoro-C₁₋₄ alkyl, or cyclopropyl; for example, each R^{21a} is independently H, methyl, ethyl, *n-*propyl, isopropyl, n-butyl, isobutyl, sec-butyl, 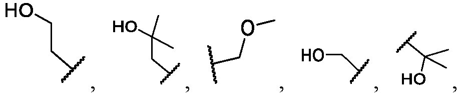 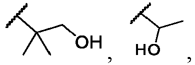 cyclopropyl, or trifluoromethyl; preferably, each R^{21a} is independently C₁₋₄ alkyl;
(3) in the compound of formula XVI as described in any one of the schemes, each R^{21b} is independently H, C₁₋₆ alkyl, C₆₋₁₀ aryl, or 5- to 10-membered heteroaryl, wherein the C₁₋₆ alkyl, C₆₋₁₀ aryl, and 5- to 10-membered heteroaryl are independently unsubstituted or substituted by q R^{b}; for example, each R^{21b} is independently H, -C₁₋₄ alkylene-OH, -C₁₋₄ alkylene-OC₁₋₄ alkyl, fluoro-C₁₋₄ alkyl, tetrahydromorpholinyl, phenyl, pyridyl, thienyl, furyl, pyrrolyl, pyrazolyl, imidazolyl, oxazolyl, isoxazolyl, thiazolyl, isothiazolyl, thiadiazolyl, pyrimidinyl, or benzopyrazolyl, wherein the phenyl, pyridyl, thienyl, furyl, pyrrolyl, pyrazolyl, imidazolyl, oxazolyl, isoxazolyl, thiazolyl, isothiazolyl, thiadiazolyl, pyrimidinyl, and benzopyrazolyl are independently unsubstituted or substituted by q R^{b}; preferably, each R^{21b} is independently phenyl, wherein the phenyl is unsubstituted or substituted by q R^{b};
preferably, each R^{21b} is independently C₆₋₁₀ aryl, wherein the C₆₋₁₀ aryl is unsubstituted or substituted by q R^{b};
(4) in the compound of formula XVI, q is 1, 2, or 3; such as 1;
(5) in the compound of formula XVI, each R^{b} is independently F, Cl, OH, COOH, CN, NO₂, methyl, trifluoromethyl, methoxy, 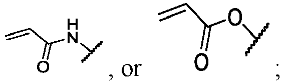 such as F; and (6) in the compound of formula XVI, when R^{C} is -C(R^{21c})(R^{21d})-OH, R^{21c} and R^{21d} are as defined in any one of the following schemes:
a) R^{21c} is H, and R^{21d} is C₂₋₄ alkyl, fluoro-C₁₋₄ alkyl, C₃₋₆ cycloalkyl, phenyl, 5- to 6-membered heteroaryl, or -L⁴-R^{21e}, wherein the phenyl is optionally substituted by 1 or 2 R^{d}, and each R^{d} is independently F, OH, CN, or C₁₋₄ alkoxy;
b) R^{21c} and R^{21d} together with the carbon atom to which they are attached form a C₃₋₆ cycloalkyl group or a 3- to 6-membered heterocycloalkyl group (*e*.*g*., 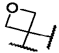 ); preferably, R^{21c} and R^{21d} together with the carbon atom to which they are attached form a cyclopropyl group, a cyclobutyl group, or 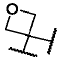 ; and
c) R^{21c} is CH₃ or trifluoromethyl, and R^{21d} is C₂₋₄ alkyl (*e.g.*, ethyl) or trifluoromethyl; for example, -C(R^{21c})(R^{21d})-OH is

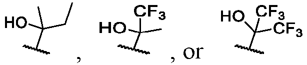

5. The compound or the pharmaceutically acceptable salt thereof according to claim 1 or 2, which satisfies one or more of the following conditions:
(1) in the compound of formula XXI, when R^{C} is -C(R^{21c})(R^{21d})-OH, R^{21c} is H, and R^{21d} is phenyl, wherein the phenyl is optionally substituted by 1 or 2 R^{d}; or R^{21c} and R^{21d} together with the carbon atom to which they are attached form a C₃₋₆ cycloalkyl group (*e.g*., a cyclopropyl group); each R^{d} is independently F or C₁₋₄ alkoxy;
(2) in the compound of formula XVI, each R^{A} is independently 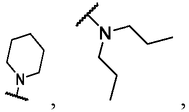 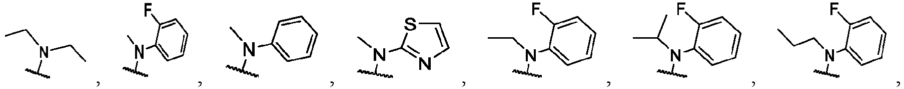 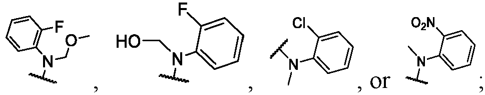 such as 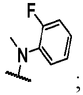 and
(3) in the compound of formula XVI, when R^{C} is -C(R^{21c})(R^{21d})-OH, R^{21c} and R^{21d} are as defined in any one of the following schemes:
a) R^{21c} is H, and R^{21d} is C₂₋₄ alkyl, fluoro-C₁₋₄ alkyl, C₃₋₆ cycloalkyl, or phenyl, wherein the phenyl is optionally substituted by 1 or 2 R^{d}, and each R^{d} is independently F, Cl, OH, fluoro-C₁₋₄ alkyl, C₁₋₄ alkoxy, or fluoro-C₁₋₄ alkoxy;
b) R^{21c} and R^{21d} together with the carbon atom to which they are attached form a C₃₋₆ cycloalkyl group; preferably, R^{21c} and R^{21d} together with the carbon atom to which they are attached form a cyclopropyl group; and
(4) R^{21c} is CH₃ or trifluoromethyl, and R^{21d} is phenyl or trifluoromethyl, wherein the phenyl is unsubstituted or substituted by j R^{d}.

6. The compound or the pharmaceutically acceptable salt thereof according to claim 1 or 2, which satisfies one or more of the following conditions:
(1) in the compound of formula XXI, -L²-R^{C} is 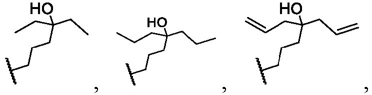 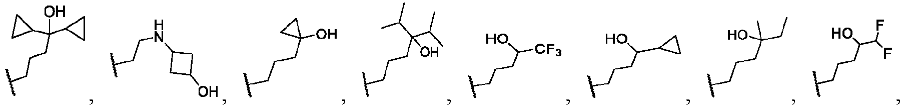 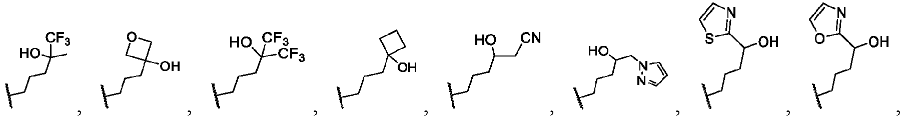 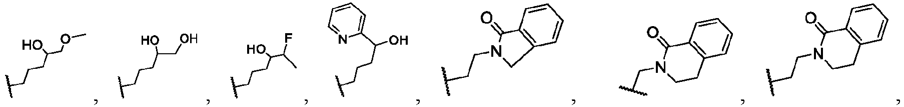 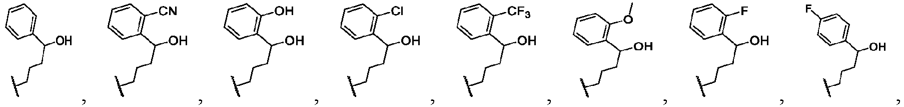 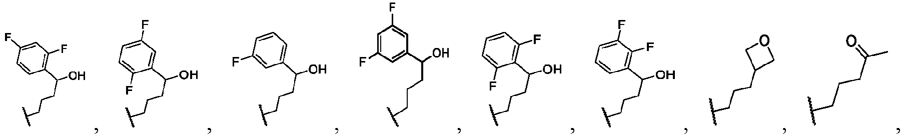 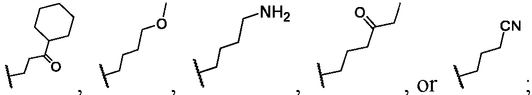 and
(2) in the compound of formula XXI, -L¹-C(O)R^{A} is 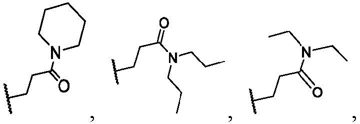 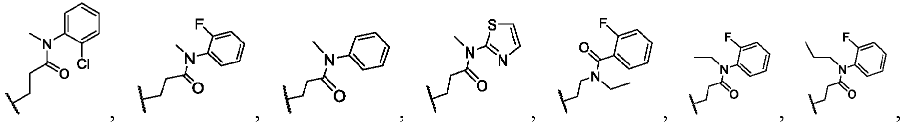 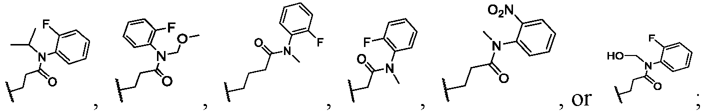 such as

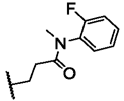

7. The compound or the pharmaceutically acceptable salt thereof according to claim 1, which satisfies one or more of the following conditions:
(1) in the compound of formula XXI, R²¹ is 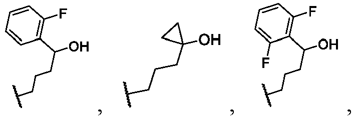 or 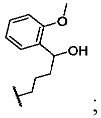 and
(2) in the compound of formula XVI, R²¹ is

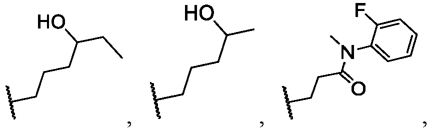

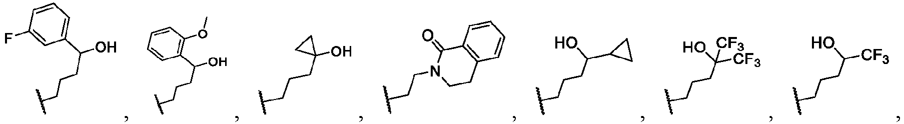

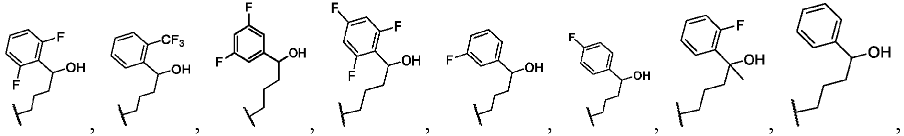

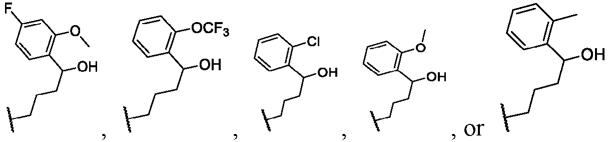

8. The compound or the pharmaceutically acceptable salt thereof according to claim 1, which satisfies one or more of the following conditions:
(1) the compound of formula XXI is a compound of formula XXI-1: 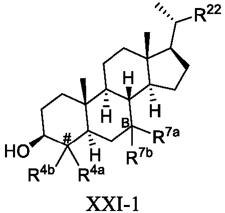
in formula XXI-1, #, B, R^{4a}, R^{4b}, R^{7a}, R^{7b}, and R²² are as defined in any one of claims 1 to 6;
preferably, the compound of formula XXI is a compound of formula XXI-2, XXI-3, or XXI-4: 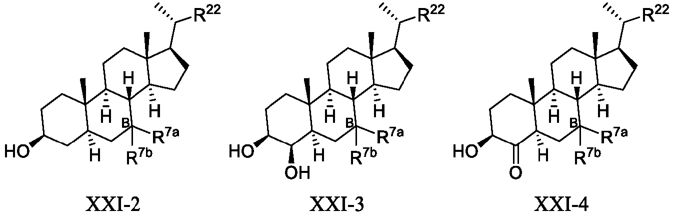
in formula XXI-2, XXI-3, or XXI-4, B, R^{7a}, R^{7b}, and R²² are as defined in any one of claims 1 to 7;
(2) the compound of formula XVI is a compound of formula XVI-1: 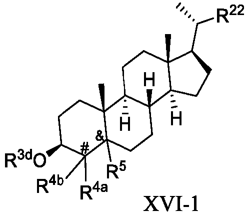 in formula XVI, #, &, R^{3d}, R^{4a}, R^{4b}, R⁵, and R²² are as defined in any one of claims 1 to 7;
(3) the compound of formula XIX is a compound of formula XIX-1: 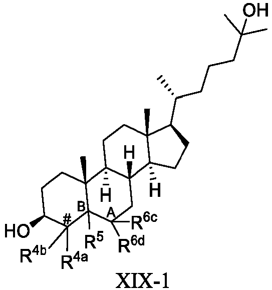 in formula XIX, #, A, B, R^{4a}, R^{4b}, R⁵, R^{6c}, and R^{6d} are as defined in any one of claims 1 to 7; and
(4) the compound of formula XXVI is a compound of formula XXVI-1: 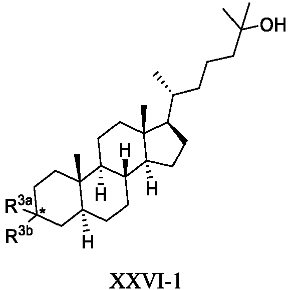 in formula XXVI, *, R^{3a}, and R^{3b} are as defined in any one of claims 1 to 7.

9. The compound or the pharmaceutically acceptable salt thereof according to claim 1 or 2, wherein the compound of formula XVI is a compound of formula VII: 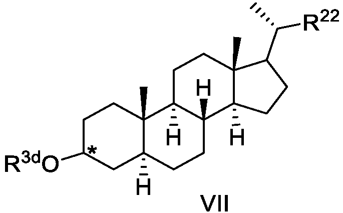 wherein R^{3d} is H or R^{3c}; R^{3c} is as defined in claims 1 to 7;
R²² is 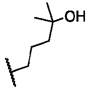 or R²¹; R²¹ is as defined in claims 1 to 7;
in formula VII, the carbon atom marked with * is in R configuration, S configuration, or a mixture of both;
preferably, R^{3d} is H;
preferably, R²² is 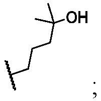
more preferably, the compound of formula VII is the following compound:

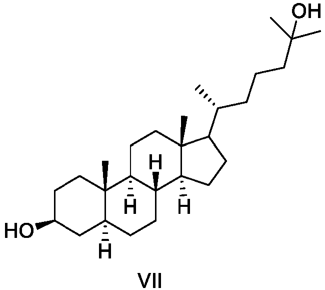

10. The compound or the pharmaceutically acceptable salt thereof according to claim 1 or 2, which satisfies one or more of the following conditions:
(1) the compound of formula XXI is any one of the following compounds: 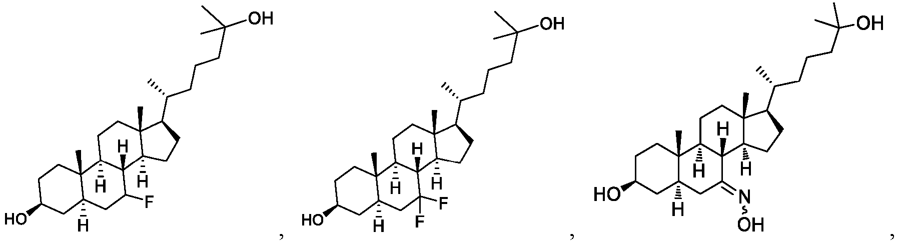 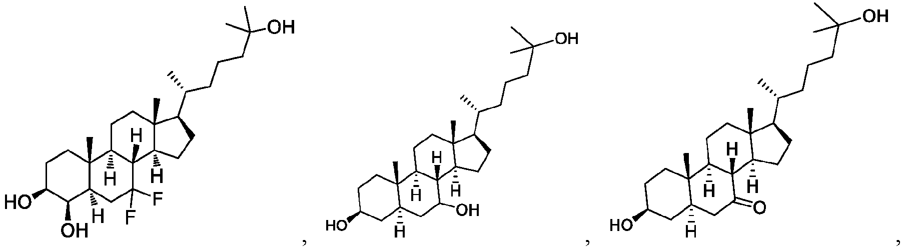 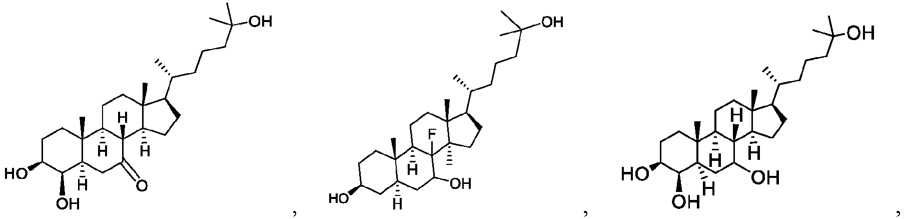 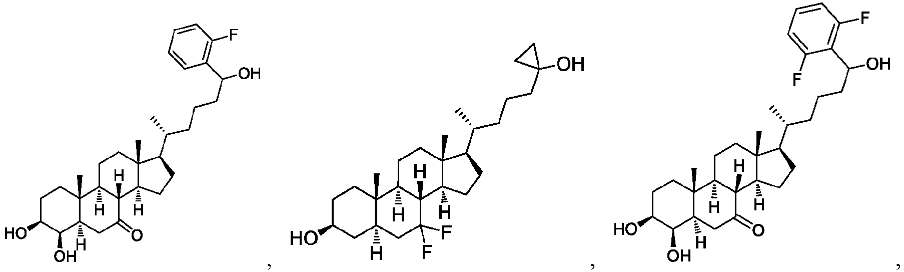 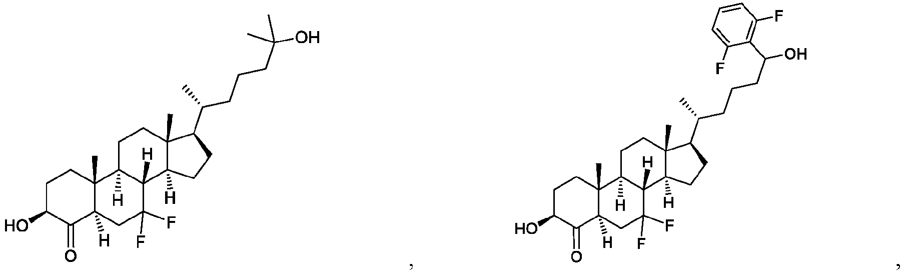 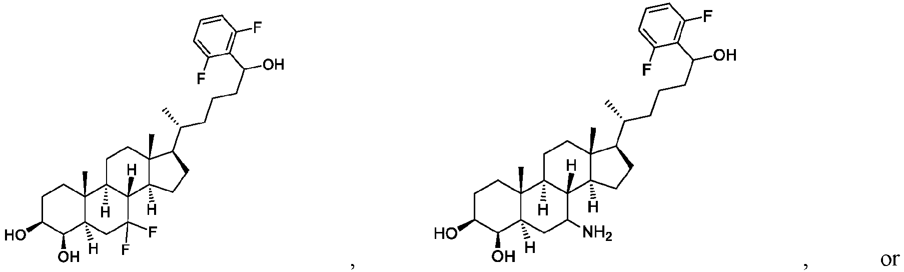 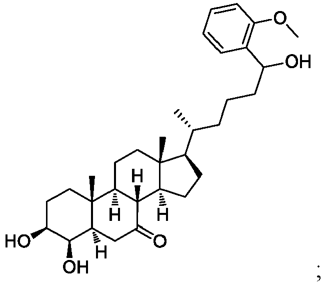
(2) the compound of formula XVI is any one of the following compounds: 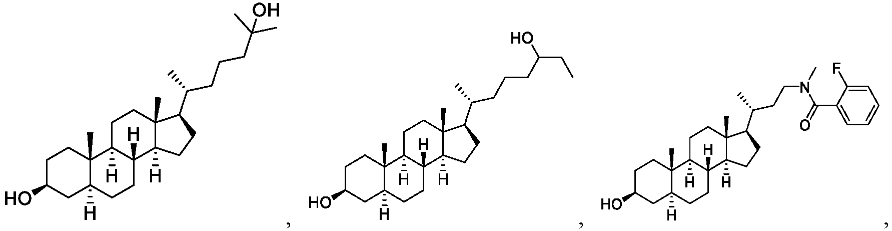 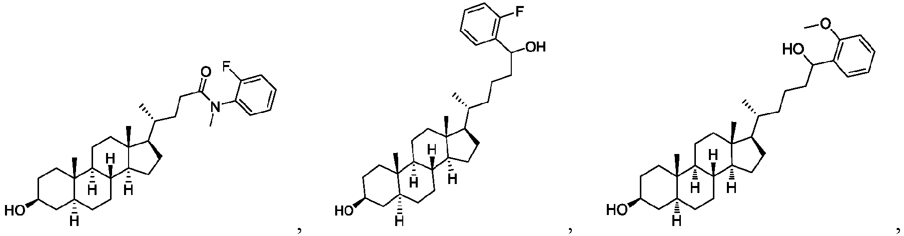 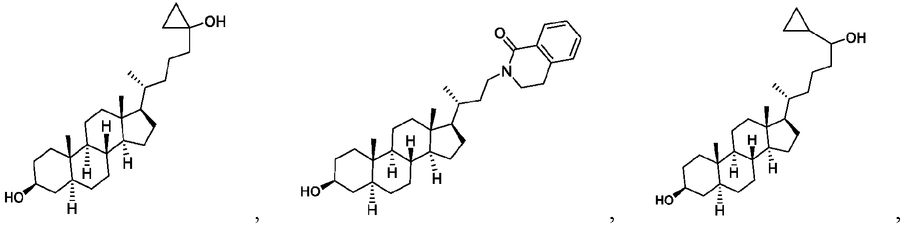 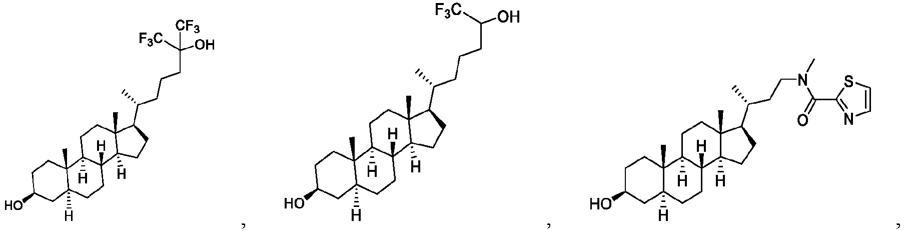 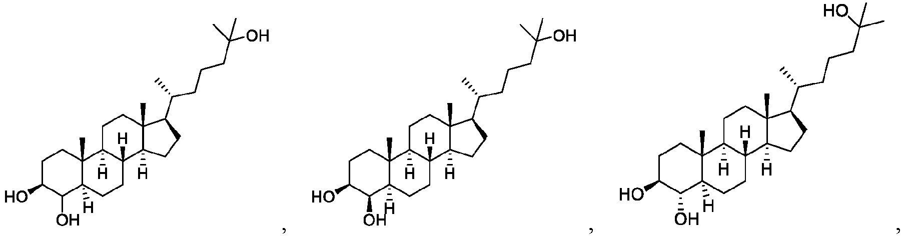 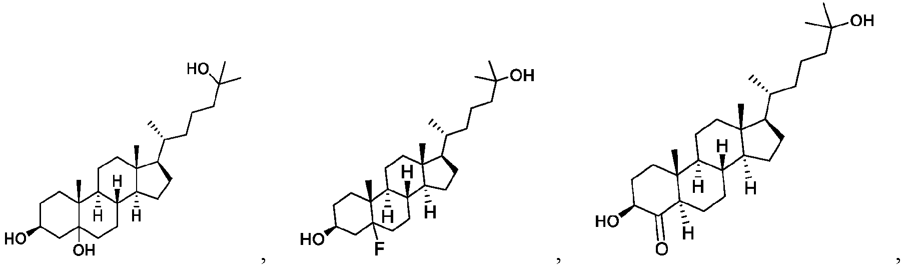 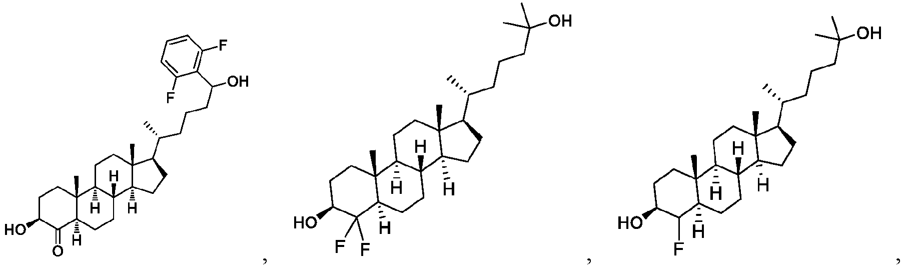 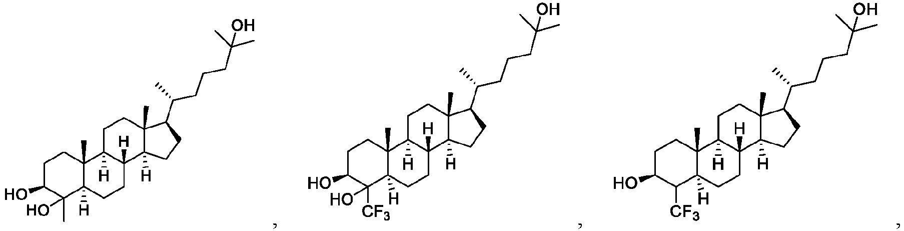 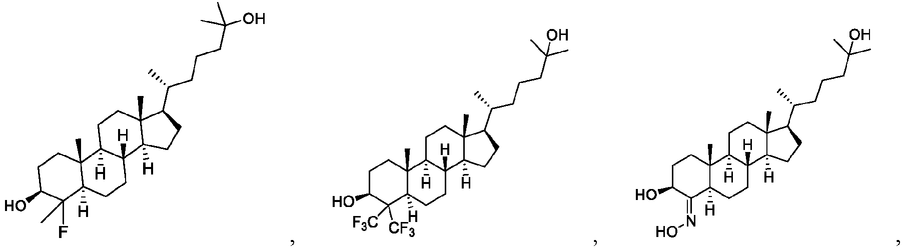 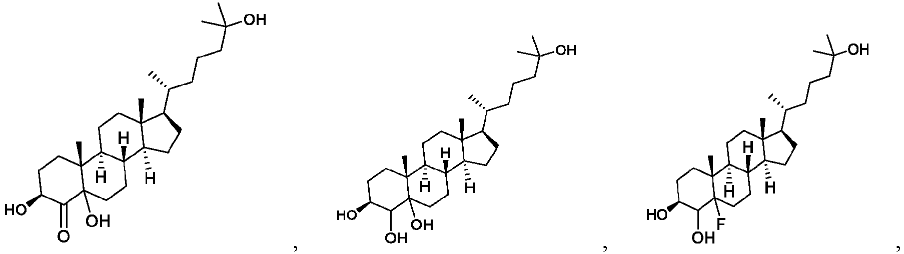 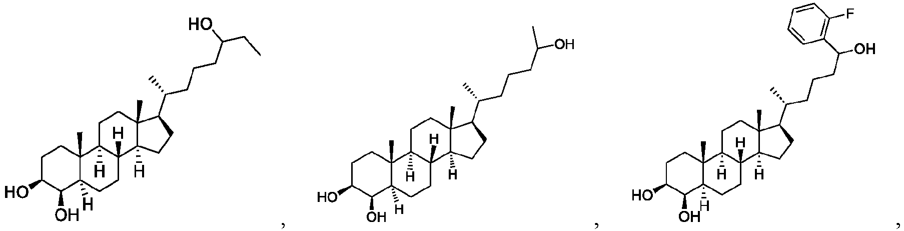 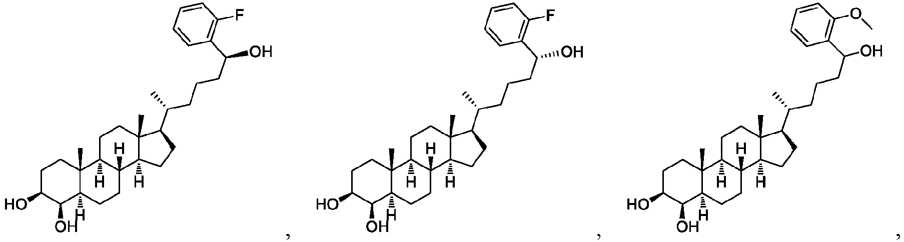 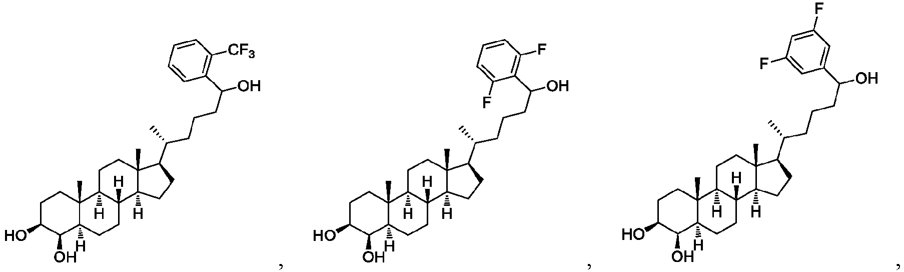 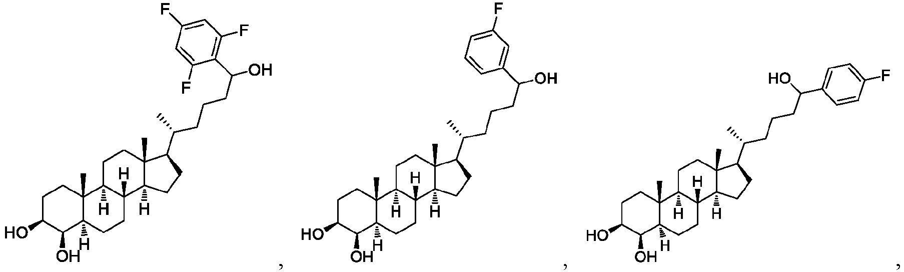 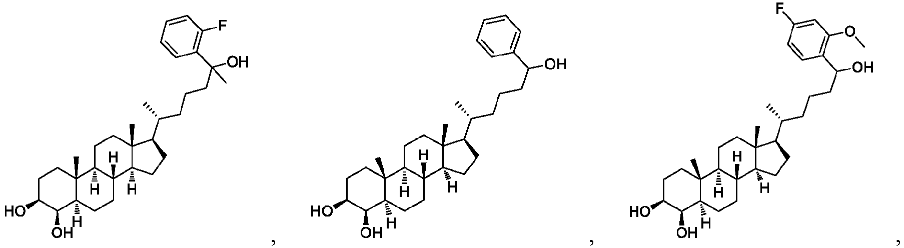 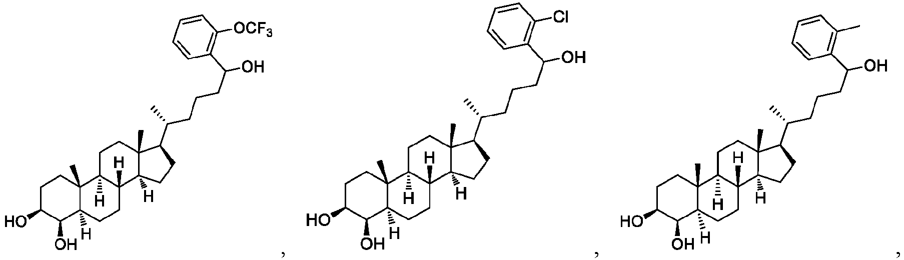 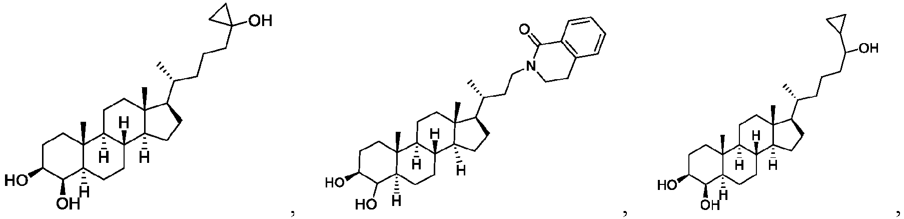 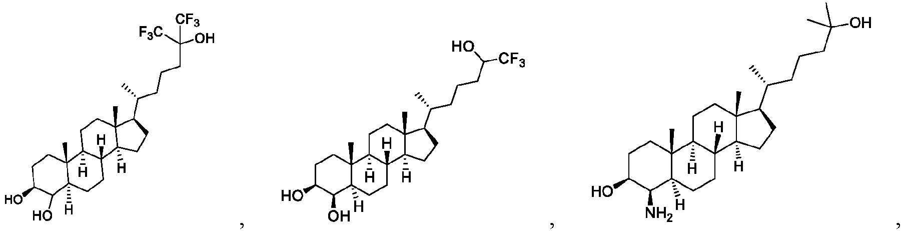 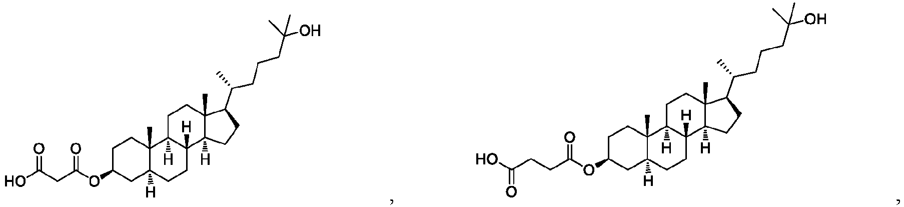 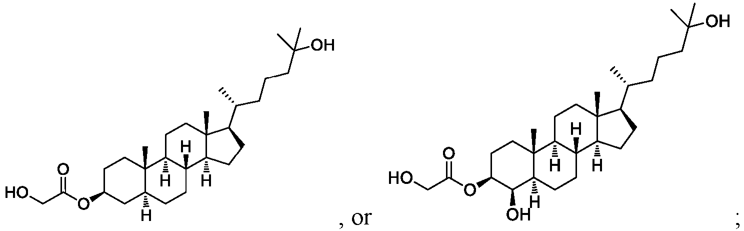
(3) the compound of formula XIX is any one of the following compounds: 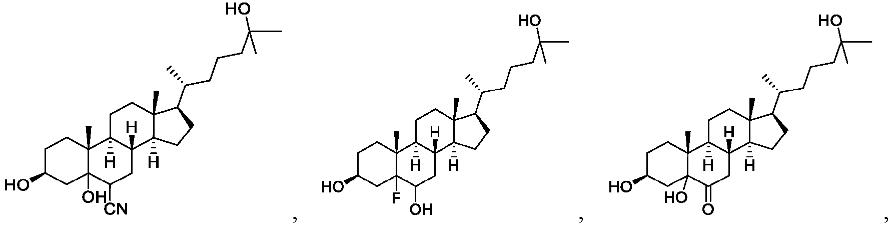 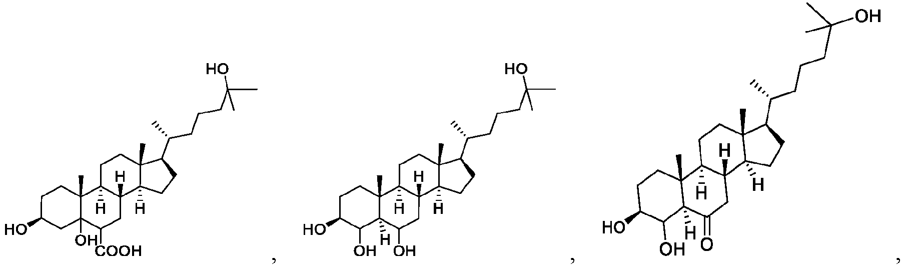 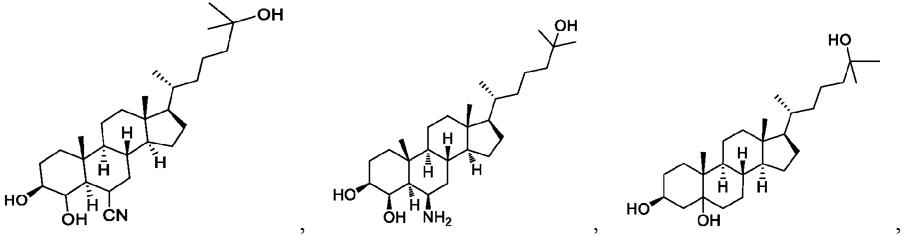 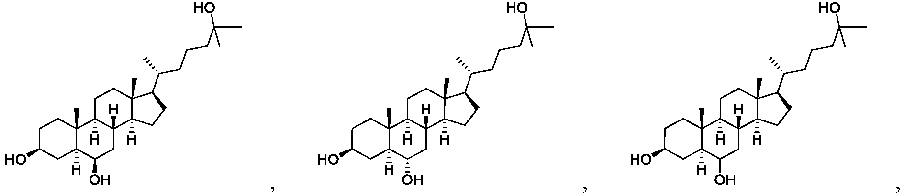 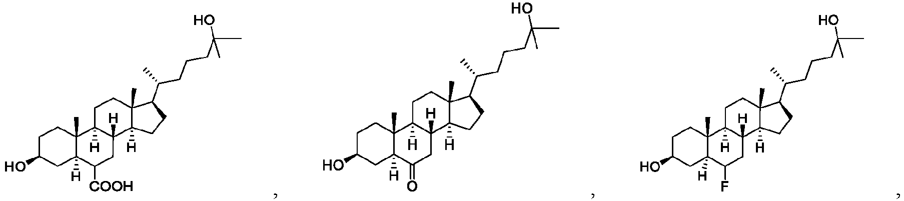 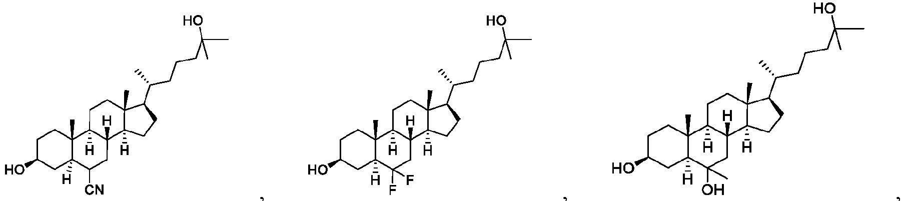 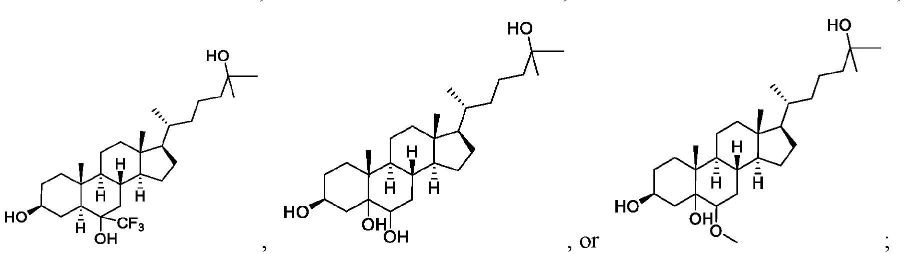
(4) the compound of formula XXIII is any one of the following compounds: 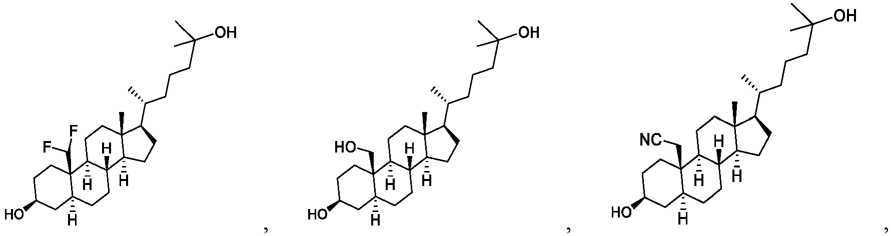 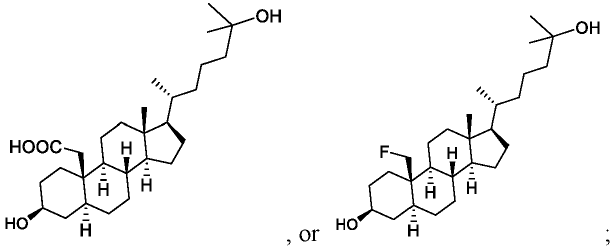
(5) the compound of formula XXIV is any one of the following compounds: 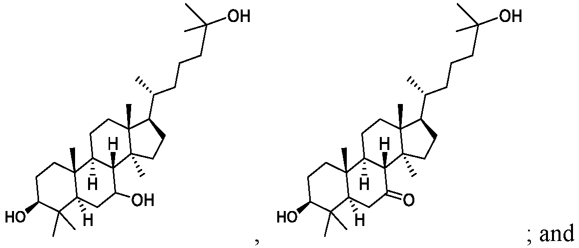
(6) the compound of formula XXVI is any one of the following compounds:

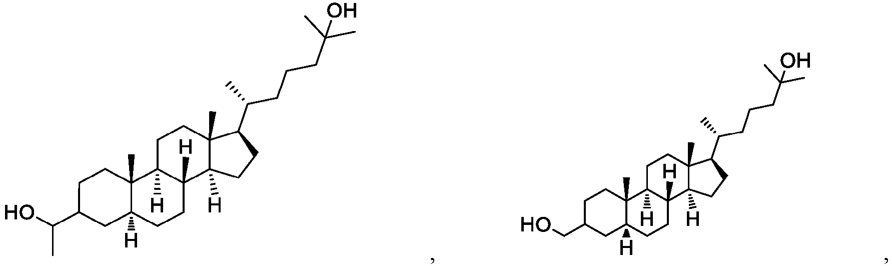

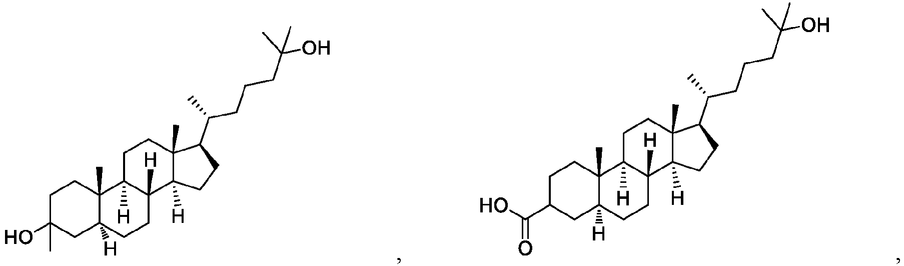

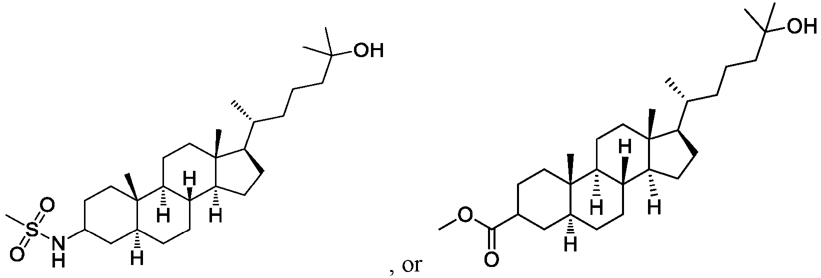

11. A pharmaceutical composition comprising the compound or the pharmaceutically acceptable salt thereof according to any one of claims 1 to 10, and at least one pharmaceutical excipient.

12. A use of the compound or the pharmaceutically acceptable salt thereof according to any one of claims 1 to 10, or the pharmaceutical composition according to claim 11 in the manufacture of a medicament for preventing and/or treating a disease, wherein the disease is obesity, hyperlipidemia, fatty liver, diabetes, atherosclerosis, cardiovascular and cerebrovascular disease, liver cancer, or skin damage.

13. A use of the compound or the pharmaceutically acceptable salt thereof according to any one of claims 1 to 10, or the pharmaceutical composition according to claim 11 in the manufacture of a medicament for SREBP pathway inhibition.

## Amended claims

### Amended claims under Art. 19.1 PCT

1. A compound or a pharmaceutically acceptable salt thereof, wherein the compound has a structure of formula XXI, XVI, XIX, XXIII, XXIV, or XXVI: 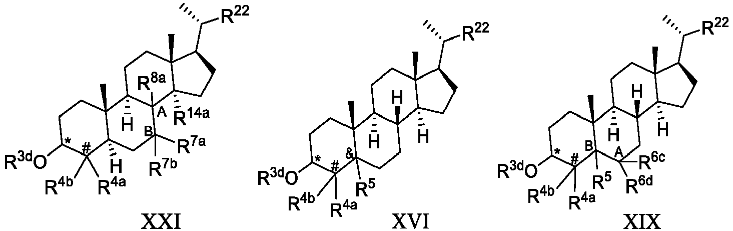 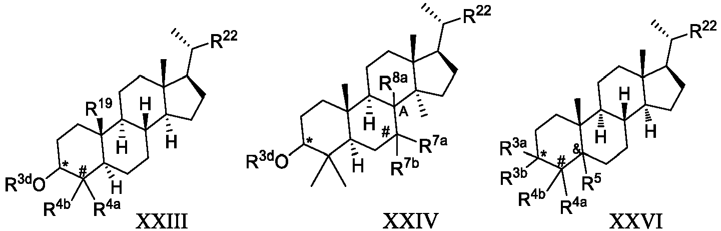 wherein R^{3d} is H or R^{3c};
R^{3c} is -S(O)₂OH, -C(O)-CH₂CH₂COOH, -C(O)-CH₂COOH, -C(O)-CH₂OH, -C(O)-COOH, -CH₂CH₂OH, or -CH₂COOH;
R^{3a} and R^{3b} are as defined in any one of the following schemes:
(i) R^{3a} is H, and R^{3b} is -NH₂, -NHS(O)₂CH₃, -NHS(O)₂CH₂CH₃, -NHS(O)₂CH₂CH₂CH₃, -NHS(O)₂CF₃, -NH-C(O)-CH₂OH, -CH₂OH, -COOH, -C(O)-OCH₃, -CH(CH₃)OH, -CH₂-C(O)-CH₂OH, or -CH(OH)-CH₂CH₂OH;
(ii) R^{3a} is CH₃, and R^{3b} is -OH; and
(iii) R^{3a} and R^{3b} together with the carbon atom to which they are attached form 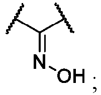
R^{4a} is H, F, CH₃, CF₃, or OH;
R^{4b} is H, F, NH₂, OH, or CF₃;
R^{4a} and R^{4b} together with the carbon atom to which they are attached form 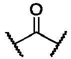
or 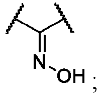
R⁵ is H or R^{5c}; R^{5c} is OH or F;
R^{6c} is H, F, CH₃, or CF₃;
R^{6d} is CN, NH₂, F, COOH, or C₁₋₄ alkoxy;
or, R^{6c} and R^{6d} together with the carbon atom to which they are attached form 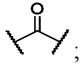
R^{7a} is H, F, NH₂, CH₃, or CF₃;
R^{7b} is F or OH;
or, R^{7a} and R^{7b} together with the carbon atom to which they are attached form 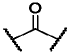
or 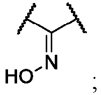
R^{8a} is H or F;
R^{14a} is H;
R¹⁹ is CH₂OH, CH₂CN, CH₂COOH, CH₂F, or CHF₂;
R²² is 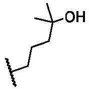
or R²¹;
R²¹ is -L¹-C(O)R^{A}, -L¹-S(O)₂R^{A}, -L¹-R^{B}, 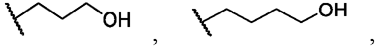 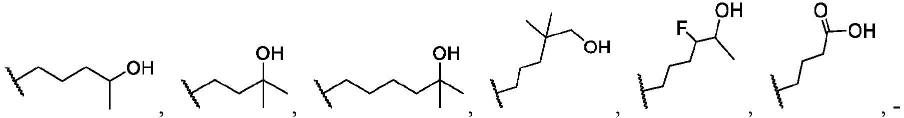 L²-R^{C}, or -L³-R^{D};
L¹ and L² are each independently a single bond, -CH₂-, -(CH₂)₂-, -(CH₂)₃-, -(CH₂)₄-, - (CH₂)₅-, or -(CH₂)₆-, wherein one -CH₂- moiety of the -CH₂-, -(CH₂)₂-, -(CH₂)₃-, -(CH₂)₄-, - (CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 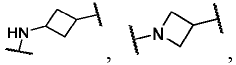 -CH=CH-, 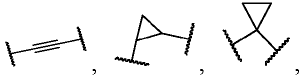 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)₂R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 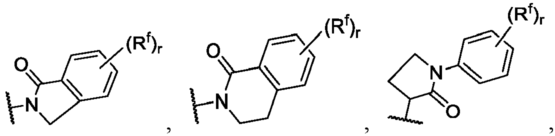 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 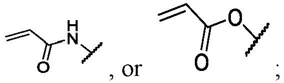
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, C₁₋₄ alkoxy, or fluoro-C₁₋₄ alkoxy;
the number of heteroatoms in the heterocycloalkyl and heteroaryl is independently 1, 2, 3, or 4, and each heteroatom is independently N, O, or S;
the carbon atom marked with * is in R configuration, S configuration, or a mixture of both;
the carbon atom marked with # is in R configuration, S configuration, or a mixture of both when it is a chiral carbon atom; the carbon atom marked with & is in R configuration, S configuration, or a mixture of both when it is a chiral carbon atom; the carbon atom marked with A is in *R* configuration, *S* configuration, or a mixture of both when it is a chiral carbon atom; the carbon atom marked with B is in *R* configuration, *S* configuration, or a mixture of both when it is a chiral carbon atom.

2. The compound or the pharmaceutically acceptable salt thereof according to claim 1, which satisfies one or more of the following conditions:
(1) in the compound of formula XXI, R^{3d} is H or R^{3c}; R^{3c} is -S(O)₂OH, -C(O)-CH₂COOH, -C(O)-CH₂OH, -C(O)-COOH, -CH₂CH₂OH, or -CH₂COOH;
R^{4a} is H, F, CH₃, CF₃, or OH;
R^{4b} is H, F, OH, CH₂OH, CN, COOH, CF₃, or cyclopropyl;
or, R^{4a} and R^{4b} together with the carbon atom to which they are attached form 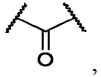 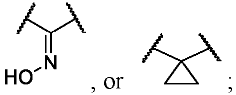
R^{7a} is H, F, CH₃, or CF₃; R^{7b} is F or OH; or, R^{7a} and R^{7b} together with the carbon atom to which they are attached form 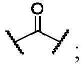
R^{8a} is H or F;
R^{14a} is H;
R²² is 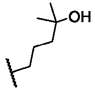
or R²¹;
R²¹ is -L¹-C(O)R^{A}, -L¹-S(O)₂R^{A}, -L¹-R^{B}, 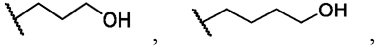 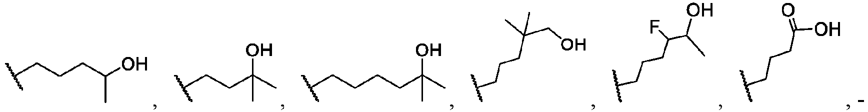 L²-R^{C}, or -L³-R^{D};
L¹ and L² are each independently a single bond, -CH₂-, -(CH₂)₂-, -(CH₂)₃-, -(CH₂)₄-, - (CH₂)₅-, or -(CH₂)₆-, wherein one -CH₂- moiety of the -CH₂-, -(CH₂)₂-, -(CH₂)₃-, -(CH₂)₄-, - (CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 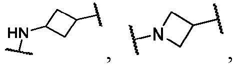 -CH=CH-, 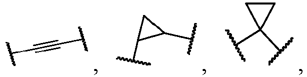 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)₂R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 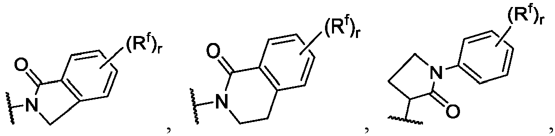 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 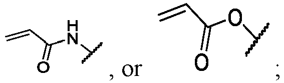
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, or C₁₋₄ alkoxy;
the number of heteroatoms in the heterocycloalkyl and heteroaryl is independently 1, 2, 3, or 4, and each heteroatom is independently N, O, or S;
in formula XXI, the carbon atom marked with * is in R configuration, S configuration, or a mixture of both; the carbon atom marked with # is in R configuration, S configuration, or a mixture of both when it is a chiral carbon atom; the carbon atom marked with A is in R configuration, S configuration, or a mixture of both when it is a chiral carbon atom; the carbon atom marked with B is in R configuration, S configuration, or a mixture of both when it is a chiral carbon atom;
(2) in the compound of formula XVI, R^{3d} is H or R^{3c};
R^{3c} is -S(O)₂OH, -C(O)-CH₂COOH, -C(O)-CH₂OH, -C(O)-COOH, -CH₂CH₂OH, or - CH₂COOH;
R^{4a} is H, F, CH₃, CF₃, or OH;
R^{4b} is H, F, OH, or CF₃;
or, R^{4a} and R^{4b} together with the carbon atom to which they are attached form 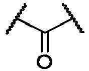 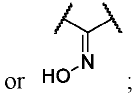
R⁵ is H or R^{5c}; R^{5c} is OH or F;
R²² is 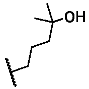 or R²¹;
R²¹ is -L¹-C(O)R^{A}, -L¹-S(O)₂R^{A}, -L¹-R^{B}, 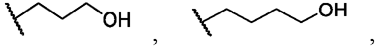 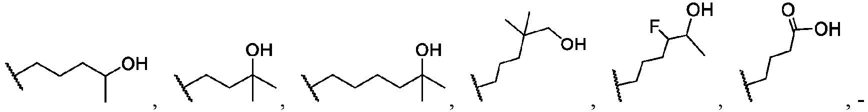 L²-R^{C}, or -L³-R^{D};
L¹ and L² are each independently a single bond, -CH₂-, -(CH₂)₂-, -(CH₂)₃-, -(CH₂)₄-, - (CH₂)₅-, or -(CH₂)₆-, wherein one -CH₂- moiety of the -CH₂-, -(CH₂)₂-, -(CH₂)₃-, -(CH₂)₄-, - (CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 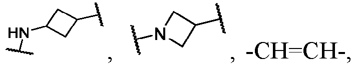 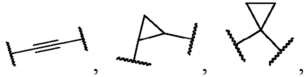 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)₂R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 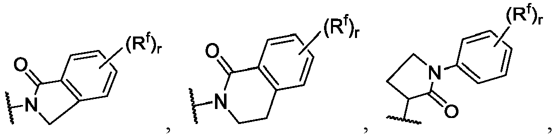 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 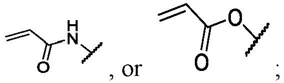
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, or C₁₋₄ alkoxy;
the number of heteroatoms in the heterocycloalkyl and heteroaryl is independently 1, 2, 3, or 4, and each heteroatom is independently N, O, or S;
in formula XVI, the carbon atom marked with * is in R configuration, S configuration, or a mixture of both; the carbon atom marked with # is in R configuration, S configuration, or a mixture of both when it is a chiral carbon atom; the carbon atom marked with & is in R configuration, S configuration, or a mixture of both when it is a chiral carbon atom;
(3) in the compound of formula XIX, R^{3d} is H or R^{3c}; R^{3c} is -S(O)₂OH, -C(O)-CH₂COOH, -C(O)-CH₂OH, -C(O)-COOH, -CH₂CH₂OH, or -CH₂COOH;
R^{4a} is H, F, CH₃, CF₃, or OH;
R^{4b} is H, F, OH, or CF₃;
or, R^{4a} and R^{4b} together with the carbon atom to which they are attached form 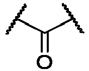 or 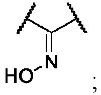
R⁵ is H or R^{5c}; R⁵ is OH or F;
R^{6c} is H, F, CH₃, or CF₃;
R^{6d} is CN, F, COOH, or C₁₋₄ alkoxy (e.g., methoxy);
or, R^{6c} and R^{6d} together with the carbon atom to which they are attached form 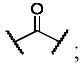
R²² is 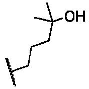 or R²¹;
R²¹ is -L¹-C(O)R^{A}, -L¹-S(O)₂R^{A}, -L¹-R^{B}, 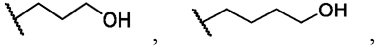 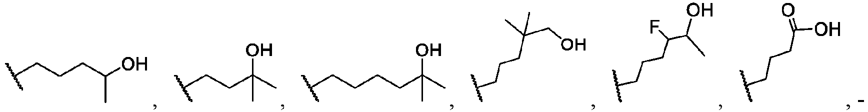 L²-R^{C}, or -L³-R^{D};
L¹ and L² are each independently a single bond, -CH₂-, -(CH₂)₂-, -(CH₂)₃-, -(CH₂)₄-, - (CH₂)₅-, or -(CH₂)₆-, wherein one -CH₂- moiety of the -CH₂-, -(CH₂)₂-, -(CH₂)₃-, -(CH₂)₄-, - (CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 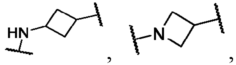 -CH=CH-, 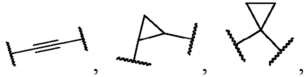 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)₂R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 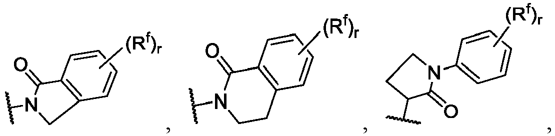 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 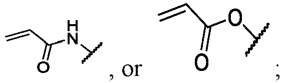
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, or C₁₋₄ alkoxy;
the number of heteroatoms in the heterocycloalkyl and heteroaryl is independently 1, 2, 3, or 4, and each heteroatom is independently N, O, or S;
in formula XIX, the carbon atom marked with * is in *R* configuration, *S* configuration, or a mixture of both; the carbon atom marked with # is in *R* configuration, *S* configuration, or a mixture of both when it is a chiral carbon atom; the carbon atom marked with A is in *R* configuration, *S* configuration, or a mixture of both when it is a chiral carbon atom; the carbon atom marked with B is in *R* configuration, *S* configuration, or a mixture of both when it is a chiral carbon atom;
(4) in the compound of formula XXIII, R^{3d} is H or R^{3c}; R^{3c} is -S(O)₂OH, -C(O)-CH₂COOH, -C(O)-CH₂OH, -C(O)-COOH, -CH₂CH₂OH, or -CH₂COOH;
R^{4a} is H, F, CH₃, CF₃, or OH;
R^{4b} is H, F, OH, or CF₃;
or, R^{4a} and R^{4b} together with the carbon atom to which they are attached form 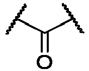 or 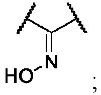
R¹⁹ is CH₂OH, CH₂CN, CH₂COOH, CH₂F, or CHF₂;
R²² is 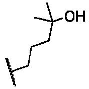 or R²¹;
R²¹ is -L¹-C(O)R^{A}, -L¹-S(O)₂R^{A}, -L¹-R^{B}, 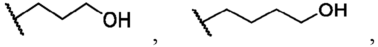 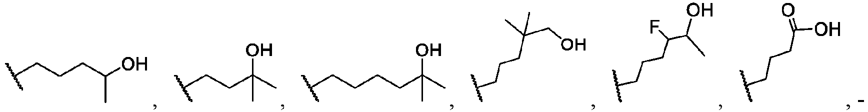 L²-R^{C}, or -L³-R^{D};
L¹ and L² are each independently a single bond, -CH₂-, -(CH₂)₂-, -(CH₂)₃-, -(CH₂)₄-, - (CH₂)₅-, or -(CH₂)₆-, wherein one -CH₂- moiety of the -CH₂-, -(CH₂)₂-, -(CH₂)₃-, -(CH₂)₄-, - (CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 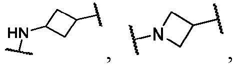 -CH=CH-, 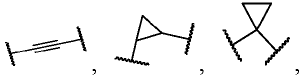 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)₂R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 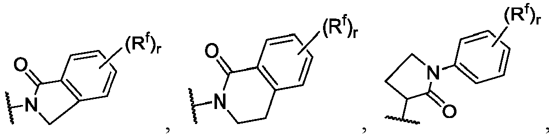 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 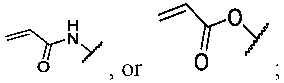
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, or C₁₋₄ alkoxy;
the number of heteroatoms in the heterocycloalkyl and heteroaryl is independently 1, 2, 3, or 4, and each heteroatom is independently N, O, or S;
in formula XXIII, the carbon atom marked with * is in *R* configuration, S configuration, or a mixture of both; the carbon atom marked with # is in *R* configuration, *S* configuration, or a mixture of both when it is a chiral carbon atom;
(5) in the compound of formula XXIV, R^{3d} is H or R^{3c}; R^{3c} is -S(O)₂OH, -C(O)-CH₂COOH, -C(O)-CH₂OH, -C(O)-COOH, -CH₂CH₂OH, or -CH₂COOH;
R^{7a} is H, F, CH₃, or CF₃; R^{7b} is F or OH; or, R^{7a} and R^{7b} together with the carbon atom to which they are attached form 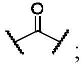
R^{8a} is H or F;
R²² is 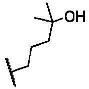 or R²¹;
R²¹ is -L¹-C(O)R^{A}, -L¹-S(O)₂R^{A}, -L¹-R^{B}, 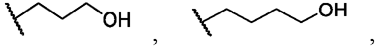 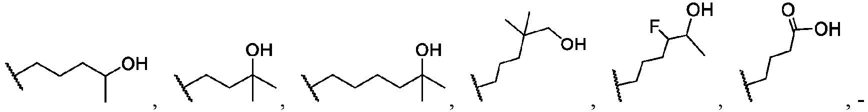 L²-R^{C}, or -L³-R^{D};
L¹ and L² are each independently a single bond, -CH₂-, -(CH₂)₂-, -(CH₂)₃-, -(CH₂)₄-, - (CH₂)₅-, or -(CH₂)₆-, wherein one -CH₂- moiety of the -CH₂-, -(CH₂)₂-, -(CH₂)₃-, -(CH₂)₄-, - (CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 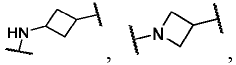 -CH=CH-, 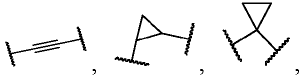 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)₂R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 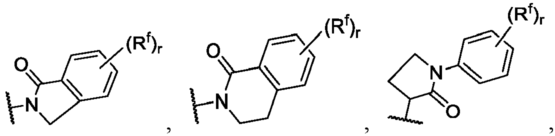 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 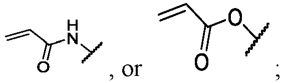
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, or C₁₋₄ alkoxy;
the number of heteroatoms in the heterocycloalkyl and heteroaryl is independently 1, 2, 3, or 4, and each heteroatom is independently N, O, or S;
in formula XXIV, the carbon atom marked with * is in R configuration, S configuration, or a mixture of both; the carbon atom marked with # is in R configuration, S configuration, or a mixture of both when it is a chiral carbon atom; the carbon atom marked with A is in *R* configuration, *S* configuration, or a mixture of both when it is a chiral carbon atom; and
(6) in the compound of formula XXVI, R^{3a} and R^{3b} are as defined in any one of the following schemes:
(i) R^{3a} is H, and R^{3b} is -NH₂, -NHS(O)₂CH₃, -NHS(O)₂CH₂CH₃, -NHS(O)₂CH₂CH₂CH₃, -NHS(O)₂CF₃, -NH-C(O)-CH₂OH, -CH₂OH, -COOH, -C(O)-OCH₃, -CH(CH₃)OH, -CH₂-C(O)-CH₂OH, or -CH(OH)-CH₂CH₂OH;
(ii) R^{3a} is CH₃, and R^{3b} is -OH; and
(iii) R^{3a} and R^{3b} together with the carbon atom to which they are attached form 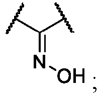
R^{4a} is H, F, CH₃, CF₃, or OH;
R^{4b} is H, F, OH, or CF₃;
or, R^{4a} and R^{4b} together with the carbon atom to which they are attached form 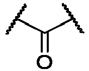 or 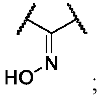
R⁵ is H or R^{5c}; R^{5c} is OH or F;
R²² is 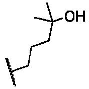 or R²¹;
R²¹ is -L¹-C(O)R^{A}, -L¹-S(O)₂R^{A}, -L¹-R^{B}, 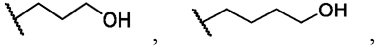 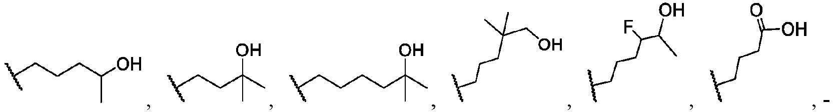 L²-R^{C}, or -L³-R^{D};
L¹ and L² are each independently a single bond, -CH₂-, -(CH₂)₂-, -(CH₂)₃-, -(CH₂)₄-, - (CH₂)₅-, or -(CH₂)₆-, wherein one -CH₂- moiety of the -CH₂-, -(CH₂)₂-, -(CH₂)₃-, -(CH₂)₄-, - (CH₂)₅-, and -(CH₂)₆- is optionally substituted by -X-;
L³ is -(CH₂)₂-, -(CH₂)₃-, -(CH₂)₄-, -(CH₂)₅-, or -(CH₂)₆-, wherein one -CH₂- moiety of the -(CH₂)₂-, -(CH₂)₃-, -(CH₂)₄-, -(CH₂)₅-, and -(CH₂)₆- is substituted by -Y-;
each X is independently -CHR^{5a}-, -CR^{5a}R^{5b}-, or -Y-;
R^{5a} and R^{5b} are each independently F, C₁₋₄ alkyl, or fluoro-C₁₋₄ alkyl;
each Y is independently -O-, -NH-, -N(C₁₋₄ alkyl)-, 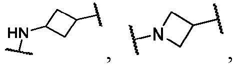 -CH=CH-, 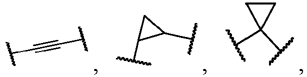 or -CHR^{g}-;
R^{g} is -C₁₋₄ alkylene-OH;
each R^{A} is independently -NR^{21a}R^{21b};
R^{B} is -N(R^{21a})-C(O)R^{21b} or -N(R^{21a})-S(O)₂R^{21b};
R^{D} is -OH, -CH₂OH, R^{C}, -CH(CH₃)-OH, or -C(CH₃)₂-OH;
each R^{C} is independently -C(R^{21c})(R^{21d})-OH, CN, -C(O)R^{E}, NH₂, C₁₋₄ alkoxy, 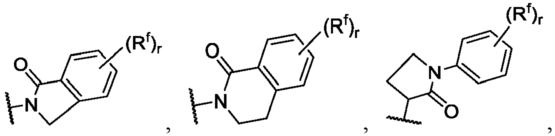 3- to 6-membered heterocycloalkyl, or 5- to 10-membered heteroaryl; the 5- to 10-membered heteroaryl is unsubstituted or substituted by p R^{c};
R^{E} is C₁₋₄ alkyl, C₁₋₄ alkoxy, C₃₋₆ cycloalkyl, or 3- to 6-membered heterocycloalkyl; wherein the C₁₋₄ alkyl, C₁₋₄ alkoxy, C₃₋₆ cycloalkyl, and 3- to 6-membered heterocycloalkyl are independently unsubstituted or substituted by m R^{a};
each R^{21a} is independently H, C₁₋₆ alkyl, C₃₋₆ cycloalkyl, or 3- to 6-membered heterocycloalkyl, wherein the C₁₋₆ alkyl, C₃₋₆ cycloalkyl, and 3- to 6-membered heterocycloalkyl are independently unsubstituted or substituted by m R^{a};
each R^{21b} is independently H, C₁₋₆ alkyl, C₃₋₁₀ cycloalkyl, 3- to 10-membered heterocycloalkyl, C₆₋₁₀ aryl, or 5- to 10-membered heteroaryl, wherein the C₁₋₆ alkyl, C₃₋₁₀ cycloalkyl, 3- to 10-membered heterocycloalkyl, C₆₋₁₀ aryl, and 5- to 10-membered heteroaryl are independently unsubstituted or substituted by q R^{b};
or, in -NR^{21a}R^{21b}, R^{21a} and R^{21b} together with the nitrogen atom to which they are attached form a 3- to 10-membered heterocycloalkyl group, wherein the 3- to 10-membered heterocycloalkyl group is unsubstituted or substituted by p R^{c};
m, p, and q are each independently 1, 2, 3, 4, or 5;
R^{a}, R^{b}, and R^{c} are each independently F, Cl, OH, COOH, CN, NO₂, C₁₋₄ alkyl, fluoro-C₁₋₄ alkyl, C₁₋₄ alkoxy, 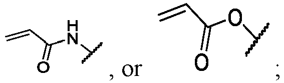
R^{21c} is H, F, C₁₋₄ alkyl, fluoro-C₁₋₄ alkyl, C₂₋₄ alkenyl, or C₃₋₆ cycloalkyl;
R^{21d} is F, C₂₋₄ alkyl, fluoro-C₁₋₄ alkyl, C₂₋₄ alkenyl, C₃₋₆ cycloalkyl, phenyl, 5- to 6-membered heteroaryl, or -L⁴-R^{21e}, wherein the phenyl and 5- to 6-membered heteroaryl are independently unsubstituted or substituted by j R^{d};
or, R^{21c} and R^{21d} together with the carbon atom to which they are attached form a C₃₋₆ cycloalkyl group or a 3- to 6-membered heterocycloalkyl group;
L⁴ is C₁₋₄ alkylene;
R^{21e} is OH, CN, C₁₋₄ alkoxy, phenyl, or 5- to 6-membered heteroaryl, wherein the phenyl and 5- to 6-membered heteroaryl are independently unsubstituted or substituted by j R^{d};
each j is independently 1, 2, 3, or 4;
each r is independently 0, 1, 2, 3, or 4;
R^{d} and R^{f} are each independently F, Cl, OH, CN, NO₂, C₁₋₄ alkyl, fluoro-C₁₋₄ alkyl, or C₁₋₄ alkoxy;
the number of heteroatoms in the heterocycloalkyl and heteroaryl is independently 1, 2, 3, or 4, and each heteroatom is independently N, O, or S;
in formula XXVI, the carbon atom marked with * is in R configuration, S configuration, or a mixture of both; the carbon atom marked with # is in R configuration, S configuration, or a mixture of both when it is a chiral carbon atom; the carbon atom marked with & is in R configuration, S configuration, or a mixture of both when it is a chiral carbon atom.

3. The compound or the pharmaceutically acceptable salt thereof according to claim 1 or 2, which satisfies one or more of the following conditions:
(1) in the compound of formula XXI, R^{3d} is H;
(2) in the compound of formula XXI, R^{4a} is H; R^{4b} is H or OH; or, R^{4a} and R^{4b} together with the carbon atom to which they are attached form 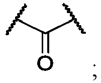 preferably, R^{4a} and R^{4b} are as defined in any one of the following schemes:
a) R^{4a} is H; R^{4b} is H;
b) R^{4a} is H; R^{4b} is OH; and
c) R^{4a} and R^{4b} together with the carbon atom to which they are attached form 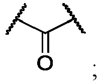
(3) in the compound of formula XXI, R^{8a} is H;
(4) in the compound of formula XXI, R^{7a} and R^{7b} are as defined in any one of the following schemes:
a) R^{7a} is H; R^{7b} is F;
b) R^{7a} is F; R^{7b} is F;
c) R^{7a} is H; R^{7b} is OH;
d) R^{7a} and R^{7b} together with the carbon atom to which they are attached form 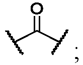
e) R^{7a} and R^{7b} together with the carbon atom to which they are attached form 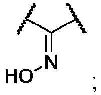 and
f) R^{7a} is H; R^{7b} is NH₂;
(5) in the compound of formula XXI, R²¹ is -L²-R^{C};
(6) in the compound of formula XXI, L² is -(CH₂)₃-;
(7) in the compound of formula XXI, each R^{C} is independently -C(R^{21c})(R^{21d})-OH;
(8) in the compound of formula XXI, in R^{C}, -C(R^{21c})(R^{21d})-OH is 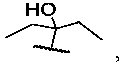 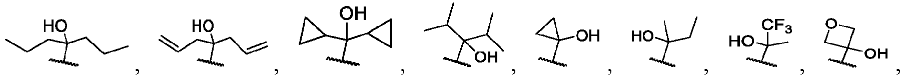 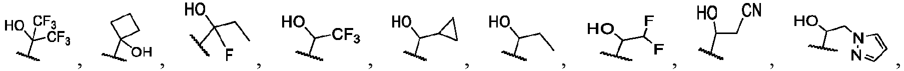 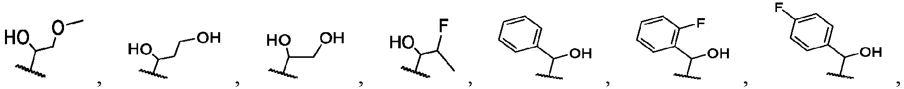 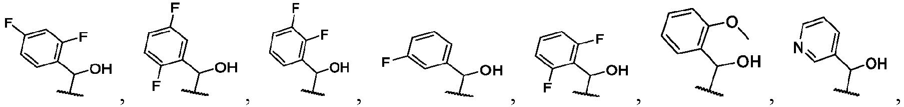 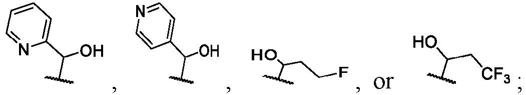 preferably, in R^{C}, -C(R^{21c})(R^{21d})-OH is 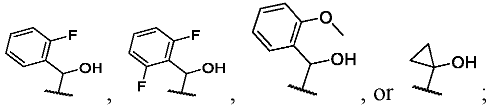
(9) in the compound of formula XVI, R^{3d} is H, -C(O)-CH₂OH, -C(O)-CH₂COOH, or - C(O)-CH₂CH₂COOH;
(10) in the compound of formula XVI, R^{4a} and R^{4b} are as defined in any one of the following schemes:
a) R^{4a} is H, R^{4b} is OH;
b) R^{4a} is CH₃, R^{4b} is F;
c) R^{4a} is F, R^{4b} is F;
d) R^{4a} is H, R^{4b} is CF₃;
e) R^{4a} is CH₃, R^{4b} is OH;
f) R^{4a} is OH, R^{4b} is CF₃;
g) R^{4a} and R^{4b} together with the carbon atom to which they are attached form 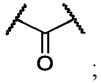
h) R^{4a} is H, R^{4b} is F;
i) R^{4a} is CF₃, R^{4b} is CF₃;
j) R^{4a} and R^{4b} together with the carbon atom to which they are attached form 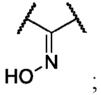
k) R^{4a} is H, R^{4b} is H; and
l) R^{4a} is H, R^{4b} is NH₂;
(11) in the compound of formula XVI, R²¹ is -L¹-C(O)R^{A}, -L²-R^{C}, or 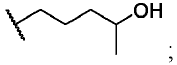
(12) in the compound of formula XVI, L¹ is -(CH₂)₂- or -CH₂-CHR^{5a}-; preferably, L¹ is - (CH₂)₂-;
(13) in the compound of formula XVI, each R^{21a} is independently methyl;
(14) in the compound of formula XVI, each R^{21b} is independently 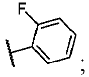
(15) in the compound of formula XVI, L² is -(CH₂)₂- or -(CH₂)₃;
(16) in the compound of formula XVI, each R^{C} is independently -C(R^{21c})(R^{21d})-OH or 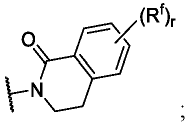
(17) in the compound of formula XVI, in R^{C}, -C(R^{21c})(R^{21d})-OH is 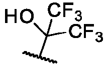 or 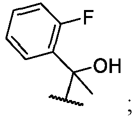
(18) in the compound of formula XVI, r is 0;
(19) in the compound of formula XIX, R^{3d} is H;
(20) in the compound of formula XIX, R^{4a} is H, R^{4b} is OH; or, R^{4a} is H, R^{4b} is H;
(21) in the compound of formula XIX, R⁵ is OH; or, R⁵ is H;
(22) in the compound of formula XIX, R^{6c} and R^{6d} are as defined in any one of the following schemes:
a) R^{6c} is H, R^{6d} is CN;
b) R^{6c} is H, R^{6d} is COOH;
c) R^{6c} is H, R^{6d} is OCH₃; and
d) R^{6c} and R^{6d} together with the carbon atom to which they are attached form 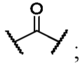
(23) in the compound of formula XIX, R²² is 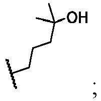
(24) in the compound of formula XXIII, R^{3d} is H;
(25) in the compound of formula XXIII, R^{4a} is H; R^{4b} is H;
(26) in the compound of formula XXIII, R²² is 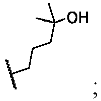
(27) in the compound of formula XXIV, R^{3d} is H;
(28) in the compound of formula XXIV, R^{7a} is H; R^{7b} is OH; or, R^{7a} and R^{7b} together with the carbon atom to which they are attached form 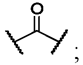
(29) in the compound of formula XXIV, R^{8a} is H;
(30) in the compound of formula XXIV, R²² is 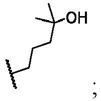
(31) in the compound of formula XXVI, R^{3a} and R^{3b} are as defined in any one of the following schemes:
a) R^{3a} is H, and R^{3b} is -NHS(O)₂CH₃, -CH₂OH, -COOH, -C(O)-OCH₃, or -CH(CH₃)OH; and
b) R^{3a} is CH₃; R^{3b} is -OH;
(32) in the compound of formula XIX, R^{4a} is H, R^{4b} is H;
(33) in the compound of formula XXVI, R⁵ is H; and
(34) in the compound of formula XXVI, R²² is

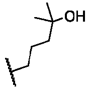

4. The compound or the pharmaceutically acceptable salt thereof according to claim 1 or 2, which satisfies one or more of the following conditions:
(1) in the compound of formula XXI, when R^{C} is -C(R^{21c})(R^{21d})-OH, R^{21c} is H, and R^{21d} is C₂₋₄ alkyl, fluoro-C₁₋₄ alkyl, C₃₋₆ cycloalkyl, phenyl, 5- to 6-membered heteroaryl, or -L⁴-R^{21e}, wherein the phenyl is optionally substituted by 1 or 2 R^{d}, and each R^{d} is independently F, OH, CN, or C₁₋₄ alkoxy;
(2) each R^{21a} is independently H, C₁₋₄ alkyl, -C₁₋₄ alkylene-OH, -C₁₋₄ alkylene-OC₁₋₄ alkyl, fluoro-C₁₋₄ alkyl, or cyclopropyl; for example, each R^{21a} is independently H, methyl, ethyl, *n-*propyl, isopropyl, n-butyl, isobutyl, sec-butyl, 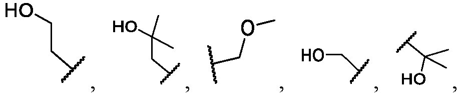 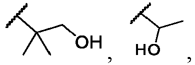 cyclopropyl, or trifluoromethyl;
preferably, each R^{21a} is independently C₁₋₄ alkyl;
(3) in the compound of formula XVI as described in any one of the schemes, each R^{21b} is independently H, C₁₋₆ alkyl, C₆₋₁₀ aryl, or 5- to 10-membered heteroaryl, wherein the C₁₋₆ alkyl, C₆₋₁₀ aryl, and 5- to 10-membered heteroaryl are independently unsubstituted or substituted by q R^{b}; for example, each R^{21b} is independently H, -C₁₋₄ alkylene-OH, -C₁₋₄ alkylene-OC₁₋₄ alkyl, fluoro-C₁₋₄ alkyl, tetrahydromorpholinyl, phenyl, pyridyl, thienyl, furyl, pyrrolyl, pyrazolyl, imidazolyl, oxazolyl, isoxazolyl, thiazolyl, isothiazolyl, thiadiazolyl, pyrimidinyl, or benzopyrazolyl, wherein the phenyl, pyridyl, thienyl, furyl, pyrrolyl, pyrazolyl, imidazolyl, oxazolyl, isoxazolyl, thiazolyl, isothiazolyl, thiadiazolyl, pyrimidinyl, and benzopyrazolyl are independently unsubstituted or substituted by q R^{b}; preferably, each R^{21b} is independently phenyl, wherein the phenyl is unsubstituted or substituted by q R^{b};
preferably, each R^{21b} is independently C₆₋₁₀ aryl, wherein the C₆₋₁₀ aryl is unsubstituted or substituted by q R^{b};
(4) in the compound of formula XVI, q is 1, 2, or 3; such as 1;
(5) in the compound of formula XVI, each R^{b} is independently F, Cl, OH, COOH, CN, NO₂, methyl, trifluoromethyl, methoxy, 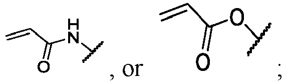 such as F; and
(6) in the compound of formula XVI, when R^{C} is -C(R^{21c})(R^{21d})-OH, R^{21c} and R^{21d} are as defined in any one of the following schemes:
a) R^{21c} is H, and R^{21d} is C₂₋₄ alkyl, fluoro-C₁₋₄ alkyl, C₃₋₆ cycloalkyl, phenyl, 5- to 6-membered heteroaryl, or -L⁴-R^{21e}, wherein the phenyl is optionally substituted by 1 or 2 R^{d}, and each R^{d} is independently F, OH, CN, or C₁₋₄ alkoxy;
b) R^{21c} and R^{21d} together with the carbon atom to which they are attached form a C₃₋₆ cycloalkyl group or a 3- to 6-membered heterocycloalkyl group (*e.g*., 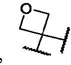 ); preferably, R^{21c} and R^{21d} together with the carbon atom to which they are attached form a cyclopropyl group, a cyclobutyl group, or 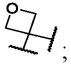 and
c) R^{21c} is CH₃ or trifluoromethyl, and R^{21d} is C₂₋₄ alkyl (*e.g.,* ethyl) or trifluoromethyl; for example, -C(R^{21c})(R^{21d})-OH is

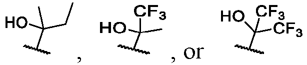

5. The compound or the pharmaceutically acceptable salt thereof according to claim 1 or 2, which satisfies one or more of the following conditions:
(1) in the compound of formula XXI, when R^{C} is -C(R^{21c})(R^{21d})-OH, R^{21c} is H, and R^{21d} is phenyl, wherein the phenyl is optionally substituted by 1 or 2 R^{d}; or R^{21c} and R^{21d} together with the carbon atom to which they are attached form a C₃₋₆ cycloalkyl group (e.g., a cyclopropyl group); each R^{d} is independently F or C₁₋₄ alkoxy;
(2) in the compound of formula XVI, each R^{A} is independently 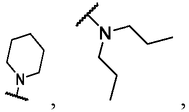 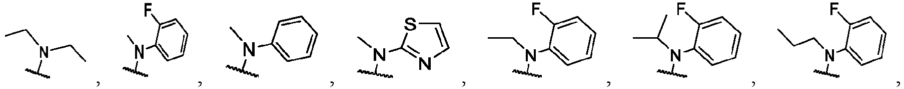 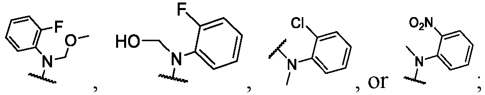 such as 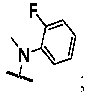 and
(3) in the compound of formula XVI, when R^{C} is -C(R^{21c})(R^{21d})-OH, R^{21c} and R^{21d} are as defined in any one of the following schemes:
a) R^{21c} is H, and R^{21d} is C₂₋₄ alkyl, fluoro-C₁₋₄ alkyl, C₃₋₆ cycloalkyl, or phenyl, wherein the phenyl is optionally substituted by 1 or 2 R^{d}, and each R^{d} is independently F, Cl, OH, fluoro-C₁₋₄ alkyl, C₁₋₄ alkoxy, or fluoro-C₁₋₄ alkoxy;
b) R^{21c} and R^{21d} together with the carbon atom to which they are attached form a C₃₋₆ cycloalkyl group; preferably, R^{21c} and R^{21d} together with the carbon atom to which they are attached form a cyclopropyl group; and
(4) R^{21c} is CH₃ or trifluoromethyl, and R^{21d} is phenyl or trifluoromethyl, wherein the phenyl is unsubstituted or substituted by j R^{d}.

6. The compound or the pharmaceutically acceptable salt thereof according to claim 1 or 2, which satisfies one or more of the following conditions:
(1) in the compound of formula XXI, -L²-R^{C} is 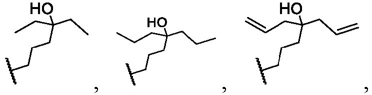 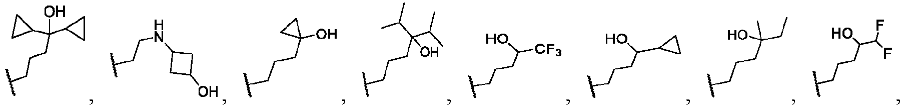 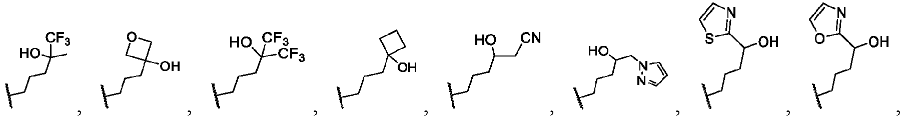 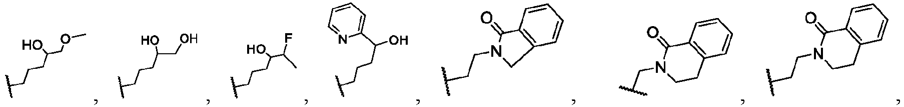 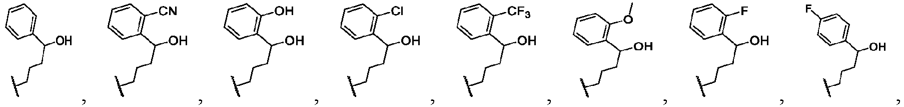 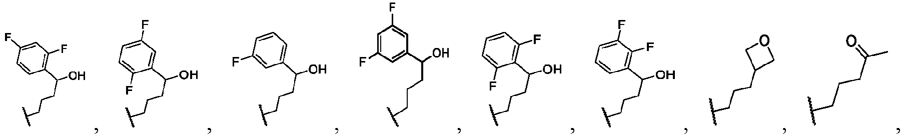 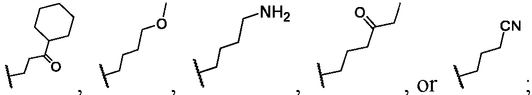 and
(2) in the compound of formula XXI, -L¹-C(O)R^{A} is 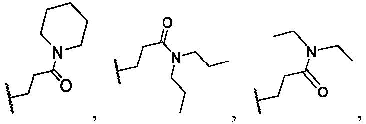 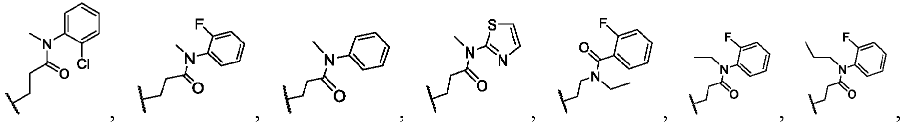 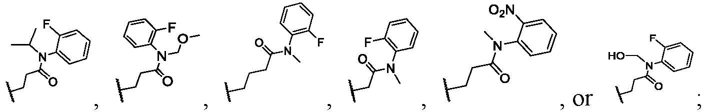 such as

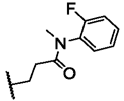

7. The compound or the pharmaceutically acceptable salt thereof according to claim 1, which satisfies one or more of the following conditions:
(1) in the compound of formula XXI, R²¹ is 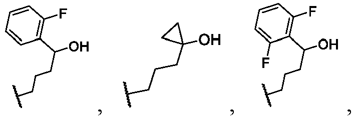 or 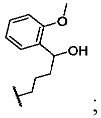 and
(2) in the compound of formula XVI, R²¹ is

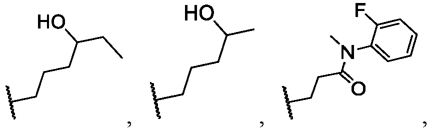

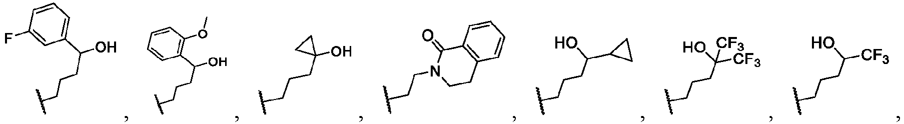

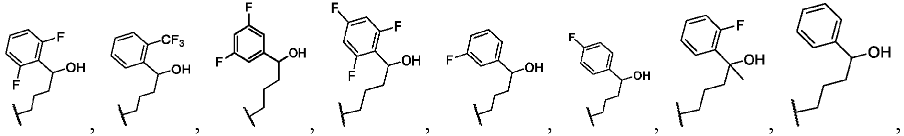

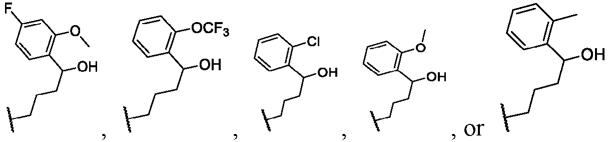

8. The compound or the pharmaceutically acceptable salt thereof according to claim 1, which satisfies one or more of the following conditions:
(1) the compound of formula XXI is a compound of formula XXI-1: 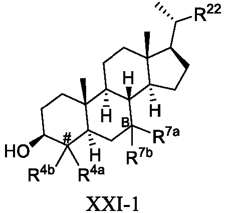
in formula XXI-1, #, B, R^{4a}, R^{4b}, R^{7a}, R^{7b}, and R²² are as defined in any one of claims 1 to 6;
preferably, the compound of formula XXI is a compound of formula XXI-2, XXI-3, or XXI-4: 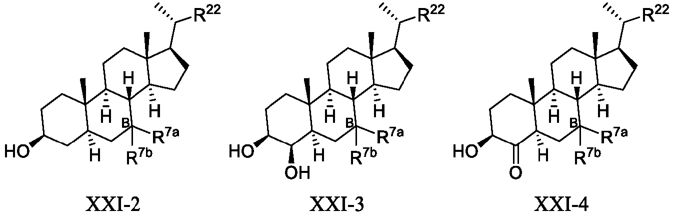
in formula XXI-2, XXI-3, or XXI-4, B, R^{7a}, R^{7b}, and R²² are as defined in any one of claims 1 to 7;
(2) the compound of formula XVI is a compound of formula XVI-1: 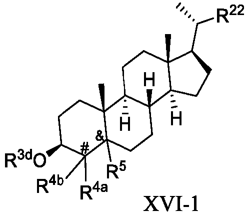 in formula XVI, #, &, R^{3d}, R^{4a}, R^{4b}, R⁵, and R²² are as defined in any one of claims 1 to 7;
(3) the compound of formula XIX is a compound of formula XIX-1: 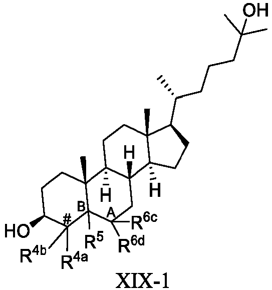 in formula XIX, #, A, B, R^{4a}, R^{4b}, R⁵, R^{6c}, and R^{6d} are as defined in any one of claims 1 to 7; and
(4) the compound of formula XXVI is a compound of formula XXVI-1: 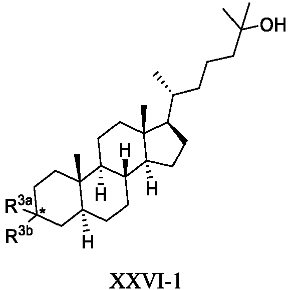 in formula XXVI, *, R^{3a}, and R^{3b} are as defined in any one of claims 1 to 7.

9. The compound or the pharmaceutically acceptable salt thereof according to claim 1 or 2, wherein the compound of formula XVI is a compound of formula VII: 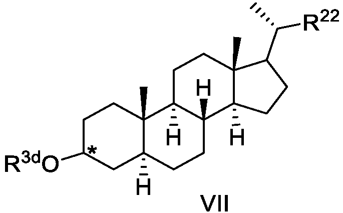 wherein R^{3d} is H or R^{3c}; R^{3c} is as defined in claims 1 to 7;
R²² is 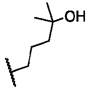 or R²¹; R²¹ is as defined in claims 1 to 7;
in formula VII, the carbon atom marked with * is in R configuration, S configuration, or a mixture of both;
preferably, R^{3d} is H;
preferably, R²² is 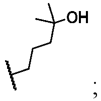
more preferably, the compound of formula VII is the following compound:

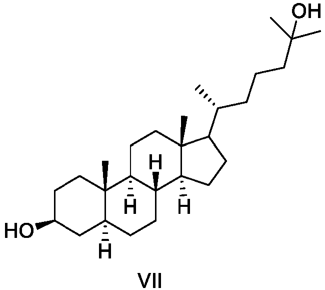

10. A compound or a pharmaceutically acceptable salt thereof, wherein the compound is any one of the following compounds:

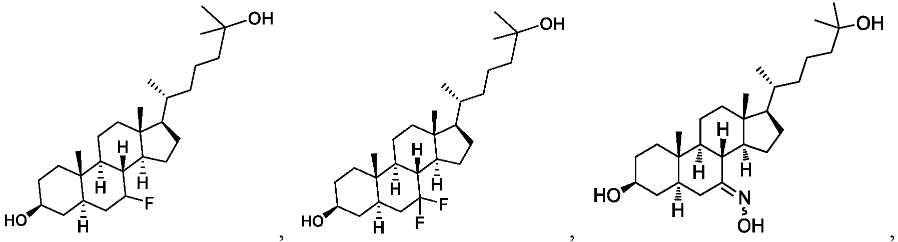

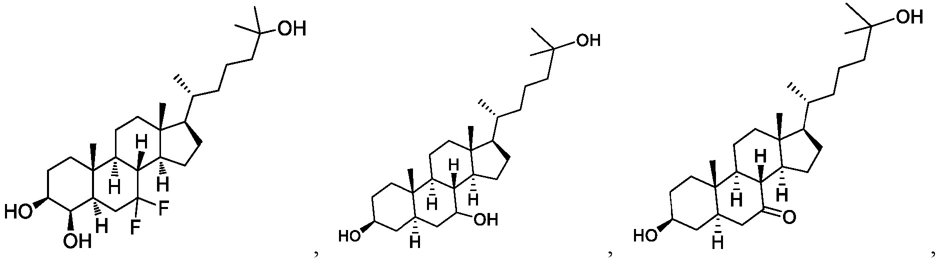

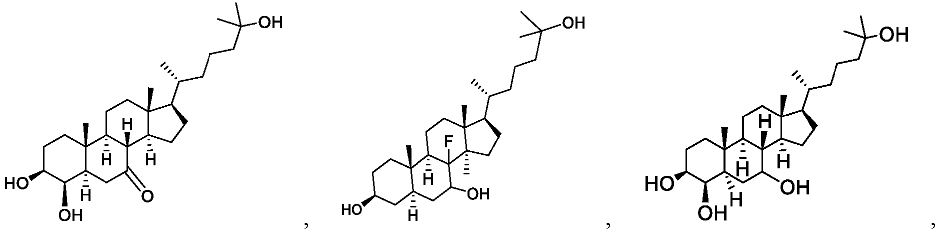

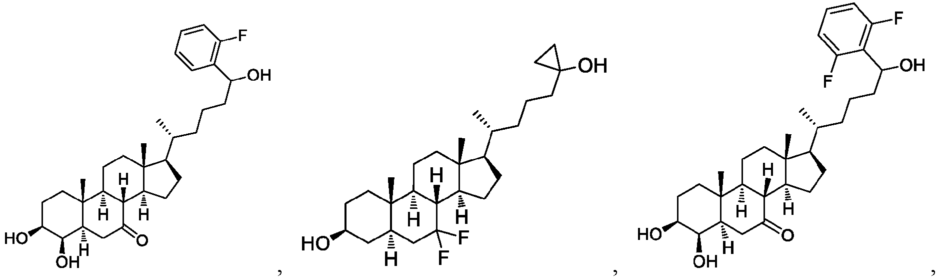

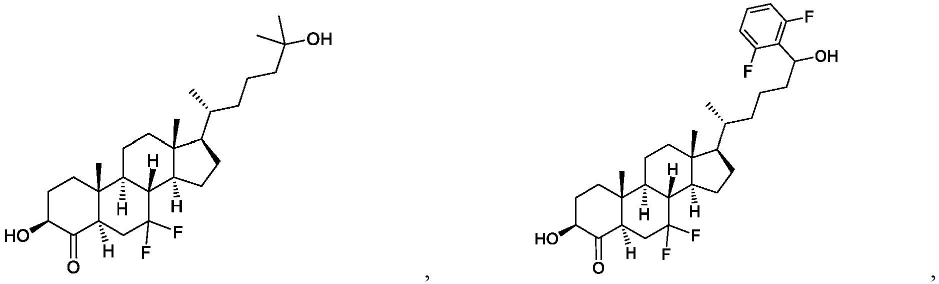

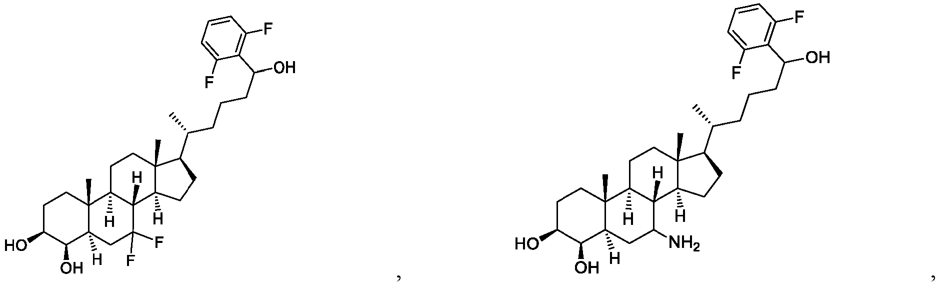

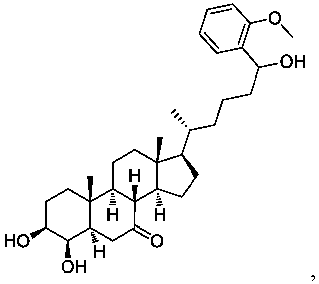

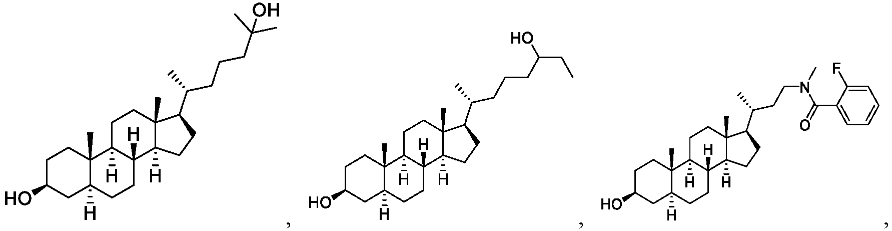

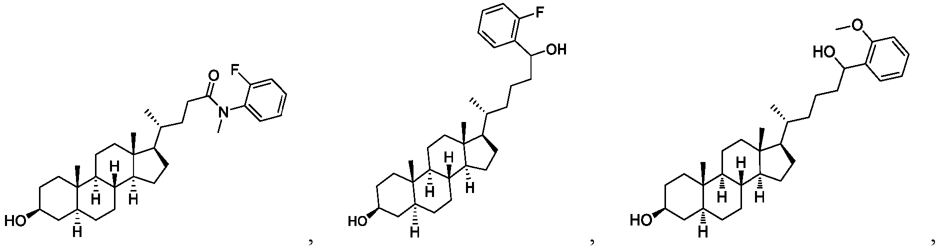

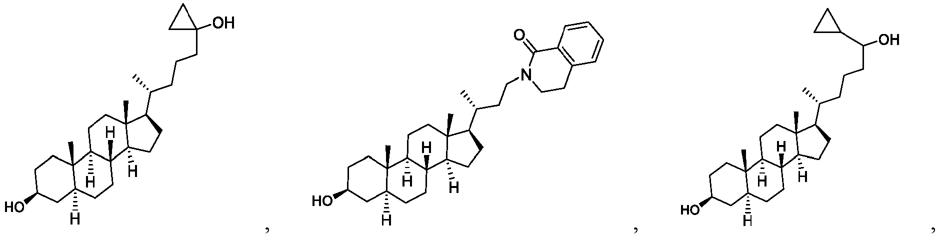

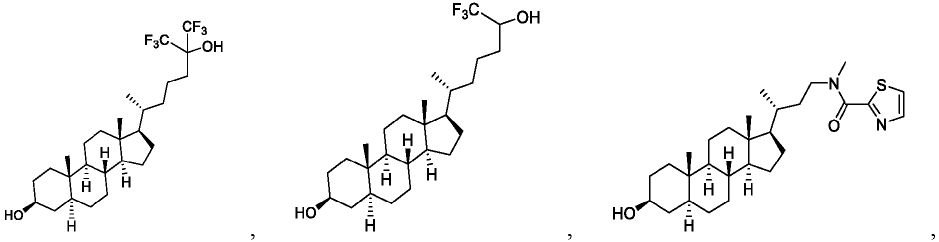

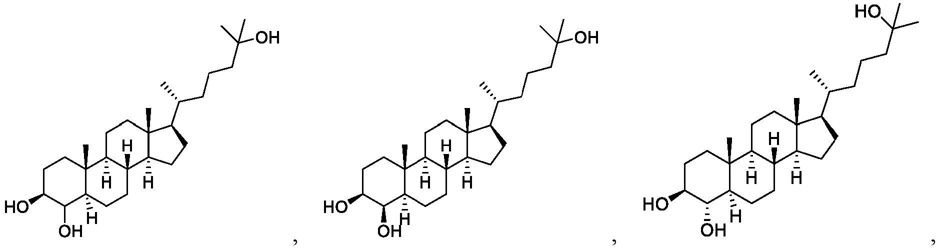

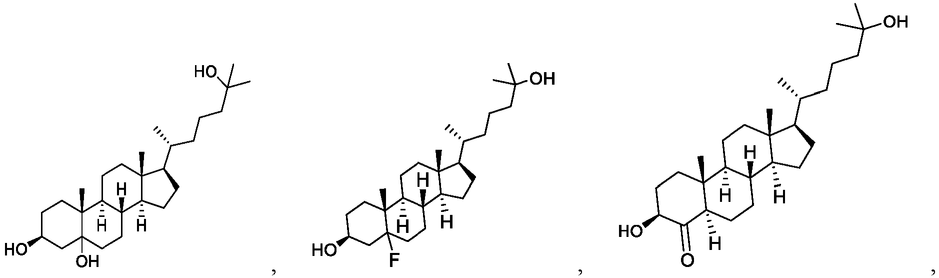

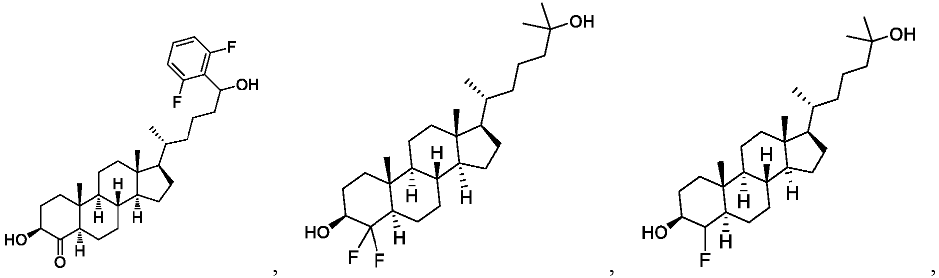

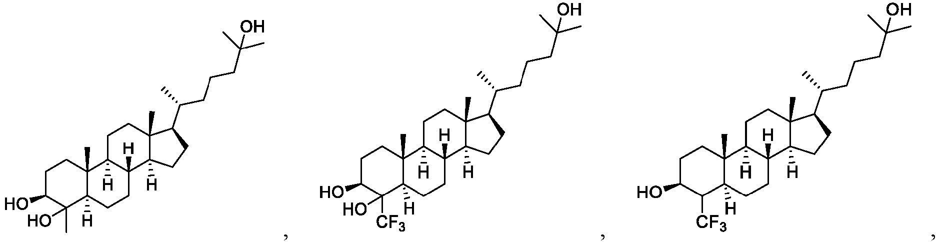

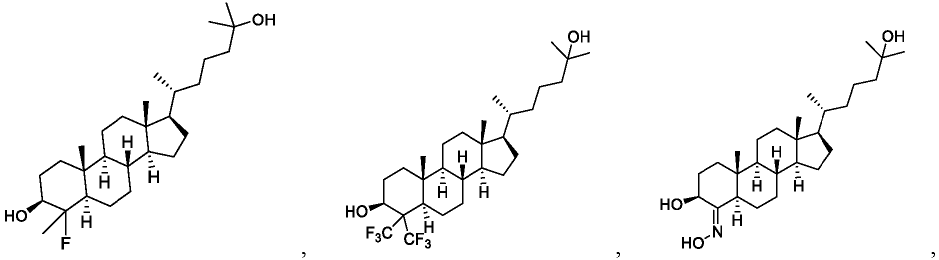

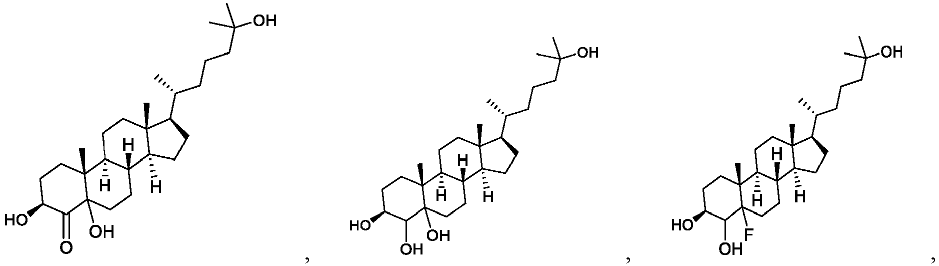

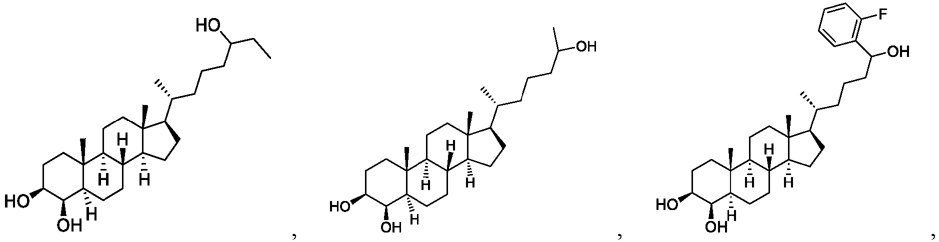

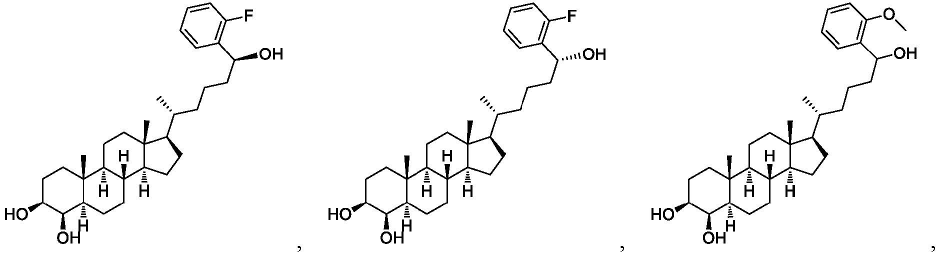

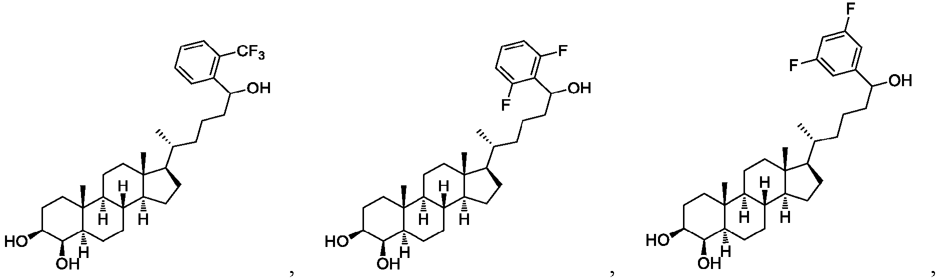

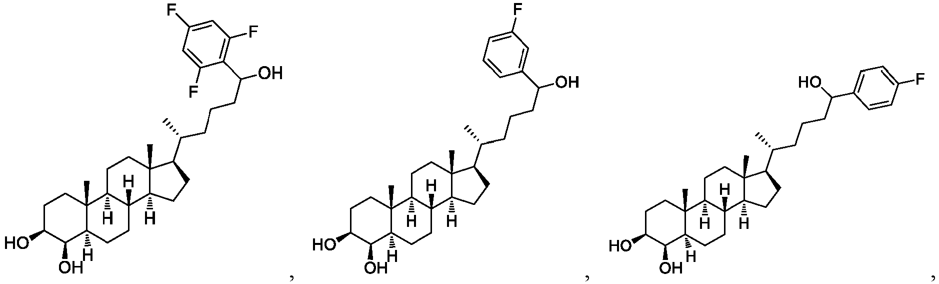

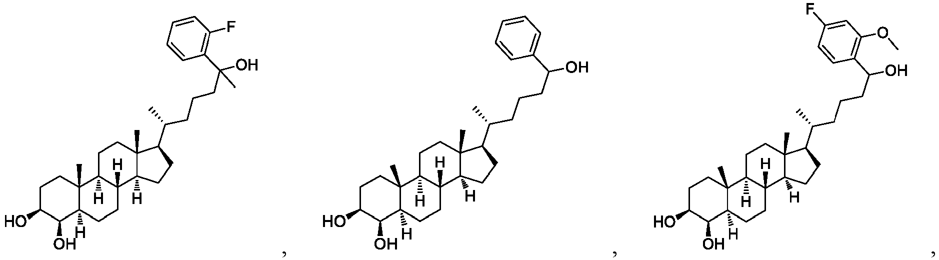

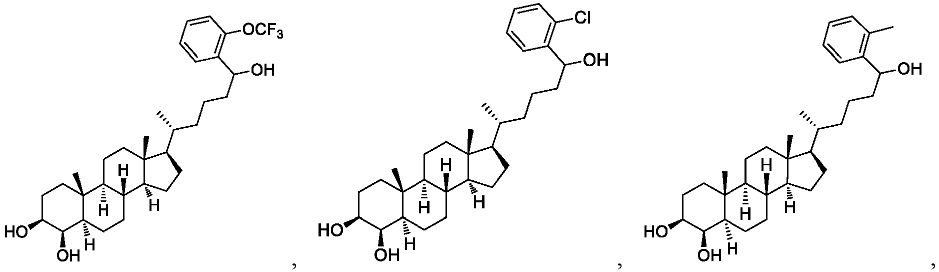

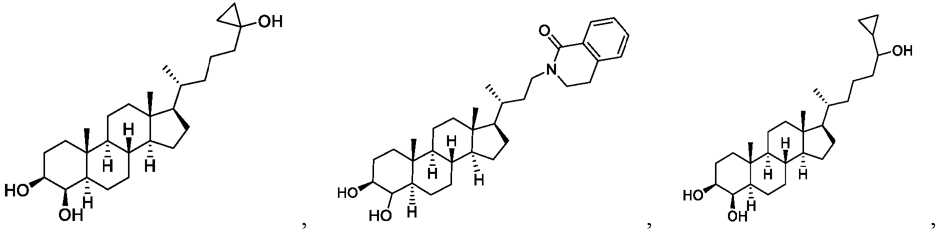

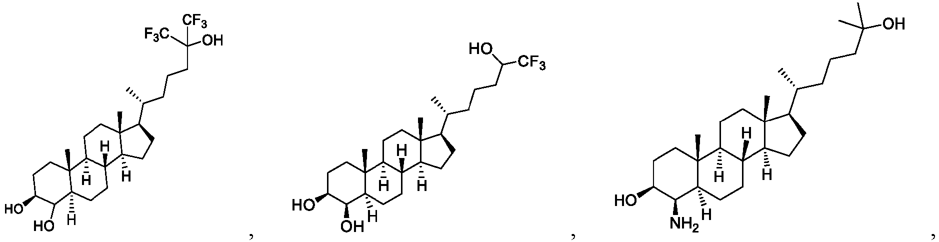

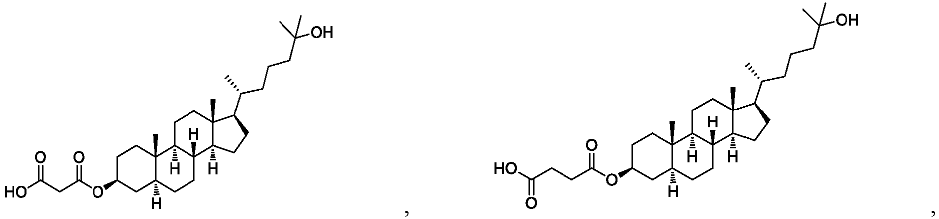

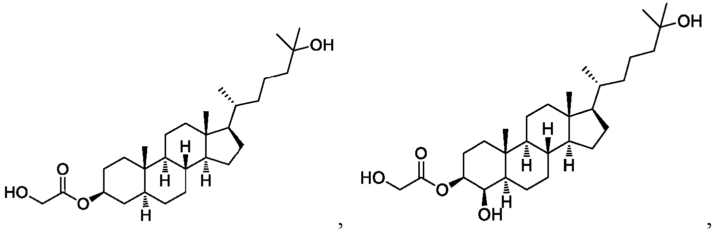

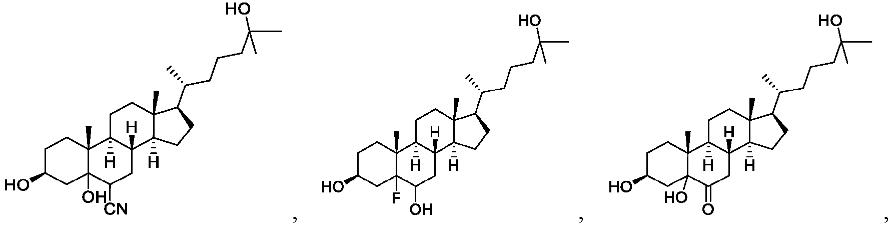

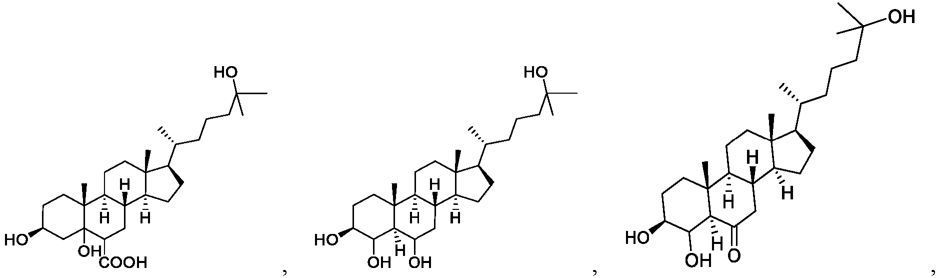

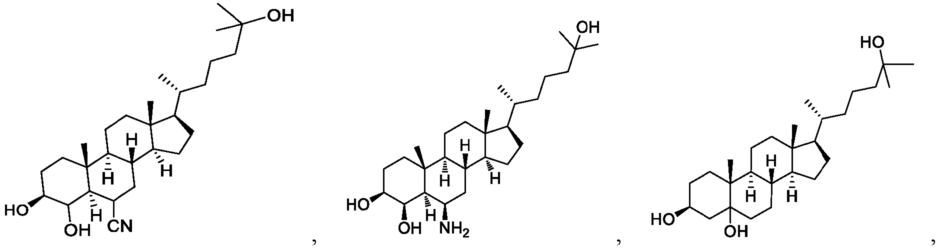

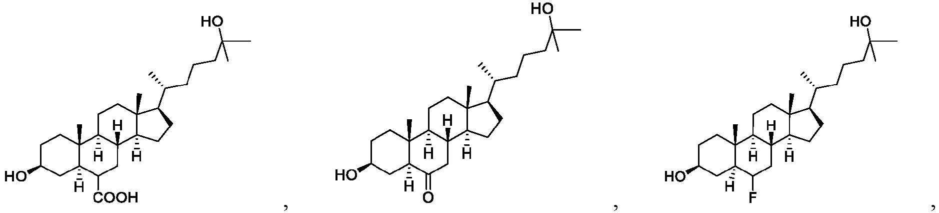

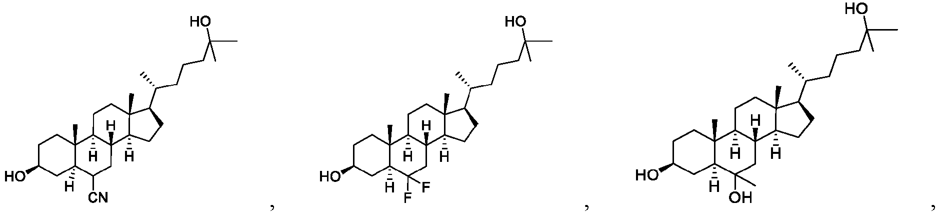

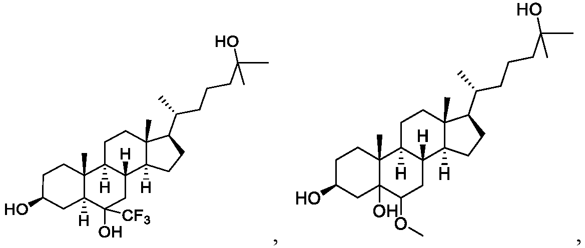

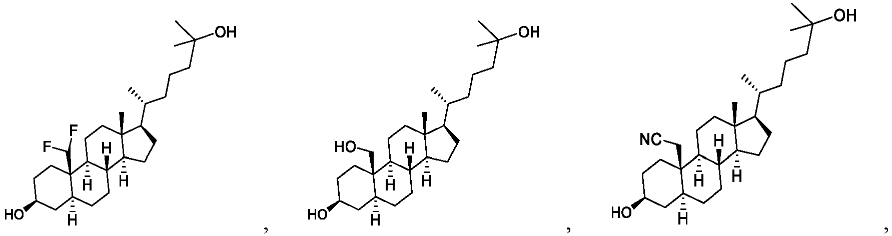

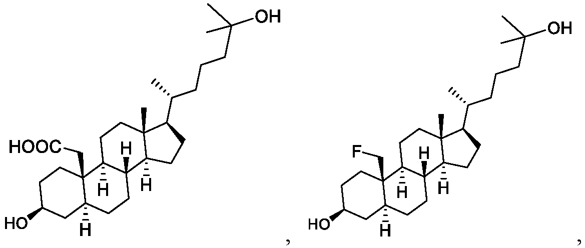

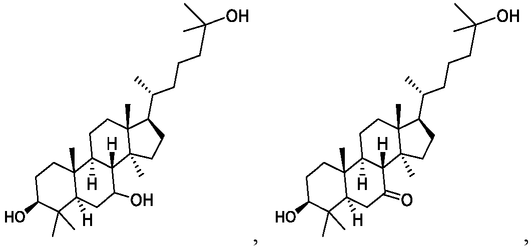

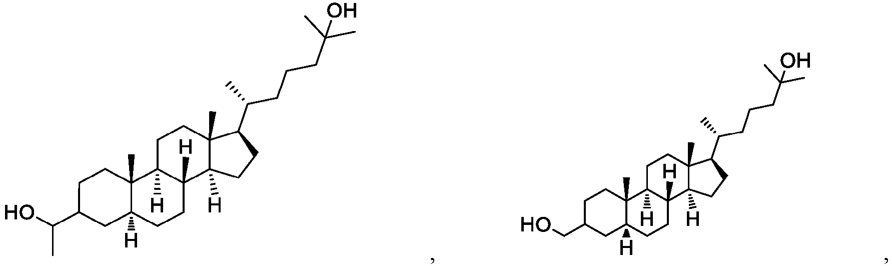

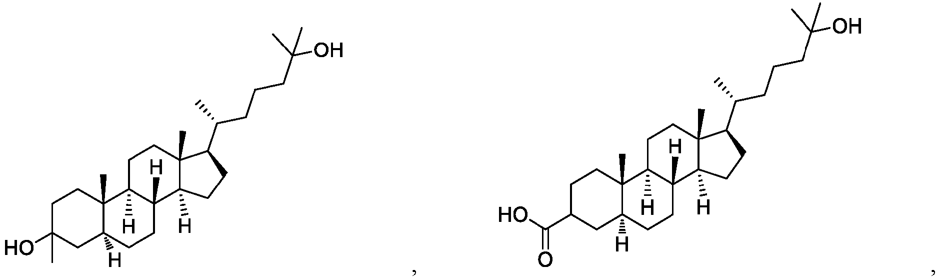

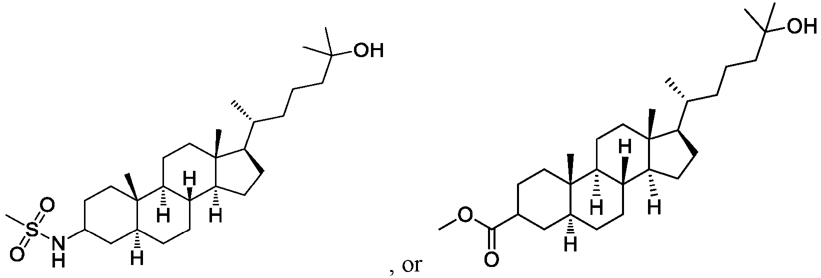

11. A pharmaceutical composition comprising the compound or the pharmaceutically acceptable salt thereof according to any one of claims 1 to 10, and at least one pharmaceutical excipient.

12. A use of the compound or the pharmaceutically acceptable salt thereof according to any one of claims 1 to 10, or the pharmaceutical composition according to claim 11 in the manufacture of a medicament for preventing and/or treating a disease, wherein the disease is obesity, hyperlipidemia, fatty liver, diabetes, atherosclerosis, cardiovascular and cerebrovascular disease, liver cancer, or skin damage.

13. A use of the compound or the pharmaceutically acceptable salt thereof according to any one of claims 1 to 10, or the pharmaceutical composition according to claim 11 in the manufacture of a medicament for SREBP pathway inhibition.
